# EUROPEAN PATENT APPLICATION

(11) **EP 4 365 180 A1**
(43) Date of publication of application: **08.05.2024**
(21) Application number: 22837651.3
(22) Date of filing: 04.07.2022
(51) Int. Cl.: C07F 9/53, C07D 213/22, C07D 213/38, C07D 215/20, C07D 221/10, C07D 221/18, C07D 311/18, C07D 311/56, C07D 311/92, C07D 403/14, C07D 405/12, C07D 407/06, C07D 409/06, C07D 471/04, C07D 471/06, G02B 5/20, H01L 31/055, H01L 33/50

(54) **RARE EARTH COMPLEX**

(30) Priority: 05.07.2021 JP 2021111199; 29.09.2021 JP 2021159217; 07.02.2022 JP 2022016953; 07.02.2022 JP 2022016954
(71) Applicant: Tosoh Corporation, Yamaguchi 746-8501 (JP); Sagami Chemical Research Institute, Ayase-shi Kanagawa 252-1193 (JP)
(72) Inventor: ARAKI Keisuke, Ayase-shi, Kanagawa 252-1193 (JP); MAEBAYASHI Haruki, Ayase-shi, Kanagawa 252-1193 (JP); MASAKI Airi, Ayase-shi, Kanagawa 252-1193 (JP); KOISO Naoyuki, Ayase-shi, Kanagawa 252-1123 (JP); NAKAGAME Ryo, Ayase-shi, Kanagawa 252-1123 (JP); YAMAMOTO Masanori, Ayase-shi, Kanagawa 252-1123 (JP); SASAKI Ippei, Ayase-shi, Kanagawa 252-1123 (JP); IWANAGA Kohei, Ayase-shi, Kanagawa 252-1123 (JP)
(74) Representative: Novagraaf International SA
(86) International application number: PCT/JP2022/026626
(87) International publication number: WO 2023/282236

(57) **Abstract**

A rare earth complex is represented by general formula (1) .

**M³⁺**•**(HC)ₙ**•**(L¹)ₘ₁**•**(L²)**ₘ₂•**(X^{L})₃₋ₙ** (1)

[In the formula, HC represents a quinolinonato ligand or coumarinato ligand, L¹ represents, for example, a nitrogen-containing ligand, L² represents, for example, water, X^{L} represents, for example, a halide ion, M³⁺ represents a trivalent rare earth metal ion, n represents 1, 2 or 3, m¹ represents 0, 1 or 2, and m² represents 0 to 3.]

## Description

### Technical Field

The present invention relates to a rare earth complex excitable with blue light and superior in light resistance at the same time.

### Background Art

Optoelectronics, such as optical communication and displays, and optical energy conversion industries, such as solar cells, are key technologies of the next generation. Types of inorganic glass materials, ceramic materials, laser materials, organic low-molecular-weight light-emitting materials, wavelength conversion materials and other materials for these technologies have been created.

Wavelength conversion materials are materials that absorb light at a particular wavelength and emit light at another wavelength and can be used as optical materials through the addition to a resin material.

In recent years, rare earth complexes having a β-diketonato ligand and a phosphine oxide ligand (e.g., PTL 1 and 2) and a rare earth complex having a phenanthroline ligand (e.g., NPL 1) have been reported as such wavelength conversion materials. These rare earth complexes are capable of absorbing short-wavelength light, such as ultraviolet light, and emitting longer-wavelength visible light and thus are expected to enable the creation of light-emitting devices for different colors of light by combining them with semiconductor light-emitting elements, such as light-emitting diodes (LEDs) (e.g., PTL 3).

Many of the above rare earth complexes are excited by ultraviolet light (having a wavelength of approximately 280 nm to approximately 400 nm) and emit light. These rare earth complexes, however, are not excited and do not emit light with blue light (approximately 450 nm to approximately 495 nm), which carries a smaller amount of energy. This prevents the use of a blue LED, which is a commonly used excitation light source, as a light source for them. In response to this, rare earth complexes excitable with blue light are under active development (e.g., NPL 2 and 3).

At the same time, it is generally known that rare earth complexes decompose under light irradiation conditions. The application of a rare earth complex to industrial products faces the challenge of its photostability. Although it has been reported that rare earth complexes having 4-hydroxy-1,5-naphthyridine as a ligand exhibit high light resistance (e.g., NPL 4), the excitation wavelength for these rare earth complexes is limited to ultraviolet light; they cannot be excited with blue light.

In NPL 5 and 6, furthermore, rare earth complexes having a quinolinone skeleton are disclosed. These rare earth complexes, however, are totally different from the rare earth complex having a quinolinone skeleton described herein in that the latter lacks a 2-nitroacyl group, a functional group prone to photodegradation. In addition, NPL 5 and 6 include no information about the light resistance of these complexes.

### Citation List

### Patent Literature

PTL 1: Soviet Union Patent No. 1453860
PTL 2: Japanese Unexamined Patent Application Publication No. 2003-81986
PTL 3: Japanese Unexamined Patent Application Publication No. 2005-15564

### Non Patent Literature

NPL 1: CrystEngComm, vol. 11, page 1197 (2009)
NPL 2: Coordination Chemistry Reviews, vols. 293-294, page 19 (2015)
NPL 3: Angewandte Chemie International Edition, vol. 43, page 5010 (2004)
NPL 4: Advanced Functional Materials, vol. 26, page 2085 (2016)
NPL 5: Applied Organometallic Chemistry, vol. 33, issue 10, page e5131 (2019)
NPL 6: Journal of Molecular Structure, vol. 1190, issue 15, page 68 (2019)

### Summary of Invention

### Technical Problem

An object of the present invention is to provide a rare earth metal complex excitable with blue light and having high light resistance at the same time, its intermediate and methods for manufacturing them.

### Solution to Problem

After extensive research to solve the above problem, the inventors found that a rare earth complex into which particular ligands have been introduced is excitable with blue light and has high light resistance at the same time, thereby having completed the present invention.

That is, the present invention provides (I) the rare earth complex of [1] to [27], (II) method 1 for manufacturing a rare earth complex, (III) method 2 for manufacturing a rare earth complex and (IV) the optical materials of [A] to [E].

(I)
   [1] A rare earth complex represented by general formula (1) .
      [Chem. 1]

      **M³⁺**·(**HC**)**ₙ·(L¹)ₘ₁·(L²)ₘ₂·**(**X^{L}**)**₃₋ₙ** **(1)**

      {HC is a quinolinonato ligand represented by general formula (1qu) or coumarinato ligand represented by general formula (1cu). 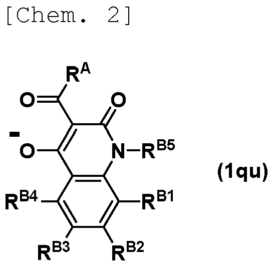 [In the formula, R^{A} represents a C1 to C6 haloalkyl group, C6 to C22 aryl group or C3 to C20 heteroaryl group. The aryl group and the heteroaryl group are optionally substituted with one or more substituents selected from the group consisting of C1 to C6 fluoroalkyl groups; C1 to C6 alkyloxy groups; C1 to C6 fluoroalkyloxy groups; C5 to C14 aryloxy groups; C1 to C6 monoalkylamino groups; C6 to C12 monoarylamino groups; C4 to C12 monoheteroarylamino groups; C2 to C12 dialkylamino groups; C10 to C24 diarylamino groups optionally substituted with one or more cyano groups, halogen atoms or C1 to C6 fluoroalkyl groups; C8 to C24 diheteroarylamino groups optionally substituted with one or more fluorine atoms or C1 to C6 fluoroalkyl groups; C3 to C20 heteroaryl groups; C2 to C13 acyl groups; methylenedioxy groups; ethylenedioxy groups; halogen atoms; hydroxyl groups; nitro groups and cyano groups.

R^{B1}, R^{B2}, R^{B3}, R^{B4} and R^{B5} each independently represent a hydrogen atom, halogen atom, C1 to C10 alkyl group, C2 to C4 alkenyl group, C1 to C6 haloalkyl group, C6 to C22 aryl group, C3 to C20 heteroaryl group, cyano group, nitro group, C7 to C14 aralkyl group optionally substituted with one or more halogen atoms, substituted or unsubstituted amino group indicated by general formula (BA1) below, substituted or unsubstituted oxy group indicated by general formula (BO1) below or substituted or unsubstituted thio group indicated by general formula (BS1) below, with the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, cyano groups and nitro groups.

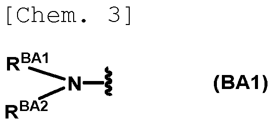

(In the formula, R^{BA1} and R^{BA2} each independently represent a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups. R^{BA1} and R^{BA2} optionally form a five-membered, six-membered or seven-membered ring including the nitrogen atom to which the R^{BA1} and R^{BA2} are bound.)

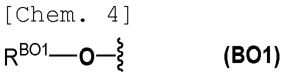

(In the formula, R^{BO1} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)

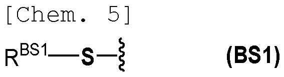

(In the formula, R^{BS1} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)

Of R^{B1}, R^{B2}, R^{B3} and R^{B4}, two adjacent substituents optionally form a five-membered, six-membered or seven-membered ring together with the benzene ring to which the substituents are bound.

R^{B5} and R^{B1} optionally form a five-membered, six-membered or seven-membered ring together with the quinolinone ring system to which the R^{B5} and R^{B1} are bound.]

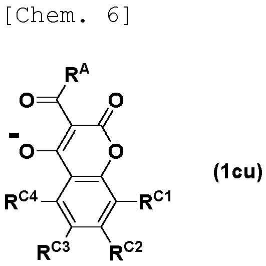

[In the formula, R^{A} is synonymous with R^{A} in general formula (1qu).

R^{C1}, R^{C2}, R^{C3} and R^{C4} each independently represent a hydrogen atom, halogen atom, C1 to C10 alkyl group, C2 to C4 alkenyl group optionally substituted with one or more C1 to C6 alkyloxy groups, C1 to C6 haloalkyl group, C6 to C22 aryl group, C3 to C20 heteroaryl group, cyano group, nitro group, C7 to C14 aralkyl group optionally substituted with one or more halogen atoms, substituted or unsubstituted amino group indicated by general formula (BA1) below, substituted or unsubstituted oxy group indicated by general formula (BO1) below or substituted or unsubstituted thio group indicated by general formula (BS1) below, with the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, cyano groups and nitro groups.

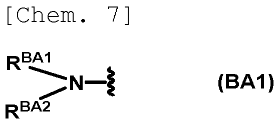

(In the formula, R^{BA1} and R^{BA2} are synonymous with the meanings described above.)

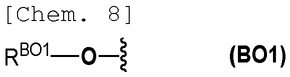

(In the formula, R^{BO1} is synonymous with the meaning described above.)

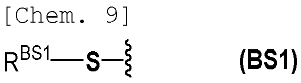

(In the formula, R^{BS1} is synonymous with the meaning described above.)

Of R^{C1}, R^{C2}, R^{C3} and R^{C4}, two adjacent substituents optionally form a five-membered, six-membered or seven-membered ring together with the benzene ring to which the substituents are bound.]

n represents 1, 2 or 3. When n is 2 or 3, the multiple quinolinonato ligands or coumarinato ligands may be the same or different from each other.

m¹ represents 0, 1 or 2, m² represents 0 to 3, and 0 ≤ m¹ + m² ≤ 3.

L¹ represents a nitrogen-containing ligand having two or more nitrogen atoms possessing a lone pair or phosphorus ligand represented by general formula (3a) below or general formula (3b) below. When m¹ is 2, the L¹s may be the same or different from each other.

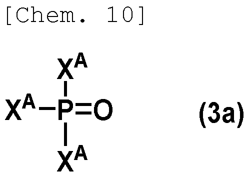

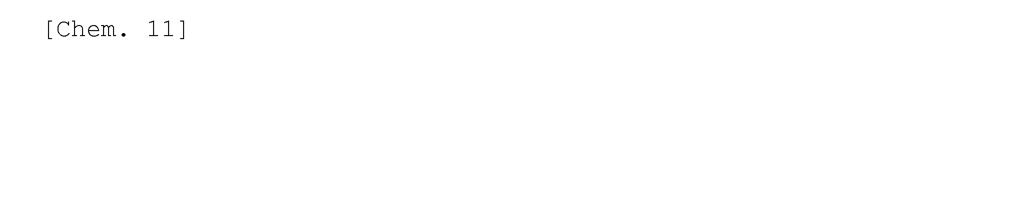

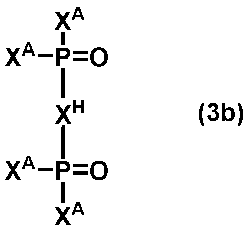

[In the formulae, the X^{A}s each independently represent a C1 to C10 alkyl group, aryl group represented by general formula (3c) below, substituted or unsubstituted amino group indicated by general formula (BA2) below or substituted or unsubstituted oxy group indicated by general formula (BO2) below.

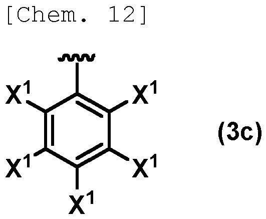

(In the formula, the X¹s each independently represent a hydrogen atom, halogen atom, C1 to C6 alkyl group, C1 to C6 alkyloxy group, C6 to C22 aryl group, C3 to C20 heteroaryl group, C5 to C14 aryloxy group, C1 to C6 haloalkyl group or C1 to C6 haloalkyloxy group, with the aryl group optionally being substituted with one or more substituents selected from the group consisting of halogen atoms, C1 to C6 alkyloxy groups, C1 to C6 haloalkyl groups, C1 to C6 haloalkyloxy groups, a hydroxyl group, hydroxyl groups, cyano groups and nitro groups.)

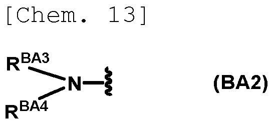

(In the formula, R^{BA3} and R^{BA4} each independently represent a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups. R^{BA3} and R^{BA4} optionally form a five-membered, six-membered or seven-membered ring including the nitrogen atom to which the R^{BA3} and R^{BA4} are bound.)

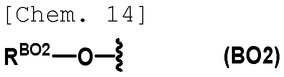

(In the formula, R^{BO2} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)

X^{H} represents a C1 to C10 alkylene group, C2 to C10 alkenylene group, C2 to C10 alkynylene group, C5 to C24 arylene group or C4 to C23 heteroarylene group.]

L² represents a neutral ligand selected from the group consisting of water, deuterium oxide, a sulfoxide compound, sulfone compound, amide compound, nitrile compound, ester compound, carbonyl compound, ether compound and alcohol.

When m² is 2 or 3, the L²s may be the same or different from each other.

X^{L} represents a halide ion, nitrate ion, carboxylate ion, sulfonate ion or C5 to C12 β-diketonato ion.

M³⁺ represents a trivalent rare earth metal ion.}

[2] The rare earth complex according to [1], wherein HC is a quinolinonato ligand represented by general formula (1qu), and n is 3.

[3] The rare earth complex according to [1] or [2], wherein HC is a quinolinonato ligand represented by general formula (1qu), and M³⁺ is a europium(III) ion, terbium(III) ion, samarium(III) ion or gadolinium(III) ion.

[4] The rare earth complex according to any of [1] to [3], wherein HC is a quinolinonato ligand represented by general formula (1qu), and R^{A} is a phenyl group optionally substituted with one or more substituents selected from the group consisting of fluorine atoms, diphenylamino group or C1 to C6 fluoroalkyl groups, with the diphenylamino group optionally being substituted with one or more fluorine atoms, C1 to C6 fluoroalkyl groups or cyano groups.

[5] The rare earth complex according to any of [1] to [4], wherein the general formula (1qu) is a quinolinonato ligand indicated by any of general formulae (1qu-1) to (1qu-8) below.

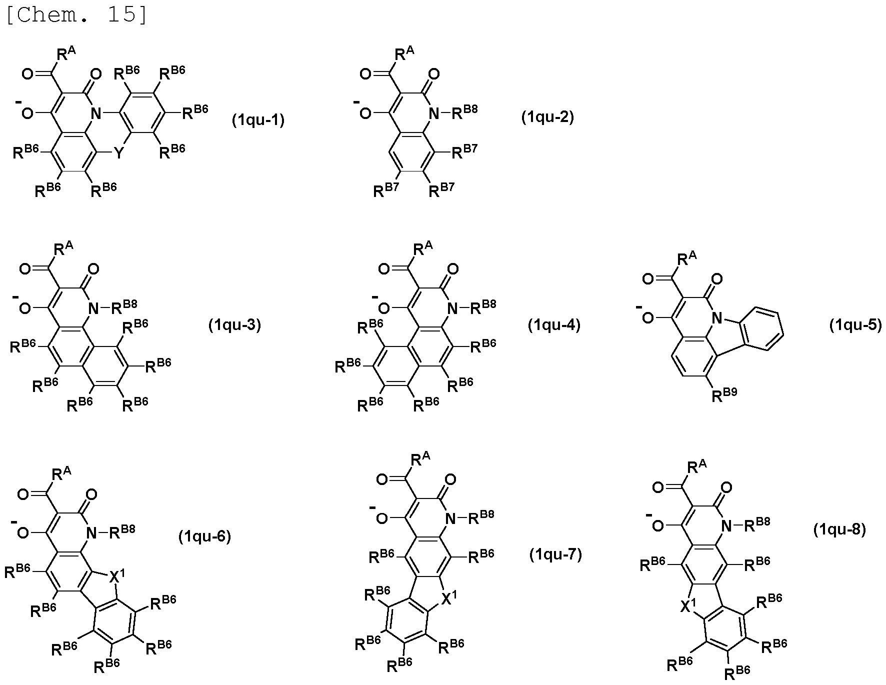

[In the formulae, R^{A} is synonymous with R^{A} in general formula (1qu).

R^{B6}s each independently represent a hydrogen atom, halogen atom or C1 to C4 fluoroalkyl group.

R^{B7}s each independently represent a hydrogen atom, halogen atom, cyano group, C1 to C8 alkyl group, C1 to C4 fluoroalkyl group, C6 to C22 aryl group, substituted or unsubstituted amino group indicated by general formula (BA3) below or substituted or unsubstituted oxy group indicated by general formula (BO3) below.

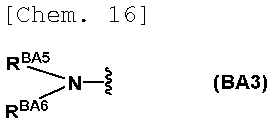

(In the formula, R^{BA5} and R^{BA6} each independently represent a C1 to C4 alkyl group or C6 to C12 aryl group, with the alkyl group and the aryl group optionally being substituted with one or more substituents selected from the group consisting of halogen atoms, C1 to C4 alkyloxy groups, C1 to C4 fluoroalkyl groups, C2 to C13 acyl groups, cyano groups and nitro groups. R^{BA5} and R^{BA6} optionally form a five-membered, six-membered or seven-membered ring including the nitrogen atom to which the R^{BA5} and R^{BA6} are bound.)

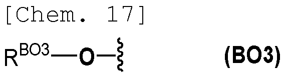

(In the formula, R^{BO3} represents a C1 to C4 alkyl group or C6 to C12 aryl group, with the alkyl group and the aryl group optionally being substituted with one or more substituents selected from the group consisting of halogen atoms, C1 to C4 alkyloxy groups, C1 to C4 fluoroalkyl groups, C2 to C13 acyl groups, cyano groups and nitro groups.)

Adjacent R^{B7}s optionally form a five-membered, six-membered or seven-membered ring together with the benzene ring to which the R^{B7}s are bound.

R^{B8} represents a hydrogen atom, C1 to C10 alkyl group or C6 to C22 aryl group optionally substituted with one or more fluorine atoms or C1 to C4 fluoroalkyl groups.

R^{B9} represents a hydrogen atom or diphenylamino group optionally substituted with one or more fluorine atoms or C1 to C4 fluoroalkyl groups.

Y represents a single bond, ethylene group, vinylene group optionally substituted with one or more methoxy groups or chlorine atoms, oxygen atom, sulfur atom, nitrogen atom optionally substituted with a C1 to C12 hydrocarbon group or methylene group optionally substituted with one or more C1 to C12 hydrocarbon groups.

W¹ represents an oxygen atom, sulfur atom, nitrogen atom optionally substituted with a C1 to C12 hydrocarbon group or methylene group optionally substituted with one or more C1 to C12 hydrocarbon groups.]

[6] The rare earth complex according to [5], wherein R^{B6} is a hydrogen atom.

[7] The rare earth complex according to [5] or [6], wherein R^{B7} is a hydrogen atom, C1 to C4 alkyl group, C1 to C4 alkyloxy group, C2 to C8 dialkylamino group or diphenylamino group optionally substituted with one or more fluorine atoms; C1 to C4 fluoroalkyl groups; or; cyano groups.

[8] The rare earth complex according to any of [5] to [7], wherein W¹ is an oxygen atom, sulfur atom, methylene group optionally substituted with one or more C1 to C4 alkyl groups or nitrogen atom optionally substituted with a C1 to C4 alkyl group or C6 to C12 aryl group.

[9] The rare earth complex according to any of [1] to [8], wherein HC is a quinolinonato ligand represented by general formula (1qu), m¹ is 1 or 2, and L¹ is phenanthroline optionally substituted with one or more methyl groups or phenyl groups, bipyridine optionally substituted with one or more methyl groups or phenyl groups, N,N-diethyl-4-{[4,6-bis(3,5-dimethyl-1H-pyrazol-1-yl)-1,3,5-triazin-2-yl]}aniline or dipyrido[3,2-a:2',3'-c]phenazine.

[10] The rare earth complex according to any of [1] to [9], wherein HC is a quinolinonato ligand represented by general formula (1qu), m² is 1, 2 or 3, and L² is water.

[11] The rare earth complex according to any of [1] to [10], wherein HC is a quinolinonato ligand represented by general formula (1qu), and, in general formulae (3a) and (3b), X^{A} is a C4 to C8 alkyl group or aryl group represented by general formula (3d) below, and X^{H} is a C1 to C4 alkylene group; diphenylether-2,2'-diyl group; naphthalen-1,8-diyl group; biphenyl-2,2'-diyl group; binaphthyl-2,2'-diyl group; bipyridin-2,2'-diyl group; or xanthendiyl group optionally substituted with one or more methyl groups.

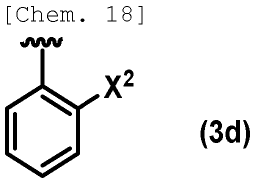

(In the formula, X² represents a hydrogen atom; fluorine atom; C1 to C4 alkyl group; C1 to C4 alkyloxy group; naphthyl group; pyridyl group; C1 to C4 fluoroalkyl group; or phenyl group optionally substituted with one or more fluorine atoms, C1 to C4 alkyl groups, C1 to C4 alkyloxy groups or C1 to C4 fluoroalkyl groups.)

[12] The rare earth complex according to [11], wherein X² in the general formula (3d) is a hydrogen atom, C1 to C4 alkyl group, C1 to C4 alkyloxy group or phenyl group optionally substituted with one or more C1 to C4 alkyl groups or C1 to C4 alkyloxy groups.

[13] The rare earth complex according to any of [1] to [12], wherein HC is a quinolinonato ligand represented by general formula (1qu), m¹ is 1 or 2, and m² is 0.

[14] The rare earth complex according to [1], wherein HC is a coumarinato ligand represented by general formula (1cu), and n is 3.

[15] The rare earth complex according to [1] or [14], wherein HC is a coumarinato ligand represented by general formula (1cu), and M³⁺ is a europium(III) ion, terbium(III) ion, samarium(III) ion or gadolinium(III) ion.

[16] The rare earth complex according to [1], [14] or [15], wherein HC is a coumarinato ligand represented by general formula (1cu), and R^{A} is a phenyl group optionally substituted with one or more substituents selected from the group consisting of fluorine atoms, diphenylamino groups or C1 to C6 fluoroalkyl groups or C1 to C6 fluoroalkyl groups, with the diphenylamino group optionally being substituted with one or more fluorine atoms, C1 to C6 fluoroalkyl groups or cyano groups.

[17] The rare earth complex according to any of [1] and [14] to [16], wherein the general formula (1cu) is a coumarinato ligand indicated by any of general formulae (1cu-1) to (1cu-6) below.

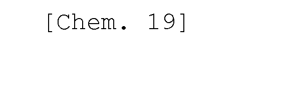

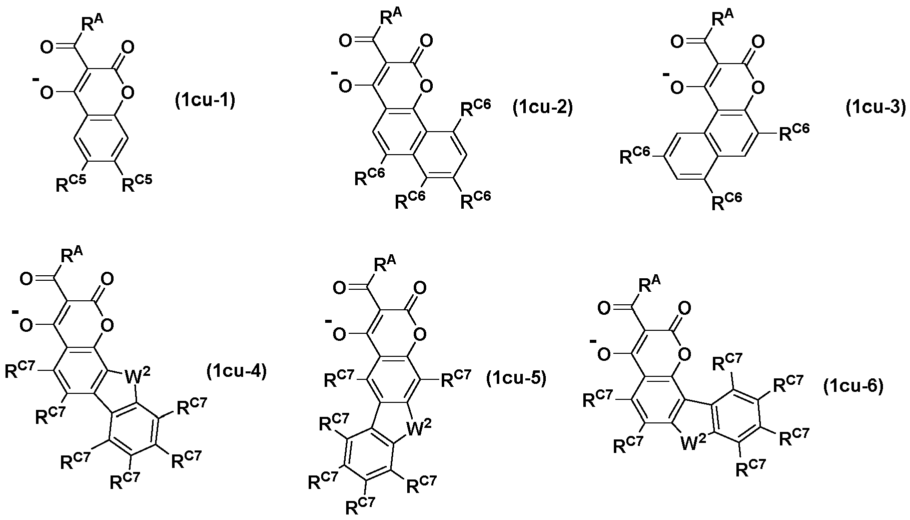

[In the formulae, R^{A} is synonymous with as R^{A} in general formula (1cu).

R^{C5}s each independently represent a hydrogen atom, halogen atom, cyano group, C1 to C8 alkyl group, C1 to C4 fluoroalkyl group, C6 to C22 aryl group, substituted or unsubstituted amino group indicated by general formula (BA3) below or substituted or unsubstituted oxy group indicated by general formula (BO3) below.

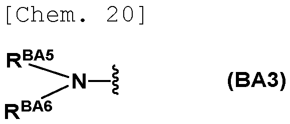

(In the formula, R^{BA5} and R^{BA6} each independently represent a C1 to C4 alkyl group or C6 to C12 aryl group, with the alkyl group and the aryl group optionally being substituted with one or more substituents selected from the group consisting of halogen atoms, C1 to C4 alkyloxy groups, C1 to C4 fluoroalkyl groups, C2 to C13 acyl groups, cyano groups and nitro groups. R^{BA5} and R^{BA6} optionally form a five-membered, six-membered or seven-membered ring including the nitrogen atom to which the R^{BA5} and R^{BA6} are bound.)

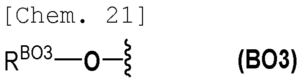

(In the formula, R^{BO3} represents a C1 to C4 alkyl group or C6 to C12 aryl group, with the alkyl group and the aryl group optionally being substituted with one or more substituents selected from the group consisting of halogen atoms, C1 to C4 alkyloxy groups, C1 to C4 fluoroalkyl groups, C2 to C13 acyl groups, cyano groups and nitro groups.)

The adjacent R^{C5}s optionally form a five-membered, six-membered or seven-membered ring together with the benzene ring to which the R^{C5}s are bound.

R^{C6}s each independently represent a hydrogen atom or C1 to C4 alkyloxy group.

R^{C7}s each independently represent a hydrogen atom, halogen atom or C1 to C4 fluoroalkyl group.

W² represents an oxygen atom, sulfur atom, nitrogen atom optionally substituted with a C1 to C12 hydrocarbon group or methylene group optionally substituted with one or more C1 to C12 hydrocarbon groups.]

[18] The rare earth complex according to [17], wherein R^{C7} is a hydrogen atom.

[19] The rare earth complex according to [17] or [18], wherein R^{C5} is a hydrogen atom, C1 to C4 alkyl group, C1 to C4 alkyloxy group, C2 to C8 dialkylamino group or diphenylamino group optionally substituted with one or more fluorine atoms; C1 to C4 fluoroalkyl groups; or; cyano groups.

[20] The rare earth complex according to any of [17] to [19], wherein W² is an oxygen atom, sulfur atom, methylene group optionally substituted with one or more C1 to C4 alkyl groups or nitrogen atom optionally substituted with a C1 to C10 alkyl group or C6 to C12 aryl group.

[21] The rare earth complex according to any of [1] and [14] to [20], wherein HC is a coumarinato ligand represented by general formula (1cu), m¹ is 1 or 2, and L¹ is phenanthroline optionally substituted with one or more methyl groups or phenyl groups, bipyridine optionally substituted with one or more methyl groups or phenyl groups, N,N-diethyl-4-{[4,6-bis(3,5-dimethyl-1H-pyrazol-1-yl)-1,3,5-triazin-2-yl]}aniline or dipyrido[3,2-a:2',3'-c]phenazine.

[22] The rare earth complex according to any of [1] and [14] to [21], wherein HC is a coumarinato ligand represented by general formula (1cu), m² is 1, 2 or 3, and L² is water.

[23] The rare earth complex according to any of [1] and [14] to [22], wherein HC is a coumarinato ligand represented by general formula (1cu), and, in general formulae (3a) and (3b), X^{A} is a C4 to C8 alkyl group or aryl group represented by general formula (3d) below, and X^{H} is a C1 to C4 alkylene group; diphenylether-2,2'-diyl group; naphthalen-1,8-diyl group; biphenyl-2,2'-diyl group; binaphthyl-2,2'-diyl group; bipyridin-2,2'-diyl group; or xanthendiyl group optionally substituted with one or more methyl groups.

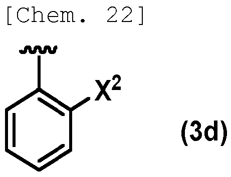

(In the formula, X² represents a hydrogen atom; fluorine atom; C1 to C4 alkyl group; C1 to C4 alkyloxy group; naphthyl group; pyridyl group; C1 to C4 fluoroalkyl group; or phenyl group optionally substituted with one or more fluorine atoms, C1 to C4 alkyl groups, C1 to C4 alkyloxy groups or C1 to C4 fluoroalkyl groups.)

[24] The rare earth complex according to [23], wherein X² in the general formula (3d) is a hydrogen atom, C1 to C4 alkyl group, C1 to C4 alkyloxy group or phenyl group optionally substituted with one or more C1 to C4 alkyl groups or C1 to C4 alkyloxy groups.

[25] The rare earth complex according to any of [1] and [14] to [24], wherein HC is a coumarinato ligand represented by general formula (1cu), m¹ is 1 or 2, and m² is 0.

[26] The rare earth complex according to any of [1] to [25], wherein all of the C6 to C22 aryl groups are C6 to C14 aryl groups.

[27] The rare earth complex according to any of [1] to [26], wherein all of the C3 to C20 heteroaryl groups are C4 to C12 heteroaryl groups.

(II) A method for manufacturing the rare earth complex according to any of [1] to [27] above, the method comprising allowing an enol represented by general formula (4b) or general formula (4c) below, a phosphorus ligand indicated by general formula (3a) below; a phosphorus ligand indicated by general formula (3b) below; or a nitrogen-containing compound having two or more nitrogen atoms possessing a lone pair, which is represented by L¹, or/and a neutral ligand, which is represented by L², selected from the group consisting of water; deuterium oxide; a sulfoxide compound; sulfone compound; amide compound; nitrile compound; ester compound; carbonyl compound; ether compound and alcohol and a rare earth compound to react together.

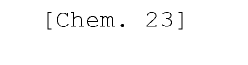

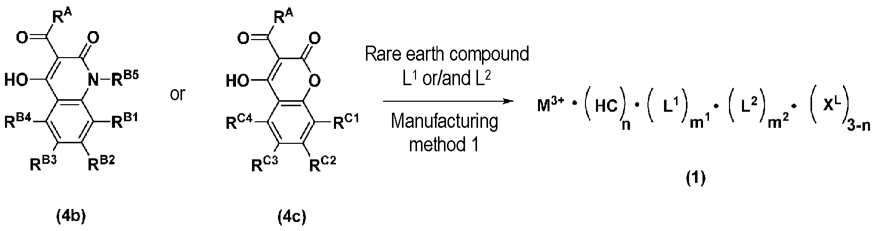

[In the formulae, R^{A}, R^{B1}, R^{B2}, R^{B3}, R^{B4}, R^{B5}, R^{C1}, R^{C2}, R^{C3}, R^{C4}, M³⁺, HC, L¹, L², X^{L}, n, m¹ and m² are synonymous with R^{A}, R^{B1}, R^{B2}, R^{B3}, R^{B4}, R^{B5}, R^{C1}, R^{C2}, R^{C3}, R^{C4}, M³⁺, HC, L¹, L², X^{L}, n, m¹ and m², respectively, in [1] above.]

(III) A method for manufacturing the rare earth complex according to any of [1] to [27] above, the method comprising allowing a diketonato complex represented by general formula (1ag) below and a phosphorus ligand represented by general formula (3a) below; a phosphorus ligand represented by general formula (3b) below; or a nitrogen-containing ligand having two or more nitrogen atoms possessing a lone pair; which is represented by L¹, or/and a neutral ligand, which is represented by L², selected from the group consisting of water; deuterium oxide; a sulfoxide compound; sulfone compound; amide compound; nitrile compound; ester compound; carbonyl compound; ether compound and alcohol to react together.

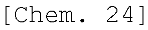

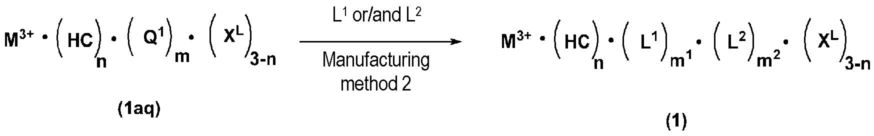

[In the formula, M³⁺, HC, X^{L}, n, m¹ and m² are synonymous with M³⁺, HC, X^{L}, n, m¹ and m², respectively, in [1] above. m represents 0, 1, 2 or 3, and Q¹ represents a neutral molecule. When m is not 0 and when m¹ is 0, it is impossible that Q¹ and L² are the same, and m and m² are equal at the same time.]

(IV)
[A] An optical material comprising the rare earth complex according to any of [1] to [27] above.
[B] An optical material comprising a resin material, inorganic glass, organic low-molecular-weight material or solvent and the rare earth complex according to any of [1] to [27] above.
[C] The optical material according to [B], wherein the resin material is polymethyl methacrylate, polyethyl methacrylate, polypropyl methacrylate, polybutyl methacrylate, polymethyl acrylate, polyethyl acrylate, polypropyl acrylate, polybutyl acrylate, polyethylene, polystyrene, polyvinyl acetate and a copolymer thereof; an epoxy resin; a polyimide resin; or a silicone resin.
[D] The optical material according to [B] or [C], wherein the solvent is halogenated hydrocarbons, alcohols, esters, glycol ethers, ethers, ketones or hydrocarbons.
[E] The optical material according to any of [A] to [D], wherein the optical material is a film for a solar cell, agricultural film, LED phosphor, light-emitting material, fluorescent material or wavelength conversion material.

### Advantageous Effects of Invention

The rare earth metal complex according to the present invention serves as an excellent wavelength conversion material excitable with blue light and having high light resistance.

### Brief Description of Drawings

[Fig. 1] UV-Vis and emission spectra of rare earth complex (1b-3), a rare earth complex according to the present invention obtained in Example 3.
[Fig. 2] UV-Vis and emission spectra of rare earth complex (1b-4), a rare earth complex according to the present invention obtained in Example 4.
[Fig. 3] UV-Vis and emission spectra of rare earth complex (1b-7), a rare earth complex according to the present invention obtained in Example 7.
[Fig. 4] UV-Vis and emission spectra of rare earth complex (1b-8), a rare earth complex according to the present invention obtained in Example 8.
[Fig. 5] UV-Vis and emission spectra of rare earth complex (1b-9), a rare earth complex according to the present invention obtained in Example 10.
[Fig. 6] UV-Vis and emission spectra of rare earth complex (1b-10), a rare earth complex according to the present invention obtained in Example 11.
[Fig. 7] UV-Vis and emission spectra of rare earth complex (1b-11), a rare earth complex according to the present invention obtained in Example 12.
[Fig. 8] UV-Vis and emission spectra of rare earth complex (1b-12), a rare earth complex according to the present invention obtained in Example 13.
[Fig. 9] UV-Vis and emission spectra of rare earth complex (1b-13), a rare earth complex according to the present invention obtained in Example 15.
[Fig. 10] UV-Vis and emission spectra of rare earth complex (1b-14), a rare earth complex according to the present invention obtained in Example 16.
[Fig. 11] UV-Vis and emission spectra of rare earth complex (1b-15), a rare earth complex according to the present invention obtained in Example 17.
[Fig. 12] UV-Vis and emission spectra of rare earth complex (1b-16), a rare earth complex according to the present invention obtained in Example 18.
[Fig. 13] UV-Vis and emission spectra of rare earth complex (1b-17), a rare earth complex according to the present invention obtained in Example 19.
[Fig. 14] UV-Vis and emission spectra of rare earth complex (1b-18), a rare earth complex according to the present invention obtained in Example 23.
[Fig. 15] UV-Vis and emission spectra of rare earth complex (1b-52), a rare earth complex according to the present invention obtained in Example 27.
[Fig. 16] UV-Vis and emission spectra of rare earth complex (1b-53), a rare earth complex according to the present invention obtained in Example 29.
[Fig. 17] UV-Vis and emission spectra of rare earth complex (1b-54), a rare earth complex according to the present invention obtained in Example 30.
[Fig. 18] UV-Vis and emission spectra of rare earth complex (1b-58), a rare earth complex according to the present invention obtained in Example 28.
[Fig. 19] UV-Vis and emission spectra of rare earth complex (1b-61), a rare earth complex according to the present invention obtained in Example 32.
[Fig. 20] UV-Vis and emission spectra of rare earth complex (1b-64), a rare earth complex according to the present invention obtained in Example 26.
[Fig. 21] UV-Vis and emission spectra of rare earth complex (1b-66), a rare earth complex according to the present invention obtained in Example 37.
[Fig. 22] UV-Vis and emission spectra of rare earth complex (1b-67), a rare earth complex according to the present invention obtained in Example 31.
[Fig. 23] UV-Vis and emission spectra of rare earth complex (1b-69), a rare earth complex according to the present invention obtained in Example 33.
[Fig. 24] UV-Vis and emission spectra of rare earth complex (1b-71), a rare earth complex according to the present invention obtained in Example 34.
[Fig. 25] UV-Vis and emission spectra of rare earth complex (1b-73), a rare earth complex according to the present invention obtained in Example 35.
[Fig. 26] UV-Vis and emission spectra of rare earth complex (1b-75), a rare earth complex according to the present invention obtained in Example 36.
[Fig. 27] UV-Vis and emission spectra of rare earth complex (1b-97), a rare earth complex according to the present invention obtained in Example 48.
[Fig. 28] UV-Vis and emission spectra of rare earth complex (1b-98), a rare earth complex according to the present invention obtained in Example 49.
[Fig. 29] UV-Vis and emission spectra of rare earth complex (1b-99), a rare earth complex according to the present invention obtained in Example 50.
[Fig. 30] UV-Vis and emission spectra of rare earth complex (1b-103), a rare earth complex according to the present invention obtained in Example 40.
[Fig. 31] UV-Vis and emission spectra of rare earth complex (1b-33), a rare earth complex according to the present invention obtained in Example 52.
[Fig. 32] UV-Vis and emission spectra of rare earth complex (1b-169), a rare earth complex according to the present invention obtained in Example 53.
[Fig. 33] UV-Vis and emission spectra of rare earth complex (1b-178), a rare earth complex according to the present invention obtained in Example 55.
[Fig. 34] UV-Vis and emission spectra of rare earth complex (1b-106), a rare earth complex according to the present invention obtained in Example 56.
[Fig. 35] UV-Vis and emission spectra of rare earth complex (1b-117), a rare earth complex according to the present invention obtained in Example 57.
[Fig. 36] UV-Vis and emission spectra of rare earth complex (1b-65), a rare earth complex according to the present invention obtained in Example 58.
[Fig. 37] UV-Vis and emission spectra of rare earth complex (1b-168), a rare earth complex according to the present invention obtained in Example 59.
[Fig. 38] UV-Vis and emission spectra of rare earth complex (1b-179), a rare earth complex according to the present invention obtained in Example 60.
[Fig. 39] UV-Vis and emission spectra of rare earth complex (1b-180), a rare earth complex according to the present invention obtained in Example 61.
[Fig. 40] UV-Vis and emission spectra of rare earth complex (1b-181), a rare earth complex according to the present invention obtained in Example 62.
[Fig. 41] UV-Vis and emission spectra of rare earth complex (1b-182), a rare earth complex according to the present invention obtained in Example 63.
[Fig. 42] UV-Vis and emission spectra of rare earth complex (1b-119), a rare earth complex according to the present invention obtained in Example 64.
[Fig. 43] UV-Vis and emission spectra of rare earth complex (1b-183), a rare earth complex according to the present invention obtained in Example 65.
[Fig. 44] UV-Vis and emission spectra of rare earth complex (1b-184), a rare earth complex according to the present invention obtained in Example 66.
[Fig. 45] UV-Vis and emission spectra of rare earth complex (1b-187), a rare earth complex according to the present invention obtained in Example 68.
[Fig. 46] UV-Vis and emission spectra of rare earth complex (1b-188), a rare earth complex according to the present invention obtained in Example 69.
[Fig. 47] UV-Vis and emission spectra of rare earth complex (1b-177), a rare earth complex according to the present invention obtained in Example 70.
[Fig. 48] UV-Vis and emission spectra of rare earth complex (1b-176), a rare earth complex according to the present invention obtained in Example 71.
[Fig. 49] UV-Vis and emission spectra of rare earth complex (1b-186), a rare earth complex according to the present invention obtained in Example 72.
[Fig. 50] UV-Vis and emission spectra of rare earth complex (1b-198), a rare earth complex according to the present invention obtained in Example 76.
[Fig. 51] UV-Vis and emission spectra of rare earth complex (1b-199), a rare earth complex according to the present invention obtained in Example 77.
[Fig. 52] UV-Vis and emission spectra of rare earth complex (1b-200), a rare earth complex according to the present invention obtained in Example 78.
[Fig. 53] UV-Vis and emission spectra of rare earth complex (1b-191), a rare earth complex according to the present invention obtained in Example 79.
[Fig. 54] UV-Vis and emission spectra of rare earth complex (1b-190), a rare earth complex according to the present invention obtained in Example 80.
[Fig. 55] UV-Vis and emission spectra of rare earth complex (1b-192), a rare earth complex according to the present invention obtained in Example 81.
[Fig. 56] UV-Vis and emission spectra of rare earth complex (1b-193), a rare earth complex according to the present invention obtained in Example 82.
[Fig. 57] UV-Vis and emission spectra of rare earth complex (1b-201), a rare earth complex according to the present invention obtained in Example 85.
[Fig. 58] UV-Vis and emission spectra of rare earth complex (1c-33), a rare earth complex according to the present invention obtained in Example 93.
[Fig. 59] UV-Vis and emission spectra of rare earth complex (1c-181), a rare earth complex according to the present invention obtained in Example 97.
[Fig. 60] UV-Vis and emission spectra of rare earth complex (1c-31), a rare earth complex according to the present invention obtained in Example 98.
[Fig. 61] UV-Vis and emission spectra of rare earth complex (1c-182), a rare earth complex according to the present invention obtained in Example 99.
[Fig. 62] UV-Vis and emission spectra of rare earth complex (1c-183), a rare earth complex according to the present invention obtained in Example 101.
[Fig. 63] UV-Vis and emission spectra of rare earth complex (1c-103), a rare earth complex according to the present invention obtained in Example 102.
[Fig. 64] UV-Vis and emission spectra of rare earth complex (1c-7), a rare earth complex according to the present invention obtained in Example 103.
[Fig. 65] UV-Vis and emission spectra of rare earth complex (1c-194), a rare earth complex according to the present invention obtained in Example 112.

### Description of Embodiments

The present invention will now be described in detail.

The definitions of substituents in a rare earth complex according to the present invention, which is represented by general formula (1), will be described individually.

As used herein, hydrogen atom includes a deuterium atom and a tritium atom.

HC is a quinolinonato ligand represented by general formula (1qu) or coumarinato ligand represented by general formula (1cu).

### <QU>

QU is a quinolinonato ligand represented by general formula (1qu) (Hereinafter also referred to as a quinolinonato ligand (1qu).).

In the rare earth complex (1) according to the present invention, the quinolinonato ligand (1qu) coordinates with M³⁺ through one or two oxygen atoms. The complex, furthermore, has the coordination structures of rare earth complexes (1bi) to (1bvi) below varying in the tautomeric structure of the quinolinonato ligand (1qu). The rare earth complex (1) according to the present invention includes all of the rare earth complexes (1bi) to (1bvi), but these isomers are herein expressed as a rare earth complex (1) for the sake of convenience.

The coordination structure of the rare earth complex (1) according to the present invention, furthermore, is preferably that of a rare earth complex (1bi) or (1bii) because this gives the complex optical characteristics suitable for use as an optical functional material. It should be noted that a rare earth complex (1bi) can tautomerize to form a rare earth complex (1bii). A rare earth complex (1bi), however, includes a rare earth complex (1bii). When a coordination structure is described herein, therefore, a rare earth complex (1bii), too, is expressed as a rare earth complex (1bi) for the sake of convenience.

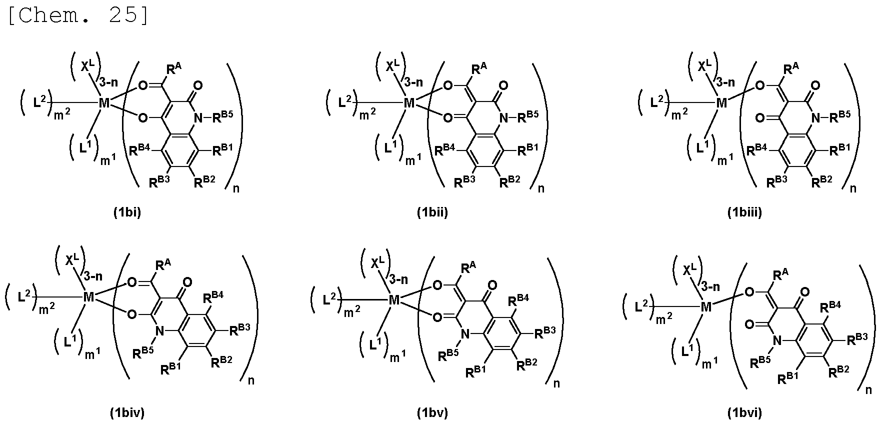

(In the formulae, R^{A}, R^{B1}, R^{B2}, R^{B3}, R^{B4}, R^{B5}, L¹, L², m¹, m², n and X^{L} are synonymous with R^{A}, R^{B1}, R^{B2}, R^{B3}, R^{B4}, R^{B5}, L¹, L², m¹, m², n and X^{L} in general formula (1) above. M represents a trivalent rare earth ion.)

Specific examples of the trivalent rare earth metal ion represented by M³⁺ include the rare earth metal ions of scandium, yttrium, lanthanum, cerium, praseodymium, neodymium, promethium, samarium, europium, gadolinium, terbium, dysprosium, holmium, erbium, thulium, ytterbium and lutetium.

The definitions of R^{A}, R^{B1}, R^{B2}, R^{B3}, R^{B4} and R^{B5} in the quinolinonato ligand (1qu) will now be described individually.

### (R^{A})

A C1 to C6 haloalkyl group represented by R^{A} may be any of a linear-chain, branched or cyclic haloalkyl group. Specific examples include groups such as a trifluoromethyl group, a difluoromethyl group, a perfluoroethyl group, a 2,2,2-trifluoroethyl group, a 1,1-difluoroethyl group, a 2,2-difluoroethyl group, a perfluoropropyl group, a 2,2,3,3,3-pentafluoropropyl group, a 2,2,3,3-tetrafluoropropyl group, a 3,3,3-trifluoropropyl group, a 1,1-difluoropropyl group, a perfluoropropan-2-yl group, a 2,2,2-trifluoro-1-(trifluoromethyl)ethyl group, a perfluorobutyl group, a 2,2,3,3,4,4,4-heptafluorobutyl group, a 3,3,4,4,4-pentafluorobutyl group, a 4,4,4-trifluorobutyl group, a 1,1,1,2,3,3,4,4,4-nonafluorobutan-2-yl group, a 1,1,1-trifluorobutan-2-yl group, a 4,4,4-trifluorobutan-2-yl group, a perfluoropentyl group, a 2,2,3,3,4,4,5,5,5-nonafluoropentyl group, a 3,3,4,4,5,5,5-heptafluoropentyl group, a 4,4,5,5,5-pentafluoropentyl group, a 5,5,5-trifluoropentyl group, a perfluoropentan-2-yl group, a 1,1,1,3,3,4,4,5,5,5-decafluoro-2-(trifluoromethyl)pentan-2-yl group, a 1,1,2,3,3,4,4,4-octafluoro-2-(trifluoromethyl)butyl group, a 1,1,2,2,3,4,4,4-octafluoro-3-(trifluoromethyl)butyl group, a 1,1,3,3,3-pentafluoro-2,2-bis(trifluoromethyl)propyl group, a 1,1,1,2,3,3,4,4,4-nonafluoro-1-(trifluoromethyl)butan-2-yl group, a perfluorocyclopentyl group, a perfluorohexyl group, a 1,1,1,2,3,3,4,4,5,5,6,6,6-decafluorohexan-2-yl group, a 1,1,2,3,3,4,4,5,5,5-decafluoro-2-(trifluoromethyl)pentyl group, a 1,1,2,2,3,4,4,5,5,5-decafluoro-3-(trifluoromethyl)pentyl group, a 1,1,2,2,3,3,4,5,5,5-decafluoro-4-(trifluoromethyl)pentyl group, a 1,1,1,2,2,3,3,4,4,5,5,5-dodecafluoro-1-(trifluoromethyl)pentan-2-yl group, a 1,1,1,3,3,4,4,5,5,5-decafluoro-2-(trifluoromethyl)pentan-2-yl group, a 1,1,1,2,2,4,4,5,5,5-decafluoro-3-(trifluoromethyl)pentan-2-yl group, a 1,1,3,3,4,4,4-heptafluoro-2,2-bis(trifluoromethyl)butyl group, a 1,2,2,3,4,4,4-heptafluoro-2,3-bis(trifluoromethyl)butyl group, a 1,1,2,2,4,4,4-heptafluoro-3,3-bis(trifluoromethyl)butyl group, a perfluorocyclohexyl group, a perfluorocyclopenthylmethyl group, a chloromethyl group, a bromomethyl group, an iodomethyl group, a 2-chloroethyl group and a 3-bromopropyl group. The present invention is not limited to these substituents listed by way of example. A trifluoromethyl group, 2,2,2-trifluoroethyl group, or perfluoropropyl group is preferred because of easy availability of raw materials.

Specific examples of C6 to C22 (preferably, C6 to C14) aryl groups represented by R^{A} include groups such as a phenyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a 2-ethylphenyl group, a 3-ethylphenyl group, a 4-ethylphenyl group, a 2,3-diethylphenyl group, a 2,4-diethylphenyl group, a 2,5-diethylphenyl group, a 2,6-diethylphenyl group, a 3,4-diethylphenyl group, a 3,5-diethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-cyclopropylphenyl group, a 3-cyclopropylphenyl group, a 4-cyclopropylphenyl group, a 2-butylphenyl group, a 3-butylphenyl group, a 4-butylphenyl group, a 2-(1-methylpropyl)phenyl group, a 3-(1-methylpropyl)phenyl group, a 4-(1-methylpropyl)phenyl group, a 2-(2-methylpropyl)phenyl group, a 3-(2-methylpropyl)phenyl group, a 4-(2-methylpropyl)phenyl group, a 2-cyclobutylphenyl group, a 3-cyclobutylphenyl group, a 4-cyclobutylphenyl group, a 1-naphthyl group, a 2-naphthyl group, a 2-biphenylyl group, a 3-biphenylyl group, a 4-biphenylyl group, a 2-fluorenyl group, a 3-fluorenyl group, a 9-anthryl group, a 2-phenanthrenyl group, a 3-phenanthrenyl group and a 9-phenanthrenyl group. The present invention is not limited to these substituents listed by way of example.

Specific examples of C3 to C20 (preferably, C4 to C12) heteroaryl groups represented by R^{A} include groups such as a triazinyl group, a furanyl group, a thienyl group, a pyrrolinyl group, a benzofuranyl group, a benzothienyl group, an indolyl group, a pyridyl group, a quinolinyl group, an isoquinolinyl group, a pyrazinyl group, a pyrimidyl group, a pyridazinyl group, a triazinyl group, a naphthyridinyl group, a cinnolinyl group, a phthalazinyl group, a quinoxalinyl group, a quinazolinyl group, a dibenzofuranyl group, a dibenzothienyl group, a carbazolyl group, a carbolinyl group, a benzimidazolyl group, an imidazopyridyl group, a benzoxazolyl group, a benzothiazolyl group and a phenanthrolinyl group. The present invention is not limited to these substituents listed by way of example.

A C6 to C22 aryl or C3 to C20 heteroaryl group represented by R^{A} may be substituted with one or more substituents selected from the group consisting of C1 to C6 fluoroalkyl groups; C1 to C6 alkyloxy groups; C1 to C6 fluoroalkyloxy groups; C5 to C14 aryloxy groups; C1 to C6 monoalkylamino groups; C6 to C12 monoarylamino groups; C4 to C12 monoheteroarylamino groups; C2 to C12 dialkylamino groups; C10 to C24 diarylamino groups optionally substituted with one or more cyano groups, halogen atoms or C1 to C6 fluoroalkyl groups; C8 to C24 diheteroarylamino groups optionally substituted with one or more fluorine atoms or C1 to C6 fluoroalkyl groups; C3 to C20 heteroaryl groups; C2 to C13 acyl groups, a methylenedioxy group; an ethylenedioxy group; halogen atoms; a hydroxyl group; a nitro group and a cyano group (group T₂1) .

A C1 to C6 fluoroalkyl group that belongs to group T₂1 may be any of linear-chain, branched or cyclic. Specific examples include groups such as a trifluoromethyl group, a difluoromethyl group, a perfluoroethyl group, a 2,2,2-trifluoroethyl group, a 1,1-difluoroethyl group, a 2,2-difluoroethyl group, a perfluoropropyl group, a 2,2,3,3,3-pentafluoropropyl group, a 2,2,3,3-tetrafluoropropyl group, a 3,3,3-trifluoropropyl group, a 1,1-difluoropropyl group, a perfluoropropan-2-yl group, a 2,2,2-trifluoro-1-(trifluoromethyl)ethyl group, a perfluorobutyl group, a 2,2,3,3,4,4,4-heptafluorobutyl group, a 3,3,4,4,4-pentafluorobutyl group, a 4,4,4-trifluorobutyl group, a 1,1,1,2,3,3,4,4,4-nonafluorobutan-2-yl group, a 1,1,1-trifluorobutan-2-yl group, a 4,4,4-trifluorobutan-2-yl group, a perfluoropentyl group, a 2,2,3,3,4,4,5,5,5-nonafluoropentyl group, a 3,3,4,4,5,5,5-heptafluoropentyl group, a 4,4,5,5,5-pentafluoropentyl group, a 5,5,5-trifluoropentyl group, a perfluoropentan-2-yl group, a 1,1,1,3,3,4,4,5,5,5-decafluoro-2-(trifluoromethyl)pentan-2-yl group, a 1,1,2,3,3,4,4,4-octafluoro-2-(trifluoromethyl)butyl group, a 1,1,2,2,3,4,4,4-octafluoro-3-(trifluoromethyl)butyl group, a 1,1,3,3,3-pentafluoro-2,2-bis(trifluoromethyl)propyl group, a 1,1,1,2,3,3,4,4,4-nonafluoro-1-(trifluoromethyl)butan-2-yl group, a perfluorocyclopentyl group, a perfluorohexyl group, a 1,1,1,2,3,3,4,4,5,5,6,6,6-decafluorohexan-2-yl group, a 1,1,2,3,3,4,4,5,5,5-decafluoro-2-(trifluoromethyl)pentyl group, a 1,1,2,2,3,4,4,5,5,5-decafluoro-3-(trifluoromethyl)pentyl group, a 1,1,2,2,3,3,4,5,5,5-decafluoro-4-(trifluoromethyl)pentyl group, a 1,1,1,2,2,3,3,4,4,5,5,5-dodecafluoro-1-(trifluoromethyl)pentan-2-yl group, a 1,1,1,3,3,4,4,5,5,5-decafluoro-2-(trifluoromethyl)pentan-2-yl group, a 1,1,1,2,2,4,4,5,5,5-decafluoro-3-(trifluoromethyl)pentan-2-yl group, a 1,1,3,3,4,4,4-heptafluoro-2,2-bis(trifluoromethyl)butyl group, a 1,2,2,3,4,4,4-heptafluoro-2,3-bis(trifluoromethyl)butyl group, a 1,1,2,2,4,4,4-heptafluoro-3,3-bis(trifluoromethyl)butyl group, a perfluorocyclohexyl group and a perfluorocyclopenthylmethyl group. The present invention is not limited to these substituents listed by way of example.

A C1 to C6 alkyloxy group that belongs to group T₂1 may be any of linear-chain, branched or cyclic. Specific examples include groups such as a methoxy group, an ethoxy group, a propyloxy group, an isopropyloxy group, a butyloxy group, a 2-methylpropyloxy group, a 2,2-dimethylpropyloxy group, a 3-cyclopropylpropyloxy group, a cyclopropyloxy group, a 2-methylbutyloxy group, a 3-methylbutyloxy group, a tert-butyloxy group, a cyclobutyloxy group, a pentyloxy group, a 2-methylpentyloxy group, a 1-methylbutyloxy group, a 1,2-dimethylbutyloxy group, a 1-ethylpropyloxy group, a cyclopentyloxy group, a hexyloxy group and a cyclohexyloxy group. The present invention is not limited to these substituents listed by way of example.

A C1 to C6 fluoroalkyloxy group that belongs to group T₂1 may be any of linear-chain, branched or cyclic. Specific examples include groups such as a trifluoromethyloxy group, a difluoromethyloxy group, a perfluoroethyloxy group, a 2,2,2-trifluoroethyloxy group, a 1,1-difluoroethyloxy group, a 2,2-difluoroethyloxy group, a perfluoropropyloxy group, a 2,2,3,3,3-pentafluoropropyloxy group, a 2,2,3,3-tetrafluoropropyloxy group, a 3,3,3-trifluoropropyloxy group, a 1,1-difluoropropyloxy group, a perfluoropropan-2-yloxy group, a 1,1,1,3,3,3-hexafluoropropan-2-yloxy group, a 1,1,1-trifluorobutan-2-yloxy group, a perfluorocyclopropyloxy group, a perfluorobutyloxy group, a 2,2,3,3,4,4,4-heptafluorobutyloxy group, a 3,3,4,4,4-pentafluorobutyloxy group, a 4,4,4-trifluorobutyloxy group, a 1,1,1,2,3,3,4,4,4-nonafluorobutyl-2-yloxy group, a 1,1,1-trifluorobutan-2-yloxy group, a 4,4,4-trifluorobutan-2-yloxy group, a perfluoropentyloxy group, a 2,2,3,3,4,4,5,5,5-nonafluoropentyloxy group, a 3,3,4,4,5,5,5-heptafluoropentyloxy group, a 4,4,5,5,5-pentafluoropentyloxy group, a 5,5,5-trifluoropentyloxy group, a perfluoropentan-2-yloxy group, a 1,1,1,3,3,4,4,5,5,5-decafluoro-2-(trifluoromethyl)pentan-2-yloxy group, a 1,1,2,2,3,4,4,4-octafluoro-3-(trifluoromethyl)butyloxy group, a 1,1,3,3,3-pentafluoro-2,2-bis(trifluoromethyl)propyloxy group, a 1,1,1,2,3,3,4,4,4-nonafluoro-1-(trifluoromethyl)butan-2-yloxy group, a perfluorocyclopentyloxy group, a perfluorohexyloxy group, a 1,1,1,2,3,3,4,4,5,5,6,6,6-decafluorohexan-2-yloxy group, a 1,1,2,3,3,4,4,5,5,5-decafluoro-2-(trifluoromethyl)pentyloxy group, a 1,1,2,2,3,4,4,5,5,5-decafluoro-3-(trifluoromethyl)pentyloxy group, a 1,1,2,2,3,3,4,5,5,5-decafluoro-4-(trifluoromethyl)pentyloxy group, a 1,1,1,2,2,3,3,4,4,5,5,5-dodecafluoro-1-(trifluoromethyl)pentan-2-yloxy group, a 1,1,1,3,3,4,4,5,5,5-decafluoro-2-(trifluoromethyl)pentan-2-yloxy group, a 1,1,1,2,2,4,4,5,5,5-decafluoro-3-(trifluoromethyl)pentan-2-yloxy group, a 1,1,3,3,4,4,4-heptafluoro-2,2-bis(trifluoromethyl)butyloxy group, a 1,2,2,3,4,4,4-heptafluoro-2,3-bis(trifluoromethyl)butyloxy group, a 1,1,2,2,4,4,4-heptafluoro-3,3-bis(trifluoromethyl)butyloxy group, a perfluorocyclohexyloxy group and a perfluorocyclopentylmethyloxy group. The present invention is not limited to these substituents listed by way of example.

Specific examples of C5 to C14 aryloxy groups that belong to group T₂1 include groups such as an indenyloxy group, a phenyloxy group, a 2-methylphenyloxy group, a 3-methylphenyloxy group, a 4-methylphenyloxy group, a 2,3-dimethylphenyloxy group, a 2,4-dimethylphenyloxy group, a 2,5-dimethylphenyloxy group, a 2,6-dimethylphenyloxy group, a 3,4-dimethylphenyloxy group, a 3,5-dimethylphenyloxy group, a 2,3,4-trimethylphenyloxy group, a 2,3,5-trimethylphenyloxy group, a 2,3,6-trimethylphenyloxy group, a 2,4,5-trimethylphenyloxy group, a 2,4,6-trimethylphenyloxy group, a 3,4,5-trimethylphenyloxy group, a 2,3,4,5-tetramethylphenyloxy group, a 2,3,4,6-tetramethylphenyloxy group, a 2,3,5,6-tetramethylphenyloxy group, a 2-ethylphenyloxy group, a 3-ethylphenyloxy group, a 4-ethylphenyloxy group, a 2,3-diethylphenyloxy group, a 2,4-diethylphenyloxy group, a 2,5-diethylphenyloxy group, a 2,6-diethylphenyloxy group, a 3,4-diethylphenyloxy group, a 3,5-diethylphenyloxy group, a 2-propylphenyloxy group, a 3-propylphenyloxy group, a 4-propylphenyloxy group, a 2-isopropylphenyloxy group, a 3-isopropylphenyloxy group, a 4-isopropylphenyloxy group, a 2-cyclopropylphenyloxy group, a 3-cyclopropylphenyloxy group, a 4-cyclopropylphenyloxy group, a 2-butylphenyloxy group, a 3-butylphenyloxy group, a 4-butylphenyloxy group, a 2-(1-methylpropyl)phenyloxy group, a 3-(1-methylpropyl)phenyloxy group, a 4-(1-methylpropyl)phenyloxy group, a 2-(2-methylpropyl)phenyloxy group, a 3-(2-methylpropyl)phenyloxy group, a 4-(2-methylpropyl)phenyloxy group, a 2-cyclobutylphenyloxy group, a 3-cyclobutylphenyloxy group, a 4-cyclobutylphenyloxy group, a (biphenyl-2-yl)oxy group, a (biphenyl-3-yl)oxy group, a (biphenyl-4-yl)oxy group, a (phenanthren-9-yl)oxy group and an (anthracen-9-yl)oxy group. The present invention is not limited to these substituents listed by way of example.

Specific examples of C1 to C6 monoalkylamino groups that belong to group T₂1 include groups such as a methylamino group, an ethylamino group, a propylamino group, an isopropylamino group, a butylamino group, a 2-methylpropylamino group, a 2,2-dimethylpropylamino group, a 3-cyclopropylamino group, a cyclopropylamino group, a tert-butylamino group, a cyclobutylamino group, a pentylamino group, a 2-methylpentylamino group and a hexylamino group. The present invention is not limited to these substituents listed by way of example.

Specific examples of C6 to C12 monoarylamino groups that belong to group T₂1 include groups such as an indenylamino group, a phenylamino group, a 2-methylphenylamino group, a 3-methylphenylamino group, a 4-methylphenylamino group, a 2,3-dimethylphenylamino group, a 2,4-dimethylphenylamino group, a 2,5-dimethylphenylamino group, a 2,6-dimethylphenylamino group, a 3,4-dimethylphenylamino group, a 3,5-dimethylphenylamino group, a 2,3,4-trimethylphenylamino group, a 2,3,5-trimethylphenylamino group, a 2,3,6-trimethylphenylamino group, a 2,4,5-trimethylphenylamino group, a 2,4,6-trimethylphenylamino group, a 3,4,5-trimethylphenylamino group, a 2,3,4,5-tetramethylphenylamino group, a 2,3,4,6-tetramethylphenylamino group, a 2,3,5,6-tetramethylphenylamino group, a 2-ethylphenylamino group, a 3-ethylphenylamino group, a 4-ethylphenylamino group, a 2,3-diethylphenylamino group, a 2,4-diethylphenylamino group, a 2,5-diethylphenylamino group, a 2,6-diethylphenylamino group, a 3,4-diethylphenylamino group, a 3,5-diethylphenylamino group, a 2-propylphenylamino group, a 3-propylphenylamino group, a 4-propylphenylamino group, a 2-isopropylphenylamino group, a 3-isopropylphenylamino group, a 4-isopropylphenylamino group, a 2-cyclopropylphenylamino group, a 3-cyclopropylphenylamino group, a 4-cyclopropylphenylamino group, a 2-butylphenylamino group, a 3-butylphenylamino group, a 4-butylphenylamino group, a 2-(1-methylpropyl)phenylamino group, a 3-(1-methylpropyl)phenylamino group, a 4-(1-methylpropyl)phenylamino group, a 2-(2-methylpropyl)phenylamino group, a 3-(2-methylpropyl)phenylamino group, a 4-(2-methylpropyl)phenylamino group, a 2-cyclobutylphenylamino group, a 3-cyclobutylphenylamino group, a 4-cyclobutylphenylamino group, a 1-naphthylamino group and a 2-naphthylamino group. The present invention is not limited to these substituents listed by way of example.

Specific examples of C4 to C12 monoheteroarylamino groups that belong to group T₂1 include groups such as a 2-pyrrolylamino group, a 2-thienylamino group, a 3-thienylamino group, a 2-furylamino group, a 3-furylamino group, a 2-(1-methylindolyl)amino group, a 2-benzothienyl group, a 3-benzothienyl group, a 2-benzofuranyl group and a 3-benzofuranyl group. The present invention is not limited to these substituents listed by way of example.

Specific examples of C2 to C12 dialkylamino groups that belong to group T₂1 include groups such as a dimethylamino group, a diethylamino group, a dipropylamino group, a diisopropylamino group, a dibutylamino group, a bis(2-methylpropyl)amino group, a bis(2,2-dimethylpropyl)amino group, a bis(3-cyclopropyl)amino group, a dicyclopropylamino group, a di(tert-butyl)amino group, a dicyclobutylamino group, a dipentylamino group, a bis(2-methylpentyl)amino group, a di(1-methylbutyl)amino group, a bis(1,2-dimethylbutyl)amino group, a di(1-ethylpropyl)amino group, a dicyclopentylamino group, a dihexylamino group, a dicyclohexylamino group, an N-ethyl-N-methylamino group, an N-methyl-N-propyl group, an N-cyclopropyl-N-methylamino group, an N-butyl-N-methylamino group, an N-cyclobutyl-N-methylamino group, an N-methyl-N-pentyl group, an N-cyclopentyl-N-methylamino group, an N-hexyl-N-methylamino group, an N-cyclohexyl-N-methylamino group, an N-cycloheptyl-N-methylamino group, an N-methyl-N-octylamino group, an N-methyl-N-nonylamino group, an N-decyl-N-methylamino group or an N-methyl-N-undecylamino group and an azetidyl group, a pyrrolidinyl group, a piperidino group, an azepanyl group, a morpholino group and a 2-morpholinyl group. The present invention is not limited to these substituents listed by way of example.

Specific examples of C10 to C24 diarylamino groups that belong to group T₂1 include groups such as a diindenylamino group, an N-indenyl-N-phenylamino group, a diphenylamino group, a bis(2-methylphenyl)amino group, a bis(3-methylphenyl)amino group, a bis(4-methylphenyl)amino group, a bis(2,3-dimethylphenyl)amino group, a bis(2,4-dimethylphenyl)amino group, a bis(2,5-dimethylphenyl)amino group, a bis(2,6-dimethylphenyl)amino group, a bis(3,4-dimethylphenyl)amino group, a bis(3,5-dimethylphenyl)amino group, a bis(2,3,4-trimethylphenyl)amino group, a bis(2,3,5-trimethylphenyl)amino group, a bis(2,3,6-trimethylphenyl)amino group, a bis(2,4,5-trimethylphenyl)amino group, a bis(2,4,6-trimethylphenyl)amino group, a bis(3,4,5-trimethylphenyl)amino group, a bis(2,3,4,5-tetramethylphenyl)amino group, a bis(2,3,4,6-tetramethylphenyl)amino group, a bis(2,3,5,6-tetramethylphenyl)amino group, a bis(2-ethylphenyl)amino group, a bis(3-ethylphenyl)amino group, a bis(4-ethylphenyl)amino group, a bis(2,3-diethylphenyl)amino group, a bis(2,4-diethylphenyl)amino group, a bis(2,5-diethylphenyl)amino group, a bis(2,6-diethylphenyl)amino group, a bis(3,4-diethylphenyl)amino group, a bis(3,5-diethylphenyl)amino group, a bis(2-propylphenyl)amino group, a bis(3-propylphenyl)amino group, a bis(4-propylphenyl)amino group, a bis(2-isopropylphenyl)amino group, a bis(3-isopropylphenyl)amino group, a bis(4-isopropylphenyl)amino group, a bis(2-cyclopropylphenyl)amino group, a bis(3-cyclopropylphenyl)amino group, a bis(4-cyclopropylphenyl)amino group, a bis(2-butylphenyl)amino group, a bis(3-butylphenyl)amino group, a bis(4-butylphenyl)amino group, a bis[2-(1-methylpropyl)phenyl]amino group, a bis[3-(1-methylpropyl)phenyl]amino group, a bis[4-(1-methylpropyl)phenyl]amino group, a bis[2-(2-methylpropyl)phenyl]amino group, a bis[3-(2-methylpropyl)phenyl]amino group, a bis[4-(2-methylpropyl)phenyl]amino group, a bis(2-cyclobutylphenyl)amino group, a bis(3-cyclobutylphenyl)amino group, a bis(4-cyclobutylphenyl)amino group, a di(1-naphthyl)amino group, a di(2-naphthyl)amino group, an N-(2-methylphenyl)-N-phenylamino group, an N-(3-methylphenyl)-N-phenylamino group, an N-(4-methylphenyl)-N-phenylamino group, an N-(2,3-dimethylphenyl)-N-phenylamino group, an N-(2,4-dimethylphenyl)-N-phenylamino group, an N-(2,5-dimethylphenyl)-N-phenylamino group, an N-(2,6-dimethylphenyl)-N-phenylamino group, an N-(3,4-dimethylphenyl)-N-phenylamino group, an N-(3,5-dimethylphenyl)-N-phenylamino group, an N-(2,3,4-trimethylphenyl)-N-phenylamino group, an N-(2,3,5-trimethylphenyl)-N-phenylamino group, an N-(2,3,6-trimethylphenyl)-N-phenylamino group, an N-(2,4,5-trimethylphenyl)-N-phenylamino group, an N-(2,4,6-trimethylphenyl)-N-phenylamino group, an N-(3,4,5-trimethylphenyl)-N-phenylamino group, an N-(1-naphthyl)-N-phenylamino group, an N-(2-naphthyl)-N-phenylamino group, a carbazoyl group, an iminostilbenyl group, a 9(10H)-acridonyl group, a 10,11-dihydro-5H-dibenz[b,f]azepinyl group and a 10,11-dihydro-10-oxo-5H-dibenz[b,f]azepinyl group. The present invention is not limited to these substituents listed by way of example.

Specific examples of C8 to C24 diheteroarylamino groups that belong to group T₂1 include groups such as a bis(2-pyrrolyl)amino group, a bis(1-methylpyrrol-2-yl)amino group, a bis(1-ethylpyrrol-2-yl)amino group, a bis(1-phenylpyrrol-2-yl)amino group, a bis(2-thienyl)amino group, a bis(3-thienyl)amino group, a bis(2-furyl)amino group, a bis(3-furyl)amino group, a bis(2-benzothienyl)amino group, a bis(3-benzothienyl)amino group, a bis(2-benzofuranyl)amino group, a bis(3-benzofuranyl)amino group, a bis(1-methylindol-2-yl)amino group, a bis(1-methylindol-2-yl)amino group, a bis(3-benzofuranyl)amino group, a phenothiazinyl group, a phenoxazinyl group, a 5,10-dihydrodihydrophenazinyl group and a 10-methyl-5,10-dihydrodihydrophenazinyl group. The present invention is not limited to these substituents listed by way of example.

A C10 to C24 diarylamino group that belongs to group T₂1, furthermore, may be substituted with one or more fluorine atoms or C1 to C6 fluoroalkyl groups. Specific examples include groups such as a bis(2-fluorophenyl)amino group, a bis(4-fluorophenyl)amino group, a bis(3,5-difluorophenyl)amino group, a bis(3,4-difluorophenyl)amino group, a bis(3,4,5-trifluorophenyl)amino group, a bis[4-(1,1,1,2,2,3,3-heptafluoropropyl)phenyl]amino group, a bis(perfluorophenyl)amino group, a bis(4-trifluoromethylphenyl)amino group, a bis[3,5-di(trifluoromethyl)phenyl]amino group, a 3-fluorocarbazoyl group, a 3,6-difluorocarbazoyl group, a 3-(trifluoromethyl)carbazoyl group, a 3,6-bis(trifluoromethyl)carbazoyl group and a 2-(trifluoromethyl)phenothiazinyl group. The present invention is not limited to these substituents listed by way of example.

A C8 to C24 diheteroarylamino group that belongs to group T₂1, furthermore, may be substituted with one or more fluorine atoms or C1 to C6 fluoroalkyl groups. Specific examples include groups such as a bis(5-fluoropyrrol-2-yl)amino group, a bis(5-fluoro-1-methylpyrrol-2-yl)amino group, a bis(5-fluoro-1-ethylpyrrol-2-yl)amino group, a bis(5-fluoro-1-phenylpyrrol-2-yl)amino group, a bis(5-fluoro-2-thienyl)amino group, a bis(5-trifluoromethyl-2-thienyl)amino group, a bis(5-fluoro-3-thienyl)amino group, a bis(5-fluoro-2-furyl)amino group, a bis(5-trifluoromethyl-2-furyl)amino group, a bis(5-fluoro-3-furyl)amino group, a bis(5-fluoro-2-benzothienyl)amino group, a bis(5-fluoro-3-benzothienyl)amino group, a bis(5-fluoro-2-benzofuranyl)amino group, a bis(5-fluoro-3-benzofuranyl)amino group, a bis(1-methylindol-2-yl)amino group, a bis(1-methylindol-3-yl)amino group, a bis(5-fluoro-3-benzofuranyl)amino group, a 2-fluorophenothiazinyl group, a 2-trifluoromethylphenothiazinyl group and a 2-trifluoromethylphenoxazinyl group. The present invention is not limited to these substituents listed by way of example.

Specific examples of C3 to C20 heteroaryl groups that belong to group T₂1 include groups such as a triazinyl group, a furanyl group, a thienyl group, a pyrrolinyl group, a benzofuranyl group, a benzothienyl group, an indolyl group, a pyridyl group, a quinolinyl group, an isoquinolinyl group, a pyrazinyl group, a pyrimidyl group, a pyridazinyl group, a triazinyl group, a naphthyridinyl group, a cinnolinyl group, a phthalazinyl group, a quinoxalinyl group, a quinazolinyl group, a dibenzofuranyl group, a dibenzothienyl group, a carbazolyl group, a di-tert-butylcarbazolyl group, a carbolinyl group, a benzimidazolyl group, an imidazopyridyl group, a benzoxazolyl group, a benzothiazolyl group and a phenanthrolinyl group. The present invention is not limited to these substituents listed by way of example.

A C2 to C13 acyl group that belongs to group T₂1 may be any of an aliphatic or aromatic acyl group. Specific examples include aliphatic acyl groups such as an acetyl group, an ethylcarbonyl group, a 1-propylcarbonyl group, an isopropylcarbonyl group, a 1-butylcarbonyl group, a 2-butylcarbonyl group, a tert-butylcarbonyl group, a 1-pentylcarbonyl group, a 2-pentylcarbonyl group, a 3-pentylcarbonyl group and a 1-hexylcarbonyl group and
aromatic acyl groups such as a phenylcarbonyl group (benzoyl group), a 1-naphthylcarbonyl group, a 2-naphthylcarbonyl group, a (biphenyl-2-yl)carbonyl group, a (biphenyl-4-yl)carbonyl group, a (4-methylphenyl)carbonyl group and a (2-methylphenyl)carbonyl group. The present invention is not limited to these substituents listed by way of example.

Examples of halogen atoms that belong to group T₂1 include a fluorine atom, a chlorine atom, a bromine atom and an iodine atom.

Specific examples of C6 to C22 aryl groups at R^{A} optionally substituted with one or more substituents selected from group T₂1, therefore, include groups such as a phenyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a 2-ethylphenyl group, a 3-ethylphenyl group, a 4-ethylphenyl group, a 2,3-diethylphenyl group, a 2,4-diethylphenyl group, a 2,5-diethylphenyl group, a 2,6-diethylphenyl group, a 3,4-diethylphenyl group, a 3,5-diethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-cyclopropylphenyl group, a 3-cyclopropylphenyl group, a 4-cyclopropylphenyl group, a 2-butylphenyl group, a 3-butylphenyl group, a 4-butylphenyl group, a 2-(1-methylpropyl)phenyl group, a 3-(1-methylpropyl)phenyl group, a 4-(1-methylpropyl)phenyl group, a 2-(2-methylpropyl)phenyl group, a 3-(2-methylpropyl)phenyl group, a 4-(2-methylpropyl)phenyl group, a 2-cyclobutylphenyl group, a 3-cyclobutylphenyl group, a 4-cyclobutylphenyl group, a 1-naphthyl group, a 2-naphthyl group, a 2-biphenylyl group, a 3-biphenylyl group, a 4-biphenylyl group, a 2-fluorenyl group, a 3-fluorenyl group, a 9-anthryl group, a 2-phenanthrenyl group, a 3-phenanthrenyl group, a 9-phenanthrenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-(trifluoromethyl)phenyl group, a 3-(trifluoromethyl)phenyl group, a 4-(trifluoromethyl)phenyl group, a 2-(perfluoropropyl)phenyl group, a 3-(perfluoropropyl)phenyl group, a 4-(perfluoropropyl)phenyl group, a 2-acetylphenyl group, a 3-acetylphenyl group, a 4-acetylphenyl group, a 2-pivaloylphenyl group, a 3-pivaloylphenyl group, a 4-pivaloylphenyl group, a 2-benzoylphenyl group, a 3-benzoylphenyl group, a 4-benzoylphenyl group, a 2-naphthoylphenyl group, a 3-naphthoylphenyl group, a 4-naphthoylphenyl group, a 4-methoxy-1-naphthyl group, a 6-methoxy-2-naphthyl group, a 4'-methoxybiphenyl-2-yl group, a 4'-methoxybiphenyl-3-yl group, a 4'-methoxybiphenyl-4-yl group, a 10-methoxy-9-anthryl group, a 2-fluorophenyl group, a 3-fluorophenyl group, a 4-fluorophenyl group, a 3,4-difluorophenyl group, a 4-(trifluoromethyloxy)phenyl group, a 4-phenyloxyphenyl group, a 4-(phenylamino)phenyl group, a 4-(diphenylamino)phenyl group, a 4-cyanophenyl group, a 4-nitrophenyl group, a 4-hydroxyphenyl group, a perfluorophenyl group, a perfluorobiphenyl-4-yl group and a deuterated phenyl group. The present invention is not limited to these substituents listed by way of example. A phenyl group, 2-methylphenyl group, 3-methylphenyl group, 4-methylphenyl group, 1-naphthyl group, 2-naphthyl group, 2-biphenylyl group, 3-biphenylyl group, 4-biphenylyl group, 2-methoxyphenyl group, 3-methoxyphenyl group, 4-methoxyphenyl group, 2-(trifluoromethyl)phenyl group, 3-(trifluoromethyl)phenyl group, 4-(trifluoromethyl)phenyl group, 2-fluorophenyl group, 3-fluorophenyl group, 4-fluorophenyl group, 3,4-difluorophenyl group, 2-(diphenylamino)phenyl group, 3-(diphenylamino)phenyl group, 4-(diphenylamino)phenyl group, deuterated phenyl group, perfluorophenyl group, 4-cyanophenyl group, 4-nitrophenyl group, 4-{bis[4-(trifluoromethyl)phenyl]amino}phenyl group, 4-{bis[4-bis(4-cyanophenyl)amino]phenyl group, 4-{bis[4-bis(3,5-difluorophenyl)amino]phenyl group or 4-[bis(4-fluorophenyl)amino]phenyl group is preferred because it gives the complex optical characteristics suitable for use as an optical functional material. A phenyl group, 4-(trifluoromethyl)phenyl group or 4-(diphenylamino)phenyl group is more preferred because of the ease of synthesis.

Specific examples of C3 to C20 heteroaryl groups at R^{A} optionally substituted with one or more substituents selected from group T₂1, furthermore, include groups such as a 1,3,5-triazin-2-yl group, a 2-furanyl group, a 3-furanyl group, a 2-thienyl group, a 3-thienyl group, a 1-pyrrolyl group, a 2-pyrrolyl group, a 3-pyrrolyl group, a 1-methylpyrrol-2-yl group, a 1-methylpyrrol-3-yl group, a 1-phenylpyrrol-2-yl group, a 1-phenylpyrrol-3-yl group, a 2-benzofuranyl group, a 3-benzofuranyl group, a 2-benzothienyl group, a 3-benzothienyl group, a 1-indolyl group, a 2-indolyl group, a 3-indolyl group, a 1-methylindol-2-yl group, a 1-methylindol-3-yl group, a 1-phenylindol-2-yl group, a 1-phenylindol-3-yl group, a 9-methylcarbazol-2-yl group, a 9-methylcarbazol-3-yl group, a 9-ethylcarbazol-2-yl group, a 9-ethylcarbazol-3-yl group, a 9-phenylcarbazol-2-yl group, a 9-phenylcarbazol-3-yl group, a dibenzofuran-2-yl group, a dibenzofuran-3-yl group, a dibenzofuran-4-yl group, a dibenzothiophen-2-yl group, a dibenzothiophen-3-yl group, a dibenzothiophen-4-yl group, a 5-(trifluoromethyl)furan-2-yl group, a 2-(trifluoromethyl)furan-3-yl group, a 5-(trifluoromethyl)thiophen-2-yl group, a 2-(trifluoromethyl)-3-thienyl group, a 5-(trifluoromethyl)benzofuran-2-yl group, a 6-(trifluoromethyl)benzofuran-2-yl group, a 5-(trifluoromethyl)benzothiophen-2-yl group, a 6-(trifluoromethyl)benzothiophen-2-yl group, a 3,6-di-tert-butyl-9H-carbazol-9-yl group, a 9-methyl-5-(trifluoromethyl)carbazol-2-yl group, a 9-methyl-6-(trifluoromethyl)carbazol-2-yl group, a 9-methyl-5-(trifluoromethyl)carbazol-3-yl group, a 9-methyl-6-(trifluoromethyl)carbazol-3-yl group, a 9-phenyl-5-(trifluoromethyl)carbazol-2-yl group, a 9-phenyl-5-(trifluoromethyl)carbazol-3-yl group, a 9-phenyl-6-(trifluoromethyl)carbazol-2-yl group, a 9-phenyl-6-(trifluoromethyl)carbazol-3-yl group, a 6-(trifluoromethyl)dibenzofuran-2-yl group, a 7-(trifluoromethyl)dibenzofuran-2-yl group, a 6-(trifluoromethyl)dibenzofuran-3-yl group, a 7-(trifluoromethyl)dibenzofuran-3-yl group, a 6-(trifluoromethyl)dibenzofuran-4-yl group, a 7-(trifluoromethyl)dibenzofuran-4-yl group, a 6-(trifluoromethyl)dibenzothiophen-2-yl group, a 7-(trifluoromethyl)dibenzothiophen-2-yl group, a 6-(trifluoromethyl)dibenzothiophen-3-yl group, a 7-(trifluoromethyl)dibenzothiophen-3-yl group, a 6-(trifluoromethyl)dibenzothiophen-4-yl group and a 7-(trifluoromethyl)dibenzothiophen-4-yl group. The present invention is not limited to these substituents listed by way of example. A 2-furanyl group, 2-thienyl group, 2-benzofuranyl group, 2-benzothienyl group, 9-methylcarbazol-3-yl group, 9-ethylcarbazol-3-yl group, 9-phenylcarbazol-3-yl group, dibenzofuran-2-yl group, dibenzofuran-3-yl group, dibenzofuran-4-yl group, dibenzothiophen-2-yl group, dibenzothiophen-3-yl group, dibenzothiophen-4-yl group, 5-(trifluoromethyl)furan-2-yl group or 5-(trifluoromethyl)thiophen-2-yl group is preferred because of the ease of synthesis.

### (R^{B1}, R^{B2}, R^{B3}, R^{B4} and R^{B5})

Examples of halogen atoms represented by R^{B1}, R^{B2}, R^{B3}, R^{B4} and R^{B5} include the substituents listed as examples of halogen atoms that belong to group T₂1.

A C1 to C10 alkyl group represented by R^{B1}, R^{B2}, R^{B3}, R^{B4} and R^{B5} may be any of a linear-chain, branched or cyclic alkyl group. Specific examples include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a 5-dimethylcyclopentyl group, a 3-ethylcyclopentyl group, a hexyl group, a cyclohexyl group, a 4-ethylcyclohexyl group, a 4-propylcyclohexyl group, a 4,4-dimethylcyclohexyl group, a 2,6-dimethylcyclohexyl group, a 3,5-dimethylcyclohexyl group, a cycloheptyl group, a cyclooctyl group, a cyclononyl group, a cyclodecanyl group, a bicyclo[2.2.1]heptan-2-yl group, a bicyclo[2.2.2]octan-2-yl group, an adamantan-2-yl group, a bicyclo[2.2.1]heptan-2-yl group and an adamantan-1-yl group. The present invention is not limited to these substituents listed by way of example.

A C2 to C4 alkenyl group represented by R^{B1}, R^{B2}, R^{B3}, R^{B4} and R^{B5} may be any of a chain or branched alkenyl group. Specific examples include groups such as a vinyl group, a 1-propenyl group, an allyl group, a 2-propenyl group, a 1-butenyl group, a 2-butenyl group and a 2-methylallyl group. The present invention is not limited to these substituents listed by way of example.

Examples of C1 to C6 haloalkyl groups represented by R^{B1}, R^{B2}, R^{B3}, R^{B4} and R^{B5} include the substituents listed as examples of C1 to C6 haloalkyl groups represented by R^{A}.

Examples of C6 to C22 aryl groups represented by R^{B1}, R^{B2}, R^{B3}, R^{B4} and R^{B5} include the substituents listed as examples of C6 to C22 aryl groups represented by R^{A}.

Examples of C3 to C20 heteroaryl groups represented by R^{B1}, R^{B2}, R^{B3}, R^{B4} and R^{B5} include the substituents listed as examples of C3 to C20 heteroaryl groups represented by R^{A}.

Specific examples of C7 to C14 aralkyl groups represented by R^{B1}, R^{B2}, R^{B3}, R^{B4} and R^{B5} optionally substituted with one or more halogen atoms include groups such as a benzyl group, a phenethyl group, a (4-methylphenyl)methyl group, a (3-methylphenyl)methyl group, a (2-methylphenyl)methyl group, a (4-fluorophenyl)methyl group, a (3-fluorophenyl)methyl group, a (2-fluorophenyl)methyl group, a (4-bromophenyl)methyl group, a (4-chlorophenyl)methyl group, a (4-iodophenyl)methyl group, a (4-trifluoromethylphenyl)methyl group, a 1-naphthylmethyl group, a 2-naphthylmethyl group, a 1-naphthylethyl group, a 2-naphthylethyl group, (6-fluoronaphthalen-2-yl)methyl, (4-fluoronaphthalen-1-yl)methyl and a (6-trifluoromethylnaphthalen-2-yl)methyl group. The present invention is not limited to these substituents listed by way of example.

A C2 to C4 alkenyl group represented by R^{B1}, R^{B2}, R^{B3}, R^{B4} and R^{B5} may be any of a chain or branched alkenyl group. Specific examples include groups such as a vinyl group, a 1-propenyl group, an allyl group, a 2-propenyl group, a 1-butenyl group, a 2-butenyl group and a 2-methylallyl group. The present invention is not limited to these substituents listed by way of example.

A C6 to C22 aryl or C3 to C20 heteroaryl group represented by R^{B1}, R^{B2}, R^{B3}, R^{B4} and R^{B5}, furthermore, may be substituted with one or more substituents selected from the group consisting of halogen atoms, C2 to C4 alkenyl groups, C1 to C6 alkyloxy groups, C1 to C6 fluoroalkyl groups, C2 to C13 acyl groups, a cyano group and a nitro group (group T₂2) .

Examples of halogen atoms that belong to group T₂2 include the substituents listed as examples of halogen atoms that belong to group T₂1.

Examples of C1 to C6 alkyloxy groups that belong to group T₂2 include the substituents listed as examples of C1 to C6 alkyloxy groups that belong to group T₂1.

Examples of C1 to C6 fluoroalkyl groups that belong to group T₂2 include the substituents listed as examples of C1 to C6 fluoroalkyl groups that belong to group T₂1.

Examples of C2 to C13 acyl groups that belong to group T₂2 include the substituents listed as examples of C2 to C13 acyl groups that belong to group T₂1.

Examples of C6 to C22 aryl groups at R^{B1}, R^{B2}, R^{B3}, R^{B4} and R^{B5} optionally substituted with one or more substituents selected from group T₂2, therefore, include the substituents listed as examples for a C6 to C22 aryl group at R^{A} optionally substituted with one or more substituents selected from group T₂1 and groups such as a 9,9-dimethylfluoren-2-yl group and a 9,9-dimethylfluoren-3-yl group.

Examples of C3 to C20 heteroaryl groups at R^{B1}, R^{B2}, R^{B3}, R^{B4} and R^{B5} optionally substituted with one or more substituents selected from group T₂2, furthermore, include the substituents listed as examples for a C3 to C20 heteroaryl group at R^{A} optionally substituted with one or more substituents selected from group T₂1.

Examples of C1 to C6 alkyl groups represented by R^{BA1} and R^{BA2} in general formula (BA1), R^{BO1} in general formula (BO1) and R^{BS1} in general formula (BS1) include the substituents listed as examples of C1 to C6 alkyl groups that belong to group T₂2.

Examples of C6 to C22 aryl groups represented by R^{BA1} and R^{BA2} in general formula (BA1), R^{BO1} in general formula (BO1) and R^{BS1} in general formula (BS1) include the substituents listed as examples of C6 to C22 aryl groups represented by R^{A}.

Examples of C3 to C20 heteroaryl groups represented by R^{BA1} and R^{BA2} in general formula (BA1), R^{BO1} in general formula (BO1) and R^{BS1} in general formula (BS1) include the substituents listed as examples of C3 to C20 heteroaryl groups represented by R^{A}.

A C1 to C6 alkyl, C6 to C22 aryl or C3 to C20 heteroaryl group represented by R^{BA1} and R^{BA2} in general formula (BA1), furthermore, may be substituted with one or more substituents selected from the group consisting of halogen atoms, C1 to C6 alkyloxy groups, C1 to C6 fluoroalkyl groups, C2 to C13 acyl groups, a hydroxyl group, a cyano group and a nitro group (group T₂3) .

A C1 to C6 alkyl, C6 to C22 aryl or C3 to C20 heteroaryl group represented by R^{BO1} in general formula (BO1), moreover, may be substituted with one or more substituents selected from group T₂3.

A C1 to C6 alkyl, C6 to C22 aryl or C3 to C20 heteroaryl group represented by R^{BS1} in general formula (BS1), moreover, may be substituted with one or more substituents selected from group T₂3.

Examples of halogen atoms that belong to group T₂3 include the substituents listed as examples of halogen atoms that belong to group T₂1.

Examples of C1 to C6 alkyloxy groups that belong to group T₂3 include the substituents listed as examples of C1 to C6 alkyloxy groups that belong to group T₂1.

Examples of C1 to C6 fluoroalkyl groups that belong to group T₂3 include the substituents listed as examples of C1 to C6 fluoroalkyl groups that belong to group T₂1.

Examples of C2 to C13 acyl groups that belong to group T₂3 include the substituents listed as examples of C2 to C13 acyl groups that belong to group T₂1.

Specific examples of C1 to C6 alkyl groups at R^{BA1} and R^{BA2} optionally substituted with one or more substituents selected from group T₂3, C1 to C6 alkyl groups at R^{BO1} optionally substituted with one or more substituents selected from group T₂3 and C1 to C6 alkyl groups at R^{BS1} optionally substituted with one or more substituents selected from group T₂3, therefore, include groups such as a 2,2,2-trifluoroethyl group, a 2-methoxyethyl group, a 3-methoxypropyl group, a 2-hydroxyethyl group, a 2-acetylethyl group, a 3-acetylpropyl group, a 2-benzoylethyl group, a 3-benzoylpropyl group and a 3-hydroxypropyl group. The present invention is not limited to these substituents listed by way of example.

Examples of C6 to C22 aryl groups at R^{BA1} and R^{BA2} optionally substituted with one or more substituents selected from group T₂3, C6 to C22 aryl groups at R^{BO1} optionally substituted with one or more substituents selected from group T₂3 and C6 to C22 aryl groups at R^{BS1} optionally substituted with one or more substituents selected from group T₂3, furthermore, include the substituents listed as examples for a C6 to C22 aryl group at R^{A} optionally substituted with one or more substituents selected from group T₂1.

Examples of C3 to C20 heteroaryl groups at R^{BA1} and R^{BA2} optionally substituted with one or more substituents selected from group T₂3, C3 to C20 heteroaryl groups at R^{BO1} optionally substituted with one or more substituents selected from group T₂3 and C3 to C20 heteroaryl groups at R^{BS1} optionally substituted with one or more substituents selected from group T₂3, moreover, include the substituents listed as examples for a C3 to C20 heteroaryl group at R^{A} optionally substituted with one or more substituents selected from group T₂1.

In addition, R^{BA1} and R^{BA2} may form a five-membered, six-membered or seven-membered ring including the nitrogen atom to which they are bound. Specific examples include groups such as an azetidyl group, a pyrrolidinyl group, a piperidino group, an azepanyl group, a morpholino group, a 4-methylpiperazinyl group, a carbazoyl group, a phenoxazinyl group, a phenothiazinyl group and a 10,11-dihydro-5H-dibenz[b,f]azepinyl group. The present invention is not limited to these substituents listed by way of example.

A substituted or unsubstituted amino group indicated by general formula (BA1), therefore, may be substituted with one or more substituents selected from group T₂3, and specific examples include groups such as;
dialkylated amino groups, such as a dimethylamino group, a diethylamino group, a dipropylamino group, a diisopropylamino group, a dibutylamino group, a bis(2-methylpropyl)amino group, a bis(2,2-dimethylpropyl)amino group, a bis(3-cyclopropyl)amino group, a dicyclopropylamino group, a bis(2-methylbutyl)amino group, a bis(3-methylbutyl)amino group, a di(tert-butyl)amino group, a dicyclobutylamino group, a dipentylamino group, a bis(2-methylpentyl)amino group, a di(1-methylbutyl)amino group, a bis(1,2-dimethylbutyl)amino group, a di(1-ethylpropyl)amino group, a dicyclopentylamino group, a dihexylamino group, a dicyclohexylamino group, an N-ethyl-N-methylamino group, an N-methyl-N-propyl group, an N-cyclopropyl-N-methylamino group, an N-butyl-N-methylamino group, an N-cyclobutyl-N-methylamino group, an N-methyl-N-pentyl group, an N-cyclopentyl-N-methylamino group, an N-hexyl-N-methylamino group, an N-cyclohexyl-N-methylamino group, an N-cycloheptyl-N-methylamino group, an N-methyl-N-octylamino group, an N-methyl-N-nonylamino group, an N-decyl-N-methylamino group, an N-methyl-N-undecylamino group, a bis(2-methoxyethyl)amino group, a bis(2-ethoxyethyl)amino group, a bis(2,2,2-trifluoroethyl)amino group, a bis(3-chloropropyl)amino group, a bis(3-bromopropyl)amino group, a bis(2-bromoethyl)amino group, a bis(2-chloroethyl)amino group, a bis(2-cyanoethyl)amino group and a bis(3-cyanopropyl)amino group;
monoalkylated amino groups, such as a methylamino group, an ethylamino group, a propylamino group, an isopropylamino group, a butylamino group, a 2-methylpropylamino group, a 2,2-dimethylpropylamino group, a 3-cyclopropylamino group, a cyclopropylamino group, a tert-butylamino group, a cyclobutylamino group, a pentylamino group, a 2-methylpentylamino group, a hexylamino group, a (2-methoxyethyl)amino group, a (2-ethoxyethyl)amino group, a (2,2,2-trifluoroethyl)amino group, a 3-chloropropylamino group, a 3-bromopropylamino group, a 2-bromoethylamino group, a 2-chloroethylamino group, a 2-cyanoethylamino group and a 3-cyanopropylamino group;
diarylated amino groups, such as a diindenylamino group, an N-indenyl-N-phenylamino group, a diphenylamino group, a bis(2-methylphenyl)amino group, a bis(3-methylphenyl)amino group, a bis(4-methylphenyl)amino group, a bis(2,3-dimethylphenyl)amino group, a bis(2,4-dimethylphenyl)amino group, a bis(2,5-dimethylphenyl)amino group, a bis(2,6-dimethylphenyl)amino group, a bis(3,4-dimethylphenyl)amino group, a bis(3,5-dimethylphenyl)amino group, a bis(2,3,4-trimethylphenyl)amino group, a bis(2,3,5-trimethylphenyl)amino group, a bis(2,3,6-trimethylphenyl)amino group, a bis(2,4,5-trimethylphenyl)amino group, a bis(2,4,6-trimethylphenyl)amino group, a bis(3,4,5-trimethylphenyl)amino group, a bis(2,3,4,5-tetramethylphenyl)amino group, a bis(2,3,4,6-tetramethylphenyl)amino group, a bis(2,3,5,6-tetramethylphenyl)amino group, a bis(2-ethylphenyl)amino group, a bis(3-ethylphenyl)amino group, a bis(4-ethylphenyl)amino group, a bis(2,3-diethylphenyl)amino group, a bis(2,4-diethylphenyl)amino group, a bis(2,5-diethylphenyl)amino group, a bis(2,6-diethylphenyl)amino group, a bis(3,4-diethylphenyl)amino group, a bis(3,5-diethylphenyl)amino group, a bis(2-propylphenyl)amino group, a bis(3-propylphenyl)amino group, a bis(4-propylphenyl)amino group, a bis(2-isopropylphenyl)amino group, a bis(3-isopropylphenyl)amino group, a bis(4-isopropylphenyl)amino group, a bis(2-cyclopropylphenyl)amino group, a bis(3-cyclopropylphenyl)amino group, a bis(4-cyclopropylphenyl)amino group, a bis(2-butylphenyl)amino group, a bis(3-butylphenyl)amino group, a bis(4-butylphenyl)amino group, a bis[2-(1-methylpropyl)phenyl]amino group, a bis[3-(1-methylpropyl)phenyl]amino group, a bis[4-(1-methylpropyl)phenyl]amino group, a bis[2-(2-methylpropyl)phenyl]amino group, a bis[3-(2-methylpropyl)phenyl]amino group, a bis[4-(2-methylpropyl)phenyl]amino group, a bis(2-cyclobutylphenyl)amino group, a bis(3-cyclobutylphenyl)amino group, a bis(4-cyclobutylphenyl)amino group, a di(1-naphthyl)amino group, a di(2-naphthyl)amino group, an N-(2-methylphenyl)-N-phenylamino group, an N-(3-methylphenyl)-N-phenylamino group, an N-(4-methylphenyl)-N-phenylamino group, an N-(2,3-dimethylphenyl)-N-phenylamino group, an N-(2,4-dimethylphenyl)-N-phenylamino group, an N-(2,5-dimethylphenyl)-N-phenylamino group, an N-(2,6-dimethylphenyl)-N-phenylamino group, an N-(3,4-dimethylphenyl)-N-phenylamino group, an N-(3,5-dimethylphenyl)-N-phenylamino group, an N-(2,3,4-trimethylphenyl)-N-phenylamino group, an N-(2,3,5-trimethylphenyl)-N-phenylamino group, an N-(2,3,6-trimethylphenyl)-N-phenylamino group, an N-(2,4,5-trimethylphenyl)-N-phenylamino group, an N-(2,4,6-trimethylphenyl)-N-phenylamino group, an N-(3,4,5-trimethylphenyl)-N-phenylamino group, an N-(1-naphthyl)-N-phenylamino group, an N-(2-naphthyl)-N-phenylamino group, a bis(4-methoxyphenyl)amino group, a bis(2-methoxyphenyl)amino group, a bis[4-(trifluoromethyl)phenyl]amino group, a bis[4-fluorophenyl]amino group, a bis[3,5-difluorophenyl]amino group, a bis(4-acetylphenyl)amino group, a (2-acetylphenyl)(4-acetylphenyl)amino group, a bis(4-pivaloylphenyl)amino group, a bis(2-acetylphenyl)amino group, a bis(4-benzoylphenyl)amino group, a (2-benzoylphenyl)(4-benzoylphenyl)amino group and a bis(4-cyanophenyl)amino group;
diheteroarylated amino groups, such as a bis(2-pyrrolyl)amino group, a bis(1-methylpyrrol-2-yl)amino group, a bis(1-ethylpyrrol-2-yl)amino group, a bis(1-phenylpyrrol-2-yl)amino group, a bis(2-thienyl)amino group, a bis(3-thienyl)amino group, a bis(2-furyl)amino group, a bis(3-furyl)amino group, a bis(2-benzothienyl)amino group, a bis(3-benzothienyl)amino group, a bis(2-benzofuranyl)amino group, a bis(3-benzofuranyl)amino group, a bis(1-methylindol-2-yl)amino group, a bis(1-methylindol-2-yl)amino group, a bis(3-benzofuranyl)amino group, a bis(5-fluoropyrrol-2-yl)amino group, a bis(5-fluoro-1-methylpyrrol-2-yl)amino group, a bis(5-fluoro-1-ethylpyrrol-2-yl)amino group, a bis(5-fluoro-1-phenylpyrrol-2-yl)amino group, a bis(5-fluoro-2-thienyl)amino group, a bis(5-trifluoromethyl-2-thienyl)amino group, a bis(5-fluoro-3-thienyl)amino group, a bis(5-fluoro-2-furyl)amino group, a bis(5-trifluoromethyl-2-furyl)amino group, a bis(5-fluoro-3-furyl)amino group, a bis(5-fluoro-2-benzothienyl)amino group, a bis(5-fluoro-3-benzothienyl)amino group, a bis(5-fluoro-2-benzofuranyl)amino group, a bis(5-fluoro-3-benzofuranyl)amino group, a bis(1-methylindol-2-yl)amino group, a bis(1-methylindol-3-yl)amino group, a bis(5-fluoro-3-benzofuranyl)amino group, a 2-fluorophenothiazinyl group, a 2-trifluoromethylphenothiazinyl group and a 2-trifluoromethylphenoxazinyl group;
monoarylated amino groups, such as an indenylamino group, a phenylamino group, a 2-methylphenylamino group, a 3-methylphenylamino group, a 4-methylphenylamino group, a 2,3-dimethylphenylamino group, a 2,4-dimethylphenylamino group, a 2,5-dimethylphenylamino group, a 2,6-dimethylphenylamino group, a 3,4-dimethylphenylamino group, a 3,5-dimethylphenylamino group, a 2,3,4-trimethylphenylamino group, a 2,3,5-trimethylphenylamino group, a 2,3,6-trimethylphenylamino group, a 2,4,5-trimethylphenylamino group, a 2,4,6-trimethylphenylamino group, a 3,4,5-trimethylphenylamino group, a 2,3,4,5-tetramethylphenylamino group, a 2,3,4,6-tetramethylphenylamino group, a 2,3,5,6-tetramethylphenylamino group, a 2-ethylphenylamino group, a 3-ethylphenylamino group, a 4-ethylphenylamino group, a 2,3-diethylphenylamino group, a 2,4-diethylphenylamino group, a 2,5-diethylphenylamino group, a 2,6-diethylphenylamino group, a 3,4-diethylphenylamino group, a 3,5-diethylphenylamino group, a 2-propylphenylamino group, a 3-propylphenylamino group, a 4-propylphenylamino group, a 2-isopropylphenylamino group, a 3-isopropylphenylamino group, a 4-isopropylphenylamino group, a 2-cyclopropylphenylamino group, a 3-cyclopropylphenylamino group, a 4-cyclopropylphenylamino group, a 2-butylphenylamino group, a 3-butylphenylamino group, a 4-butylphenylamino group, a 2-(1-methylpropyl)phenylamino group, a 3-(1-methylpropyl)phenylamino group, a 4-(1-methylpropyl)phenylamino group, a 2-(2-methylpropyl)phenylamino group, a 3-(2-methylpropyl)phenylamino group, a 4-(2-methylpropyl)phenylamino group, a 2-cyclobutylphenylamino group, a 3-cyclobutylphenylamino group, a 4-cyclobutylphenylamino group, a 1-naphthylamino group, a 2-naphthylamino group, a (4-methoxyphenyl)amino group, a (2-methoxyphenyl)amino group, a [4-(trifluoromethyl)phenyl]amino group, a (4-acetylphenyl)amino group, a (2-acetylphenyl)amino group, a (4-pivaloylphenyl)amino group and a (4-benzoylphenyl)amino group;
monoheteroarylated amino groups, such as a 2-pyrrolylamino group, a 2-thienylamino group, a 3-thienylamino group, a 2-furylamino group, a 3-furylamino group, a 2-(1-methylindolyl)amino group, a 2-benzothienyl group, a 3-benzothienyl group, a 2-benzofuranyl group and a 3-benzofuranyl group; and
cyclic amino groups, such as an azetidyl group, a pyrrolidinyl group, a piperidino group, an azepanyl group, a morpholino group, a 4-methylpiperazinyl group, a carbazoyl group, a phenoxazinyl group, a phenothiazinyl group and a 10,11-dihydro-5H-dibenz[b,f]azepinyl group. The present invention is not limited to these substituents listed by way of example. A diarylated or diheteroarylated amino group is preferred because it gives the complex optical characteristics suitable for use as an optical functional material. A diphenylamino group, bis[4-(trifluoromethyl)phenyl]amino group or bis(4-cyanophenyl)amino group is more preferred because of the ease of synthesis.

A substituted or unsubstituted oxy group indicated by general formula (BO1), furthermore, may be substituted with one or more substituents selected from group T₂3, and specific examples include groups such as;
substituted alkyloxy groups, such as a methoxy group, an ethoxy group, a propyloxy group, an isopropyloxy group, a butyloxy group, a 2-methylpropyloxy group, a 2,2-dimethylpropyloxy group, a 3-cyclopropylpropyloxy group, a cyclopropyloxy group, a 2-methylbutyloxy group, a 3-methylbutyloxy group, a tert-butyloxy group, a cyclobutyloxy group, a pentyloxy group, a 2-methylpentyloxy group, a 1-methylbutyloxy group, a 1,2-dimethylbutyloxy group, a 1-ethylpropyloxy group, a cyclopentyloxy group, a hexyloxy group and a cyclohexyloxy group; and
substituted aryloxy groups, such as an indenyloxy group, a phenyloxy group, a 2-methylphenyloxy group, a 3-methylphenyloxy group, a 4-methylphenyloxy group, a 2,3-dimethylphenyloxy group, a 2,4-dimethylphenyloxy group, a 2,5-dimethylphenyloxy group, a 2,6-dimethylphenyloxy group, a 3,4-dimethylphenyloxy group, a 3,5-dimethylphenyloxy group, a 2,3,4-trimethylphenyloxy group, a 2,3,5-trimethylphenyloxy group, a 2,3,6-trimethylphenyloxy group, a 2,4,5-trimethylphenyloxy group, a 2,4,6-trimethylphenyloxy group, a 3,4,5-trimethylphenyloxy group, a 2,3,4,5-tetramethylphenyloxy group, a 2,3,4,6-tetramethylphenyloxy group, a 2,3,5,6-tetramethylphenyloxy group, a 2-ethylphenyloxy group, a 3-ethylphenyloxy group, a 4-ethylphenyloxy group, a 2,3-diethylphenyloxy group, a 2,4-diethylphenyloxy group, a 2,5-diethylphenyloxy group, a 2,6-diethylphenyloxy group, a 3,4-diethylphenyloxy group, a 3,5-diethylphenyloxy group, a 2-propylphenyloxy group, a 3-propylphenyloxy group, a 4-propylphenyloxy group, a 2-isopropylphenyloxy group, a 3-isopropylphenyloxy group, a 4-isopropylphenyloxy group, a 2-cyclopropylphenyloxy group, a 3-cyclopropylphenyloxy group, a 4-cyclopropylphenyloxy group, a 2-butylphenyloxy group, a 3-butylphenyloxy group, a 4-butylphenyloxy group, a 2-(1-methylpropyl)phenyloxy group, a 3-(1-methylpropyl)phenyloxy group, a 4-(1-methylpropyl)phenyloxy group, a 2-(2-methylpropyl)phenyloxy group, a 3-(2-methylpropyl)phenyloxy group, a 4-(2-methylpropyl)phenyloxy group, a 2-cyclobutylphenyloxy group, a 3-cyclobutylphenyloxy group, a 4-cyclobutylphenyloxy group, a (biphenyl-2-yl)oxy group, a (biphenyl-3-yl)oxy group, a (biphenyl-4-yl)oxy group, a 4-fluorophenyloxy group, a 4-trifluoromethylphenyl group a (phenanthren-9-yl)oxy group and an (anthracen-9-yl)oxy group. The present invention is not limited to these substituents listed by way of example. A methoxy group, ethoxy group, or phenoxy group is preferred because of the ease of synthesis.

A substituted or unsubstituted thio group indicated by general formula (BS1), moreover, may be substituted with one or more substituents selected from group T₂3, and specific examples include groups such as;
substituted alkylthio groups, such as a methylthio group, an ethylthio group, a propylthio group, a 1-(1-methylethyl)thio group, a butylthio group, a 2-methylpropylthio group, a 2,2-dimethylpropylthio group, a 3-cyclopropylpropylthio group, a cyclopropylthio group, a 2-methylbutylthio group, a 3-methylbutylthio group, a 1-(1,1-dimethylethyl)thio group, a cyclobutylthio group, a pentylthio group, a 2-methylpentylthio group, a 1-methylbutylthio group, a 1,2-dimethylbutylthio group, a 1-ethylpropylthio group, a cyclopentylthio group, a hexylthio group and a cyclohexylthio group; and
substituted arylthio groups, such as a phenylthio group, a 2-methylphenylthio group, a 3-methylphenylthio group, a 4-methylphenylthio group, a 2,3-dimethylphenylthio group, a 2,4-dimethylphenylthio group, a 2,5-dimethylphenylthio group, a 2,6-dimethylphenylthio group, a 3,4-dimethylphenylthio group, a 3,5-dimethylphenylthio group, a 2,3,4-trimethylphenylthio group, a 2,3,5-trimethylphenylthio group, a 2,3,6-trimethylphenylthio group, a 2,4,5-trimethylphenylthio group, a 2,4,6-trimethylphenylthio group, a 3,4,5-trimethylphenylthio group, a 2,3,4,5-tetramethylphenylthio group, a 2,3,4,6-tetramethylphenylthio group, a 2,3,5,6-tetramethylphenylthio group, a 2-ethylphenylthio group, a 3-ethylphenylthio group, a 4-ethylphenylthio group, a 2,3-diethylphenylthio group, a 2,4-diethylphenylthio group, a 2,5-diethylphenylthio group, a 2,6-diethylphenylthio group, a 3,4-diethylphenyl group, a 3,5-diethylphenyl group, a 2-propylphenyl group, a 3-propylphenylthio group, a 4-propylphenylthio group, a 2-isopropylphenylthio group, a 3-isopropylphenylthio group, a 4-isopropylphenylthio group, a 2-cyclopropylphenylthio group, a 3-cyclopropylphenylthio group, a 4-cyclopropylphenylthio group, a 2-butylphenylthio group, a 3-butylphenylthio group, a 4-butylphenylthio group, a 2-(1-methylpropyl)phenylthio group, a 3-(1-methylpropyl)phenylthio group, a 4-(1-methylpropyl)phenylthio group, a 2-(2-methylpropyl)phenylthio group, a 3-(2-methylpropyl)phenylthio group, a 4-(2-methylpropyl)phenylthio group, a 2-cyclobutylphenylthio group, a 3-cyclobutylphenylthio group, a 4-cyclobutylphenylthio group, a 1-naphthylthio group, a 2-naphthylthio group, a (biphenyl-2-yl)thio group, a (biphenyl-3-yl)thio group, a (biphenyl-4-yl)thio group, a 9-anthrylthio group, a 2-phenanthrenylthio group, a 3-phenanthrenylthio group and a 9-phenanthrenylthio group. The present invention is not limited to these substituents listed by way of example.

Of R^{B1}, R^{B2}, R^{B3} and R^{B4}, furthermore, two adjacent substituents may form a five-membered, six-membered or seven-membered ring together with the benzene ring to which they are bound. For example, possible structures of the quinolinonato ligand (1qu) include the structures represented by (1qu-6) to (1qu-12), (1qu-3) and (1qu-4) below. The present invention is not limited to these.

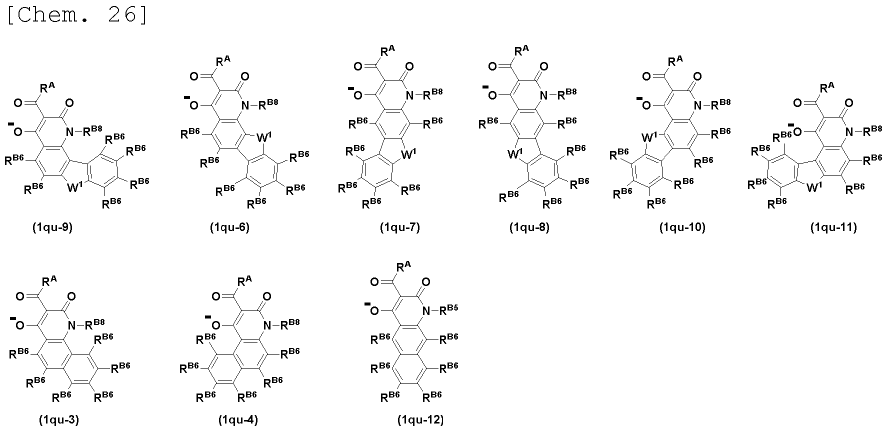

(In the formulae, R^{A} is synonymous with the meaning described above.

R^{B6} represents a hydrogen atom, halogen atom or C1 to C4 fluoroalkyl group.

R^{B8} represents a hydrogen atom, C1 to C10 alkyl group or C6 to C22 aryl group optionally substituted with one or more fluorine atoms or C1 to C4 fluoroalkyl groups.

W¹ represents an oxygen atom, sulfur atom, nitrogen atom optionally substituted with a C1 to C12 hydrocarbon group or methylene group optionally substituted with one or more C1 to C12 hydrocarbon groups. It should be noted that unsubstituted nitrogen atom refers to an -NH- group.)

The definitions of R^{B6}, R^{B8} and W¹ will now be described individually.

### (R^{B6} and R^{B8})

Examples of halogen atoms represented by R^{B6} include the substituents listed as examples of halogen atoms that belong to group T₂1.

A C1 to C4 fluoroalkyl group represented by R^{B6} may be any of a linear-chain, branched or cyclic alkyl group. Specific examples include groups such as a trifluoromethyl group, a difluoromethyl group, a perfluoroethyl group, a 2,2,2-trifluoroethyl group, a 1,1-difluoroethyl group, a 2,2-difluoroethyl group, perfluoropropyl group, a 2,2,3,3,3-pentafluoropropyl group, a 2,2,3,3-tetrafluoropropyl group, a 3,3,3-trifluoropropyl group, a 1,1-difluoropropyl group, a 1,1,1,2,3,3,3-hexafluoro-2-propyl group, a 2,2,2-trifluoro-1-(trifluoromethyl)ethyl group, a perfluorobutyl group, a 2,2,3,3,4,4,4-heptafluorobutyl group, a 3,3,4,4,4-pentafluorobutyl group, a 4,4,4-trifluorobutyl group, a 1,2,2,3,3,3-hexafluoro-1-(trifluoromethyl)propyl group, a 1-(trifluoromethyl)propyl group, a 1-methyl-3,3,3-trifluoropropyl group and a perfluorocyclobutyl group. The present invention is not limited to these substituents listed by way of example.

R^{B6} is preferably a hydrogen atom because of the ease of synthesis.

Examples of C1 to C10 alkyl groups represented by R^{B8} include the groups listed as examples of C1 to C10 alkyl groups represented by R^{B1}, R^{B2}, R^{B3}, R^{B4} and R^{B5}.

When R^{B8} is a C6 to C22 aryl group, the C6 to C22 aryl group may be substituted with one or more fluorine atoms or C1 to C4 fluoroalkyl groups. Specific examples include groups such as a phenyl group, a 2-fluorophenyl group, a 3-fluorophenyl group, a 4-fluorophenyl group, a 4-(trifluoromethyl)phenyl group, a 2,3-difluorophenyl group, a 2,4-difluorophenyl group, a 2,5-difluorophenyl group, a 2,6-difluorophenyl group, a 3,4-difluorophenyl group, a 3,5-difluorophenyl group, a 2,3,4-trifluorophenyl group, a 2,3,5-trifluorophenyl group, a 2,3,6-trifluorophenyl group, a 2,4,5-trifluorophenyl group, a 2,4,6-trifluorophenyl group, a 3,4,5-trifluorophenyl group, a 2,3,4,5-tetrafluorophenyl group, a 2,3,4,6-tetrafluorophenyl group, a 2,3,5,6-tetrafluorophenyl group, a perfluorophenyl group, a 1-naphthyl group, a 2-naphthyl group, a 1-fluoronaphthyl group, a 2-fluoronaphthyl group, a 4'-fluorobiphenyl-2-yl group, a 4'-fluorobiphenyl-2-yl group, a 4'-fluorobiphenyl-3-yl group, a 4'-fluorobiphenyl-4-yl group, a 4'-(trifluoromethyl)biphenyl-4-yl group, a 9-anthryl group, a 2-phenanthrenyl group, a 3-phenanthrenyl group and a 9-phenanthrenyl group. The present invention is not limited to these substituents listed by way of example.

### (W1)

When W¹ is a nitrogen atom optionally substituted with a C1 to C12 hydrocarbon group, specific examples of the C1 to C12 hydrocarbon group include groups such as a methyl group, an ethyl group, a propyl group, an isopropyl group, a butyl group, a 2-methylpropyl group, a 2,2-dimethylpropyl group, a 3-cyclopropylpropyl group, a cyclopropyl group, a 2-methylbutyl group, a 3-methylbutyl group, a tert-butyl group, a cyclobutyl group, a pentyl group, a 2-methylpentyl group, a 1-methylbutyl group, a 1,2-dimethylbutyl group, a 1-ethylpropyl group, a cyclopentyl group, a hexyl group, a cyclohexyl group, a heptyl group, an octyl group, a nonyl group, a decyl group, a phenyl group, a naphthyl group and a biphenylyl group. The present invention is not limited to these substituents listed by way of example.

When W¹ is a methylene group optionally substituted with one or more C1 to C12 hydrocarbon groups, examples of the C1 to C12 hydrocarbon groups include the groups listed as examples in the context of the C1 to C12 hydrocarbon group of a nitrogen atom optionally substituted with a C1 to C12 hydrocarbon group.

W¹ is preferably an oxygen atom, sulfur atom, methylene group optionally substituted with one or more C1 to C4 alkyl groups or nitrogen atom optionally substituted with a C1 to C4 alkyl group or C6 to C12 aryl group because of the ease of synthesis, more preferably an oxygen atom, sulfur atom or dimethylmethylene group because of easy availability of raw materials.

When W¹ is a methylene group optionally substituted with one or more C1 to C4 alkyl groups, the C1 to C4 alkyl groups may be any of linear-chain, branched or cyclic. Examples include a methyl group, an ethyl group, a propyl group, an isopropyl group, a butyl group, a 1-methylpropyl group, a 2-methylpropyl group, a cyclopropyl group, a butyl group, a tert-butyl group and a cyclobutyl group.

When W¹ is a nitrogen atom optionally substituted with a C1 to C4 alkyl group, examples of the C1 to C4 alkyl group include the groups listed as examples in the context of the C1 to C4 alkyl groups of a methylene group optionally substituted with one or more C1 to C4 alkyl groups.

When W¹ is a nitrogen atom optionally substituted with a C6 to C12 aryl group, specific examples of the C6 to C12 aryl group include groups such as a phenyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a 2-ethylphenyl group, a 3-ethylphenyl group, a 4-ethylphenyl group, a 2,3-diethylphenyl group, a 2,4-diethylphenyl group, a 2,5-diethylphenyl group, a 2,6-diethylphenyl group, a 3,4-diethylphenyl group, a 3,5-diethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-cyclopropylphenyl group, a 3-cyclopropylphenyl group, a 4-cyclopropylphenyl group, a 2-butylphenyl group, a 3-butylphenyl group, a 4-butylphenyl group, a 2-(1-methylpropyl)phenyl group, a 3-(1-methylpropyl)phenyl group, a 4-(1-methylpropyl)phenyl group, a 2-(2-methylpropyl)phenyl group, a 3-(2-methylpropyl)phenyl group, a 4-(2-methylpropyl)phenyl group, a 2-cyclobutylphenyl group, a 3-cyclobutylphenyl group, a 4-cyclobutylphenyl group, a 1-naphthyl group, a 2-naphthyl group, a 2-biphenyl group, a 3-biphenyl group, a 4-biphenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-(trifluoromethyl)phenyl group, a 3-(trifluoromethyl)phenyl group, a 4-(trifluoromethyl)phenyl group, a 2-(perfluoropropyl)phenyl group, a 3-(perfluoropropyl)phenyl group, a 4-(perfluoropropyl)phenyl group, a 2-acetylphenyl group, a 3-acetylphenyl group, a 4-acetylphenyl group, a 2-pivaloylphenyl group, a 3-pivaloylphenyl group, a 4-pivaloylphenyl group, a 2-benzoylphenyl group, a 3-benzoylphenyl group, a 4-benzoylphenyl group, a 2-naphthoylphenyl group, a 3-naphthoylphenyl group, a 4-naphthoylphenyl group, a 4-methoxy-1-naphthyl group, a 6-methoxy-2-naphthyl group, a 4'-methoxybiphenyl-2-yl group, a 4'-methoxybiphenyl-3-yl group, a 4'-methoxybiphenyl-4-yl group, a 10-methoxy-9-anthryl group, a 2-fluorophenyl group, a 3-fluorophenyl group, a 4-fluorophenyl group and a 3,4-difluorophenyl group. The present invention is not limited to these substituents listed by way of example.

R^{B5} and R^{B1}, furthermore, may form a quinolinone five-membered, six-membered or seven-membered ring, which has a ring of five members, six members or seven members added through annulation, together with the quinolinone ring system to which they are bound. For example, a possible substructure of the quinolinonato ligand is the structure represented by (1qu-1) or (1qu-14) below. The present invention is not limited to these.

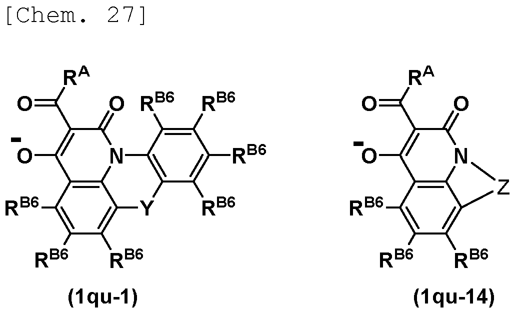

(In the formulae, R^{A} and R^{B6} are synonymous with the meanings described above.

Y represents a single bond, ethylene group, vinylene group optionally substituted with one or more methoxy groups or chlorine atoms, oxygen atom, sulfur atom, nitrogen atom optionally substituted with a C1 to C12 hydrocarbon group or methylene group optionally substituted with one or more C1 to C12 hydrocarbon groups.

Z represents an ethylene group, vinylene group or trimethylene group, and one of the methylene groups in the trimethylene group may be substituted with an oxygen atom.)

### (Y)

When Y is a nitrogen atom optionally substituted with a C1 to C12 hydrocarbon group, examples of the C1 to C12 hydrocarbon group include the groups listed as examples in the context of the C1 to C12 hydrocarbon group of a nitrogen atom represented by W¹ optionally substituted with a C1 to C12 hydrocarbon group.

When Y is a methylene group optionally substituted with one or more C1 to C12 hydrocarbon groups, examples of the C1 to C12 hydrocarbon groups include the groups listed as examples in the context of the C1 to C12 hydrocarbon group of a nitrogen atom represented by W¹ optionally substituted with a C1 to C12 hydrocarbon group.

Y is preferably a single bond, oxygen atom, sulfur atom, dimethylmethylene group, diphenylmethylene group or nitrogen atom substituted with a methyl group or phenyl group because of easy availability of raw materials, more preferably an oxygen atom or sulfur atom because of the ease of synthesis.

### (Z)

When Z is a trimethylene group, one of the methylene groups in the trimethylene group may be substituted with an oxygen atom. Specific examples include a trimethylene group, an oxyethylene group (-O-CH₂CH₂-), a methyleneoxymethylene group (-CH₂OCH₂-) and a methylenedioxy group (-OCH₂O-). A trimethylene group or oxyethylene group (-O-CH₂CH₂-) because of easy availability of raw materials.

### <CU>

CU is a coumarinato ligand represented by general formula (1cu) (Hereinafter also referred to as a coumarinato ligand (1cu).).

In the rare earth complex (1) according to the present invention, the coumarinato ligand (1cu) coordinates with M³⁺ through one or two oxygen atoms. The complex, furthermore, has the coordination structures of rare earth complexes (1ci) to (1cvi) below varying in the tautomeric structure of the coumarinato ligand (1cu). The rare earth complex (1) according to the present invention includes all of the rare earth complexes (1ci) to (1cvi), but these isomers are herein expressed as a rare earth complex (1c) for the sake of convenience.

The coordination structure of the rare earth complex (1) according to the present invention, furthermore, is preferably that of a rare earth complex (1ci) or (1cii) because this gives the complex optical characteristics suitable for use as an optical functional material. It should be noted that a rare earth complex (1ci) can tautomerize to form a rare earth complex (1cii). A rare earth complex (1ci), however, includes a rare earth complex (1cii). When a coordination structure is described herein, therefore, a rare earth complex (1cii), too, is expressed as a rare earth complex (1ci) for the sake of convenience.

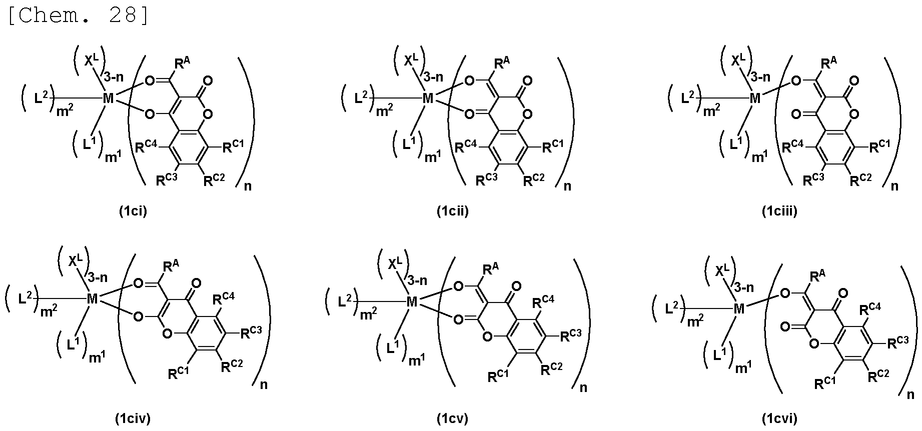

(In the formulae, R^{A}, R^{C1}, R^{C2}, R^{C3}, R^{C4}, L¹, L², m¹, m², n and X^{L} are synonymous with R^{A}, R^{C1}, R^{C2}, R^{C3}, R^{C4}, L¹, L², m¹, m², n and X^{L} in general formula (1) above. M represents a trivalent rare earth ion.)

Specific examples of the trivalent rare earth metal ion represented by M³⁺ include the rare earth metal ions of scandium, yttrium, lanthanum, cerium, praseodymium, neodymium, promethium, samarium, europium, gadolinium, terbium, dysprosium, holmium, erbium, thulium, ytterbium and lutetium.

The definitions of R^{A}, R^{C1}, R^{C2}, R^{C3} and R^{C4} in the coumarinato ligand (1cu) will now be described individually.

### (R^{A})

A C1 to C6 haloalkyl group represented by R^{A} may be any of a linear-chain, branched or cyclic haloalkyl group. Specific examples include groups such as a trifluoromethyl group, a difluoromethyl group, a perfluoroethyl group, a 2,2,2-trifluoroethyl group, a 1,1-difluoroethyl group, a 2,2-difluoroethyl group, a perfluoropropyl group, a 2,2,3,3,3-pentafluoropropyl group, a 2,2,3,3-tetrafluoropropyl group, a 3,3,3-trifluoropropyl group, a 1,1-difluoropropyl group, a perfluoropropan-2-yl group, a 2,2,2-trifluoro-1-(trifluoromethyl)ethyl group, a perfluorobutyl group, a 2,2,3,3,4,4,4-heptafluorobutyl group, a 3,3,4,4,4-pentafluorobutyl group, a 4,4,4-trifluorobutyl group, a 1,1,1,2,3,3,4,4,4-nonafluorobutan-2-yl group, a 1,1,1-trifluorobutan-2-yl group, a 4,4,4-trifluorobutan-2-yl group, a perfluoropentyl group, a 2,2,3,3,4,4,5,5,5-nonafluoropentyl group, a 3,3,4,4,5,5,5-heptafluoropentyl group, a 4,4,5,5,5-pentafluoropentyl group, a 5,5,5-trifluoropentyl group, a perfluoropentan-2-yl group, a 1,1,1,3,3,4,4,5,5,5-decafluoro-2-(trifluoromethyl)pentan-2-yl group, a 1,1,2,3,3,4,4,4-octafluoro-2-(trifluoromethyl)butyl group, a 1,1,2,2,3,4,4,4-octafluoro-3-(trifluoromethyl)butyl group, a 1,1,3,3,3-pentafluoro-2,2-bis(trifluoromethyl)propyl group, a 1,1,1,2,3,3,4,4,4-nonafluoro-1-(trifluoromethyl)butan-2-yl group, a perfluorocyclopentyl group, a perfluorohexyl group, a 1,1,1,2,3,3,4,4,5,5,6,6,6-decafluorohexan-2-yl group, a 1,1,2,3,3,4,4,5,5,5-decafluoro-2-(trifluoromethyl)pentyl group, a 1,1,2,2,3,4,4,5,5,5-decafluoro-3-(trifluoromethyl)pentyl group, a 1,1,2,2,3,3,4,5,5,5-decafluoro-4-(trifluoromethyl)pentyl group, a 1,1,1,2,2,3,3,4,4,5,5,5-dodecafluoro-1-(trifluoromethyl)pentan-2-yl group, a 1,1,1,3,3,4,4,5,5,5-decafluoro-2-(trifluoromethyl)pentan-2-yl group, a 1,1,1,2,2,4,4,5,5,5-decafluoro-3-(trifluoromethyl)pentan-2-yl group, a 1,1,3,3,4,4,4-heptafluoro-2,2-bis(trifluoromethyl)butyl group, a 1,2,2,3,4,4,4-heptafluoro-2,3-bis(trifluoromethyl)butyl group, a 1,1,2,2,4,4,4-heptafluoro-3,3-bis(trifluoromethyl)butyl group, a perfluorocyclohexyl group, a perfluorocyclopenthylmethyl group, a chloromethyl group, a bromomethyl group, an iodomethyl group, a 2-chloroethyl group and a 3-bromopropyl group. The present invention is not limited to these substituents listed by way of example. A trifluoromethyl group, 2,2,2-trifluoroethyl group, or perfluoropropyl group is preferred because of easy availability of raw materials, and a trifluoromethyl group is preferred because of inexpensiveness of raw materials.

Specific examples of C6 to C22 aryl groups represented by R^{A} include groups such as a phenyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a 2-ethylphenyl group, a 3-ethylphenyl group, a 4-ethylphenyl group, a 2,3-diethylphenyl group, a 2,4-diethylphenyl group, a 2,5-diethylphenyl group, a 2,6-diethylphenyl group, a 3,4-diethylphenyl group, a 3,5-diethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-cyclopropylphenyl group, a 3-cyclopropylphenyl group, a 4-cyclopropylphenyl group, a 2-butylphenyl group, a 3-butylphenyl group, a 4-butylphenyl group, a 2-(1-methylpropyl)phenyl group, a 3-(1-methylpropyl)phenyl group, a 4-(1-methylpropyl)phenyl group, a 2-(2-methylpropyl)phenyl group, a 3-(2-methylpropyl)phenyl group, a 4-(2-methylpropyl)phenyl group, a 2-cyclobutylphenyl group, a 3-cyclobutylphenyl group, a 4-cyclobutylphenyl group, a 1-naphthyl group, a 2-naphthyl group, a 2-biphenylyl group, a 3-biphenylyl group, a 4-biphenylyl group, a 9-anthryl group, a 2-phenanthrenyl group, a 3-phenanthrenyl group and a 9-phenanthrenyl group. The present invention is not limited to these substituents listed by way of example.

Specific examples of C3 to C20 heteroaryl groups represented by R^{A} include groups such as a triazinyl group, a furanyl group, a thienyl group, a pyrrolinyl group, a benzofuranyl group, a benzothienyl group, an indolyl group, a pyridyl group, a quinolinyl group, an isoquinolinyl group, a pyrazinyl group, a pyrimidyl group, a pyridazinyl group, a triazinyl group, a naphthyridinyl group, a cinnolinyl group, a phthalazinyl group, a quinoxalinyl group, a quinazolinyl group, a dibenzofuranyl group, a dibenzothienyl group, a carbazolyl group, a di-tert-butylcarbazolyl group, a carbolinyl group, a benzimidazolyl group, an imidazopyridyl group, a benzoxazolyl group, a benzothiazolyl group and a phenanthrolinyl group. The present invention is not limited to these substituents listed by way of example.

A C6 to C22 aryl or C3 to C20 heteroaryl group represented by R^{A} may be substituted with one or more substituents selected from the group consisting of C1 to C6 fluoroalkyl groups; C1 to C6 alkyloxy groups; C1 to C6 fluoroalkyloxy groups; C5 to C14 aryloxy groups; C1 to C6 monoalkylamino groups; C6 to C12 monoarylamino groups; C4 to C12 monoheteroarylamino groups; C2 to C12 dialkylamino groups; C10 to C24 diarylamino groups optionally substituted with one or more cyano groups, halogen atoms or C1 to C6 fluoroalkyl groups; C8 to C24 diheteroarylamino groups optionally substituted with one or more fluorine atoms or C1 to C6 fluoroalkyl groups; C3 to C20 heteroaryl groups; C2 to C13 acyl groups, a methylenedioxy group; an ethylenedioxy group; halogen atoms; a hydroxyl group; a nitro group and a cyano group (group T₂1).

A C1 to C6 fluoroalkyl group that belongs to group T₂1 may be any of linear-chain, branched or cyclic. Specific examples include groups such as a trifluoromethyl group, a difluoromethyl group, a perfluoroethyl group, a 2,2,2-trifluoroethyl group, a 1,1-difluoroethyl group, a 2,2-difluoroethyl group, a perfluoropropyl group, a 2,2,3,3,3-pentafluoropropyl group, a 2,2,3,3-tetrafluoropropyl group, a 3,3,3-trifluoropropyl group, a 1,1-difluoropropyl group, a perfluoropropan-2-yl group, a 1,1,1,3,3,3-hexafluoropropan-2-yl group, a perfluorobutyl group, a 2,2,3,3,4,4,4-heptafluorobutyl group, a 3,3,4,4,4-pentafluorobutyl group, a 4,4,4-trifluorobutyl group, a 1,1,1,2,3,3,4,4,4-nonafluorobutan-2-yl group, a 1,1,1-trifluorobutan-2-yl group, a 4,4,4-trifluorobutan-2-yl group, a perfluoropentyl group, a 2,2,3,3,4,4,5,5,5-nonafluoropentyl group, a 3,3,4,4,5,5,5-heptafluoropentyl group, a 4,4,5,5,5-pentafluoropentyl group, a 5,5,5-trifluoropentyl group, a perfluoropentan-2-yl group, a 1,1,1,3,3,4,4,5,5,5-decafluoro-2-(trifluoromethyl)pentan-2-yl group, a 1,1,2,3,3,4,4,4-octafluoro-2-(trifluoromethyl)butyl group, a 1,1,2,2,3,4,4,4-octafluoro-3-(trifluoromethyl)butyl group, a 1,1,3,3,3-pentafluoro-2,2-bis(trifluoromethyl)propyl group, a 1,1,1,2,3,3,4,4,4-nonafluoro-1-(trifluoromethyl)butan-2-yl group, a perfluorocyclopentyl group, a perfluorohexyl group, a 1,1,1,2,3,3,4,4,5,5,6,6,6-decafluorohexan-2-yl group, a 1,1,2,3,3,4,4,5,5,5-decafluoro-2-(trifluoromethyl)pentyl group, a 1,1,2,2,3,4,4,5,5,5-decafluoro-3-(trifluoromethyl)pentyl group, a 1,1,2,2,3,3,4,5,5,5-decafluoro-4-(trifluoromethyl)pentyl group, a 1,1,1,2,2,3,3,4,4,5,5,5-dodecafluoro-1-(trifluoromethyl)pentan-2-yl group, a 1,1,1,3,3,4,4,5,5,5-decafluoro-2-(trifluoromethyl)pentan-2-yl group, a 1,1,1,2,2,4,4,5,5,5-decafluoro-3-(trifluoromethyl)pentan-2-yl group, a 1,1,3,3,4,4,4-heptafluoro-2,2-bis(trifluoromethyl)butyl group, a 1,2,2,3,4,4,4-heptafluoro-2,3-bis(trifluoromethyl)butyl group, a 1,1,2,2,4,4,4-heptafluoro-3,3-bis(trifluoromethyl)butyl group, a perfluorocyclohexyl group and a perfluorocyclopenthylmethyl group. The present invention is not limited to these substituents listed by way of example.

A C1 to C6 alkyloxy group that belongs to group T₂1 may be any of linear-chain, branched or cyclic. Specific examples include groups such as a methoxy group, an ethoxy group, a propyloxy group, an isopropyloxy group, a butyloxy group, a 2-methylpropyloxy group, a 2,2-dimethylpropyloxy group, a 3-cyclopropylpropyloxy group, a cyclopropyloxy group, a 2-methylbutyloxy group, a 3-methylbutyloxy group, a tert-butyloxy group, a cyclobutyloxy group, a pentyloxy group, a 2-methylpentyloxy group, a 1-methylbutyloxy group, a 1,2-dimethylbutyloxy group, a 1-ethylpropyloxy group, a cyclopentyloxy group, a hexyloxy group and a cyclohexyloxy group. The present invention is not limited to these substituents listed by way of example.

A C1 to C6 fluoroalkyloxy group that belongs to group T₂1 may be any of linear-chain, branched or cyclic. Specific examples include groups such as a trifluoromethyloxy group, a difluoromethyloxy group, a perfluoroethyloxy group, a 2,2,2-trifluoroethyloxy group, a 1,1-difluoroethyloxy group, a 2,2-difluoroethyloxy group, a perfluoropropyloxy group, a 2,2,3,3,3-pentafluoropropyloxy group, a 2,2,3,3-tetrafluoropropyloxy group, a 3,3,3-trifluoropropyloxy group, a 1,1-difluoropropyloxy group, a perfluoropropan-2-yloxy group, a 1,1,1,3,3,3-hexafluoropropan-2-yloxy group, a perfluorobutyloxy group, a 2,2,3,3,4,4,4-heptafluorobutyloxy group, a 3,3,4,4,4-pentafluorobutyloxy group, a 4,4,4-trifluorobutyloxy group, a 1,1,1,2,3,3,4,4,4-nonafluorobutyl-2-yloxy group, a 1,1,1-trifluorobutan-2-yloxy group, a 4,4,4-trifluorobutan-2-yloxy group, a perfluoropentyloxy group, a 2,2,3,3,4,4,5,5,5-nonafluoropentyloxy group, a 3,3,4,4,5,5,5-heptafluoropentyloxy group, a 4,4,5,5,5-pentafluoropentyloxy group, a 5,5,5-trifluoropentyloxy group, a perfluoropentan-2-yloxy group, a 1,1,1,3,3,4,4,5,5,5-decafluoro-2-(trifluoromethyl)pentan-2-yloxy group, a 1,1,2,3,3,4,4,4-octafluoro-2-(trifluoromethyl)butyloxy group, a 1,1,2,2,3,4,4,4-octafluoro-3-(trifluoromethyl)butyloxy group, a 1,1,3,3,3-pentafluoro-2,2-bis(trifluoromethyl)propyloxy group, a 1,1,1,2,3,3,4,4,4-nonafluoro-1-(trifluoromethyl)butan-2-yloxy group, a perfluorocyclopentyloxy group, a perfluorohexyloxy group, a 1,1,1,2,3,3,4,4,5,5,6,6,6-decafluorohexan-2-yloxy group, a 1,1,2,3,3,4,4,5,5,5-decafluoro-2-(trifluoromethyl)pentyloxy group, a 1,1,2,2,3,4,4,5,5,5-decafluoro-3-(trifluoromethyl)pentyloxy group, a 1,1,2,2,3,3,4,5,5,5-decafluoro-4-(trifluoromethyl)pentyloxy group, a 1,1,1,2,2,3,3,4,4,5,5,5-dodecafluoro-1-(trifluoromethyl)pentan-2-yloxy group, a 1,1,1,3,3,4,4,5,5,5-decafluoro-2-(trifluoromethyl)pentan-2-yloxy group, a 1,1,1,2,2,4,4,5,5,5-decafluoro-3-(trifluoromethyl)pentan-2-yloxy group, a 1,1,3,3,4,4,4-heptafluoro-2,2-bis(trifluoromethyl)butyloxy group, a 1,2,2,3,4,4,4-heptafluoro-2,3-bis(trifluoromethyl)butyloxy group, a 1,1,2,2,4,4,4-heptafluoro-3,3-bis(trifluoromethyl)butyloxy group, a perfluorocyclohexyloxy group and a perfluorocyclopentylmethyloxy group. The present invention is not limited to these substituents listed by way of example.

Specific examples of C5 to C14 aryloxy groups that belong to group T₂1 include groups such as an indenyloxy group, a phenyloxy group, a 2-methylphenyloxy group, a 3-methylphenyloxy group, a 4-methylphenyloxy group, a 2,3-dimethylphenyloxy group, a 2,4-dimethylphenyloxy group, a 2,5-dimethylphenyloxy group, a 2,6-dimethylphenyloxy group, a 3,4-dimethylphenyloxy group, a 3,5-dimethylphenyloxy group, a 2,3,4-trimethylphenyloxy group, a 2,3,5-trimethylphenyloxy group, a 2,3,6-trimethylphenyloxy group, a 2,4,5-trimethylphenyloxy group, a 2,4,6-trimethylphenyloxy group, a 3,4,5-trimethylphenyloxy group, a 2,3,4,5-tetramethylphenyloxy group, a 2,3,4,6-tetramethylphenyloxy group, a 2,3,5,6-tetramethylphenyloxy group, a 2-ethylphenyloxy group, a 3-ethylphenyloxy group, a 4-ethylphenyloxy group, a 2,3-diethylphenyloxy group, a 2,4-diethylphenyloxy group, a 2,5-diethylphenyloxy group, a 2,6-diethylphenyloxy group, a 3,4-diethylphenyloxy group, a 3,5-diethylphenyloxy group, a 2-propylphenyloxy group, a 3-propylphenyloxy group, a 4-propylphenyloxy group, a 2-isopropylphenyloxy group, a 3-isopropylphenyloxy group, a 4-isopropylphenyloxy group, a 2-cyclopropylphenyloxy group, a 3-cyclopropylphenyloxy group, a 4-cyclopropylphenyloxy group, a 2-butylphenyloxy group, a 3-butylphenyloxy group, a 4-butylphenyloxy group, a 2-(1-methylpropyl)phenyloxy group, a 3-(1-methylpropyl)phenyloxy group, a 4-(1-methylpropyl)phenyloxy group, a 2-(2-methylpropyl)phenyloxy group, a 3-(2-methylpropyl)phenyloxy group, a 4-(2-methylpropyl)phenyloxy group, a 2-cyclobutylphenyloxy group, a 3-cyclobutylphenyloxy group, a 4-cyclobutylphenyloxy group, a (biphenyl-2-yl)oxy group, a (biphenyl-3-yl)oxy group, a (biphenyl-4-yl)oxy group, a (phenanthren-9-yl)oxy group and an (anthracen-9-yl)oxy group. The present invention is not limited to these substituents listed by way of example.

Specific examples of C1 to C6 monoalkylamino groups that belong to group T₂1 include groups such as a methylamino group, an ethylamino group, a propylamino group, an isopropylamino group, a butylamino group, a 2-methylpropylamino group, a 2,2-dimethylpropylamino group, a 3-cyclopropylamino group, a cyclopropylamino group, a tert-butylamino group, a cyclobutylamino group, a pentylamino group, a 2-methylpentylamino group and a hexylamino group. The present invention is not limited to these substituents listed by way of example.

Specific examples of C6 to C12 monoarylamino groups that belong to group T₂1 include groups such as an indenylamino group, a phenylamino group, a 2-methylphenylamino group, a 3-methylphenylamino group, a 4-methylphenylamino group, a 2,3-dimethylphenylamino group, a 2,4-dimethylphenylamino group, a 2,5-dimethylphenylamino group, a 2,6-dimethylphenylamino group, a 3,4-dimethylphenylamino group, a 3,5-dimethylphenylamino group, a 2,3,4-trimethylphenylamino group, a 2,3,5-trimethylphenylamino group, a 2,3,6-trimethylphenylamino group, a 2,4,5-trimethylphenylamino group, a 2,4,6-trimethylphenylamino group, a 3,4,5-trimethylphenylamino group, a 2,3,4,5-tetramethylphenylamino group, a 2,3,4,6-tetramethylphenylamino group, a 2,3,5,6-tetramethylphenylamino group, a 2-ethylphenylamino group, a 3-ethylphenylamino group, a 4-ethylphenylamino group, a 2,3-diethylphenylamino group, a 2,4-diethylphenylamino group, a 2,5-diethylphenylamino group, a 2,6-diethylphenylamino group, a 3,4-diethylphenylamino group, a 3,5-diethylphenylamino group, a 2-propylphenylamino group, a 3-propylphenylamino group, a 4-propylphenylamino group, a 2-isopropylphenylamino group, a 3-isopropylphenylamino group, a 4-isopropylphenylamino group, a 2-cyclopropylphenylamino group, a 3-cyclopropylphenylamino group, a 4-cyclopropylphenylamino group, a 2-butylphenylamino group, a 3-butylphenylamino group, a 4-butylphenylamino group, a 2-(1-methylpropyl)phenylamino group, a 3-(1-methylpropyl)phenylamino group, a 4-(1-methylpropyl)phenylamino group, a 2-(2-methylpropyl)phenylamino group, a 3-(2-methylpropyl)phenylamino group, a 4-(2-methylpropyl)phenylamino group, a 2-cyclobutylphenylamino group, a 3-cyclobutylphenylamino group, a 4-cyclobutylphenylamino group, a 1-naphthylamino group and a 2-naphthylamino group. The present invention is not limited to these substituents listed by way of example.

Specific examples of C4 to C12 monoheteroarylamino groups that belong to group T₂1 include groups such as a 2-pyrrolylamino group, a 2-thienylamino group, a 3-thienylamino group, a 2-furylamino group, a 3-furylamino group, a 2-(1-methylindolyl)amino group, a 2-benzothienyl group, a 3-benzothienyl group, a 2-benzofuranyl group and a 3-benzofuranyl group. The present invention is not limited to these substituents listed by way of example.

Specific examples of C2 to C12 dialkylamino groups that belong to group T₂1 include groups such as a dimethylamino group, a diethylamino group, a dipropylamino group, a diisopropylamino group, a dibutylamino group, a bis(2-methylpropyl)amino group, a bis(2,2-dimethylpropyl)amino group, a bis(3-cyclopropyl)amino group, a dicyclopropylamino group, a di(tert-butyl)amino group, a dicyclobutylamino group, a dipentylamino group, a bis(2-methylpentyl)amino group, a di(1-methylbutyl)amino group, a bis(1,2-dimethylbutyl)amino group, a di(1-ethylpropyl)amino group, a dicyclopentylamino group, a dihexylamino group, a dicyclohexylamino group, an N-ethyl-N-methylamino group, an N-methyl-N-propyl group, an N-cyclopropyl-N-methylamino group, an N-butyl-N-methylamino group, an N-cyclobutyl-N-methylamino group, an N-methyl-N-pentyl group, an N-cyclopentyl-N-methylamino group, an N-hexyl-N-methylamino group, an N-cyclohexyl-N-methylamino group, an N-cycloheptyl-N-methylamino group, an N-methyl-N-octylamino group, an N-methyl-N-nonylamino group, an N-decyl-N-methylamino group or an N-methyl-N-undecylamino group and an azetidyl group, a pyrrolidinyl group, a piperidino group, an azepanyl group, a morpholino group and a 2-morpholinyl group. The present invention is not limited to these substituents listed by way of example.

Specific examples of C10 to C24 diarylamino groups that belong to group T₂1 include groups such as a diindenylamino group, an N-indenyl-N-phenylamino group, a diphenylamino group, a bis(2-methylphenyl)amino group, a bis(3-methylphenyl)amino group, a bis(4-methylphenyl)amino group, a bis(2,3-dimethylphenyl)amino group, a bis(2,4-dimethylphenyl)amino group, a bis(2,5-dimethylphenyl)amino group, a bis(2,6-dimethylphenyl)amino group, a bis(3,4-dimethylphenyl)amino group, a bis(3,5-dimethylphenyl)amino group, a bis(2,3,4-trimethylphenyl)amino group, a bis(2,3,5-trimethylphenyl)amino group, a bis(2,3,6-trimethylphenyl)amino group, a bis(2,4,5-trimethylphenyl)amino group, a bis(2,4,6-trimethylphenyl)amino group, a bis(3,4,5-trimethylphenyl)amino group, a bis(2,3,4,5-tetramethylphenyl)amino group, a bis(2,3,4,6-tetramethylphenyl)amino group, a bis(2,3,5,6-tetramethylphenyl)amino group, a bis(2-ethylphenyl)amino group, a bis(3-ethylphenyl)amino group, a bis(4-ethylphenyl)amino group, a bis(2,3-diethylphenyl)amino group, a bis(2,4-diethylphenyl)amino group, a bis(2,5-diethylphenyl)amino group, a bis(2,6-diethylphenyl)amino group, a bis(3,4-diethylphenyl)amino group, a bis(3,5-diethylphenyl)amino group, a bis(2-propylphenyl)amino group, a bis(3-propylphenyl)amino group, a bis(4-propylphenyl)amino group, a bis(2-isopropylphenyl)amino group, a bis(3-isopropylphenyl)amino group, a bis(4-isopropylphenyl)amino group, a bis(2-cyclopropylphenyl)amino group, a bis(3-cyclopropylphenyl)amino group, a bis(4-cyclopropylphenyl)amino group, a bis(2-butylphenyl)amino group, a bis(3-butylphenyl)amino group, a bis(4-butylphenyl)amino group, a bis[2-(1-methylpropyl)phenyl]amino group, a bis[3-(1-methylpropyl)phenyl]amino group, a bis[4-(1-methylpropyl)phenyl]amino group, a bis[2-(2-methylpropyl)phenyl]amino group, a bis[3-(2-methylpropyl)phenyl]amino group, a bis[4-(2-methylpropyl)phenyl]amino group, a bis(2-cyclobutylphenyl)amino group, a bis(3-cyclobutylphenyl)amino group, a bis(4-cyclobutylphenyl)amino group, a di(1-naphthyl)amino group, a di(2-naphthyl)amino group, an N-(2-methylphenyl)-N-phenylamino group, an N-(3-methylphenyl)-N-phenylamino group, an N-(4-methylphenyl)-N-phenylamino group, an N-(2,3-dimethylphenyl)-N-phenylamino group, an N-(2,4-dimethylphenyl)-N-phenylamino group, an N-(2,5-dimethylphenyl)-N-phenylamino group, an N-(2,6-dimethylphenyl)-N-phenylamino group, an N-(3,4-dimethylphenyl)-N-phenylamino group, an N-(3,5-dimethylphenyl)-N-phenylamino group, an N-(2,3,4-trimethylphenyl)-N-phenylamino group, an N-(2,3,5-trimethylphenyl)-N-phenylamino group, an N-(2,3,6-trimethylphenyl)-N-phenylamino group, an N-(2,4,5-trimethylphenyl)-N-phenylamino group, an N-(2,4,6-trimethylphenyl)-N-phenylamino group, an N-(3,4,5-trimethylphenyl)-N-phenylamino group, an N-(1-naphthyl)-N-phenylamino group, an N-(2-naphthyl)-N-phenylamino group, a carbazoyl group, an iminostilbenyl group, a 9(10H)-acridonyl group, a 10,11-dihydro-5H-dibenz[b.f.]azepinyl group and a 10,11-dihydro-10-oxo-5H-dibenz[b.f.]azepinyl group. The present invention is not limited to these substituents listed by way of example.

Specific examples of C8 to C24 diheteroarylamino groups that belong to group T₂1 include groups such as a bis(2-pyrrolyl)amino group, a bis(1-methylpyrrol-2-yl)amino group, a bis(1-ethylpyrrol-2-yl)amino group, a bis(1-phenylpyrrol-2-yl)amino group, a bis(2-thienyl)amino group, a bis(3-thienyl)amino group, a bis(2-furyl)amino group, a bis(3-furyl)amino group, a bis(2-benzothienyl)amino group, a bis(3-benzothienyl)amino group, a bis(2-benzofuranyl)amino group, a bis(3-benzofuranyl)amino group, a bis(1-methylindol-2-yl)amino group, a bis(1-methylindol-2-yl)amino group, a bis(3-benzofuranyl)amino group, a phenothiazinyl group, a phenoxazinyl group, a 5,10-dihydrodihydrophenazinyl group and a 10-methyl-5,10-dihydrodihydrophenazinyl group. The present invention is not limited to these substituents listed by way of example.

A C10 to C24 diarylamino group that belongs to group T₂1, furthermore, may be substituted with one or more fluorine atoms or C1 to C6 fluoroalkyl groups. Specific examples include groups such as a bis(2-fluorophenyl)amino group, a bis(4-fluorophenyl)amino group, a bis(3,5-difluorophenyl)amino group, a bis(3,4-difluorophenyl)amino group, a bis(3,4,5-trifluorophenyl)amino group, a bis[4-(1,1,1,2,2,3,3-heptafluoropropyl)phenyl]amino group, a bis(perfluorophenyl)amino group, a bis(4-trifluoromethylphenyl)amino group, a bis[3,5-di(trifluoromethyl)phenyl]amino group, a 3-fluorocarbazoyl group, a 3,6-difluorocarbazoyl group, a 3-(trifluoromethyl)carbazoyl group, a 3,6-bis(trifluoromethyl)carbazoyl group and a 2-(trifluoromethyl)phenothiazinyl group. The present invention is not limited to these substituents listed by way of example.

A C8 to C24 diheteroarylamino group that belongs to group T₂1, furthermore, may be substituted with one or more fluorine atoms or C1 to C6 fluoroalkyl groups. Specific examples include groups such as a bis(5-fluoropyrrol-2-yl)amino group, a bis(5-fluoro-1-methylpyrrol-2-yl)amino group, a bis(5-fluoro-1-ethylpyrrol-2-yl)amino group, a bis(5-fluoro-1-phenylpyrrol-2-yl)amino group, a bis(5-fluoro-2-thienyl)amino group, a bis(5-trifluoromethyl-2-thienyl)amino group, a bis(5-fluoro-3-thienyl)amino group, a bis(5-fluoro-2-furyl)amino group, a bis(5-trifluoromethyl-2-furyl)amino group, a bis(5-fluoro-3-furyl)amino group, a bis(5-fluoro-2-benzothienyl)amino group, a bis(5-fluoro-3-benzothienyl)amino group, a bis(5-fluoro-2-benzofuranyl)amino group, a bis(5-fluoro-3-benzofuranyl)amino group, a bis(1-methylindol-2-yl)amino group, a bis(1-methylindol-3-yl)amino group, a bis(5-fluoro-3-benzofuranyl)amino group, a 2-fluorophenothiazinyl group, a 2-trifluoromethylphenothiazinyl group and a 2-trifluoromethylphenoxazinyl group. The present invention is not limited to these substituents listed by way of example.

Specific examples of C3 to C20 heteroaryl groups that belong to group T₂1 include groups such as a triazinyl group, a furanyl group, a thienyl group, a pyrrolinyl group, a benzofuranyl group, a benzothienyl group, an indolyl group, a pyridyl group, a quinolinyl group, an isoquinolinyl group, a pyrazinyl group, a pyrimidyl group, a pyridazinyl group, a triazinyl group, a naphthyridinyl group, a cinnolinyl group, a phthalazinyl group, a quinoxalinyl group, a quinazolinyl group, a dibenzofuranyl group, a dibenzothienyl group, a carbazolyl group, a di-tert-butylcarbazolyl group, a carbolinyl group, a benzimidazolyl group, an imidazopyridyl group, a benzoxazolyl group, a benzothiazolyl group and a phenanthrolinyl group. The present invention is not limited to these substituents listed by way of example.

A C2 to C13 acyl group that belongs to group T₂1 may be any of an aliphatic or aromatic acyl group. Specific examples include aliphatic acyl groups such as an acetyl group, an ethylcarbonyl group, a 1-propylcarbonyl group, an isopropylcarbonyl group, a 1-butylcarbonyl group, a 2-butylcarbonyl group, a tert-butylcarbonyl group, a 1-pentylcarbonyl group, a 2-pentylcarbonyl group, a 3-pentylcarbonyl group and a 1-hexylcarbonyl group and aromatic acyl groups such as a phenylcarbonyl group (benzoyl group), a 1-naphthylcarbonyl group, a 2-naphthylcarbonyl group, a (biphenyl-2-yl)carbonyl group, a (biphenyl-4-yl)carbonyl group, a (4-methylphenyl)carbonyl group and a (2-methylphenyl)carbonyl group. The present invention is not limited to these substituents listed by way of example.

Examples of halogen atoms that belong to group T₂1 include a fluorine atom, a chlorine atom, a bromine atom and an iodine atom.

Specific examples of C6 to C22 aryl groups at R^{A} optionally substituted with one or more substituents selected from group T₂1, therefore, include groups such as a phenyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a 2-ethylphenyl group, a 3-ethylphenyl group, a 4-ethylphenyl group, a 2,3-diethylphenyl group, a 2,4-diethylphenyl group, a 2,5-diethylphenyl group, a 2,6-diethylphenyl group, a 3,4-diethylphenyl group, a 3,5-diethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-cyclopropylphenyl group, a 3-cyclopropylphenyl group, a 4-cyclopropylphenyl group, a 2-butylphenyl group, a 3-butylphenyl group, a 4-butylphenyl group, a 2-(1-methylpropyl)phenyl group, a 3-(1-methylpropyl)phenyl group, a 4-(1-methylpropyl)phenyl group, a 2-(2-methylpropyl)phenyl group, a 3-(2-methylpropyl)phenyl group, a 4-(2-methylpropyl)phenyl group, a 2-cyclobutylphenyl group, a 3-cyclobutylphenyl group, a 4-cyclobutylphenyl group, a 1-naphthyl group, a 2-naphthyl group, a 2-biphenylyl group, a 3-biphenylyl group, a 4-biphenylyl group, a 9-anthryl group, a 2-phenanthrenyl group, a 3-phenanthrenyl group, a 9-phenanthrenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-(trifluoromethyl)phenyl group, a 3-(trifluoromethyl)phenyl group, a 4-(trifluoromethyl)phenyl group, a 2-(perfluoropropyl)phenyl group, a 3-(perfluoropropyl)phenyl group, a 4-(perfluoropropyl)phenyl group, a 2-acetylphenyl group, a 3-acetylphenyl group, a 4-acetylphenyl group, a 2-pivaloylphenyl group, a 3-pivaloylphenyl group, a 4-pivaloylphenyl group, a 2-benzoylphenyl group, a 3-benzoylphenyl group, a 4-benzoylphenyl group, a 2-naphthoylphenyl group, a 3-naphthoylphenyl group, a 4-naphthoylphenyl group, a 4-methoxy-1-naphthyl group, a 6-methoxy-2-naphthyl group, a 4'-methoxybiphenyl-2-yl group, a 4'-methoxybiphenyl-3-yl group, a 4'-methoxybiphenyl-4-yl group, a 10-methoxy-9-anthryl group, a 2-fluorophenyl group, a 3-fluorophenyl group, a 4-fluorophenyl group, a 3,4-difluorophenyl group, a 4-(trifluoromethyloxy)phenyl group, a 4-phenyloxyphenyl group, a 4-(phenylamino)phenyl group, a 4-(diphenylamino)phenyl group, a 4-cyanophenyl group, a 4-nitrophenyl group, a 4-hydroxyphenyl group and a deuterated phenyl group. The present invention is not limited to these substituents listed by way of example. A phenyl group, 2-methylphenyl group, 3-methylphenyl group, 4-methylphenyl group, 1-naphthyl group, 2-naphthyl group, 2-biphenylyl group, 3-biphenylyl group, 4-biphenylyl group, 2-methoxyphenyl group, 3-methoxyphenyl group, 4-methoxyphenyl group, 2-(trifluoromethyl)phenyl group, 3-(trifluoromethyl)phenyl group, 4-(trifluoromethyl)phenyl group, 2-fluorophenyl group, 3-fluorophenyl group, 4-fluorophenyl group, 3,4-difluorophenyl group, 2-(diphenylamino)phenyl group, 3-(diphenylamino)phenyl group, 4-(diphenylamino)phenyl group, deuterated phenyl group, perfluorophenyl group, 4-cyanophenyl group, 4-nitrophenyl group, 4-{bis[4-(trifluoromethyl)phenyl]amino}phenyl group, 4-{bis[4-bis(4-cyanophenyl)amino]phenyl group, 4-{bis[4-bis(3,5-difluorophenyl)amino]phenyl group or 4-[bis(4-fluorophenyl)amino]phenyl group is preferred because it gives the complex optical characteristics suitable for use as an optical functional material. A phenyl group, 4-(trifluoromethyl)phenyl group or 4-(diphenylamino)phenyl group is more preferred because of the ease of synthesis.

Specific examples of C3 to C20 heteroaryl groups at R^{A} optionally substituted with one or more substituents selected from group T₂1, furthermore, include groups such as a 1,3,5-triazin-2-yl group, a 2-furanyl group, a 3-furanyl group, a 2-thienyl group, a 3-thienyl group, a 1-pyrrolyl group, a 2-pyrrolyl group, a 3-pyrrolyl group, a 1-methylpyrrol-2-yl group, a 1-methylpyrrol-3-yl group, a 1-phenylpyrrol-2-yl group, a 1-phenylpyrrol-3-yl group, a 2-benzofuranyl group, a 3-benzofuranyl group, a 2-benzothienyl group, a 3-benzothienyl group, a 1-indolyl group, a 2-indolyl group, a 3-indolyl group, a 1-methylindol-2-yl group, a 1-methylindol-3-yl group, a 1-phenylindol-2-yl group, a 1-phenylindol-3-yl group, a 9-methylcarbazol-2-yl group, a 9-methylcarbazol-3-yl group, a 9-ethylcarbazol-2-yl group, a 9-ethylcarbazol-3-yl group, a 9-phenylcarbazol-2-yl group, a 9-phenylcarbazol-3-yl group, a dibenzofuran-2-yl group, a dibenzofuran-3-yl group, a dibenzofuran-4-yl group, a dibenzothiophen-2-yl group, a dibenzothiophen-3-yl group, a dibenzothiophen-4-yl group, a 5-(trifluoromethyl)furan-2-yl group, a 2-(trifluoromethyl)furan-3-yl group, a 5-(trifluoromethyl)thiophen-2-yl group, a 2-(trifluoromethyl)-3-thienyl group, a 5-(trifluoromethyl)benzofuran-2-yl group, a 6-(trifluoromethyl)benzofuran-2-yl group, a 5-(trifluoromethyl)benzothiophen-2-yl group, a 6-(trifluoromethyl)benzothiophen-2-yl group, a 3,6-di-tert-butyl-9H-carbazol-9-yl group, a 9-methyl-5-(trifluoromethyl)carbazol-2-yl group, a 9-methyl-6-(trifluoromethyl)carbazol-2-yl group, a 9-methyl-5-(trifluoromethyl)carbazol-3-yl group, a 9-methyl-6-(trifluoromethyl)carbazol-3-yl group, a 9-phenyl-5-(trifluoromethyl)carbazol-2-yl group, a 9-phenyl-5-(trifluoromethyl)carbazol-3-yl group, a 9-phenyl-6-(trifluoromethyl)carbazol-2-yl group, a 9-phenyl-6-(trifluoromethyl)carbazol-3-yl group, a 6-(trifluoromethyl)dibenzofuran-2-yl group, a 7-(trifluoromethyl)dibenzofuran-2-yl group, a 6-(trifluoromethyl)dibenzofuran-3-yl group, a 7-(trifluoromethyl)dibenzofuran-3-yl group, a 6-(trifluoromethyl)dibenzofuran-4-yl group, a 7-(trifluoromethyl)dibenzofuran-4-yl group, a 6-(trifluoromethyl)dibenzothiophen-2-yl group, a 7-(trifluoromethyl)dibenzothiophen-2-yl group, a 6-(trifluoromethyl)dibenzothiophen-3-yl group, a 7-(trifluoromethyl)dibenzothiophen-3-yl group, a 6-(trifluoromethyl)dibenzothiophen-4-yl group and a 7-(trifluoromethyl)dibenzothiophen-4-yl group. The present invention is not limited to these substituents listed by way of example. A 2-furanyl group, 2-thienyl group, 2-benzofuranyl group, 2-benzothienyl group, 9-methylcarbazol-3-yl group, 9-ethylcarbazol-3-yl group, 9-phenylcarbazol-3-yl group, dibenzofuran-2-yl group, dibenzofuran-3-yl group, dibenzofuran-4-yl group, dibenzothiophen-2-yl group, dibenzothiophen-3-yl group, dibenzothiophen-4-yl group, 5-(trifluoromethyl)furan-2-yl group or 5-(trifluoromethyl)thiophen-2-yl group is preferred because of the ease of synthesis, and a 2-furanyl group, 2-thienyl group, 2-benzofuranyl group or 2-benzothienyl group is preferred because of inexpensiveness of raw materials.

### (R^{C1}, R^{C2}, R^{C3} and R^{C4})

Examples of halogen atoms represented by R^{C1}, R^{C2}, R^{C3} and R^{C4} include the substituents listed as examples of halogen atoms that belong to group T₂1.

A C1 to C10 alkyl group represented by R^{C1}, R^{C2}, R^{C3} and R^{C4} may be any of a linear-chain, branched or cyclic alkyl group. Specific examples include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a 5-dimethylcyclopentyl group, a 3-ethylcyclopentyl group, a hexyl group, a cyclohexyl group, a 4-ethylcyclohexyl group, a 4-propylcyclohexyl group, a 4,4-dimethylcyclohexyl group, a 2,6-dimethylcyclohexyl group, a 3,5-dimethylcyclohexyl group, a cycloheptyl group, a cyclooctyl group, a cyclononyl group, a cyclodecanyl group, a bicyclo[2.2.1]heptan-2-yl group, a bicyclo[2.2.2]octan-2-yl group, an adamantan-2-yl group, a bicyclo[2.2.1]heptan-2-yl group and an adamantan-1-yl group. The present invention is not limited to these substituents listed by way of example.

A C2 to C4 alkenyl group represented by R^{C1}, R^{C2}, R^{C3} and R^{C4} optionally substituted with one or more C1 to C6 alkyloxy groups may be any of a chain or branched alkenyl group. Specific examples include groups such as a vinyl group, a 1-propenyl group, an allyl group, a 2-propenyl group, a 1-butenyl group, a 2-butenyl group, a 2-methylallyl group, a 1-methoxyvinyl group, a 2-methoxyvinyl group, a 1-ethoxyvinyl group, a 2-ethoxyvinyl group, a 1-isopropyloxyvinyl group, a 2-isopropyloxyvinyl group, a 1-(tert-butyl)oxyvinyl group, a 2-(tert-butyl)oxyvinyl group, a 1-hexyloxyvinyl group and a 2-hexyloxyvinyl group. The present invention is not limited to these substituents listed by way of example.

Examples of C1 to C6 haloalkyl groups represented by R^{C1}, R^{C2}, R^{C3} and R^{C4} include the substituents listed as examples of C1 to C6 haloalkyl groups represented by R^{A}.

Examples of C6 to C22 aryl groups represented by R^{C1}, R^{C2}, R^{C3} and R^{C4} include the substituents listed as examples of C6 to C22 aryl groups represented by R^{A}.

Examples of C3 to C20 heteroaryl groups represented by R^{C1}, R^{C2}, R^{C3} and R^{C4} include the substituents listed as examples of C3 to C20 heteroaryl groups represented by R^{A}.

Specific examples of C7 to C14 aralkyl groups represented by R^{C1}, R^{C2}, R^{C3} and R^{C4} optionally substituted with one or more halogen atoms include groups such as a benzyl group, a phenethyl group, a (4-methylphenyl)methyl group, a (3-methylphenyl)methyl group, a (2-methylphenyl)methyl group, a (4-fluorophenyl)methyl group, a (3-fluorophenyl)methyl group, a (2-fluorophenyl)methyl group, a (4-bromophenyl)methyl group, a (4-chlorophenyl)methyl group, a (4-iodophenyl)methyl group, a (4-trifluoromethylphenyl)methyl group, a 1-naphthylmethyl group, a 2-naphthylmethyl group, a 1-naphthylethyl group, a 2-naphthylethyl group, a (6-fluoronaphthalen-2-yl)methyl, a (4-fluoronaphthalen-1-yl)methyl and a (6-trifluoromethylnaphthalen-2-yl)methyl group. The present invention is not limited to these substituents listed by way of example.

A C6 to C22 aryl or C3 to C20 heteroaryl group represented by R^{C1}, R^{C2}, R^{C3} and R^{C4}, furthermore, may be substituted with one or more substituents selected from the group consisting of halogen atoms, C2 to C4 alkenyl groups, C1 to C6 alkyloxy groups, C1 to C6 fluoroalkyl groups, C2 to C13 acyl groups, a cyano group and a nitro group (group T₂2).

Examples of halogen atoms that belong to group T₂2 include the substituents listed as examples of halogen atoms that belong to group T₂1.

Examples of C1 to C6 alkyloxy groups that belong to group T₂2 include the substituents listed as examples of C1 to C6 alkyloxy groups that belong to group T₂1.

Examples of C1 to C6 fluoroalkyl groups that belong to group T₂2 include the substituents listed as examples of C1 to C6 fluoroalkyl groups that belong to group T₂1.

Examples of C2 to C13 acyl groups that belong to group T₂2 include the substituents listed as examples of C2 to C13 acyl groups that belong to group T₂1.

Examples of C6 to C22 aryl groups at R^{C1}, R^{C2}, R^{C3} and R^{C4} optionally substituted with one or more substituents selected from group T₂2, therefore, include the substituents listed as examples for a C6 to C22 aryl group at R^{A} optionally substituted with one or more substituents selected from group T₂1.

Examples of C3 to C20 heteroaryl groups at R^{C1}, R^{C2}, R^{C3} and R^{C4} optionally substituted with one or more substituents selected from group T₂2, furthermore, include the substituents listed as examples for a C3 to C20 heteroaryl group at R^{A} optionally substituted with one or more substituents selected from group T₂1.

Specific examples of C1 to C6 alkyl groups represented by R^{BA1} and R^{BA2} in general formula (BA1), R^{BO1} in general formula (BO1) and R^{BS1} in general formula (BS1) include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a hexyl group and a cyclohexyl group. The present invention is not limited to these substituents listed by way of example.

Examples of C6 to C22 aryl groups represented by R^{BA1} and R^{BA2} in general formula (BA1), R^{BO1} in general formula (BO1) and R^{BS1} in general formula (BS1) include the substituents listed as examples of C6 to C22 aryl groups represented by R^{A}.

Examples of C3 to C20 heteroaryl groups represented by R^{BA1} and R^{BA2} in general formula (BA1), R^{BO1} in general formula (BO1) and R^{BS1} in general formula (BS1) include the substituents listed as examples of C3 to C20 heteroaryl groups represented by R^{A}.

A C1 to C6 alkyl, C6 to C22 aryl or C3 to C20 heteroaryl group represented by R^{BA1} and R^{BA2} in general formula (BA1), furthermore, may be substituted with one or more substituents selected from the group consisting of halogen atoms, C1 to C6 alkyloxy groups, C1 to C6 fluoroalkyl groups, C2 to C13 acyl groups, a hydroxyl group, a cyano group and a nitro group (group T₂3).

A C1 to C6 alkyl, C6 to C22 aryl or C3 to C20 heteroaryl group represented by R^{BO1} in general formula (BO1), moreover, may be substituted with one or more substituents selected from group T₂3.

A C1 to C6 alkyl, C6 to C22 aryl or C3 to C20 heteroaryl group represented by R^{BS1} in general formula (BS1), moreover, may be substituted with one or more substituents selected from group T₂3.

Examples of halogen atoms that belong to group T₂3 include the substituents listed as examples of halogen atoms that belong to group T₂1.

Examples of C1 to C6 alkyloxy groups that belong to group T₂3 include the substituents listed as examples of C1 to C6 alkyloxy groups that belong to group T₂1.

Examples of C1 to C6 fluoroalkyl groups that belong to group T₂3 include the substituents listed as examples of C1 to C6 fluoroalkyl groups that belong to group T₂1.

Examples of C2 to C13 acyl groups that belong to group T₂3 include the substituents listed as examples of C2 to C13 acyl groups that belong to group T₂1.

Specific examples of C1 to C6 alkyl groups at R^{BA1} and R^{BA2} optionally substituted with one or more substituents selected from group T₂3, C1 to C6 alkyl groups at R^{BO1} optionally substituted with one or more substituents selected from group T₂3 and C1 to C6 alkyl groups at R^{BS1} optionally substituted with one or more substituents selected from group T₂3, therefore, include groups such as a 2,2,2-trifluoroethyl group, a 2-methoxyethyl group, a 3-methoxypropyl group, a 2-hydroxyethyl group, a 2-acetylethyl group, a 3-acetylpropyl group, a 2-benzoylethyl group, a 3-benzoylpropyl group and a 3-hydroxypropyl group. The present invention is not limited to these substituents listed by way of example.

Examples of C6 to C22 aryl groups at R^{BA1} and R^{BA2} optionally substituted with one or more substituents selected from group T₂3, C6 to C22 aryl groups at R^{BO1} optionally substituted with one or more substituents selected from group T₂3 and C6 to C22 aryl groups at R^{BS1} optionally substituted with one or more substituents selected from group T₂3, furthermore, include the substituents listed as examples for a C6 to C22 aryl group at R^{A} optionally substituted with one or more substituents selected from group T₂1.

Examples of C3 to C20 heteroaryl groups at R^{BA1} and R^{BA2} optionally substituted with one or more substituents selected from group T₂3, C3 to C20 heteroaryl groups at R^{BO1} optionally substituted with one or more substituents selected from group T₂3 and C3 to C20 heteroaryl groups at R^{BS1} optionally substituted with one or more substituents selected from group T₂3, moreover, include the substituents listed as examples for a C3 to C20 heteroaryl group at R^{A} optionally substituted with one or more substituents selected from group T₂1.

In addition, R^{BA1} and R^{BA2} may form a five-membered, six-membered or seven-membered ring including the nitrogen atom to which they are bound. Specific examples include groups such as an azetidyl group, a pyrrolidinyl group, a piperidino group, an azepanyl group, a morpholino group, a 4-methylpiperazinyl group, a carbazoyl group, a phenoxazinyl group, a phenothiazinyl group and a 10,11-dihydro-5H-dibenz[b.f]azepinyl group. The present invention is not limited to these substituents listed by way of example.

A substituted or unsubstituted amino group indicated by general formula (BA1), therefore, may be substituted with one or more substituents selected from group T₂3, and specific examples include groups such as;
dialkylated amino groups, such as a dimethylamino group, a diethylamino group, a dipropylamino group, a diisopropylamino group, a dibutylamino group, a bis(2-methylpropyl)amino group, a bis(2,2-dimethylpropyl)amino group, a bis(3-cyclopropyl)amino group, a dicyclopropylamino group, a bis(2-methylbutyl)amino group, a bis(3-methylbutyl)amino group, a di(tert-butyl)amino group, a dicyclobutylamino group, a dipentylamino group, a bis(2-methylpentyl)amino group, a di(1-methylbutyl)amino group, a bis(1,2-dimethylbutyl)amino group, a di(1-ethylpropyl)amino group, a dicyclopentylamino group, a dihexylamino group, a dicyclohexylamino group, an N-ethyl-N-methylamino group, an N-methyl-N-propyl group, an N-cyclopropyl-N-methylamino group, an N-butyl-N-methylamino group, an N-cyclobutyl-N-methylamino group, an N-methyl-N-pentyl group, an N-cyclopentyl-N-methylamino group, an N-hexyl-N-methylamino group, an N-cyclohexyl-N-methylamino group, an N-cycloheptyl-N-methylamino group, an N-methyl-N-octylamino group, an N-methyl-N-nonylamino group, an N-decyl-N-methylamino group, an N-methyl-N-undecylamino group, a bis(2-methoxyethyl)amino group, a bis(2-ethoxyethyl)amino group, a bis(2,2,2-trifluoroethyl)amino group, a bis(3-chloropropyl)amino group, a bis(3-bromopropyl)amino group, a bis(2-bromoethyl)amino group, a bis(2-chloroethyl)amino group, a bis(2-cyanoethyl)amino group and a bis(3-cyanopropyl)amino group;
monoalkylated amino groups, such as a methylamino group, an ethylamino group, a propylamino group, an isopropylamino group, a butylamino group, a 2-methylpropylamino group, a 2,2-dimethylpropylamino group, a 3-cyclopropylamino group, a cyclopropylamino group, a tert-butylamino group, a cyclobutylamino group, a pentylamino group, a 2-methylpentylamino group, a hexylamino group, a (2-methoxyethyl)amino group, a (2-ethoxyethyl)amino group, a (2,2,2-trifluoroethyl)amino group, a 3-chloropropylamino group, a 3-bromopropylamino group, a 2-bromoethylamino group, a 2-chloroethylamino group, a 2-cyanoethylamino group and a 3-cyanopropylamino group;
diarylated amino groups, such as a diindenylamino group, an N-indenyl-N-phenylamino group, a diphenylamino group, a bis(2-methylphenyl)amino group, a bis(3-methylphenyl)amino group, a bis(4-methylphenyl)amino group, a bis(2,3-dimethylphenyl)amino group, a bis(2,4-dimethylphenyl)amino group, a bis(2,5-dimethylphenyl)amino group, a bis(2,6-dimethylphenyl)amino group, a bis(3,4-dimethylphenyl)amino group, a bis(3,5-dimethylphenyl)amino group, a bis(2,3,4-trimethylphenyl)amino group, a bis(2,3,5-trimethylphenyl)amino group, a bis(2,3,6-trimethylphenyl)amino group, a bis(2,4,5-trimethylphenyl)amino group, a bis(2,4,6-trimethylphenyl)amino group, a bis(3,4,5-trimethylphenyl)amino group, a bis(2,3,4,5-tetramethylphenyl)amino group, a bis(2,3,4,6-tetramethylphenyl)amino group, a bis(2,3,5,6-tetramethylphenyl)amino group, a bis(2-ethylphenyl)amino group, a bis(3-ethylphenyl)amino group, a bis(4-ethylphenyl)amino group, a bis(2,3-diethylphenyl)amino group, a bis(2,4-diethylphenyl)amino group, a bis(2,5-diethylphenyl)amino group, a bis(2,6-diethylphenyl)amino group, a bis(3,4-diethylphenyl)amino group, a bis(3,5-diethylphenyl)amino group, a bis(2-propylphenyl)amino group, a bis(3-propylphenyl)amino group, a bis(4-propylphenyl)amino group, a bis(2-isopropylphenyl)amino group, a bis(3-isopropylphenyl)amino group, a bis(4-isopropylphenyl)amino group, a bis(2-cyclopropylphenyl)amino group, a bis(3-cyclopropylphenyl)amino group, a bis(4-cyclopropylphenyl)amino group, a bis(2-butylphenyl)amino group, a bis(3-butylphenyl)amino group, a bis(4-butylphenyl)amino group, a bis[2-(1-methylpropyl)phenyl]amino group, a bis[3-(1-methylpropyl)phenyl]amino group, a bis[4-(1-methylpropyl)phenyl]amino group, a bis[2-(2-methylpropyl)phenyl]amino group, a bis[3-(2-methylpropyl)phenyl]amino group, a bis[4-(2-methylpropyl)phenyl]amino group, a bis(2-cyclobutylphenyl)amino group, a bis(3-cyclobutylphenyl)amino group, a bis(4-cyclobutylphenyl)amino group, a di(1-naphthyl)amino group, a di(2-naphthyl)amino group, an N-(2-methylphenyl)-N-phenylamino group, an N-(3-methylphenyl)-N-phenylamino group, an N-(4-methylphenyl)-N-phenylamino group, an N-(2,3-dimethylphenyl)-N-phenylamino group, an N-(2,4-dimethylphenyl)-N-phenylamino group, an N-(2,5-dimethylphenyl)-N-phenylamino group, an N-(2,6-dimethylphenyl)-N-phenylamino group, an N-(3,4-dimethylphenyl)-N-phenylamino group, an N-(3,5-dimethylphenyl)-N-phenylamino group, an N-(2,3,4-trimethylphenyl)-N-phenylamino group, an N-(2,3,5-trimethylphenyl)-N-phenylamino group, an N-(2,3,6-trimethylphenyl)-N-phenylamino group, an N-(2,4,5-trimethylphenyl)-N-phenylamino group, an N-(2,4,6-trimethylphenyl)-N-phenylamino group, an N-(3,4,5-trimethylphenyl)-N-phenylamino group, an N-(1-naphthyl)-N-phenylamino group, an N-(2-naphthyl)-N-phenylamino group, a bis(4-methoxyphenyl)amino group, a bis(2-methoxyphenyl)amino group, a bis[4-(trifluoromethyl)phenyl]amino group, a bis[4-fluorophenyl]amino group, a bis[3,5-difluorophenyl]amino group, a bis(4-acetylphenyl)amino group, a (2-acetylphenyl)(4-acetylphenyl)amino group, a bis(4-pivaloylphenyl)amino group, a bis(2-acetylphenyl)amino group, a bis(4-benzoylphenyl)amino group, a (2-benzoylphenyl)(4-benzoylphenyl)amino group and a bis(4-cyanophenyl)amino group;
diheteroarylated amino groups, such as a bis(2-pyrrolyl)amino group, a bis(1-methylpyrrol-2-yl)amino group, a bis(1-ethylpyrrol-2-yl)amino group, a bis(1-phenylpyrrol-2-yl)amino group, a bis(2-thienyl)amino group, a bis(3-thienyl)amino group, a bis(2-furyl)amino group, a bis(3-furyl)amino group, a bis(2-benzothienyl)amino group, a bis(3-benzothienyl)amino group, a bis(2-benzofuranyl)amino group, a bis(3-benzofuranyl)amino group, a bis(1-methylindol-2-yl)amino group, a bis(1-methylindol-2-yl)amino group, a bis(3-benzofuranyl)amino group, a bis(5-fluoropyrrol-2-yl)amino group, a bis(5-fluoro-1-methylpyrrol-2-yl)amino group, a bis(5-fluoro-1-ethylpyrrol-2-yl)amino group, a bis(5-fluoro-1-phenylpyrrol-2-yl)amino group, a bis(5-fluoro-2-thienyl)amino group, a bis(5-trifluoromethyl-2-thienyl)amino group, a bis(5-fluoro-3-thienyl)amino group, a bis(5-fluoro-2-furyl)amino group, a bis(5-trifluoromethyl-2-furyl)amino group, a bis(5-fluoro-3-furyl)amino group, a bis(5-fluoro-2-benzothienyl)amino group, a bis(5-fluoro-3-benzothienyl)amino group, a bis(5-fluoro-2-benzofuranyl)amino group, a bis(5-fluoro-3-benzofuranyl)amino group, a bis(1-methylindol-2-yl)amino group, a bis(1-methylindol-3-yl)amino group, a bis(5-fluoro-3-benzofuranyl)amino group, a 2-fluorophenothiazinyl group, a 2-trifluoromethylphenothiazinyl group and a 2-trifluoromethylphenoxazinyl group;
monoarylated amino groups, such as an indenylamino group, a phenylamino group, a 2-methylphenylamino group, a 3-methylphenylamino group, a 4-methylphenylamino group, a 2,3-dimethylphenylamino group, a 2,4-dimethylphenylamino group, a 2,5-dimethylphenylamino group, a 2,6-dimethylphenylamino group, a 3,4-dimethylphenylamino group, a 3,5-dimethylphenylamino group, a 2,3,4-trimethylphenylamino group, a 2,3,5-trimethylphenylamino group, a 2,3,6-trimethylphenylamino group, a 2,4,5-trimethylphenylamino group, a 2,4,6-trimethylphenylamino group, a 3,4,5-trimethylphenylamino group, a 2,3,4,5-tetramethylphenylamino group, a 2,3,4,6-tetramethylphenylamino group, a 2,3,5,6-tetramethylphenylamino group, a 2-ethylphenylamino group, a 3-ethylphenylamino group, a 4-ethylphenylamino group, a 2,3-diethylphenylamino group, a 2,4-diethylphenylamino group, a 2,5-diethylphenylamino group, a 2,6-diethylphenylamino group, a 3,4-diethylphenylamino group, a 3,5-diethylphenylamino group, a 2-propylphenylamino group, a 3-propylphenylamino group, a 4-propylphenylamino group, a 2-isopropylphenylamino group, a 3-isopropylphenylamino group, a 4-isopropylphenylamino group, a 2-cyclopropylphenylamino group, a 3-cyclopropylphenylamino group, a 4-cyclopropylphenylamino group, a 2-butylphenylamino group, a 3-butylphenylamino group, a 4-butylphenylamino group, a 2-(1-methylpropyl)phenylamino group, a 3-(1-methylpropyl)phenylamino group, a 4-(1-methylpropyl)phenylamino group, a 2-(2-methylpropyl)phenylamino group, a 3-(2-methylpropyl)phenylamino group, a 4-(2-methylpropyl)phenylamino group, a 2-cyclobutylphenylamino group, a 3-cyclobutylphenylamino group, a 4-cyclobutylphenylamino group, a 1-naphthylamino group, a 2-naphthylamino group, a (4-methoxyphenyl)amino group, a (2-methoxyphenyl)amino group, a [4-(trifluoromethyl)phenyl]amino group, a (4-acetylphenyl)amino group, a (2-acetylphenyl)amino group, a (4-pivaloylphenyl)amino group and a (4-benzoylphenyl)amino group;
monoheteroarylated amino groups, such as a 2-pyrrolylamino group, a 2-thienylamino group, a 3-thienylamino group, a 2-furylamino group, a 3-furylamino group, a 2-(1-methylindolyl)amino group, a 2-benzothienyl group, a 3-benzothienyl group, a 2-benzofuranyl group and a 3-benzofuranyl group; and
cyclic amino groups, such as an azetidyl group, a pyrrolidinyl group, a piperidino group, an azepanyl group, a morpholino group, a 4-methylpiperazinyl group, a carbazoyl group, a phenoxazinyl group, a phenothiazinyl group and a 10,11-dihydro-5H-dibenz[b,f]azepinyl group. The present invention is not limited to these substituents listed by way of example. A diarylated or diheteroarylated amino group is preferred because it gives the complex optical characteristics suitable for use as an optical functional material. A diphenylamino group, bis[4-(trifluoromethyl)phenyl]amino group or bis(4-cyanophenyl)amino group is more preferred because of the ease of synthesis.

A substituted or unsubstituted oxy group indicated by general formula (BO1), furthermore, may be substituted with one or more substituents selected from group T₂3, and specific examples include groups such as;
substituted alkyloxy groups, such as a methoxy group, an ethoxy group, a propyloxy group, an isopropyloxy group, a butyloxy group, a 2-methylpropyloxy group, a 2,2-dimethylpropyloxy group, a 3-cyclopropylpropyloxy group, a cyclopropyloxy group, a 2-methylbutyloxy group, a 3-methylbutyloxy group, a tert-butyloxy group, a cyclobutyloxy group, a pentyloxy group, a 2-methylpentyloxy group, a 1-methylbutyloxy group, a 1,2-dimethylbutyloxy group, a 1-ethylpropyloxy group, a cyclopentyloxy group, a hexyloxy group and a cyclohexyloxy group; and
substituted aryloxy groups, such as an indenyloxy group, a phenyloxy group, a 2-methylphenyloxy group, a 3-methylphenyloxy group, a 4-methylphenyloxy group, a 2,3-dimethylphenyloxy group, a 2,4-dimethylphenyloxy group, a 2,5-dimethylphenyloxy group, a 2,6-dimethylphenyloxy group, a 3,4-dimethylphenyloxy group, a 3,5-dimethylphenyloxy group, a 2,3,4-trimethylphenyloxy group, a 2,3,5-trimethylphenyloxy group, a 2,3,6-trimethylphenyloxy group, a 2,4,5-trimethylphenyloxy group, a 2,4,6-trimethylphenyloxy group, a 3,4,5-trimethylphenyloxy group, a 2,3,4,5-tetramethylphenyloxy group, a 2,3,4,6-tetramethylphenyloxy group, a 2,3,5,6-tetramethylphenyloxy group, a 2-ethylphenyloxy group, a 3-ethylphenyloxy group, a 4-ethylphenyloxy group, a 2,3-diethylphenyloxy group, a 2,4-diethylphenyloxy group, a 2,5-diethylphenyloxy group, a 2,6-diethylphenyloxy group, a 3,4-diethylphenyloxy group, a 3,5-diethylphenyloxy group, a 2-propylphenyloxy group, a 3-propylphenyloxy group, a 4-propylphenyloxy group, a 2-isopropylphenyloxy group, a 3-isopropylphenyloxy group, a 4-isopropylphenyloxy group, a 2-cyclopropylphenyloxy group, a 3-cyclopropylphenyloxy group, a 4-cyclopropylphenyloxy group, a 2-butylphenyloxy group, a 3-butylphenyloxy group, a 4-butylphenyloxy group, a 2-(1-methylpropyl)phenyloxy group, a 3-(1-methylpropyl)phenyloxy group, a 4-(1-methylpropyl)phenyloxy group, a 2-(2-methylpropyl)phenyloxy group, a 3-(2-methylpropyl)phenyloxy group, a 4-(2-methylpropyl)phenyloxy group, a 2-cyclobutylphenyloxy group, a 3-cyclobutylphenyloxy group, a 4-cyclobutylphenyloxy group, a (biphenyl-2-yl)oxy group, a (biphenyl-3-yl)oxy group, a (biphenyl-4-yl)oxy group, a 4-fluorophenyloxy group, a 4-trifluoromethylphenyl group a (phenanthren-9-yl)oxy group and an (anthracen-9-yl)oxy group. The present invention is not limited to these substituents listed by way of example. A methoxy group, ethoxy group, or phenoxy group is preferred because of the ease of synthesis.

A substituted or unsubstituted thio group indicated by general formula (BS1), moreover, may be substituted with one or more substituents selected from group T₂3, and specific examples include groups such as;
substituted alkylthio groups, such as a methylthio group, an ethylthio group, a propylthio group, a 1-(1-methylethyl)thio group, a butylthio group, a 2-methylpropylthio group, a 2,2-dimethylpropylthio group, a 3-cyclopropylpropylthio group, a cyclopropylthio group, a 2-methylbutylthio group, a 3-methylbutylthio group, a 1-(1,1-dimethylethyl)thio group, a cyclobutylthio group, a pentylthio group, a 2-methylpentylthio group, a 1-methylbutylthio group, a 1,2-dimethylbutylthio group, a 1-ethylpropylthio group, a cyclopentylthio group, a hexylthio group and a cyclohexylthio group; and
substituted arylthio groups, such as a phenylthio group, a 2-methylphenylthio group, a 3-methylphenylthio group, a 4-methylphenylthio group, a 2,3-dimethylphenylthio group, a 2,4-dimethylphenylthio group, a 2,5-dimethylphenylthio group, a 2,6-dimethylphenylthio group, a 3,4-dimethylphenylthio group, a 3,5-dimethylphenylthio group, a 2,3,4-trimethylphenylthio group, a 2,3,5-trimethylphenylthio group, a 2,3,6-trimethylphenylthio group, a 2,4,5-trimethylphenylthio group, a 2,4,6-trimethylphenylthio group, a 3,4,5-trimethylphenylthio group, a 2,3,4,5-tetramethylphenylthio group, a 2,3,4,6-tetramethylphenylthio group, a 2,3,5,6-tetramethylphenylthio group, a 2-ethylphenylthio group, a 3-ethylphenylthio group, a 4-ethylphenylthio group, a 2,3-diethylphenylthio group, a 2,4-diethylphenylthio group, a 2,5-diethylphenylthio group, a 2,6-diethylphenylthio group, a 3,4-diethylphenyl group, a 3,5-diethylphenyl group, a 2-propylphenyl group, a 3-propylphenylthio group, a 4-propylphenylthio group, a 2-isopropylphenylthio group, a 3-isopropylphenylthio group, a 4-isopropylphenylthio group, a 2-cyclopropylphenylthio group, a 3-cyclopropylphenylthio group, a 4-cyclopropylphenylthio group, a 2-butylphenylthio group, a 3-butylphenylthio group, a 4-butylphenylthio group, a 2-(1-methylpropyl)phenylthio group, a 3-(1-methylpropyl)phenylthio group, a 4-(1-methylpropyl)phenylthio group, a 2-(2-methylpropyl)phenylthio group, a 3-(2-methylpropyl)phenylthio group, a 4-(2-methylpropyl)phenylthio group, a 2-cyclobutylphenylthio group, a 3-cyclobutylphenylthio group, a 4-cyclobutylphenylthio group, a 1-naphthylthio group, a 2-naphthylthio group, a (biphenyl-2-yl)thio group, a (biphenyl-3-yl)thio group, a (biphenyl-4-yl)thio group, a 9-anthrylthio group, a 2-phenanthrenylthio group, a 3-phenanthrenylthio group and a 9-phenanthrenylthio group. The present invention is not limited to these substituents listed by way of example.

Of R^{C1}, R^{C2}, R^{C3} and R^{C4}, furthermore, two adjacent substituents may form a five-membered, six-membered or seven-membered ring together with the benzene ring to which they are bound. For example, possible structures of the coumarinato ligand (1cu) include the structures represented by (1cu-2) to (1cu-10) below. The present invention is not limited to these.

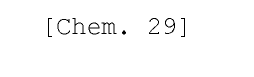

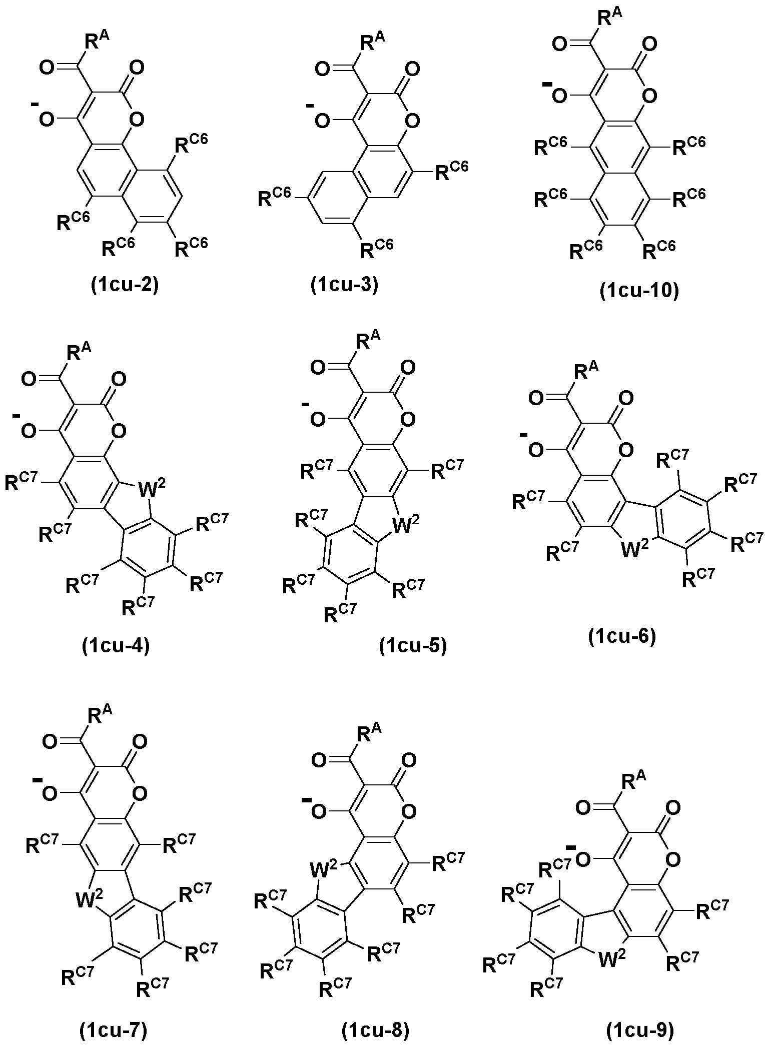

(In the formulae, R^{A} is synonymous with the meaning described above. The R^{C6}s each independently represent a hydrogen atom or C1 to C4 alkyloxy group. The R^{C7}s each independently represent a hydrogen atom, halogen atom or C1 to C4 fluoroalkyl group. W² represents an oxygen atom, sulfur atom, nitrogen atom optionally substituted with a C1 to C12 hydrocarbon group or methylene group optionally substituted with one or more C1 to C12 hydrocarbon groups.)

The definitions of R^{C6}, R^{C7} and W² will now be described individually.

### (R^{C6} and R^{C7})

A C1 to C4 alkyloxy group represented by R^{C6} may be any of a linear-chain, branched or cyclic alkyl group. Specific examples include a methoxy group, an ethoxy group, a propyloxy group, an isopropyloxy group, a butyloxy group, a 2-methylpropyloxy group, a cyclopropyloxy group, a tert-butyloxy group and a cyclobutyloxy group.

Examples of halogen atoms represented by R^{C7} include the substituents listed as examples of halogen atoms that belong to group T₂1.

A C1 to C4 fluoroalkyl group represented by R^{C7} may be any of a linear-chain, branched or cyclic alkyl group. Specific examples include groups such as a trifluoromethyl group, a difluoromethyl group, a perfluoroethyl group, a 2,2,2-trifluoroethyl group, a 1,1-difluoroethyl group, a 2,2-difluoroethyl group, a perfluoropropyl group, a 2,2,3,3,3-pentafluoropropyl group, a 2,2,3,3-tetrafluoropropyl group, a 3,3,3-trifluoropropyl group, a 1,1-difluoropropyl group, a 1,1,1,2,3,3,3-hexafluoropropan-2-yl group, a 1,1,1,3,3,3-hexafluoropropan-2-yl group, a perfluorobutyl group, a 2,2,3,3,4,4,4-heptafluorobutyl group, a 3,3,4,4,4-pentafluorobutyl group, a 4,4,4-trifluorobutyl group, a 1,1,1,2,3,3,4,4,4-nonafluorobutan-2-yl group, a 1,1,1-trifluorobutan-2-yl group, a 3,3,3-trifluorobutan-2-yl group and a perfluorocyclobutyl group. The present invention is not limited to these substituents listed by way of example.

R^{C6} is preferably a hydrogen atom or methoxy group because of the ease of synthesis.

R^{C7} is preferably a hydrogen atom because of the ease of synthesis.

### (W2)

When W² is a nitrogen atom optionally substituted with a C1 to C12 hydrocarbon group, specific examples of the C1 to C12 hydrocarbon group include groups such as a methyl group, an ethyl group, a propyl group, an isopropyl group, a butyl group, a 2-methylpropyl group, a 2,2-dimethylpropyl group, a 3-cyclopropylpropyl group, a cyclopropyl group, a 2-methylbutyl group, a 3-methylbutyl group, a tert-butyl group, a cyclobutyl group, a pentyl group, a 2-methylpentyl group, a 1-methylbutyl group, a 1,2-dimethylbutyl group, a 1-ethylpropyl group, a cyclopentyl group, a hexyl group, a cyclohexyl group, a heptyl group, an octyl group, a nonyl group, a decyl group, a phenyl group, a naphthyl group and a biphenylyl group. The present invention is not limited to these substituents listed by way of example.

When W² is a methylene group optionally substituted with one or more C1 to C12 hydrocarbon groups, examples of the C1 to C12 hydrocarbon groups include the groups listed as examples in the context of the C1 to C12 hydrocarbon group of a nitrogen atom optionally substituted with a C1 to C12 hydrocarbon group.

W² is preferably an oxygen atom, sulfur atom, methylene group optionally substituted with one or more C1 to C4 alkyl groups or nitrogen atom optionally substituted with a C1 to C8 alkyl group or C6 to C12 aryl group because of easy availability of raw materials, more preferably an oxygen atom, sulfur atom, dimethylmethylene group or nitrogen atom substituted with a C1 to C8 alkyl group or phenyl group because of the ease of synthesis, particularly preferably a nitrogen atom substituted with a C1 to C8 alkyl group or phenyl group because of inexpensiveness of raw materials.

When W² is a methylene group optionally substituted with one or more C1 to C4 alkyl groups, the C1 to C4 alkyl groups may be any of linear-chain, branched or cyclic. Examples include a methyl group, an ethyl group, a propyl group, an isopropyl group, a butyl group, a 1-methylpropyl group, a 2-methylpropyl group, a cyclopropyl group, a butyl group, a tert-butyl group and a cyclobutyl group.

When W² is a nitrogen atom optionally substituted with a C1 to C8 alkyl group, the C1 to C8 alkyl group may be any of a linear-chain, branched or cyclic alkyl group. Specific examples include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a 5-dimethylcyclopentyl group, a 3-ethylcyclopentyl group, a hexyl group, a cyclohexyl group, a 4-ethylcyclohexyl group, a 4-propylcyclohexyl group, a 4,4-dimethylcyclohexyl group, a 2,6-dimethylcyclohexyl group, a 3,5-dimethylcyclohexyl group, a cycloheptyl group, an octyl group and a cyclooctyl group. The present invention is not limited to these substituents listed by way of example.

When W² is a nitrogen atom optionally substituted with a C6 to C12 aryl group, specific examples of the C6 to C12 aryl group include groups such as a phenyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a 2-ethylphenyl group, a 3-ethylphenyl group, a 4-ethylphenyl group, a 2,3-diethylphenyl group, a 2,4-diethylphenyl group, a 2,5-diethylphenyl group, a 2,6-diethylphenyl group, a 3,4-diethylphenyl group, a 3,5-diethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-cyclopropylphenyl group, a 3-cyclopropylphenyl group, a 4-cyclopropylphenyl group, a 2-butylphenyl group, a 3-butylphenyl group, a 4-butylphenyl group, a 2-(1-methylpropyl)phenyl group, a 3-(1-methylpropyl)phenyl group, a 4-(1-methylpropyl)phenyl group, a 2-(2-methylpropyl)phenyl group, a 3-(2-methylpropyl)phenyl group, a 4-(2-methylpropyl)phenyl group, a 2-cyclobutylphenyl group, a 3-cyclobutylphenyl group, a 4-cyclobutylphenyl group, a 1-naphthyl group, a 2-naphthyl group, a 2-biphenyl group, a 3-biphenyl group, a 4-biphenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-(trifluoromethyl)phenyl group, a 3-(trifluoromethyl)phenyl group, a 4-(trifluoromethyl)phenyl group, a 2-(perfluoropropyl)phenyl group, a 3-(perfluoropropyl)phenyl group, a 4-(perfluoropropyl)phenyl group, a 2-acetylphenyl group, a 3-acetylphenyl group, a 4-acetylphenyl group, a 2-pivaloylphenyl group, a 3-pivaloylphenyl group, a 4-pivaloylphenyl group, a 2-benzoylphenyl group, a 3-benzoylphenyl group, a 4-benzoylphenyl group, a 2-naphthoylphenyl group, a 3-naphthoylphenyl group, a 4-naphthoylphenyl group, a 4-methoxy-1-naphthyl group, a 6-methoxy-2-naphthyl group, a 4'-methoxybiphenyl-2-yl group, a 4'-methoxybiphenyl-3-yl group, a 4'-methoxybiphenyl-4-yl group, a 10-methoxy-9-anthryl group, a 2-fluorophenyl group, a 3-fluorophenyl group, a 4-fluorophenyl group and a 3,4-difluorophenyl group. The present invention is not limited to these substituents listed by way of example.

### <n>

n represents 1, 2 or 3. When n is 2 or 3, the multiple QUs or CUs may be the same or different from each other. n is preferably 3 because this gives the complex optical characteristics suitable for use as an optical functional material. It is, furthermore, preferred that the QUs or CUs be the same because of the ease of synthesis.

### <L¹>

L¹ represents a phosphorus ligand represented by general formula (3a) or (3b) or nitrogen-containing ligand having two or more nitrogen atoms possessing a lone pair. When m¹ is 2, the L¹s may be the same or different from each other. The L¹s are preferably the same because of the ease of synthesis.

When L¹ in the rare earth complex (1) according to the present invention is a phosphorus ligand represented by general formula (3a) or (3b), a lone pair of the oxygen atom bound to the phosphorus atom coordinates with M³⁺.

### (X^{A})

Examples of C1 to C10 alkyl groups represented by X^{A} in general formulae (3a) and (3b) include the groups listed as examples of C1 to C10 alkyl groups represented by R^{B1}, R^{B2}, R^{B3}, R^{B4} and R^{B5}. A cyclopentyl group, cyclohexyl group or tert-butyl group is preferred because of easy availability of raw materials, and a cyclohexyl group is more preferred because it gives the complex optical characteristics suitable for use as an optical functional material.

### (X1)

When X^{A} represents an aryl group represented by general formula (3c), furthermore, examples of halogen atoms represented by X¹ in general formula (3c) include the substituents listed as examples of halogen atoms that belong to group T₂1.

Specific examples of C1 to C6 alkyl groups represented by X¹ in general formula (3c) include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a hexyl group and a cyclohexyl group. The present invention is not limited to these substituents listed by way of example.

Examples of C1 to C6 alkyloxy groups represented by X¹ in general formula (3c) include the groups listed as examples of C1 to C6 alkyloxy groups that belong to group T₂1.

Examples of C6 to C22 aryl groups represented by X¹ in general formula (3c) include the groups listed as examples of C6 to C22 aryl groups listed by way of example in the context of R^{A}.

Examples of C3 to C20 heteroaryl groups represented by X¹ in general formula (3c) include the groups listed as examples in the context of a C3 to C20 heteroaryl group represented by R^{A}.

Examples of C5 to C14 aryloxy groups represented by X¹ in general formula (3c) include the groups listed as examples of C5 to C14 aryloxy groups that belong to group T₂1.

Examples of C1 to C6 haloalkyl groups represented by X¹ in general formula (3c) include the C1 to C6 haloalkyl groups listed by way of example in the context of R^{A}.

A C1 to C6 haloalkyloxy group represented by X¹ in general formula (3c) may be any of a linear-chain, branched or cyclic haloalkyloxy group. Specific examples include groups such as a trifluoromethyloxy group, a difluoromethyloxy group, a perfluoroethyloxy group, a 2,2,2-trifluoroethyloxy group, a 1,1-difluoroethyloxy group, a 2,2-difluoroethyloxy group, a perfluoropropyloxy group, a 2,2,3,3,3-pentafluoropropyloxy group, a 2,2,3,3-tetrafluoropropyloxy group, a 3,3,3-trifluoropropyloxy group, a 1,1-difluoropropyloxy group, a 1,1,1,2,3,3,3-heptafluoropropan-2-yloxy group, a 2,2,2-trifluoro-1-(trifluoromethyl)ethyloxy group, a perfluoropentyloxy group, a perfluorocyclopentyloxy group, a perfluorohexyloxy group, a perfluorocyclohexyloxy group, a chloromethyloxy group, a bromomethyloxy group, an iodomethyloxy group, a 2-chloroethyloxy group, a 3-bromopropyloxy group and a 3-iodopropyloxy group.

When X¹ in general formula (3c) is a C6 to C22 aryl group, the C6 to C22 aryl group may be substituted with one or more substituents selected from the group consisting of halogen atoms, C1 to C6 alkyloxy groups, C1 to C6 haloalkyl groups, C1 to C6 haloalkyloxy groups, a hydroxyl group, a cyano group and a nitro group (group T₂6). Specific examples include groups such as a phenyl group, a deuterated phenyl group, a 1-naphthyl group, a 2-naphthyl group, a deuterated naphthyl group, a 2-methylphenyl group, a 4-methylphenyl group, a 2,5-dimethylphenyl group, a 2,4,6-trimethylphenyl group, a 4-ethylphenyl group, a 4-propylphenyl group, a 2,4,6-triisopropylphenyl group, a 4-tert-butylphenyl group, a 1-methylnaphthalen-2-yl group, a 2-methylnaphthalen-1-yl group, a 4-methylnaphthalen-1-yl group, a 4-methylnaphthalen-2-yl group, a 2-trifluoromethylphenyl group, a 3-(trifluoromethyl)phenyl group, a 4-trifluoromethylphenyl group, a 3,5-bis(trifluoromethyl)phenyl group, a 1-(trifluoromethyl)naphthalen-2-yl group, a 2-(trifluoromethyl)naphthalen-1-yl group, a 4-(trifluoromethyl)naphthalen-2-yl group, a 4-(trifluoromethyl)naphthalen-1-yl group, a 4-methoxyphenyl group, a 4-(isopropyloxy)phenyl group, a 1-methoxynaphthalen-2-yl group, a 2-methoxynaphthalen-1-yl group, a 4-methoxynaphthalen-2-yl group, a 4-trifluoromethoxyphenyl group, a 1-(trifluoromethyloxy)naphthalen-2-yl group, a 2-(trifluoromethyloxy)naphthalen-1-yl group, a 2-fluorophenyl group, a 3-fluorophenyl group, a 4-fluorophenyl group, a 2-chlorophenyl group, a 3-chlorophenyl group, a 4-chlorophenyl group, a 3,5-dichlorophenyl group, a 2,6-dichlorophenyl group, a 2-bromophenyl group, a 3-bromophenyl group, a 4-bromophenyl group, a 1-bromonaphthalen-2-yl group, a 2-bromonaphthalen-1-yl group, a 4-bromonaphthalen-2-yl group, a 6 bromonaphthalen-2-yl group, a 2-cyanophenyl group, a 3-cyanophenyl group, a 4-cyanophenyl group, a 1-cyanonaphthalen-2-yl group, a 2-cyanonaphthalen-1-yl group, a 4-cyanonaphthalen-2-yl group, a 6-cyanonaphthalen-2-yl group, a 2-nitrophenyl group, a 3-nitrophenyl group and a 4-nitrophenyl group. The present invention is not limited to these substituents listed by way of example.

X^{A}, moreover, represents a substituted or unsubstituted amino group indicated by general formula (BA2) or substituted or unsubstituted oxy group indicated by general formula (BO2).

Specific examples of C1 to C6 alkyl groups represented by R^{BA3} and R^{BA4} in general formula (BA2) and R^{BO2} in general formula (BO2) include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a hexyl group and a cyclohexyl group. The present invention is not limited to these substituents listed by way of example.

Examples of C6 to C22 aryl groups represented by R^{BA3} and R^{BA4} in general formula (BA2) and R^{BO2} in general formula (BO2) include the groups listed as examples of C6 to C22 aryl groups represented by R^{A}.

Examples of C3 to C20 heteroaryl groups represented by R^{BA3} and R^{BA4} in general formula (BA2) and R^{BO2} in general formula (BO2) include the groups listed as examples of C3 to C20 heteroaryl groups represented by R^{A}.

A C1 to C6 alkyl, C6 to C22 aryl or C3 to C20 heteroaryl group represented by R^{BA3} and R^{BA4} in general formula (BA2), furthermore, may be substituted with one or more substituents selected from group T₂3.

A C1 to C6 alkyl, C6 to C22 aryl or C3 to C20 heteroaryl group represented by R^{BO2} in general formula (BO2), moreover, may be substituted with one or more substituents selected from group T₂3.

Specific examples of C1 to C6 alkyl groups at R^{BA3} and R^{BA4} optionally substituted with one or more substituents selected from group T₂3 and C1 to C6 alkyl groups at R^{BO2} optionally substituted with one or more substituents selected from group T₂3, therefore, include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a hexyl group and a cyclohexyl group. The present invention is not limited to these substituents listed by way of example.

Examples of C1 to C6 alkyl groups at R^{BA3} and R^{BA4} optionally substituted with one or more substituents selected from group T₂3 and C6 to C22 aryl groups at R^{BO2} optionally substituted with one or more substituents selected from group T₂3, furthermore, include the substituents listed as examples for a C6 to C22 aryl group at R^{A} optionally substituted with one or more substituents selected from group T₂1.

Examples of C1 to C6 alkyl groups at R^{BA3} and R^{BA4} optionally substituted with one or more substituents selected from group T₂3 and C3 to C20 heteroaryl groups at R^{BO2} optionally substituted with one or more substituents selected from group T₂3, moreover, include the substituents listed as examples for a C3 to C20 heteroaryl group at R^{A} optionally substituted with one or more substituents selected from group T₂1.

In addition, R^{BA3} and R^{BA4} may form a five-membered, six-membered or seven-membered ring including the nitrogen atom to which they are bound. Specific examples include groups such as an azetidyl group, a pyrrolidinyl group, a piperidino group, an azepanyl group, a morpholino group, a 4-methylpiperazinyl group, a carbazoyl group, a phenoxazinyl group, a phenothiazinyl group and a 10,11-dihydro-5H-dibenz[b,f]azepinyl group. The present invention is not limited to these substituents listed by way of example.

A substituted or unsubstituted amino group indicated by general formula (BA2), therefore, may be substituted with one or more substituents selected from group T₂3, and specific examples include the substituents listed as examples for a substituted or unsubstituted amino group indicated by general formula (BA1).

A substituted or unsubstituted oxy group indicated by general formula (BO2), furthermore, may be substituted with one or more substituents selected from group T₂3, and specific examples include the substituents listed as examples for a substituted or unsubstituted amino group indicated by general formula (BO1).

### (XH)

A C1 to C10 alkylene group represented by X^{H} in general formula (3b) may be any of linear-chain, branched or cyclic. Specific examples include groups such as a methylene group, an ethylene group, a trimethylene group, a tetramethylene group, a pentamethylene group, a hexamethylene group, a heptamethylene group, an octamethylene group, a nonamethylene group, a decamethylene group, a methylethylene group, a 2-methylpropan-1,3-diyl group, a butan-1,2-diyl group, a butan-2,3-diyl group, a 2-methylpropan-2,3-diyl group, a pentan-1,3-diyl group, a 2-ethylpropan-1,3-diyl group, a 2-methylbutan-1,3-diyl group, a 2,2-dimethylpropan-1,3-diyl group, a hexan-1,3-diyl group, a 2-propylpropan-1,3-diyl group, a 1-methylpentan-1,3-diyl group, a 2-ethyl-butan-1,3-diyl group, a 2-methylpentan-1,3-diyl group, a 2-methyl-2-ethyl-propan-1,3-diyl group, a pentan-1,4-diyl group, a 2-methylbutan-1,4-diyl group, a 2-ethylbutan-1,4-diyl group, a hexan-1,5-diyl group, a heptan-1,5-diyl group, a heptan-1,6-diyl group, an octan-1,7-diyl group, a nonan-1,8-diyl group, a decan-1,9-diyl group, a cyclopentan-1,2-diyl group, a cyclohexan-1,2-diyl group, a cyclohexan-1,3-diyl group, a cyclohexan-1,4-diyl group, a 1,2-cyclohexylenebis(methylene) group, a 1,3-cyclohexylenebis(methylene) group, a 1,4-cyclohexylenebis(methylene) group, and a cycloheptan-1,2-diyl group. The present invention is not limited to these substituents listed by way of example.

A C2 to C10 alkenylene group represented by X^{H} in general formula (3b) may be any of linear-chain, branched or cyclic. Specific examples include groups such as a vinylene group, a 1-methylvinylene group, a prop-1-en-1,3-diyl group, a but-1-en-1,4-diyl group, a but-2-en-1,4-diyl group, a pent-1-en-1,5-diyl group, a pent-2-en-1,5-diyl group, a cyclopent-1-en-1,2-diyl group, a cyclohex-1-en-1,2-diyl group and a cyclooct-1-en-1,2-diyl group. The present invention is not limited to these substituents listed by way of example.

A C2 to C10 alkynylene group represented by X^{H} in general formula (3b) may be any of linear-chain or branched. Specific examples include groups such as an ethynylenic group, a prop-1-yn-1,3-diyl group, a but-1-yn-1,3-diyl group, a but-1-yn-1,4-diyl group, a buta-1,3-diyn-1,4-diyl group, a pent-2-yn-1,5-diyl group, a penta-2,4-diyn-1,5-diyl group, a hex-2-yn-1,6-diyl group, a hexa-1,3,5-triyn-1,6-diyl group, a hept-3-yn-1, 7-diyl group, a oct-4-yn-1,8-diyl group, a nonan-4-yn-1,9-diyl group and a dec-5-yn-1,10-diyl group. The present invention is not limited to these substituents listed by way of example.

Specific examples of C5 to C24 arylene groups represented by X^{H} in general formula (3b) include groups such as a 1,2-phenylene group, a 1,3-phenylene group, a 1,4-phenylene group, a naphthalen-1,2-diyl group, a naphthalen-1,4-diyl group, a naphthalen-1,6-diyl group, a naphthalen-1,8-diyl group, a phenanthren-1,2-diyl group, a 9,10-phenanthren-1,2-diyl group, a naphthacen-1,2-diyl group, a naphthacen-2,3-diyl group, a naphthacen-1,12-diyl group, a naphthacen-5,6-diyl group, a pyren-1,6-diyl group, a pyren-1,8-diyl group, a pyren-2,7-diyl group, a biphenyl-2,2'-diyl group, a biphenyl-4,4'-diyl group, a biphenyl-2,3-diyl group, a biphenyl-3,4-diyl group, a p-terphenyl-4,4"-diyl group, a m-terphenyl-4,4''-diyl group, a p-terphenyl-3,3"-diyl group, an o-terphenyl-4,4''-diyl group, an o-terphenyl-3,3''-diyl group, a chrysen-6,12-diyl group, a coronen-1,8-diyl triphenylen-2,7-diyl group, a binaphthyl-2,2'-diyl group, a diphenylether-2,2'-diyl group, a xanthen-4,5-diyl group and a 9,9-dimethylxanthen-4,5-diyl group. The present invention is not limited to these substituents listed by way of example.

Specific examples of C4 to C23 heteroarylene groups represented by X^{H} in general formula (3b) include groups such as a furan-2,5-diyl group, a thiophen-2,5-diyl group, a benzo[b]thiophen-2,3-diyl group, a benzo[1,2-b:4,5-b]dithiophen-2,6-diyl group, a 9-phenylcarbazol-2,7-diyl group, a 9-phenylcarbazol-3,6-diyl group, a dibenzofuran-2,8-diyl group, a dibenzofuran-4,6-diyl group, a dibenzothiophen-2,8-diyl group, a dibenzothiophen-3,7-diyl group, a dibenzothiophen-4,6-diyl group and a 1,10-phenanthrolin-3,8-diyl group. The present invention is not limited to these substituents listed by way of example.

More specific examples of phosphorus ligands represented by general formula (3a) or (3b), therefore, include triphenylphosphine oxide, cyclohexyldiphenylphosphine oxide, tri(p-tolyl)phosphine oxide, triphenylphosphine oxide-d₁₅, tributylphosphine oxide, tri(tert-butyl)phosphine oxide, trioctylphosphine oxide, tricyclohexylphosphine oxide, dicyclohexylphenylphosphine oxide, dicyclohexyl(o-tolyl)phosphine oxide, 2-biphenylyldicyclohexylphosphine oxide, tripentylphosphine oxide, 2-biphenylyldiphenylphosphine oxide, tri(o-tolyl)phosphine oxide, tris(2-methoxyphenyl)phosphine oxide, 1,2-bis(diphenylphosphinyl)ethane, 1,3-bis(diphenylphosphinyl)propane, 1,4-bis(diphenylphosphinyl)butane, 1,2-bis(dicyclohexylphosphinyl)ethane, 1,2-bis(diphenylphosphinyl)benzene, 1,8-bis(diphenylphosphinyl)naphthalene, 6,6'-bis(diphenylphosphinyl)-2,2'-bipyridine, bis[2-[(oxo)diphenylphosphino]phenyl]ether, 1,1'-biphenyl-2,2'-diylbis(1,1-diphenylphosphine oxide), 2,2'-bis(diphenylphosphinyl)-1,1'-binaphthyl, 2,2'-bis(diphenylphosphinyl)-1,1'-binaphthyl, 4,5-bis(diphenylphosphinyl)-9,9-dimethylxanthene and 4,5-bis[di(tert-butyl)phosphinyl]-9,9-dimethylxanthene. The present invention is not limited to these substituents listed by way of example.

L¹ in general formula (1), furthermore, represents a nitrogen-containing ligand having two or more nitrogen atoms possessing a lone pair. When this nitrogen-containing ligand in the rare earth complex (1) according to the present invention coordinates, one or more lone pairs of the nitrogen atoms in the nitrogen ligand coordinate with M³⁺. Specific examples of the nitrogen-containing ligand include 1,10-phenanthroline, 2-methyl-1,10-phenanthroline, 5-methyl-1,10-phenanthroline, 5,6-dimethyl-1,10-phenanthroline, 2,9-dimethyl-1,10-phenanthroline, 4,7-dimethyl-1,10-phenanthroline, 3,4,7,8-tetramethyl-1,10-phenanthroline, 2,4,7,9-tetramethyl-1,10-phenanthroline, 2,9-dimethyl-4,7-diphenyl-1,10-phenanthroline, 5-chloro-1,10-phenanthroline, 2-chloro-1,10-phenanthroline, 2,9-dichloro-1,10-phenanthroline, 4,7-dichloro-1,10-phenanthroline, 5-bromo-1,10-phenanthroline, 2-bromo-1,10-phenanthroline, 3-bromo-1,10-phenanthroline, 3,8-dibromo-1,10-phenanthroline, 4,7-dibromo-1,10-phenanthroline, 3,5,6,8-tetrabromo-1,10-phenanthroline, 5-hydroxy-1,10-phenanthroline, 4,7-dihydroxy-1,10-phenanthroline, 4,7-diphenyl-1,10-phenanthroline, 2,9-diphenyl-1,10-phenanthroline, 5-amino-1,10-phenanthroline, 5,6-diamino-1,10-phenanthroline, 5-nitro-1,10-phenanthroline, 1,10-phenanthrolin-5,6-dione, 2,2'-bipyridine, 2,2'-bipyridine-d₈, 2,2'-bipyridine-6-carbonitrile, 5,5'-dimethyl-2,2'-bipyridine, 4,4'-dimethyl-2,2'-bipyridine, 6,6'-dimethyl-2,2'-bipyridine, 4,4'-diamino-2,2'-bipyridine, 2,2'-bipyridyl-1,1'-dioxide, 2,2'-bipyridine-5,5'-diol, 6,6'-dicyano-2,2'-bipyridine, 4,4'-bis(dihydroxymethyl)-2,2'-bipyridine, 4,4'-dinonyl-2,2'-bipyridine, 4,4'-di(tert-butyl)-2,2'-bipyridine, 4,4'-dimethoxy-2,2'-bipyridine, 2,2':6'2''-terpyridine, 6-bromo-2,2'-bipyridine, 4-bromo-2,2'-bipyridine, 4,4'-dibromo-2,2'-bipyridine, 5,5'-dibromo-2,2'-bipyridine, 4,4'-diphenyl-2,2'-bipyridine, 5,5'-dimethyl-2,2'-bipyridine, 4,4',5,5'-tetramethyl-2,2'-bipyridine, 4,4'-bis(1,1-dimethylethyl)-2,2'-bipyridine, 4,4'-bis(dimethylamino)-2,2'-bipyridine, 4,4'-difluoro-2,2'-bipyridine, 5,5'-difluoro-2,2'-bipyridine, 4,4'-bis(trifluoromethyl)-2,2'-bipyridine, 5,5'-bis(trifluoromethyl)-2,2'-bipyridine, dimethyl-2,2'-bipyridine-4,4'-dicarboxylate, dimethyl-2,2'-bipyridine-5,5'-dicarboxylate diethyl-2,2'-bipyridine-4,4'-dicarboxylate, diethyl-2,2'-bipyridine-5,5'-dicarboxylate, dipyrido[3,2-a:2',3'-c]phenazine, 2,2'-biquinoline, 4,5-diazafluoren-9-one; and N,N-diethyl-4-{[4,6-bis(3,5-dimethyl-1H-pyrazol-1-yl)-1,3,5-triazin-2-yl]}aniline. The present invention is not limited to these.

L¹ in general formula (1) is preferably; a phosphorus ligand represented by general formula (3a) or (3b) (preferably, one in which X^{A} is a C1 to C10 alkyl group or aryl group represented by general formula (3c)); phenanthroline optionally substituted with one or more methyl groups or phenyl groups; bipyridine optionally substituted with one or more methyl groups or phenyl groups; or; N,N-diethyl-4-{[4,6-bis(3,5-dimethyl-1H-pyrazol-1-yl)-1,3,5-triazin-2-yl]}aniline; or dipyrido[3,2-a:2',3'-c]phenazine because this gives the complex optical characteristics suitable for use as an optical functional material, and it is more preferred that in general formulae (3a) and (3b), X^{A} be a C4 to C8 alkyl group or aryl group represented by general formula (3d), and X^{H} be; a C1 to C4 alkylene group; diphenylether-2,2'-diyl group; naphthalen-1,8-diyl group; biphenyl-2,2'-diyl group; binaphthyl-2,2'-diyl group; bipyridin-2,2'-diyl group; or xanthen-4,5-diyl group optionally substituted with one or more methyl groups because of the ease of synthesis.

Specific examples of the phenanthroline optionally substituted with one or more methyl groups or phenyl groups include compounds such as 1,10-phenanthroline, 4,7-dimethyl-1,10-phenanthroline, 3,4,7,8-tetramethyl-1,10-phenanthroline, 2,9-dimethyl-4,7-diphenyl-1,10-phenanthroline, 4,7-diphenyl-1,10-phenanthroline and 2,9-diphenyl-1,10-phenanthroline.

Specific examples of the bipyridine optionally substituted with one or more methyl groups or phenyl groups include compounds such as 2,2'-bipyridine, 5,5'-dimethyl-2,2'-bipyridine, 4,4'-dimethyl-2,2'-bipyridine, 6,6'-dimethyl-2,2'-bipyridine, 4,4'-diphenyl-2,2'-bipyridine, 5,5'-dimethyl-2,2'-bipyridine and 4,4',5,5'-tetramethyl-2,2'-bipyridine.

The C4 to C8 alkyl group may be any of a linear-chain, branched or cyclic alkyl group. Specific examples include groups such as a butyl group, a 1-methylpropyl group, a 2-methylpropyl group, a tert-butyl group, a cyclobutyl group, a pentyl group, a cyclopentyl group, a 5-dimethylcyclopentyl group, a 3-ethylcyclopentyl group, a hexyl group, a cyclohexyl group, a 4-ethylcyclohexyl group, a 4,4-dimethylcyclohexyl group, a 2,6-dimethylcyclohexyl group, a 3,5-dimethylcyclohexyl group, a heptyl group, a cycloheptyl group, an octyl group and a cyclooctyl group. A butyl group, tert-butyl group, octyl group, cyclopentyl group or cyclohexyl group is preferred because of easy availability of raw materials, and a butyl group, octyl group or cyclohexyl group is more preferred because of the ease of synthesis.

Examples the C1 to C4 alkylene group include groups such as a methylene group, an ethylene group, a trimethylene group, a tetramethylene group, a 2-methylpropan-1,3-diyl group, a butan-1,2-diyl group, a butan-2,3-diyl group and a 2-methylpropan-2,3-diyl group. A methylene group, ethylene group or trimethylene group is preferred because of easy availability of raw materials.

X² in general formula (3d), furthermore, is preferably a hydrogen atom, C1 to C4 alkyl group, C1 to C4 alkyloxy group or phenyl group optionally substituted with one or more C1 to C4 alkyl groups or C1 to C4 alkyloxy groups because of easy availability of raw materials.

Examples of the C1 to C4 alkyl group include the groups listed as examples in the context of the C1 to C4 alkyl groups of a methylene group at W¹ optionally substituted with one or more C1 to C4 alkyl groups.

The C1 to C4 alkyloxy group may be any of a linear-chain, branched or cyclic alkyl group. Specific examples include a methoxy group, an ethoxy group, a propyloxy group, an isopropyloxy group, a butyloxy group, a 2-methylpropyloxy group, a cyclopropyloxy group, a tert-butyloxy group and a cyclobutyloxy group.

Examples of the phenyl group optionally substituted with one or more C1 to C4 alkyl groups or C1 to C4 alkyloxy groups include groups such as a phenyl group, a 2-methylphenyl group, a 3-methylphenyl group, a 4-methylphenyl group, a 2,5-dimethylphenyl group, a 2,4,6-trimethylphenyl group, a 4-ethylphenyl group, a 4-propylphenyl group, a 4-butylphenyl group, a 2,4,6-triisopropylphenyl group, a 1-methylpropylphenyl group, a 2-methylpropylphenyl group, a 4-tert-butylphenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-(isopropyloxy)phenyl group, a 4-(isopropyloxy)phenyl group, a 2,4,6-(triisopropyloxy)phenyl group, a 1-methylpropyloxyphenyl group, a 2-methylpropyloxyphenyl group, a 4-(tert-butyloxy)phenyl group, a 4-(cyclopropyloxy)phenyl group and a 4-(cyclobutyloxy)phenyl group. The present invention is not limited to these substituents listed by way of example.

### <L²>

L² in general formula (1) represents a neutral ligand selected from the group consisting of water, deuterium oxide, sulfoxide compounds, sulfone compounds, amide compounds, nitrile compounds, ester compounds, carbonyl compounds, ether compounds and alcohols. When this neutral ligand in the rare earth complex (1) according to the present invention is water, deuterium oxide, a sulfoxide compound, a sulfone compound, an amide compound, an ester compound, a carbonyl compound, an ether compound or an alcohol, a lone pair on an oxygen atom in the neutral ligand coordinates with the rare earth metal ion, represented by M³⁺. When the neutral ligand is a nitrile compound, the lone pair on the nitrogen atom in the cyano group coordinates with the rare earth metal ion.

When L² is a sulfoxide compound, specific examples of the sulfoxide compound include compounds such as dimethylsulfoxide and deuterated dimethylsulfoxide. The present invention is not limited to these.

When L² is a sulfone compound, specific examples of the sulfone compound include compounds such as dimethyl sulfone, diphenyl sulfone and sulfolane. The present invention is not limited to these.

When L² is an amide compound, specific examples of the amide compound include compounds such as N,N-dimethylformamide, N,N-dimethylacetamide, 1,3-dimethyl-2-imidazolidinone and 1,3-dimethyl-3,4,5,6-tetrahydro-2(1H)-pyrimidinone. The present invention is not limited to these.

When L² is a nitrile compound, specific examples of the nitrile compound include compounds such as acetonitrile, deuterated acetonitrile, propionitrile and benzonitrile. The present invention is not limited to these.

When L² is an ester compound, specific examples of the ester compound include compounds such as ethyl acetate and ethyl benzoate. The present invention is not limited to these.

When L² is a carbonyl compound, specific examples of the carbonyl compound include compounds such as acetone, deuterated acetone, acetophenone, methyl ethyl ketone, benzaldehyde, acetaldehyde and formaldehyde. The present invention is not limited to these.

When L² is an ether compound, specific examples of the ether compound include compounds such as diethyl ether, tetrahydrofuran and tetrahydrofuran-d₈. The present invention is not limited to these.

When L² is an alcohol, specific examples of the alcohol include compounds such as methanol, deuterated methanol, propanol, isopropyl alcohol, butanol and tert-butanol. The present invention is not limited to these.

L² in general formula (1) is preferably acetone, deuterated acetone, acetonitrile, deuterated acetonitrile, methanol, deuterated methanol, ethanol, propanol, isopropyl alcohol, dimethylsulfoxide, deuterated dimethylsulfoxide, water or deuterium oxide because of easy availability of raw materials, more preferably water because of the ease of synthesis.

### <m¹ and m²>

m¹ is an integer of 0, 1 or 2, m² is 0 to 3, and 0 ≤ m¹ + m² ≤ 3. For m¹ and m², it is preferred that m¹ be 1 or 2 and that m² be 0 because these give the complex optical characteristics suitable for use as an optical functional material.

### <XL>

X^{L} in general formula (1) represents a halide ion, nitrate ion, carboxylate ion, sulfonate ion or C5 to C12 β-diketonato ion.

Specific examples of halide ions represented by X^{L} include a fluoride ion, a chloride ion, a bromide ion or an iodide ion.

Specific examples of carboxylate ions represented by X^{L} include; saturated aliphatic carboxylate ions, such as an acetate ion, a propionate ion, a butyrate ion, an isobutyrate ion, a valerate ion, a pivalate ion or a stearin ion; aromatic carboxylate ions, such as a benzoate ion or p-toluate ion; unsaturated aliphatic carboxylate ions, such as an acrylate ion or methacrylate ion; or; heteroaromatic carboxylate ions, such as a nicotinate ion or isonicotinate ion. The present invention is not limited to these.

Specific examples of sulfonate ions represented by X^{L} include; organic sulfonate ions, such as a methanesulfonate ion, p-toluenesulfonate ion, benzenesulfonate ion or trifluoromethanesulfonate ion; or; inorganic sulfate ions, such as a hydrogen sulfate ion. The present invention is not limited to these.

Specific examples of C5 to C12 β-diketonato ions represented by X^{L} include an acetylacetonato ion (acac), a hexafluoroacetylacetonato ion (hfa), a dibenzoylmethanato ion (dbm), a thenoyltrifluoroacetonato ion (tta) and a 2-acetyl-5,5-dimethyl-1,3-cyclohexanedionato ion. The present invention is not limited to these.

### <M3+>

M³⁺ in general formula (1) represents a trivalent rare earth metal ion, and specific examples include a scandium(III) ion, a yttrium(III) ion, a lanthanum(III) ion, a cerium(III) ion, a praseodymium(III) ion, a neodymium(III) ion, a promethium(III) ion, a samarium(III) ion, a europium(III) ion, a gadolinium(III) ion, a terbium(III) ion, a dysprosium(III) ion, a holmium(III) ion, an erbium(III) ion, a thulium(III) ion, a ytterbium(III) ion and a lutetium(III) ion.

M³⁺ in general formula (1) is preferably a europium(III) ion, terbium(III) ion, samarium(III) ion or gadolinium(III) ion because of the ease of synthesis, more preferably a europium(III) ion because this gives the complex optical characteristics suitable for use as an optical functional material.

Examples of preferred embodiments of the rare earth complex (1) according to the present invention include rare earth complexes in which the quinolinonato ligand (1qu) in the rare earth complex (1) is indicated by general formulae (1qu-1) to (1qu-8) below.

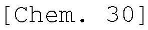

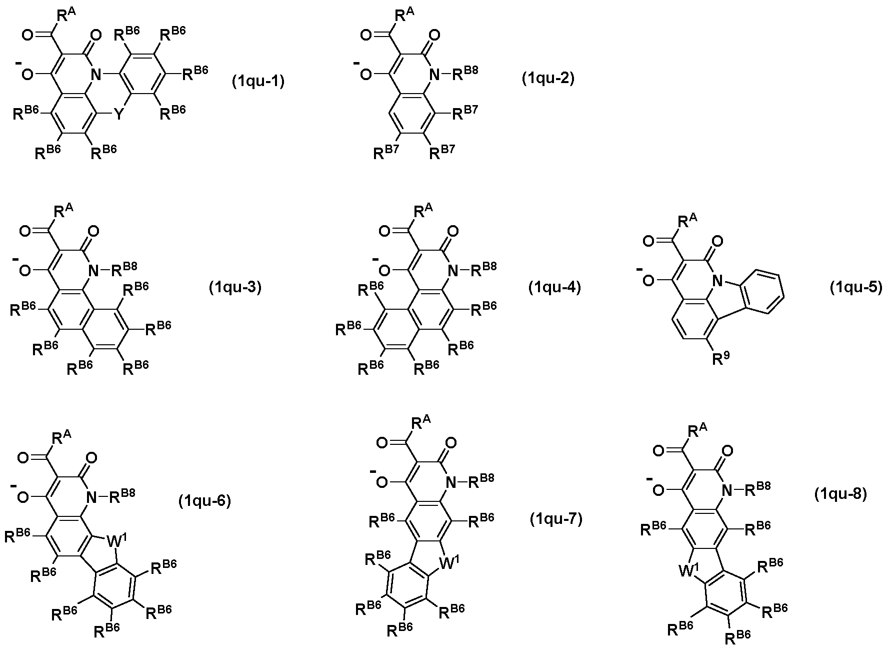

(In the formulae, R^{A}, R^{B6}, R^{B8}, W¹ and Y are defined the same as above.

R^{B7} represents a hydrogen atom, halogen atom, cyano group, C1 to C8 alkyl group, C1 to C4 fluoroalkyl group, C6 to C22 aryl group, substituted or unsubstituted amino group indicated by general formula (BA3) below or substituted or unsubstituted oxy group indicated by general formula (BOS) below.)

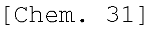

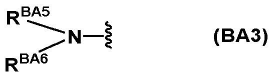

(In the formula, R^{BA5} and R^{BA6} each independently represent a C1 to C4 alkyl group or C6 to C12 aryl group, and the alkyl group and the aryl group may be substituted with one or more substituents selected from the group consisting of halogen atoms, C1 to C4 alkyloxy groups, C1 to C4 fluoroalkyl groups, C2 to C13 acyl groups, a cyano group and a nitro group. R^{BA5} and R^{BA6}, furthermore, may form a five-membered, six-membered or seven-membered ring including the nitrogen atom to which they are bound.)

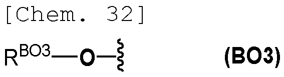

(In the formula, R^{BO3} represents a C1 to C4 alkyl group or C6 to C12 aryl group, and the alkyl group and the aryl group may be substituted with one or more substituents selected from the group consisting of halogen atoms, C1 to C4 alkyloxy groups, C1 to C4 fluoroalkyl groups, C2 to C13 acyl groups, a cyano group and a nitro group.)

Adjacent R^{B7}s, furthermore, may form a five-membered, six-membered or seven-membered ring together with the benzene ring to which they are bound.

R^{B9} represents a hydrogen atom or diphenylamino group optionally substituted with one or more fluorine atoms or C1 to C4 fluoroalkyl groups.)

### (R^{B7})

Examples of halogen atoms represented by R^{B7} include the substituents listed as examples of halogen atoms that belong to group T₂1.

A C1 to C8 alkyl group represented by R^{B7} may be any of a linear-chain, branched or cyclic alkyl group. Specific examples include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a 5-dimethylcyclopentyl group, a 3-ethylcyclopentyl group, a hexyl group, a cyclohexyl group, a 4-ethylcyclohexyl group, a 4-propylcyclohexyl group, a 4,4-dimethylcyclohexyl group, a 2,6-dimethylcyclohexyl group, a 3,5-dimethylcyclohexyl group, a cycloheptyl group, an octyl group and a cyclooctyl group. The present invention is not limited to these substituents listed by way of example.

Examples of C1 to C4 fluoroalkyl groups represented by R^{B7} include the groups listed as examples of C1 to C4 fluoroalkyl groups represented by R^{B6}.

Specific examples of C6 to C22 aryl groups represented by R^{B7} include groups such as a phenyl group, a naphthyl group, a methylphenyl group, a dimethylphenyl group, a trimethylphenyl group, an ethylphenyl group, a propylphenyl group, a triisopropylphenyl group, a tert-butylphenyl group, a methylnaphthyl group, a biphenylyl group, a methylbiphenylyl group, a phenanthrenyl group, a methylphenanthrenyl group, a dimethylphenanthrenyl group and an anthracenyl group. The present invention is not limited to these substituents listed by way of example.

The R^{B7}s, furthermore, each independently represent a substituted or unsubstituted amino group indicated by general formula (BA3) below or substituted or unsubstituted oxy group indicated by general formula (BOS) below.

Examples of C1 to C4 alkyl groups represented by R^{BA5} and R^{BA6} in general formula (BA3) or R^{BO3} in general formula (BOS) include the groups listed as examples of the C1 to C4 alkyl groups of a methylene group at W¹ optionally substituted with one or more C1 to C4 alkyl groups.

Examples of C6 to C12 aryl groups represented by R^{BA5} and R^{BA6} in general formula (BA3) or R^{BO3} in general formula (BOS) include the groups listed as examples of the C6 to C12 aryl group of a nitrogen atom at W¹ optionally substituted with a C6 to C12 aryl group.

A C1 to C4 alkyl or C6 to C12 aryl group represented by R^{BA5} and R^{BA6} in general formula (BA3), moreover, may be substituted with one or more substituents selected from group T₂3.

A C1 to C4 alkyl or C6 to C12 aryl group represented by R^{BO3} in general formula (BO3), furthermore, may be substituted with one or more substituents selected from group T₂3.

Examples of halogen atoms that belong to group T₂3 include the groups listed as examples of halogen atoms that belong to group T₂1.

Examples of C1 to C4 alkyl groups that belong to group T₂3 include the groups listed as examples in the context of the C1 to C4 alkyl groups of a methylene group at W¹ optionally substituted with one or more C1 to C4 alkyl groups.

Examples of C1 to C4 alkyloxy groups that belong to group T₂3 include the groups listed as examples of C1 to C4 alkyloxy groups at X² in general formula (3d).

Examples of C1 to C4 fluoroalkyl groups that belong to group T₂3 include the groups listed as examples of C1 to C4 fluoroalkyl groups represented by R^{B6}.

Examples of C2 to C13 acyl groups that belong to group T₂3 include the substituents listed as examples of C2 to C13 acyl groups that belong to group T₂1.

Specific examples of C1 to C4 alkyl groups at R^{BA5} and R^{BA6} optionally substituted with one or more substituents selected from group T₂3 and C1 to C4 alkyl groups at R^{BO3} optionally substituted with one or more substituents selected from group T₂3, therefore, include groups such as a 2,2,2-trifluoroethyl group, a 2-methoxyethyl group and a 3-methoxypropyl group. The present invention is not limited to these substituents listed by way of example.

Examples of C6 to C12 aryl groups at R^{BA5} and R^{BA6} optionally substituted with one or more substituents selected from group T₂3 and C6 to C12 aryl groups at R^{BO3} optionally substituted with one or more substituents selected from group T₂3, furthermore, include the groups listed as examples of the C6 to C12 aryl group of a nitrogen atom at W¹ optionally substituted with a C6 to C12 aryl group.

In addition, R^{BA5} and R^{BA6} may form a five-membered, six-membered or seven-membered ring including the nitrogen atom to which they are bound. Specific examples include groups such as an azetidyl group, a pyrrolidinyl group, a piperidino group, an azepanyl group, a morpholino group, a 4-methylpiperazinyl group, a carbazoyl group, a phenoxazinyl group, a phenothiazinyl group and a 10,11-dihydro-5H-dibenz[b,f]azepinyl group. The present invention is not limited to these substituents listed by way of example.

A substituted or unsubstituted amino group indicated by general formula (BA3), therefore, may be substituted with one or more substituents selected from group T₂3, and specific examples include the substituents listed as examples for a substituted or unsubstituted amino group indicated by general formula (BA1).

A substituted or unsubstituted oxy group indicated by general formula (BO3), furthermore, may be substituted with one or more substituents selected from group T₂3, and specific examples include the substituents listed as examples for a substituted or unsubstituted amino group indicated by general formula (BO1).

R^{B7} is preferably a hydrogen atom, C1 to C4 alkyl group, C1 to C4 alkyloxy group, C2 to C8 dialkylamino group or diphenylamino group optionally substituted with one or more fluorine atoms; C1 to C4 fluoroalkyl groups; C2 to C5 acyl groups; or; cyano groups, more preferably a hydrogen atom, C1 to C4 alkyl group, C1 to C4 alkyloxy group, C2 to C8 dialkylamino group or diphenylamino group optionally substituted with one or more fluorine atoms; C1 to C4 fluoroalkyl groups; or; cyano groups, particularly preferably a hydrogen atom or diphenylamino group optionally substituted with one or more fluorine atoms; or; cyano groups because of the ease of synthesis.

Examples of the C1 to C4 alkyl group include the groups listed as examples in the context of the C1 to C4 alkyl groups of a methylene group at W¹ optionally substituted with one or more C1 to C4 alkyl groups.

Examples of the C1 to C4 alkyloxy group include the groups listed as examples of C1 to C4 alkyloxy groups at X² in general formula (3d).

Specific examples of the C2 to C8 dialkylamino group include groups such as a dimethylamino group, a diethylamino group, a dipropylamino group, a diisopropylamino group, a dibutylamino group, a bis(2-methylpropyl)amino group, a bis(2,2-dimethylpropyl)amino group, a bis(3-cyclopropyl)amino group, a dicyclopropylamino group, a bis(2-methylbutyl)amino group, a bis(3-methylbutyl)amino group, a di(tert-butyl)amino group, a dicyclobutylamino group, an N-ethyl-N-methylamino group, an N-methyl-N-propyl group, an N-cyclopropyl-N-methylamino group, an N-butyl-N-methylamino group, an N-cyclobutyl-N-methylamino group, an N-methyl-N-pentyl group, an N-cyclopentyl-N-methylamino group, an N-hexyl-N-methylamino group, an N-cyclohexyl-N-methylamino group and an N-cycloheptyl-N-methylamino group.

The C2 to C5 acyl groups may be any of linear-chain, branched or cyclic. Specific examples include groups such as an acetyl group, an ethylcarbonyl group, a 1-propylcarbonyl group, an isopropylcarbonyl group and a tert-butylcarbonyl group. The present invention is not limited to these substituents listed by way of example.

The diphenylamino group may be substituted with one or more fluorine atoms; C1 to C4 fluoroalkyl groups; or; cyano groups, and specific examples include groups such as a diphenylamino group, a bis(4-fluorophenyl)amino group, a bis(3,4-difluorophenyl)amino group, a bis(3,5-difluorophenyl)amino group, a bis[4-(trifluoromethyl)phenyl]amino group, a bis[4-(perfluoropropyl)phenyl]amino group, a bis[4-(perfluorobutyl)phenyl]amino group, a bis(4-cyanophenyl)amino group and a bis(3,5-dicyanophenyl)amino group. The present invention is not limited to these substituents listed by way of example.

### (R^{B9})

A diphenylamino group represented by R^{B9} may be substituted with one or more fluorine atoms or C1 to C4 fluoroalkyl groups, and specific examples include groups such as a diphenylamino group, a bis(4-fluorophenyl)amino group, a bis(3,4-difluorophenyl)amino group, a bis(3,5-difluorophenyl)amino group and a bis[4-(trifluoromethyl)phenyl]amino group. The present invention is not limited to these substituents listed by way of example.

R^{B9} is preferably a hydrogen atom, diphenylamino group or bis(4-fluorophenyl)amino group because of the ease of synthesis, more preferably a hydrogen atom or diphenylamino group because of easy availability of raw materials.

The rare earth complex (1) according to the present invention may contain a solvent of crystallization formed during purification, for example by reprecipitation or recrystallization.

Specific examples of rare earth complexes (1) according to the present invention include the rare earth complexes represented by formulae (1b-1) to (1b-204) and formulae (1baq-1) to (1baq-93) below, but the present invention is not limited to these. Herein, Ph represents a phenyl group, Ac represents an acetyl group, Me represents a methyl group, Et represents an ethyl group, iPr represents an isopropyl group, and tBu represents a tert-butyl group.

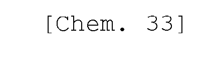

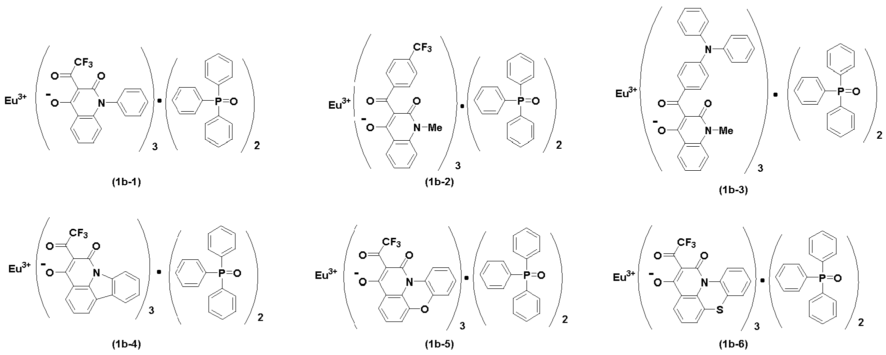

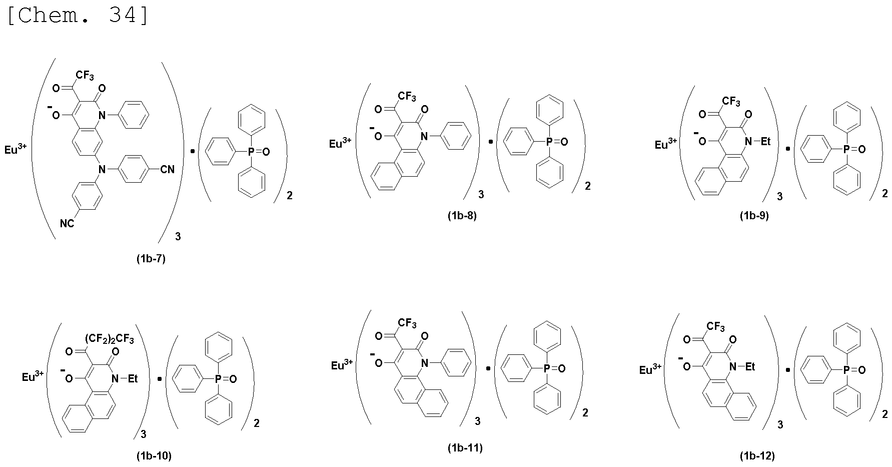

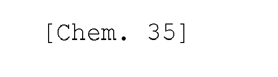

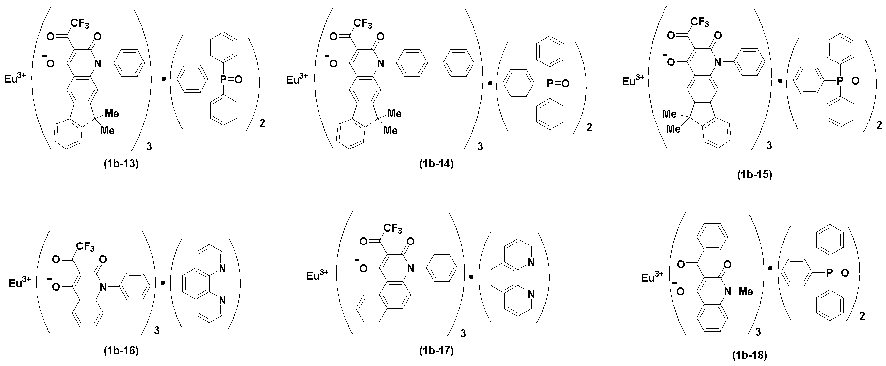

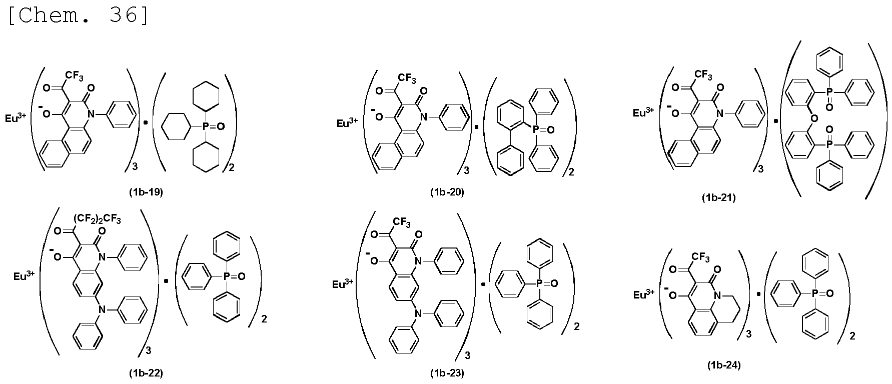

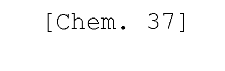

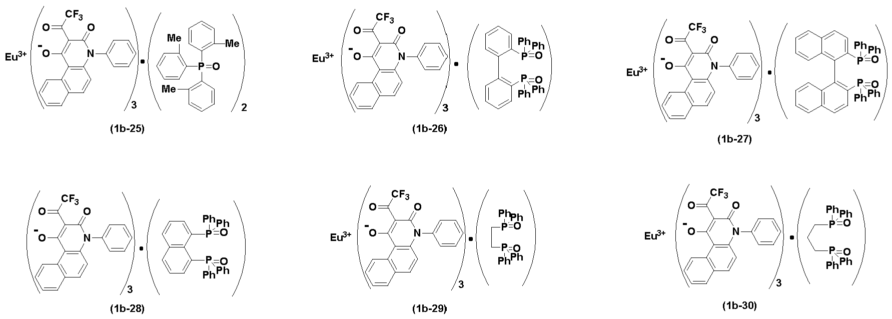

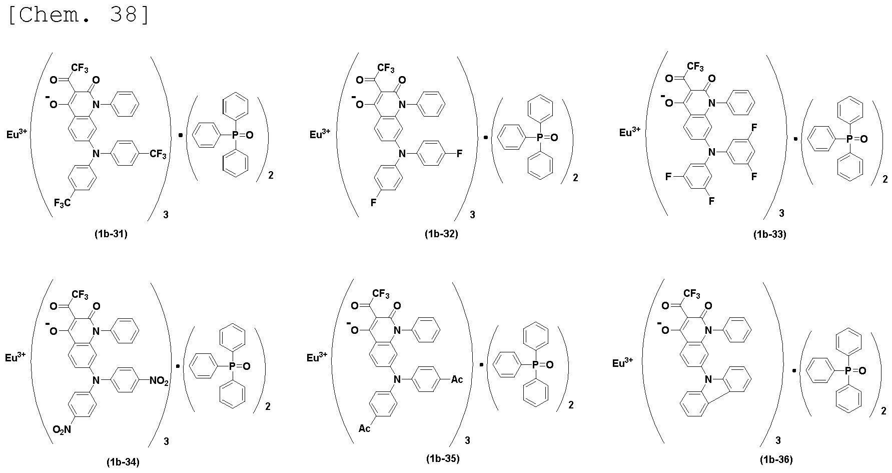

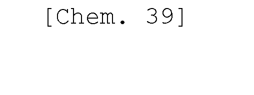

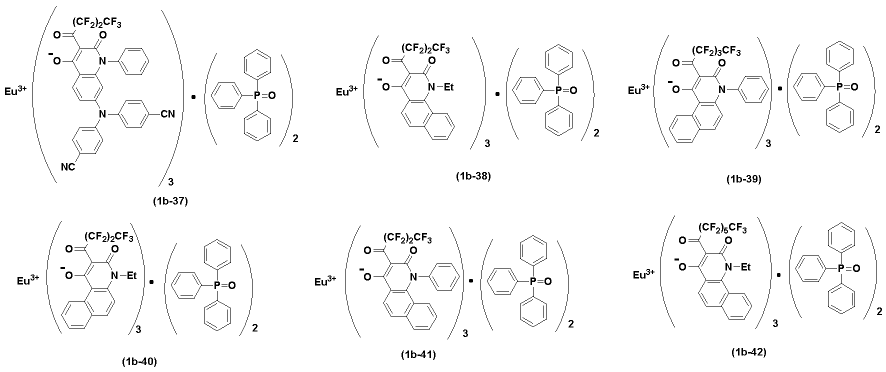

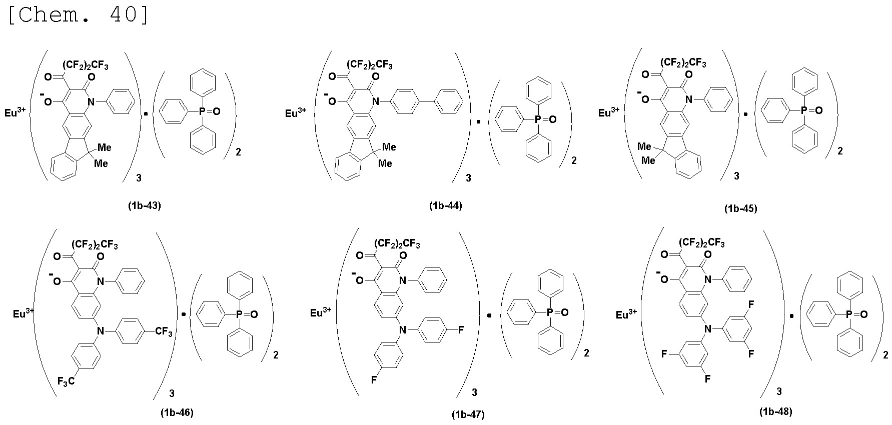

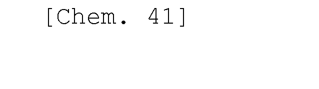

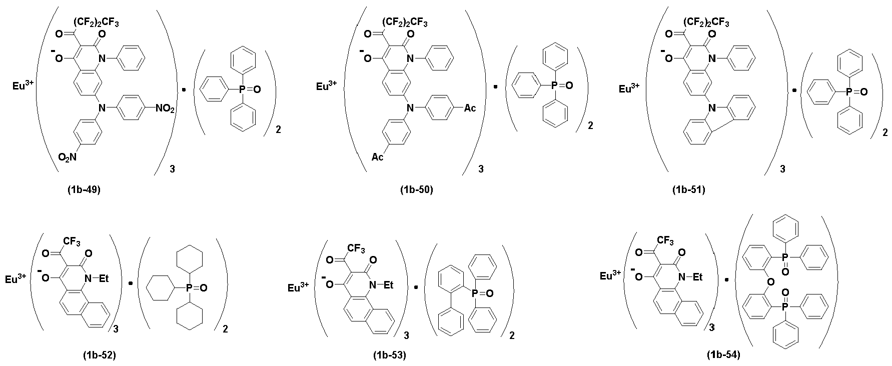

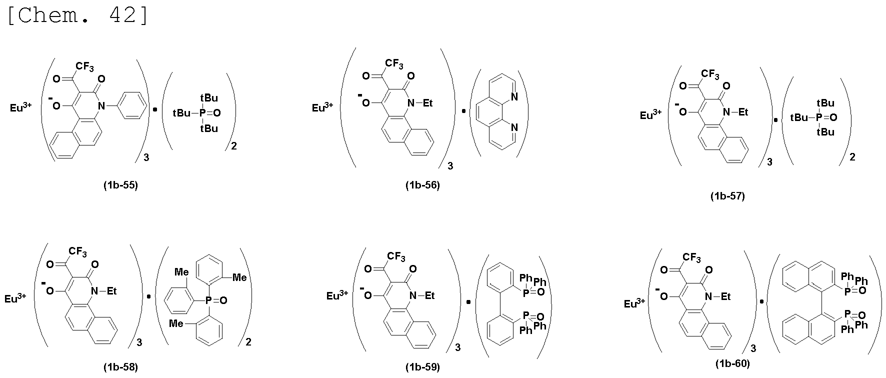

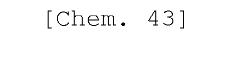

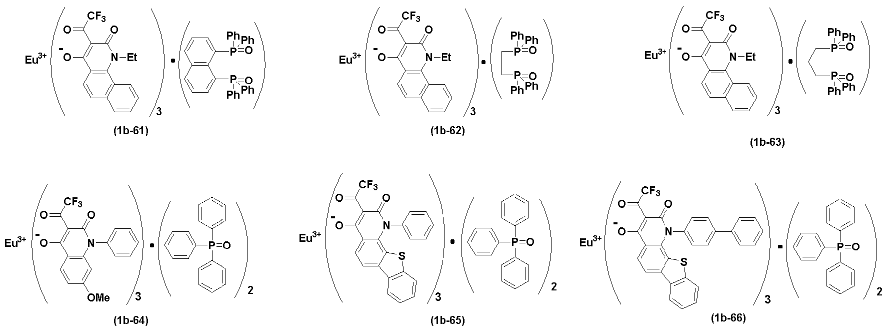

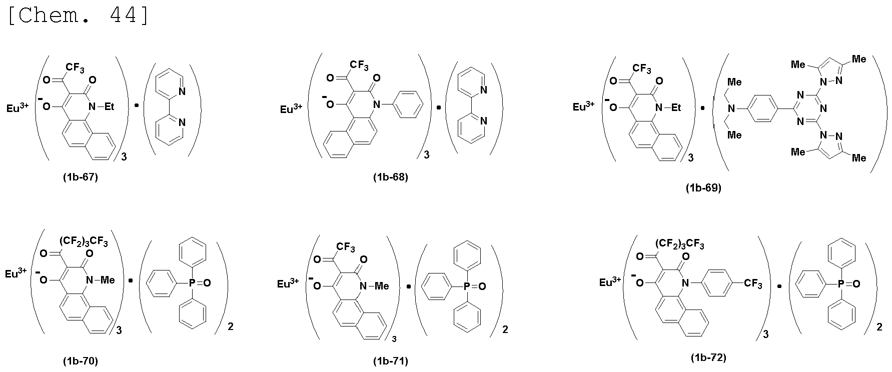

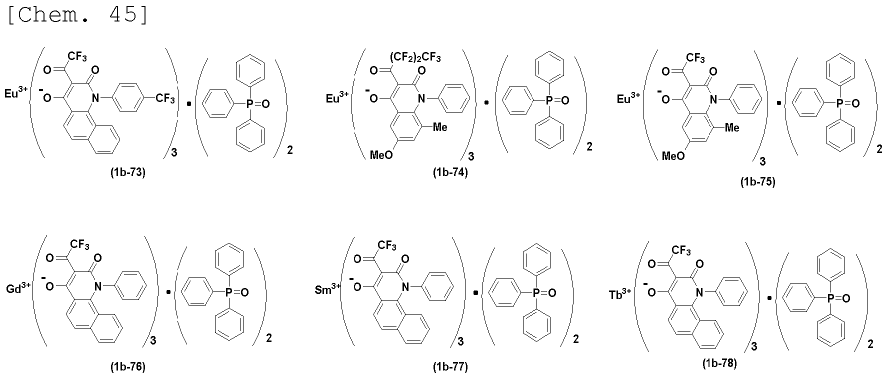

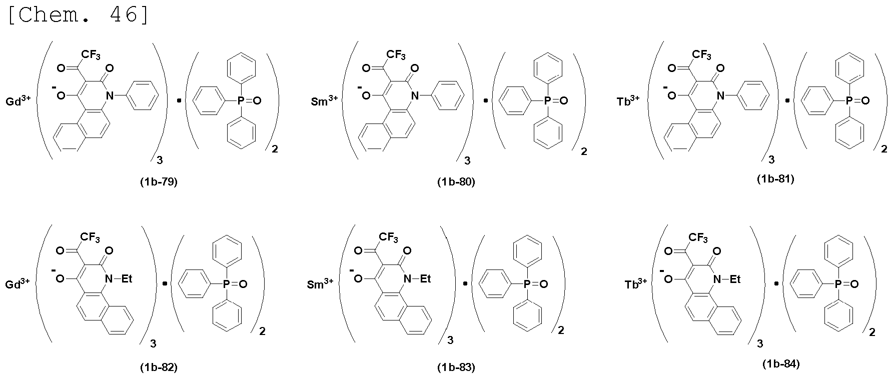

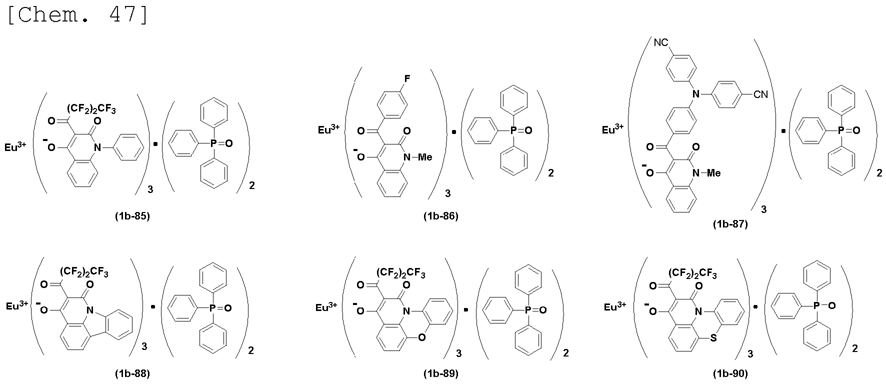

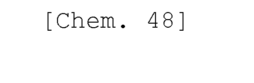

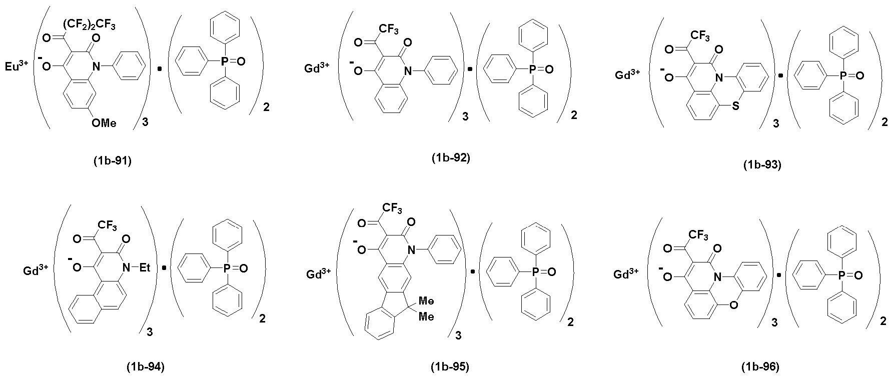

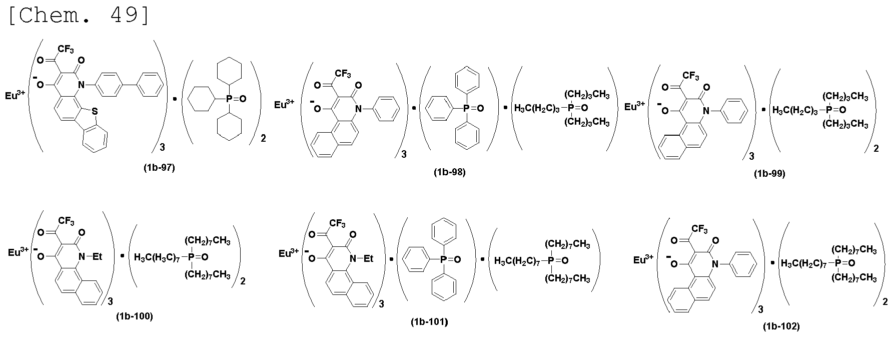

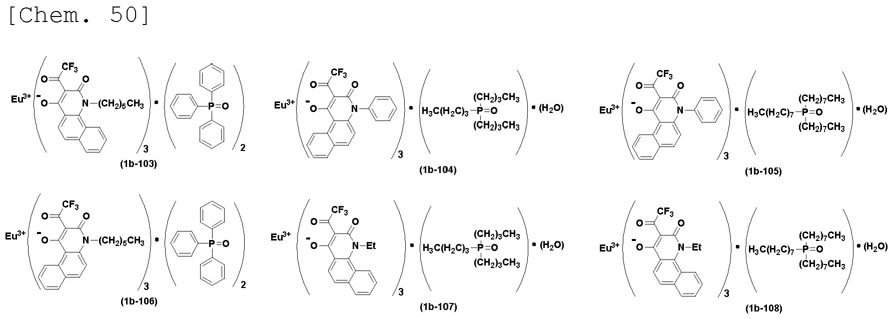

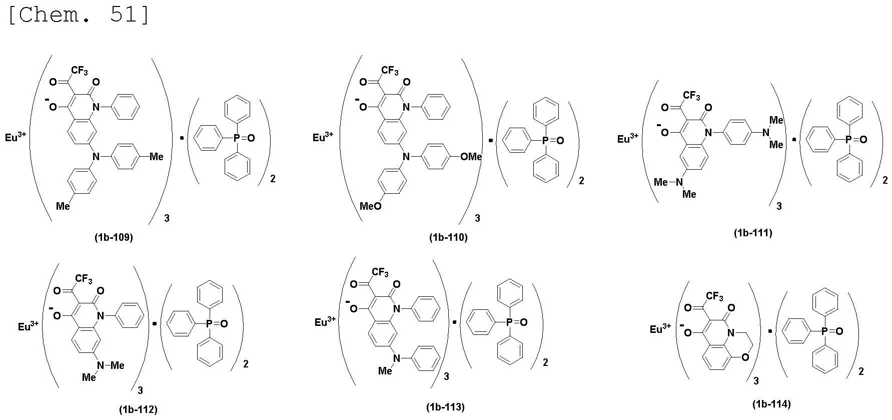

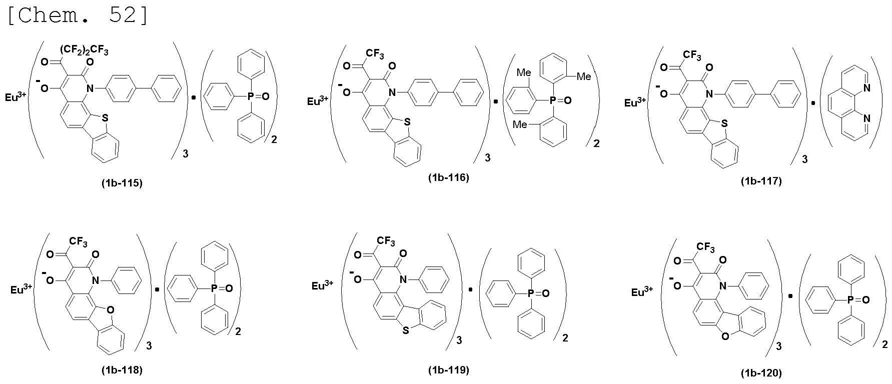

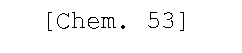

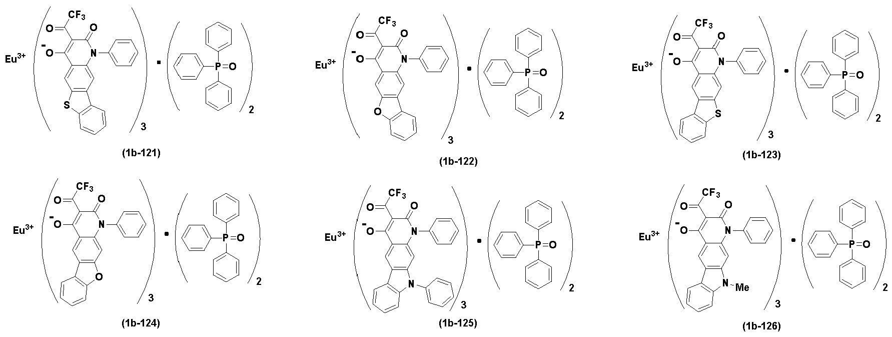

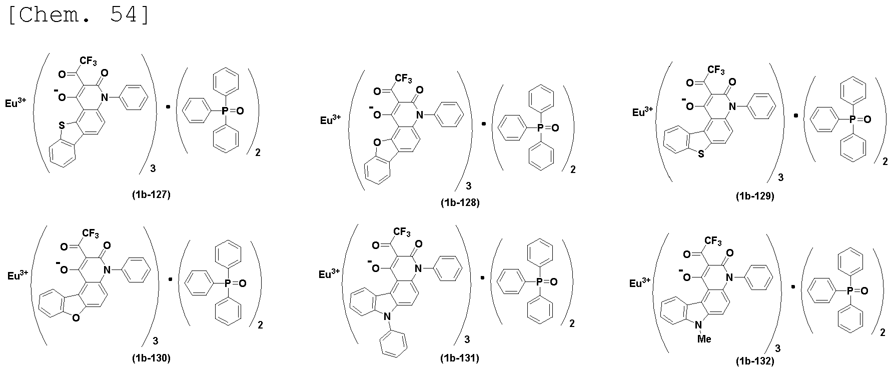

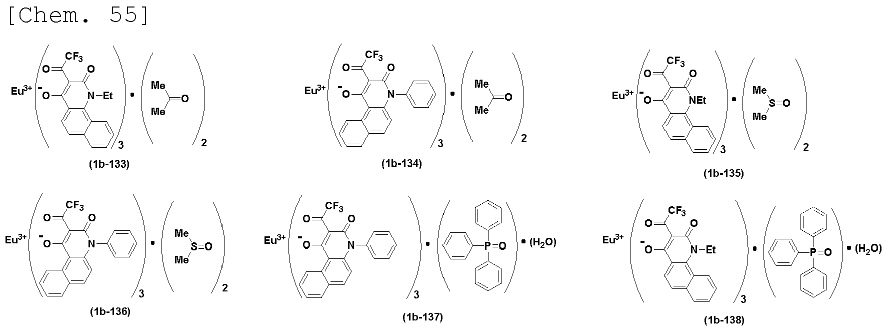

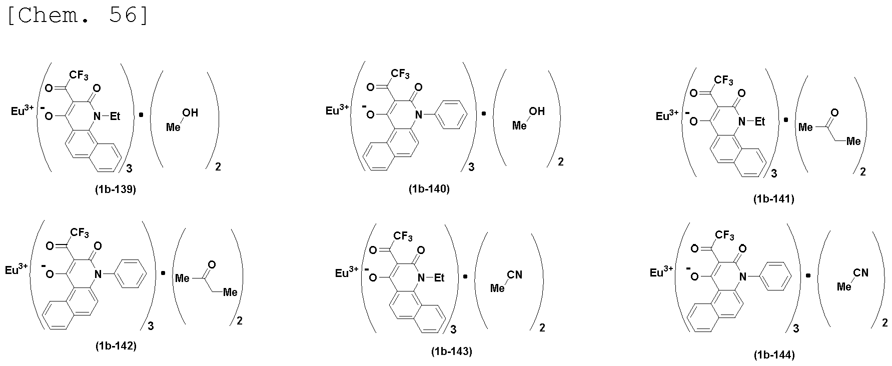

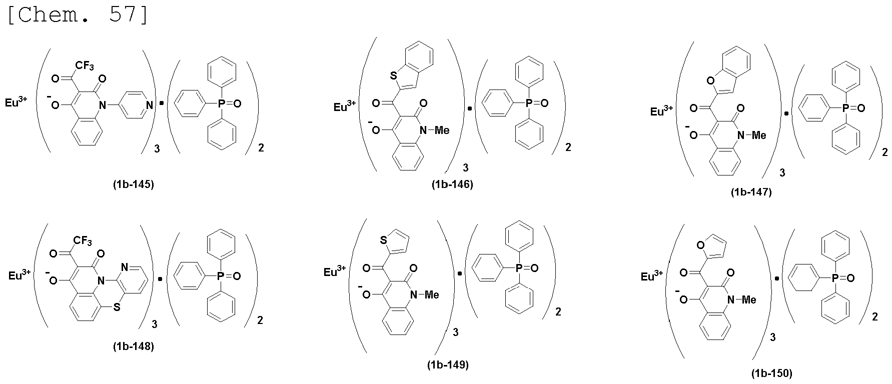

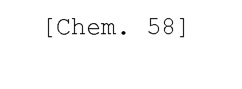

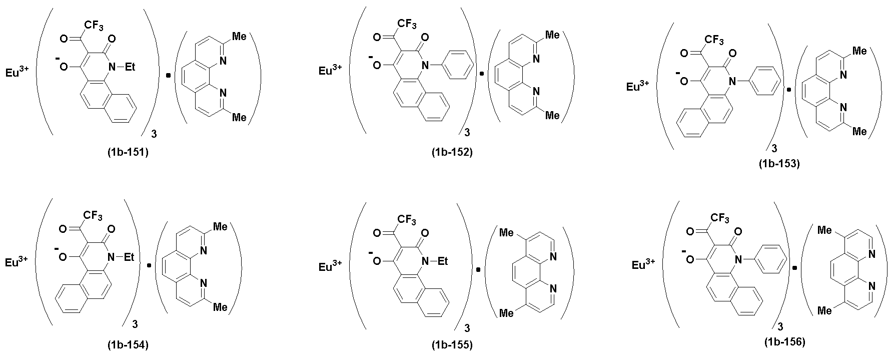

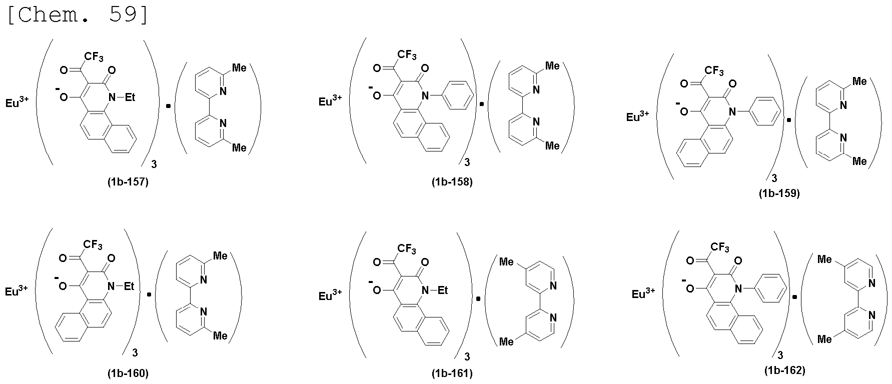

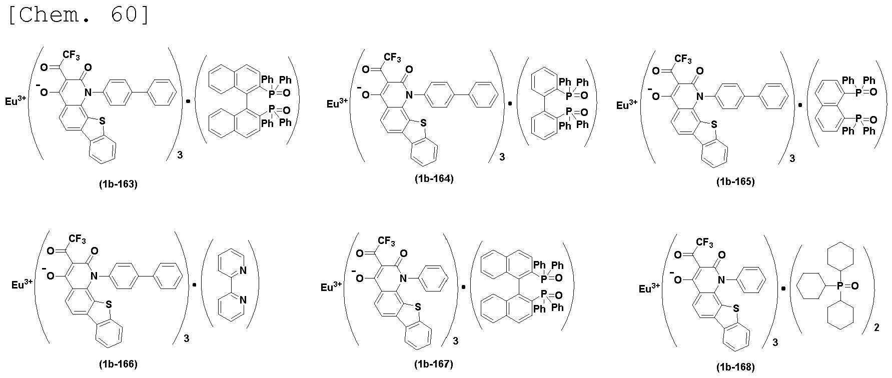

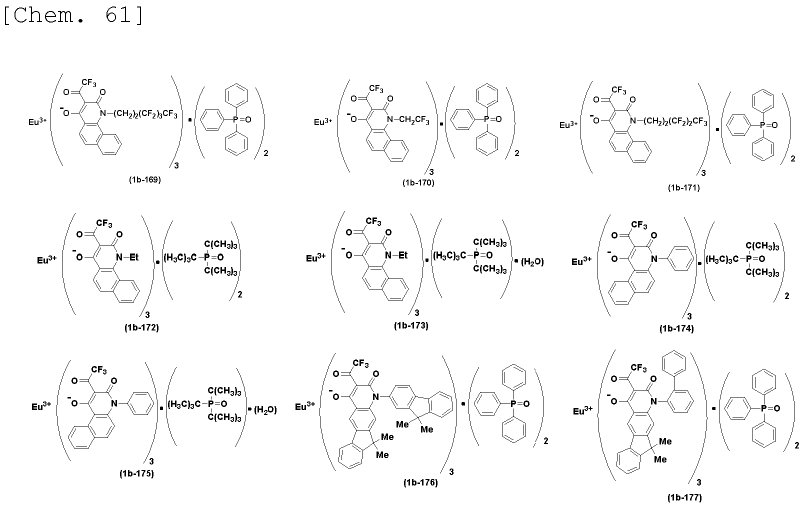

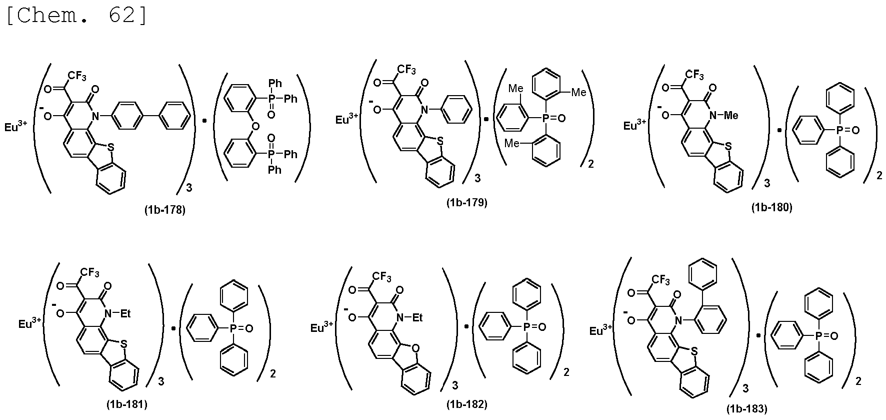

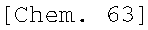

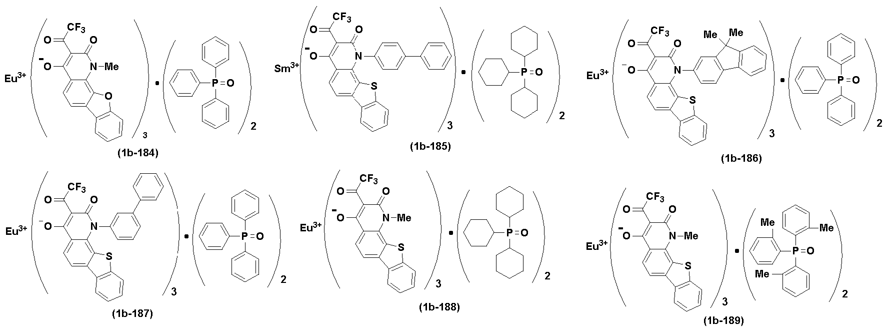

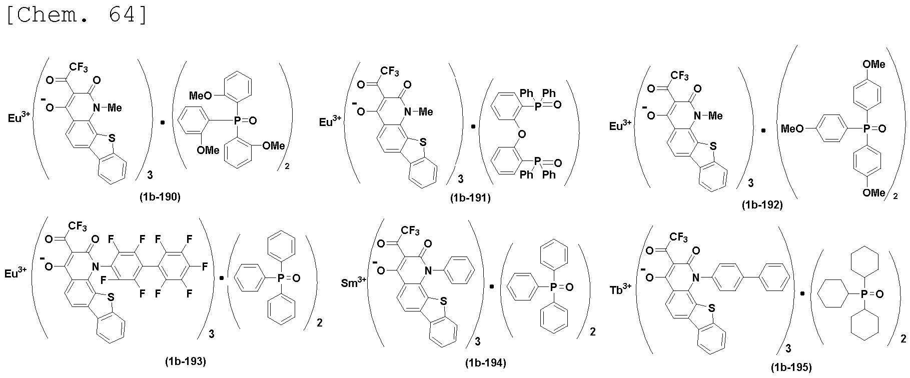

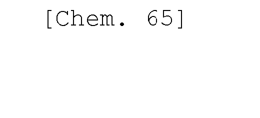

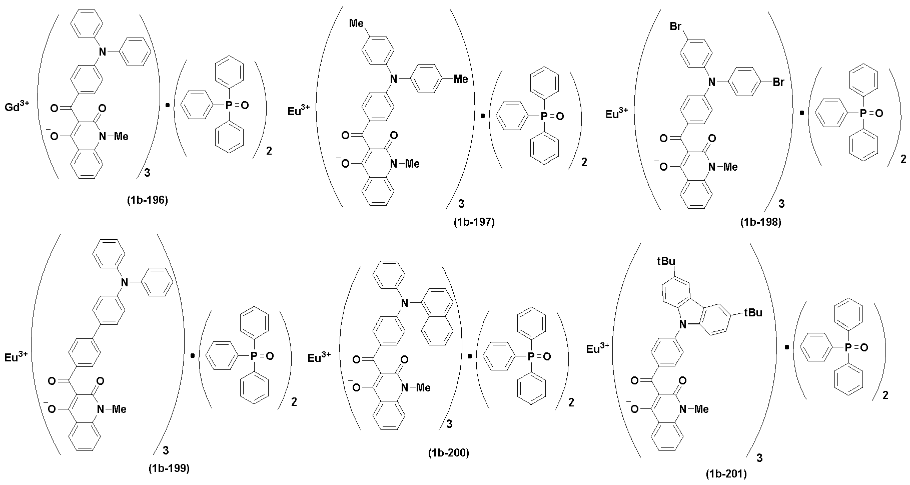

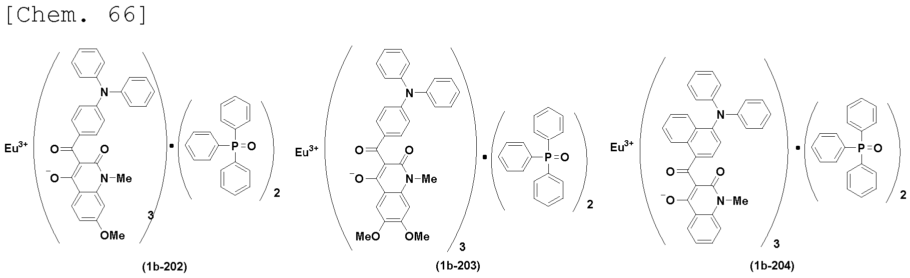

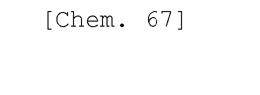

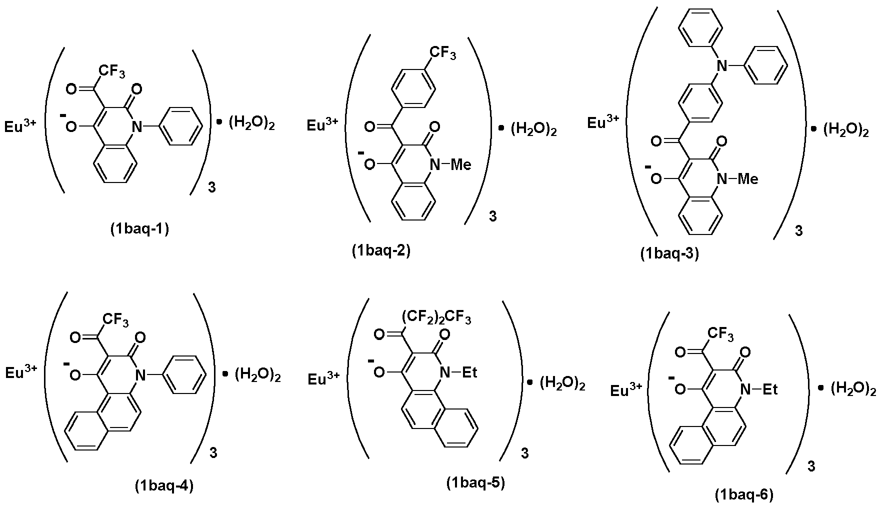

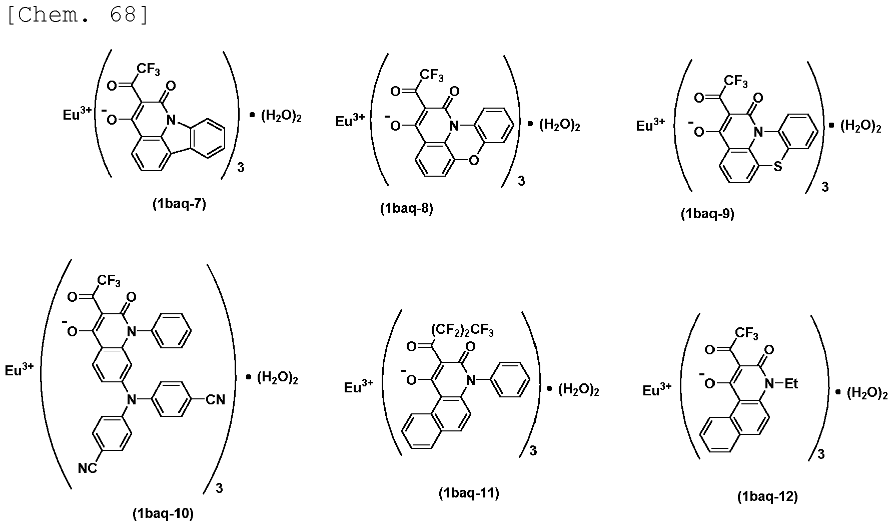

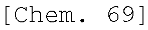

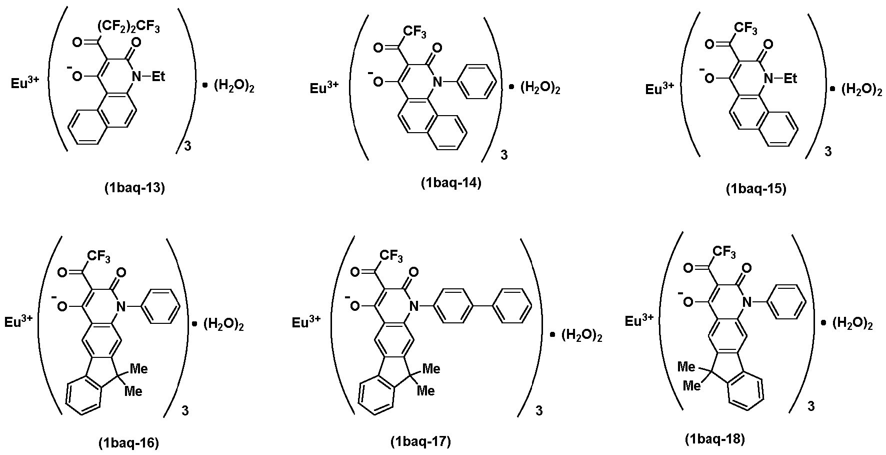

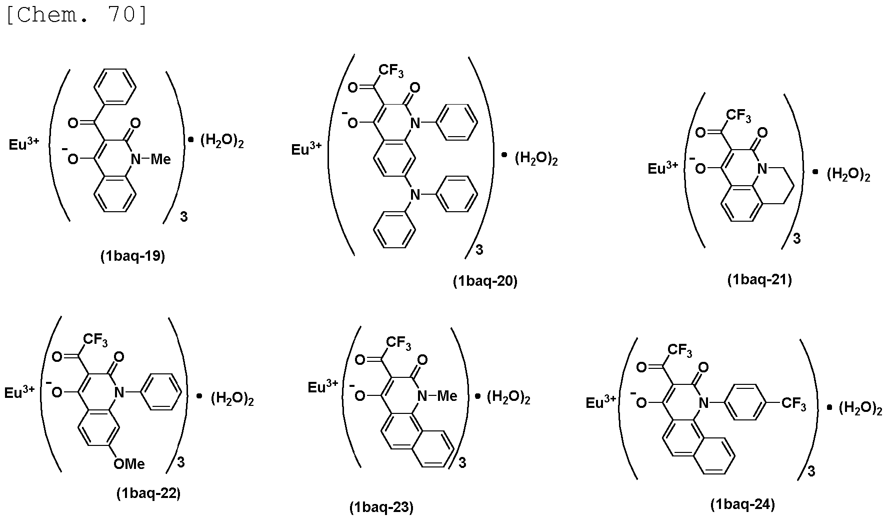

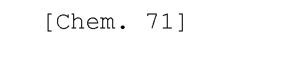

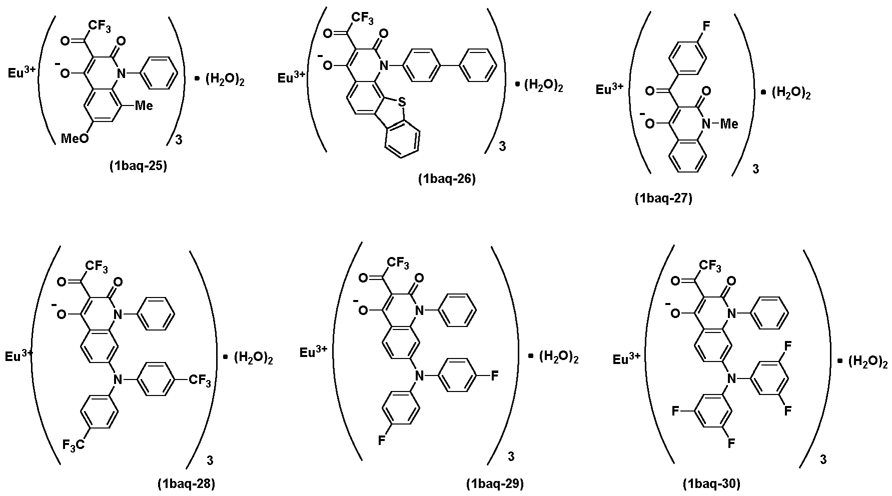

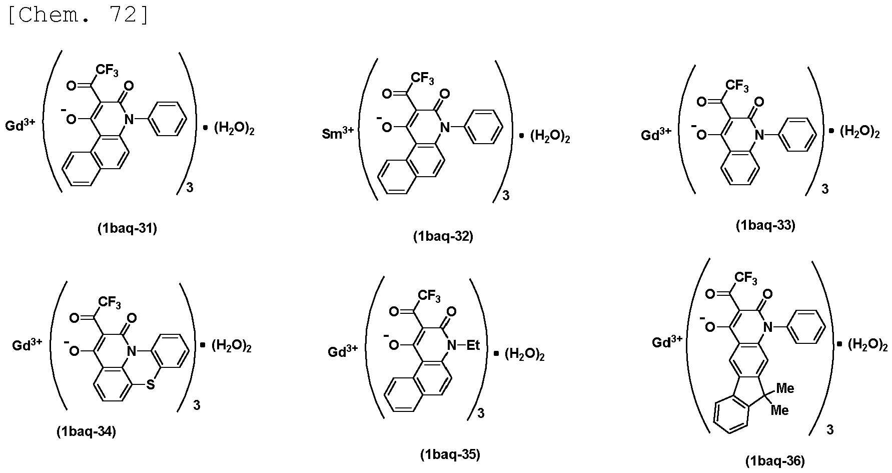

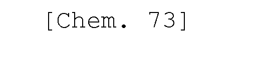

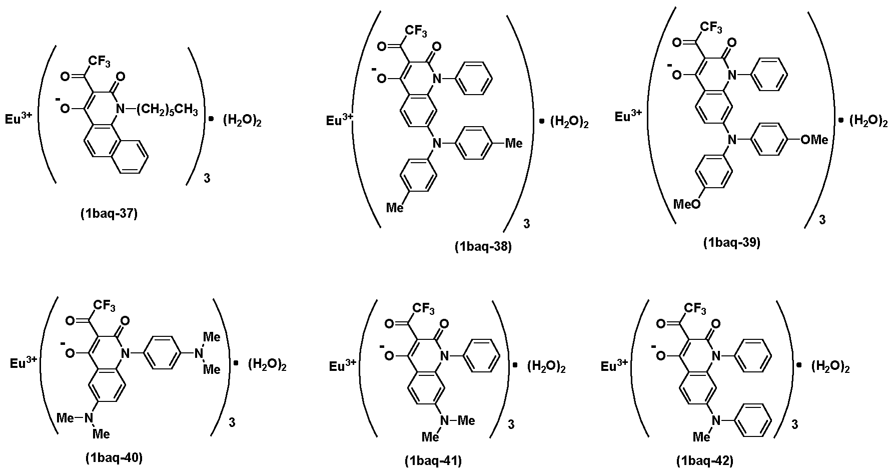

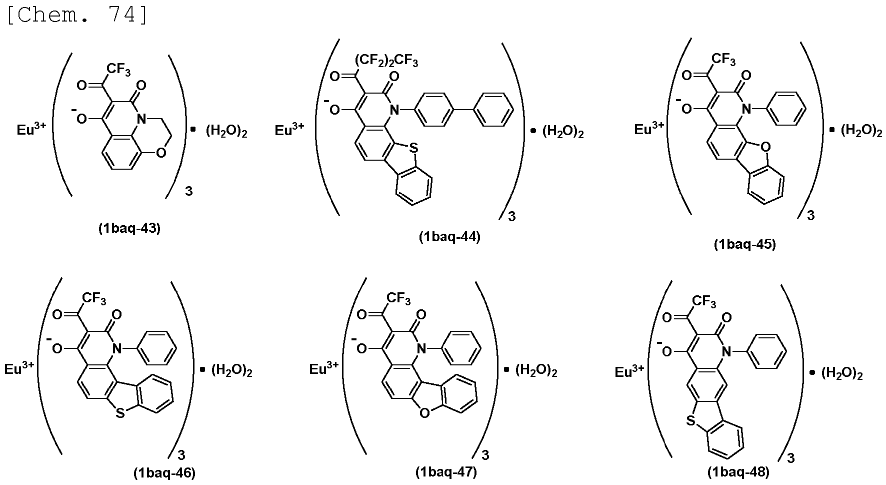

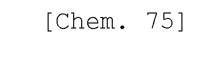

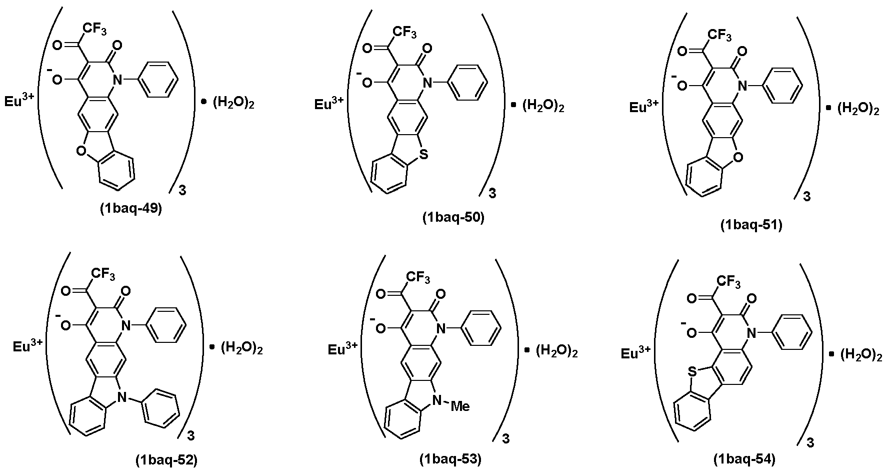

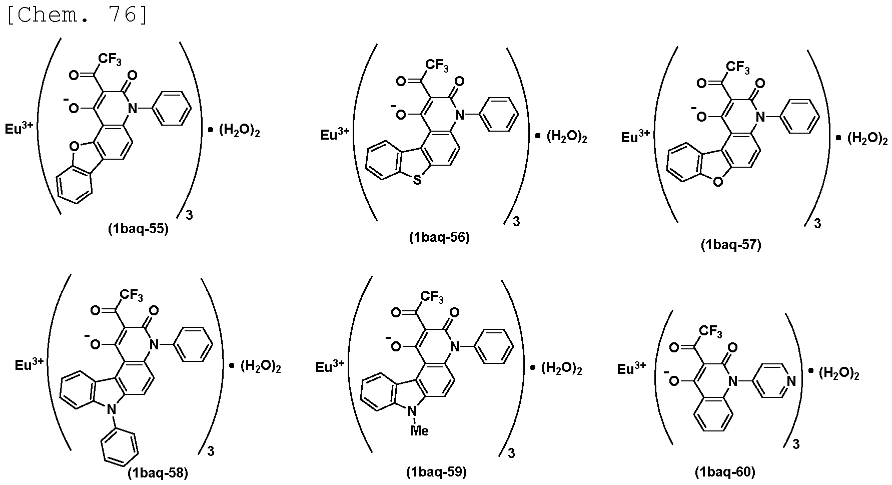

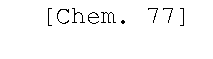

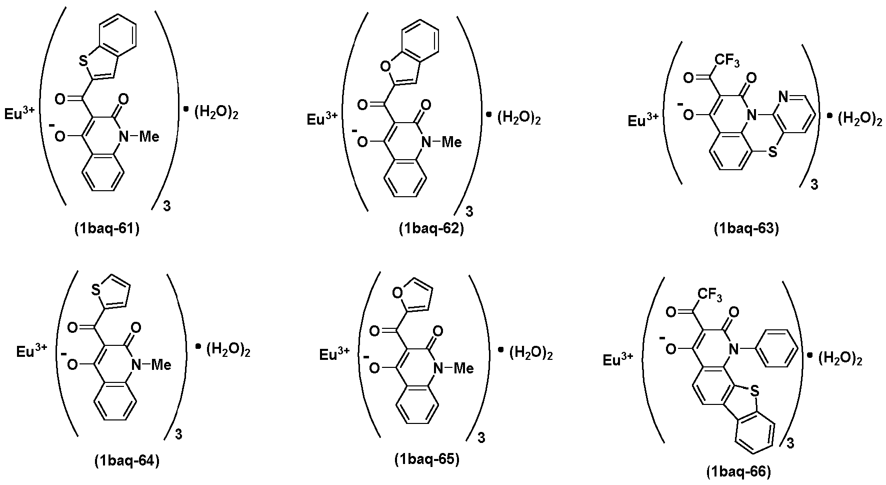

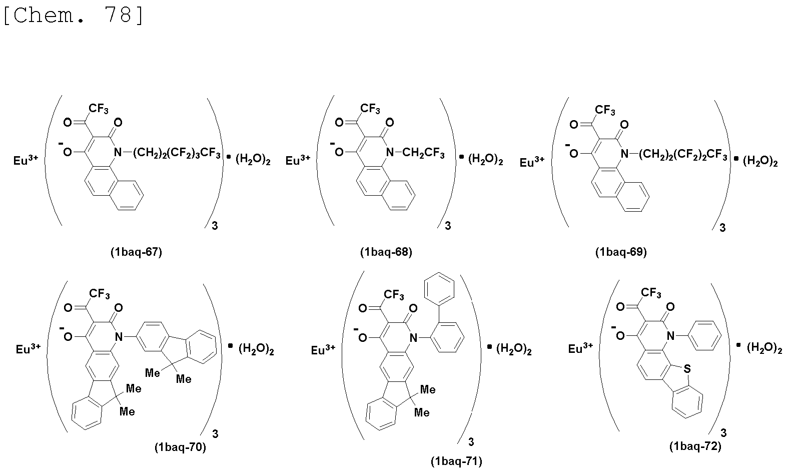

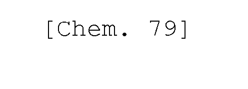

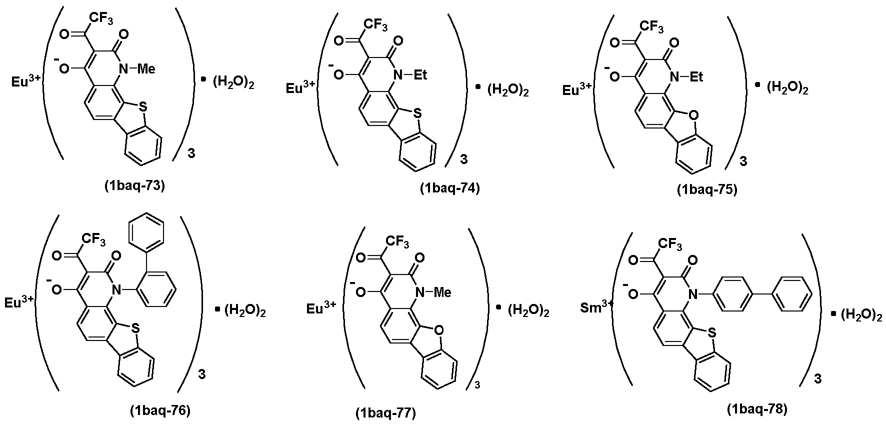

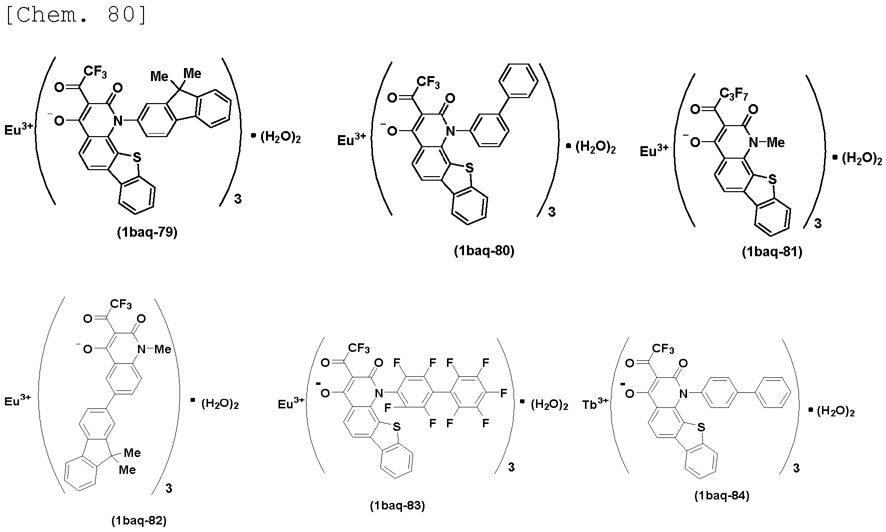

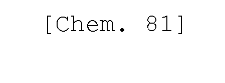

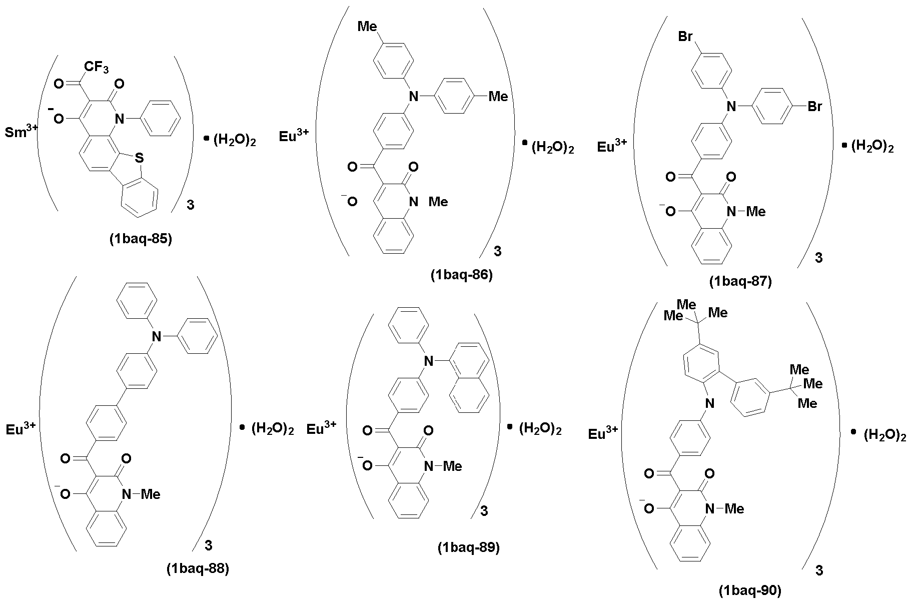

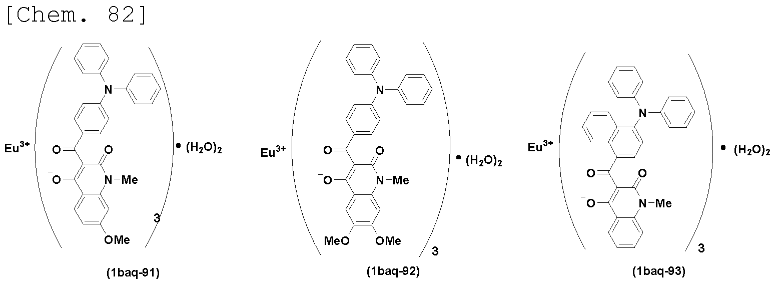

Of formulae (1b-1) to (1b-204) and formulae (1baq-1) to (1baq-93) above, the compounds represented by formulae (1b-1) to (1b-3), (1b-5) to (1b-21), (1b-25) to (1b-28), (1b-31) to (1b-33), (1b-36) to (1b-48), (1b-51) to (1b-61), (1b-64) to (1b-68), (1b-70) to (1b-111), (1b-113) to (1b-150), (1b-163) to (1b-165), (1b-166) to (1b-201) or (1baq-1) to (1baq-88) are preferred because of the ease of synthesis, the compounds represented by formulae (1b-1) to (1b-3), (1b-5) to (1b-6), (1b-8) to (1b-14), (1b-15) to (1b-21), (1b-25) to (1b-28), (1b-31) to (1b-33), (1b-37) to (1b-44), (1b-46) to (1b-48), (1b-52) to (1b-61), (1b-64) to (1b-68), (1b-70) to (1b-87), (1b-89) to (1b-103), (1b-114) to (1b-116), (1b-114) to (1b-116), (1b-133) to (1b-144), (1b-163) to (1b-201), (1baq-1) to (1baq-17), (1baq-19), (1baq-22) to (1baq-26), (1baq-28) to (1baq-37) or (1baq-67) to (1baq-88) are more preferred because of easy availability of raw materials, and formula (1b-2), (1b-3), (1b-7) to (1b-12), (1b-15), (1b-17), (1b-52) to (1b-54), (1b-66), (1b-73), (1b-97), (1b-180), (1b-185), (1b-190), (1b-192), (1b-194) or (1b-197) to (1b-201) are particularly preferred because they give the complex optical characteristics suitable for use as an optical functional material.

An example of a more preferred embodiment is a rare earth complex represented by general formula (1bi) (Hereinafter also referred to as a rare earth complex (1bi).). Specific examples include the rare earth complexes represented by formulae (1bi-1) to (1bi-204) and formulae (1biaq-1) to (1biaq-93) below, but the present invention is not limited to these.

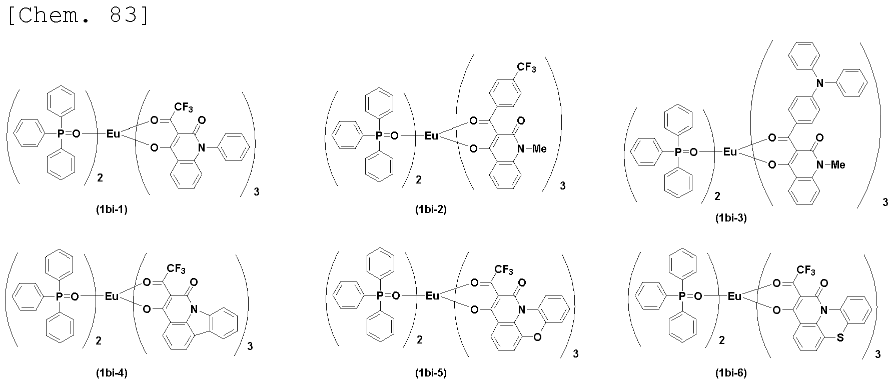

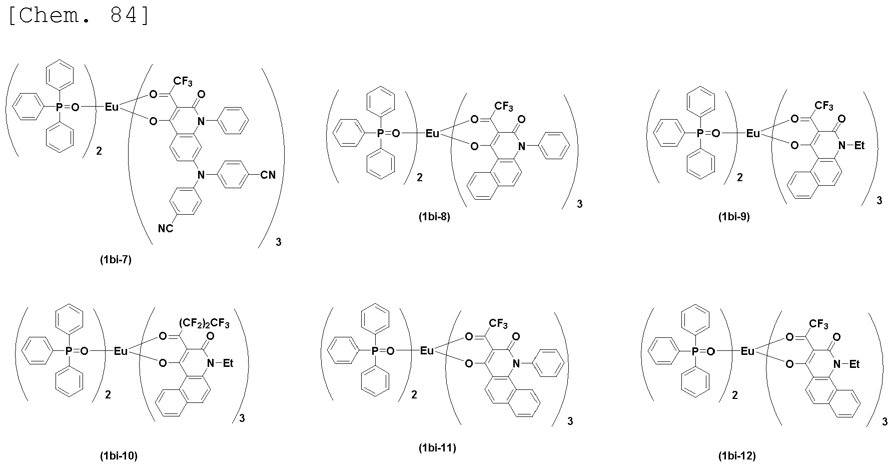

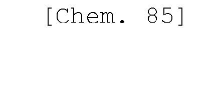

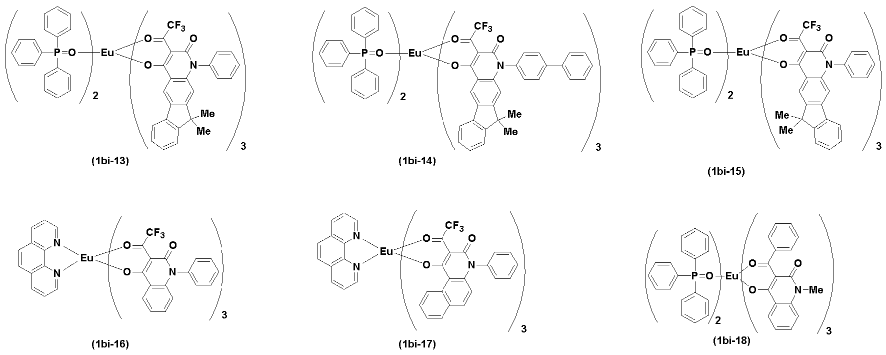

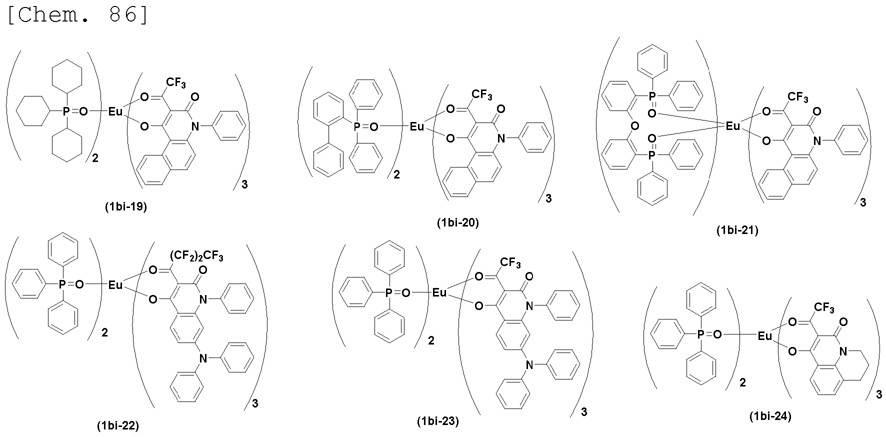

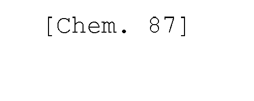

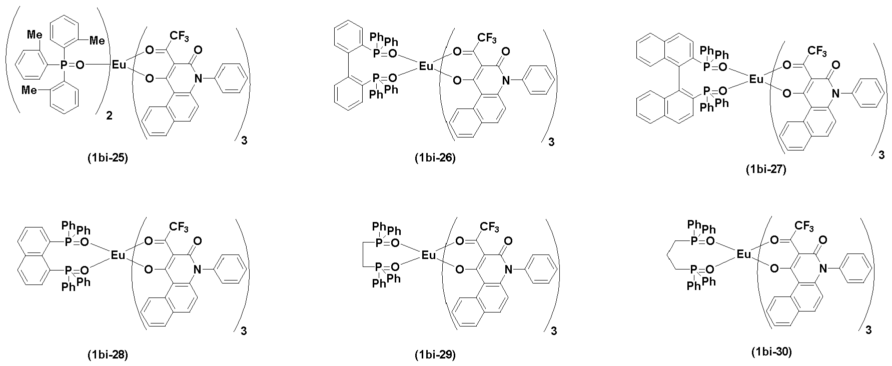

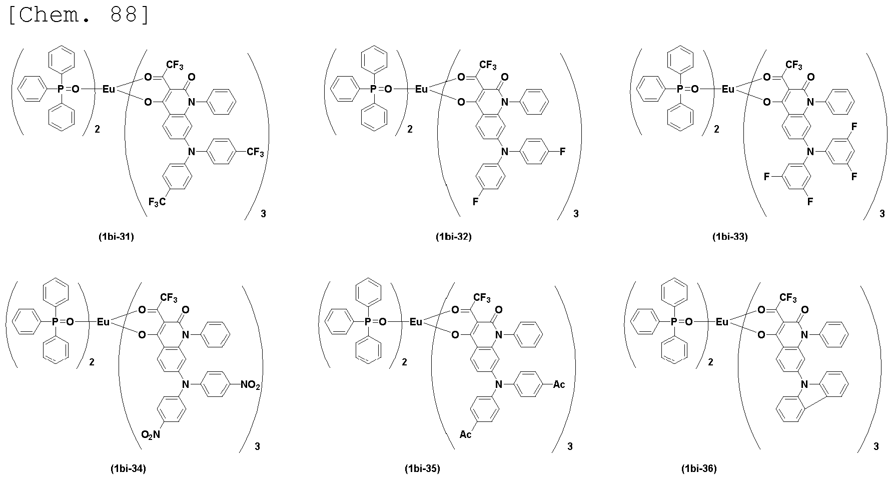

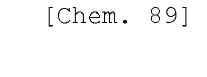

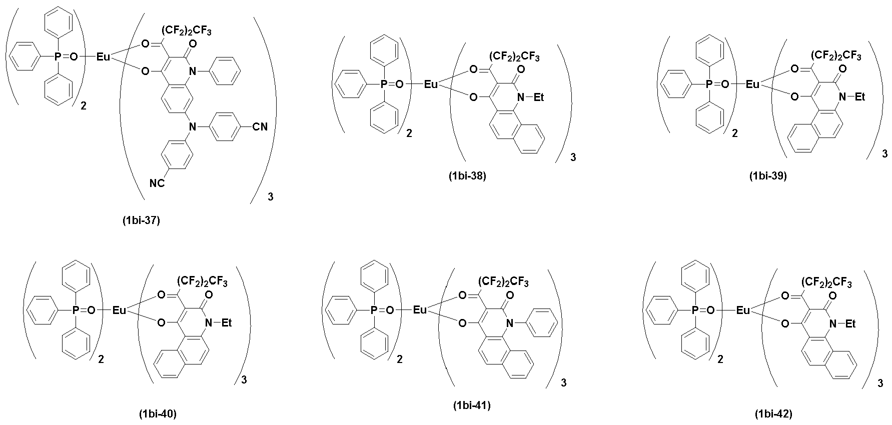

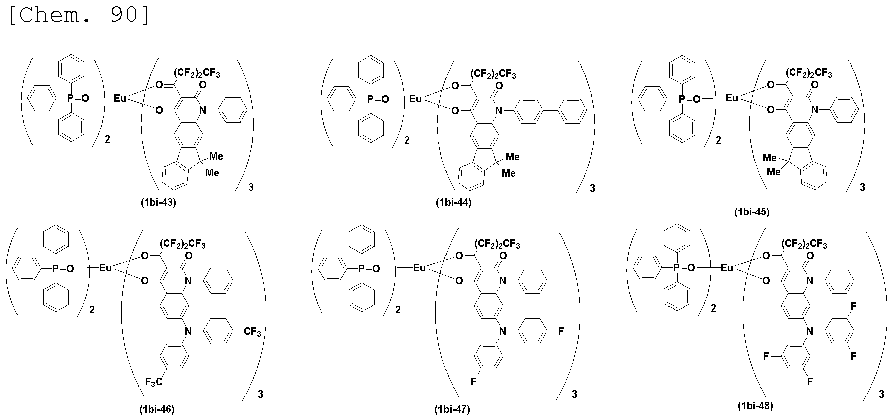

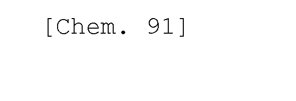

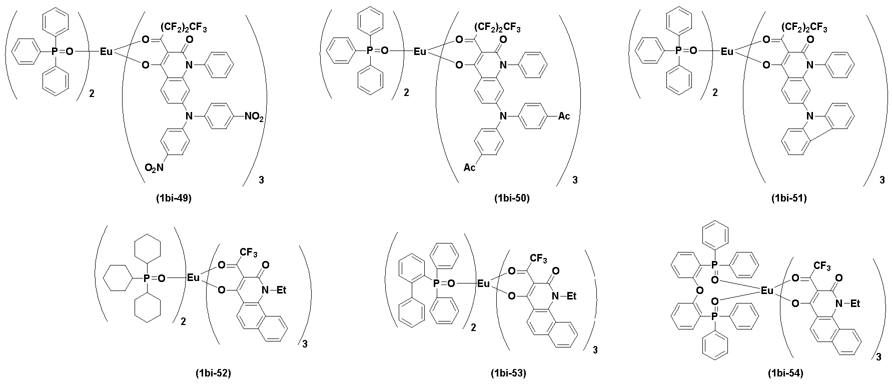

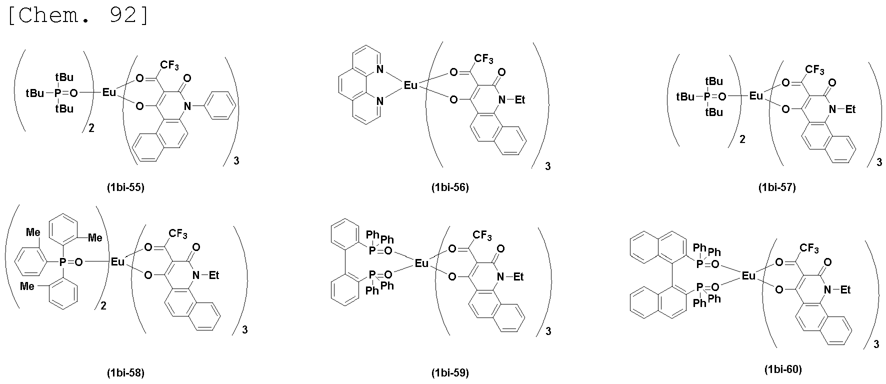

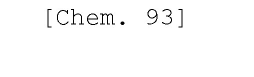

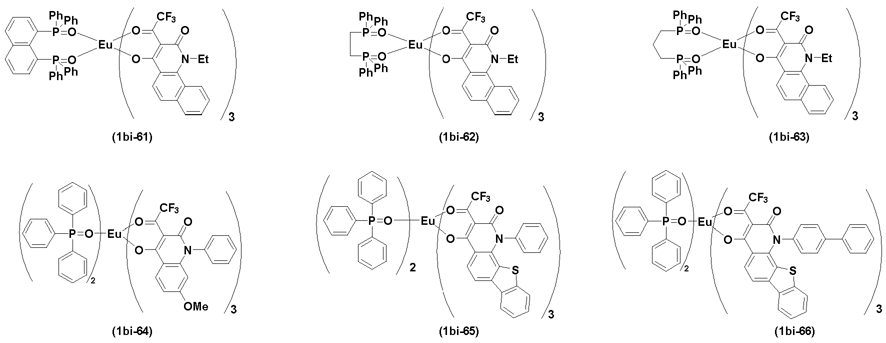

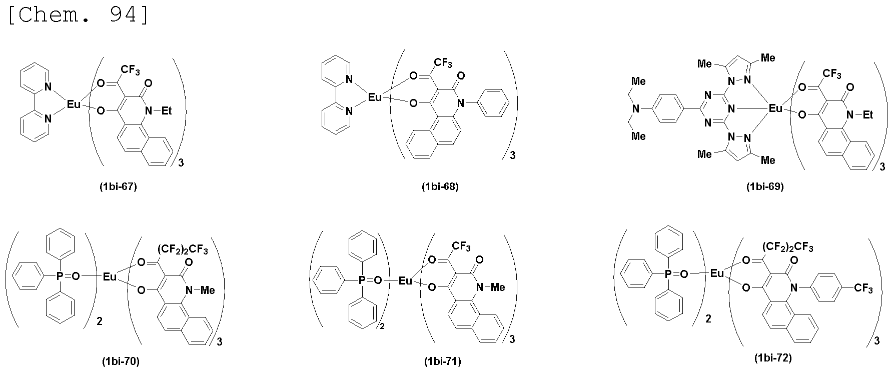

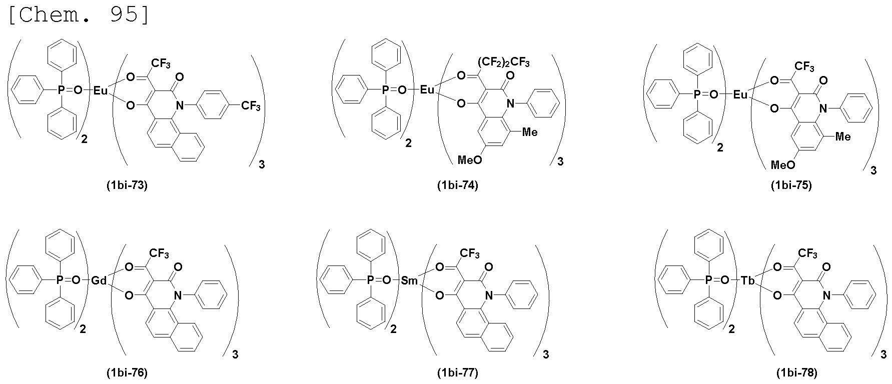

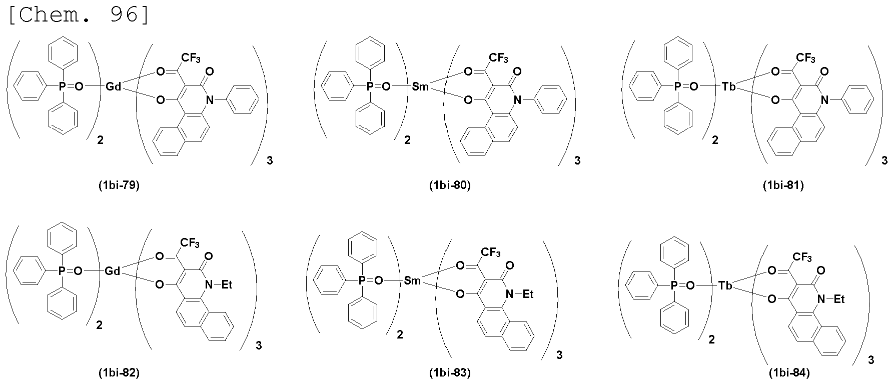

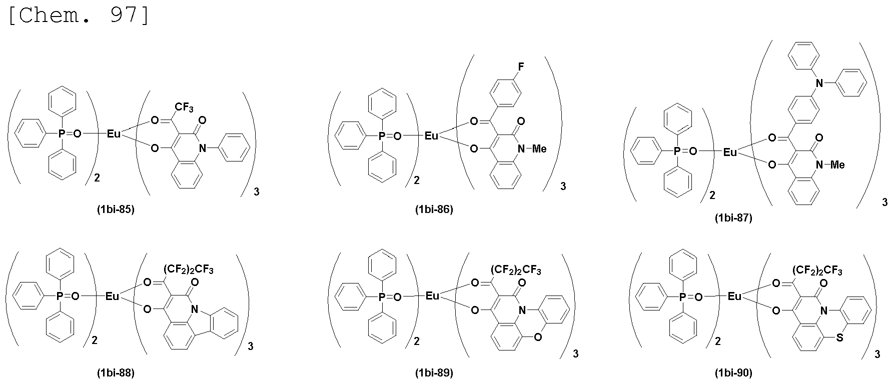

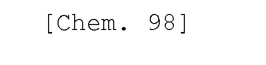

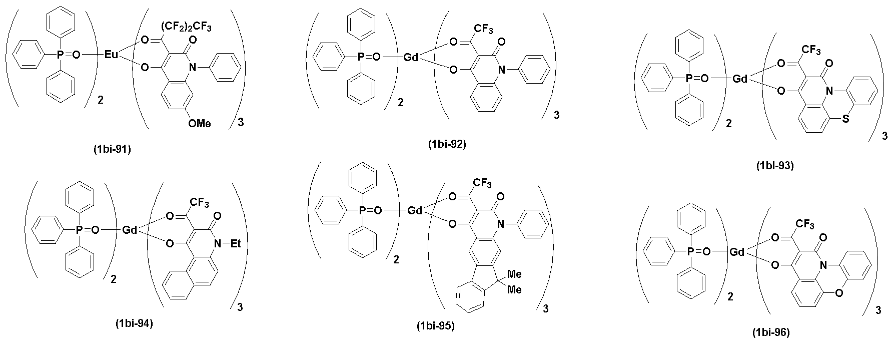

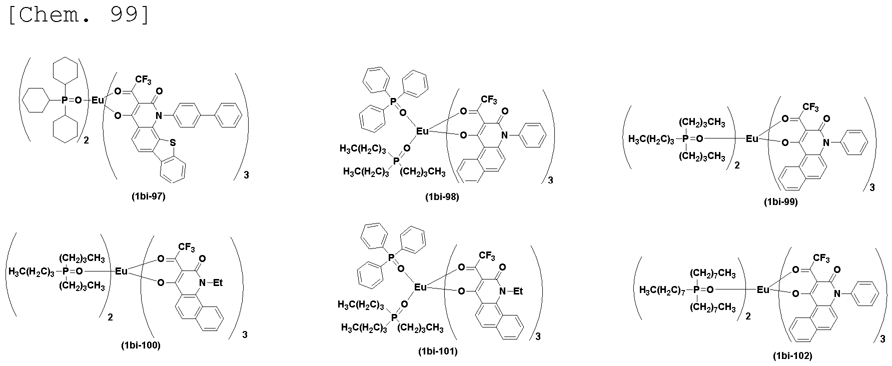

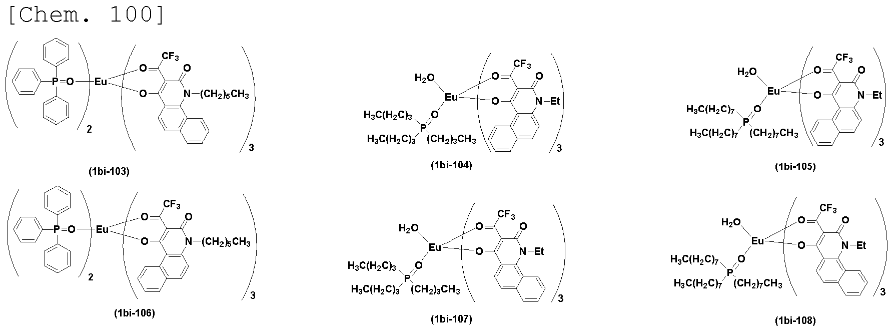

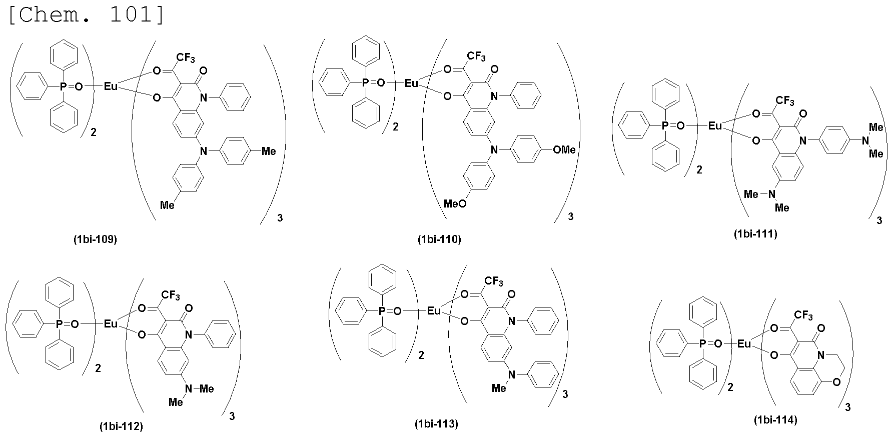

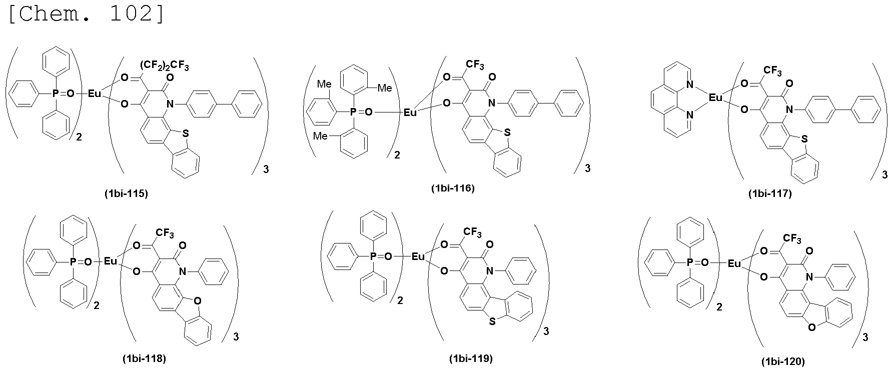

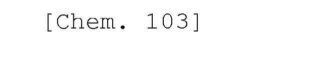

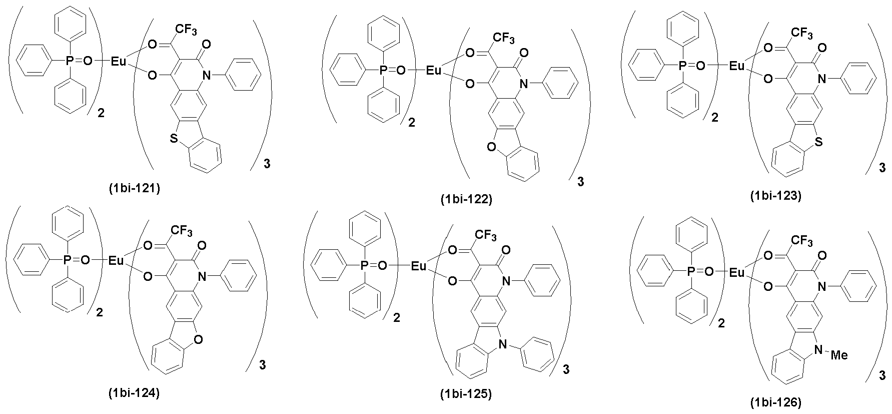

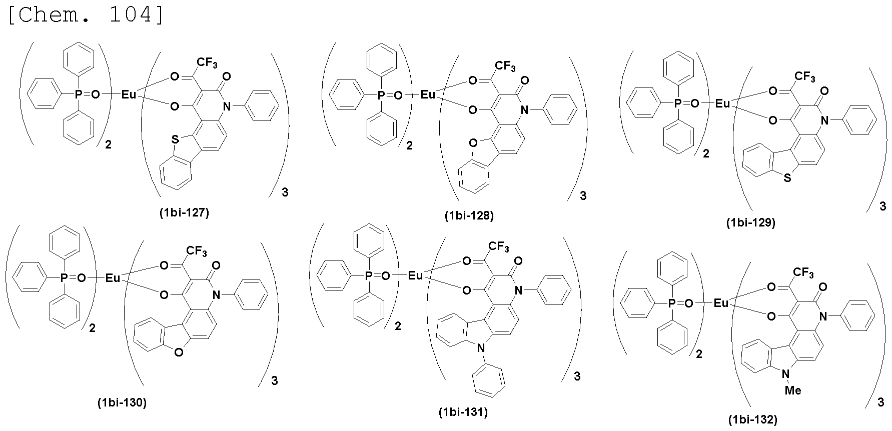

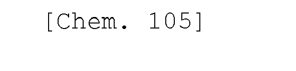

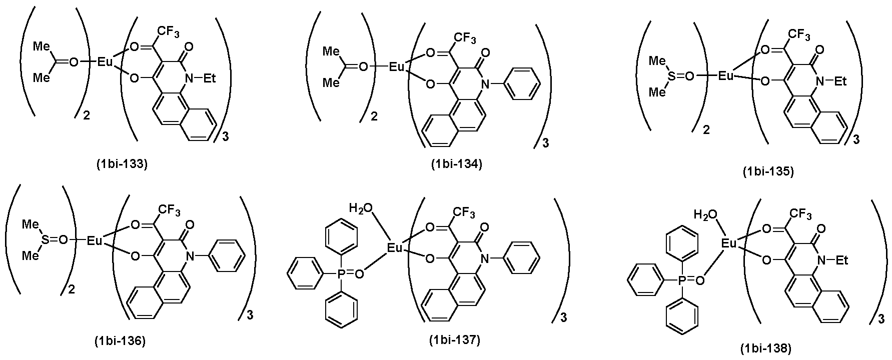

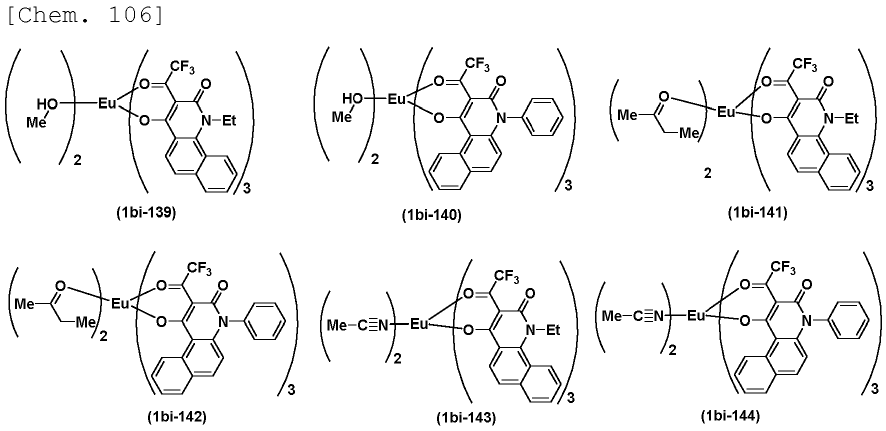

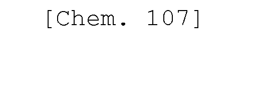

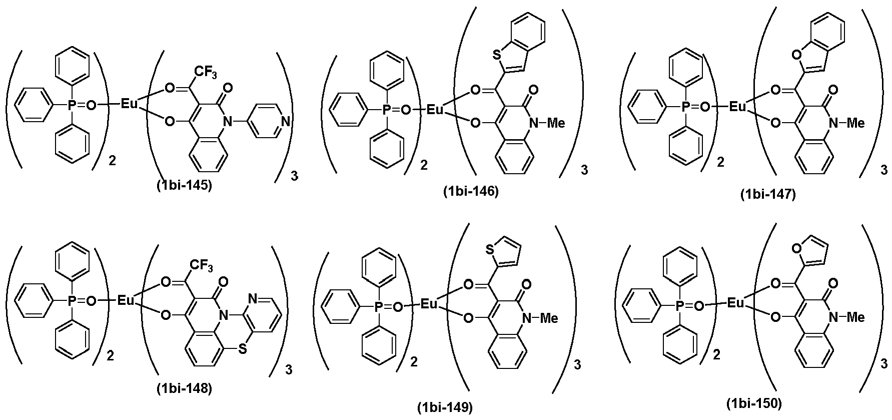

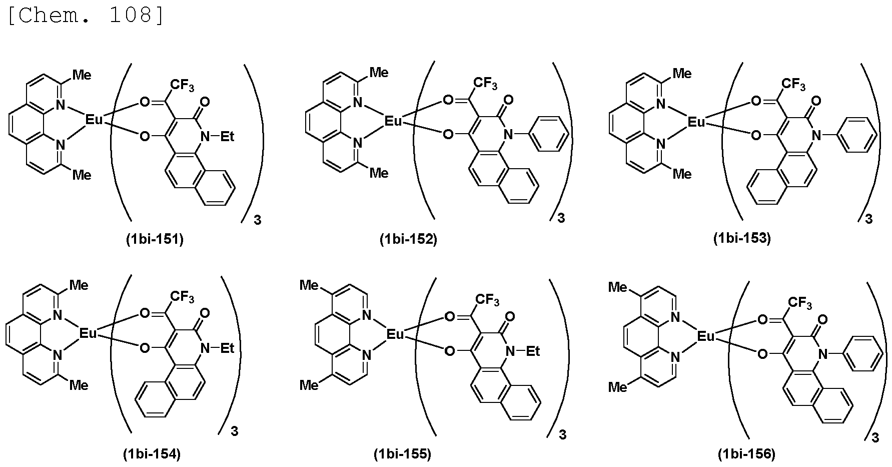

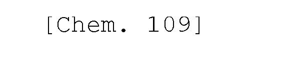

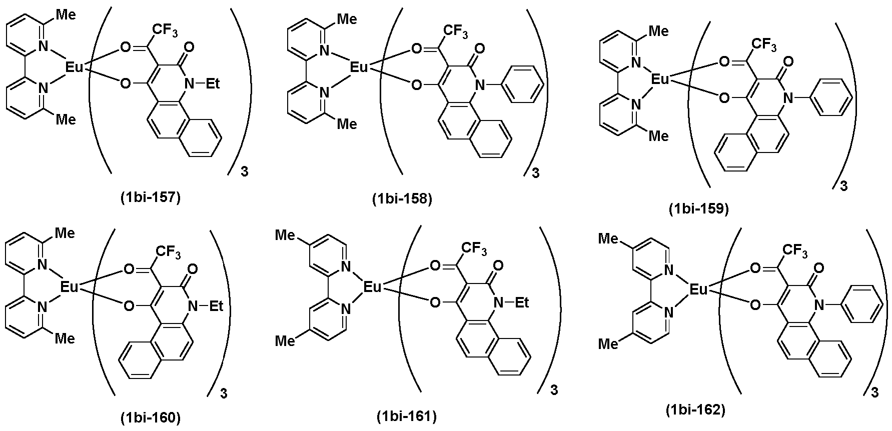

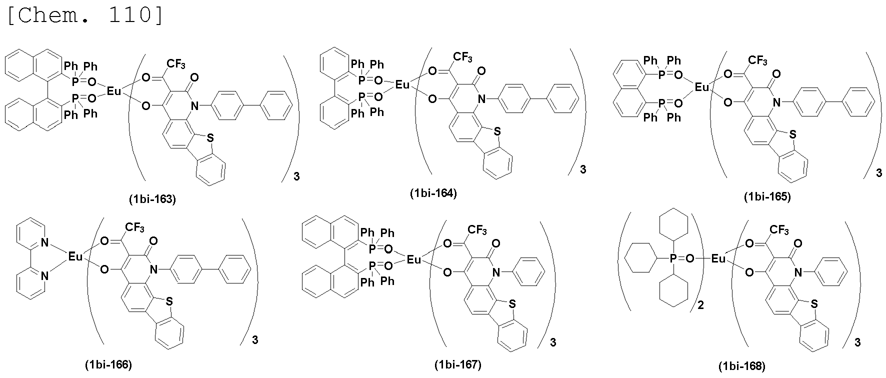

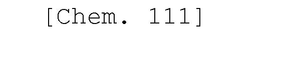

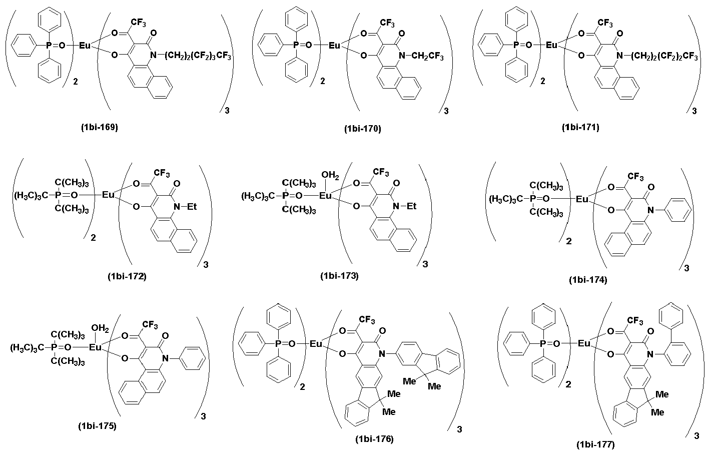

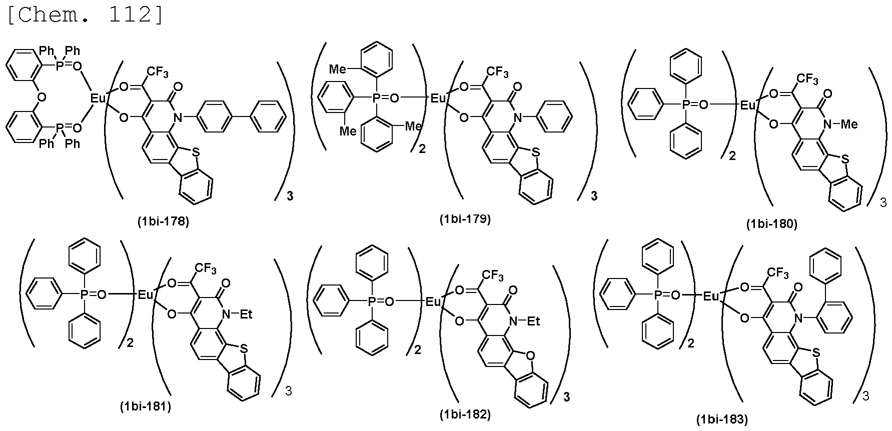

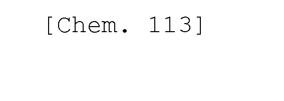

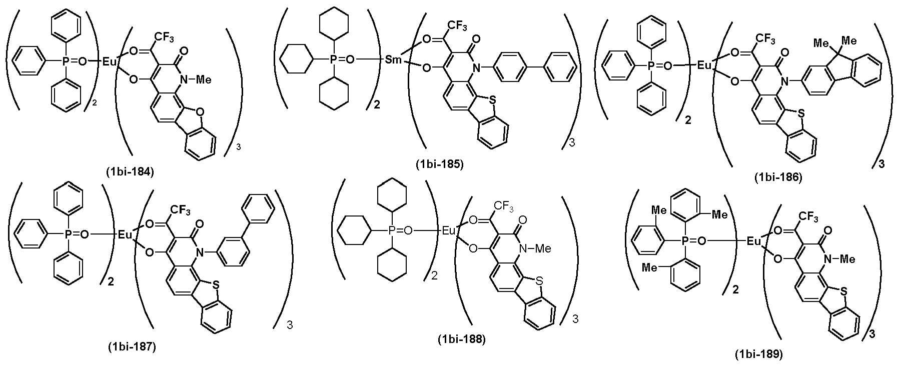

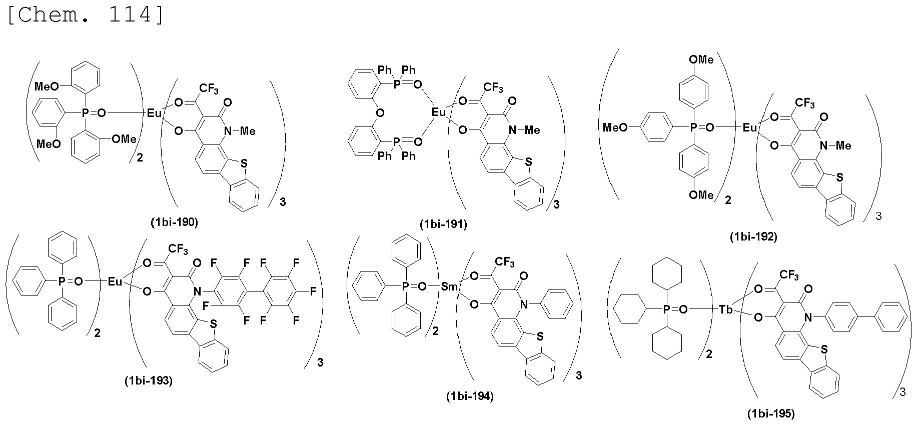

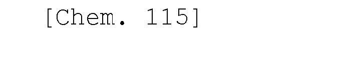

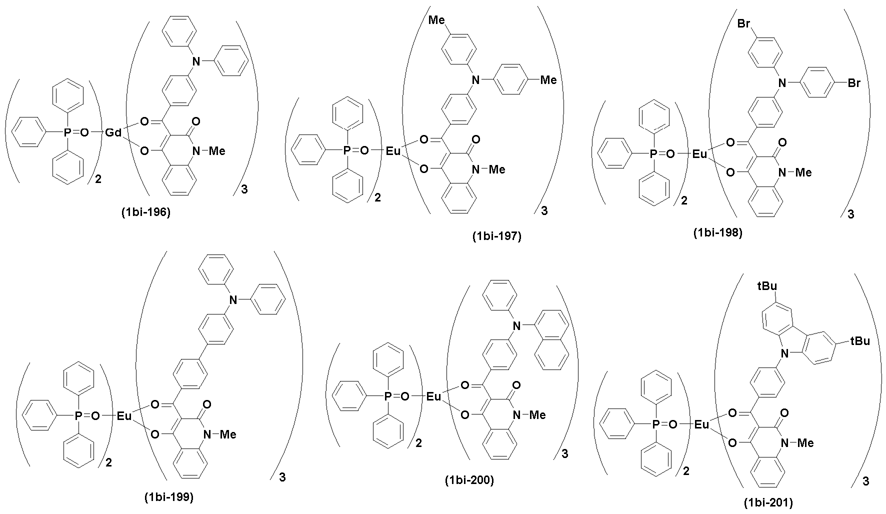

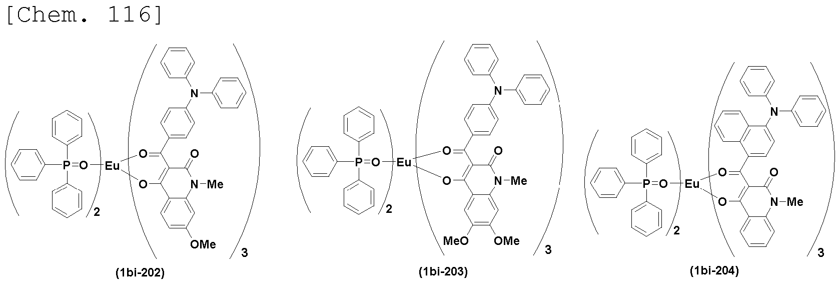

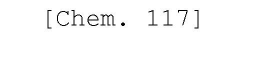

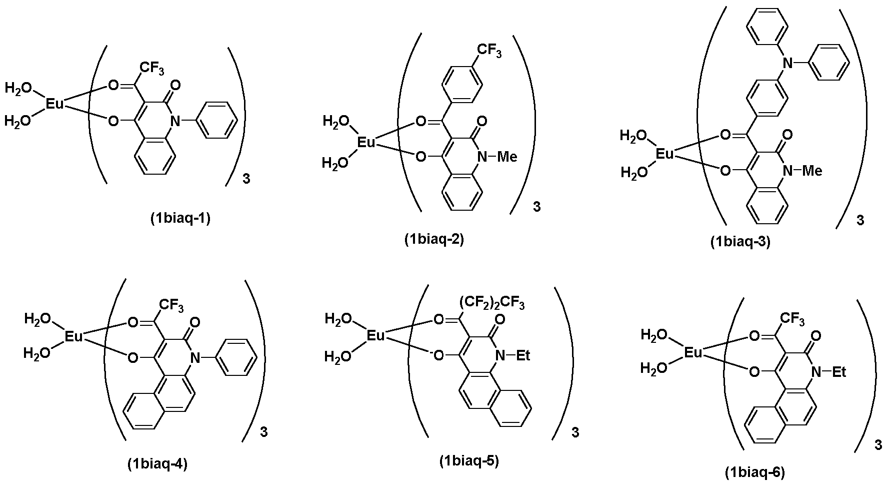

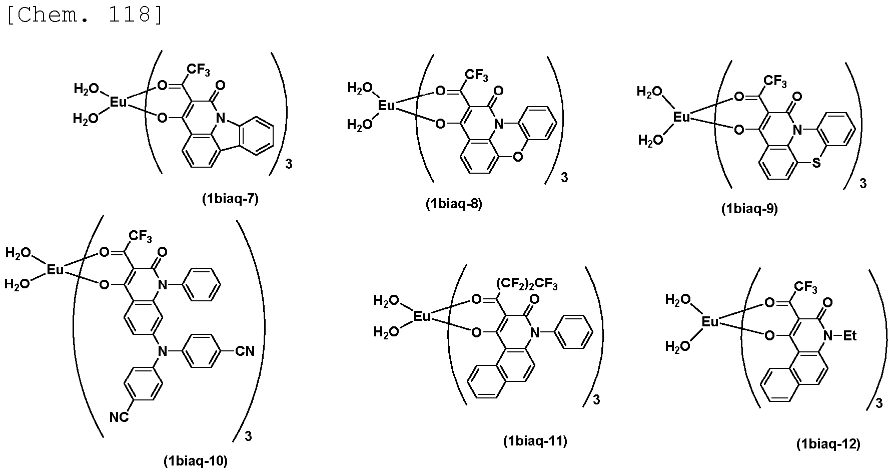

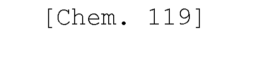

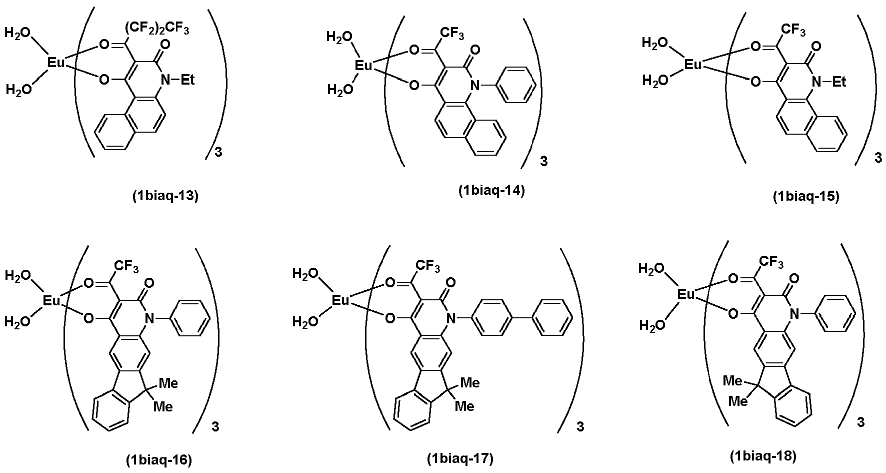

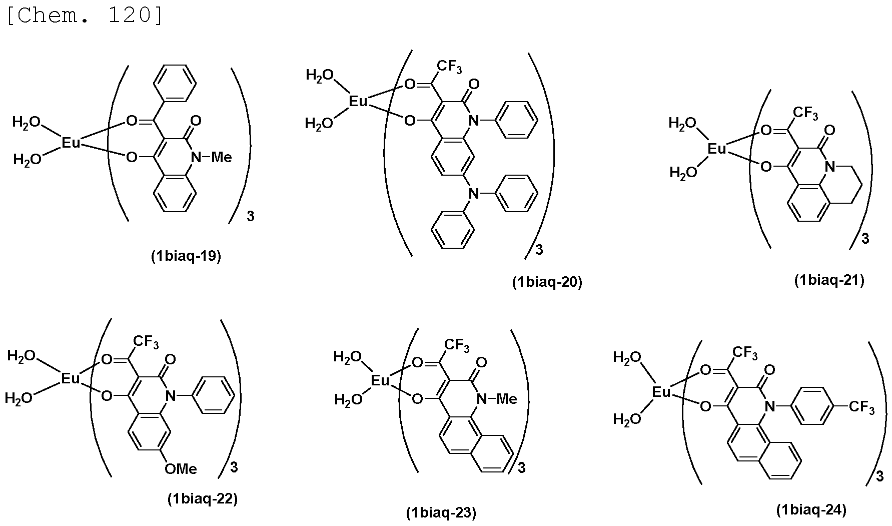

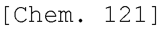

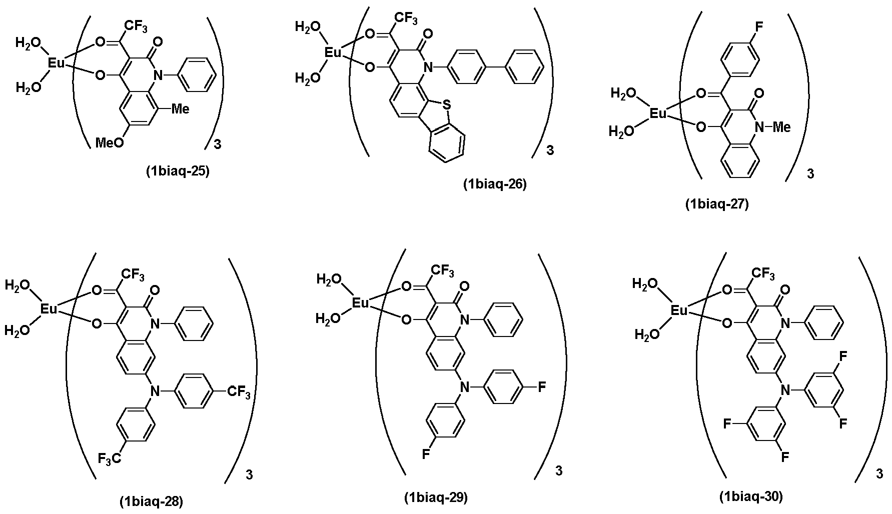

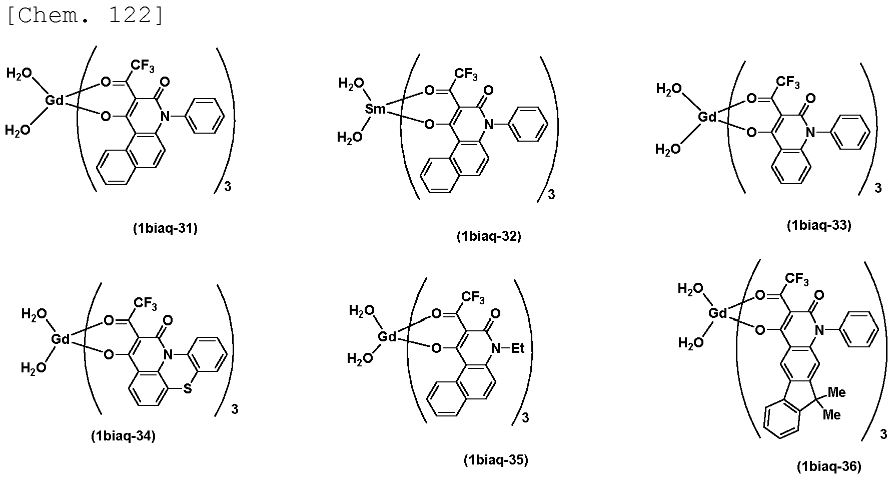

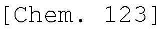

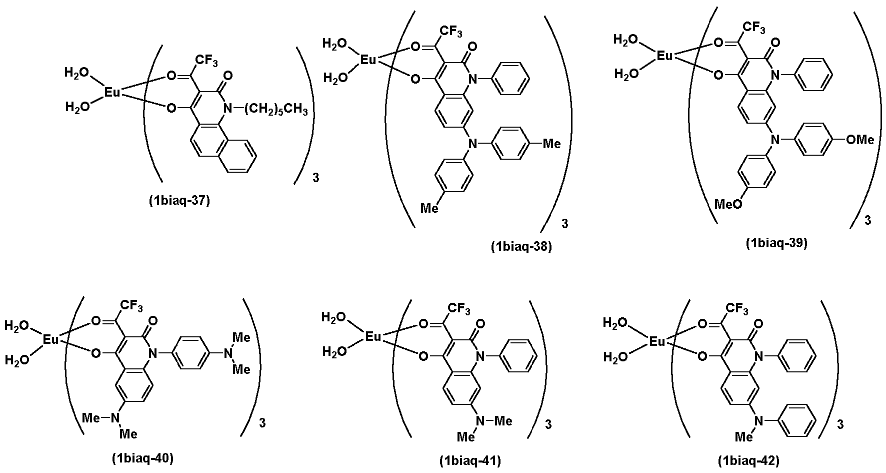

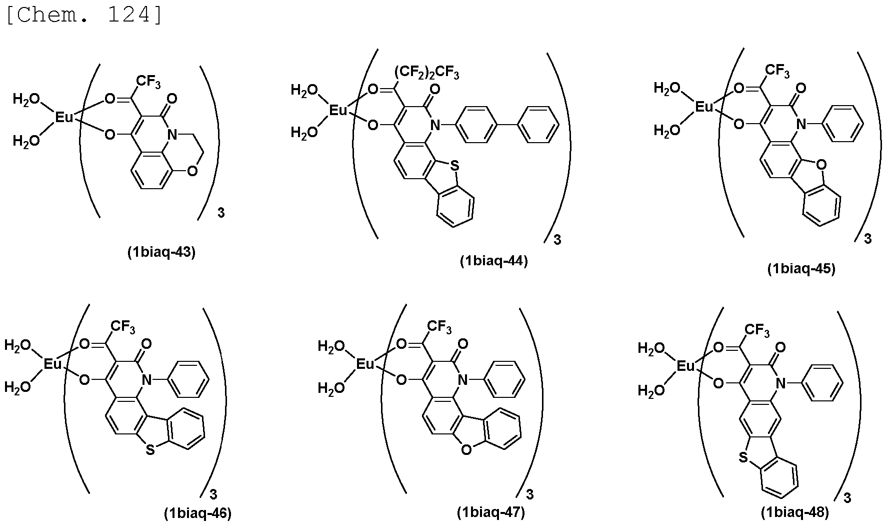

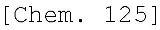

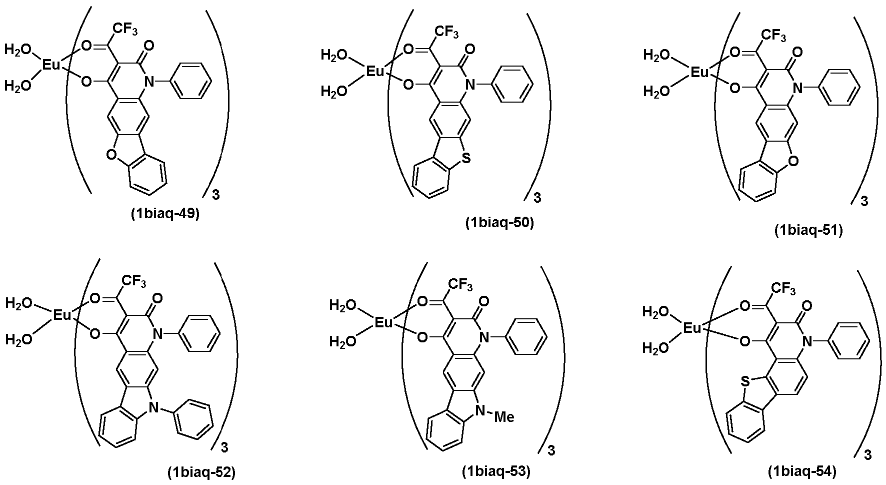

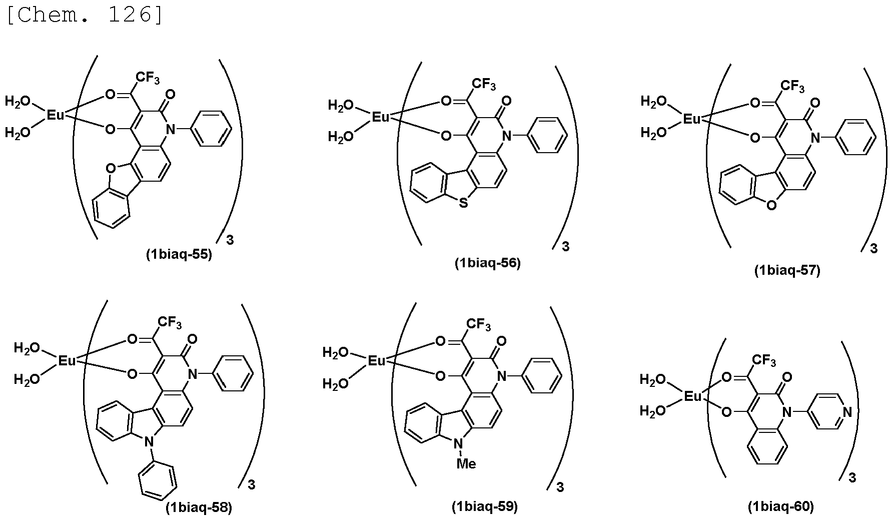

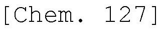

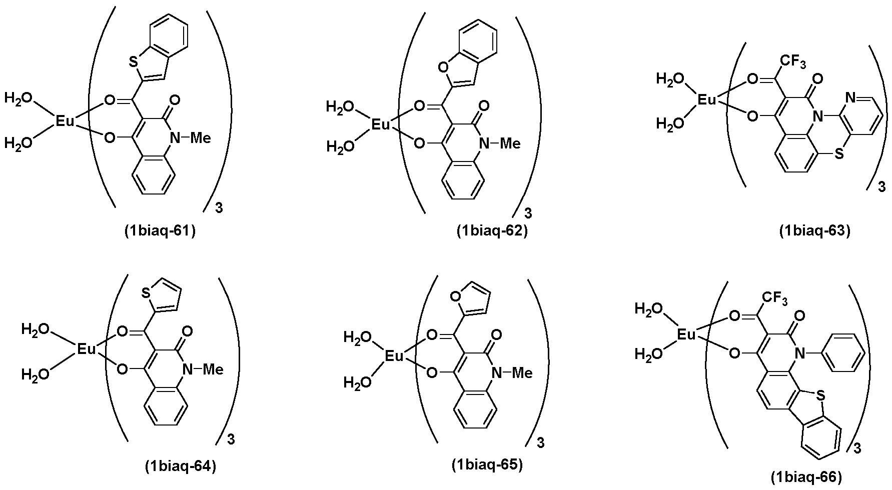

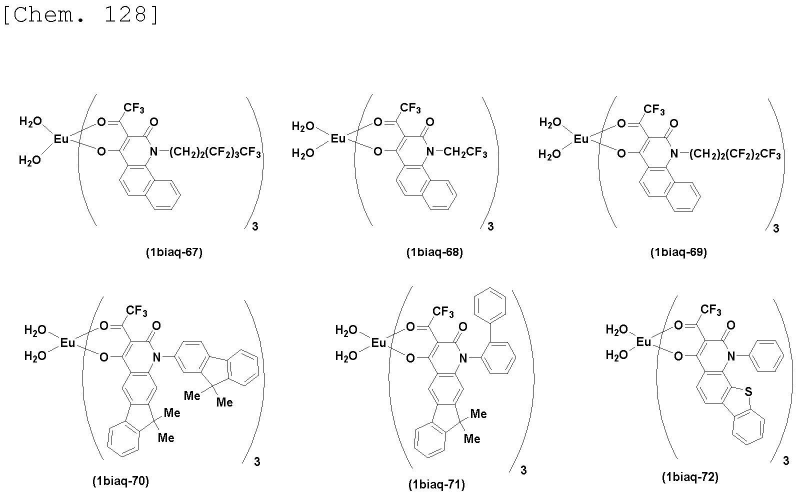

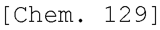

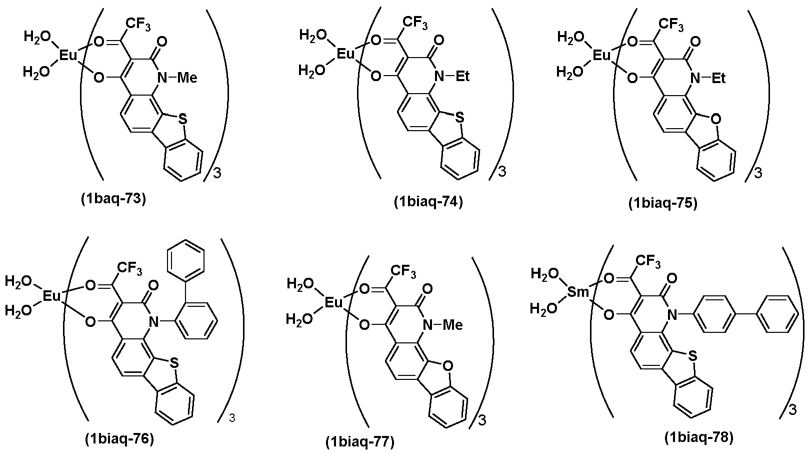

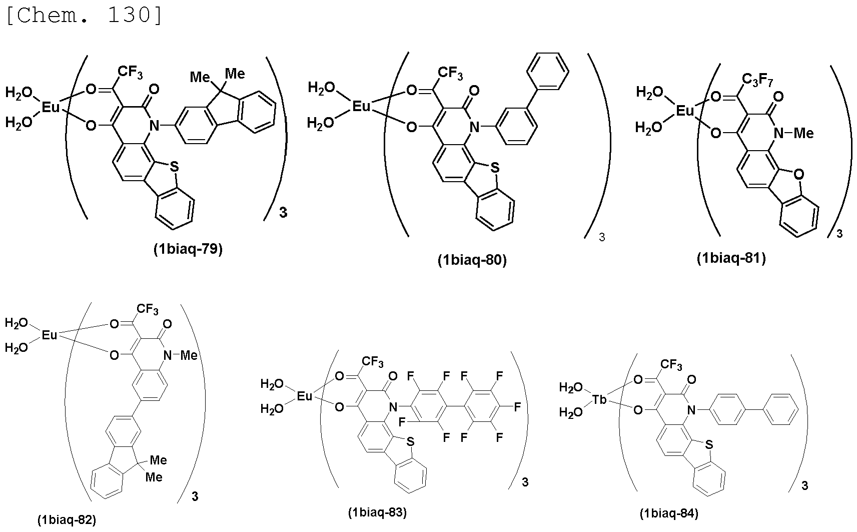

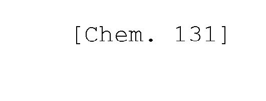

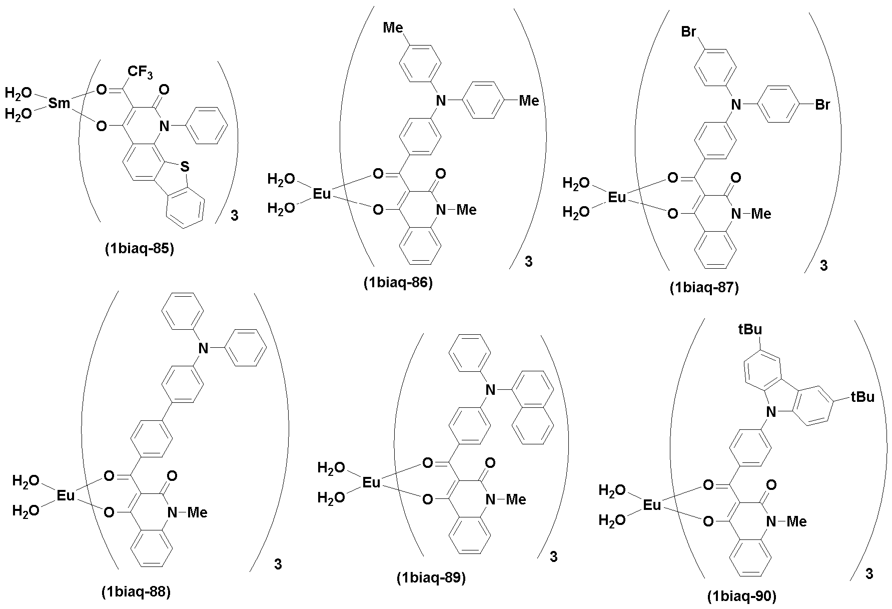

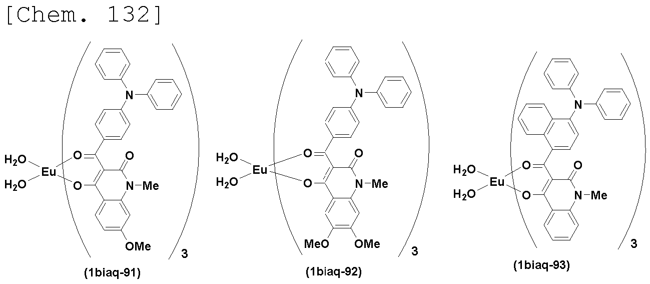

Of formulae (1bi-1) to (1bi-204) and formulae (1biaq-1) to (1biaq-93) above, the compounds represented by formulae (1bi-1) to (1bi-3), (1bi-5) to (1bi-21), (1bi-25) to (1bi-28), (1bi-31) to (1bi-33), (1bi-36) to (1bi-48), (1bi-51) to (1bi-61), (1bi-64) to (1bi-68), (1bi-70) to (1bi-111), (1bi-113) to (1bi-150), (1bi-163) to (1bi-165), (1bi-166) to (1bi-201) or (1biaq-1) to (1biaq-88) are preferred because of the ease of synthesis, the compounds represented by formulae (1bi-1) to (1bi-3), (1bi-5) to (1bi-6), (1bi-8) to (1bi-14), (1bi-15) to (1bi-21), (1bi-25) to (1bi-28), (1bi-31) to (1bi-33), (1bi-37) to (1bi-44), (1bi-46) to (1bi-48), (1bi-52) to (1bi-61), (1bi-64) to (1bi-68), (1bi-70) to (1bi-87), (1bi-89) to (1bi-103), (1bi-114) to (1bi-116), (1bi-114) to (1bi-116), (1bi-133) to (1bi-144), (1bi-163) to (1bi-201), (1biaq-1) to (1biaq-17), (1biaq-19), (1biaq-22) to (1biaq-26), (1biaq-28) to (1biaq-37) or (1biaq-67) to (1biaq-88) are more preferred because of easy availability of raw materials, and formula (1bi-2), (1bi-3), (1bi-7) to (1bi-12), (1bi-15), (1bi-17), (1bi-52) to (1bi-54), (1bi-66), (1bi-73), (1bi-97), (1bi-180), (1bi-185), (1bi-190), (1bi-192), (1bi-194) or (1bi-197) to (1bi-201) are particularly preferred because they give the complex optical characteristics suitable for use as an optical functional material.

Examples of preferred embodiments of the rare earth complex (1) according to the present invention include rare earth complexes in which the coumarinato ligand (1cu) in the rare earth complex (1) is indicated by general formulae (1cu-1) to (1qu-6) below.

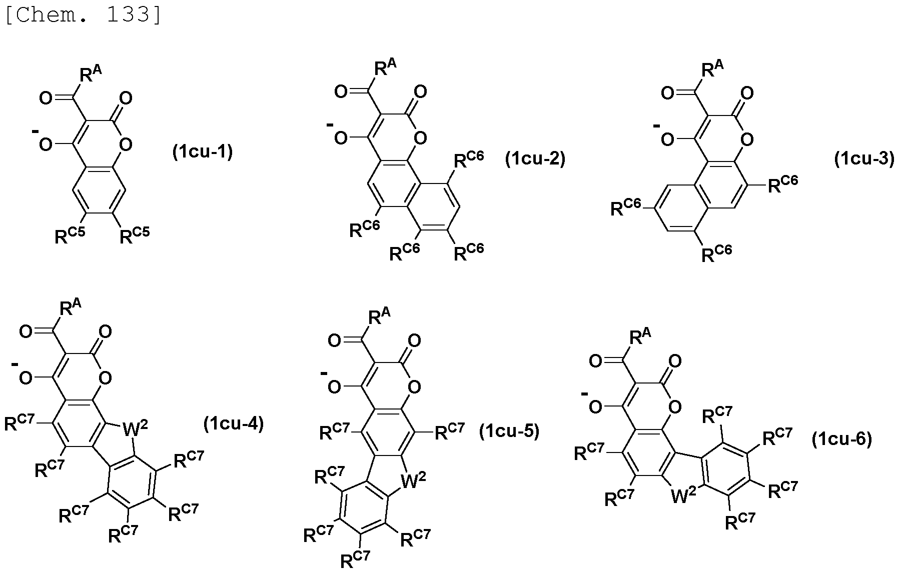

[In the formulae, R^{A}, R^{C6}, R^{C7}, R^{C8} and W² are defined the same as above. The R^{C5}s each independently represent a hydrogen atom, halogen atom, cyano group, C1 to C8 alkyl group, C1 to C4 fluoroalkyl group, C6 to C22 aryl group, substituted or unsubstituted amino group indicated by general formula (BA3) below or substituted or unsubstituted oxy group indicated by general formula (BO3) below.

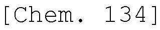

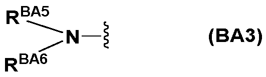

(In the formula, R^{BA5} and R^{BA6} each independently represent a C1 to C4 alkyl group or C6 to C12 aryl group, and the alkyl group and the aryl group may be substituted with one or more substituents selected from the group consisting of halogen atoms, C1 to C4 alkyloxy groups, C1 to C4 fluoroalkyl groups, C2 to C13 acyl groups, a cyano group and a nitro group. R^{BA5} and R^{BA6}, furthermore, may form a five-membered, six-membered or seven-membered ring including the nitrogen atom to which they are bound.)

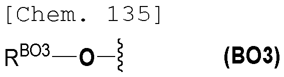

(In the formula, R^{BO3} represents a C1 to C4 alkyl group or C6 to C12 aryl group, and the alkyl group and the aryl group may be substituted with one or more substituents selected from the group consisting of halogen atoms, C1 to C4 alkyloxy groups, C1 to C4 fluoroalkyl groups, C2 to C13 acyl groups, a cyano group and a nitro group.) The adjacent R^{C5}s, furthermore, may form a five-membered, six-membered or seven-membered ring together with the benzene ring to which they are bound.]

### (R^{C5})

Examples of halogen atoms represented by R^{C5} include the substituents listed as examples of halogen atoms that belong to group T₂1.

A C1 to C8 alkyl group represented by R^{C5} may be any of a linear-chain, branched or cyclic alkyl group. Specific examples include groups such as a methyl group, an ethyl group, a propyl group, an isopropyl group, a butyl group, a 1-methylpropyl group, a 2-methylpropyl group, a 2,2-dimethylpropyl group, a 3-cyclopropylpropyl group, a cyclopropyl group, a 2-methylbutyl group, a 3-methylbutyl group, a 1-(1,1-dimethylethyl) group, a cyclobutyl group, a pentyl group, a 2-methylpentyl group, a 1-methylbutyl group, a 1,2-dimethylbutyl group, a 1-ethylpropyl group, a cyclopentyl group, a 5-dimethylcyclopentyl group, a 3-ethylcyclopentyl group, a hexyl group, a cyclohexyl group, a 4-ethylcyclohexyl group, a 4-propylcyclohexyl group, a 4,4-dimethylcyclohexyl group, a 2,6-dimethylcyclohexyl group, a 3,5-dimethylcyclohexyl group, a cycloheptyl group, an octyl group and a cyclooctyl group. The present invention is not limited to these substituents listed by way of example.

Examples of C1 to C4 fluoroalkyl groups represented by R^{C5} include the groups listed as examples of C1 to C4 fluoroalkyl groups represented by R^{C7}.

Specific examples of C6 to C22 aryl groups represented by R^{C5} include groups such as a phenyl group, a naphthyl group, a methylphenyl group, a dimethylphenyl group, a trimethylphenyl group, an ethylphenyl group, a propylphenyl group, a triisopropylphenyl group, a tert-butylphenyl group, a methylnaphthyl group, a biphenylyl group, a methylbiphenylyl group, a phenanthrenyl group, a methylphenanthrenyl group, a dimethylphenanthrenyl group and an anthracenyl group. The present invention is not limited to these substituents listed by way of example.

The R^{C5}s, furthermore, each independently represent a substituted or unsubstituted amino group indicated by general formula (BA3) below or substituted or unsubstituted oxy group indicated by general formula (BO3) below.

Examples of C1 to C4 alkyl groups represented by R^{BA5} and R^{BA6} in general formula (BA3) or R^{BO3} in general formula (BO3) include the groups listed as examples of the C1 to C4 alkyl groups of a methylene group at W¹ optionally substituted with one or more C1 to C4 alkyl groups.

Examples of C6 to C12 aryl groups represented by R^{BA5} and R^{BA6} in general formula (BA3) or R^{BO3} in general formula (BO3) include the groups listed as examples of the C6 to C12 aryl group of a nitrogen atom at W¹ optionally substituted with a C6 to C12 aryl group.

A C1 to C4 alkyl or C6 to C12 aryl group represented by R^{BA5} and R^{BA6} in general formula (BA3), moreover, may be substituted with one or more substituents selected from group T₂3.

A C1 to C4 alkyl or C6 to C12 aryl group represented by R^{BO3} in general formula (BO3), furthermore, may be substituted with one or more substituents selected from group T₂3.

Examples of halogen atoms that belong to group T₂3 include the groups listed as examples of halogen atoms that belong to group T₂1.

Examples of C1 to C4 alkyl groups that belong to group T₂3 include the groups listed as examples in the context of the C1 to C4 alkyl groups of a methylene group at W¹ optionally substituted with one or more C1 to C4 alkyl groups.

Examples of C1 to C4 alkyloxy groups that belong to group T₂3 include the groups listed as examples of C1 to C4 alkyloxy groups at X² in general formula (3d).

Examples of C1 to C4 fluoroalkyl groups that belong to group T₂3 include the groups listed as examples of C1 to C4 fluoroalkyl groups represented by R^{B6}.

Examples of C2 to C13 acyl groups that belong to group T₂3 include the substituents listed as examples of C2 to C13 acyl groups that belong to group T₂1.

Specific examples of C1 to C4 alkyl groups at R^{BA5} and R^{BA6} optionally substituted with one or more substituents selected from group T₂3 and C1 to C4 alkyl groups at R^{BO3} optionally substituted with one or more substituents selected from group T₂3, therefore, include groups such as a 2,2,2-trifluoroethyl group, a 2-methoxyethyl group and a 3-methoxypropyl group. The present invention is not limited to these substituents listed by way of example.

Examples of C6 to C12 aryl groups at R^{BA5} and R^{BA6} optionally substituted with one or more substituents selected from group T₂3 and C6 to C12 aryl groups at R^{BO3} optionally substituted with one or more substituents selected from group T₂3, furthermore, include the groups listed as examples of the C6 to C12 aryl group of a nitrogen atom at W¹ optionally substituted with a C6 to C12 aryl group.

In addition, R^{BA5} and R^{BA6} may form a five-membered, six-membered or seven-membered ring including the nitrogen atom to which they are bound. Specific examples include groups such as an azetidyl group, a pyrrolidinyl group, a piperidino group, an azepanyl group, a morpholino group, a 4-methylpiperazinyl group, a carbazoyl group, a phenoxazinyl group, a phenothiazinyl group and a 10,11-dihydro-5H-dibenz[b,f]azepinyl group. The present invention is not limited to these substituents listed by way of example.

A substituted or unsubstituted amino group indicated by general formula (BA3), therefore, may be substituted with one or more substituents selected from group T₂3, and specific examples include the substituents listed as examples for a substituted or unsubstituted amino group indicated by general formula (BA1).

A substituted or unsubstituted oxy group indicated by general formula (BO3), furthermore, may be substituted with one or more substituents selected from group T₂3, and specific examples include the substituents listed as examples for a substituted or unsubstituted amino group indicated by general formula (BO1).

R^{C5} is preferably a hydrogen atom, C1 to C4 alkyl group, C1 to C4 alkyloxy group, C2 to C8 dialkylamino group or diphenylamino group optionally substituted with one or more fluorine atoms; C1 to C4 fluoroalkyl groups; C2 to C5 acyl groups; or; cyano groups because of easy availability of raw materials, more preferably a hydrogen atom, C1 to C4 alkyl group, C2 to C8 dialkylamino group or diphenylamino group optionally substituted with one or more fluorine atoms or C1 to C4 fluoroalkyl groups because of the ease of synthesis.

Examples of the C1 to C4 alkyl group include the groups listed as examples in the context of the C1 to C4 alkyl groups of a methylene group at W¹ optionally substituted with one or more C1 to C4 alkyl groups.

Examples of the C1 to C4 alkyloxy group include the groups listed as examples of C1 to C4 alkyloxy groups at X² in general formula (3d).

Specific examples of the C2 to C8 dialkylamino group include groups such as a dimethylamino group, a diethylamino group, a dipropylamino group, a diisopropylamino group, a dibutylamino group, a bis(2-methylpropyl)amino group, a bis(2, 2-dimethylpropyl)amino group, a bis(3-cyclopropyl)amino group, a dicyclopropylamino group, a bis(2-methylbutyl)amino group, a bis(3-methylbutyl)amino group, a di(tert-butyl)amino group, a dicyclobutylamino group, an N-ethyl-N-methylamino group, an N-methyl-N-propyl group, an N-cyclopropyl-N-methylamino group, an N-butyl-N-methylamino group, an N-cyclobutyl-N-methylamino group, an N-methyl-N-pentyl group, an N-cyclopentyl-N-methylamino group, an N-hexyl-N-methylamino group, an N-cyclohexyl-N-methylamino group and an N-cycloheptyl-N-methylamino group.

The C2 to C5 acyl groups may be any of linear-chain, branched or cyclic. Specific examples include groups such as an acetyl group, an ethylcarbonyl group, a 1-propylcarbonyl group, an isopropylcarbonyl group and a tert-butylcarbonyl group. The present invention is not limited to these substituents listed by way of example.

The diphenylamino group may be substituted with one or more fluorine atoms; C1 to C4 fluoroalkyl groups; or; cyano groups, and specific examples include groups such as a diphenylamino group, a bis(4-fluorophenyl)amino group, a bis(3,4-difluorophenyl)amino group, a bis(3,5-difluorophenyl)amino group, a bis[4-(trifluoromethyl)phenyl]amino group, a bis[4-(perfluoropropyl)phenyl]amino group, a bis[4-(perfluorobutyl)phenyl]amino group, a bis(4-cyanophenyl)amino group and a bis(3,5-dicyanophenyl)amino group. The present invention is not limited to these substituents listed by way of example.

The rare earth complex (1) according to the present invention may contain a solvent of crystallization formed during purification, for example by reprecipitation or recrystallization.

Specific examples of rare earth complexes (1) according to the present invention include the rare earth complexes represented by formulae (1c-1) to (1c-225) and formulae (1caq-1) to (1caq-89) below, but the present invention is not limited to these. Herein, Ph represents a phenyl group, Ac represents an acetyl group, Me represents a methyl group, Et represents an ethyl group, iPr represents an isopropyl group, and tBu represents a tert-butyl group.

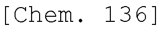

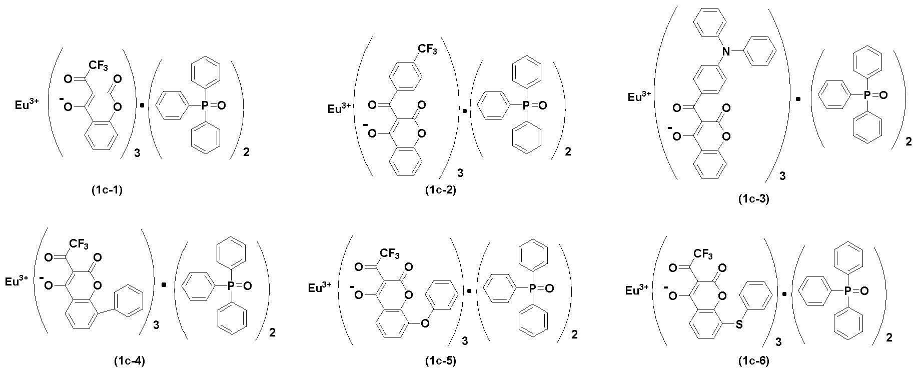

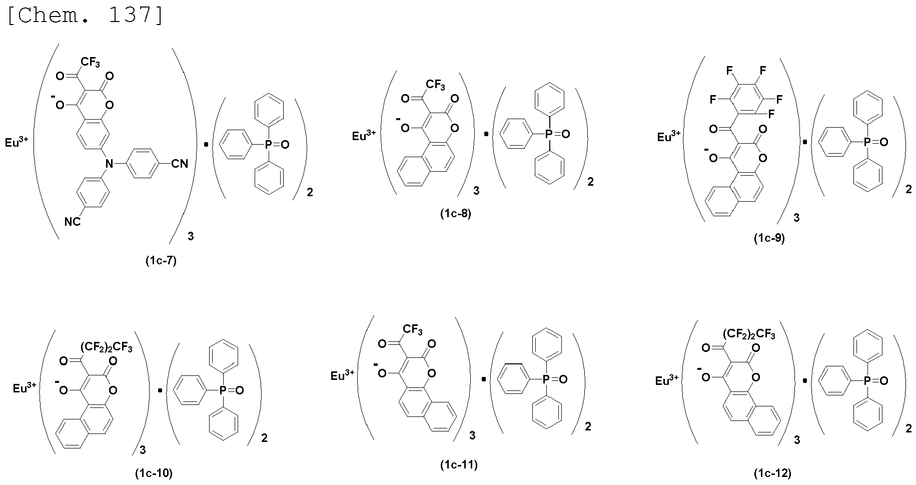

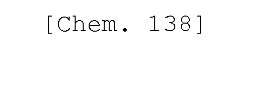

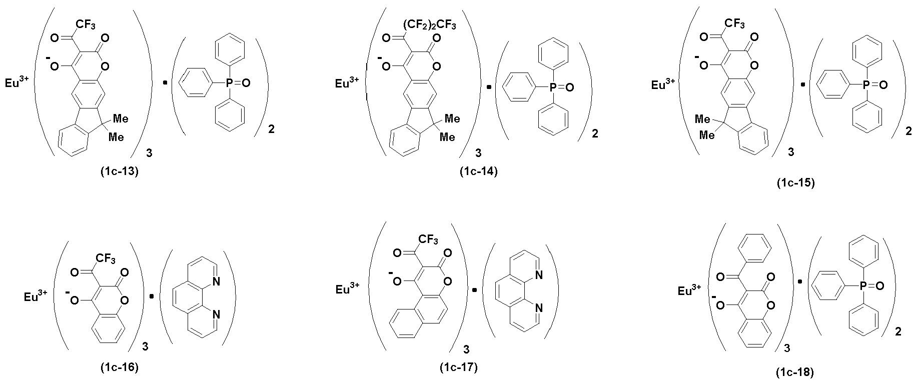

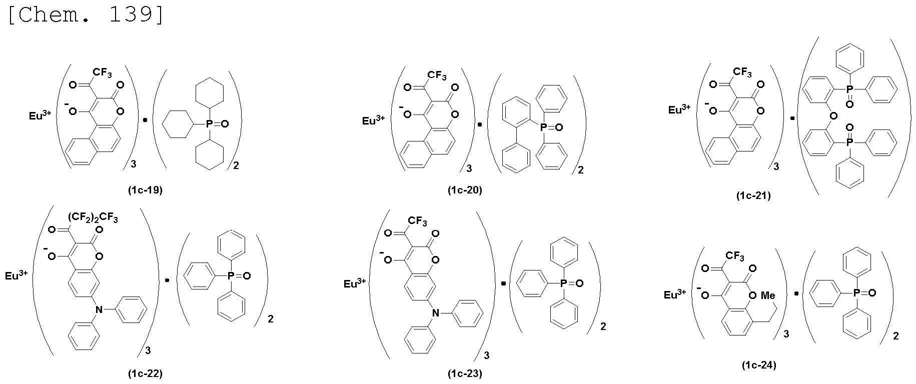

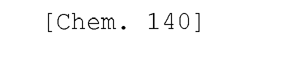

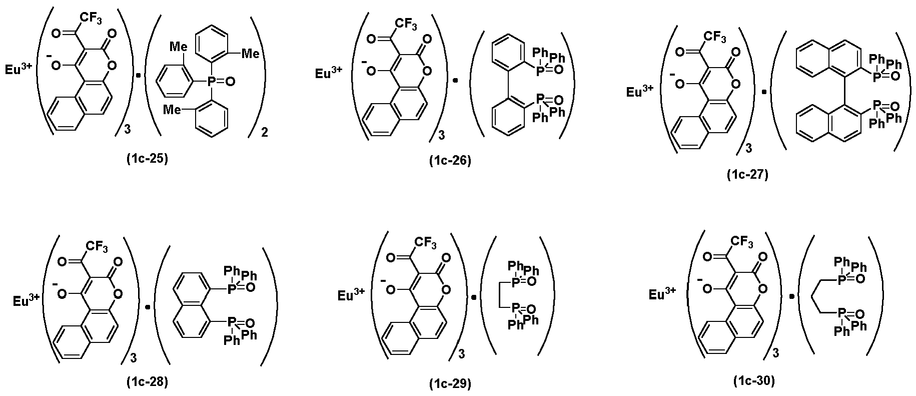

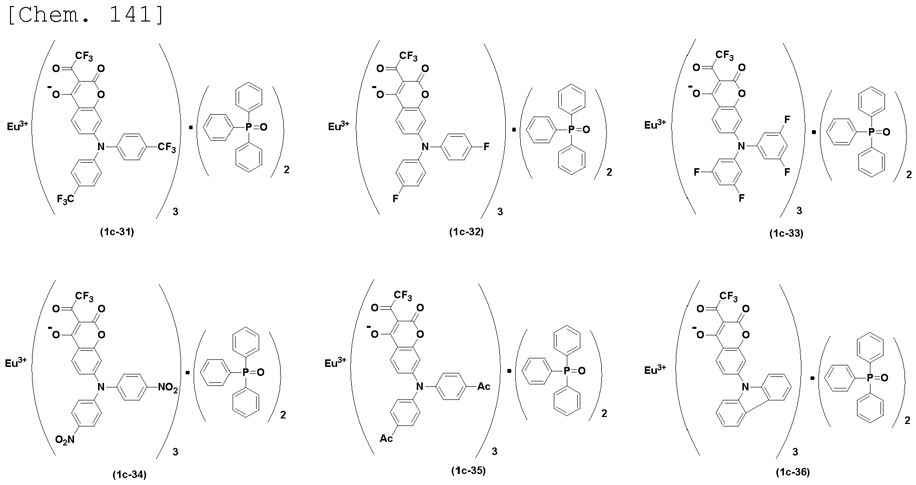

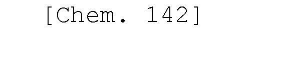

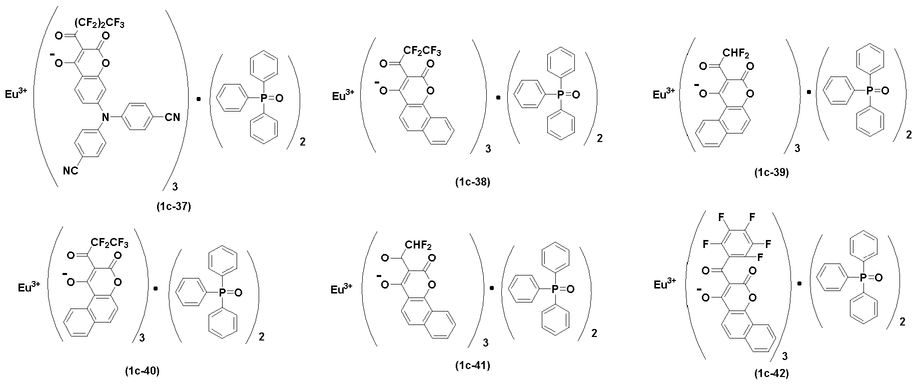

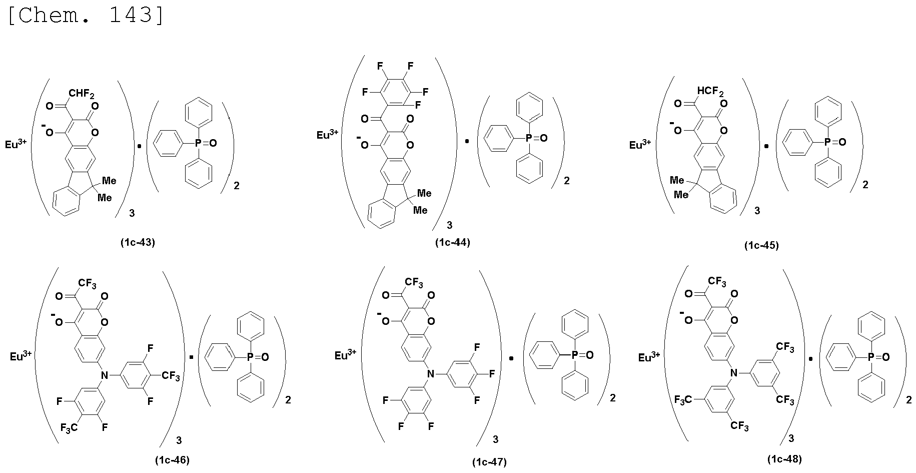

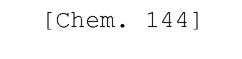

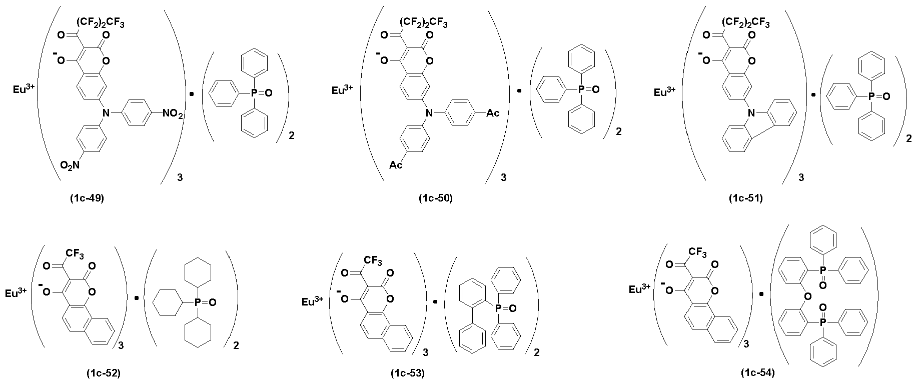

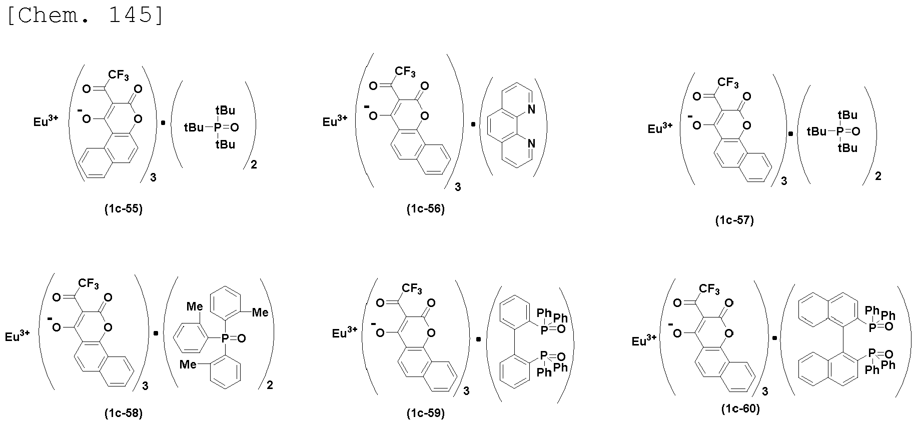

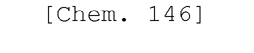

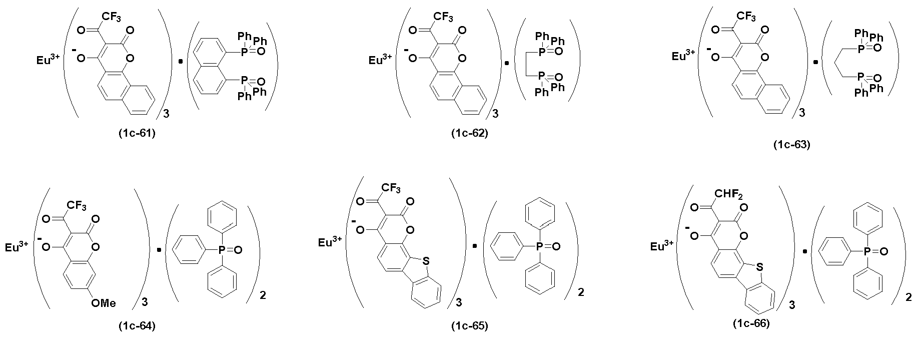

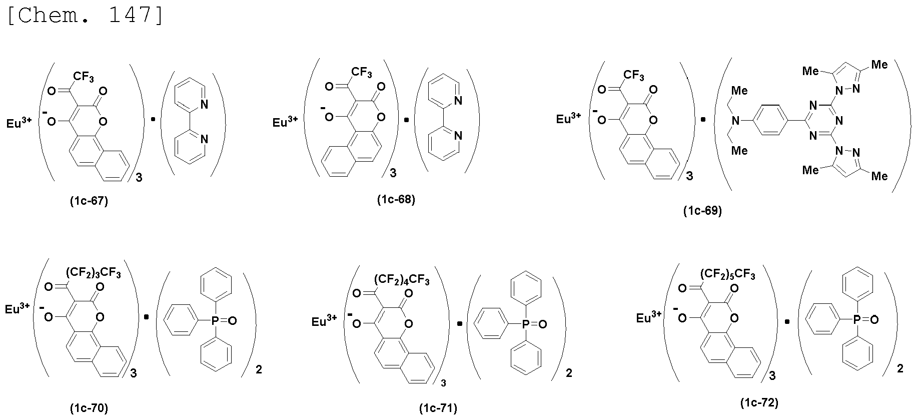

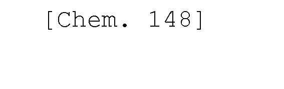

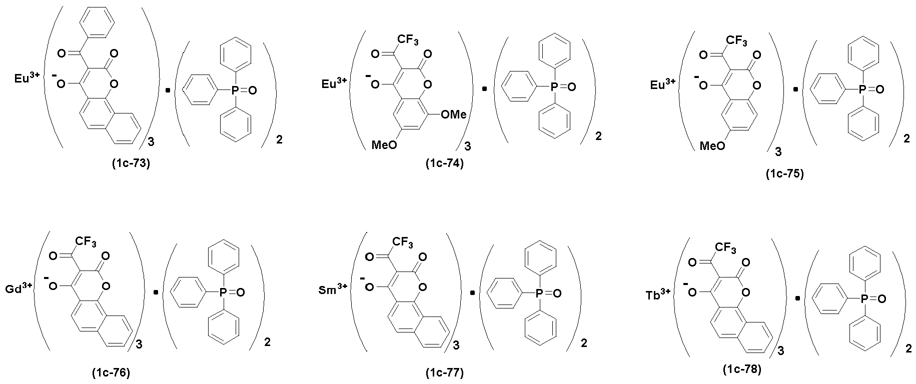

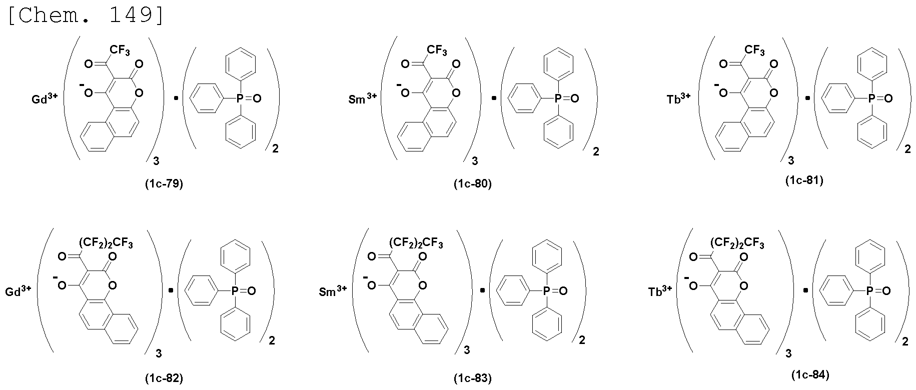

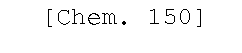

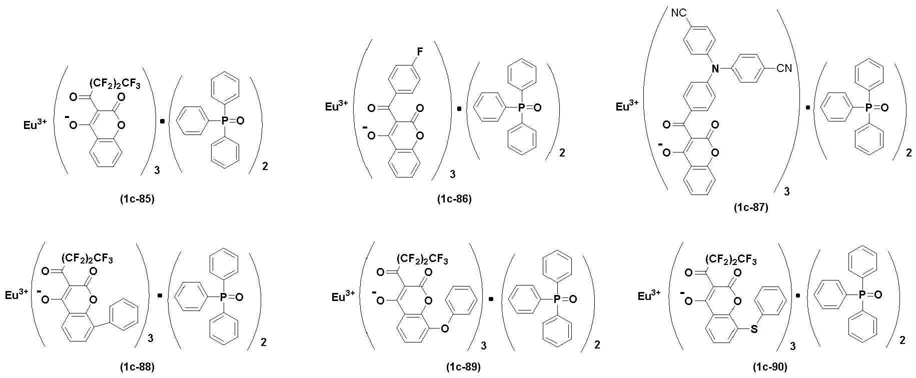

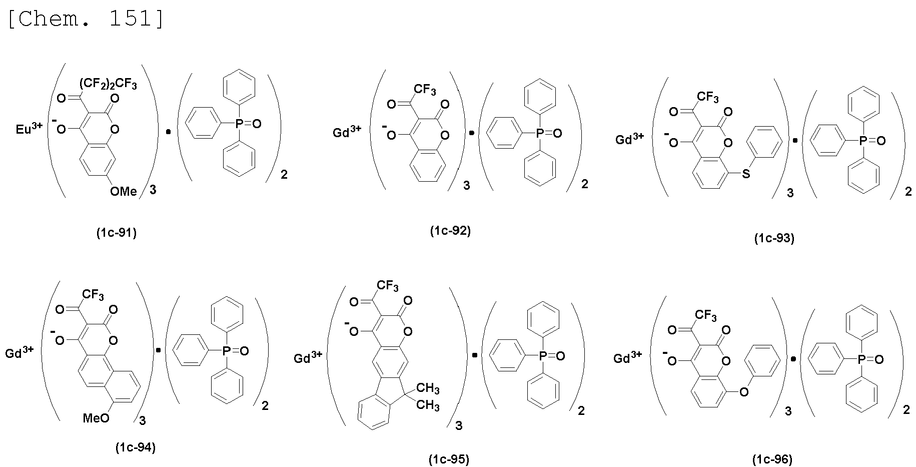

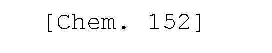

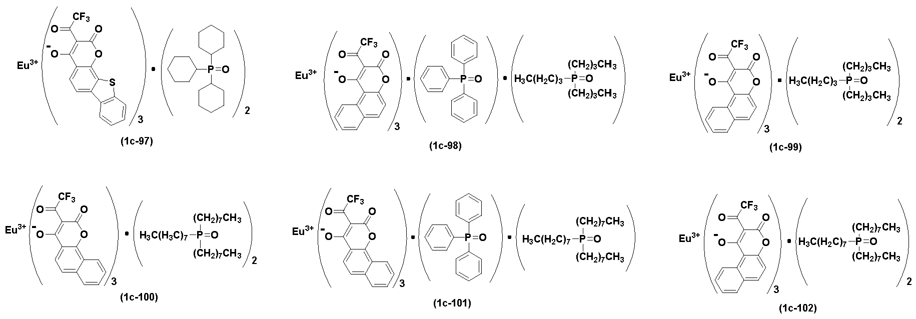

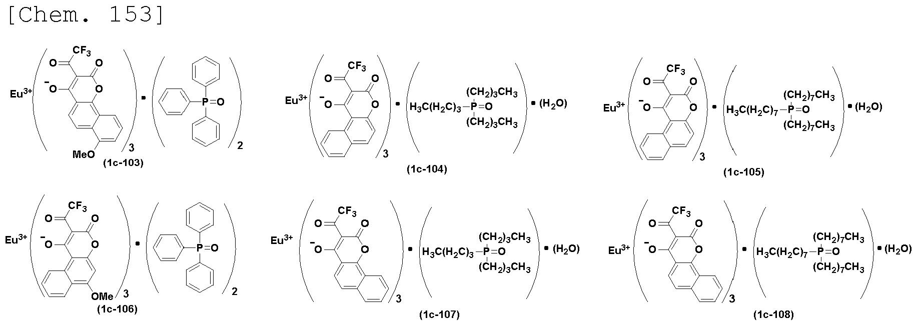

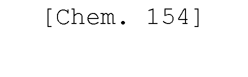

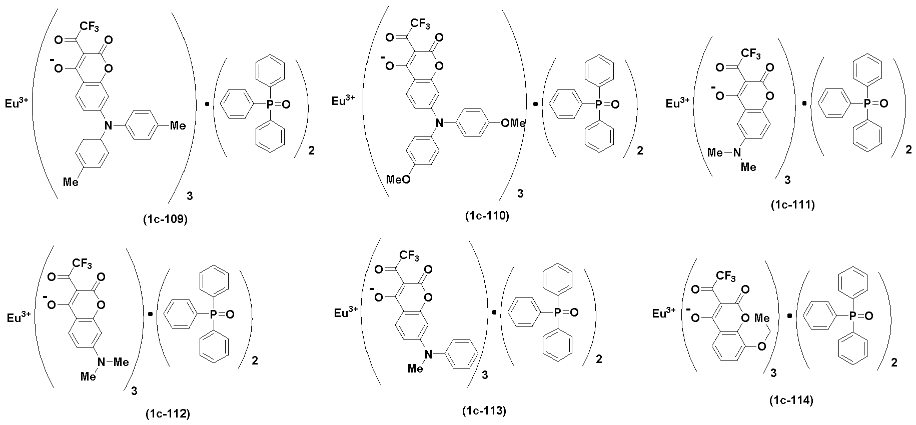

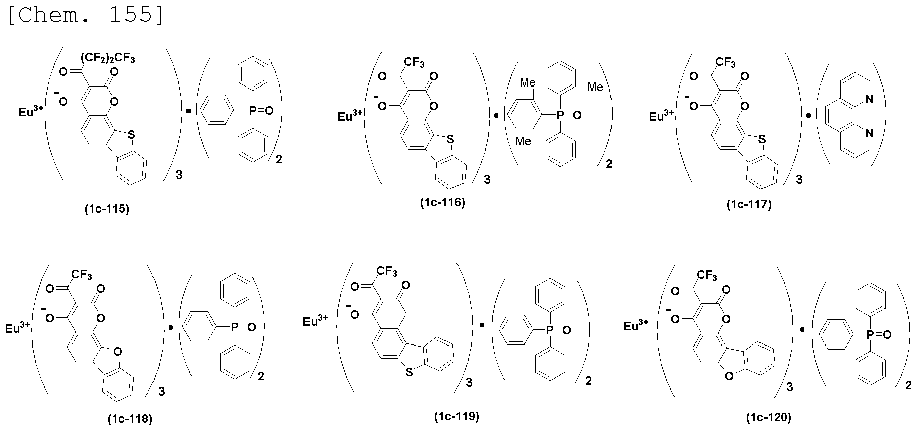

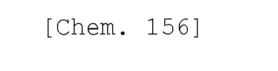

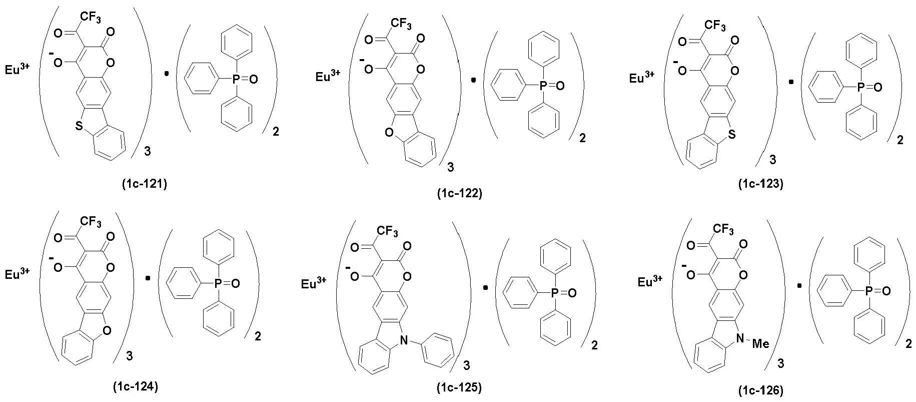

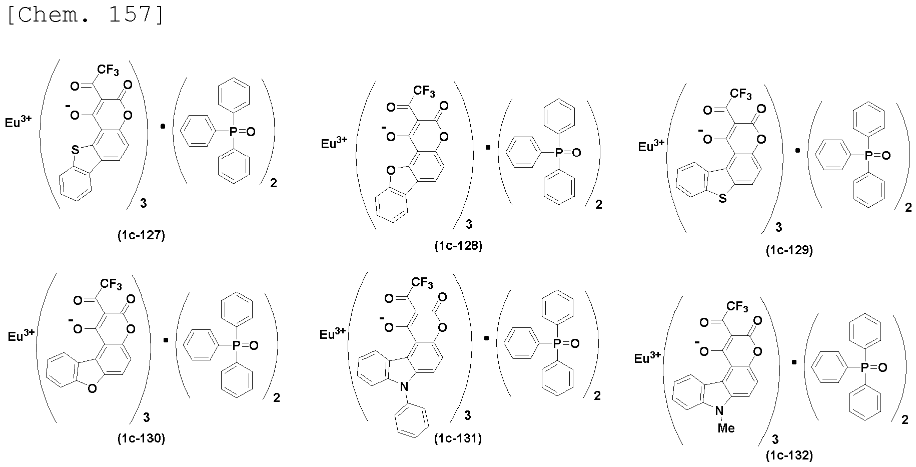

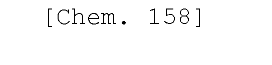

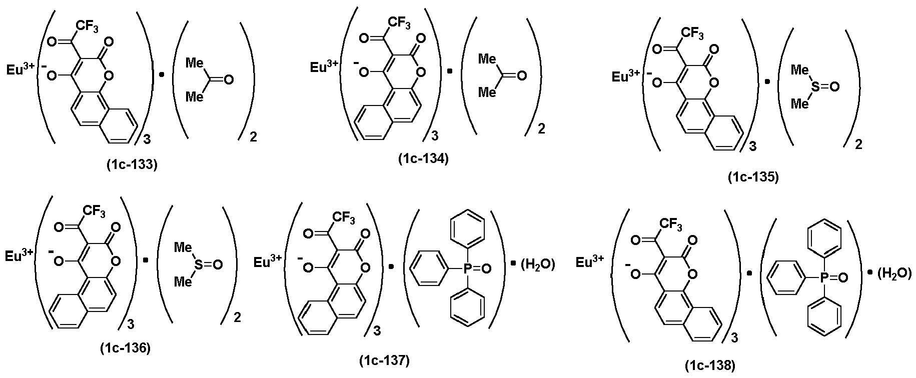

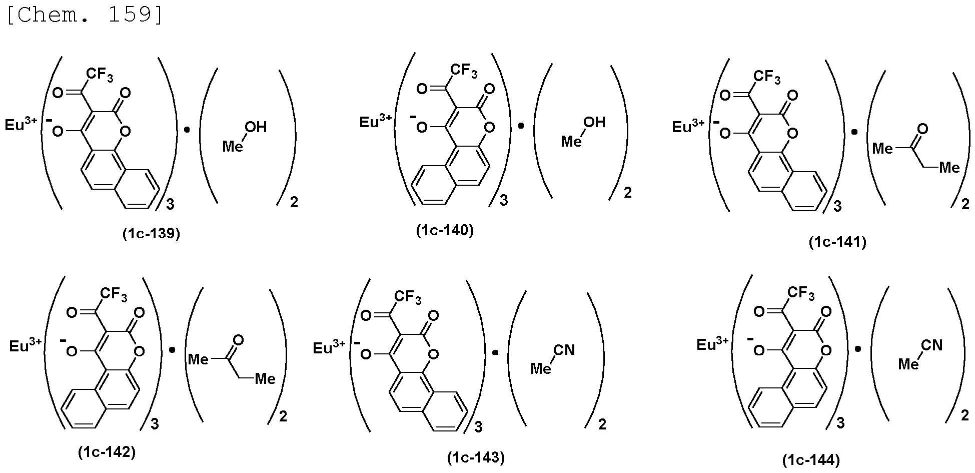

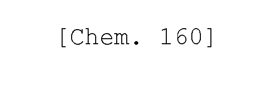

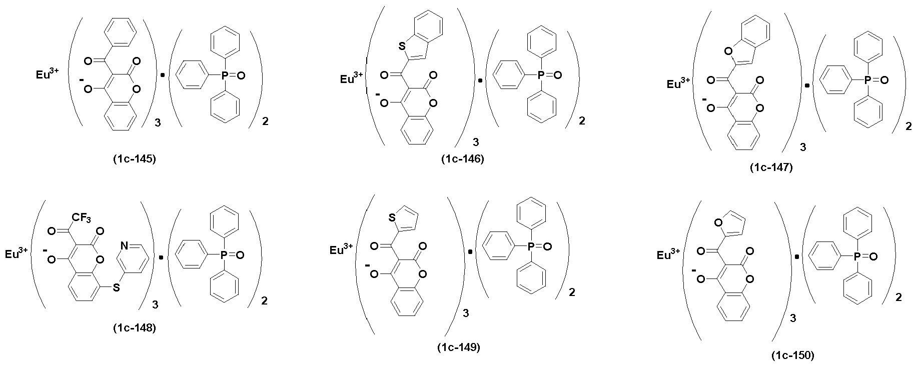

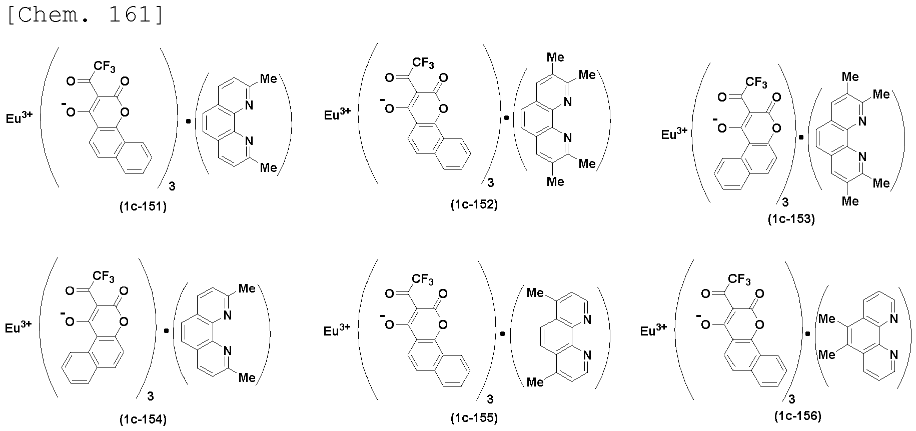

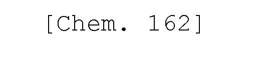

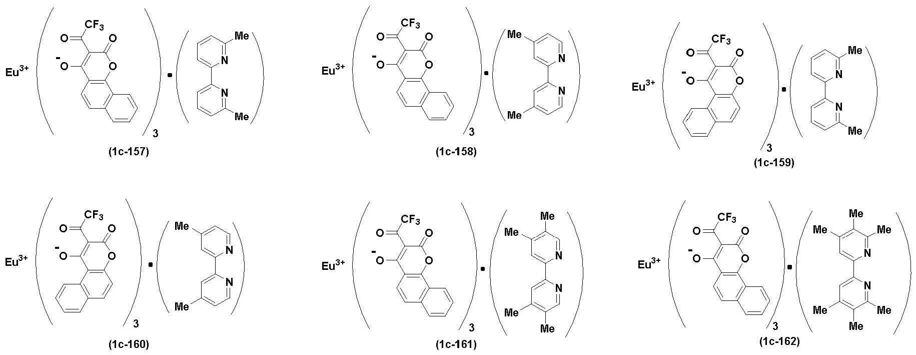

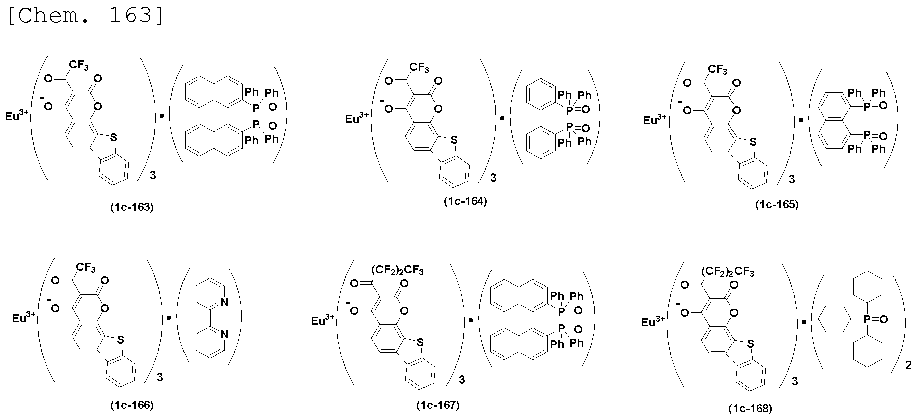

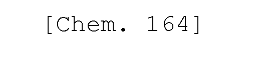

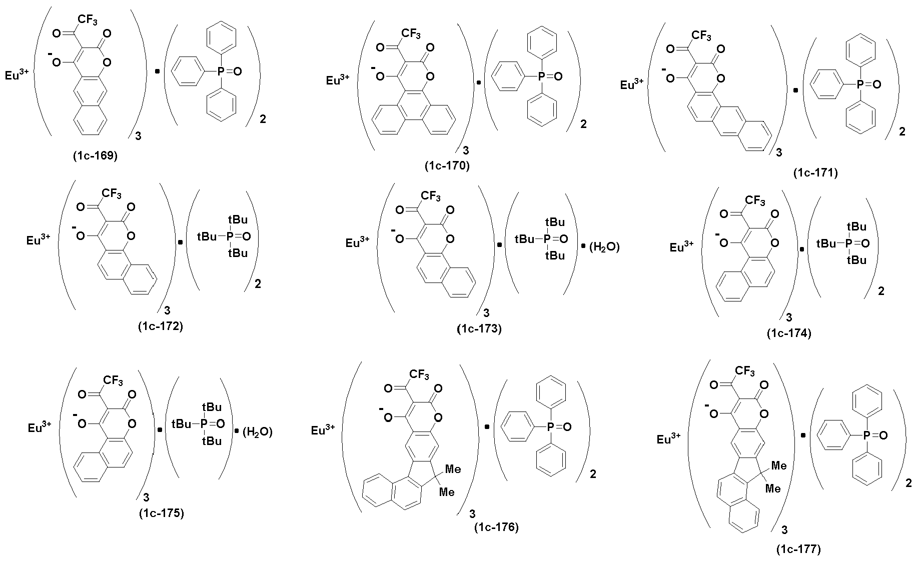

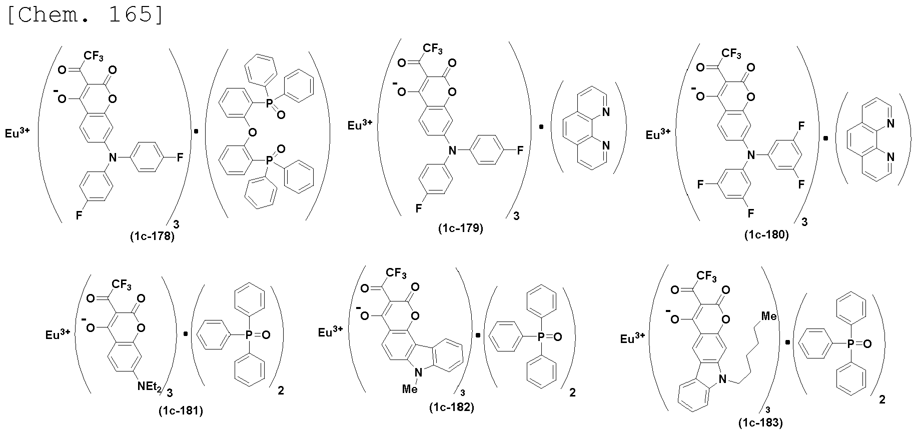

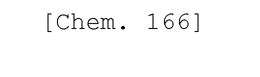

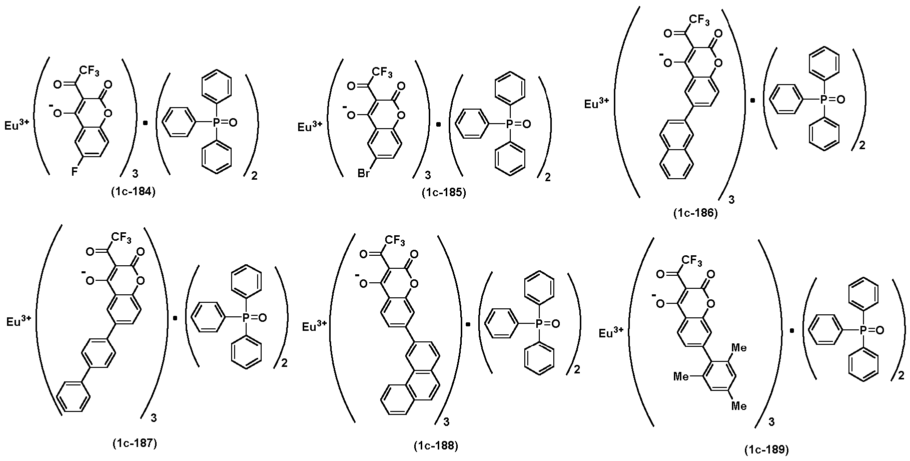

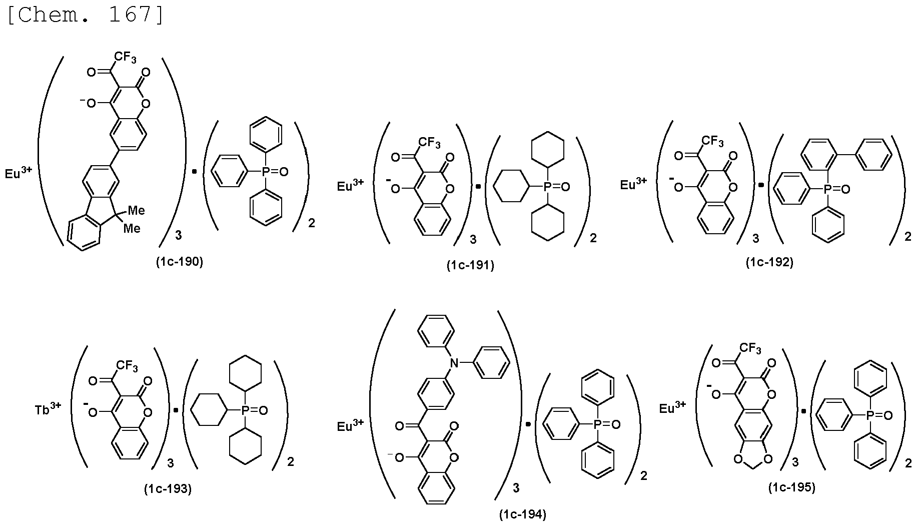

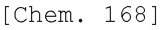

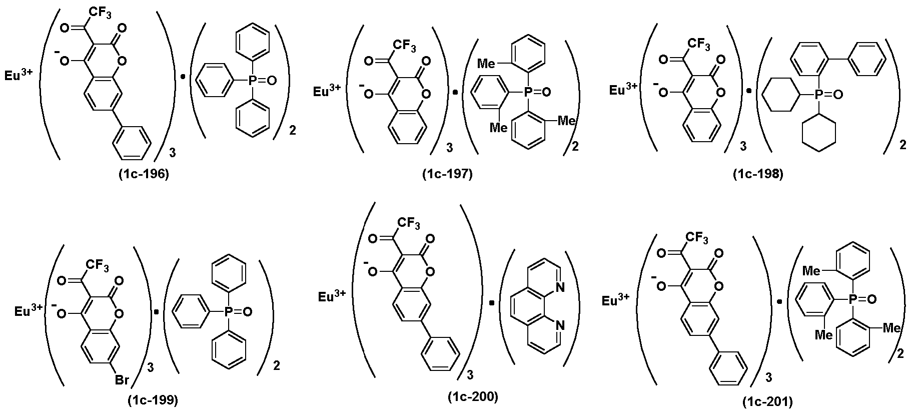

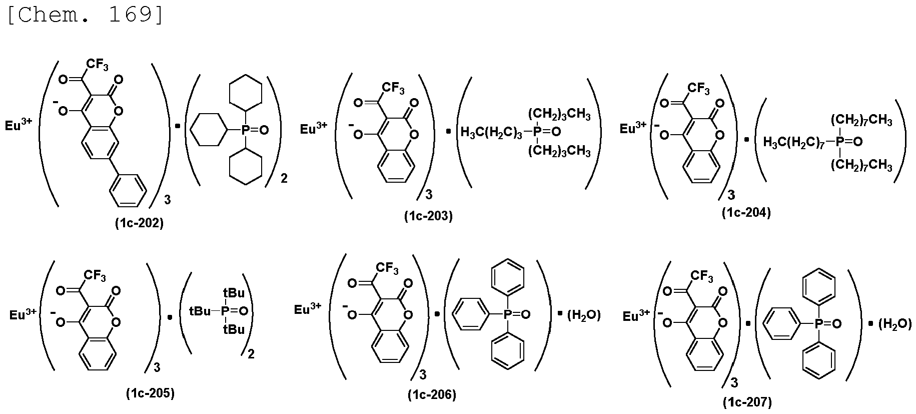

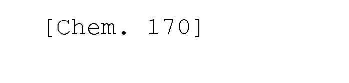

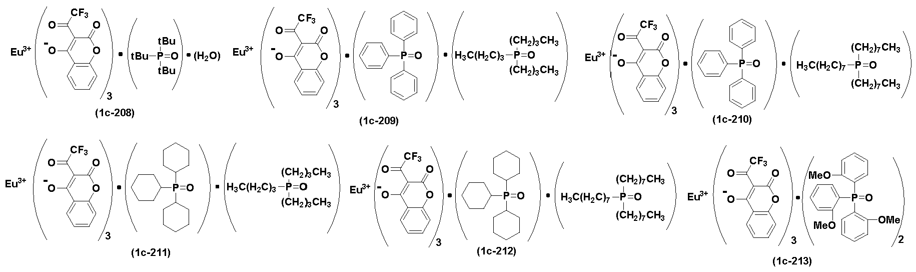

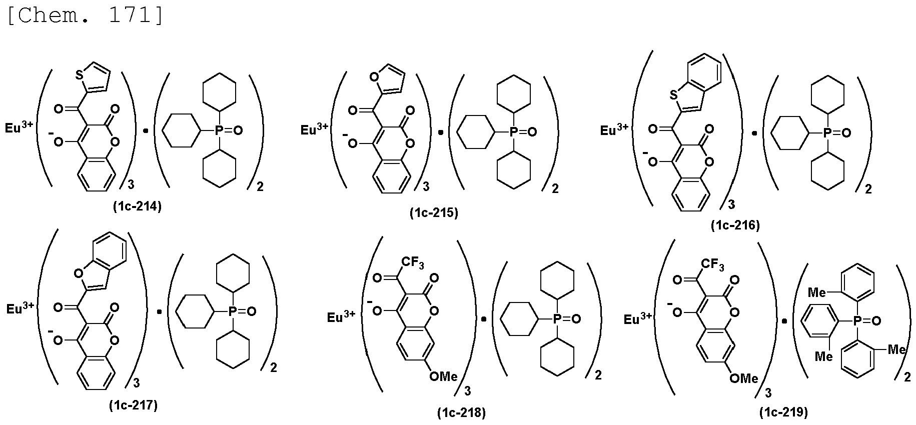

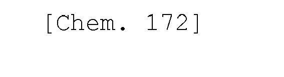

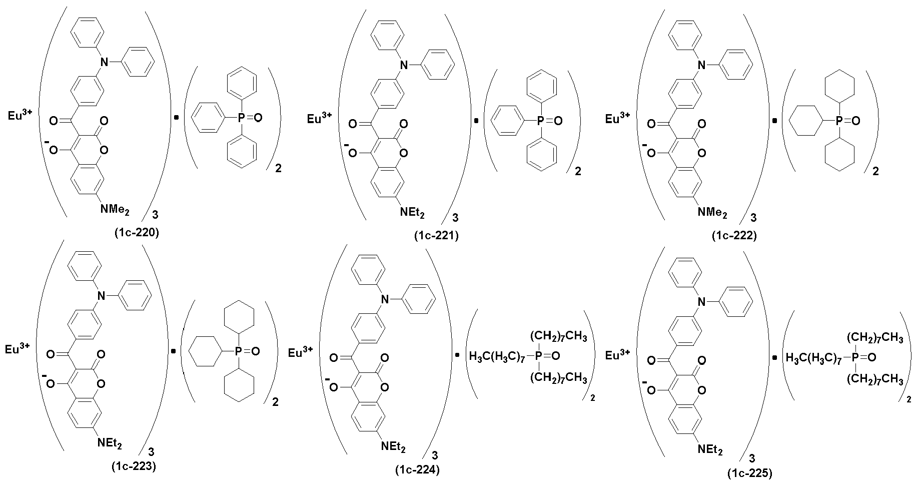

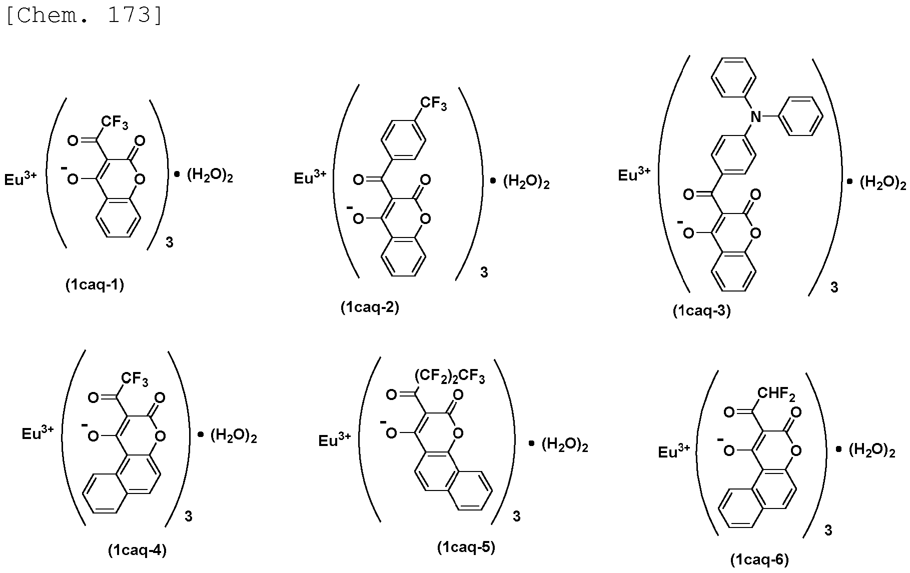

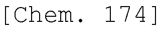

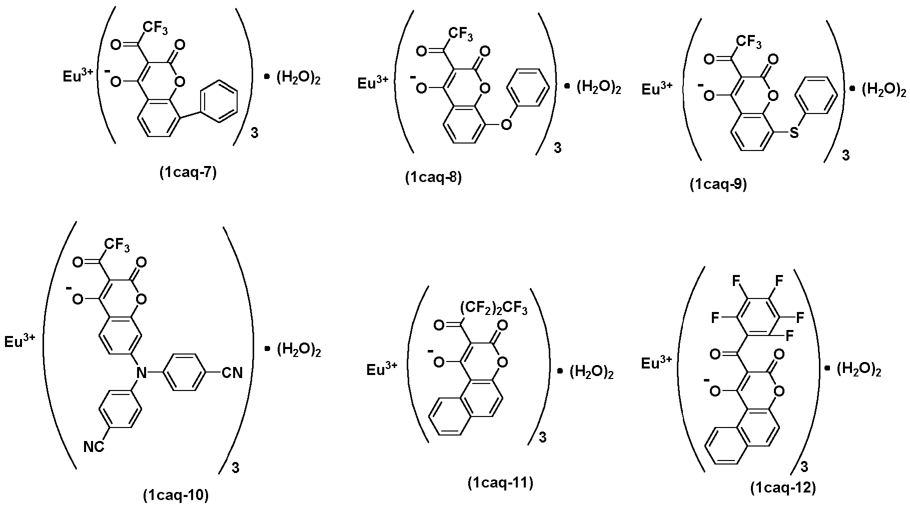

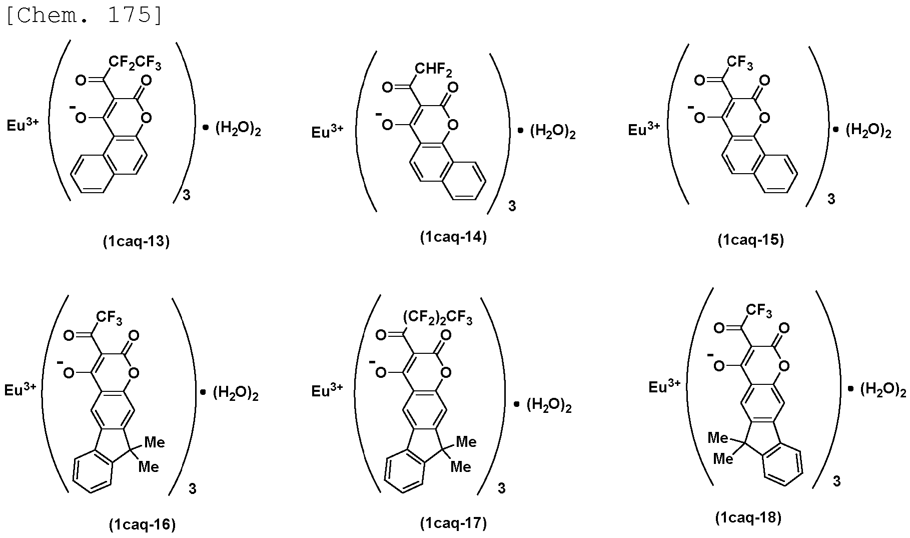

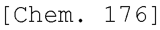

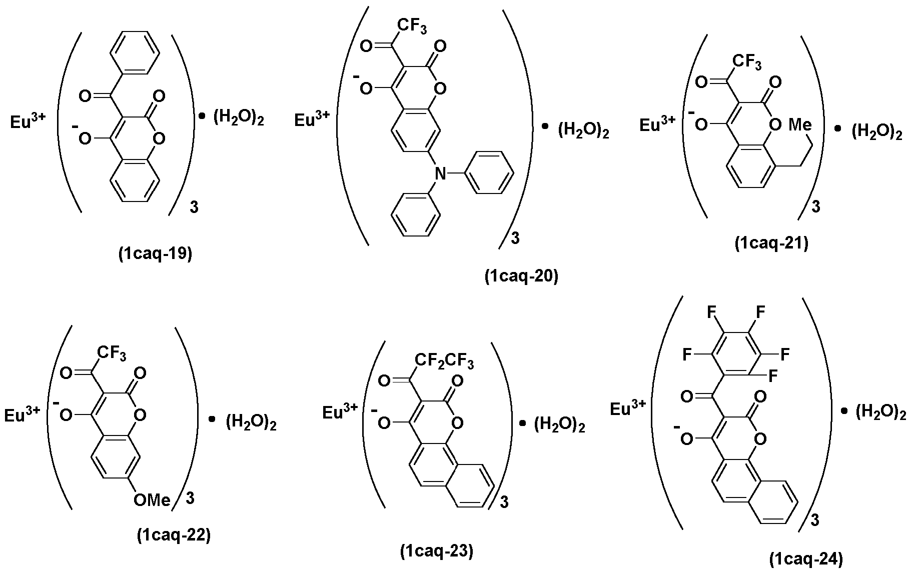

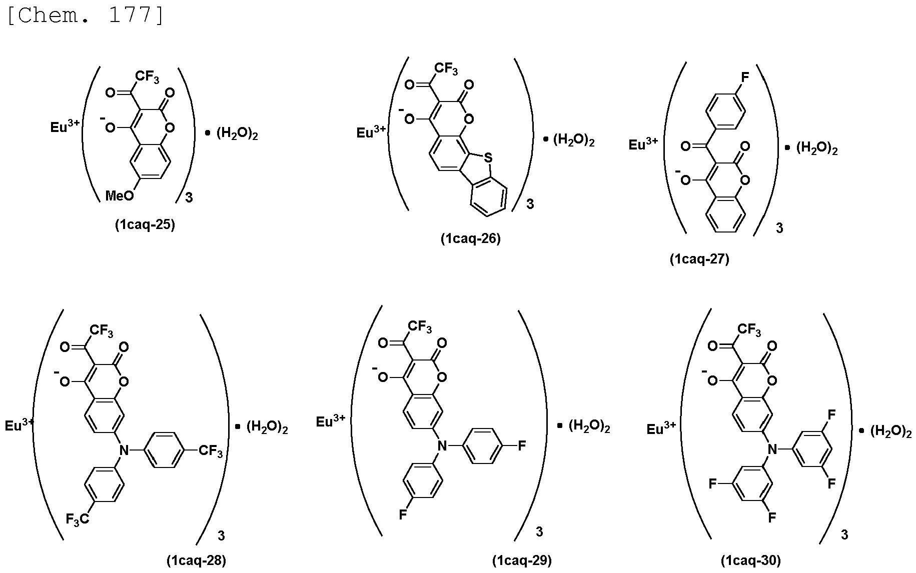

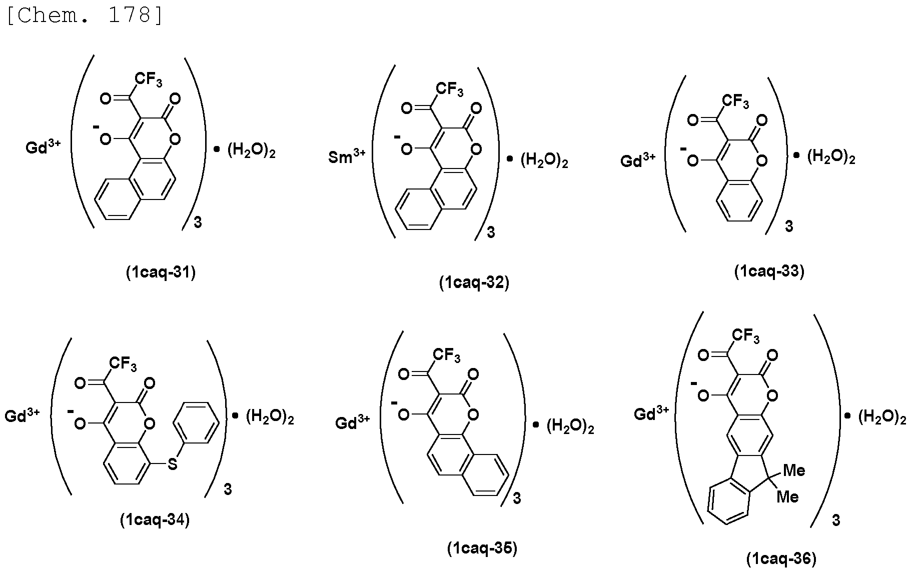

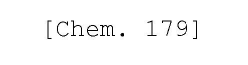

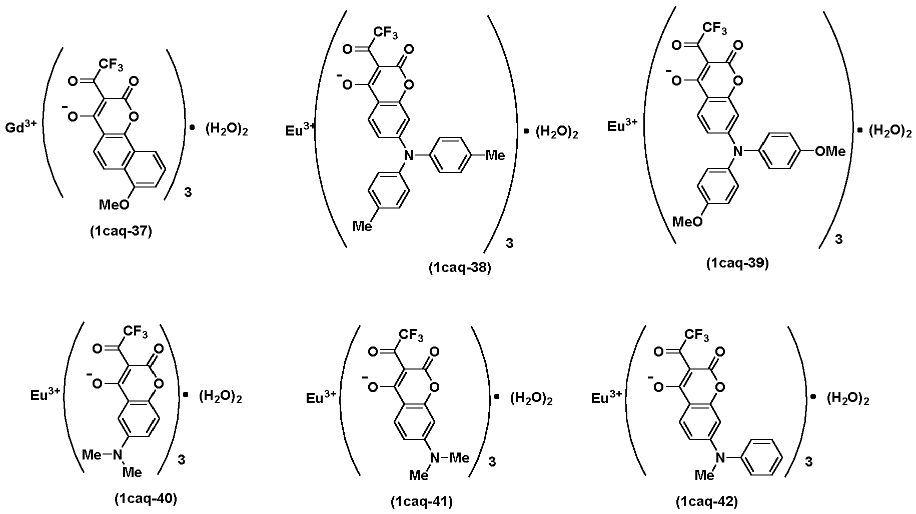

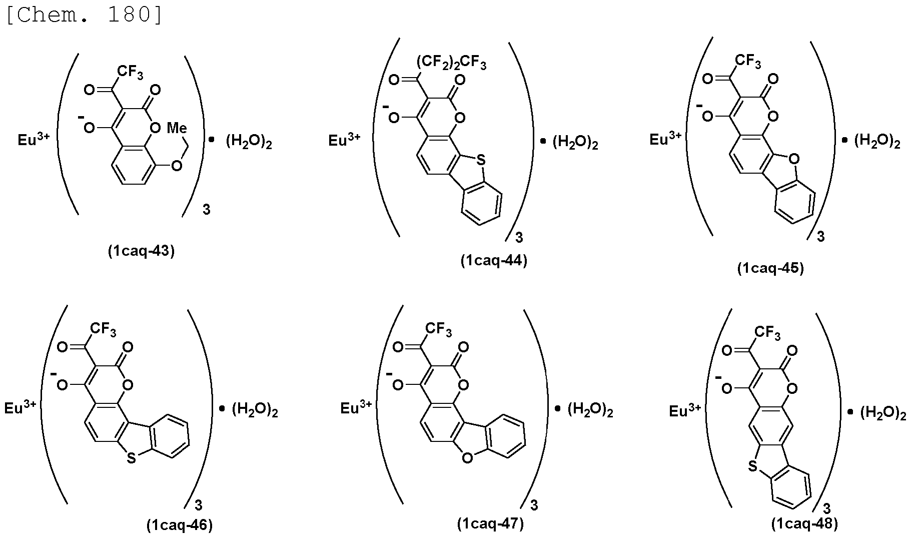

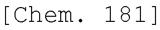

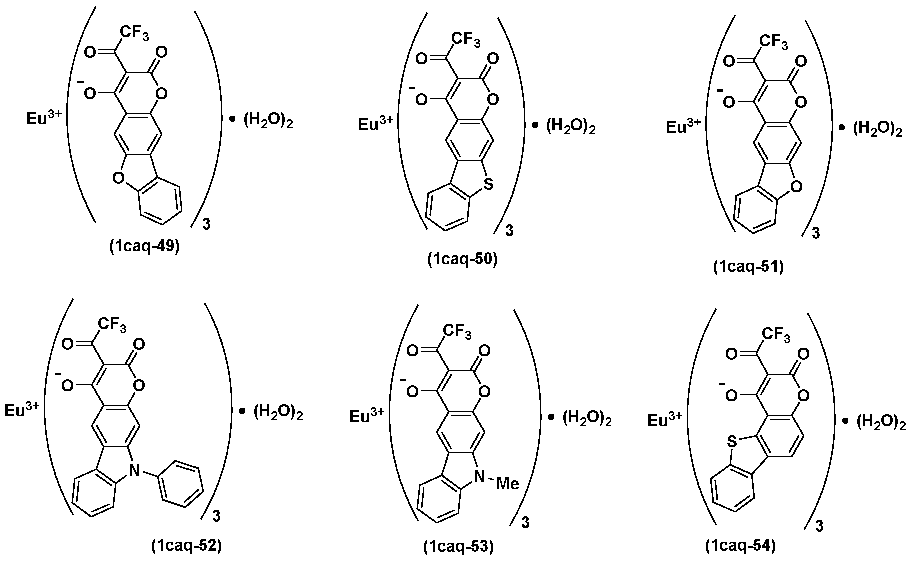

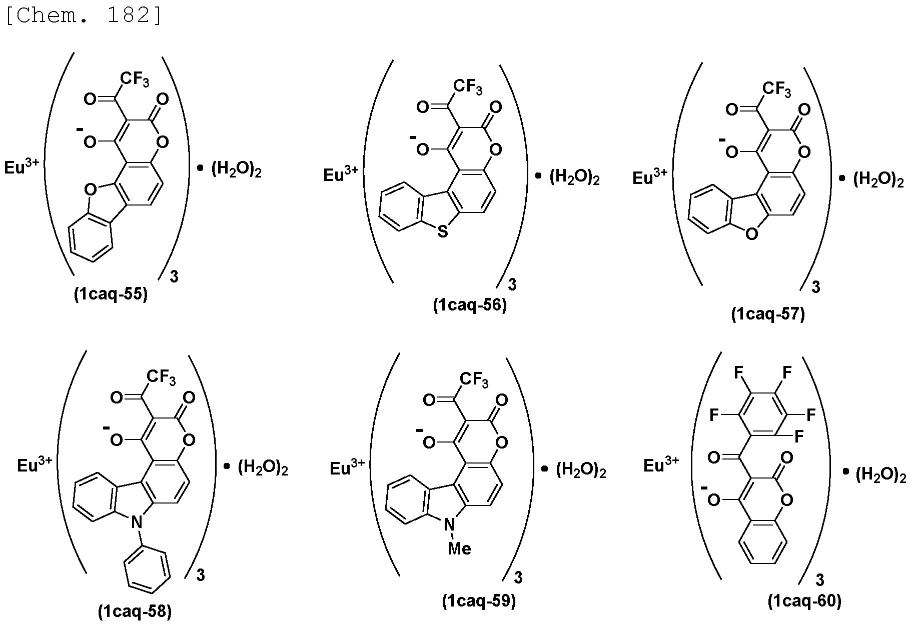

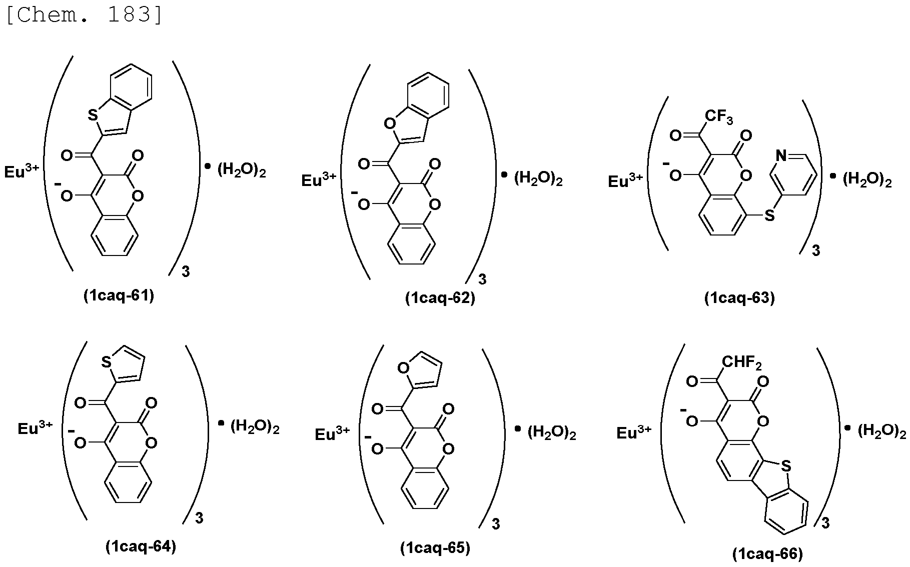

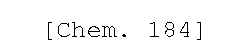

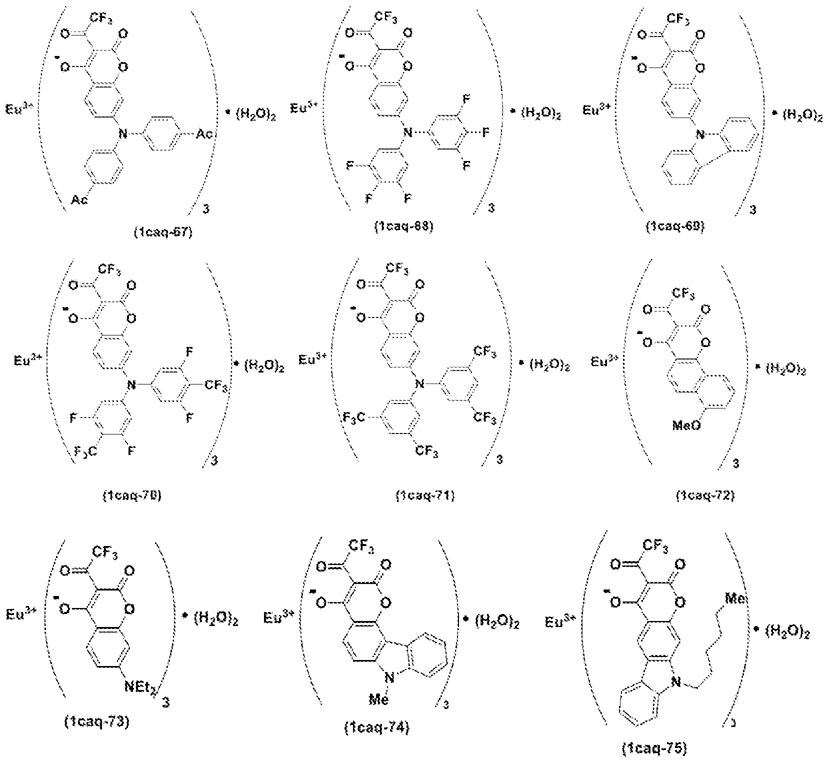

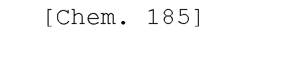

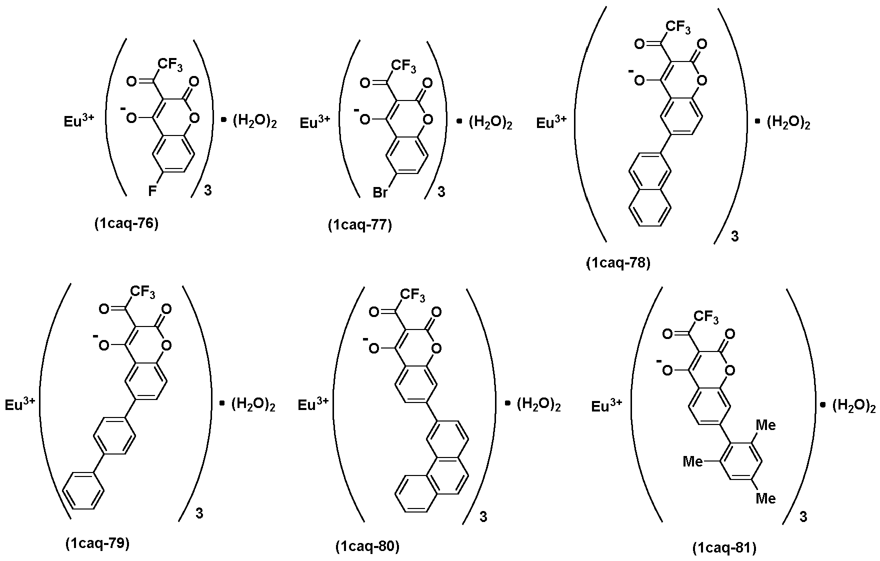

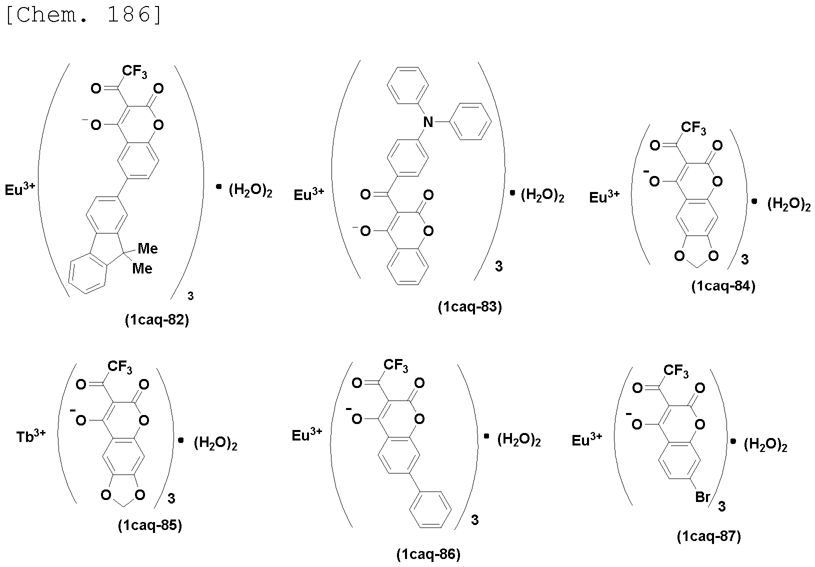

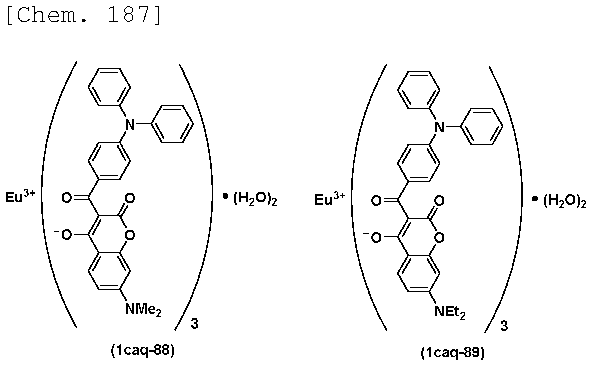

Of formulae (1c-1) to (1c-225) and formulae (1caq-1) to (1caq-89) above, the compounds represented by formulae (1c-1) to (1c-8), (1c-10) to (1c-12), (1c-16) to (1c-23), (1c-25) to (1c-33), (1c-35) to (1c-41), (1c-47) to (1c-48), (1c-50) to (1c-68), (1c-70) to (1c-89), (1c-91) to (1c-94), (1c-96) to (1c-108), (1c-111) to (1c-147), (1c-149) to (1c-168), (1c-172) to (1c-175), (1c-178) to (1c-185), (1c-190) to (1c-225), (1caq-1) to (1caq-11), (1caq-13) to (1caq-15), (1caq-19) to (1caq-20), (1caq-22) to (1caq-23), (1caq-25) to (1caq-35), (1caq-37), (1caq-40) to (1caq-59), (1caq-61) to (1caq-69), (1caq-71) to (1caq-77) or (1caq-82) to (1caq-89) are preferred because of easy availability of raw materials, the compounds represented by formulae (1c-1) to (1c-3), (1c-7) to (1c-8), (1c-10) to (1c-12), (1c-16) to (1c-23), (1c-25) to (1c-33), (1c-38) to (1c-41), (1c-52) to (1c-68), (1c-76) to (1c-85), (1c-91) to (1c-92), (1c-94), (1c-97), (1c-99) to (1c-100), (1c-106), (1c-112), (1c-115) to (1c-122), (1c-124) to (1c-147), (1c-149) to (1c-168), (1c-172) to (1c-175), (1c-178) to (1c-185), (1c-190) to (1c-218), (1caq-1) to (1caq-6), (1caq-10) to (1caq-11), (1caq-13) to (1caq-15), (1caq-19) to (1caq-20), (1caq-22) to (1caq-23), (1caq-25) to (1caq-33), (1caq-35), (1caq-37), (1caq-41), (1caq-44) to (1caq-49), (1caq-51) to (1caq-62), (1caq-64) to (1caq-66), (1caq-72) to (1caq-77) or (1caq-82) to (1caq-87) are more preferred because of the ease of synthesis, and formulae (1c-7) to (1c-8), (1c-11), (1c-23), (1c-31) to (1c-33), (1c-103), (1c-178), (1c-183) or (1c-194) is particularly preferred because it gives the complex optical characteristics suitable for use as an optical functional material.

An example of a more preferred embodiment is a rare earth complex represented by general formula (1ci) (Hereinafter also referred to as a rare earth complex (1ci).). Specific examples include the rare earth complexes represented by formulae (1ci-1) to (1ci-225) and formulae (1ciaq-1) to (1ciaq-89) below, but the present invention is not limited to these.

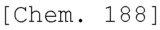

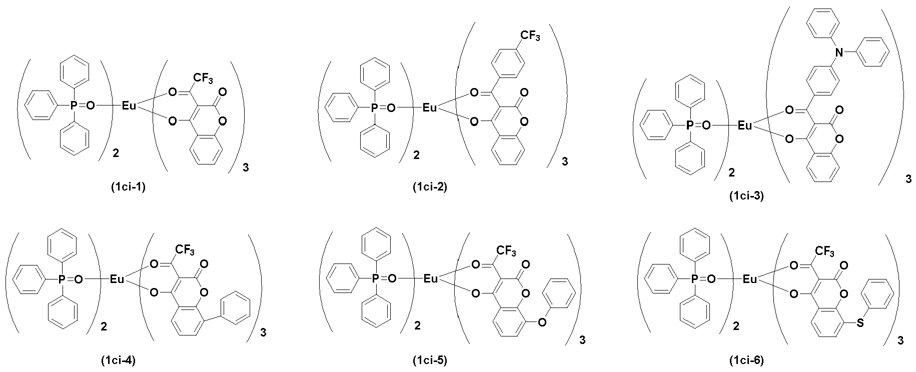

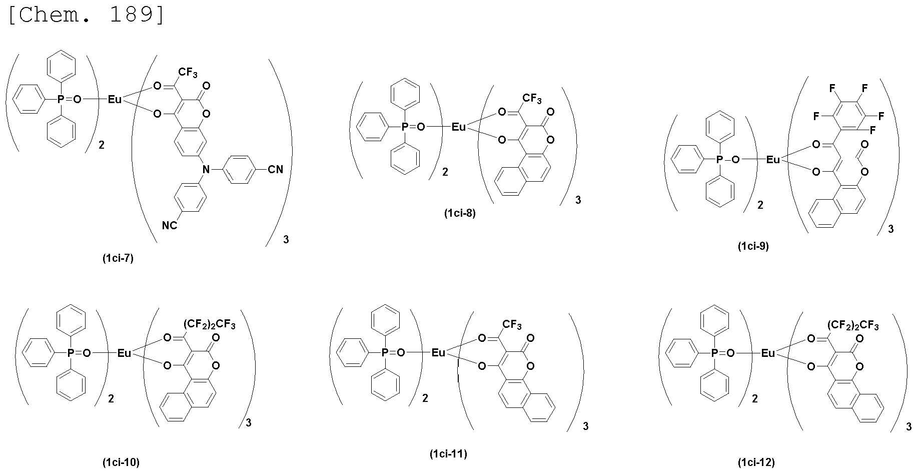

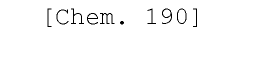

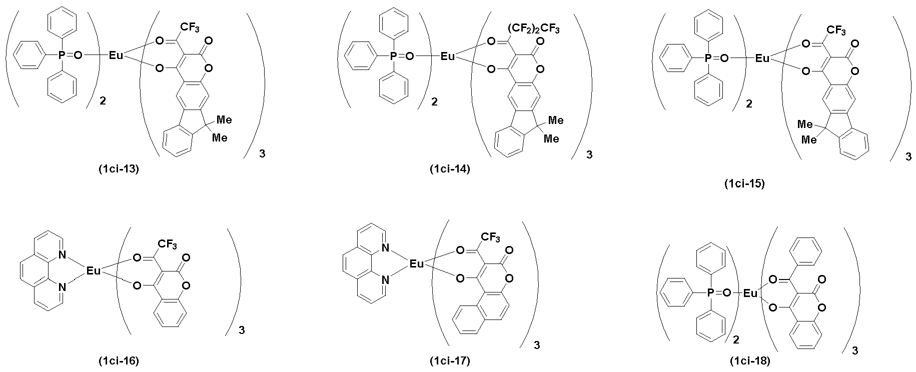

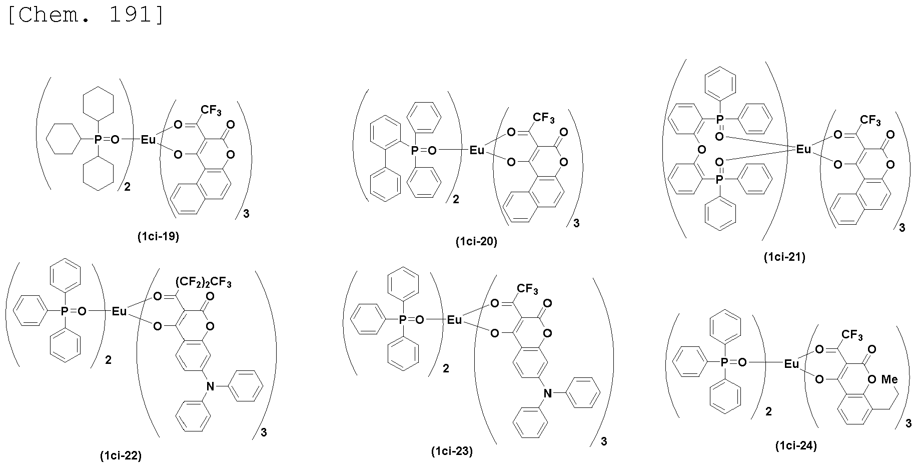

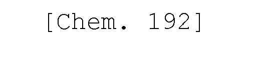

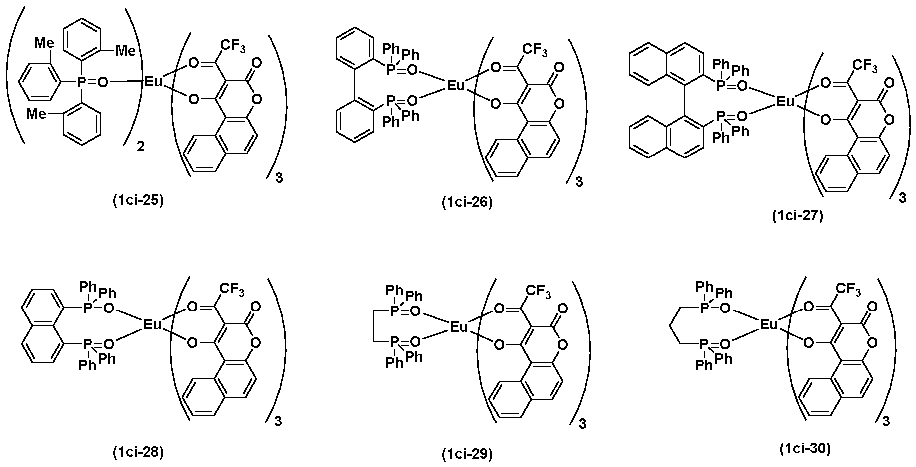

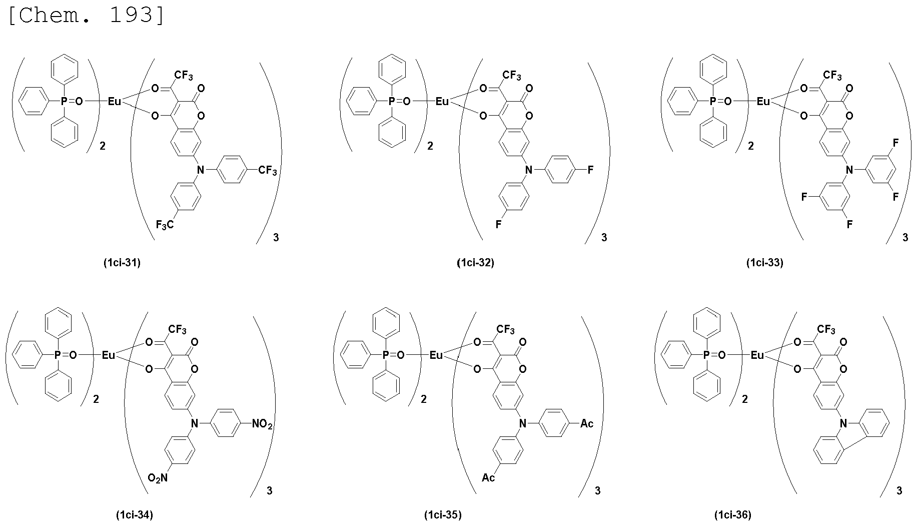

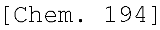

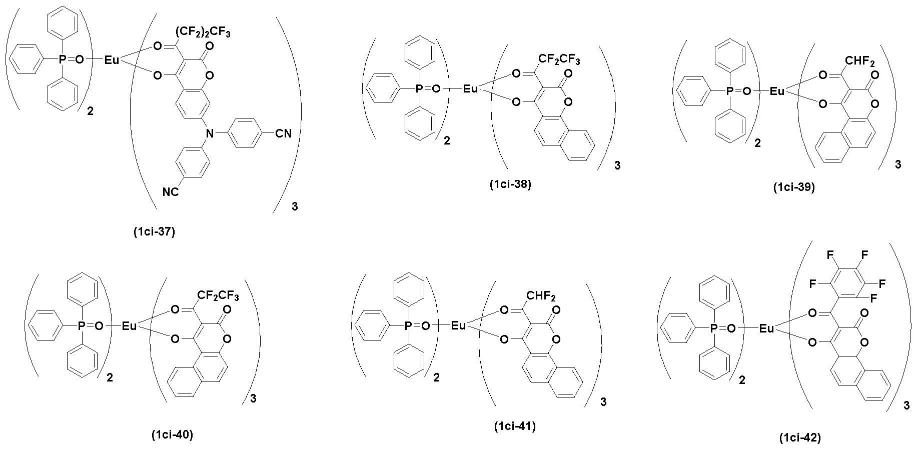

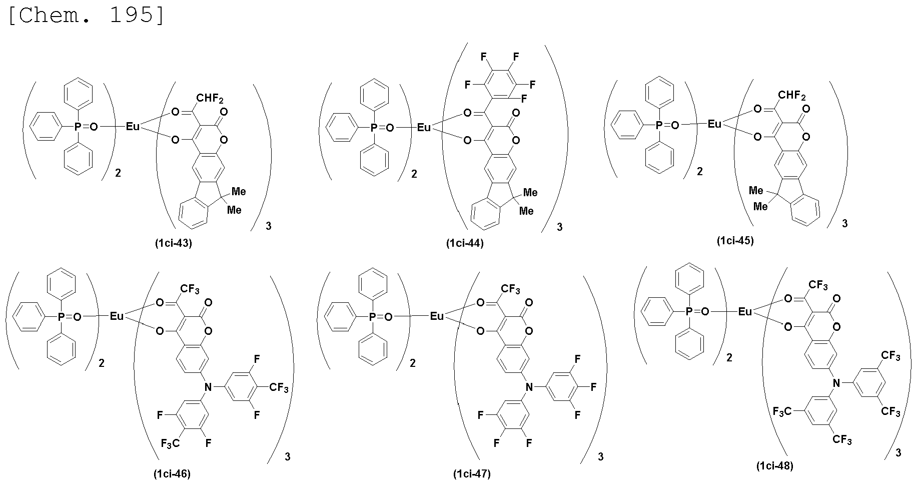

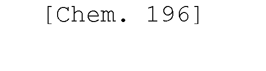

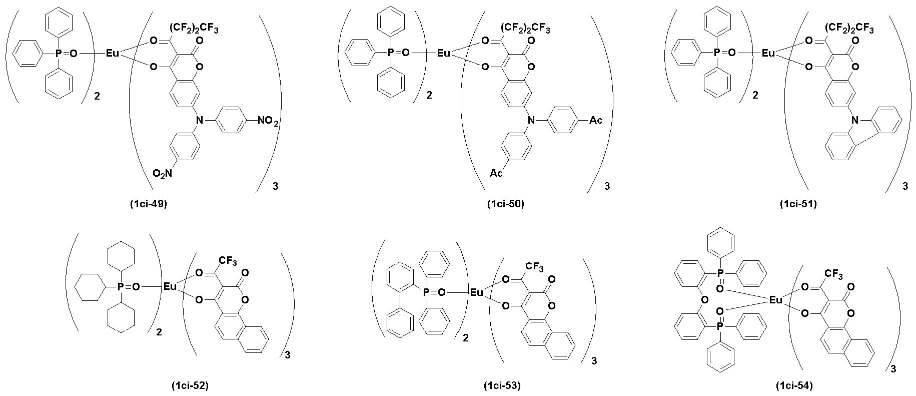

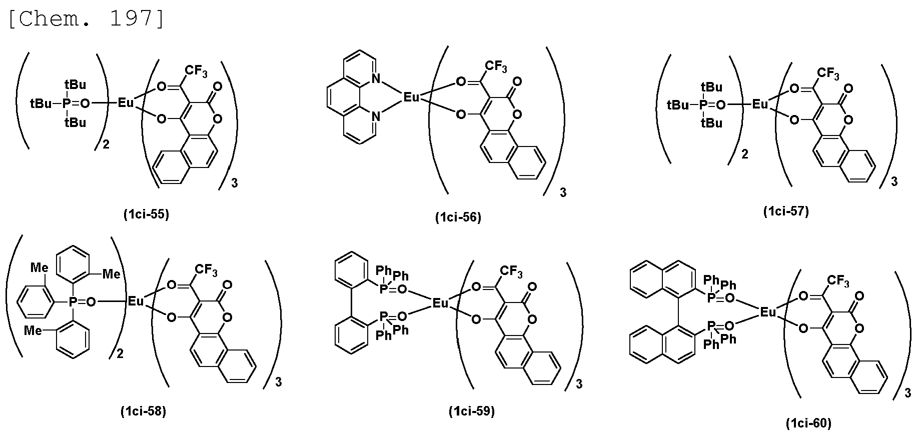

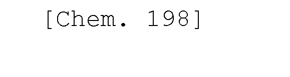

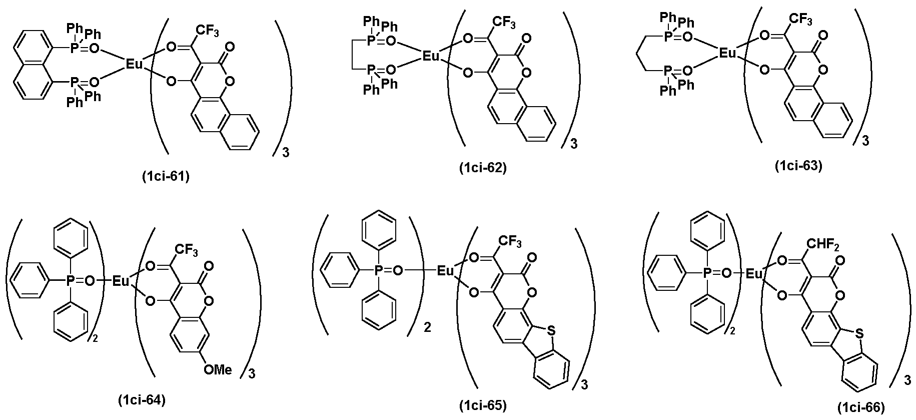

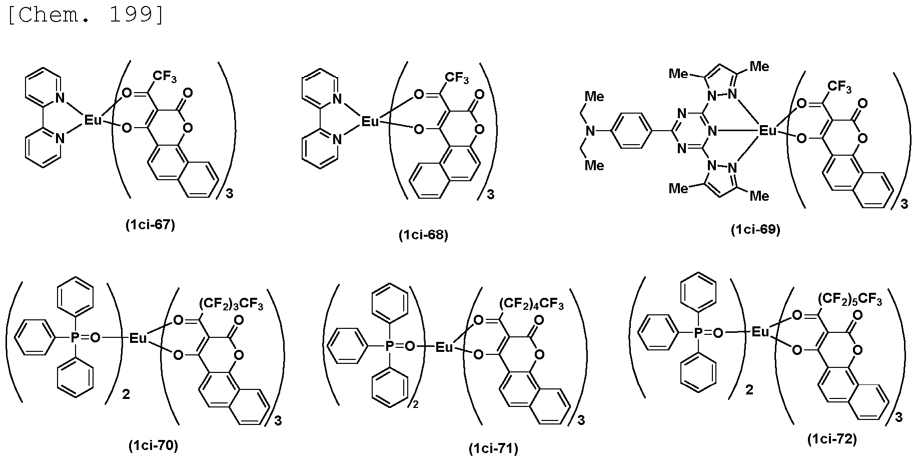

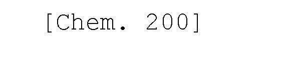

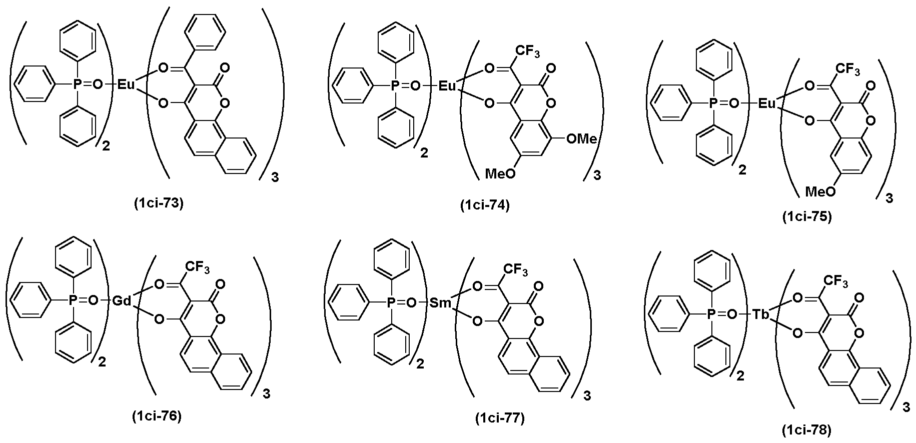

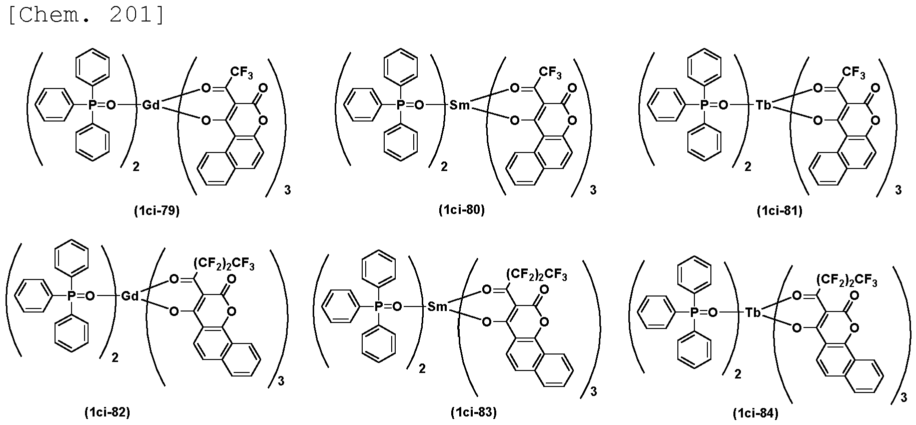

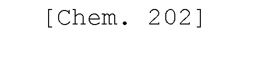

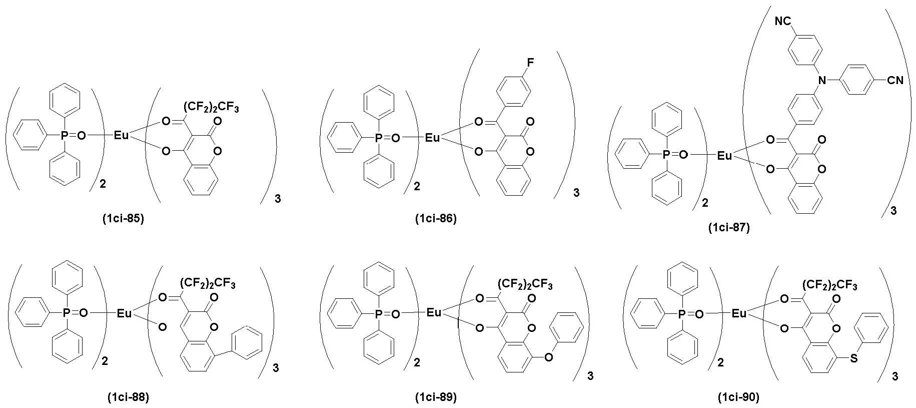

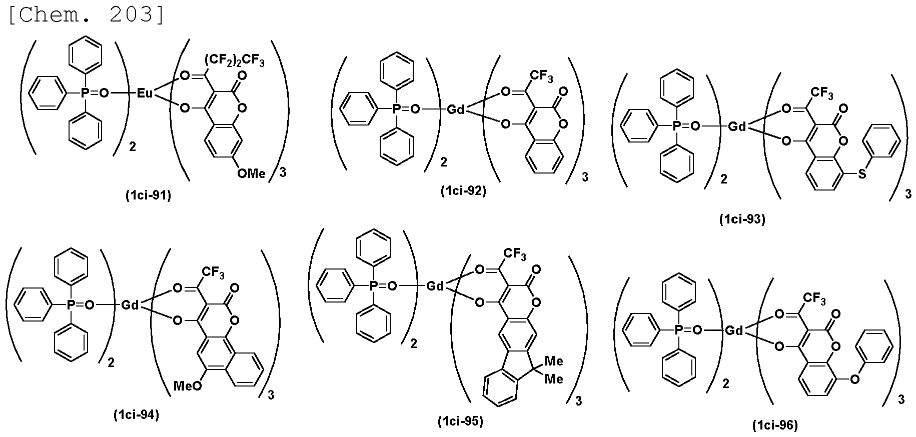

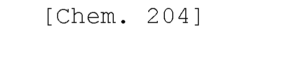

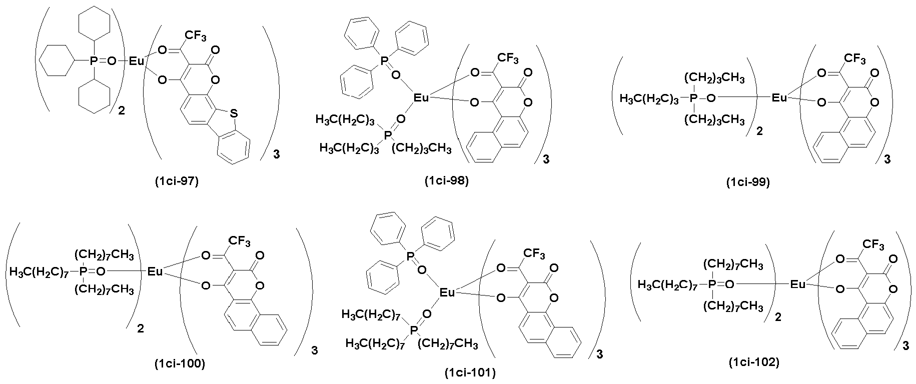

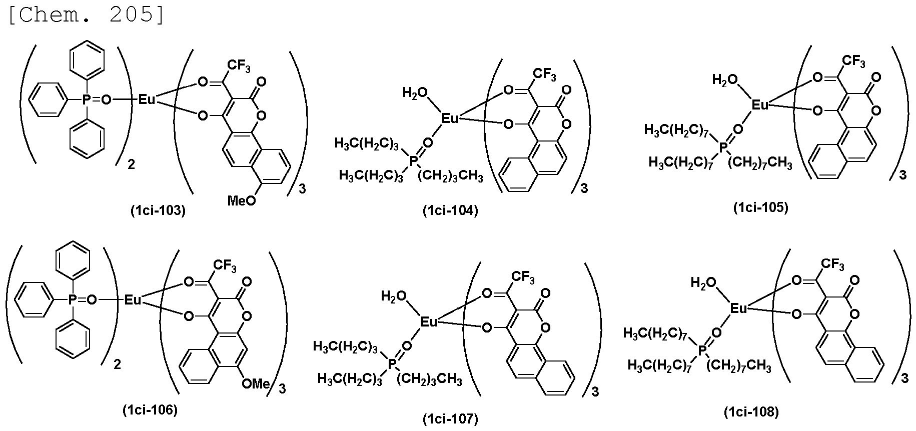

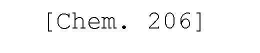

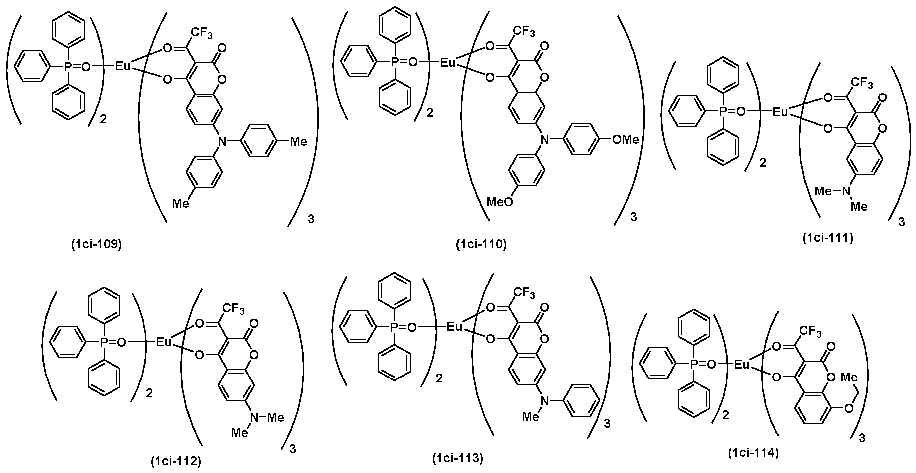

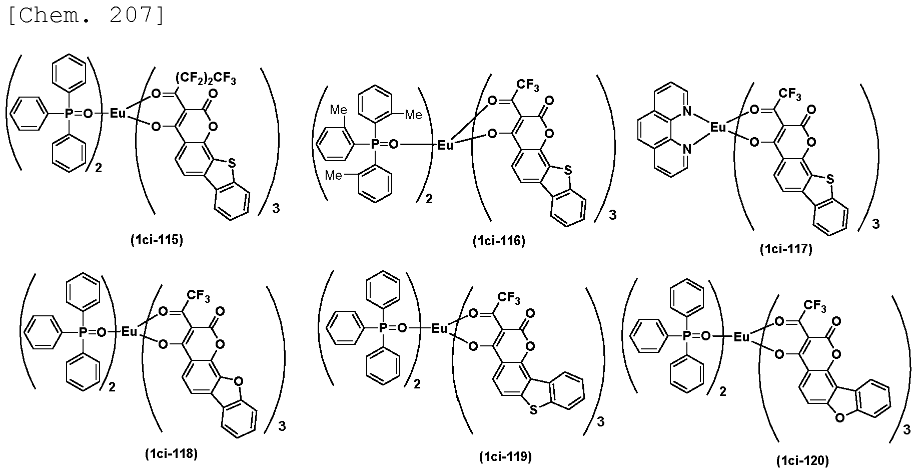

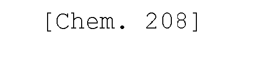

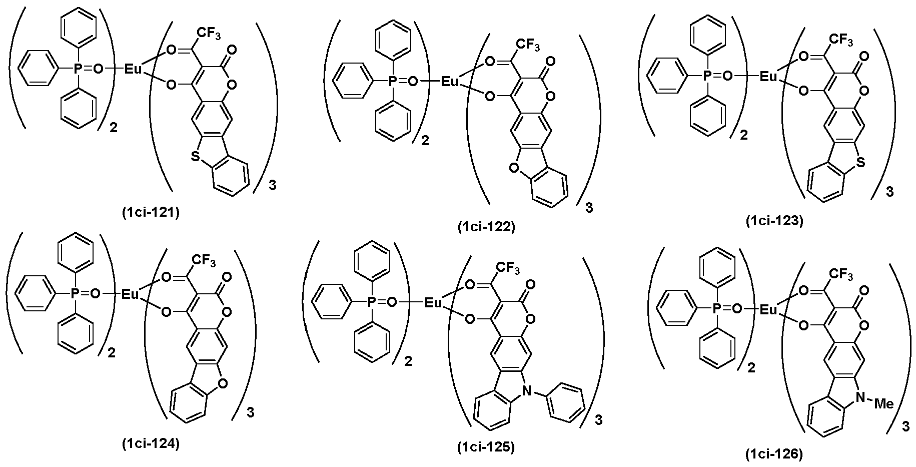

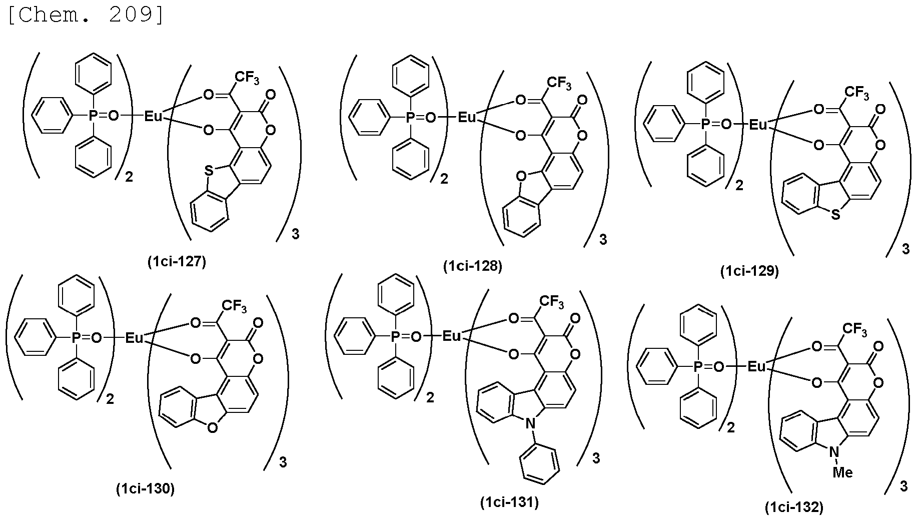

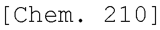

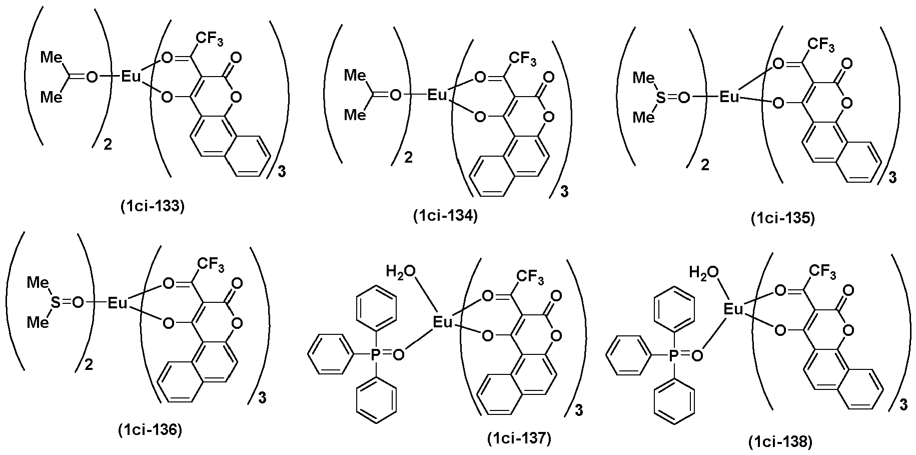

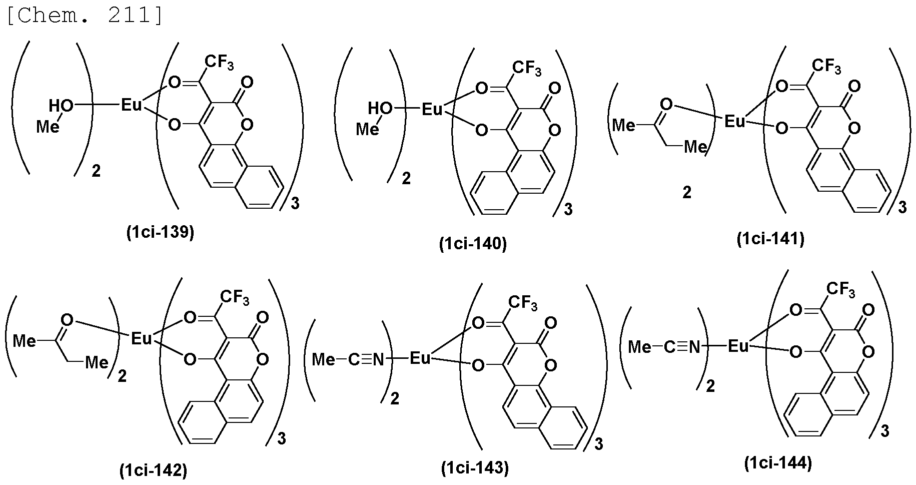

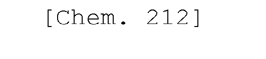

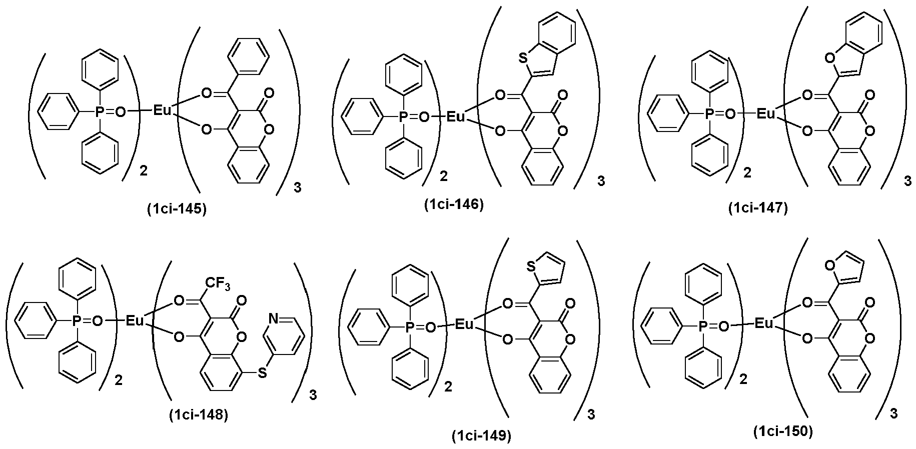

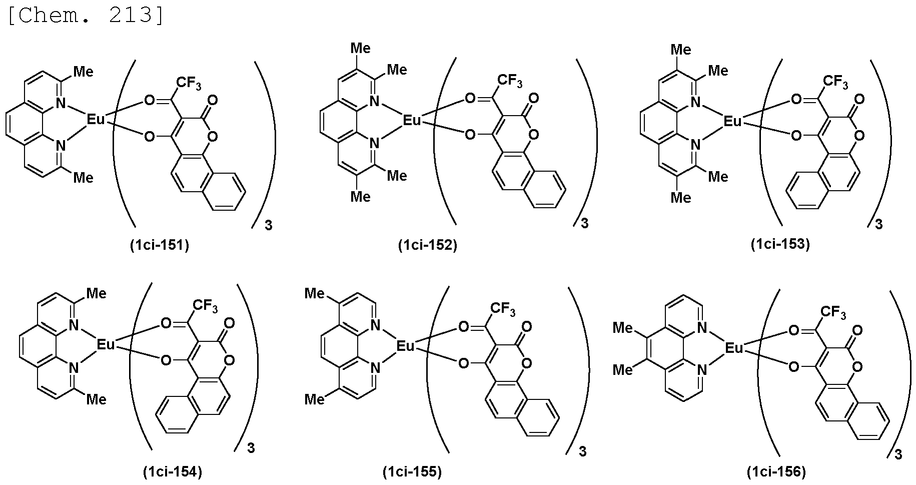

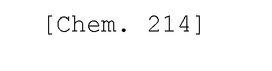

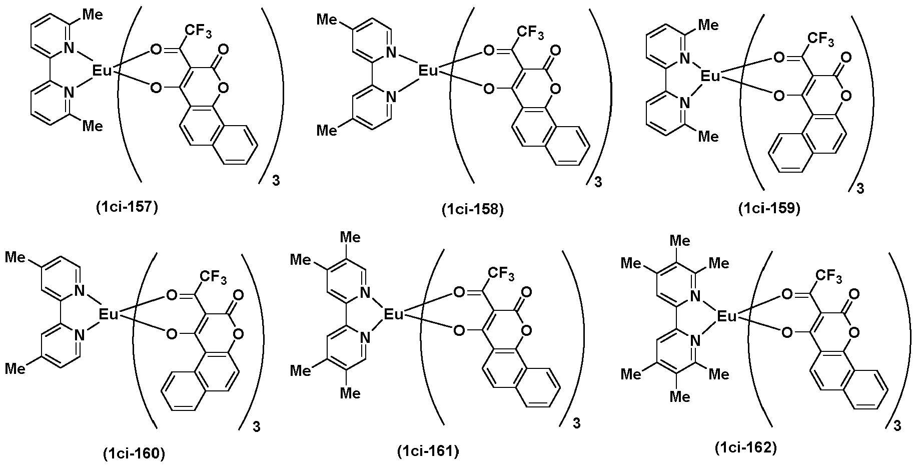

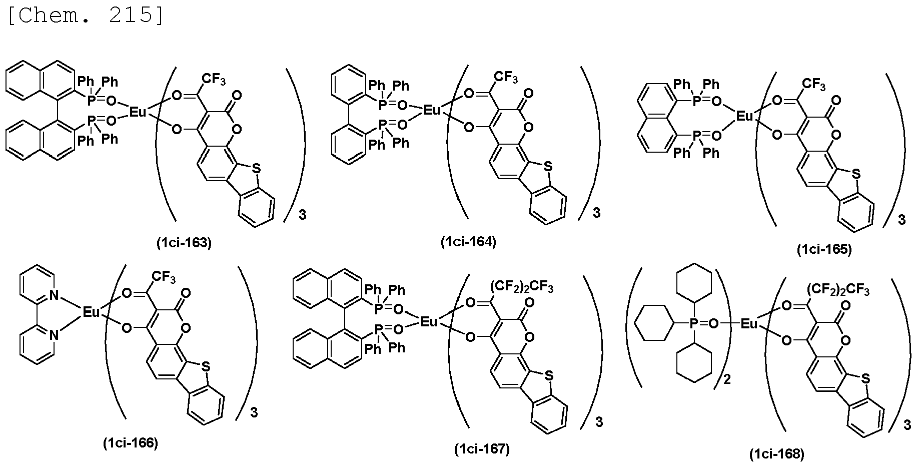

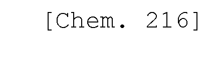

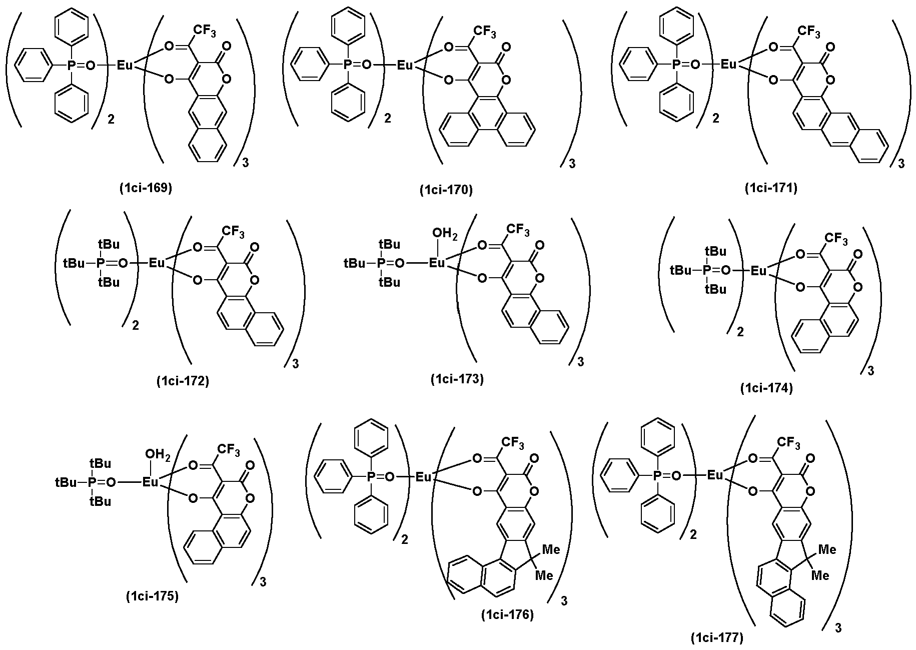

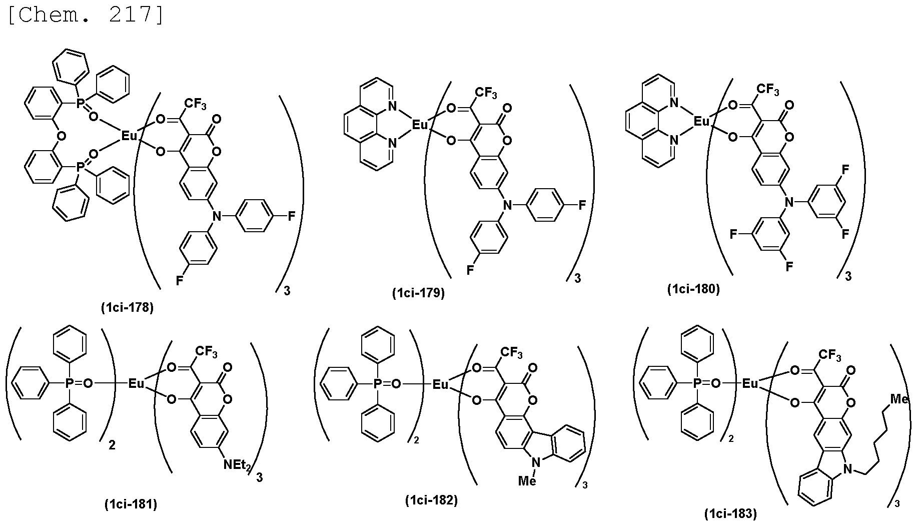

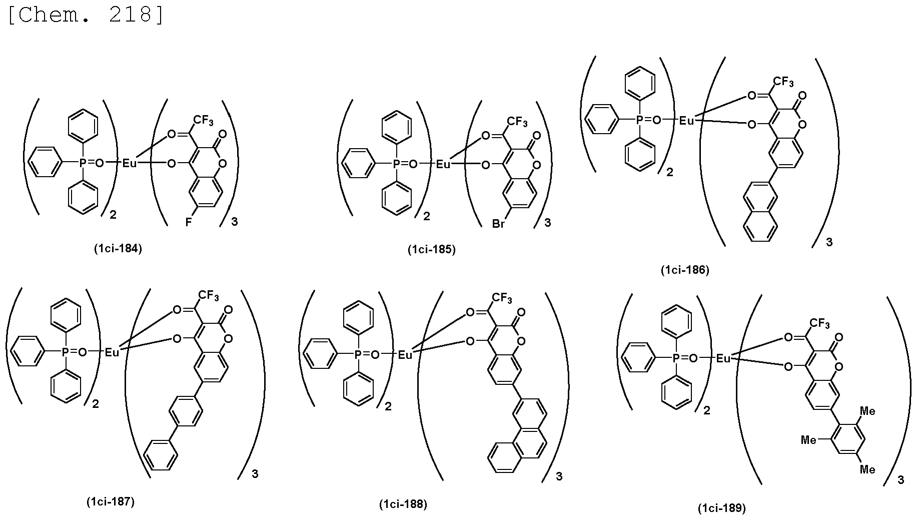

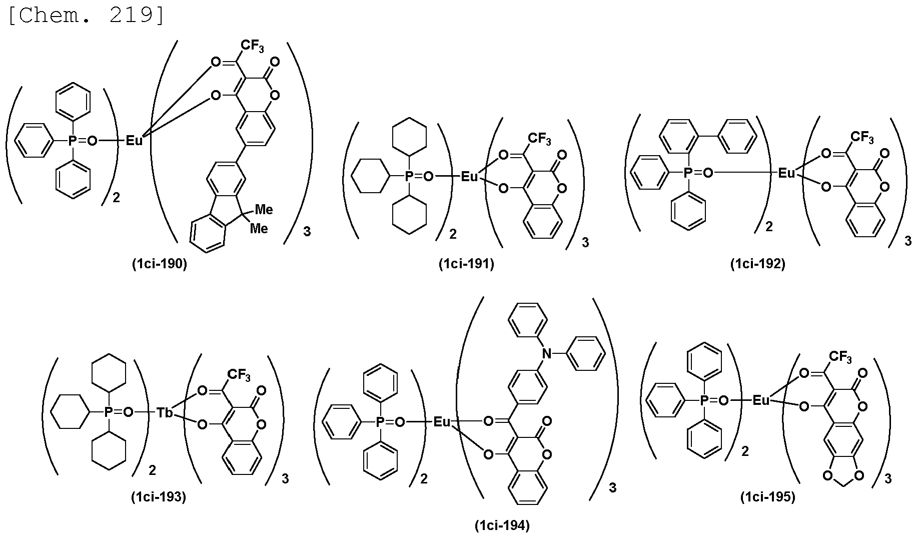

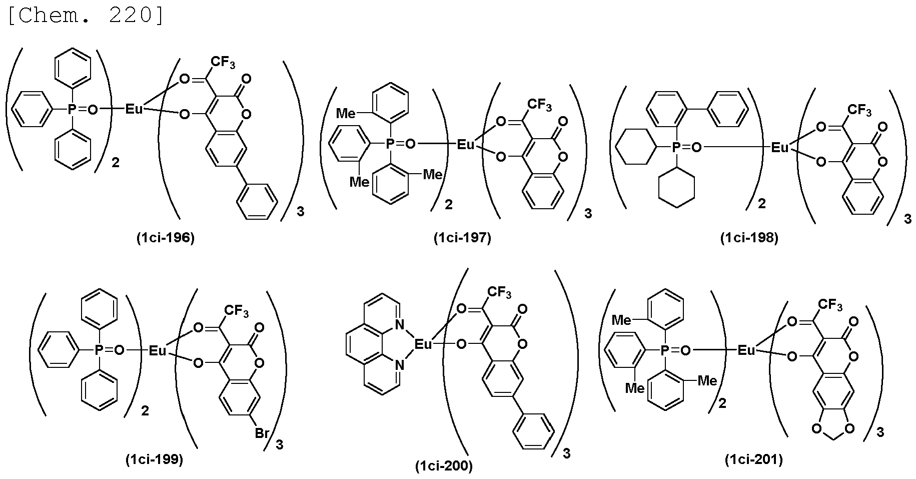

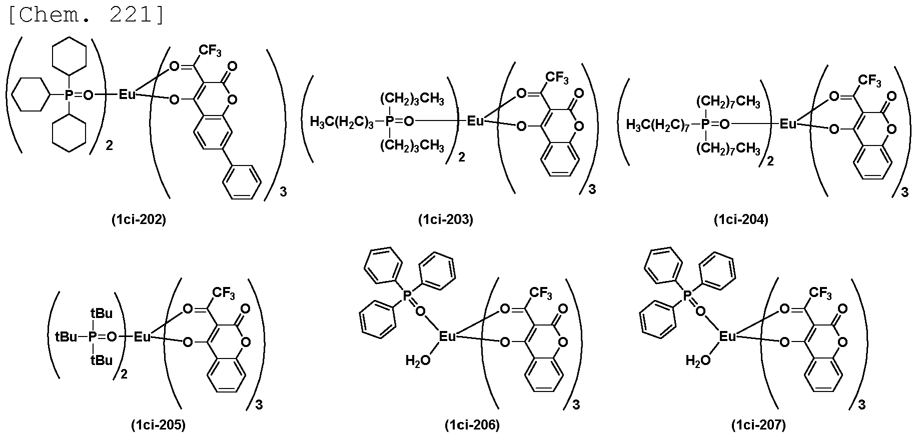

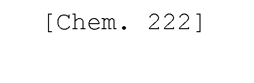

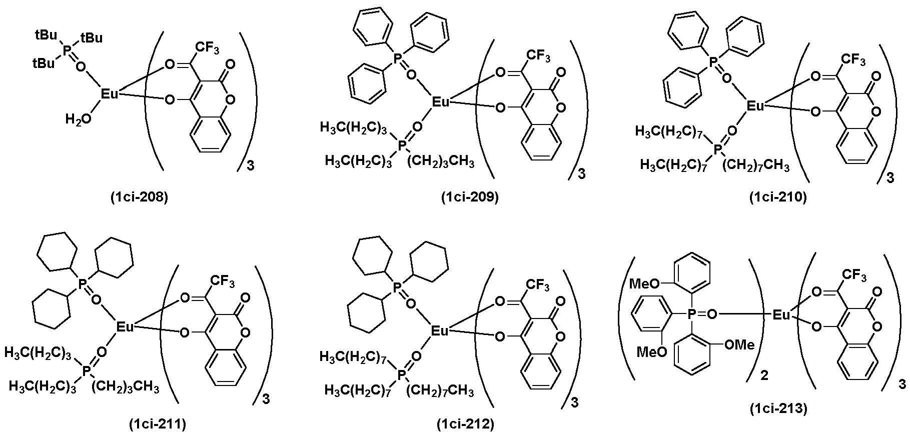

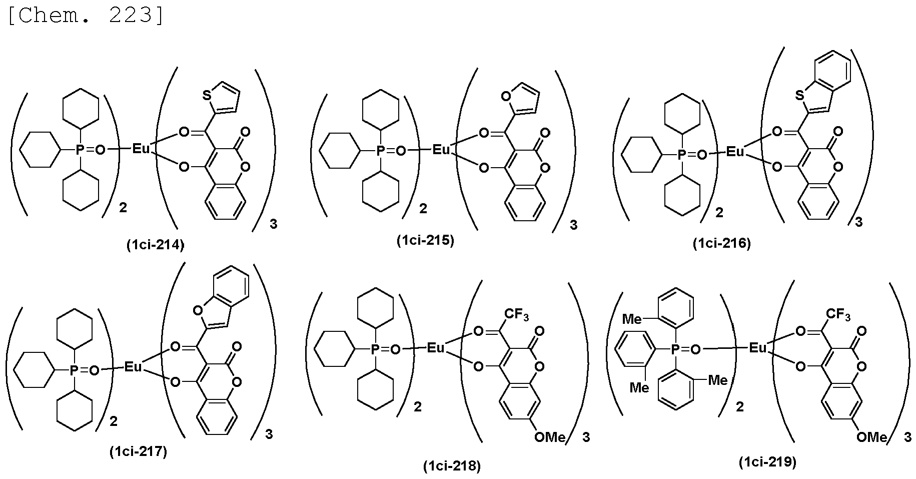

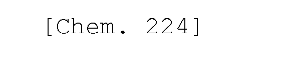

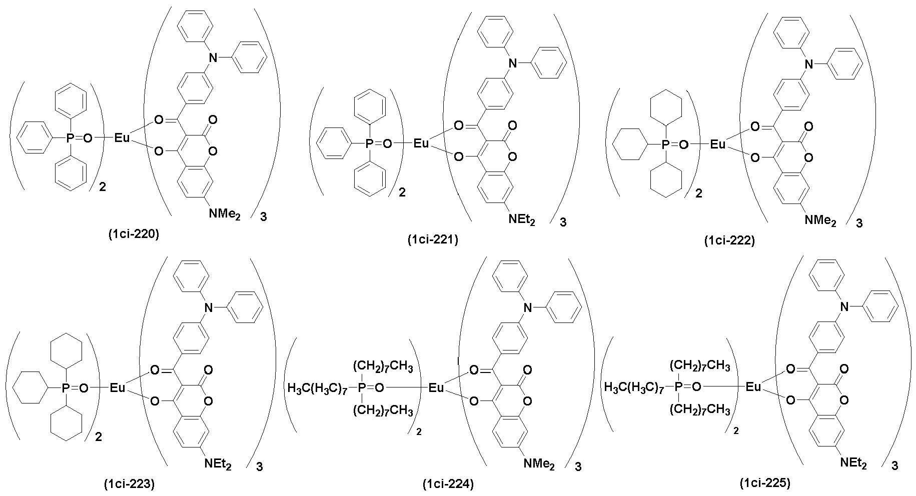

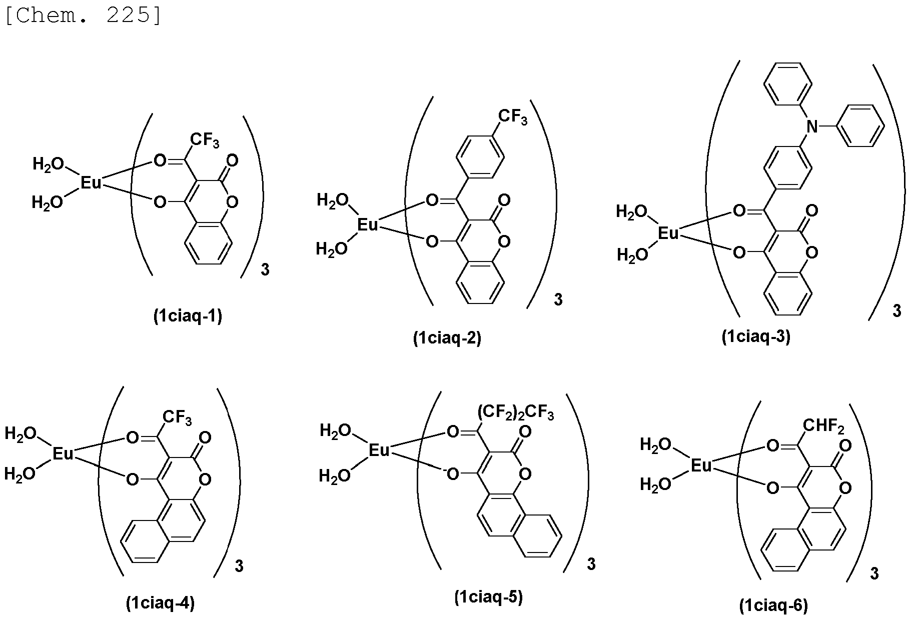

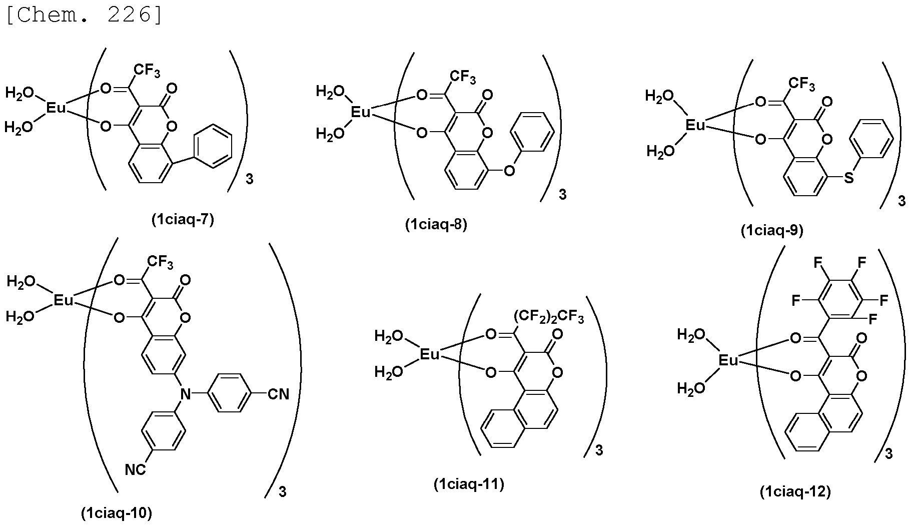

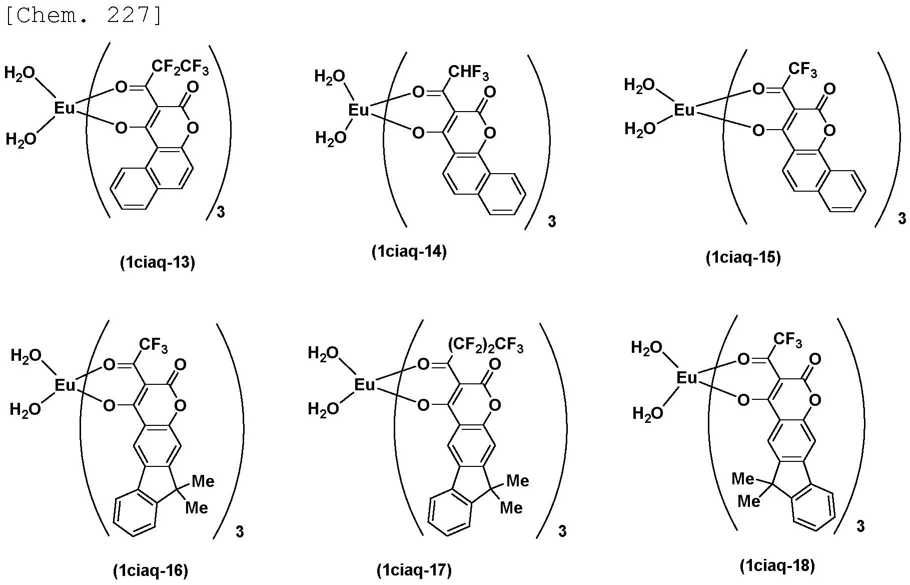

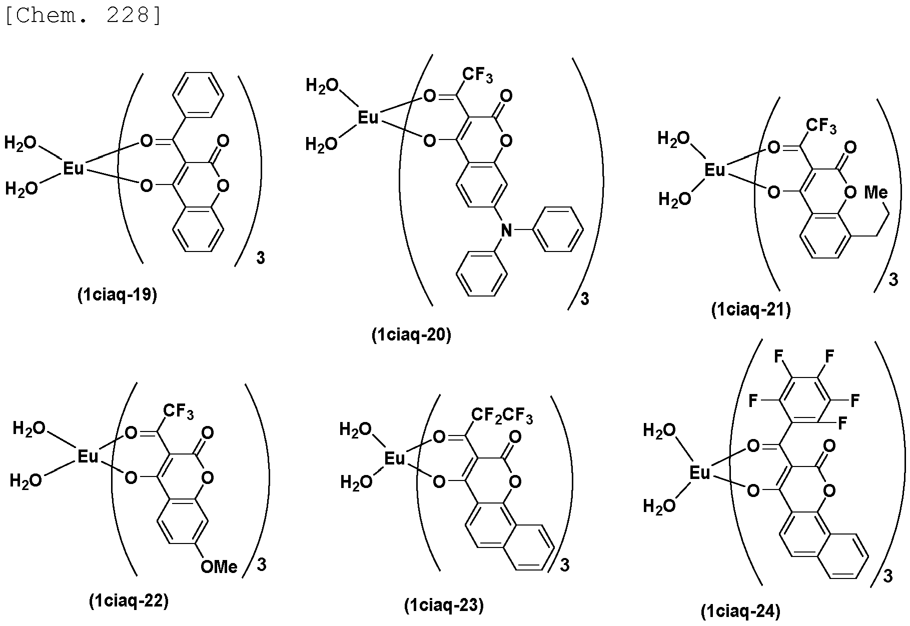

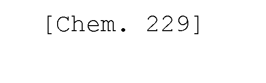

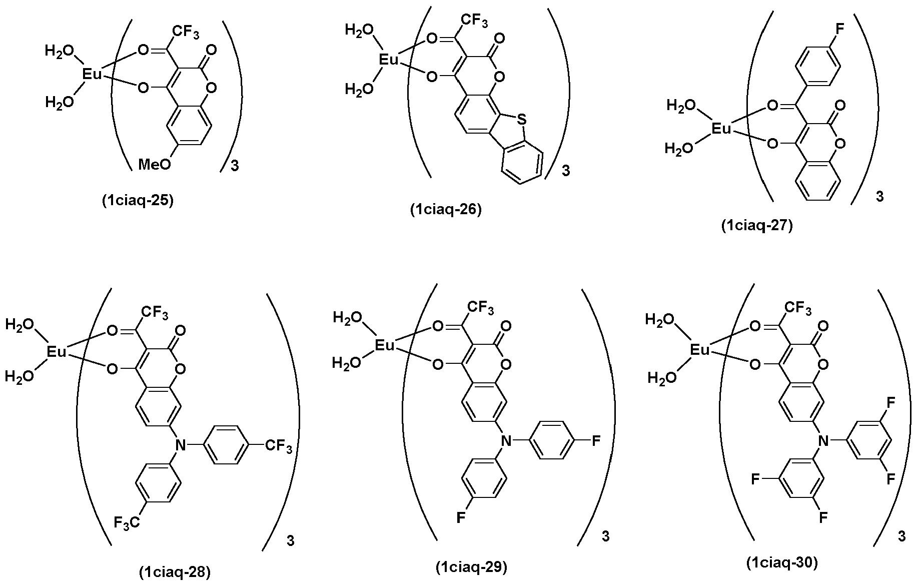

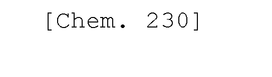

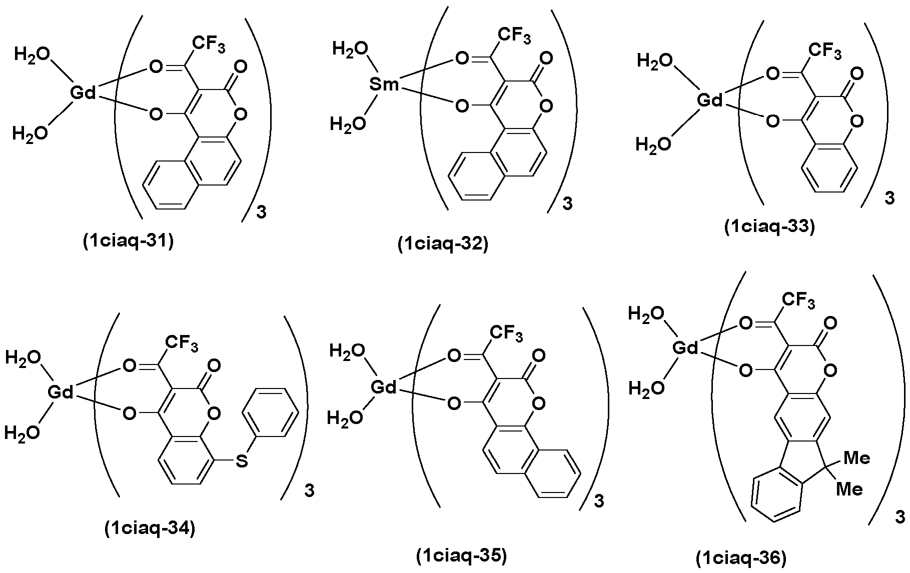

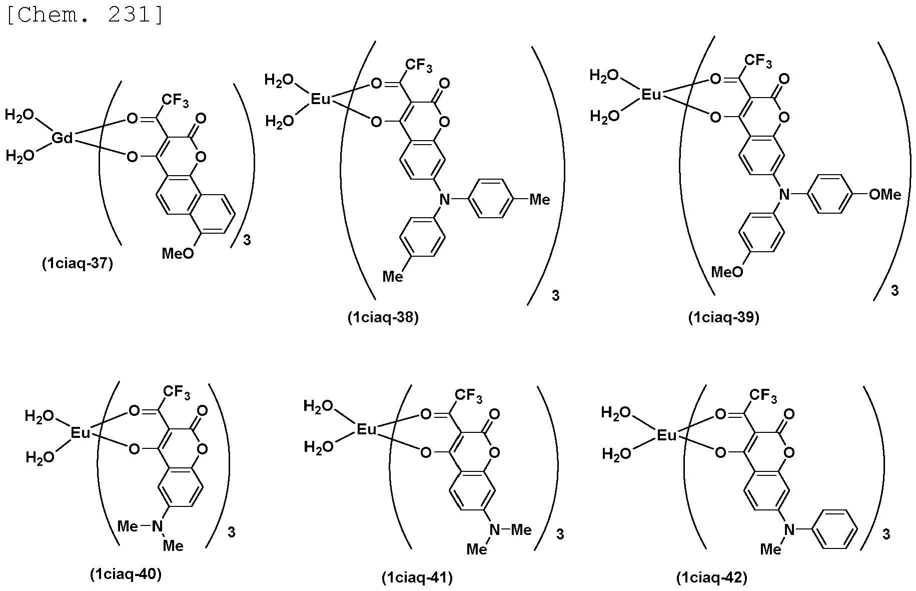

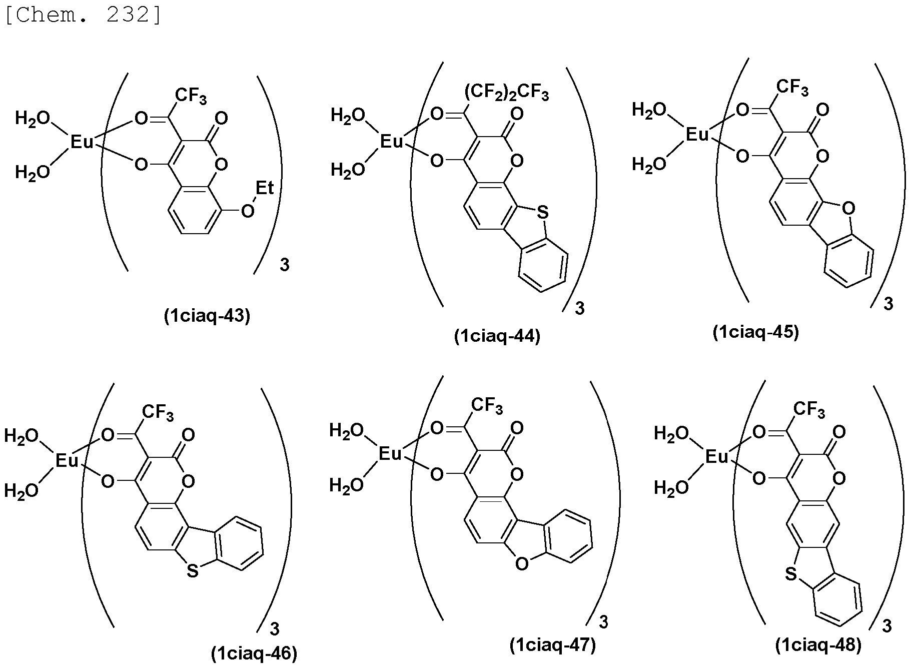

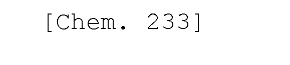

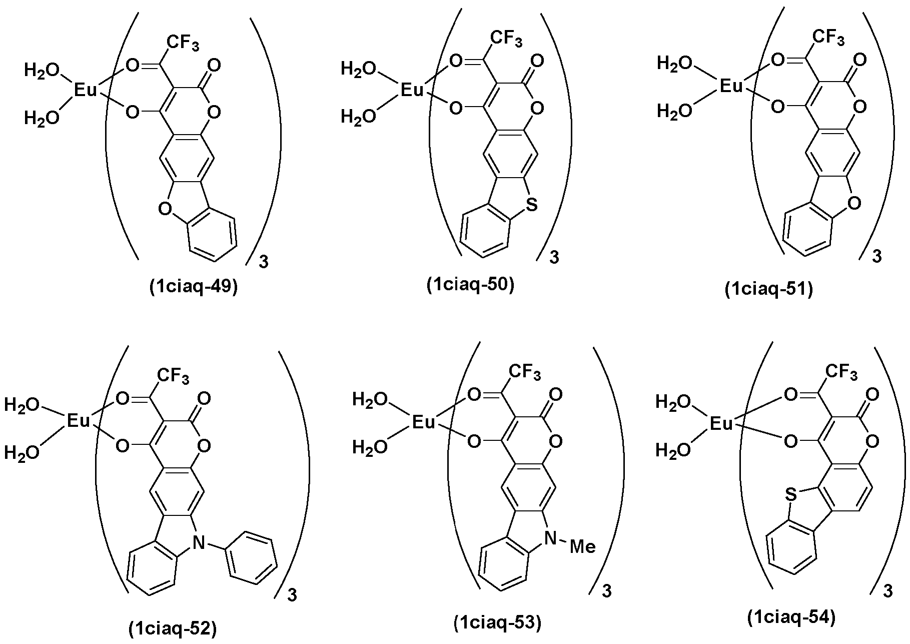

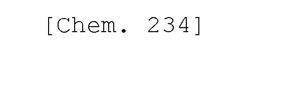

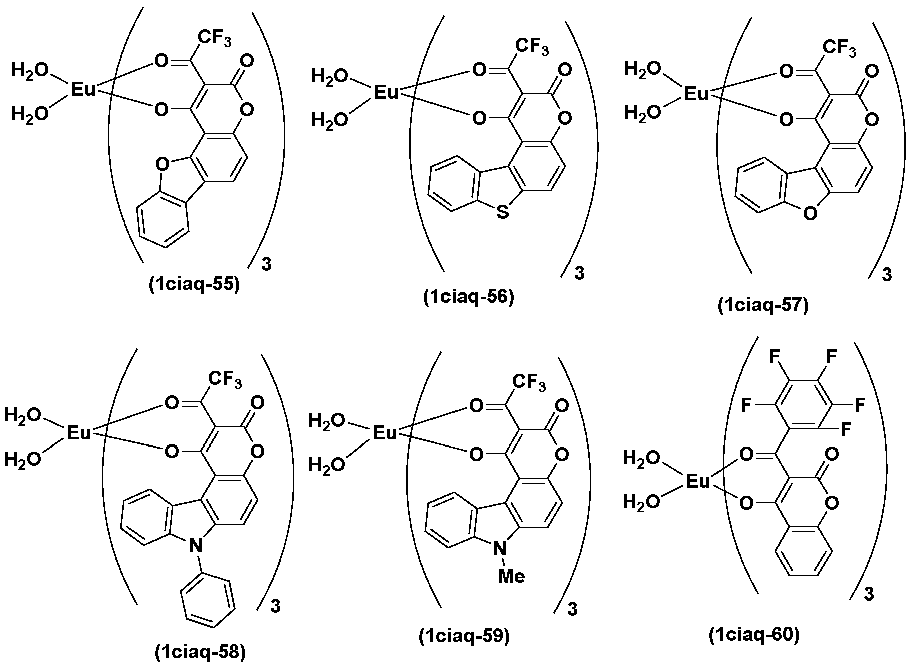

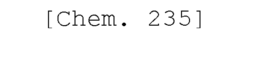

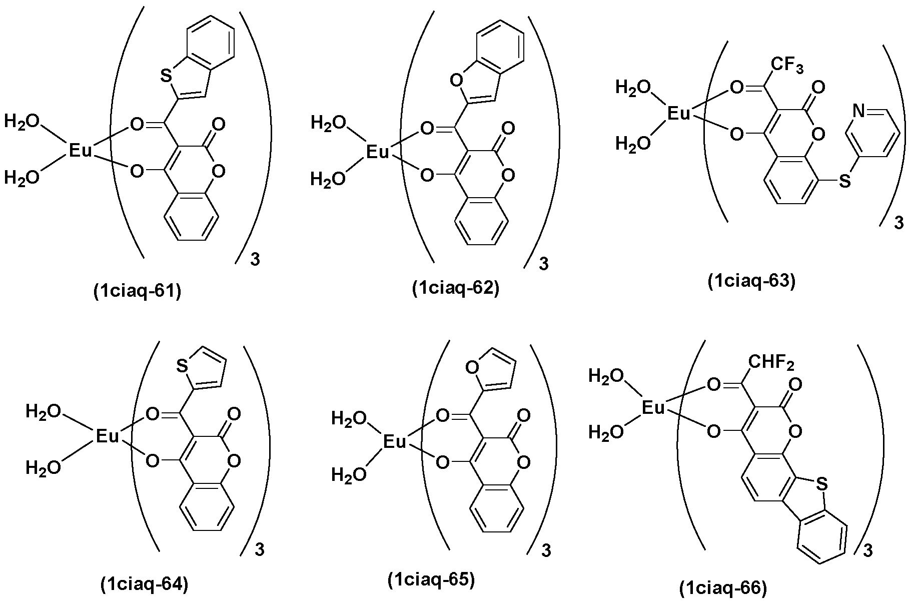

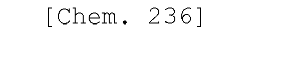

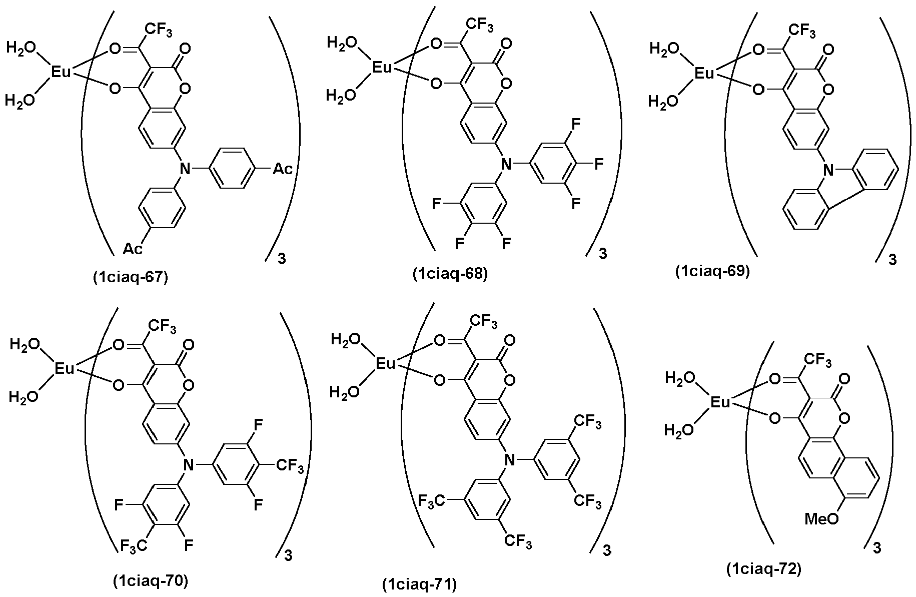

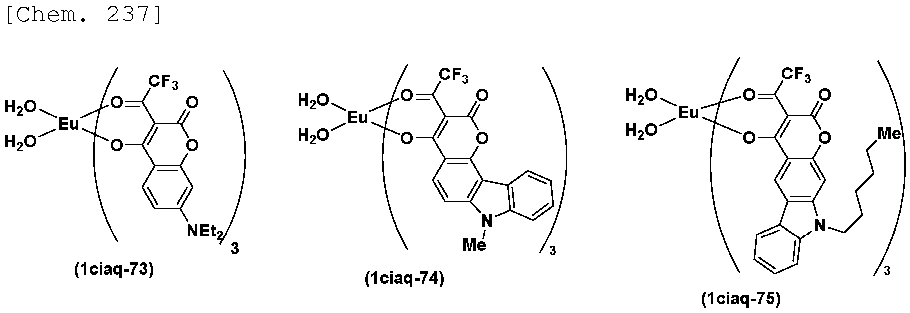

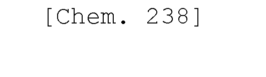

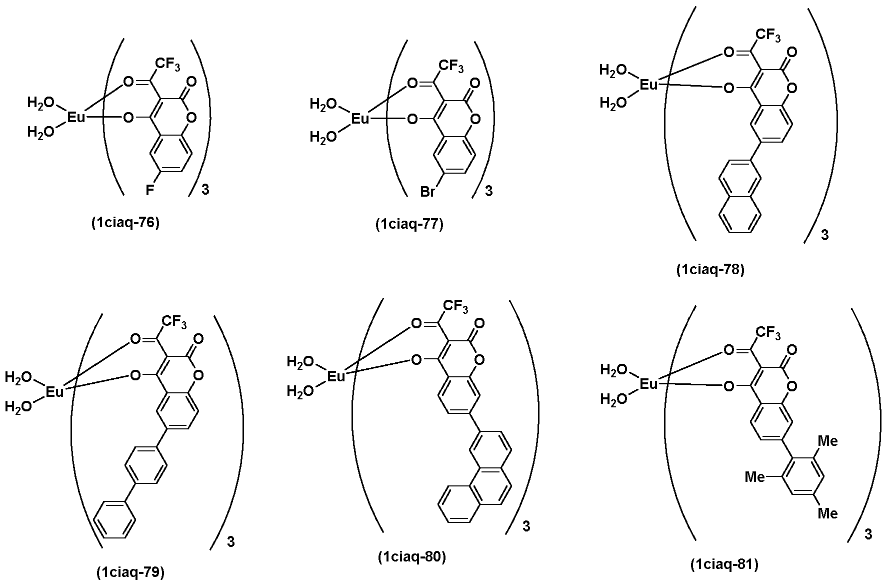

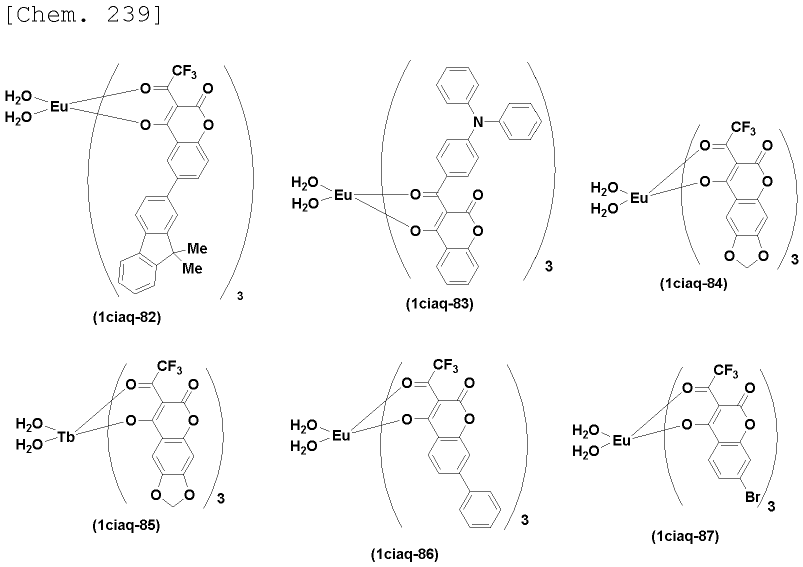

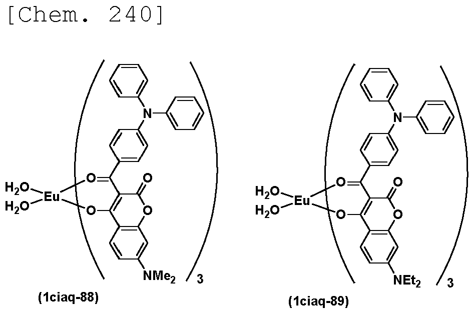

Of formulae (1ci-1) to (1ci-225) and formulae (1ciaq-1) to (1ciaq-89) above, the compounds represented by formulae (1ci-1) to (1ci-8), (1ci-10) to (1ci-12), (1ci-16) to (1ci-23), (1ci-25) to (1ci-33), (1ci-35) to (1ci-41), (1ci-47) to (1ci-48), (1ci-50) to (1ci-68), (1ci-70) to (1ci-89), (1ci-91) to (1ci-94), (1ci-96) to (1ci-108), (1ci-111) to (1ci-147), (1ci-149) to (1ci-168), (1ci-172) to (1ci-175), (1ci-178) to (1ci-185), (1ci-190) to (1ci-225), (1ciaq-1) to (1ciaq-11), (1ciaq-13) to (1ciaq-15), (1ciaq-19) to (1ciaq-20), (1ciaq-22) to (1ciaq-23), (1ciaq-25) to (1ciaq-35), (1ciaq-37), (1ciaq-40) to (1ciaq-59), (1ciaq-61) to (1ciaq-69), (1ciaq-71) to (1ciaq-77) or (1ciaq-82) to (1ciaq-89) are preferred because of easy availability of raw materials, the compounds represented by formulae (1ci-1) to (1ci-3), (1ci-7) to (1ci-8), (1ci-10) to (1ci-12), (1ci-16) to (1ci-23), (1ci-25) to (1ci-33), (1ci-38) to (1ci-41), (1ci-52) to (1ci-68), (1ci-76) to (1ci-85), (1ci-91) to (1ci-92), (1ci-94), (1ci-97), (1ci-99) to (1ci-100), (1ci-106), (1ci-112), (1ci-115) to (1ci-122), (1ci-124) to (1ci-147), (1ci-149) to (1ci-168), (1ci-172) to (1ci-175), (1ci-178) to (1ci-185), (1ci-190) to (1ci-218), (1ciaq-1) to (1ciaq-6), (1ciaq-10) to (1ciaq-11), (1ciaq-13) to (1ciaq-15), (1ciaq-19) to (1ciaq-20), (1ciaq-22) to (1ciaq-23), (1ciaq-25) to (1ciaq-33), (1ciaq-35), (1ciaq-37), (1ciaq-41), (1ciaq-44) to (1ciaq-49), (1ciaq-51) to (1ciaq-62), (1ciaq-64) to (1ciaq-66), (1ciaq-72) to (1ciaq-77) or (1ciaq-82) to (1ciaq-87) are more preferred because of the ease of synthesis, and formulae (1ci-7) to (1ci-8), (1ci-11), (1ci-23), (1ci-31) to (1ci-33), (1ci-103), (1ci-178), (1ci-183) or (1ci-194) is particularly preferred because it gives the complex optical characteristics suitable for use as an optical functional material.

### (Methods for Manufacturing the Rare Earth Complex (1))

Methods for manufacturing the rare earth complex (1) (Hereinafter referred to as manufacturing methods according to the present invention.) will now be described.

### (Manufacturing Method 1)

An example of a method for manufacturing the rare earth complex (1) is manufacturing method 1 (Hereinafter also referred to as method 1.), which comprises allowing an enol represented by general formula (4b) below (Hereinafter referred to as an enol (4b).) or enol represented by general formula (4c) below (Hereinafter referred to as an enol (4c).), a phosphorus ligand represented by general formula (3a) above; a phosphorus ligand represented by general formula (3b) above; or a nitrogen-containing compound having two or more nitrogen atoms possessing a lone pair; which is represented by L¹, or/and a neutral ligand, which is represented by L², selected from the group consisting of; water; deuterium oxide; sulfoxide compounds; sulfone compounds; amide compounds; nitrile compounds; ester compounds; carbonyl compounds; ether compounds and alcohols and a rare earth compound to react together.

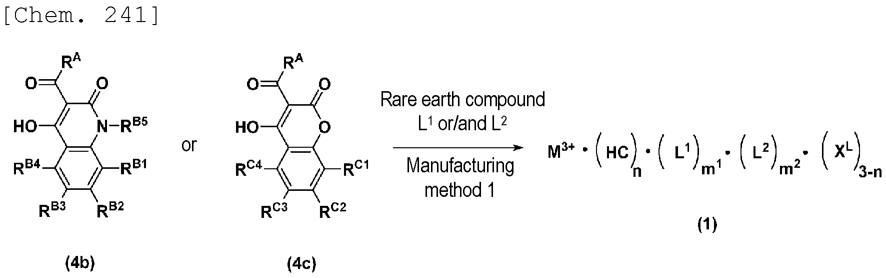

{HC is a quinolinonato ligand represented by general formula (1qu) or coumarinato ligand represented by general formula (1cu).

(In the formulae, R^{A}, R^{B1}, R^{B2}, R^{B3}, R^{B4}, R^{B5}, R^{C1}, R^{C2}, R^{C3}, R^{C4}, M³⁺, HC, L¹, L², X^{L}, n, m¹ and m² are synonymous with R^{A}, R^{B1}, R^{B2}, R^{B3}, R^{B4}, R^{B5}, R^{C1}, R^{C2}, R^{C3}, R^{C4}, M³⁺, HC, L¹, L², X^{L}, n, m¹ and m², respectively, in general formulae (1), (1qu) and (1cu) .)

In method 1, the definitions and specific examples presented for R^{A}, R^{B1}, R^{B2}, R^{B3}, R^{B4}, R^{B5}, R^{C1}, R^{C2}, R^{C3}, R^{C4}, M³⁺, HC, L¹, L², X^{L}, n, m¹ and m² are the same as for R^{A}, R^{B1}, R^{B2}, R^{B3}, R^{B4}, R^{B5}, R^{C1}, R^{C2}, R^{C3}, R^{C4}, M³⁺, HC, L¹, L², X^{L}, n, m¹ and m², respectively, in general formulae (1), (1qu) and (1cu) above.

When L¹ is phosphorus ligands represented by general formulae (3a) and (3b) (Hereinafter referred to as a phosphorus ligand (3a) and a phosphorus ligand (3b).), the phosphorus ligands (3a) and (3b) can be obtained by, for example, methods described in Chemical Reviews, vol. 60, pages 243 to 260, 1960. Commercially available phosphorus ligands may also be used.

A specific example of a phosphorus ligand (3a) used in method 1 is any of formulae (3a-1) to (3a-16) below. The present invention is not limited to these. Herein, Cy represents a cyclohexyl group.

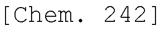

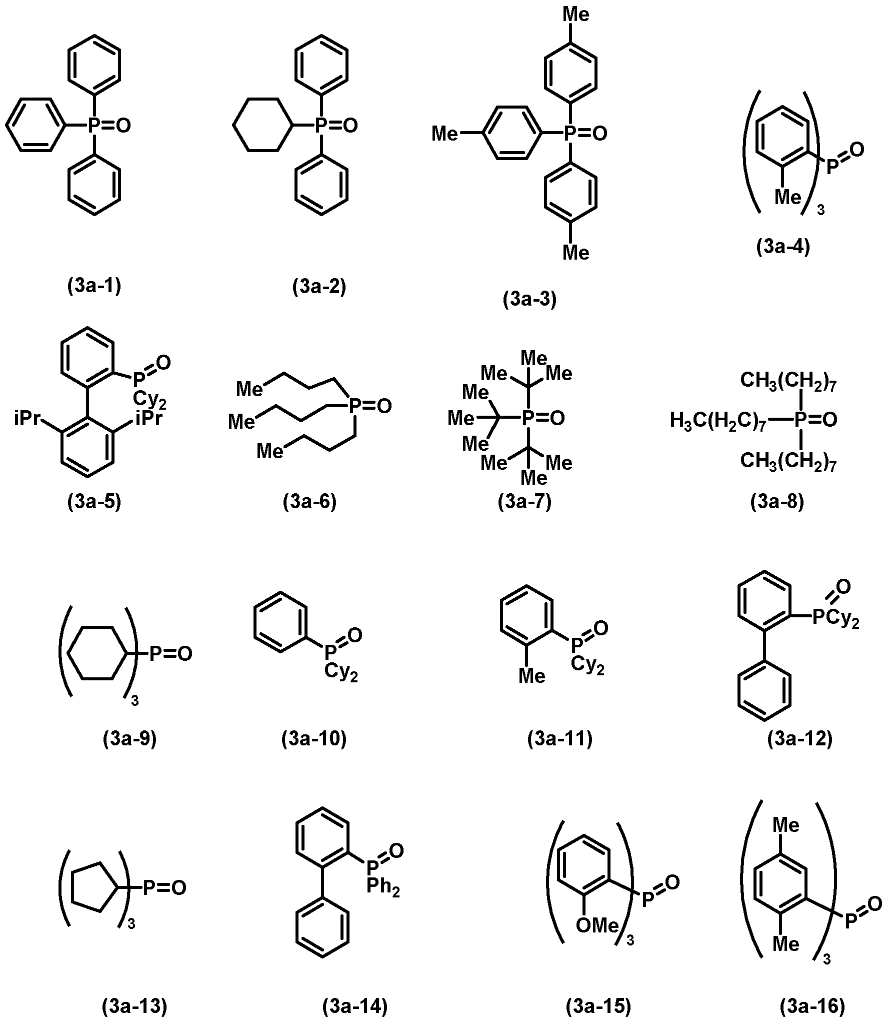

A specific example of a phosphorus ligand (3b) used in method 1 is any of formulae (3b-1) to (3b-13) below. The present invention is not limited to these.

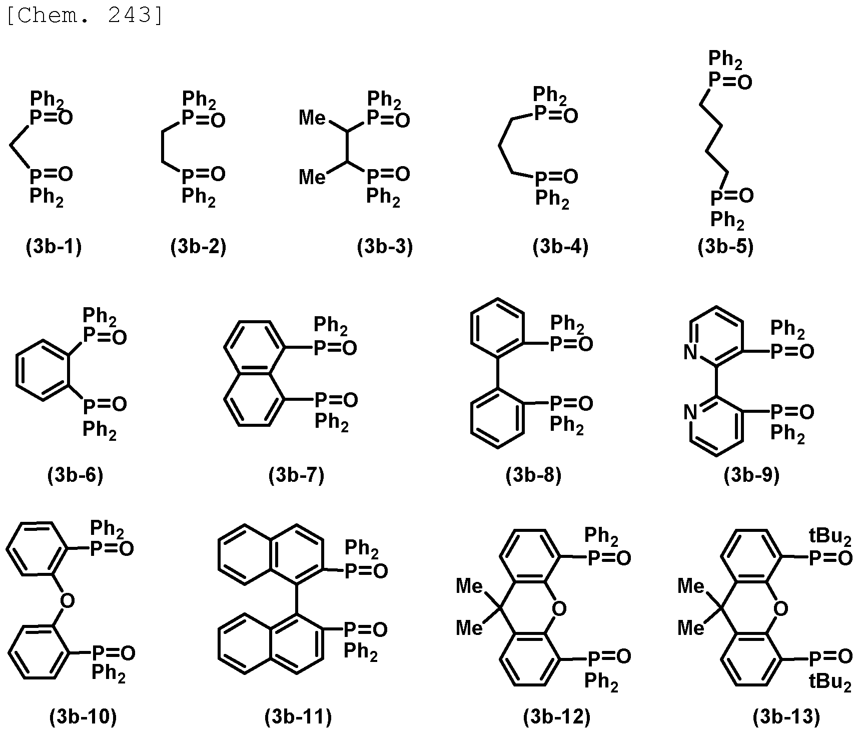

Specific examples of nitrogen-containing ligands, represented by L¹, having two or more nitrogen atoms possessing a lone pair or neutral ligands, represented by L², used in method 1 include compounds such as 1,10-phenanthroline, 4,7-dimethyl-1,10-phenanthroline, 3,4,7,8-tetramethyl-1,10-phenanthroline, 2,9-dimethyl-4,7-diphenyl-1,10-phenanthroline, 4,7-diphenyl-1,10-phenanthroline, 2,9-diphenyl-1,10-phenanthroline, 2,2'-bipyridine, 5,5'-dimethyl-2,2'-bipyridine, 4,4'-dimethyl-2,2'-bipyridin-d₈, 6,6'-dimethyl-2,2'-bipyridine, 4,4'-diphenyl-2,2'-bipyridine, 5,5'-diphenyl-2,2'-bipyridine, 4,4',5,5'-tetramethyl-2,2'-bipyridine, water, deuterium oxide, acetone, deuterated acetone, methyl ethyl ketone, ethyl acetate, methanol, deuterated methanol, ethanol, propanol, isopropyl alcohol, acetonitrile, propionitrile, deuterated acetonitrile, diethyl ether, tetrahydrofuran, tetrahydrofuran-d₈, 1,4-dioxane, dimethylsulfoxide, deuterated dimethylsulfoxide, dimethyl sulfone, diphenyl sulfone and sulfolane. The present invention is not limited to these.

A nitrogen-containing ligand, represented by L¹, having two or more nitrogen atoms possessing a lone pair or neutral ligand, represented by L², used in method 1 may be a commercially available one.

The enol (4b) or (4c) used in method 1 can be obtained by methods described in publications such as The Journal of Organic Chemistry, vol. 75, pages 2741 to 2744, 2010; Journal of the American Chemical Society, vol. 66, pages 1220 to 1222, 1944; Tetrahedron, vol. 74, pages 2762 to 2768, 2018; and The Journal of Organic Chemistry, vol. 80, pages 10643 to 10650, 2015. A commercially available enol may also be used.

An enol (4b) or (4c) used in method 1 loses its active proton and turns into an organic salt indicated in formulae (4ba) to (4bc) when a base is allowed to act on it. This organic salt (4ba) to (4bc) may be used as the enol (4b). In that case, specific examples of M'⁺, indicated as the countercation in the organic salt (4ba) to (4bc), include ions such as alkali metal ions, e.g., a lithium ion, a sodium ion, a potassium ion and a cesium ion, tertiary ammonium ions, e.g., triethylammonium, trimethylammonium and diisopropylethylammonium, secondary ammonium ions, e.g., diethylammonium and diisopropylammonium, pyridinium ions, e.g., pyridinium and 2,6-dimethylpyridinium, imidazolium ions, e.g., imidazolium and N-methylimidazolium, and ammonium ions. The present invention is not limited to these.

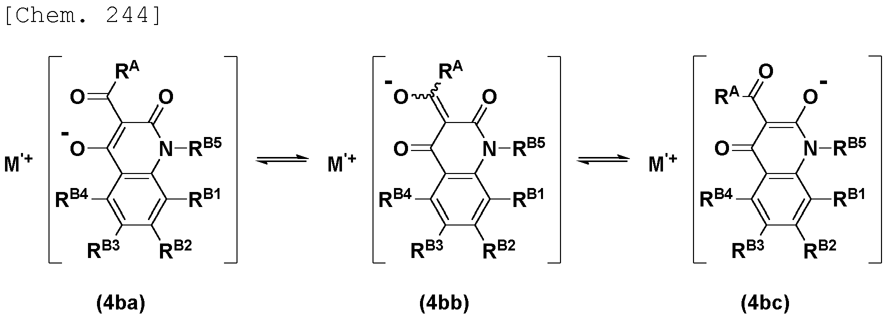

(In the formulae, R^{A}, R^{B1}, R^{B2}, R^{B3}, R^{B4} and R^{B5} are synonymous with R^{A}, R^{B1}, R^{B2}, R^{B3}, R^{B4} and R^{B5}, respectively, in general formula (qu). M'⁺ represents a countercation.)

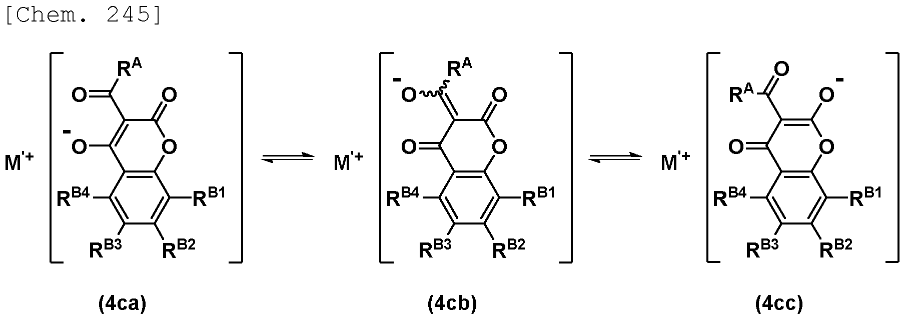

(In the formulae, R^{A}, R^{C1}, R^{C2}, R^{C3} and R^{C4} are synonymous with R^{A}, R^{C1}, R^{C2}, R^{C3} and R^{C4}, respectively, in general formula (cu). M'⁺ represents a countercation.)

Examples of rare earth compounds used in method 1, for europium compounds for example, include; halide salts, such as europium(III) fluoride, europium(III) chloride, europium(III) bromide and europium(III) iodide, or their hydrates; organic acid salts, such as europium(III) oxalate, europium(III) acetate, europium(III) trifluoroacetate and europium(III) trifluoromethanesulfonate, or their hydrates; metal alkoxides, such as tris[N,N-bis(trimethylsilyl)amide]europium(III), europium(III) trimethoxide, europium(III) triethoxide and europium(III) tri(isopropoxide); or inorganic acid salts, such as europium(III) phosphate, europium(III) sulfate and europium(III) nitrate, or their hydrates. Of these, europium(III) chloride, inorganic acid salts, such as europium(III) nitrate, or their hydrates; or; organic acid salts, such as europium(III) oxalate, europium(III) acetate, europium(III) trifluoroacetate; and europium(III) trifluoromethanesulfonate; are particularly preferred because of a good reaction yield, and europium(III) acetate; europium(III) chloride; or; europium(III) nitrate or their hydrates; are more preferred.

The rare earth compound used in method 1 may be a commercially available one.

In method 1, L¹ or/and L² may be contained in the rare earth compound used. Specific examples include inorganic salt hydrates, such as europium(III) acetate n-hydrate, europium(III) nitrate pentahydrate and europium(III) chloride hexahydrate, and complex compounds, such as bis(triphenylphosphine oxide)europium(III) chloride, bis(tricyclohexylphosphine oxide)europium(III) chloride, bis[tri(o-tolyl)phosphine oxide]europium(III) chloride, tris(triphenylphosphine oxide)europium(III) chloride, bis(triphenylphosphine oxide)europium(III) nitrate, bis(triphenylphosphine oxide)europium(III) acetate, (phenanthroline)europium(III) chloride, bis(phenanthroline)europium(III) chloride and (bipyridine)europium(III) chloride. The present invention is not limited to these compounds listed by way of example. Of these, inorganic salt hydrates, such as europium(III) acetate n-hydrate, europium(III) nitrate pentahydrate and europium(III) chloride hexahydrate, are preferred because of easy availability of raw materials. The complex compounds can be obtained by, for example, methods described in RSC Advances, vol. 6, pages 90934 to 90943, 2016. Commercially available complex compounds may also be used.

In method 1, it is preferred to perform the reaction in a solvent because of a good yield of the rare earth complex (1). The types of solvents that can be used are not particularly limited unless the reaction is inhibited. Examples of solvents that can be used include; a halogenated hydrocarbon, such as dichloromethane, chloroform or chlorobenzene; an alcohol, such as methanol, ethanol, propanol or isopropyl alcohol; an ester, such as methyl acetate, ethyl acetate, butyl acetate or isoamyl acetate; a glycol ether, such as ethylene glycol monoethyl ether, ethylene glycol monomethyl ether or ethylene glycol monobutyl ether; an ether, such as diethyl ether, tert-butyl methyl ether, glyme, diglyme, triglyme, tetrahydrofuran or cyclopentyl methyl ether; a ketone, such as tert-butyl methyl ketone, isobutyl methyl ketone, ethyl butyl ketone, dipropyl ketone, diisobutyl ketone, cyclohexanone or acetone; a hydrocarbon, such as hexane, cyclohexane, methylcyclohexane, ethylcyclohexane, heptane, octane, benzene, toluene or xylene; or; water. One of these solvents or a mixture of two or more in any proportions can also be used. The solvent is preferably dichloromethane, chloroform, methylcyclohexane, acetone, methanol, ethanol or water because this leads to a good reaction yield of the rare earth complex (1).

The molar ratio between the rare earth compound and the enols (4b) and (4c) in method 1 will be described. It is preferred to use 1.0 to 5.0 moles of the enols (4b) and (4c) per mole of the rare earth compound. More preferably, it is preferred to use 3.0 to 4.0 moles of the enols (4b) and (4c) .

For method 1 in which L¹ is a phosphorus ligand (3a), the molar ratio between the rare earth compound and the phosphorus ligand (3a) will be described. It is preferred to use 0.5 to 5.0 moles of the phosphorus ligand (3a) per mole of the rare earth compound. More preferably, it is preferred to use 1.0 to 3.0 moles of the phosphorus ligand (3a) .

For method 1 in which L¹ is a phosphorus ligand (3b), the molar ratio between the rare earth compound and the phosphorus ligand (3b) will be described. It is preferred to use 0.5 to 2.5 moles of the phosphorus ligand (3b) per mole of the rare earth compound. More preferably, it is preferred to use 1.0 to 1.5 moles of the phosphorus ligand (3b) .

For method 1 in which L¹ is a nitrogen-containing ligand having two or more nitrogen atoms possessing a lone pair, the molar ratio between the rare earth compound and the nitrogen-containing ligand will be described. It is preferred to use 0.5 to 2.5 moles of the nitrogen-containing ligand per mole of the rare earth compound. More preferably, it is preferred to use 1.0 to 1.5 moles of the nitrogen-containing ligand.

The molar ratio between the rare earth compound and/or the neutral ligand in method 1 will be described. It is preferred to use 0.5 to 10 moles of the neutral ligand per mole of the rare earth compound. More preferably, it is preferred to use 1.0 to 8.0 moles of the neutral ligand.

When m¹ in method 1 is 2 and when the two L¹s are different, the two L¹s may be added simultaneously or may be added separately in the manufacture of the rare earth complex (1).

In method 1, the reaction may be performed with an added base for accelerated reaction. Examples of the base include; an organic amine, such as trimethylamine, triethylamine, diethylamine, pyridine or quinoline; or; a carbonate, such as sodium carbonate or potassium carbonate, a hydrogencarbonate, such as sodium hydrogencarbonate or potassium hydrogencarbonate, a hydroxide salt, such as sodium hydroxide, potassium hydroxide or lithium hydroxide or an inorganic base, such as ammonia. As for the number of equivalents of the base, it is desirable to use 1.0 to 10 moles of base per mole of the enol (4b) or (4c). More desirably, 2.0 to 8.0 moles of base is used. Even more desirably, 3.0 to 5.0 moles of base is used.

In method 1, the reaction temperature and the duration of reaction are not particularly limited; conditions commonly used in the manufacture of a metal complex by one skilled in the art can be used. To give a specific example, the rare earth complex (1) can be manufactured with a good yield by selecting a duration of reaction of 1 minute to 120 hours as appropriate at a reaction temperature of -80°C to 120°C.

The rare earth complex (1) manufactured by method 1 can be purified by selecting, as appropriate, a purification method commonly used in the purification of a metal complex by one skilled in the art and using it. Specific purification methods include methods such as filtration, extraction, centrifugation, decantation, distillation, sublimation, crystallization and column chromatography.

### (Manufacturing Method 2)

Another example of a method for manufacturing the rare earth complex (1) according to the present invention is method 2 for manufacturing a rare earth complex (1) (Hereinafter also referred to as method 2.), which comprises allowing a diketonato complex represented by general formula (1aq) below and a phosphorus ligand represented by general formula (3a) above; a phosphorus ligand represented by general formula (3b) above; or a nitrogen-containing ligand having two or more nitrogen atoms possessing a lone pair; which is represented by L¹, or a neutral ligand, which is represented by L², selected from the group consisting of; water; deuterium oxide; sulfoxide compounds; sulfone compounds; amide compounds; nitrile compounds; ester compounds; carbonyl compounds; ether compounds and alcohols to react together.

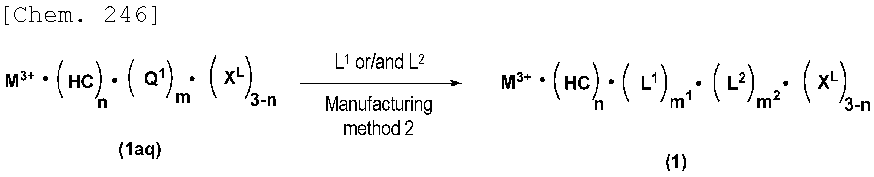

(In the formulae, M³⁺, HC, X^{L}, n, m¹ and m² are synonymous with M³⁺, HC, X^{L}, n, m¹ and m², respectively, in general formula (1).

In the formulae, Q¹ represents a neutral molecule. When m is not 0 and when m¹ is 0, however, it is impossible that Q¹ and L² are the same, and m and m² are equal at the same time.)

In method 2, the definitions and specific examples presented for M³⁺, HC, X^{L}, n, m¹ and m² are the same as for M³⁺, HC, X^{L}, n, m¹ and m², respectively, in general formula (1) above.

In method 2, specific examples of neutral molecules, represented by Q¹, include; water; deuterium oxide; pyridine; imidazole; ketones, such as acetone and methyl ethyl ketone; alcohols, such as methanol, ethanol, propanol and isopropyl alcohol; nitriles, such as acetonitrile and propionitrile; amines, such as ammonia, diethylamine and triethylamine; ethers, such as dimethyl ether, diethyl ether and tetrahydrofuran; and sulfur compounds, such as dimethylsulfoxide and dimethyl sulfone. The present invention is not limited to these.

The diketonato complex (1aq) used in method 2 can be obtained according to, for example, the method presented in reference example 13.

Specific examples of diketonato complexes (1aq) used in method 2 include complexes such as the structure represented by any of formulae (1baq-1) to (1baq-81) or (1caq-1) to (1caq-89) above. The present invention is not limited to these.

When L¹ in method 2 is a phosphorus ligand (3a) or (3b), it can be obtained by the methods mentioned by way of example in the description of method 1 above. A commercially available phosphorus ligand may also be used.

When L¹ in method 2 is a phosphorus ligand (3a), examples of phosphorus ligands (3a) used are the same as those listed by way of example in the description of method 1 above.

When L¹ in method 2 is a phosphorus ligand (3b), examples of phosphorus ligands (3b) used are the same as those listed by way of example in the description of method 1 above.

When L¹ in method 2 is a nitrogen-containing ligand having two or more nitrogen atoms possessing a lone pair, examples of the nitrogen-containing ligand used are the same as those listed by way of example in the description of method 1 above.

When L² in method 2 is a neutral ligand, examples of neutral ligands used are the same as those listed by way of example in the description of method 1 above.

In method 2, it is preferred to perform the reaction in a solvent because of a good yield of the rare earth complex (1). The types of solvents that can be used are not particularly limited unless the reaction is inhibited. Examples of solvents that can be used include; a halogenated hydrocarbon, such as dichloromethane, chloroform or chlorobenzene; an alcohol, such as methanol, ethanol, propanol or isopropyl alcohol; an ester, such as ethyl acetate, butyl acetate or isoamyl acetate; a glycol ether, such as ethylene glycol monoethyl ether, ethylene glycol monomethyl ether or ethylene glycol monobutyl ether; an ether, such as diethyl ether, tert-butyl methyl ether, glyme, diglyme, triglyme or tetrahydrofuran; a ketone, such as tert-butyl methyl ketone, isobutyl methyl ketone, ethyl butyl ketone, dipropyl ketone, diisobutyl ketone, cyclohexanone or acetone; a hydrocarbon, such as hexane, cyclohexane, methylcyclohexane, ethylcyclohexane, heptane, octane, benzene, toluene or xylene; or; water. One of these solvents alone or a mixture of two or more in any proportions can be used. The solvent is preferably dichloromethane, chloroform, acetone, methylcyclohexane, methanol, ethanol or water because this leads to a good yield of the rare earth complex (1).

For method 2 in which L¹ is a phosphorus ligand (3a), the molar ratio between the diketonato complex (1aq) and the phosphorus ligand (3a) will be described. It is preferred to use 0.5 to 5.0 moles of the phosphorus ligand (3a) per mole of the diketonato complex (1aq). More preferably, it is preferred to use 1.0 to 3.0 moles of the phosphorus ligand (3a).

For method 2 in which L¹ is a phosphorus ligand (3b), the molar ratio between the diketonato complex (1aq) and the phosphorus ligand (3b) will be described. It is preferred to use 0.5 to 2.5 moles of the phosphorus ligand (3b) per mole of the diketonato complex (1aq). More preferably, it is preferred to use 1.0 to 1.5 moles of the phosphorus ligand (3b).

For method 2 in which L¹ is a nitrogen-containing ligand having two or more nitrogen atoms possessing a lone pair, the molar ratio between the diketonato complex (1aq) and the nitrogen-containing ligand will be described. It is preferred to use 0.5 to 2.5 moles of the nitrogen-containing ligand per mole of the diketonato complex (1aq). More preferably, it is preferred to use 1.0 to 1.5 moles of the nitrogen-containing ligand.

When m¹ in method 2 is 2 and when the two L¹s are different, the two L¹s may be added simultaneously or may be added separately in the manufacture of the rare earth complex (1).

The molar ratio between the diketonato complex (1aq) and the neutral ligand in method 2 will be described. It is preferred to use 0.5 to 10 moles of the neutral ligand per mole of the diketonato complex (1aq). More preferably, it is preferred to use 1.0 to 8.0 moles of the neutral ligand.

In method 2, furthermore, the reaction temperature and the duration of reaction are not particularly limited; conditions commonly used in the manufacture of a metal complex by one skilled in the art can be used. To give a specific example, the rare earth complex (1) can be manufactured with a good reaction yield by selecting a duration of reaction of 1 minute to 120 hours at a reaction temperature of -80°C to 120°C.

The rare earth complex (1) manufactured by method 2 can be purified by selecting, as appropriate, a purification method commonly used in the purification of a metal complex by one skilled in the art and using it. Specific purification methods include methods such as filtration, extraction, centrifugation, decantation, distillation, sublimation, crystallization and column chromatography.

An enol (4b) can isomerize to form an enol (4bi) or β-diketone (4bii). The present invention includes all of the enol (4b), enol (4bi) and β-diketone (4bii), but these isomers are herein expressed as an enol (4b) for the sake of convenience.

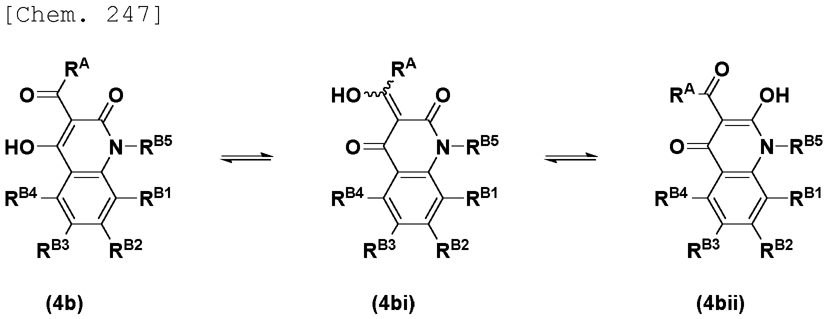

(In the formulae, R^{A}, R^{B1}, R^{B2}, R^{B3}, R^{B4} and R^{B5} are synonymous with R^{A}, R^{B1}, R^{B2}, R^{B3}, R^{B4} and R^{B5}, respectively, in general formula (1qu) above.)

A diketonato complex (1baq) has the coordination structures of diketonato complexes (1baqi) to (1baqvi) below varying in the tautomeric structure of the quinolinonato ligand (1qu). A diketonato complex (1baq) includes all of the diketonato complexes (1bi) to (1bvi), but these isomers are herein expressed as a diketonato complex (1baq) or (1baqi) for the sake of convenience.

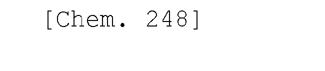

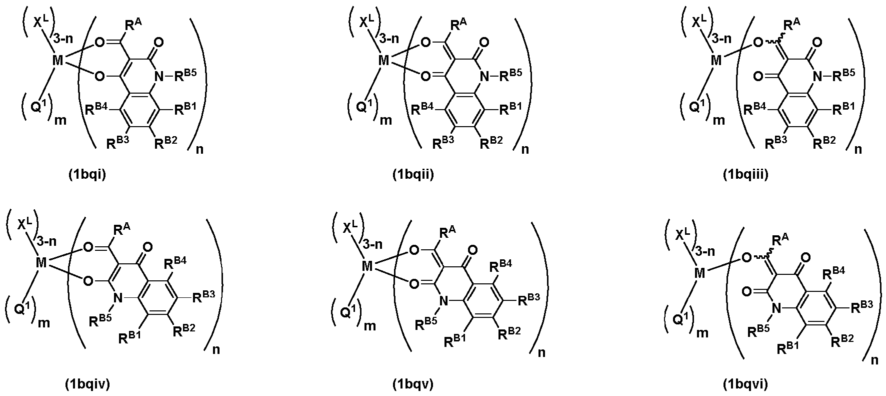

(In the formulae, R^{A}, R^{B1}, R^{B2}, R^{B3}, R^{B4}, R^{B5}, X^{L}, m, n, Q¹ and M are synonymous with R^{A}, R^{B1}, R^{B2}, R^{B3}, R^{B4}, R^{B5}, X^{L}, m, n, Q¹ and M, respectively, in general formula (1baq) or (1bi) above.)

An enol (4c) can isomerize to form an enol (4ci) or β-diketone (4cii). The present invention includes all of the enol (4c), enol (4ci) and β-diketone (4cii), but these isomers are herein expressed as an enol (4c) for the sake of convenience.

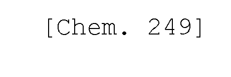

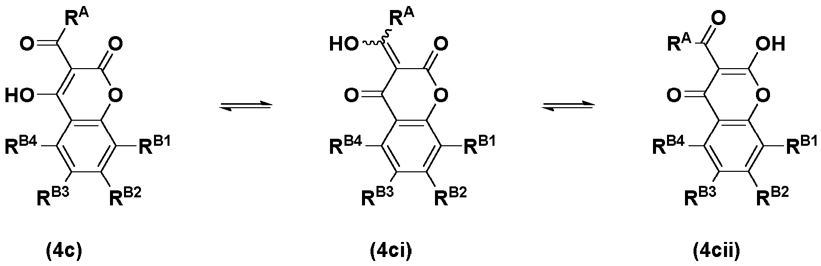

(In the formulae, R^{A}, R^{C1}, R^{C2}, R^{C3} and R^{C4} are synonymous with R^{A}, R^{C1}, R^{C2}, R^{C3} and R^{C4}, respectively, in general formula (1cu) above.)

A diketonato complex (1caq) has the coordination structures of diketonato complexes (1caqi) to (1caqvi) below varying in the tautomeric structure of the coumarinato ligand (1cu). A diketonato complex (1caq) includes all of the diketonato complexes (1cai) to (1cvi), but these isomers are herein expressed as a diketonato complex (1caq) or (1caqi) for the sake of convenience.

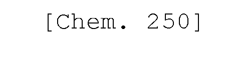

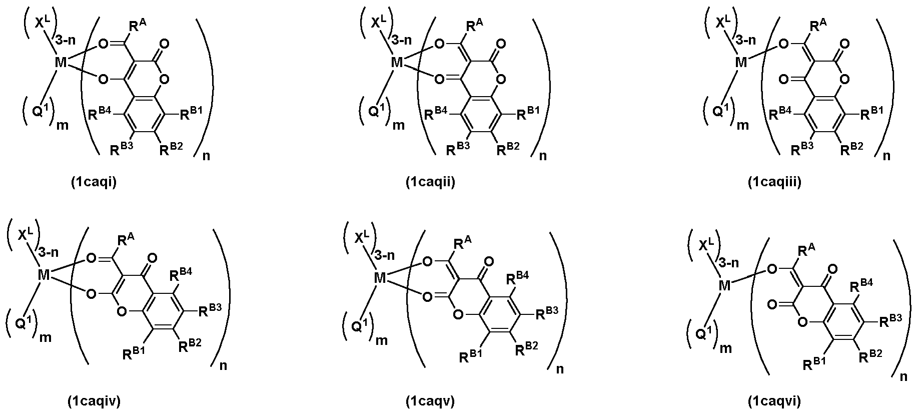

(In the formulae, R^{A}, R^{C1}, R^{C2}, R^{C3} and R^{C4}, X^{L}, m, n, Q¹ and M are synonymous with R^{A}, R^{C1}, R^{C2}, R^{C3} and R^{C4}, X^{L}, m, n, Q¹ and M, respectively, in general formula (1caq) or (1cai) above.)

The rare earth complex (1) according to the present invention is excitable with blue light and has high light resistance. The rare earth complex (1), therefore, is useful as an optical material containing it, and particularly useful forms of the optical material include a film for solar cells, an agricultural film, an LED phosphor and a light-emitting material, for example for security inks, and a wavelength conversion material used in them.

The rare earth complex (1) according to the present invention, furthermore, can be used as an optical material containing one or more selected from a resin material, inorganic glass, an organic low-molecular-weight material and a solvent. By virtue of its high dispersibility, it is particularly preferred to use the rare earth complex (1) as an optical material containing a resin material. Examples of the resin material include resins such as polymethacrylates, e.g., polymethyl methacrylate, polyethyl methacrylate, polypropyl methacrylate, polyisopropyl methacrylate, polybutyl methacrylate, poly-sec-butyl methacrylate, polyisobutyl methacrylate, poly-tert-butyl methacrylate, fluorinated polymethyl methacrylate, fluorinated polyethyl methacrylate, fluorinated polypropyl methacrylate, fluorinated polyisopropyl methacrylate, fluorinated polybutyl methacrylate, fluorinated poly-sec-butyl methacrylate, fluorinated polyisobutyl methacrylate and fluorinated poly-tert-butyl methacrylate, polyacrylates, e.g., polymethyl acrylate, polyethyl acrylate, polypropyl acrylate, polyisopropyl acrylate, polybutyl acrylate, poly-sec-butyl acrylate, polyisobutyl acrylate, poly-tert-butyl acrylate, fluorinated polymethyl acrylate, fluorinated polyethyl acrylate, fluorinated polypropyl acrylate, fluorinated polyisopropyl acrylate, fluorinated polybutyl acrylate, fluorinated poly-sec-butyl acrylate, fluorinated polyisobutyl acrylate and fluorinated poly-tert-butyl acrylate, polyolefins, e.g., polystyrene, polyethylene, polypropylene, polybutene, fluorinated polyethylene, fluorinated polypropylene and fluorinated polybutene, polyvinyl ether, fluorinated polyvinyl ether, polyvinyl acetate and polyvinyl chloride or their copolymers; cellulose; polyacetals; polyesters; polycarbonates; epoxy resins; polyamide resins; polyimide resins; polyurethanes; Nafion; petroleum resins; rosin; and silicone resins.

Of these, resins such as polymethyl methacrylate, polyethyl methacrylate, polypropyl methacrylate, polyisopropyl methacrylate, polybutyl methacrylate, poly-sec-butyl methacrylate, polyisobutyl methacrylate, poly-tert-butyl methacrylate, polymethyl acrylate, polyethyl acrylate, polypropyl acrylate, polyisopropyl acrylate, polybutyl acrylate, poly-sec-butyl acrylate, polyisobutyl acrylate, poly-tert-butyl acrylate, polyethylene, polystyrene and polyvinyl acetate or their copolymers; epoxy resins; polyimide resins; and silicone resins are particularly preferred. Polymethyl methacrylate, polyethyl methacrylate, polypropyl methacrylate, polybutyl methacrylate, polymethyl acrylate, polyethyl acrylate, polypropyl acrylate, polybutyl acrylate, polyethylene, polystyrene and polyvinyl acetate or their copolymers; epoxy resins; polyimide resins; silicone resins; are particularly preferred. One of these may be used alone, and a combination of two or more is also acceptable.

In an optical material containing the rare earth complex (1) according to the present invention and a resin material, the percentage of the rare earth complex (1) is preferably from 0.001% to 99% by weight, more preferably from 0.01% to 50% by weight.

The inorganic glass can be of a type commonly used by those skilled in the art. Examples include variations such as soda-lime glass, lead glass and borosilicate glass.

The organic low-molecular-weight material can be of a type commonly used by those skilled in the art. Examples include materials such as an ionic liquid, e.g., amyltriethylammonium bis(trifluoromethanesulfonyl)imide or tetraamylammonium chloride; or a hydrocarbon, e.g., pentadecane, hexadecane, octadecane, nonadecane, icosane or paraffin.

Examples of methods for obtaining an optical material containing the rare earth complex (1) according to the present invention include methods such as one in which the rare earth complex (1) is used alone to serve as an optical material, one in which the rare earth complex (1) is incorporated into an optical material containing one or more selected from a resin material, inorganic glass and organic low-molecular-weight material to give an optical material, one in which in the polymerization of the resin material, the corresponding monomer(s) and the rare earth complex (1) are mixed together, and the monomer(s) is polymerized to give an optical material and one in which the rare earth complex (1) is dissolved or dispersed in a solvent to give an optical material.

When the rare earth complex (1) according to the present invention is dissolved or dispersed in a solvent to give an optical material, examples of solvents that can be used include; a halogenated hydrocarbon, such as dichloromethane, chloroform or chlorobenzene; an alcohol, such as methanol, ethanol, propanol or isopropyl alcohol; an ester, such as ethyl acetate, butyl acetate or isoamyl acetate; a glycol ether, such as ethylene glycol monoethyl ether, ethylene glycol monomethyl ether or ethylene glycol monobutyl ether; an ether, such as diethyl ether, tert-butyl methyl ether, glyme, diglyme, triglyme or tetrahydrofuran; a ketone, such as tert-butyl methyl ketone, isobutyl methyl ketone, ethyl butyl ketone, dipropyl ketone, diisobutyl ketone, cyclohexanone or acetone; a hydrocarbon, such as hexane, cyclohexane, methylcyclohexane, ethylcyclohexane, heptane, octane, benzene, toluene or xylene; or water. One of these solvents alone or a mixture of two or more in any proportions can be used.

Of these, a halogenated hydrocarbon, alcohol, ester, glycol ether, ether, ketone or hydrocarbon is particularly preferred.

### EXAMPLES

The present invention will now be described in further detail with examples, a comparative example and an evaluation example, but the present invention is not to be construed as being limited to these.

In the identification of the rare earth complexes (1), the following analytical methods were used.

The measurement of the ¹H-NMR, ¹⁹F-NMR and ³¹P-NMR spectra was performed using ULTRASHIELD PLUS AVANCE III (400 MHz, 376 MHz and 162 MHz) and ASCEND AVANCE III HD (400 MHz, 376 MHz and 162 MHz), manufactured by BRUKER CORPORATION. In ¹H-NMR, the measurement was performed using deuterated chloroform (CDCl₃) or deuterated acetone (Acetone-d₆) as a measurement solvent and tetramethylsilane (TMS) as an internal standard. In ¹⁹F-NMR, the measurement was performed using deuterated chloroform (CDCl₃), deuterated acetone (Acetone-d₆), deuterated acetonitrile (CD₃CN) or deuterated dimethylsulfoxide (DMSO-d₆) as a measurement solvent. In ³¹P-NMR, the measurement was performed using deuterated chloroform (CDCl₃) or deuterated acetone (Acetone-d₆).

The mass spectroscopic measurement was performed using waters 2695-micromass ZQ 4000, manufactured by waters corporation.

In the measurement of the excitation and emission spectra, the measurement was performed using a spectrophotometer (FP-6500, manufactured by JASCO Corporation).

In the measurement of the UV-Vis spectrum, the measurement was performed using an ultraviolet-visible/near-infrared spectrophotometer (V-670, manufactured by JASCO Corporation).

In the measurement of the emission quantum yield, the measurement was performed using absolute PL quantum yield spectrometers (C9920-03 and C11347-01, manufactured by Hamamatsu Photonics K.K.).

The reagents, furthermore, were commercially available ones.

### (REFERENCE EXAMPLE 1)

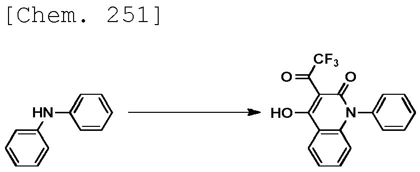

N-methylimidazole (159 µL, 2.00 mmol) and N,N-dimethylformamide (40 mL) were added to a mixture of cyanoacetic acid (3.40 g, 40.0 mmol) and diphenylamine (6.76 g, 40.0 mmol). Trifluoroacetic anhydride (22.6 mL, 160 mmol) was added dropwise over 1 hour in a water bath at room temperature, and the resulting mixture was further stirred for 23 hours at the same temperature. The reaction solution was emptied into water, and the precipitated solid was collected by suction filtration, washed with acetonitrile and then vacuum-dried to give a yellow solid of 4-hydroxy-1-phenyl-3-(2,2,2-trifluoroethan-1-on-1-yl)quinolin-2(1H)-one (actual yield, 10.2 g; percent yield, 760). ¹H-NMR (400 MHz, CDCl₃) δ (ppm): 14.7 (s, 1H), 8.26 (dd, J = 8.2, 1.4 Hz, 1H), 7.63-7.57 (m, 2H), 7.53 (ddd, J = 8.7, 7.3, 1.3 Hz, 2H), 7.30-7.24 (m, 3H), 6.62 (d, J = 8.7 Hz, 1H). ¹⁹F-NMR (376 MHz, CDCl₃) δ (ppm) : -74.0.

### (REFERENCE EXAMPLE 2)

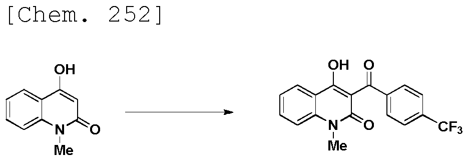

4-hydroxy-1-methylquinolin-2(1H)-one (1.76 g, 10.0 mmol) was added to dichloromethane (35.0 mL). After the addition of N,N-diisopropylethylamine (2.10 mL, 12.0 mmol) and 4-(trifluoromethyl)benzoyl chloride (1.63 mL, 11.0 mmol), the resulting mixture was stirred for 4 hours at room temperature. The reaction solution was diluted with dichloromethane (50 mL), and then the diluted solution was washed three times with 1 M hydrochloric acid (50 mL), an aqueous solution of sodium hydrogencarbonate (50 mL) and a saturated saline solution (50 mL). The resulting organic layer was dried with magnesium sulfate and then concentrated under reduced pressure to give a white solid. The resulting crude product was dissolved in acetonitrile (35.0 mL). After the addition of acetone cyanohydrin (1.01 mL, 11.0 mmol) and triethylamine (1.55 mL, 11.0 mmol), the resulting mixture was stirred for 17 hours at 50°C. Ethyl acetate (100 mL) was added to the reaction mixture, and then the mixture was washed with 1 M hydrochloric acid (50 mL) and a saturated saline solution (50 mL). The resulting organic layer was dried with magnesium sulfate and then concentrated under reduced pressure. The resulting crude product was washed with diethyl ether, then the residue was dissolved in acetone, and an undissolved portion was removed by filtration. The filtrate was concentrated under reduced pressure, and the solid was reprecipitated with acetone/hexane to give a white solid of 4-hydroxy-1-methyl-3-[4-(trifluoromethyl)benzoyl]quinolin-2(1H)-one (actual yield, 995 mg; percent yield, 29%). ¹H-NMR (400 MHz, CDCl₃) δ (ppm): 15.42 (s, 1H), 8.28 (brdd, J = 8.1, 1.6 Hz, 1H), 7.75 (ddd, J = 8.7, 7.2, 1.6 Hz, 1H), 7.72-7.67 (m, 4H), 7.36-7.29 (m, 2H), 3.59 (s, 3H). ¹⁹F-NMR (376 MHz, CDCl₃) δ (ppm): -62.9.

### (REFERENCE EXAMPLE 3)

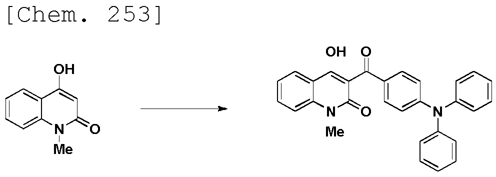

4-hydroxy-1-methylquinolin-2(1H)-one (1.48 g, 8.44 mmol) was dissolved in dichloromethane (25.0 mL). Then 4-(diphenylamino)benzoyl chloride (2.86 g, 9.28 mmol) dissolved in N,N-diisopropylethylamine (1.77 mL, 10.1 mmol) and dichloromethane (7.6 mL) was added, and the resulting mixture was stirred for 2 hours at room temperature. The reaction solution was diluted with dichloromethane (150 mL), and then the diluted solution was washed three times with 1 M hydrochloric acid (100 mL), an aqueous solution of sodium hydrogencarbonate (100 mL) and a saturated saline solution (100 mL). The resulting organic layer was dried with magnesium sulfate and then concentrated under reduced pressure to give a brown solid. The resulting crude product was dissolved in acetonitrile (35.0 mL). After the addition of acetone cyanohydrin (852 µL, 9.28 mmol) and triethylamine (1.31 mL, 9.28 mmol), the resulting mixture was stirred for 18 hours at 50°C. Ethyl acetate (100 mL) was added to the reaction mixture, and then the mixture was washed with 1 M hydrochloric acid (100 mL) and a saturated saline solution (100 mL). The resulting organic layer was dried with magnesium sulfate and then concentrated under reduced pressure. The resulting crude product was reprecipitated twice with chloroform/hexane to give a white solid of 4-hydroxy-1-methyl-3-[4-(diphenylamino)benzoyl]quinolin-2(1H)-one (actual yield, 2.35 g; percent yield, 62 %). ¹H-NMR (400 MHz, CDCl₃) δ (ppm): 15.33 (s, 1H), 8.23 (brdd, J = 8.0, 1.4 Hz, 1H), 7.69 (ddd, J = 8.6, 7.4, 1.5 Hz, 1H), 7.62 (brd, J = 8.8 Hz, 2H), 7.35-7.27 (m, 6H), 7.18 (brd, J = 7.5 Hz, 4H), 7.12 (brt, J = 7.4 Hz, 2H), 6.96 (brd, J = 8.9 Hz, 2H), 3.63 (s, 3H).

### (REFERENCE EXAMPLE 4)

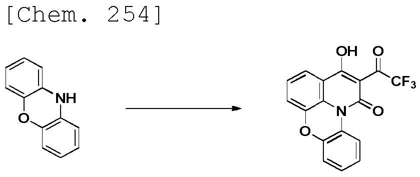

10H-phenoxazine (920 mg, 5.00 mmol) and cyanoacetic acid (430 mg, 500 mmol) were dissolved in N,N-dimethylformamide (5.0 mL). After the reaction vessel was cooled in a water bath, trifluoroacetic anhydride (2.80 mL, 20.0 mmol) was added dropwise over 1 hour, and then the resulting mixture was stirred for 21 hours at room temperature. The reaction solution was emptied into water, and the precipitated solid was collected by suction filtration and washed with water. The resulting solid was washed with acetonitrile to give a yellow-orange solid of 3-hydroxy-2-(2,2,2-trifluoroethan-1-on-yl)-1H-pyrido[3,2,1-kl]phenoxazin-1-one (actual yield, 268 mg; percent yield, 15%). ¹H-NMR (400 MHz, CDCl₃) δ (ppm): 14.39 (s, 1H), 8.65 (dd, J = 8.5, 1.6 Hz, 1H), 7.72 (brdd, J = 7.7, 1.9 Hz, 1H), 7.22-7.11 (m, 3H), 7.05 (ddd, J = 8.6, 7.5, 1.7 Hz, 1H), 7.00 (dd, J = 8.0,1.6 Hz, 1H). ¹⁹F-NMR (376 MHz, CDCl₃) δ (ppm): -73.6.

### (REFERENCE EXAMPLE 5)

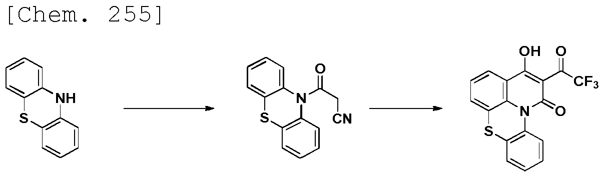

Acetic anhydride (10.0 mL) was added to a mixture of 10H-phenothiazine (2.00 g, 10.0 mmol) and cyanoacetic acid (850 mg, 10.0 mmol), and the resulting mixture was stirred for 1 hour at 80°C. The reaction solution was cooled to room temperature, and the precipitated solid was suction-filtered and washed with ethanol to give a white solid of 3-oxo-3-(10H-phenothiazin-10-yl)propionitrile (actual yield, 2.17 g; percent yield, 82%). ¹H-NMR (400 MHz, CDCl₃) δ (ppm): 7.58-7.47 (m, 4H), 7.38 (dt, J = 7.5, 1.5 Hz, 2H), 7.31 (brt, J = 7.5 Hz, 2H), 3.58 (s, 2H).

N,N-dimethylformamide (8.0 mL) was added to the 3-oxo-3-(10H-phenothiazin-10-yl)propionitrile (2.17 g, 8.15 mmol). After cooling in a water bath, trifluoroacetic anhydride (3.45 mL, 24.5 mmol) was added dropwise. The resulting mixture was stirred for 4 hours at 50°C, and then the reaction solution was cooled to room temperature and put into water, and the precipitated solid was collected by suction filtration and washed with water. Acetone was added to the resulting solid, an undissolved portion was removed by filtration, and then the filtrate was concentrated under reduced pressure and washed with diethyl ether to give a yellow-orange solid of 3-hydroxy-2-(2,2,2-trifluoroethan-1-on-1-yl)-1H-pyrido[3,2,1-kl]phenothiazin-1-one (actual yield, 728 mg; percent yield, 25%). ¹H-NMR (400 MHz, CDCl₃) δ (ppm): 14.5 (s, 1H), 7.96 (dd, J = 8.1, 1.4 Hz, 1H), 7.77 (brdd, J = 8.6, 1.1 Hz, 1H), 7.48 (brdd, J = 7.6, 1.4 Hz, 1H), 7.31-7.17 (m, 4H). ¹⁹F-NMR (376 MHz, CDCl₃) δ (ppm): - 73.7.

### (REFERENCE EXAMPLE 6)

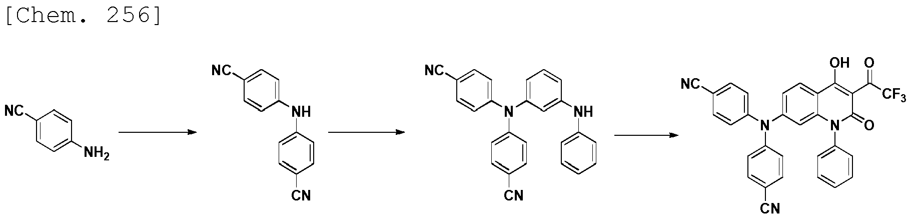

4-aminobenzonitrile (7.09 g, 60.0 mmol), 4-fluorobenzonitrile (7.27 g, 60.0 mmol) and sodium t-butoxide (11.5 g, 120 mmol) were suspended in dimethylsulfoxide (150 mL), and the resulting suspension was stirred for 5 hours at room temperature. The reaction solution was poured into water (800 mL) cooled in an ice water bath, and the precipitated solid was collected by filtration and washed with water. The resulting solid was washed with acetonitrile to give a pink solid of 4,4'-iminobisbenzonitrile (actual yield, 9.58 g; percent yield, 73%). ¹H-NMR (400 MHz, CDCl₃) δ (ppm): 7.59 (ddd, J = 9.2, 4.4, 2.1 Hz, 4H), 7.15 (ddd, J = 9.2, 4.4, 2.1 Hz, 4H), 6.31 (s, 1H).

The 4,4'-iminobisbenzonitrile (2.19 g, 10.0 mmol), copper(I) iodide (380 mg, 2.00 mmol), potassium carbonate (5.53 g, 40.0 mmol), 1,10-phenanthroline (721 mg, 4.00 mmol) and 3-bromodiphenylamine (2.59 mL, 15.0 mmol) were suspended in N,N-diethylformamide (33.3 mL), and the resulting suspension was stirred for 24 hours at 120°C. The reaction mixture was filtered through a glass filter packed with silica gel and washed with ethyl acetate. The filtrate was concentrated under reduced pressure, water (150 mL) was added to the resulting crude product, and the precipitated solid was collected by filtration. The resulting solid was washed with acetone to give a gray solid of 4,4'-{[3-(phenylamino)phenyl]imino}bisbenzonitrile (actual yield, 1.80 g; percent yield, 47%). ¹H-NMR (400 MHz, CDCl₃) δ (ppm): 7.53 (ddd, J = 9.2, 4.4, 2.4 Hz, 4H) ,7.30-7.22 (m, 3H), 7.13 (ddd, J = 9.2, 4.4, 2.4 Hz, 4H), 7.04-6.90 (m, 4H), 6.77 (t, J = 2.1 Hz, 1H), 6.62 (ddd, J = 7.9, 2.1, 0.8 Hz, 1H), 5.73 (s, 1H).

The 4,4'-{[3-(phenylamino)phenyl]imino}bisbenzonitrile (1.08 g, 2.80 mmol) and cyanoacetic acid (238 mg, 2.80 mmol) were dissolved in 1,3-dimethyl-2-imidazolidinone (5.6 mL). After the reaction vessel was cooled in a water bath, trifluoroacetic anhydride (1.57 mL, 11.2 mmol) was added dropwise over 1 hour, and then the resulting mixture was stirred for 40 hours at room temperature. The reaction solution was emptied into water, and the precipitated solid was collected by suction filtration and washed with water. The resulting solid was reprecipitated with acetone/hexane, the resulting mixture was filtered, and then the filtrate was concentrated. The resulting solid was washed with methanol to give a yellow solid of 4,4'-{[4-hydroxy-1-phenyl-3-(2,2,2-trifluoroethan-1-on-1-yl)quinolin-2(1H)-on-7-yl]imino}bisdibenzonitrile (actual yield, 32.0 mg; percent yield, 2%) . ¹H-NMR (400 MHz, CDCl₃) δ (ppm) : 14.72 (brs, 1H), 8.13 (brd, J = 9.0 Hz, 1H), 7.56 (brd, J = 8.8 Hz, 4H), 7.46-7.48 (m, 3H), 7.13-7.07 (m, 6H), 6.87 (dd, J = 9.0, 2.2 Hz, 1H), 6.00 (d, J = 2.2 Hz, 1H). ¹⁹F-NMR (376 MHz, CDCl₃) δ (ppm): -73.9.

### (REFERENCE EXAMPLE 7)

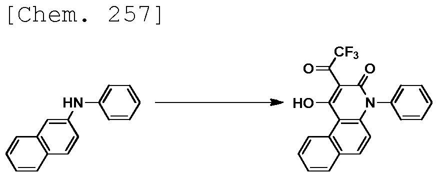

N,N-dimethylformamide (40 mL) was added to a mixture of cyanoacetic acid (3.40 g, 40.0 mmol) and N-phenyl-2-naphthylamine (8.76 g, 40.0 mmol). Trifluoroacetic anhydride (22.6 mL, 0.16 mol) was added dropwise over 1 hour in a water bath at room temperature, and the resulting mixture was further stirred for 23 hours at the same temperature. The reaction solution was emptied into water, and the precipitated solid was collected by suction filtration, washed with acetonitrile and then vacuum-dried to give a yellow-orange solid of 1-hydroxy-4-phenyl-2-(2,2,2-trifluoroethan-1-on-1-yl)benzo[f]quinolin-3(4H)-one (actual yield, 10.9 g; percent yield, 710). ¹H-NMR (400 MHz, CDCl₃) δ (ppm): 16.5 (s, 1H), 9.47 (d, J = 8.9 Hz, 1H), 7.90 (d, J = 9.8 Hz, 1H), 7.84 (d, J = 7.6 Hz, 1H), 7.77 (ddd, J = 8.6, 7.2, 1.6 Hz, 1H), 7.66-7.53 (m, 4H), 7.32-7.29 (m, 2H), 6.79 (d, J = 9.2 Hz, 1H) . ¹⁹F-NMR (376 MHz, CDCl₃) δ (ppm): -73.7.

### (REFERENCE EXAMPLE 8)

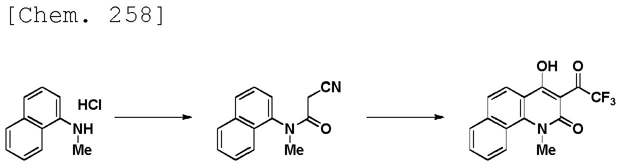

In an argon atmosphere, acetic anhydride (28.6 mL, 303 mmol) and pyridine (2.25 mL, 27.9 mmol) were added to N-methyl-1-naphthylamine hydrochloride (5.32 g, 27.5 mmol) and cyanoacetic acid (2.34 g, 27.5 mmol), and the resulting mixture was stirred for 3.3 hours at 80°C. After natural cooling, ice (100 g) and water (20 mL) were added, and the resulting mixture was stirred for 1 hour. The precipitated solid was filtered, washed with water (100 mL) and then dried by heating to give a white solid of 2-cyano-N-methyl-N-(1-naphthyl)acetamide (actual yield, 5.05 g; percent yield, 82%). ¹H-NMR (400 MHz, CDCl₃) δ (ppm): 7.99-7.93 (m, 2H), 7.75 (m, 1H), 7.68-7.58 (m, 2H), 7.55 (dd, J = 8.2, 7.2 Hz, 1H), 7.44 (dd, J = 7.2, 1.1 Hz, 1H), 3.43 (s, 3H), 3.19 (d, J = 18.1 Hz, 1H), 3.02 (d, J = 18.1 Hz, 1H).

In an argon atmosphere, the 2-cyano-N-methyl-N-(1-naphthyl)acetamide (4.01 g, 17.9 mmol) was dissolved in dehydrated N,N-dimethylformamide (18.0 mL). In a water bath, trifluoroacetic anhydride (7.50 mL, 53.6 mmol) was added dropwise over 1 hour. After 18 hours of stirring at room temperature, the addition of water (200 mL) and 1.3 hours of stirring were carried out in an ice bath. The precipitated solid was filtered and washed with water (100 mL). The washed solid was dried by heating, and acetonitrile (50 mL) was added to the resulting solid. After 10 minutes of sonication, the mixture was filtered, and the residue was washed with acetonitrile (30 mL) and dried by heating to give a deep yellow solid of 4-hydroxy-1-methyl-3-(2,2,2-trifluoroethan-1-on-1-yl)benzo[h]quinolin-2(1H)-one (actual yield, 4.08 g; percent yield, 710). ¹H-NMR (400 MHz, CDCl₃) δ (ppm): 14.31 (s, 1H), 8.39 (brd, J = 8.8 Hz, 1H), 8.07 (d, J = 8.8 Hz, 1H), 7.92 (dd, J = 8.2, 1.3 Hz, 1H), 7.70 (ddd, J = 8.2, 7.0, 1.1 Hz, 1H), 7.66 (brd, J = 8.8 Hz, 1H), 7.60 (ddd, J = 8.6, 6.9, 1.5 Hz, 1H), 3.98 (s, 3H). ¹⁹F-NMR (376 MHz, CDCl₃) δ (ppm): -74.2.

### (REFERENCE EXAMPLE 9)

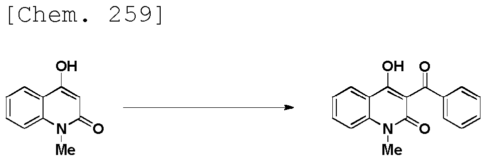

4-hydroxy-1-methylquinolin-2(1H)-one (1.75 g, 10.0 mmol) was dissolved in dichloromethane (35.0 mL). Then N,N-diisopropylethylamine (2.10 mL, 12.0 mmol) and benzoyl chloride (1.28 mL, 11.0 mmol) were added, and the resulting mixture was stirred for 2 hours at room temperature. The reaction solution was diluted with dichloromethane (175 mL), and then the diluted solution was washed three times with 1 M hydrochloric acid (120 mL), an aqueous solution of sodium hydrogencarbonate (120 mL) and a saturated saline solution (120 mL). The resulting organic layer was dried with magnesium sulfate and then concentrated under reduced pressure to give a brown solid. The resulting crude product was dissolved in acetonitrile (35.0 mL). After the addition of acetone cyanohydrin (1.12 mL, 11.1 mmol) and triethylamine (1.60 mL, 11.1 mmol), the resulting mixture was stirred for 17 hours at 50°C. Ethyl acetate (175 mL) was added to the reaction mixture, and then the mixture was washed with 1 M hydrochloric acid (120 mL) and a saturated saline solution (120 mL). The resulting organic layer was dried with magnesium sulfate and then concentrated under reduced pressure. The resulting crude product was reprecipitated with chloroform/hexane to give a white solid of 3-benzoyl-4-hydroxy-1-methylquinolin-2(1H)-one (actual yield, 1.78 g; percent yield, 64%). ¹H-NMR (400 MHz, CDCl₃) δ (ppm): 15.45 (s, 1H), 8.27 (brdd, J = 8.0, 1.5 Hz, 1H), 7.72 (ddd, J = 8.6, 7.1, 1.6 Hz, 1H), 7.64-7.61 (m, 2H), 7.52 (tt, J = 7.3, 1.3 Hz, 1H), 7.44 (brt, J = 7.0 Hz, 2H), 7.35-7.27 (m, 2H), 3.60 (s, 3H).

### (REFERENCE EXAMPLE 10)

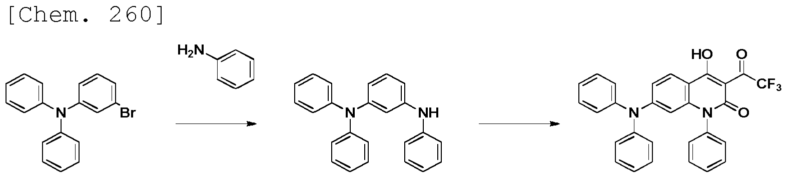

In an argon atmosphere, 3-bromotriphenylamine (4.99 g, 15.4 mmol) was dissolved in toluene (33.5 mL), and aniline (2.82 mL, 30.9 mmol) and sodium t-butoxide (4.44 g, 46.2 mmol) were added. After degassing and argon purging, tri-t-butylphosphonium tetrafluoroborate (196 mg, 675 µmol) and tris(dibenzylideneacetone)dipalladium(0) (192 mg, 157 µmol) were added, and the resulting mixture was heated under reflux for 24 hours at 120°C. After the system was returned to room temperature, water (80 mL) and chloroform (120 mL) were added, and separation was performed. The aqueous layer was subjected to extraction with chloroform (30 mL × 2), the organic layers were combined, the combined liquid was dried with sodium sulfate and filtered, and the filtrate was concentrated under reduced pressure. The resulting crude product was purified by silica gel column chromatography (eluent: hexane/ethyl acetate) to give a yellow viscous solid of N,N,N'-triphenyl-1,3-benzenediamine (actual yield, 4.64 g; percent yield, 90%). ¹H-NMR (400 MHz, CDCl₃) δ (ppm): 7.28-7.16 (m, 6H), 7.14-7.08 (m, 5H), 7.03-6.96 (m, 4H), 6.87 (tt, J = 7.3, 1.1 Hz, 1H), 6.75 (t, J = 2.2 Hz, 1H), 6.72 (ddd, J = 7.8, 2.1, 0.8 Hz, 1H), 6.62 (ddd, J = 8.2, 2.1, 0.8 Hz, 1H), 5.61 (brs, 1H).

In an argon atmosphere, the N,N,N'-triphenyl-1,3-benzenediamine (2.23 g, 6.62 mmol) and cyanoacetic acid (567 mg, 6.67 mmol) were dissolved in N,N-dimethylformamide (6.60 mL). In a water bath, trifluoroacetic anhydride (3.71 mL, 26.5 mmol) was added dropwise over 1 hour. After 19 hours of stirring at room temperature, cold water (50 mL) was poured, and the precipitated solid was filtered and washed with water (50 mL). The resulting solid was dried by heating, and acetonitrile (50 mL) was added. After 10 minutes of sonication, the mixture was filtered, and the residue was washed with acetonitrile (10 mL) and dried by heating to give an orange-yellow solid of 7-diphenylamino-4-hydroxy-1-phenyl-3-(2,2,2-trifluoroethan-1-on-1-yl)quinolin-2(1H)-one (actual yield, 565 mg; percent yield, 17%). ¹H-NMR (400 MHz, CDCl₃) δ (ppm): 14.78 (s, 1H), 7.95 (d, J = 9.1 Hz, 1H), 7.35 (tt, J = 7.1, 1.6 Hz, 2H), 7.31-7.23 (m, 4H), 7.14 (tt, J = 7.5, 1.2 Hz, 2H), 7.12-7.03 (m, 7H), 6.74 (dd, J = 9.1, 2.3 Hz, 1H), 5.78 (d, J = 2.3 Hz, 1H). ¹⁹F-NMR (376 MHz, CDCl₃) δ (ppm): -73.7.

### (REFERENCE EXAMPLE 11)

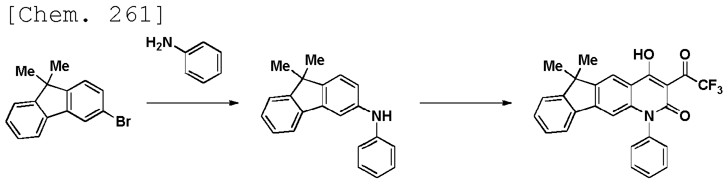

In an argon atmosphere, toluene (78.5 mL) was added to 3-bromo-9,9-dimethyl-9H-fluorene (9.86 g, 36.1 mmol). Aniline (6.60 mL, 72.3 mmol) and sodium t-butoxide (10.4 g, 108 mmol) were added, and degassing and argon purging were performed. Tri-t-butylphosphonium tetrafluoroborate (453 mg, 1.56 mmol) and tris(dibenzylideneacetone)dipalladium(0) (449 mg, 368 µmol) were added, and the resulting mixture was stirred for 14 minutes at room temperature and heated under reflux for 24 hours at 120°C. The system was returned to room temperature, water (120 mL) and chloroform (150 mL) were added, and separation was performed. The aqueous layer was subjected to extraction with chloroform (50 mL × 2), the organic layers were combined, the combined liquid was dried with sodium sulfate and filtered, and the filtrate was concentrated under reduced pressure. The resulting crude product was purified by silica gel column chromatography (eluent: hexane/ethyl acetate) to give a light orange-yellow solid of (9,9-dimethyl-9H-fluoren-3-yl)phenylamine (actual yield, 8.21 g; percent yield, 80%). ¹H-NMR (400 MHz, CDCl₃) δ (ppm): 7.64 (m, 1H), 7.47 (d, J = 2.0 Hz, 1H), 7.44-7.39 (m, 1H), 7.34-7.28 (m, 3H), 7.28-7.24 (m, 2H), 7.12-7.06 (m, 2H), 7.03 (dd, J = 8.1, 2.1 Hz, 1H), 6.92 (dddd, J = 8.1, 7.1, 1.4, 1.1 Hz, 1H), 5.74 (brs, 1H), 1.48 (s, 6H).

In an argon atmosphere, the (9,9-dimethyl-9H-fluoren-3-yl)phenylamine (4.01 g, 14.0 mmol) and cyanoacetic acid (1.20 g, 14.2 mmol) were dissolved in N,N-dimethylformamide (14.0 mL). In a water bath, trifluoroacetic anhydride (7.85 mL, 56.1 mmol) was added dropwise over 1 hour. After 19 hours of stirring at room temperature, cold water (50 mL) was poured, the supernatant was removed by decantation, and the residue was further washed with water. Diethyl ether (50 mL) was added to the resulting orange viscous solid, the resulting mixture was sonicated for 10 minutes and then filtered, and the residue was washed with diethyl ether (50 mL). The resulting mother liquor was concentrated and then purified by silica gel column chromatography (eluent: chloroform/ethyl acetate) to give an orange-yellow solid of 1-hydroxy-10,10-dimethyl-4-phenyl-2-(2,2,2-trifluoroethan-1-on-1-yl)-10H-indeno[2,3-g]quinolin-3(4H)-one (actual yield, 832 mg; percent yield, 13%). ¹H-NMR (400 MHz, CDCl₃) δ (ppm): 14.78 (s, 1H), 8.25 (s, 1H), 7.68-7.61 (m, 2H), 7.57 (m, 1H), 7.53-7.44 (m, 2H), 7.41 (td, J = 7.5, 1.1 Hz, 1H), 7.36-7.32 (m, 2H), 7.30 (td, J = 7.5, 1.2 Hz, 1H), 6.84 (s, 1H), 1.55 (s, 6H). ¹⁹F-NMR (376 MHz, CDCl₃) δ (ppm): -73.8.

### (REFERENCE EXAMPLE 12)

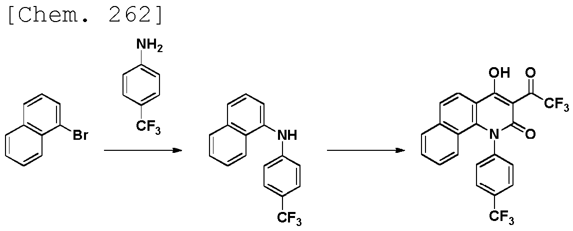

In an argon atmosphere, dehydrated toluene (12.5 mL) was added o 1-bromonaphthalene (700 µL, 5.00 mmol) and 4-(trifluoromethyl)aniline (930 µL, 7.50 mmol). After degassing, the system was placed in an argon atmosphere. Tris(dibenzylideneacetone)dipalladium(0) (74.6 mg, 81.5 µmol), sodium t-butoxide (1.21 g, 12.6 mmol) and tri-t-butylphosphine (a 1 M solution in hexane) (300 µL, 300 µmol) were added, and the resulting mixture was stirred for 24 minutes at 180°C under microwave irradiation. Ethyl acetate (10 mL) was added to the reaction mixture, the resulting mixture was filtered through Celite, then the residue was washed with ethyl acetate (300 mL), and the filtrate was concentrated under reduced pressure. Hexane (100 mL) was added to the resulting brown viscous solid, the resulting mixture was sonicated for 10 minutes and filtered, and the filtrate was concentrated to give a crude product as a light brown viscous solution. The crude product was purified by silica gel column chromatography (eluent: hexane/ethyl acetate) to give a yellow oily substance, N-(1-naphthyl)-N-[4-(trifluoromethyl)phenyl]amine (actual yield, 467 mg; percent yield, 32%). ¹H-NMR (400 MHz, CDCl₃) δ (ppm): 7.98 (m, 1H), 7.90 (m, 1H), 7.71 (dd, J = 7.4, 1.7 Hz, 1H), 7.55-7.47 (m, 2H), 7.47-7.41 (m, 4H), 6.88 (d, J = 8.5 Hz, 2H), 6.10 (brs, 1H). ¹⁹F-NMR (376 MHz, CDCl₃) δ (ppm): -61.3.

In an argon atmosphere, the N-(1-naphthyl)-N-[4-(trifluoromethyl)phenyl]amine (466 mg, 1.62 mmol) and cyanoacetic acid (142 mg, 1.67 mmol) were dissolved in dehydrated 1,3-dimethyl-2-imidazolidinone (8.10 mL). In a water bath, trifluoroacetic anhydride (910 µL, 6.50 mmol) was added dropwise over 1 hour. After 17 hours of stirring at room temperature, the addition of water (100 mL) and 1 hour of stirring were carried out in an ice bath. The precipitated solid was filtered and washed with water (50 mL). The washed solid was dried by heating, and acetonitrile (1 mL) was added to the resulting solid. After 10 minutes of sonication, the mixture was filtered, and the residue was washed with acetonitrile (5 mL) and dried by heating to give a yellow solid powder of 4-hydroxy-3-(2,2,2-trifluoroethan-1-on-1-yl)-1-[4-(trifluoromethyl)phenyl]benzo[h]quinolin-2(1H)-one (actual yield, 56.8 mg; percent yield, 7.8%). ¹H-NMR (400 MHz, CDCl₃) δ (ppm): 14.61 (s, 1H), 8.19 (d, J = 8.8 Hz, 1H), 7.85 (brd, J = 8.4 Hz, 1H), 7.76 (brd, J = 8.3 Hz, 2H), 7.73 (d, J = 8.8 Hz, 1H), 7.52 (ddd, J = 8.0, 6.7, 1.1 Hz, 1H), 7.45 (brd, J = 8.3 Hz, 2H), 7.18 (d, J = 8.8 Hz, 1H), 7.11 (ddd, J = 8.8, 6.7, 1.4 Hz, 1H). ¹⁹F-NMR (376 MHz, CDCl₃) δ (ppm): -62.6 (s, 3F), -74.2 (s, 3F) .

### (REFERENCE EXAMPLE 13)

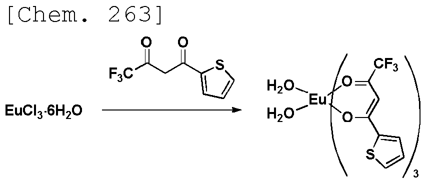

Ethanol (20 mL) and a 1 M aqueous solution of sodium hydroxide (4.5 mL, 4.5 mmol) were added to 4,4,4-trifluoro-1-(2-thienyl)-1,3-butanedione (999 mg, 4.50 mmol). Europium chloride hexahydrate (550 mg, 1.50 mmol) was added to this mixture, and the resulting mixture was stirred for 30 minutes at room temperature. Water (150 mL) was added to this reaction solution, and the resulting mixture was stirred for another 30 minutes at 60°C. The solid that formed was collected by filtration and washed with water to give a white solid of diaquatris[4,4,4-trifluoro-1-(2-thienyl)-1,3-butanedionato]europium(III) (actual yield, 695 mg; percent yield, 54%) . ¹⁹F-NMR (376 MHz, DMSO-d₆) δ (ppm) : -78.4 (brs). ESI-MS (m/z), MeOH: 839.1 [M + Na]⁺.

### (REFERENCE EXAMPLE 14)

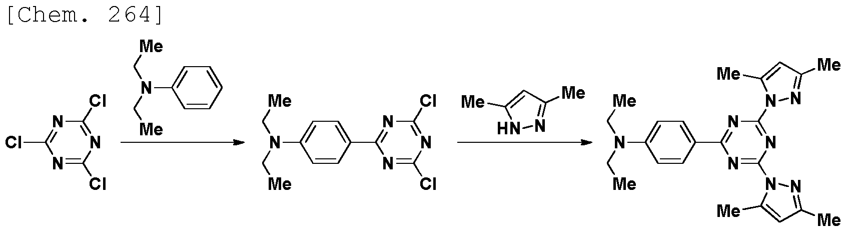

2,4,6-trichlorotriazine (18.4 g, 100 mmol) and N,N-diethylaniline (29.7 g, 199 mmol) were mixed together, and the resulting mixture was stirred for 18 hours at 70°C. After cooling to room temperature, warmed chloroform (200 mL) was added, the resulting mixture was filtered, and the filtrate was allowed to stand. The precipitated solid was collected by filtration, washed with chloroform and dried. The resulting crude product was recrystallized in acetone to give yellow-brown crystals of 4-[4,6-dichloro-1,3,5-triazin-2-yl]-N,N-diethylaniline (actual yield, 1.54 g; percent yield, 5.2%). ¹H-NMR (400 MHz, CDCl₃) δ (ppm): 8.31 (brd, J = 9.3 Hz, 2H), 6.68 (brd, J = 9.3 Hz, 2H), 3.47 (q, J = 7.1 Hz, 4H), 1.24 (t, J = 7.1 Hz, 6H).

The 4-[4,6-dichloro-1,3,5-triazin-2-yl]-N,N-diethylaniline (650 mg, 2.19 mmol) and 3,5-dimethylpyrazole (640 mg, 6.66 mmol) were dissolved in toluene (30 mL). N,N-diisopropylethylamine (3.30 mL, 19.3 mmol) was added, and the resulting mixture was stirred for 12 hours at 80°C. After cooling to room temperature, the solid was collected by filtration, washed with cooled acetonitrile and dried to give a light yellow powder of 4-[4,6-bis(3,5-dimethyl-1H-pyrazol-1-yl)-1,3,5-triazin-2-yl]-N,N-diethylaniline (actual yield, 638 mg; percent yield, 70%). ¹H-NMR (400 MHz, CDCl₃) δ (ppm): 8.40 (brd, J = 9.1 Hz, 2H), 6.72 (brd, J = 9.3 Hz, 2H), 6.08 (brs, 2H), 3.47 (q, J = 7.1 Hz, 4H), 2.85 (brs, 6H), 2.35 (s, 6H), 1.24 (t, J = 7.1 Hz, 6H).

### (REFERENCE EXAMPLE 15)

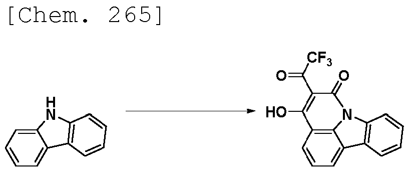

N,N-dimethylformamide (10.0 mL) was added to cyanoacetic acid (850 mg, 10.0 mmol) and carbazole (1.67 g, 10.0 mmol). Trifluoroacetic anhydride (5.64 mL, 40.0 mmol) was added dropwise over 1 hour in a water bath at room temperature, and the resulting mixture was further stirred for 23 hours at the same temperature. The reaction solution was emptied into water, and the precipitated solid was collected by suction filtration, washed with acetonitrile and then vacuum-dried to give a light yellow solid of 4-hydroxy-5-(2,2,2-trifluoroethan-1-on-1-yl)-6H-pyrido[3,2,1-jk]carbazol-6-one (actual yield, 1.62 g; percent yield, 49%). ¹H-NMR (400 MHz, CDCl₃) δ (ppm): 15.2 (s, 1H), 8.68 (d, J = 8.1 Hz, 1H), 8.29 (dd, J = 7.5, 0.8 Hz, 1H), 8.16 (dd, J = 7.8, 0.8 Hz, 1H), 8.04 (ddd, J = 7.7, 1.1, 0.7 Hz, 1H), 7.62-7.57 (m, 2H), 7.49 (td, J = 7.5, 0.8 Hz, 1H). ¹⁹F-NMR (376 MHz, CDCl₃) δ (ppm): -73.7.

### (REFERENCE EXAMPLE 16)

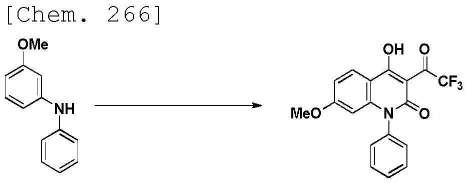

1,3-dimethyl-2-imidazolidinone (20.0 mL) was added to 3-methoxydiphenylamine (1.74 g, 10.0 mmol) and cyanoacetic acid (851 mg, 10.0 mmol). After the reaction vessel was cooled in a water bath, trifluoroacetic anhydride (5.60 mL, 40.0 mmol) was added dropwise over 1 hour, and then the resulting mixture was stirred for 19 hours at room temperature. Water (80 mL) was added to the reaction solution, and the precipitated solid was collected by suction filtration and washed with a 50% aqueous solution of ethanol. The resulting solid was washed with acetone to give a white solid of 4-hydroxy-7-methoxy-1-phenyl-3-(2,2,2-trifluoroethyl-1-on-1-yl)quinolin-2(1H)-one (actual yield, 357 mg; percent yield, 10%). ¹H-NMR (400 MHz, CDCl₃) δ (ppm): 14.78 (brs, 1H), 8.17 (d, J = 8.9 Hz, 1H), 7.61-7.56 (m, 2H), 7.54-7.49 (m, 1H), 7.29-7.26 (m, 2H), 6.82 (dd, 9.1, 2.4 Hz, 1H), 5.98 (d, 2.4 Hz, 1H), 3.70 (s, 3H). ¹⁹F-NMR (376 MHz, CDCl₃) δ (ppm): -73.9.

### (REFERENCE EXAMPLE 17)

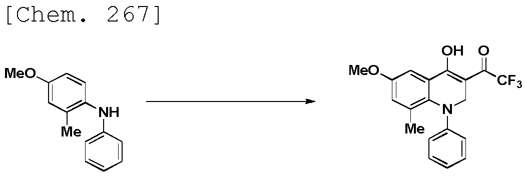

1,3-dimethyl-2-imidazolidinone (20.0 mL) was added to 4-methoxy-2-methyldiphenylamine (2.13 g, 10.0 mmol) and cyanoacetic acid (851 mg, 10.0 mmol). After the reaction vessel was cooled in a water bath, trifluoroacetic anhydride (4.20 mL, 30.0 mmol) was added dropwise over 1 hour, and then the resulting mixture was stirred for 19 hours at room temperature. Water (100 mL) was added to the reaction solution, and the resulting mixture was subjected three times to extraction with diethyl ether (100 mL). The organic layers were combined, and the combined liquid was dried with sodium sulfate and then concentrated under reduced pressure. The resulting solid was purified by silica gel column chromatography (eluent:
chloroform/methanol) to give a white solid of 4-hydroxy-6-methoxy-8-methyl-1-phenyl-3-(2,2,2-trifluoroethyl-1-on-1-yl)quinolin-2(1H)-one (actual yield, 522 mg; percent yield, 14%). ¹H-NMR (400 MHz, CDCl₃) δ (ppm): 14.55 (brs, 1H), 8.25 (dd, J = 8.0, 1.5 Hz, 1H), 7.54 (ddd, J = 7.1, 7.1, 1.5 Hz, 1H), 7.29-7.24 (m, 1H), 7.07 (d, J = 8.3 Hz, 1H), 6.92 (ddd, J = 9.3, 9.3, 2.9 Hz, 2H), 6.62 (d, 8.3 Hz, 1H), 3.85 (s, 3H), 2.01 (s, 3H). ¹⁹F-NMR (376 MHz, CDCl₃) δ (ppm): -74.0.

### (REFERENCE EXAMPLE 18)

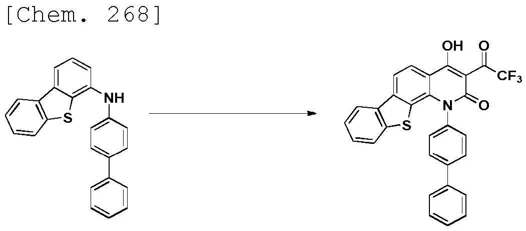

1,3-dimethyl-2-imidazolidinone (20.0 mL) was added to N-(4-biphenylyl)dibenzo[b,d]thiophen-4-amine (2.13 g, 6.07 mmol) and cyanoacetic acid (851 mg, 10.0 mmol). After the reaction vessel was cooled in a water bath, trifluoroacetic anhydride (4.20 mL, 30.0 mmol) was added dropwise over 1 hour, and then the resulting mixture was stirred for 19 hours at room temperature. Water (100 mL) was added to the reaction solution, the precipitated solid was collected by suction filtration and washed with acetonitrile and ethanol, and then the resulting solid was washed with acetone to give a yellow solid of 1-(4-biphenylyl)-4-hydroxy-3-(2,2,2-trifluoroethan-1-on-1-yl)-[1]benzothieno[3,2-h]quinolin-2(1H)-one (actual yield, 357 mg; percent yield, 10%). ¹H-NMR (400 MHz, CDCl₃) δ (ppm): 14.77 (brs, 1H), 8.39 (d, J = 8.6 Hz, 1H), 8.22-8.18 (m, 1H), 8.09 (d, J = 8.6 Hz, 1H), 7.85 (ddd, J = 8.6, 8.6, 2.0 Hz, 2H), 7.79-7.75 (m, 2H), 7.67-7.61 (m, 1H), 7.56-7.42 (m, 7H). ¹⁹F-NMR (376 MHz, CDCl₃) δ (ppm): -74.0.

### (REFERENCE EXAMPLE 19)

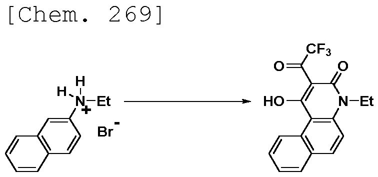

1,3-dimethyl-2-imidazolidinone (10.0 mL) was added to N-ethyl-2-naphthylamine hydrobromide (2.52 g, 10.0 mmol) and cyanoacetic acid (850 mg, 10.0 mmol). N-methylimidazole (2.39 mL, 30.0 mmol) and trifluoroacetic anhydride (5.64 mL, 40.0 mmol) were added dropwise over 2 hours in a water bath at room temperature, and the resulting mixture was further stirred for 24 hours at the same temperature. The reaction solution was emptied into water, and the precipitated solid was collected by suction filtration, washed with water, washed with acetonitrile and vacuum-dried to give 4-ethyl-1-hydroxy-2-(2,2,2-trifluoroethan-1-on-1-yl)benzo[f]quinolin-3(4H)-one as a yellow-orange solid (actual yield, 1.49 g; percent yield, 44%). ¹H-NMR (400 MHz, CDCl₃) δ (ppm): 15.7 (s, 1H), 9.43 (d, J = 8.9 Hz, 1H), 8.17 (d, J = 9.0 Hz, 1H), 7.89 (dd, J = 8.0, 1.2 Hz, 1H), 7.74 (ddd, J = 8.6, 7.2, 1.7 Hz, 1H), 7.59 (ddd, J = 7.9, 6.9, 0.8 Hz, 1H), 7.53 (d, J = 9.3 Hz, 1H), 4.46 (q, J = 7.2 Hz, 2H), 1.43 (t, J = 7.2 Hz, 3H). ¹⁹F-NMR (376 MHz, CDCl₃) δ (ppm): -73.6 (s).

### (REFERENCE EXAMPLE 20)

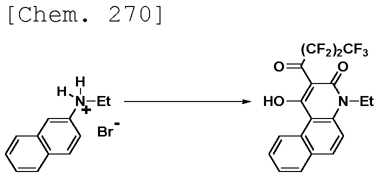

N,N-dimethylformamide (10.0 mL) was added to N-ethyl-2-naphthylamine hydrobromide (2.52 g, 10.0 mmol) and cyanoacetic acid (850 mg, 10.0 mmol). N-methylimidazole (2.39 mL, 30.0 mmol) and heptafluorobutyric anhydride (9.80 mL, 40.0 mmol) were added dropwise over 2 hours in a water bath at room temperature, and the resulting mixture was further stirred for 24 hours at the same temperature. The reaction solution was emptied into water, and the precipitated solid was collected by suction filtration, washed with water, washed with acetonitrile and vacuum-dried to give 4-ethyl-2-(2,2,2,3,3,4,4-heptafluorobutan-1-on-1-yl)-1-hydroxybenzo[f]quinolin-3(4H)-one as a yellow solid (actual yield, 1.69 g; percent yield, 390). ¹H-NMR (400 MHz, CDCl₃) δ (ppm): 15.3 (s, 1H), 9.41 (d, J = 8.9 Hz, 1H), 8.17 (d, J = 9.5 Hz, 1H), 7.89 (dd, J = 7.9, 1.2 Hz, 1H), 7.74 (ddd, J = 8.6, 7.2, 1.7 Hz, 1H), 7.58 (ddd, J = 8.0, 7.0, 1.0 Hz, 1H), 7.53 (d, J = 9.2 Hz, 1H), 4.46 (q, J = 7.1 Hz, 2H), 1.42 (t, J = 7.1 Hz, 3H). ¹⁹F-NMR (376 MHz, CDCl₃) δ (ppm): -80.1 (t, J = 10.2 Hz, 3F), -113.2 (m, 2F), -122.2 (m, 2F).

### (REFERENCE EXAMPLE 21)

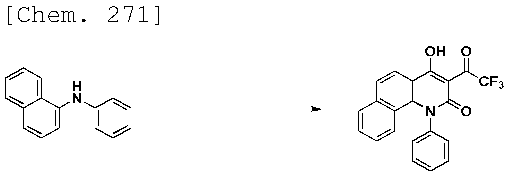

N,N-dimethylformamide (40 mL) was added to a mixture of cyanoacetic acid (1.71 g, 20.1 mmol) and N-phenyl-1-naphthylamine (4.40 g, 20.1 mmol). Trifluoroacetic anhydride (11.2 mL, 80.0 mmol) was added dropwise over 1 hour in a water bath at room temperature, and the resulting mixture was further stirred for 21 hours at the same temperature. Water (100 mL) was added to the reaction solution, and the precipitated solid was collected by suction filtration, washed with ethanol and then vacuum-dried to give an orange-yellow solid of 4-hydroxy-1-phenyl-3-(2,2,2-trifluoroethan-1-on-1-yl)benzo[h]quinolin-2(1H)-one (actual yield, 76.3 mg; percent yield, 1%). ¹H-NMR (400 MHz, CDCl₃) δ (ppm): 14.5 (s, 1H), 8.17 (d, J = 8.9 Hz, 1H), 7.81 (d, J = 8.1 Hz, 1H), 7.68 (d, J = 8.9 Hz, 1H), 7.53-7.44 (m, 4H), 7.32-7.29 (m, 2H), 7.21 (d, J = 8.9 Hz, 1H), 7.05 (ddd, J = 8.,6.8,1.4 Hz, 1H). ¹⁹F-NMR (376 MHz, CDCl₃) δ (ppm): - 74.1.

### (REFERENCE EXAMPLE 22)

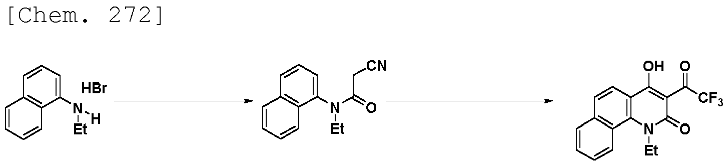

Acetic anhydride (20.0 mL, 212 mmol) was added to N-ethylnaphthylamine hydrobromide (5.04 g, 20.0 mmol) and cyanoacetic acid (1.75 g, 20.0 mmol). Pyridine (2.00 mL, 24.8 mmol) was put into this, and the resulting mixture was stirred for 5 hours at 80°C. Water with ice (120 mL) was added to the reaction solution, and the resulting mixture was stirred for 2 hours at room temperature. The solid that formed was collected by filtration, and the resulting solid was dried for 2 hours at 60°C under reduced pressure to give a light orange powder of N-ethyl-N-(1-naphthyl)-2-cyanoacetamide (actual yield, 4.00 g; percent yield, 84%). ¹H-NMR (400 MHz, CDCl₃) δ (ppm): 7.99-7.93 (m, 2H), 7.78 (brd, J = 7.5 Hz, 1H), 7.67-7.56 (m, 2H), 7.54 (brd, J = 8.3, 7.4 Hz, 1H), 7.39 (dd, J = 7.4, 1.1 Hz, 1H), 4.27 (dq, J = 13.3, 7.3 Hz, 1H), 3.51 (dq, J = 13.3, 7.3 Hz, 1H), 3.15 (d, J = 18.1 Hz, 1H), 2.99 (d, J = 18.1 Hz, 1H), 1.21 (t, J = 7.3, 3H).

N,N-dimethylformamide (10.0 mL) was added to the N-ethyl-N-(1-naphthyl)-2-cyanoacetamide (4.00 g, 16.8 mmol), and the reaction vessel was cooled in a water bath. Trifluoroacetic anhydride (5.31 mL, 50.4 mmol) was added dropwise to this over 1 hour, and the resulting mixture was stirred for 12.5 hours at room temperature. Trifluoroacetic anhydride (5.31 mL, 50.4 mmol) was added dropwise to the reaction solution over 1 hour again, and the resulting mixture was stirred for 70 hours at room temperature. Water (40 mL) was added to the reaction solution, and the resulting mixture was stirred for 30 minutes at room temperature. The solid that formed was collected by filtration, washed with water and then dried for 2 hours at 60°C under reduced pressure to give an orange solid of 1-ethyl-4-hydroxy-3-(2,2,2-trifluoroethan-1-on-1-yl)benzo[h]quinolin-2(1H)-one (actual yield, 4.88 g; percent yield, 87%) . ¹H-NMR (400 MHz, CDCl₃) δ (ppm) : 14.22 (brs, 1H), 8.37 (brd, J = 8.7 Hz, 1H), 8.07 (d, J = 8.7 Hz, 1H), 7.92 (brd, J = 8.1 Hz, 1H), 7.70 (ddd, J = 8.1, 6.9, 1.0 Hz, 1H), 7.64 (brd, J = 8.5 Hz, 1H), 7.60 (ddd, J = 8.5, 6.9, 1.5 Hz, 1H), 4.47 (q, J = 6.9 Hz, 2H), 1.70 (t, J = 6.9 Hz, 3H). ¹⁹F-NMR (376 MHz, CDCl₃) δ (ppm): -74.0.

### (REFERENCE EXAMPLE 23)

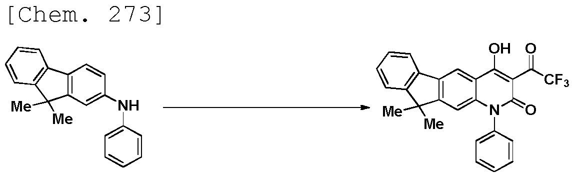

N,N-dimethylformamide (4.30 mL) was added to 2-anilino-9,9-dimethylfluorene (1.20 g, 4.20 mmol) and cyanoacetic acid (375 mg, 4.41 mmol), and the reaction vessel was cooled in a water bath. Trifluoroacetic anhydride (2.35 mL, 16.8 mmol) was added dropwise to this over 1 hour, and the resulting mixture was stirred for 17 hours at room temperature. Water (40 mL) was added to the reaction solution, and the solid that formed was collected by filtration. The resulting solid was washed with water and a small amount of acetonitrile and then dried for 1 hour at 60°C under reduced pressure. The resulting crude product was purified by silica gel column chromatography (eluent: hexane/ethyl acetate to give an orange solid of 4-hydroxy-10,10-dimethyl-1-phenyl-3-(2,2,2-trifluoroethyl-1-on-1-yl)-10H-indeno[3,2-g]quinolin-2(1H)-one (actual yield, 520 mg; percent yield, 28%). ¹H-NMR (400 MHz, CDCl₃) δ (ppm): 14.77 (brs, 1H), 8.52 (s, 1H), 7.83 (brd, J = 6.5 Hz, 1H), 7.67-7.61 (m, 2H), 7.57 (brt, J = 7.5 Hz, 1H), 7.43-7.30 (m, 5H), 6.59 (s, 1H), 1.34 (s, 6H). ¹⁹F-NMR (376 MHz, CDCl₃) δ (ppm): -73.9.

### (REFERENCE EXAMPLE 24)

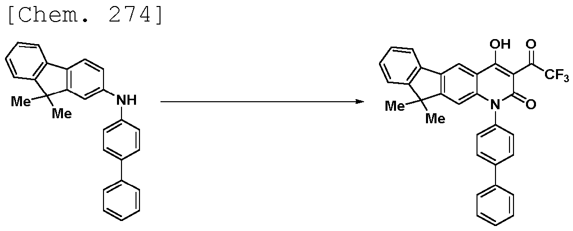

N,N-dimethylformamide (97 mL) was added to 2-(4-biphenylyl)amino-9,9-dimethylfluorene (5.30 g, 14.7 mmol) and cyanoacetic acid (1.27 g, 14.9 mmol), and the reaction vessel was cooled in a water bath. Trifluoroacetic anhydride (8.22 mL, 59.0 mmol) was added dropwise to this over 2 hours, and the resulting mixture was stirred for 40 hours at room temperature. After the reaction solution was cooled to 0°C, water (300 mL) was added, and the resulting mixture was stirred for 1 hour at room temperature. The solid that formed was collected by filtration, and the solid was washed with water and then dried for 10 hours at 60°C under reduced pressure. Acetonitrile was added to the resulting crude product, an undissolved portion was removed by filtration, and the filtrate was concentrated under reduced pressure. Diethyl ether was added to the resulting solid, and an undissolved portion was removed by filtration. This operation was repeated again to give an orange solid of 1-(4-biphenylyl)-4-hydroxy-10,10-dimethyl-3-(2,2,2-trifluoroethan-1-on-1-yl)-10H-indeno[3,2-g]quinolin-2(1H)-one (actual yield, 1.03 g; percent yield, 130). ¹H-NMR (400 MHz, CDCl₃) δ (ppm): 14.77 (brs, 1H), 8.54 (s, 1H), 7.87-7.80 (m, 3H), 7.70 (brd, J = 7.3 Hz, 2H), 7.54-7.47 (m, 2H), 7.45-7.33 (m, 6H), 6.71 (s, 1H), 1.37 (s, 6H). ¹⁹F-NMR (376 MHz, CDCl₃) δ (ppm): -73.9.

### (REFERENCE EXAMPLE 25)

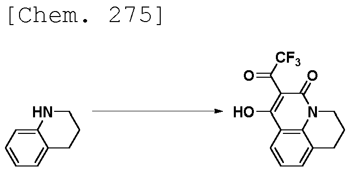

N,N-dimethylformamide (10.0 mL), N-methylimidazole (1.59 mL, 20.0 mmol) and 1,2,3,4-tetrahydroquinoline (1.26 mL, 10.0 mmol) were added to cyanoacetic acid (850 mg, 10.0 mmol). Trifluoroacetic anhydride (5.64 mL, 40.0 mmol) was added dropwise over 1 hour in a water bath at room temperature, and the resulting mixture was further stirred for 23 hours at the same temperature. The reaction solution was emptied into water, and the precipitated solid was collected by suction filtration, washed with acetonitrile and then vacuum-dried to give a light yellow solid of 6,7-dihydro-1-hydroxy-2-(trifluoroacetyl)-3H,5H-benzo[ij]quinolizin-3-one (actual yield, 1.62 g; percent yield, 54%) . ¹H-NMR (400 MHz, CDCl₃) δ (ppm): 14.4 (s, 1H), 8.06 (brd, J = 8.2 Hz, 1H), 7.50 (dq, J = 7.3, 1.3 Hz, 1H), 7.18 (t, J = 7.7 Hz, 1H), 4.16-4.11 (m, 2H), 2.96 (brt, J = 6.2 Hz, 2H), 2.14-2.07 (m, 2H). ¹⁹F-NMR (376 MHz, CDCl₃) δ (ppm): -74.0.

### (REFERENCE EXAMPLE 26)

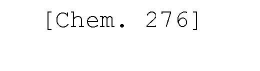

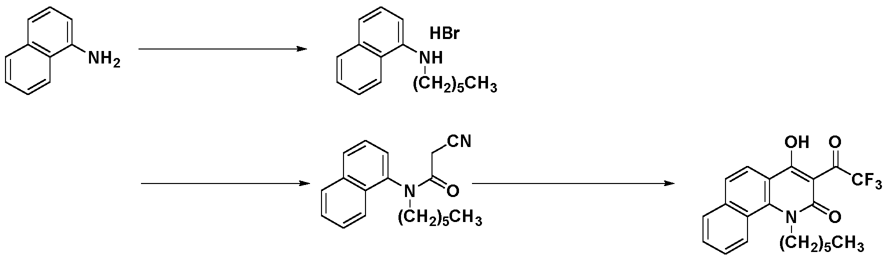

1-bromohexane (4.20 mL, 30.0 mmol) was added to 1-naphthylamine (3.00 g, 20.1 mmol) and ethanol (3.0 mL), and the resulting mixture was stirred for 22 hours with heating under reflux. After the solvent was distilled away from the reaction mixture under reduced pressure, ethyl acetate was added, and the precipitated light purple solid was collected by filtration. Chloroform was added to this, the resulting mixture was filtered through Celite, and then the chloroform was distilled away under reduced pressure to give an oily substance in light red. Acetone was added to this, and the precipitated white powder was collected by filtration to give a white powder of N-hexyl-1-naphthylamine hydrobromide (actual yield, 2.32 g; percent yield, 49%). ¹H-NMR (400 MHz, CDCl₃) δ (ppm): 11.25 (brs, 2H), 8.45 (brd, J = 8.5 Hz, 1H), 8.03 (brd, J = 7.3, 1H), 7.91-7.84 (m, 2H), 7.69 (ddd, J = 8.5, 7.0, 1.3 Hz, 1H), 7.57 (ddd, J = 8.2, 7.3, 0.7 Hz, 1H), 7.44 (dd, J = 8.1, 7.6 Hz, 1H), 3.54-3.47 (m, 2H), 2.00-1.90 (m, 2H), 1.28-1.04 (m, 6H), 0.75 (t, J = 6.9 Hz, 3H).

Acetic anhydride (6.75 mL, 71.4 mmol) was added to the N-hexyl-1-naphthylamine hydrobromide (1.94 g, 6.29 mmol) and cyanoacetic acid (537 mg, 6.31 mmol). Pyridine (530 µL, 6.57 mmol) was put into this, and the resulting mixture was stirred for 3 hours at 80°C. Water with ice (120 mL) was added to the reaction solution, and the resulting mixture was stirred for 2 hours at room temperature. The oily substance that formed was collected by filtration, and the resulting oily substance was dried for 2 hours at room temperature under reduced pressure to give a light brown liquid of N-hexyl-N-(1-naphthyl)-2-cyanoacetamide (actual yield, 1.78 g; percent yield, 96%). ¹H-NMR (400 MHz, CDCl₃) δ (ppm): 7.99-7.93 (m, 2H), 7.76 (brd, J = 7.8 Hz, 1H), 7.66-7.57 (m, 2H), 7.56-7.51 (m, 1H), 7.38 (dd, J = 7.3, 1.2 Hz, 1H), 4.27-4.19 (m, 1H), 3.41-3.31 (m, 1H), 3.15 (d, J = 18.4 Hz, 1H), 2.97 (d, J = 18.4 Hz, 1H), 1.38-1.16 (m, 8H) 0.91-0.78 (m, 3H).

N,N-dimethylformamide (2.33 mL) was added to the N-hexyl-N-(1-naphthyl)-2-cyanoacetamide (687 mg, 2.33 mmol), and the reaction vessel was cooled in a water bath. Trifluoroacetic anhydride (1.11 mL, 7.93 mmol) was added dropwise to this over 1 hour, and the resulting mixture was stirred for 19 hours at room temperature and then stirred for 25 hours at 40°C. Water (36 mL) was added to the reaction solution, and the resulting mixture was stirred for 1 hour at room temperature. The solid that formed was collected by filtration, washed with water, then dried for 1 hour at 60°C under reduced pressure and then washed with hexane. After the resulting powder was dissolved in diethyl ether, the resulting solution was washed with water. The solvent in the organic layer was distilled away under reduced pressure, and the residue was dried for 3 hours at 60°C under reduced pressure to give a yellow solid of 1-hexyl-4-hydroxy-3-(2,2,2-trifluoroethan-1-on-1-yl)benzo[h]quinolin-2(1H)-one (actual yield, 423 mg; percent yield, 46%) . ¹H-NMR (400 MHz, CDCl₃) δ (ppm) : 14.22 (brs, 1H), 8.30 (brd, J = 8.6 Hz, 1H), 8.06 (d, J = 8.6 Hz, 1H), 7.91 (brd, J = 8.1 Hz, 1H), 7.69 (ddd, J = 8.0, 6.9, 1.0 Hz, 1H), 7.63 (brd, J = 8.8 Hz, 1H), 7.59 (ddd, J = 8.6, 6.9, 1.5 Hz, 1H), 4.39 (dd, J = 8.6, 7.0 Hz, 2H), 2.11-1.97 (m, 2H), 1.39-1.22 (m, 6H), 0.86 (t, J = 6.7 Hz, 3H). ¹⁹F-NMR (376 MHz, CDCl₃) δ (ppm) : -74.0.

### (REFERENCE EXAMPLE 27)

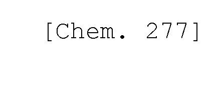

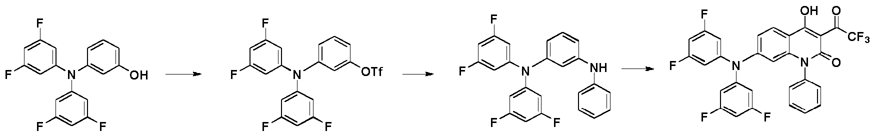

3-[bis(3,5-difluorophenyl)amino]phenol (1.17 g, 3.51 mmol) was dissolved in dichloromethane (12 mL), and pyridine (1.14 mL, 14.1 mmol) was added. After cooling to 0°C, trifluoromethanesulfonic anhydride (870 µL, 5.28 mmol) was added dropwise, and the resulting mixture was stirred for 3 hours at 0°C and then diluted with dichloromethane. The reaction mixture was washed with 1 M hydrochloric acid (20 mL), a 1 M aqueous solution of sodium hydrogencarbonate (20 mL) and a saturated saline solution (20 mL), and the organic layer was dried with sodium sulfate and then concentrated under reduced pressure to give a brown oily solid of 3-[bis(3,5-difluorophenyl)amino]phenyltrifluoromethanesulfonic acid (actual yield, 1.33 g; percent yield, 81%). ¹H-NMR (400 MHz, CDCl₃) δ (ppm): 7.39 (t, J = 8.3 Hz, 1H), 7.11 (dd, J = 8.3, 1.9 Hz, 1H), 7.05 (dd, J = 8.3, 2.2 Hz, 1H), 6.98 (t, J = 2.2 Hz, 1H), 6.61-6.55 (m, 6H). ¹⁹F-NMR (376 MHz, CDCl₃) δ (ppm): -72.7 (s, 4F), -107.8 (s, 6F).

The 3-[bis(3,5-difluorophenyl)amino]phenyltrifluoromethanesulfonic acid (487 g, 1.05 mmol), aniline (110 µL, 1.20 mmol), cesium carbonate (512 mg, 1.57 mmol), palladium acetate (24.0 mmol, 110 µmol) and 2-dicyclohexylphosphino-2',4',6'-triisopropyl-1,1'-biphenyl (100 mg, 210 µmol) were suspended in toluene (10.5 mL), and the resulting suspension was heated under reflux for 31 hours at 110°C. After the system was returned to room temperature, the suspension was diluted with dichloromethane (20 mL) and filtered through Celite, and then the filtrate was concentrated under reduced pressure. The resulting crude product was purified by silica gel column chromatography (eluent: hexane/ethyl acetate) to give a brown oily solid of N,N-bis(3,5-difluorophenyl)-N'-phenylbenzene-1,3-diamine (actual yield, 347 mg; percent yield, 81%). ¹H-NMR (400 MHz, CD₂Cl₂) δ (ppm): 7.18-7.13 (m, 3H), 6.97-6.93 (m, 2H), 6.87-6.80 (m, 2H), 6.73 (t, J = 2.2 Hz, 1H), 6.58-6.50 (m, 5H), 6.42 (tt, J = 8.9, 2.3 Hz, 2H), 5.85 (brs, 1H).

The N,N-bis(3,5-difluorophenyl)-N'-phenylbenzene-1,3-diamine (615 mg, 1.51 mmol) and cyanoacetic acid (128 mg, 1.51 mmol) were dissolved in N,N-dimethylformamide (3.00 mL), and trifluoroacetic anhydride (630 µL, 4.50 mmol) was added dropwise. After 18 hours of stirring at 80°C, water (20 mL) was poured, the precipitated solid was filtered, and the residue was washed with water (20 mL). The resulting solid was dried by heating and dissolved in N,N-dimethylformamide (3.00 mL), and then another portion of trifluoroacetic anhydride (630 µL, 4.50 mmol) was added dropwise. After 18 hours of stirring at 80°C, water (20 mL) was poured, the precipitated solid was filtered, and the residue was washed with water (20 mL). The resulting solid was dried by heating and suspended in hot hexane (10 mL), then ethanol (1.0 mL) was added, and the solid was collected by filtration to give an orange solid of 7-[bis(3,5-difluorophenyl)amino]-4-hydroxy-1-phenyl-3-(2,2,2-trifluoroethan-1-on-1-yl)quinolin-2(1H)-one (actual yield, 271 mg; percent yield, 31%). ¹H-NMR (400 MHz, CDCl₃) δ (ppm): 14.75 (s, 1H), 8.08 (d, J = 8.9 Hz, 1H), 7.48-7.42 (m, 2H), 7.14 (tt, J = 8.9, 2.2 Hz, 1H), 6.62 (tt, J = 8.7, 2.2 Hz, 1H), 6.58-6.52 (m, 4H), 5.97 (d, J = 2.2 Hz, 1H). ¹⁹F-NMR (376 MHz, CDCl₃) δ (ppm): -83.9 (s, 3F), -107.2 (s, 4F) .

### (REFERENCE EXAMPLE 28)

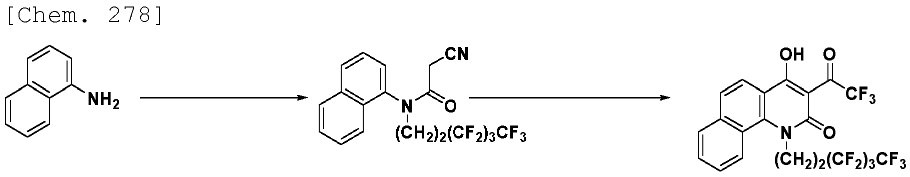

1H,1H,2H,2H-perfluorohexyl iodide (5.89 mL, 30.5 mmol) was added to 1-naphthylamine (3.06 g, 21.4 mmol) and ethanol (3.05 mL), and the resulting mixture was stirred for 17 hours with heating under reflux. After the solvent was distilled away from the reaction mixture under reduced pressure, chloroform was added, and the precipitated light blue solid was removed by filtration. Hexane was added to this, filtration was performed, and then the hexane was distilled away under reduced pressure. The resulting crude product was purified by silica gel column chromatography (eluent: hexane/ethyl acetate) to give a light brown liquid of N-(1H,1H,2H,2H-perfluorohexyl)-1-naphthylamine (actual yield, 3.37 g; percent yield, 41%). ¹H-NMR (400 MHz, CDCl₃) δ (ppm): 7.84-7.80 (m, 1H), 7.79-7.75 (m, 1H), 7.51-7.43 (m, 2H), 7.41-7.34 (m, 1H), 7.33-7.28 (m, 1H), 4.50 (brs, 1H), 3.73 (brs. 2H) 2.65-2.48 (m, 2H). ¹⁹F-NMR (376 MHz, CDCl₃) δ (ppm): -80.9 to -80.1 (m, 3F), -113.9 to -114.0 (m, 2H), - 124.3 to -124.5 (m, 2H), -125.8 to -126.1 (m, 2H).

N,N-dimethylformamide (5.02 mL) was added to the N-(1H,1H,2H,2H-perfluorohexyl)-1-naphthylamine (1.96 g, 5.02 mmol) and cyanoacetic acid (427 mg, 5.02 mmol), and the reaction vessel was cooled in a water bath. Trifluoroacetic anhydride (3.52 mL, 25.1 mmol) was added dropwise to this over 1 hour, and the resulting mixture was stirred for 21 hours at 40°C. Water (130 mL) was added to the reaction solution, and the resulting mixture was stirred for 2 hours at room temperature. The supernatant was removed by decantation, and the residue was dried for 1 hour at room temperature under reduced pressure to give a black oily substance. After washing with hexane, this was heated with added hexane until it boiled, the residue was filtered, and then the filtrate was cooled on ice. An undissolved material that precipitated in the filtrate was filtered, and then the hexane was distilled away under reduced pressure to give an orange solid. This was washed with hexamethyldisiloxane and then dried for 30 minutes at 60°C under reduced pressure to give a yellow solid of 1-(1H,1H,2H,2H-perfluorohexyl)-4-hydroxy-3-(2,2,2-trifluoroethan-1-on-1-yl)benzo[h]quinolin-2(1H)-one (actual yield, 237 mg; percent yield, 9%). ¹H-NMR (400 MHz, CDCl₃) δ (ppm): 8.13 (d, J = 8.7 Hz, 1H), 8.08 (d, J = .8.8 Hz, 1H), 7.95 (d, J = 8.2 Hz, 1H), 7.76-7.68 (m, 2H), 7.63 (ddd, J = 8.2, 7.3, 1.2, 1H), 4.74 (t, J = 7.4 Hz, 2H), 3.13-2.85 (m, 2H). ¹⁹F-NMR (376 MHz, CDCl₃) δ (ppm): -74.1 (s, 3F), -81.0 (tt, J = 9.6 Hz, 2.7 Hz, 3F), -114.3 to -114.4 (m, 2F) - 124.1 to -124.2 (m, 2H), -125.9 to -126.0 (m, 2H).

### (REFERENCE EXAMPLE 29)

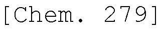

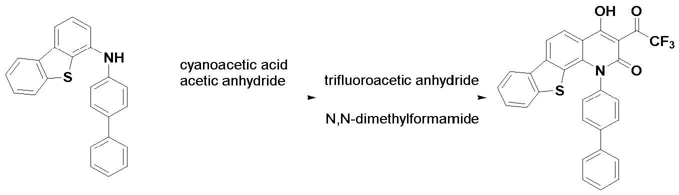

N-(4-biphenylyl)dibenzo[b,d]thiophen-4-amine (351 mg, 1.00 mmol) and cyanoacetic acid (85.0 mg, 1.00 mmol) were dissolved in acetic anhydride (2.00 mL), and the resulting solution was stirred for 4 hours at 80°C. The reaction mixture was poured into cold water (20 mL), and ethanol (1.0 mL) was added. After 30 minutes of stirring, the precipitated solid was collected by suction filtration, and the resulting solid was washed with ethanol to give a whitish brown solid. The resulting whitish brown solid was dissolved in N,N-dimethylformamide (2.0 mL), trifluoroacetic anhydride (290 µL, 2.07 mmol) was added dropwise, and then the resulting mixture was stirred for 24 hours at 40°C. Water (20 mL) was added to the reaction solution, the precipitated solid was collected by suction filtration, and a 1 M aqueous solution of sodium hydroxide (10 mL) was added. Water (20 mL) was added to the suspension, the resulting mixture was stirred for 1 hour and then filtered through Celite, and the residue was washed with water. 1 M hydrochloric acid (10 mL) was added to the mother liquor, the precipitated solid was collected by suction filtration, and the resulting solid was washed with diethyl ether to give a yellow solid of 1-(4-biphenylyl)-4-hydroxy-3-(2,2,2-trifluoroethan-1-on-1-yl)-[1]benzothieno[3,2-h]quinolin-2(1H)-one (actual yield, 296 mg; percent yield, 57%). ¹H-NMR (400 MHz, CDCl₃) δ (ppm): 14.77 (brs, 1H), 8.39 (d, J = 8.6 Hz, 1H), 8.22-8.18 (m, 1H), 8.09 (d, J = 8.6 Hz, 1H), 7.85 (ddd, J = 8.6, 8.6, 2.0 Hz, 2H), 7.79-7.75 (m, 2H), 7.67-7.61 (m, 1H), 7.56-7.42 (m, 7H). ¹⁹F-NMR (376 MHz, CDCl₃) δ (ppm): -74.0.

### (REFERENCE EXAMPLE 30)

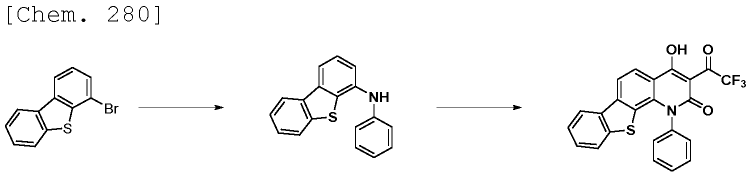

Toluene (30.0 mL) was put into 4-bromodibenzothiophene (2.63 g, 10.0 mmol) and sodium t-butoxide (1.44 g, 15.0 mmol), and aniline (1.10 mL, 12.1 mmol) was added to this. After a degassing operation was performed, a 1 M solution of tri-t-butylphosphine in hexane (200 µL, 200 µmol) and tris(dibenzylideneacetone)dipalladium(0) (91.6 mg, 100 µmol) were added, and the resulting mixture was stirred for 15 hours 30 minutes at 100°C. After natural cooling to room temperature, water (30 mL) was added, and extraction with ethyl acetate (30 mL) was performed three times. The organic layers were combined, and the combined liquid was washed with water (10 mL) and a saturated saline solution (10 mL), then dried with magnesium sulfate and concentrated under reduced pressure. The resulting crude product was washed with methanol to give a yellow-green solid of N-phenyldibenzo[b,d]thiophen-4-amine (actual yield, 2.21 g; percent yield, 80%). The washing, furthermore, was concentrated under reduced pressure, and the resulting residue was washed with ethanol to give a yellow-green solid of N-phenyldibenzo[b,d]thiophen-4-amine (actual yield, 264 mg; percent yield, 10%). ¹H-NMR (400 MHz, DMSO-d₆) δ (ppm): 8.35 (m, 1H), 8.18 (brs, 1H), 8.03 (m, 1H), 7.55-7.49 (m, 2H), 7.46 (dd, J = 7.8, 7.8 Hz, 1H), 7.32 (brd, J = 7.8 Hz, 1H), 7.26-7.20 (m, 2H), 6.99 (bd, J = 8,6 Hz, 2H), 6.95 (brt, J = 7.3 Hz, 1H).

The N-phenyldibenzo[b,d]thiophen-4-amine (551 mg, 2.00 mmol) and cyanoacetic acid (170 mg, 2.00 mmol) were dissolved in acetic anhydride (4.00 mL), and the resulting solution was stirred for 4 hours at 80°C. The reaction mixture was poured into water (20 mL), and ethanol (2.0 mL) was added. After 30 minutes of stirring, the precipitated solid was collected by suction filtration and washed with ethanol to give a brown solid. The resulting brown solid was dissolved in N,N-dimethylformamide (4.0 mL), trifluoroacetic anhydride (840 µL, 6.00 mmol) was added dropwise, and then the resulting mixture was stirred for 24 hours at 40°C. Water (20 mL) was added to the reaction solution, the precipitated solid was collected by suction filtration, and hot hexane (10 mL) and ethanol (1.0 mL) were added. The solid that settled down was collected by filtration and washed with hexane:ethanol = 1:1 (20 mL) to give a yellow solid of 1-phenyl-4-hydroxy-3-(2,2,2-trifluoroethan-1-on-1-yl)-[1]benzothieno[3,2-h]quinolin-2(1H)-one (actual yield, 539 mg; percent yield, 61%). ¹H-NMR (400 MHz, CDCl₃) δ (ppm): 14.75 (brs, 1H), 8.38 (d, J = 8.7 Hz, 1H), 8.29 (m, 1H), 8.07 (d, J = 8.7 Hz, 1H), 7.70 (ddd, J = 6.3, 6.3, 1.2 Hz, 1H), 7.65-7.59 (m, 3H), 7.50-7.45 (m, 4H). ¹⁹F-NMR (376 MHz, CDCl₃) δ (ppm): -74.0.

### (REFERENCE EXAMPLE 31)

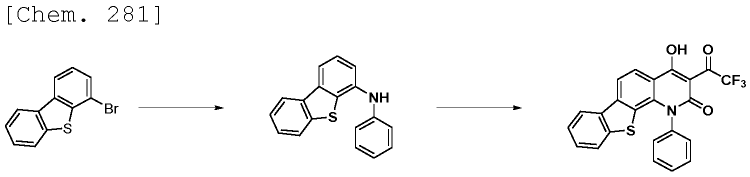

Aniline (5.00 mL, 54.8 mmol), sodium t-butoxide (5.78 g, 60.2 mmol), tri-t-butylphosphine (a 1 M solution in hexane, 500 µL, 500 µmol), tris(dibenzylideneacetone)dipalladium(0) (115 mg, 126 µmol) and 4-bromodibenzothiophene (13.2 g, 50.0 mmol) were suspended in toluene (50.0 mL), and then the resulting suspension was stirred for 16 hours at 100°C. The reaction mixture was diluted with ethyl acetate (40.0 mL) and then filtered through Celite, the residue was washed with ethyl acetate, and then the filtrate was concentrated under reduced pressure. The resulting solid was washed with methanol to give a white solid of N-phenyldibenzo[b,d]thiophen-4-amine (actual yield, 13.0 g; percent yield, 94%). ¹H-NMR (400 MHz, CDCl₃) δ (ppm): 8.16 (m, 1H), 7.89-7.84 (m, 2H), 7.50-7.45 (m, 2H), 7.41 (dd, 7.7, 7.7 Hz, 1H), 7.35 (dd, 7.7, 0.7 Hz, 1H), 7.33-7.28 (m, 2H), 7.08 (dd, 8.6, 1.2 Hz, 2H), 7.00 (tt, 7.4, 1.2 Hz, 1H), 5.66 (s, 1H).

The N-phenyldibenzo[b,d]thiophen-4-amine (13.0 g, 47.1 mmol) and cyanoacetic acid (4.00 g, 47.1 mmol) were dissolved in N,N-dimethylformamide (94.0 mL), trifluoroacetic anhydride (26.4 mL, 188 mmol) was added dropwise, and then the resulting mixture was stirred for 24 hours at room temperature. Water (300 mL) was added to the reaction solution, and the precipitated solid was collected by suction filtration. Hexane (350 mL) and ethanol (350 mL) were added to the resulting solid, and the resulting mixture was stirred for 20 minutes while being heated and then filtered by hot filtration. The residue was washed with hot hexane to give a yellow solid of 1-phenyl-4-hydroxy-3-(2,2,2-trifluoroethan-1-on-1-yl)-[1]benzothieno[3,2-h]quinolin-2(1H)-one (actual yield, 13.1 g; percent yield, 63%). ¹H-NMR (400 MHz, CDCl₃) δ (ppm): 14.75 (brs, 1H), 8.38 (d, J = 8.7 Hz, 1H), 8.29 (m, 1H), 8.07 (d, J = 8.7 Hz, 1H), 7.70 (ddd, J = 6.3, 6.3, 1.2 Hz, 1H), 7.65-7.59 (m, 3H), 7.50-7.45 (m, 4H). ¹⁹F-NMR (376 MHz, CDCl₃) δ (ppm): -74.0.

### (REFERENCE EXAMPLE 32)

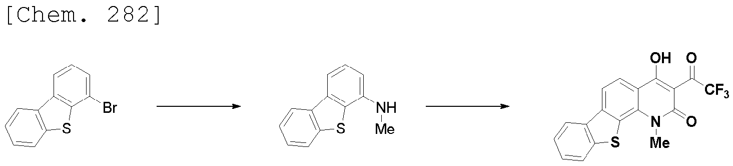

Toluene (30.0 mL) was put into 4-bromodibenzothiophene (2.63 g, 9.99 mmol), sodium t-butoxide (1.45 g, 15.1 mmol), and a 2.0 M solution of methylamine in tetrahydrofuran (7.50 mL, 15.0 mmol) was added to this. After 5 minutes of stirring at room temperature, a degassing operation was performed. 2-dicyclohexylphosphino-2',4',6'-triisopropylbiphenyl (477 mg, 1.00 mmol) and tris(dibenzylideneacetone)dipalladium(0) (232 mg, 253 µmol) were put into this, and the resulting mixture was stirred for 14 hours at 80°C. After natural cooling to room temperature, the mixture was cooled to 0°C, water (30 mL) was added, and extraction with ethyl acetate (30 mL) was performed three times. The organic layers were combined, and the combined liquid was washed with water (30 mL) and a saturated saline solution (30 mL), then dried with magnesium sulfate and concentrated under reduced pressure. The resulting crude product was purified with a silica gel column chromatograph (eluent: hexane/chloroform) to give a pink oily substance, N-methyldibenzo[b,d]thiophen-4-amine (actual yield, 1.13 g; percent yield, 530). ¹H-NMR (400 MHz, CDCl₃) δ (ppm): 8.13 (m, 1H), 7.87 (m, 1H), 7.61 (dd, J = 7.8, 0.9 Hz, 1H), 7.48-7.42 (m, 2H), 7.41 (dd, J = 7.8, 7.8 Hz, 1H), 6.73 (brd, J = 7.8 Hz, 1H), 3.72 (brs, 1H), 3.06 (s, 3H).

The N-methyldibenzo[b,d]thiophen-4-amine (920 mg, 4.32 mmol) and cyanoacetic acid (367 mg, 4.32 mmol) were dissolved in N,N-dimethylformamide (10.0 mL). Trifluoroacetic anhydride (2.42 mL, 17.3 mmol) was added dropwise in a water bath, and the resulting mixture was stirred for 40 hours at room temperature. Water (40 mL) was added to the reaction solution, the precipitated solid was collected by suction filtration, and the residue was washed with diethyl ether (20 mL) and acetone (10 mL) to give a yellow solid of 1-methyl-4-hydroxy-3-(2,2,2-trifluoroethan-1-on-1-yl)-[1]benzothieno[3,2-h]quinolin-2(1H)-one (actual yield, 102 mg; percent yield, 27%). ¹H-NMR (400 MHz, CDCl₃) δ (ppm): 14.48 (s, 1H), 8.32 (d, J = 8.6 Hz, 1H), 8.28 (d, J = 7.3 Hz, 1H), 8.07 (d, J = 8.6 Hz, 1H), 7.95 (brd, J = 7.3 Hz, 1H), 7.66-7.56 (m, 2H), 4.27 (s, 3H). ¹⁹F-NMR (376 MHz, CDCl₃) δ (ppm) : -74.0.

### (REFERENCE EXAMPLE 33)

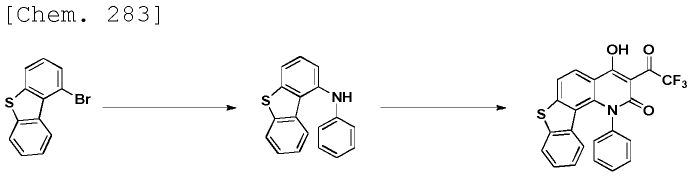

Aniline (205 mg, 2.20 mmol), sodium t-butoxide (480 mg, 4.99 mmol), tri-t-butylphosphine (a 1 M solution in hexane, 120 µL, 120 µmol), tris(dibenzylideneacetone)dipalladium(0) (31.0 mg, 33.9 µmol) and 1-bromodibenzothiophene (526 mg, 2.00 mmol) were suspended in toluene (14.0 mL), and then the resulting mixture was stirred for 24 hours at 120°C. Water (10 mL) was added to the reaction mixture, and extraction with ethyl acetate (10 mL) was performed three times. The organic layers were combined, and the combined liquid was dried with sodium sulfate and then concentrated under reduced pressure. After washing with methanol, the residue was recrystallized in methanol/water to give a light brown solid of N-phenyldibenzo[b,d]thiophen-1-amine (actual yield, 531 mg; percent yield, 97%). ¹H-NMR (400 MHz, DMSO-d₆) δ (ppm): 8.34 (d, 7.8 Hz, 1H), 8.26 (s, 1H), 8.01 (d, 7.8 Hz, 1H), 7.89 (d, 7.8 Hz, 1H), 7.49 (d, 7.8 Hz, 1H), 7.44 (d, 7.8 Hz, 1H), 7.37 (ddd, 7.8, 7.8, 1.2 Hz, 1H), 7.31 (d, 7.8 Hz, 1H), 7.17 (dd, 8.6, 7.2 Hz, 2H), 7.83 (dd, 8.6, 1.2 Hz, 2H), 6.77 (brt, 7.2 Hz, 1H).

The N-phenyldibenzo[b,d]thiophen-1-amine (531 mg, 1.93 mmol) and cyanoacetic acid (164 mg, 1.93 mmol) were dissolved in acetic anhydride (4.00 mL), and the resulting solution was stirred for 23 hours at 80°C. The reaction mixture was poured into water (30 mL), and ethanol (2.0 mL) was added. After 30 minutes of stirring, the precipitated solid was collected by suction filtration to give a brown solid. The resulting brown solid was dissolved in N,N-dimethylformamide (4.00 mL), trifluoroacetic anhydride (820 µL, 1.93 mmol) was added dropwise, and then the resulting mixture was stirred for 24 hours at 40°C. Water (20 mL) was added to the reaction solution, and the precipitated solid was collected by suction filtration and washed with a small amount of acetone. Diethyl ether was added to the resulting solid, and an undissolved portion was removed by filtration. After reprecipitation with acetone/hexane, the supernatant was concentrated to give a yellow solid of 4-hydroxy-1-phenyl-3-(2,2,2-trifluoroethan-1-on-1-yl)-[1]benzothieno[2,3-h]quinolin-2(1H)-one (actual yield, 226 mg; percent yield, 27%). ¹H-NMR (400 MHz, CDCl₃) δ (ppm): 14.72 (brs, 1H), 8.35 (dd, J = 8.1, 1.5 Hz, 1H), 8.04 (dd, J = 8.1, 0.9 Hz, 1H), 7.85 (d, 8.1 Hz, 1H), 7.66 (dd, 7.8, 7.8 Hz, 1H), 7.44 (ddd, 8.6, 7.1, 1.5, 1H), 7.38 (ddd, 8.1, 7.1, 1.5, 1H), 7.30 (ddd, 8.6, 7.8, 0.9, 2H), 7.24-7.14 (m, 2H), 6.55 (d, 8.1 Hz, 1H). ¹⁹F-NMR (376 MHz, CDCl₃) δ (ppm): - 74.0.

### (REFERENCE EXAMPLE 34)

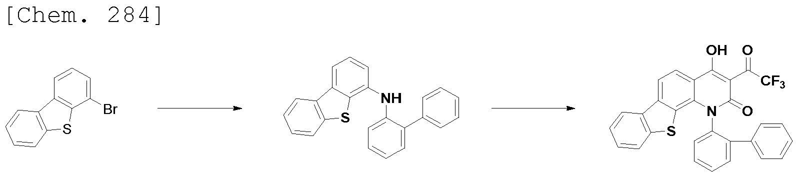

Toluene (30.5 mL) was put into 4-bromodibenzothiophene (2.63 g, 10.0 mmol), 2-aminobiphenyl (2.54 g, 15.0 mmol) and sodium t-butoxide (1.47 g, 15.3 mmol). After a degassing operation was performed, a 1 M solution of tri-t-butylphosphine in hexane (1.00 mL, 1.00 mmol) and tris(dibenzylideneacetone)dipalladium(0) (232 mg, 253 µmol) were added, and the resulting mixture was stirred for 24 hours at 80°C. After natural cooling to room temperature, water (30 mL) was added, and extraction with ethyl acetate (30 mL) was performed three times. The organic layers were combined, and the combined liquid was washed with water (30 mL) and a saturated saline solution (30 mL), then dried with magnesium sulfate and concentrated under reduced pressure. The resulting crude product was washed with methanol, and the resulting solid was recrystallized in hexane/chloroform to give a pink solid of N-([1,1'-biphenyl]-2-yl)dibenzo[b,d]thiophen-4-amine (actual yield, 2.41 g; percent yield, 69%) . ¹H-NMR (400 MHz, CDCl₃) δ (ppm) : 8.14 (m, 1H), 7.85 (dd, J = 7.6, 1.0 Hz, 1H), 7.56-7.52 (m, 2H), 7.49-7.34 (m, 7H), 7.32 (dd, J = 7.6, 1.6 Hz, 1H), 7.25 (m, 1H), 7.20 (dd, J = 8.1, 1.4 Hz, 1H), 7.04 (ddd, J = 8.7, 7.6, 1.5 Hz, 2H), 6.74 (brs, 1H).

The N-([1,1'-biphenyl]-2-yl)dibenzo[b,d]thiophen-4-amine (738 mg, 2.10 mmol) and cyanoacetic acid (178 mg, 2.10 mmol) were dissolved in acetic anhydride (2.00 mL), and the resulting solution was stirred for 4 hours at 80°C. The reaction mixture was poured into cold water (20 mL), ethanol (2.0 mL) was added, and the precipitated solid was collected by suction filtration. The resulting solid was reprecipitated with acetone/hexane to give a white individual. The resulting white solid was dissolved in N,N-dimethylformamide (2.0 mL), trifluoroacetic anhydride (882 µL, 6.30 mmol) was added dropwise, and then the resulting mixture was stirred for 17 hours at 40°C. Water (20 mL) was added to the reaction solution, and the precipitated solid was collected by suction filtration. After reprecipitation with acetone/hexane, the solid was washed with a small amount of acetone to give a yellow solid of 1-([1,1'-biphenyl]-2-yl)-4-hydroxy-3-(2,2,2-trifluoroethan-1-on-1-yl)-[1]benzothieno[3,2-h]quinolin-2(1H)-one (actual yield, 190 mg; percent yield, 22%). ¹H-NMR (400 MHz, CDCl₃) δ (ppm): 14.50 (brs, 1H), 8.20 (d, J = 8.6 Hz, 1H), 8.17 (dd, 7.0, 2.1 Hz, 1H), 7.96 (d, J = 8.6 Hz, 1H), 7.76 (ddd, J = 7.6, 7.6, 1.4 Hz, 1H), 7.70 (dd, 7.0, 2.1 Hz, 1H), 7.62 (ddd, J = 7.6, 7.6, 1.4 Hz, 1H), 7.57-7.45 (m, 4H), 7.17-7.03 (m, 5H). ¹⁹F-NMR (376 MHz, CDCl₃) δ (ppm): -74.0.

### (REFERENCE EXAMPLE 35)

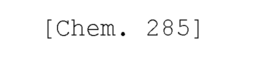

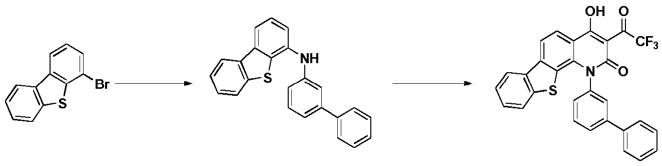

3-aminobiphenyl (931 mg, 5.50 mmol), sodium t-butoxide (1.20 g, 12.5 mmol), tri-t-butylphosphine (a 1 M solution in hexane, 300 µL, 300 µmol), tris(dibenzylideneacetone)dipalladium(0) (70.0 mg, 76.4 µmol) and 4-bromodibenzothiophene (1.32 g, 5.00 mmol) were suspended in toluene (10.0 mL), and then the resulting suspension was stirred for 24 hours at 120°C. The reaction mixture was filtered through silica gel, the residue was washed with chloroform, and then the filtrate was concentrated under reduced pressure. The resulting solid was reprecipitated with chloroform/hexane, and the supernatant was concentrated under reduced pressure to give a light brown solid of N-([1,1'-biphenyl]-3-yl)dibenzo[b,d]thiophen-4-amine (actual yield, 1.22 g; percent yield, 70%). ¹H-NMR (400 MHz, CDCl₃) δ (ppm): 8.17 (m, 1H), 7.89-7.84 (m, 2H), 7.60-7.55 (m, 2H), 7.49-7.46 (m, 2H), 7.44-7.39 (m, 4H), 7.38-7.42 (m, 2H), 7.31 (dd, 1.8, 1.8 Hz, 1H), 7.21 (ddd, 7.8, 1.8, 1.0 Hz, 1H), 7.06 (ddd, 7.8, 2.3, 1.0 Hz, 2H), 5.75 (s, 1H).

The N-([1,1'-biphenyl]-3-yl)dibenzo[b,d]thiophen-4-amine (702 mg, 2.00 mmol) and cyanoacetic acid (170 mg, 2.00 mmol) were dissolved in acetic anhydride (4.00 mL), and the resulting solution was stirred for 17 hours at 80°C. The reaction mixture was poured into cold water (20 mL), and the precipitated solid was collected by suction filtration. The resulting solid was washed with diethyl ether, and the filtrate was concentrated under reduced pressure to give a brown individual. The resulting brown solid was dissolved in N,N-dimethylformamide (4.0 mL), and trifluoroacetic anhydride (840 µL, 6.00 mmol) was added dropwise. After 21 hours of stirring at 40°C, another portion of trifluoroacetic anhydride (840 µL, 6.00 mmol) was added dropwise, and the resulting mixture was stirred for 21 hours at 40°C. Water (20 mL) was added to the reaction solution, and the precipitated solid was collected by suction filtration and purified by silica gel column chromatography (eluent: chloroform/methanol) to give a yellow solid of 1-([1,1'-biphenyl]-3-yl)-4-hydroxy-3-(2,2,2-trifluoroethan-1-on-1-yl)-[1]benzothieno[3,2-h]quinolin-2(1H)-one (actual yield, 23.9 mg; percent yield, 2%). ¹H-NMR (400 MHz, CDCl₃) δ (ppm): 14.77 (brs, 1H), 8.40 (d, J = 8.6 Hz, 1H), 8.20 (dd, 6.1, 3.1 Hz, 1H), 8.08 (d, J = 8.6 Hz, 1H), 7.76 (ddd, J = 7.8, 1.4, 1.4 Hz, 1H), 7.72-7.60 (m, 5H), 7.50-7.40 (m, 5H), 7.36 (ddd, 6.1, 1.4, 1.4 Hz, 1H). ¹⁹F-NMR (376 MHz, CDCl₃) δ (ppm): -74.0.

### (REFERENCE EXAMPLE 36)

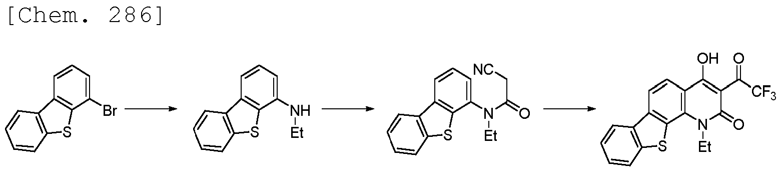

Toluene (32.0 mL) was put into 4-bromodibenzothiophene (2.74 g, 10.2 mmol) and sodium t-butoxide (1.52 g, 15.8 mmol), and a 2.0 M solution of ethylamine in tetrahydrofuran (8.00 mL, 16.0 mmol) was added to this. After 5 minutes of stirring at room temperature, a degassing operation was performed. A 1 M solution of tri-t-butylphosphine in hexane (1.05 mL, 1.05 mmol) and tris(dibenzylideneacetone)dipalladium(0) (239 mg, 251 µmol) were put into this, and the resulting mixture was stirred for 14 hours at 80°C. After natural cooling to room temperature, the mixture was cooled to 0°C, water (30 mL) was added, and extraction with ethyl acetate (30 mL) was performed three times. The organic layers were combined, and the combined liquid was washed with water (30 mL) and a saturated saline solution (30 mL), then dried with magnesium sulfate and concentrated under reduced pressure. The resulting crude product was purified with a silica gel column chromatograph (eluent: hexane/chloroform) to give a pink oily substance, N-ethyldibenzo[b,d]thiophen-4-amine (actual yield, 1.35 g; percent yield, 570). ¹H-NMR (400 MHz, CDCl₃) δ (ppm): 8.13 (m, 1H), 7.87 (m, 1H), 7.60 (dd, J = 7.8, 0.9 Hz, 1H), 7.47-7.42 (m, 2H), 7.38 (dd, J = 7.8, 7.8 Hz, 1H), 6.73 (brd, J = 7.8 Hz, 1H), 3.54 (brs, 1H), 3.49 (q, J = 7.1 Hz, 2H), 1.39 (t, 7.1 Hz, 3H).

Dichloromethane (27.0 mL) was added to the N-ethyldibenzo[b,d]thiophen-4-amine (619 mg, 2.72 mmol), cyanoacetic acid (270 mg, 3.12 mmol) and 4-dimethylaminopyridine (33.3 mg, 273 µmol). After cooling to 0°C, 1-(3-dimethylaminopropyl)-3-ethylcarbodiimide hydrochloride (557 mg, 2.91 mmol) was added, and the resulting mixture was stirred for 4 hours at room temperature. The reaction solution was washed with 1 M hydrochloric acid (5 mL × 2) and a saturated aqueous solution of sodium hydrogencarbonate (5 mL), dried with sodium sulfate and then concentrated under reduced pressure to give a light yellow solid of 2-cyano-N-ethyl-N-(dibenzo[b,d]thiophen-4-yl)acetamide (actual yield, 853 mg; percent yield, quant.). This crude product was used in the next reaction directly, without further purification. ¹H-NMR (400 MHz, CDCl₃) δ (ppm): 8.13 (m, 1H), 7.87 (m, 1H), 7.60 (dd, J = 7.8, 0.9 Hz, 1H), 7.47-7.42 (m, 2H), 7.38 (dd, J = 7.8, 7.8 Hz, 1H), 6.73 (brd, J = 7.8 Hz, 1H), 3.54 (brs, 1H), 3.49 (q, J = 7.1 Hz, 2H), 1.39 (t, 7.1 Hz, 3H).

N,N-dimethylformamide (14.5 mL) was added to the 2-cyano-N-ethyl-N-(dibenzo[b,d]thiophen-4-yl)acetamide obtained in the above and cyanoacetic acid (44.1 mg, 581 µmol). Trifluoroacetic anhydride (1.65 mL, 11.8 mmol) was added to this, and the resulting mixture was stirred for 24 hours at 80°C. The reaction solution was put into water with ice (60 mL), and the resulting mixture was stirred for 30 minutes at room temperature. The precipitated solid was collected by filtration, washed with water and then dried for 2 hours at 60°C under reduced pressure. The resulting crude product was washed with acetonitrile to give a yellow-green solid of 1-ethyl-4-hydroxy-3-(2,2,2-trifluoroethan-1-on-1-yl)-[1]benzothieno[3,2-h]quinolin-2-one (actual yield, 507 mg; percent yield, 45%). ¹H-NMR (400 MHz, CDCl₃) δ (ppm): 14.38 (brs, 1H), 8.35 (d, J = 8.6 Hz, 1H), 8.26 (brd, J = 7.4 Hz, 1H), 8.08 (d, J = 8.6 Hz, 1H), 7.95 (brd, J = 7.6 Hz, 1H), 7.64 (brdd, J = 7.6, 7.4 Hz, 1H), 7.58 (brdd, J = 7.6, 7.4 Hz, 1H), 4.86 (q, J = 7.1 Hz, 2H), 1.59 (t, 7.1 Hz, 3H). ¹⁹F-NMR (376 MHz, CDCl₃) δ (ppm): -74.0 (s).

### (REFERENCE EXAMPLE 37)

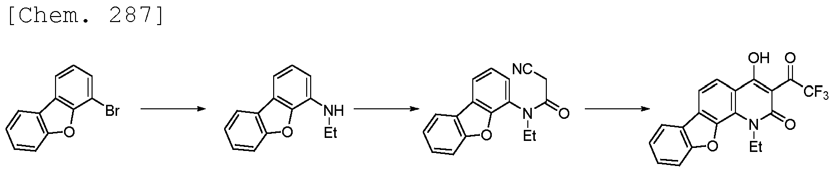

Toluene (34.0 mL) was put into 4-bromodibenzofuran (2.75 g, 11.2 mmol) and sodium t-butoxide (1.62 g, 16.9 mmol), and a 2.0 M solution of ethylamine in tetrahydrofuran (8.50 mL, 17.0 mmol) was added to this. After 5 minutes of stirring at room temperature, a degassing operation was performed. A 1 M solution of tri-t-butylphosphine in hexane (1.10 mL, 1.10 mmol) and tris(dibenzylideneacetone)dipalladium(0) (255 mg, 278 µmol) were put into this, and the resulting mixture was stirred for 14 hours at 80°C. After natural cooling to room temperature, the mixture was cooled to 0°C, water (30 mL) was added, and extraction with ethyl acetate (30 mL) was performed three times. The organic layers were combined, and the combined liquid was washed with water (30 mL) and a saturated saline solution (30 mL), then dried with magnesium sulfate and concentrated under reduced pressure. The resulting crude product was purified with a silica gel column chromatograph (eluent: hexane/chloroform) to give a pink oily substance, N-ethyldibenzofuran-4-amine (actual yield, 528 g; percent yield, 22%). ¹H-NMR (400 MHz, CDCl₃) δ (ppm): 7.92 (brd, J = 7.8 Hz, 1H), 7.55 (brd, J = 8.2 Hz, 1H), 7.42 (ddd, J = 8.2, 7.8, 1.3 Hz, 1H), 7.31 (ddd, J = 8.2, 7.8, 0.9 Hz, 1H), 7.30 (dd, J = 7.8, 1.0 Hz, 1H), 7.21 (dd, J = 7.8, 7.8 Hz, 1H), 6.73 (brd, J = 7.8 Hz, 1H), 4.11 (brs, 1H), 3.42-3.41 (m, 2H), 1.39 (t, J = 7.3 Hz, 3H).

Dichloromethane (25.0 mL) was added to the N-ethyldibenzofuran-4-amine (516 mg, 2.44 mmol) and 4-dimethylaminopyridine (30.8 mg, 252 µmol). After cooling to 0°C, 1-(3-dimethylaminopropyl)-3-ethylcarbodiimide hydrochloride (545 mg, 2.84 mmol) was added, and the resulting mixture was stirred for 5 hours at room temperature. The reaction solution was washed with 1 M hydrochloric acid (5 mL × 2) and a saturated aqueous solution of sodium hydrogencarbonate (5 mL), dried with sodium sulfate and then concentrated under reduced pressure to give a light yellow solid of 2-cyano-N-(dibenzofuran-4-yl)-N-methylacetamide (actual yield, 743 mg; percent yield, quant.). This crude product was used in the next reaction directly, without further purification. ¹H-NMR (400 MHz, CDCl₃) δ (ppm): 8.02 (dd, J = 7.8, 1.2 Hz, 1H), 8.00 (brd, J = 7.8 Hz, 1H), 7.63 (brd, J = 8.2 Hz, 1H), 7.55 (ddd, J = 8.2, 7.2, 1.4 Hz, 1H), 7.44 (brd, J = 7.8, 7.8, 1H), 7.43 (m, 1H), 7.32 (dd, J = 7.8, 1.2 Hz, 1H), 4.00-3.89 (m, 2H), 3.25 (s, 2H), 1.13 (t, J = 7.3 Hz, 3H).

N,N-dimethylformamide (13.0 mL) was added to the 2-cyano-N-(dibenzofuran-4-yl)-N-methylacetamide obtained in the above. Trifluoroacetic anhydride (1.05 mL, 7.50 mmol) was added to this, and the resulting mixture was stirred for 24 hours at 80°C. The reaction solution was put into water with ice (50 mL), and the resulting mixture was stirred for 30 minutes at room temperature. The precipitated solid was collected by filtration, washed with water and then dried for 3 hours at 60°C under reduced pressure. The resulting crude product was washed with acetonitrile, ethanol and diethyl ether to give a yellow solid of 1-ethyl-4-hydroxy-3-(2,2,2-trifluoroethan-1-on-1-yl)benzofuro[3,2-h]quinolin-2-one (actual yield, 122 mg; percent yield, 130). ¹H-NMR (400 MHz, CDCl₃) δ (ppm): 14.44 (brs, 1H), 8.24 (d, J = 8.4 Hz, 1H), 8.07 (brd, J = 8.0 Hz, 1H), 7.83 (d, J = 8.4 Hz, 1H), 7.70 (brd, J = 8.4 Hz, 1H), 7.64 (ddd, J = 8.4, 7.0, 1.2 Hz, 1H), 7.48 (ddd, J = 7.8, 7.0, 1.1 Hz, 1H), 4.90 (q, J = 7.3 Hz, 2H), 1.62 (t, J = 7.3 Hz, 3H). ¹⁹F-NMR (376 MHz, CDCl₃) δ (ppm): -73.9 (s).

### (REFERENCE EXAMPLE 38)

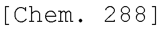

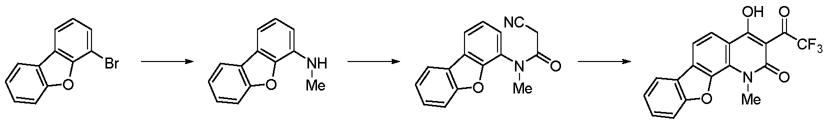

Toluene (30.0 mL) was put into 4-bromodibenzofuran (2.47 g, 10.0 mmol) and sodium t-butoxide (1.44 g, 15.0 mmol), and a 2.0 M solution of methylamine in tetrahydrofuran (7.50 mL, 15.0 mmol) was added to this. After 5 minutes of stirring at room temperature, a degassing operation was performed. 2-dicyclohexylphosphino-2',4',6'-triisopropylbiphenyl (477 mg, 1.00 mmol) and tris(dibenzylideneacetone)dipalladium(0) (231 mg, 252 µmol) were put into this, and the resulting mixture was stirred for 13 hours at 80°C. After natural cooling to room temperature, the mixture was cooled to 0°C, water (30 mL) was added, and extraction with ethyl acetate (30 mL) was performed three times. The organic layers were combined, and the combined liquid was washed with water (30 mL) and a saturated saline solution (30 mL), then dried with magnesium sulfate and concentrated under reduced pressure. The resulting crude product was purified with a silica gel column chromatograph (eluent: hexane/chloroform) to give a colorless oily substance, N-methyl-4-dibenzofuranamine (actual yield, 1.53 g; percent yield, 720). ¹H-NMR (400 MHz, CDCl₃) δ (ppm): 7.92 (brd, J = 7.8 Hz, 1H), 7.55 (brd, J = 8.2 Hz, 1H), 7.42 (ddd, J = 8.2, 7.8, 1.3 Hz, 1H), 7.33 (dd, J = 7.8, 0.9 Hz, 1H), 7.31 (dd, J = 7.8, 0.9 Hz, 1H), 7.23 (d, J = 7.8 Hz, 1H), 6.72 (brd, J = 7.8 Hz, 1H), 4.30 (brs, 1H), 3.04 (s, 3H).

Dichloromethane (87.0 mL) was added to the N-methyl-4-dibenzofuranamine (1.72 g, 8.72 mmol), cyanoacetic acid (781 mg, 9.18 mmol) and 4-dimethylaminopyridine (107 mg, 876 µmol). After cooling to 0°C, 1-(3-dimethylaminopropyl)-3-ethylcarbodiimide hydrochloride (1.87 g, 9.75 mmol) was added, and the resulting mixture was stirred for 4 hours at room temperature. The reaction solution was washed with 1 M hydrochloric acid (15 mL × 2) and a saturated aqueous solution of sodium hydrogencarbonate (15 mL), dried with sodium sulfate and then concentrated under reduced pressure to give a light yellow solid of 2-cyano-N-(dibenzofuran-4-yl)-N-methylacetamide (actual yield, 2.42 g; percent yield, quant.). This crude product was used in the next reaction directly, without further purification. ¹H-NMR (400 MHz, CDCl₃) δ (ppm): 8.01 (dd, J = 7.6, 1.0 Hz, 1H), 7.99 (brd, J = 7.6 Hz, 1H), 7.63 (brd, J = 8.3 Hz, 1H), 7.55 (brdd, J = 8.3, 7.6 Hz, 1H), 7.46-7.40 (m, 2H), 7.35 (dd, J = 7.8, 1.0 Hz, 1H), 3.46 (s, 3H), 3.30 (s, 2H).

1,3-dimethyl-2-imidazolidinone (43.5 mL) was added to the 2-cyano-N-(dibenzofuran-4-yl)-N-methylacetamide obtained in the above. Trifluoroacetic anhydride (3.66 mL, 26.1 mmol) was added dropwise to this over 1 hour, and the resulting mixture was stirred for 24 hours at 80°C. The reaction solution was put into water with ice (150 mL), and the resulting mixture was stirred for 30 minutes at room temperature. The precipitated solid was collected by filtration, washed with water and then dried for 8 hours at 60°C under reduced pressure. The resulting crude product was washed with acetonitrile to give a yellow solid of 1-methyl-4-hydroxy-3-(2,2,2-trifluoroethan-1-on-1-yl)benzofuro[3,2-h]quinolin-2-one (actual yield, 437 mg; percent yield, 14%). ¹H-NMR (400 MHz, CDCl₃) δ (ppm): 14.57 (brs, 1H), 8.22 (d, J = 8.4 Hz, 1H), 8.06 (brd, J = 7.8 Hz, 1H), 7.82 (d, J = 8.4 Hz, 1H), 7.69 (brd, J = 8.4 Hz, 1H), 7.63 (ddd, J = 8.4, 7.1, 1.0 Hz, 1H), 7.47 (ddd, J = 7.8, 7.1, 1.0 Hz, 1H), 4.24 (s, 3H). ¹⁹F-NMR (376 MHz, CDCl₃) δ (ppm): -73.9 (s).

### (REFERENCE EXAMPLE 39)

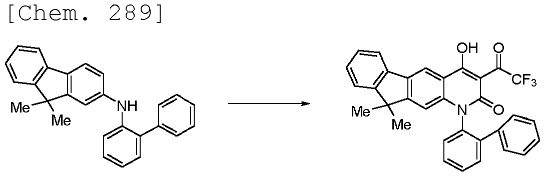

N,N-dimethylformamide (16.6 mL) was added to 2-(2-biphenylyl)amino-9,9-dimethylfluorene (1.20 g, 3.32 mmol) and cyanoacetic acid (290 mg, 3.41 mmol), and the reaction vessel was cooled in a water bath. Trifluoroacetic anhydride (1.86 mL, 13.3 mmol) was added dropwise to this over 2 hours, and the resulting mixture was stirred for 12 hours at room temperature. After the reaction solution was cooled to 0°C, water (80 mL) was added, and the solid that formed was collected by filtration. The solid was washed with water and then dried for 30 minutes at 60°C under reduced pressure. N,N-dimethylformamide (16.6 mL) and trifluoroacetic anhydride (1.86 mL, 13.3 mmol) were put into the resulting solid, and the resulting mixture was heated and stirred for 8 hours at 80°C. Water (120 mL) was added to the reaction solution, and the precipitated solid was collected by suction filtration and dried by heating for 4 hours at 60°C under reduced pressure. The resulting solid was washed with diethyl ether and then purified with a silica gel column chromatograph (eluent: hexane/ethyl acetate) to give an orange solid of 1-(2-biphenylyl)-4-hydroxy-10,10-dimethyl-3-(2,2,2-trifluoroethan-1-on-1-yl)-10H-indeno[3,2-g]quinolin-2(1H)-one (actual yield, 1.06 g; percent yield, 61%) . ¹H-NMR (400 MHz, CDCl₃) δ (ppm) : 14.51 (brs, 1H), 8.33 (brs, 1H), 7.73 (m, 1H), 7.67-7.56 (m, 3H), 7.41-7.30 (m, 4H), 7.21-7.16 (m, 2H), 7.13-7.07 (m, 2H), 6.48 (s, 1H), 1.34 (s, 3H), 1.33 (s, 3H). ¹⁹F-NMR (376 MHz, CDCl₃) δ (ppm) : -73.8 (s).

### (REFERENCE EXAMPLE 40)

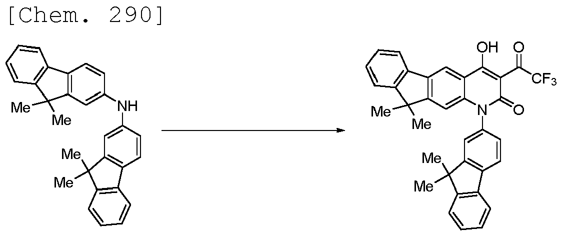

Bis(9,9-dimethyl-9H-fluoren-2-yl)amine (1.13 g, 2.82 mmol) and cyanoacetic acid (245 mg, 2.88 mmol) were put into N,N-dimethylformamide (14.0 mL). In a water bath, trifluoroacetic anhydride (1.58 mL, 11.3 mmol) was added dropwise, and then the resulting mixture was stirred for 13 hours at room temperature. Water (70 mL) was added to the reaction solution, and the precipitated solid was collected by suction filtration and dried by heating for 30 minutes at 60°C under reduced pressure. The resulting solid was washed with acetonitrile and diethyl ether and dried by heating for 30 minutes at 60°C under reduced pressure. N,N-dimethylformamide (14.0 mL) and trifluoroacetic anhydride (1.58 mL, 11.3 mmol) were put into the resulting solid, and the resulting mixture was heated and stirred for 8 hours at 80°C. Water (120 mL) was added to the reaction solution, and the precipitated solid was collected by suction filtration and dried by heating for 4 hours at 60°C in a vacuum. The resulting solid was purified with a silica gel column chromatograph (eluent: hexane/ethyl acetate) to give an orange solid of 1-(9,9-dimethyl-9H-fluoren-2-yl)-4-hydroxy-10,10-dimethyl-3-(2,2,2-trifluoroethyl-1-on-1-yl)-10H-indeno[3,2-g]quinolin-2(1H)-one (actual yield, 855 mg; percent yield, 54%). ¹H-NMR (400 MHz, CDCl₃) δ (ppm): 14.76 (brs, 1H), 8.52 (s, 1H), 7.96 (d, J = 7.9 Hz, 1H), 7.85-7.80 (m, 2H), 7.49 (m, 1H), 7.44-7.30 (m, 6H), 6.61 (s, 1H), 1.55 (s, 3H), 1.52 (s, 3H), 1.30 (s, 3H), 1.28 (s, 3H). ¹⁹F-NMR (376 MHz, CDCl₃) δ (ppm) : -73.8 (s) .

### (REFERENCE EXAMPLE 41)

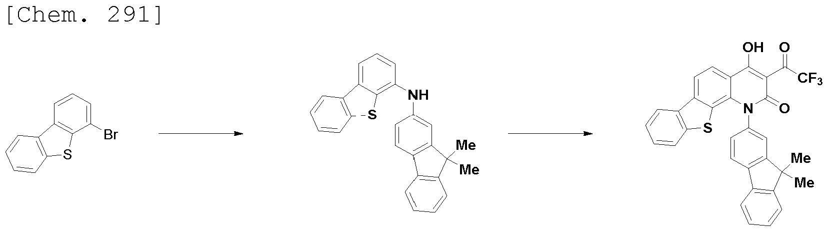

Toluene (33.5 mL) was put into 4-bromodibenzothiophene (2.56 g, 9.74 mmol), 2-amino-9,9-dimethylfluorene (2.43 g, 11.6 mmol) and sodium t-butoxide (1.63 g, 17.0 mmol), and aniline (1.47 mL, 15.3 mmol) was added to this. After a degassing operation was performed, a 1 M solution of tri-t-butylphosphine in hexane (190 µL, 190 µmol) and tris(dibenzylideneacetone)dipalladium(0) (93.5 mg, 102 µmol) were added, and the resulting mixture was stirred for 14 hours at 100°C. After natural cooling to room temperature, water (30 mL) was added, and extraction with ethyl acetate (30 mL) was performed three times. The organic layers were combined, and the combined liquid was washed with water (10 mL) and a saturated saline solution (10 mL), then dried with magnesium sulfate and concentrated under reduced pressure. The resulting crude product was washed with methanol to give a yellow-green solid of N-(9,9-dimethyl-9H-fluoren-2-yl)dibenzo[b,d]thiophen-4-amine (actual yield, 2.21 g; percent yield, 80%). The washing, furthermore, was concentrated under reduced pressure, and the resulting residue was washed with ethanol to give a yellow-green solid of N-(9,9-dimethyl-9H-fluoren-2-yl)dibenzo[b,d]thiophen-4-amine (total actual yield, 3.72 g; percent yield, 98%). ¹H-NMR (400 MHz, CDCl₃) δ (ppm): 8.18 (m, 1H), 7.87 (m, 1H), 7.85 (dd, J = 7.4, 1.4 Hz, 1H), 7.65 (brd, J = 7.4 Hz, 1H), 7.64 (d, J = 8.0 Hz, 1H), 7.52-7.37 (m, 5H), 7.32 (ddd, J = 8.5, 7.4, 1.4 Hz, 1H), 7.26 (m, 1H), 7.11 (brd, 2.1 Hz, 1H), 7.08 (dd, 8.1, 2.1 Hz, 1H), 6.78 (brs, 1H).

The N-(9,9-dimethyl-9H-fluoren-2-yl)dibenzo[b,d]thiophen-4-amine (2.00 g, 5.11 mmol) and cyanoacetic acid (435 mg, 5.11 mmol) were dissolved in acetic anhydride (10.0 mL), and the resulting solution was stirred for 18 hours at 80°C. The reaction mixture was poured into cold water (100 mL), and the precipitated solid was collected by suction filtration. Ethanol was added to the resulting solid, undissolved solids were removed, and then the residue was concentrated under reduced pressure to give a brown solid. The resulting brown solid was dissolved in N,N-dimethylformamide (10.0 mL), trifluoroacetic anhydride (2.90 mL, 20.5 mmol) was added dropwise, and then the resulting mixture was stirred for 18 hours at 40°C. Another portion of trifluoroacetic anhydride (2.90 mL, 20.5 mmol), and the resulting mixture was stirred for 24 hours at 40°C. Water (20.0 mL) was added to the reaction solution, the precipitated solid was collected by suction filtration, and the resulting crude product was purified by silica gel column chromatography (eluent: chloroform/hexane) to give a yellow solid of 1-(9,9-dimethyl-9H-fluoren-2-yl)-4-hydroxy-3-(2,2,2-trifluoroethan-1-on-1-yl)-[1]benzothieno[3,2-h]quinolin-2(1H)-one (actual yield, 232 mg; percent yield, 8%). ¹H-NMR (400 MHz, CDCl₃) δ (ppm): 14.74 (brs, 1H), 8.39 (d, J = 8.6 Hz, 1H), 8.19 (m, 1H), 8.07 (d, J = 8.6 Hz, 1H), 7.94 (d, J = 8.6 Hz, 1H), 7.88 (m, 1H), 7.57-7.50 (m, 2H), 7.48-7.40 (m, 6H), 1.56 (s, 3H), 1.49 (s, 3H). ¹⁹F-NMR (376 MHz, CDCl₃) δ (ppm): -74.0.

### (REFERENCE EXAMPLE 42)

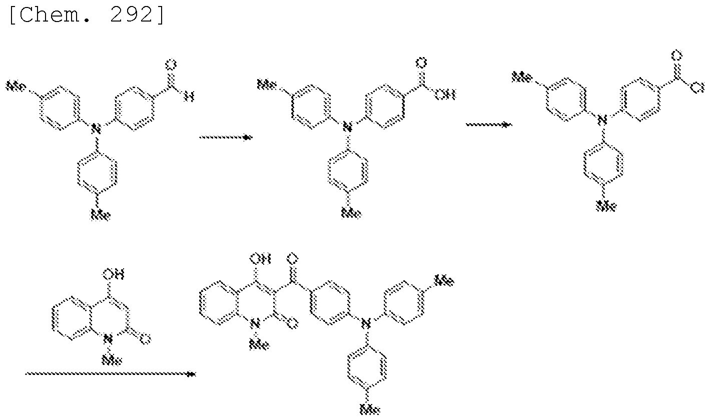

4-(di-p-tolylamino)benzaldehyde (5.37 g, 17.8 mmol) was dissolved in acetone (124 mL) and water (32 mL), and the resulting solution was heated to 60°C. Potassium permanganate (12.9 g, 89.1 mmol) was added gradually, and the resulting mixture was stirred for 3 hours at 60°C. The reaction mixture was concentrated under reduced pressure, water (150 mL) was added, then the resulting mixture was filtered, and 2 M hydrochloric acid (20 mL) was added to the filtrate. The precipitated solid was collected by filtration and dried by heating to give a white solid of 4-(di-p-tolylamino)benzoic acid (actual yield, 4.38 g; percent yield, 78%) . ¹H-NMR (400 MHz, CDCl₃) δ (ppm) : 7.85 (brd, J = 8.8 Hz, 2H), 7.13 (brd, J = 8.8 Hz, 4H), 7.05 (brd, J = 8.8 Hz, 4H), 6.91 (brd, J = 8.8 Hz, 2H), 2.34 (s, 6H).

The 4-(di-p-tolylamino)benzoic acid (952 mg, 3.00 mmol)was dissolved in dichloromethane (15.0 mL). Oxalyl dichloride (386 µL, 4.50 mmol) and N,N-dimethylformamide (10 µL, 130 µmol) were added at 0°C, and the resulting mixture was stirred for 4 hours while the temperature was increased to room temperature. The reaction mixture was concentrated under reduced pressure to give a whitish orange solid of 4-(di-p-tolylamino)benzoyl chloride. This crude product was used in the next reaction without further purification. ¹H-NMR (400 MHz, CDCl₃) δ (ppm): 7.86 (d, J = 9.1 Hz, 2H), 7.16 (d, J = 8.2 Hz, 4H), 7.07 (d, J = 8.2 Hz, 4H), 6.85 (d, J = 9.1 Hz, 2H), 2.35 (s, 6H).

4-hydroxy-1-methyl-2-quinolone (860 mg, 4.91 mmol) was dissolved in dichloromethane (10.4 mL), then the 4-(di-p-tolylamino)benzoyl chloride (1.92 g, 5.40 mmol) dissolved in N,N-diisopropylethylamine (1.00 mL, 5.88 mmol) and dichloromethane (6.00 mL) was added, and the resulting mixture was stirred for 16 hours at room temperature. The reaction solution was diluted with dichloromethane (20.0 mL), and then the diluted solution was washed with 1 M hydrochloric acid (30.0 mL), a saturated aqueous solution of sodium hydrogencarbonate (30 mL) and a saturated saline solution (30.0 mL). The resulting organic layer was dried with magnesium sulfate and then concentrated under reduced pressure to give a brown solid. The resulting crude product was dissolved in acetonitrile (16.4 mL), acetone cyanohydrin (500 µL, 5.46 mmol) and triethylamine (760 µL, 5.45 mmol) were added, and then the resulting mixture was stirred for 19 hours at 50°C. Ethyl acetate (20.0 mL) was added to the reaction mixture, and then the mixture was washed with 1 M hydrochloric acid (30.0 mL) and a saturated saline solution (30.0 mL). The resulting organic layer was dried with magnesium sulfate and then concentrated under reduced pressure. The resulting crude product was washed with methanol and then washed with acetone/hexane = 3/1 for the removal of slurry to give a yellow solid of 3-[4-(di-p-tolylamino)benzoyl]-4-hydroxy-1-methylquinolin-2(1H)-one (actual yield, 1.02 g; percent yield, 440). ¹H-NMR (400 MHz, CDCl₃) δ (ppm): 15.32 (s, 1H), 8.22 (dd, J = 8.3, 1.3 Hz, 1H), 7.68 (ddd, J = 7.1, 7.1, 1.6 Hz, 1H), 7.59 (ddd, J = 9.5, 4.6, 2.6 Hz, 2H), 7.32 (d, J = 8.3 Hz, 1H), 7.28 (dd, J = 7.1, 1.3 Hz, 1H), 7.14-7.06 (m, 8H), 6.89 (ddd, J = 9.5, 4.6, 2.6 Hz, 2H), 3.62 (s, 3H), 2.33 (s, 6H).

### (REFERENCE EXAMPLE 43)

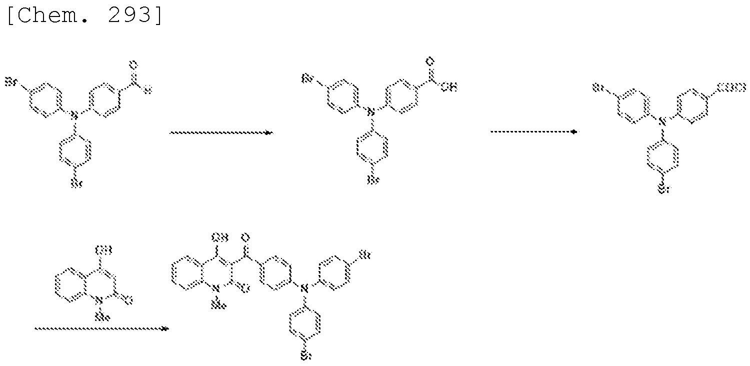

4-[bis(4-bromophenyl)amino]benzaldehyde (1.11 g, 2.57 mmol) was dissolved in acetone (20.5 mL) and water (5.10 mL), and the resulting solution was heated to 60°C. Potassium permanganate (1.83 g, 11.6 mmol) was added gradually, and the resulting mixture was stirred for 3 hours at 60°C. The reaction mixture was concentrated under reduced pressure, water (150 mL) was added, the resulting mixture was filtered, and 1 M hydrochloric acid was added to the filtrate until pH = 3. The precipitated solid was collected by filtration and dried by heating to give a white solid of 4-[bis(4-bromophenyl)amino]benzoic acid (actual yield, 902 mg; percent yield, 79%). ¹H-NMR (400 MHz, CDCl₃) δ (ppm): 7.91 (brd, J = 8.8 Hz, 2H), 7.43 (brd, J = 8.8 Hz, 4H), 7.01 (brd, J = 8.8 Hz, 6H).

The 4-[bis(4-bromophenyl)amino]benzoic acid (902 mg, 2.02 mmol) was dissolved in dichloromethane (10.0 mL). Oxalyl dichloride (260 µL, 3.03 mmol) and N,N-dimethylformamide (2 µL, 25.8 µmol) were added at 0°C, and the resulting mixture was stirred for 4 hours while the temperature was increased to room temperature. The reaction mixture was concentrated under reduced pressure to give a whitish orange solid of 4-[bis(4-bromophenyl)amino]benzoyl chloride. This crude product was used directly in the next reaction without purification. ¹H-NMR (400 MHz, CDCl₃) δ (ppm): 7.93 (d, J = 9.0 Hz, 2H), 7.47 (d, J = 9.0 Hz, 4H), 7.03 (d, J = 9.0 Hz, 4H), 6.96 (d, J = 9.0 Hz, 2H).

4-hydroxy-1-methyl-2-quinolone (353 mg, 2.02 mmol) and the 4-[bis(4-bromophenyl)amino]benzoyl chloride (939 mg, 2.02 mmol) were dissolved in dichloromethane (6.80 mL), then N,N-diisopropylethylamine (412 µL, 2.42 mmol) was added, and the resulting mixture was stirred for 3 hours at room temperature. The reaction solution was diluted with dichloromethane (10.0 mL), and then the diluted solution was washed with 1 M hydrochloric acid (20.0 mL), a saturated aqueous solution of sodium hydrogencarbonate (20 mL) and a saturated saline solution (20.0 mL). The resulting organic layer was dried with magnesium sulfate and then concentrated under reduced pressure to give a brown solid. The resulting crude product was dissolved in acetonitrile (6.80 mL), acetone cyanohydrin (210 µL, 2.29 mmol) and triethylamine (310 µL, 2.22 mmol) were added, and then the resulting mixture was stirred for 20 hours at 60°C. Ethyl acetate (20.0 mL) was added to the reaction mixture, and then the mixture was washed with 1 M hydrochloric acid (30.0 mL) and a saturated saline solution (30.0 mL). The resulting organic layer was dried with magnesium sulfate and then concentrated under reduced pressure. The resulting crude product was washed with methanol to give a yellow solid of 3-{4-[bis(4-bromophenyl)amino]benzoyl}-4-hydroxy-1-methylquinolin-2(1H)-one (actual yield, 395 mg; percent yield, 32%). ¹H-NMR (400 MHz, CDCl₃) δ (ppm): 15.33 (s, 1H), 8.24 (dd, J = 8.0, 1.2 Hz, 1H), 7.71 (ddd, J = 8.7, 7.2, 1.6 Hz, 1H), 7.62 (dd, J = 6.8, 2.0 Hz, 2H), 7.41 (dd, J = 6.8, 2.0 Hz, 4H), 7.33 (d, J = 8.7 Hz, 1H), 7.29 (ddd, J = 8.0 ,7.2, 1.2 Hz, 1H), 7.03 (dd, J = 8.9, 2.0 Hz, 4H), 6.98 (dd, J = 8.9, 2.0 Hz, 2H), 3.62 (s, 3H).

### (REFERENCE EXAMPLE 44)

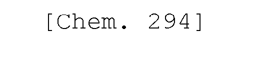

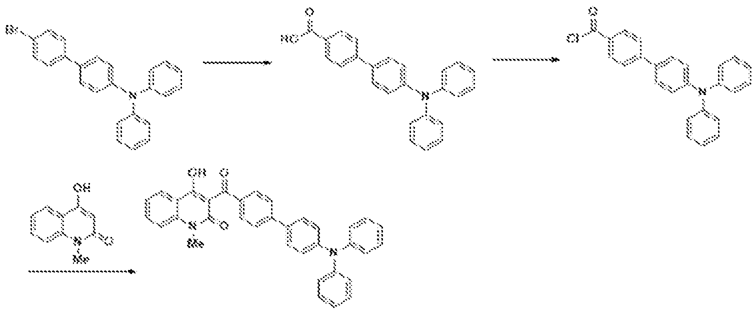

4'-bromo-N,N-diphenyl-[1,1'-biphenyl]-4-amine (1.30 g, 3.25 mmol) was dissolved in tetrahydrofuran (30.0 mL). After cooling to -78°C, a solution of t-butyllithium in pentane (1.52 M, 4.70 mL, 7.15 mmol) was added dropwise, and the resulting mixture was stirred for 30 minutes. Dry ice (1.43 g, 32.5 mmol) was added to the reaction solution, and the resulting mixture was stirred for 5 hours while the temperature was increased to room temperature. The reaction solution was cooled to 0°C, and 2 M hydrochloric acid (5.00 mL) was added. After it was observed that the reaction solution became acidic, ethyl acetate (20.0 mL) and water (20.0 mL) were added, and the aqueous layer was subjected three times to extraction with ethyl acetate. The organic layers were combined, and the combined liquid was dried with sodium sulfate and then concentrated under reduced pressure to give a whitish yellow solid of 4-[4'-(diphenylamino)-[1,1'-biphenyl]]benzoic acid (actual yield, 1.07 g; percent yield, 90%). ¹H-NMR (400 MHz, CDCl₃) δ (ppm): 8.14 (d, J = 8.4 Hz, 2H), 7.67 (d, J = 8.4 Hz, 2H), 7.52 (brd, J = 8.4 Hz, 2H), 7.32-7.26 (m, 4H), 7.17-7.13 (m, 6H), 7.09-7.04 (m, 2H) .

The 4-[4'-(diphenylamino)-[1,1'-biphenyl]]benzoic acid (308 mg, 843 µmol) was dissolved in dichloromethane (4.00 mL). Oxalyl dichloride (110 µL, 1.28 mmol) and N,N-dimethylformamide (10 µL, 129 µmol) were added at 0°C, and the resulting mixture was stirred for 4 hours while the temperature was increased to room temperature. The reaction mixture was concentrated under reduced pressure to give a whitish orange solid of 4-[4'-(diphenylamino)-[1,1'-biphenyl]]benzoyl chloride. This crude product was used in the next reaction without further purification. ¹H-NMR (400 MHz, CDCl₃) δ (ppm): 8.15 (d, J = 8.7 Hz, 2H), 7.69 (d, J = 8.7 Hz, 2H), 7.52 (d, J = 8.7 Hz, 2H), 7.33-7.28 (m, 4H), 7.17-7.12 (m, 6H), 7.11-7.06 (m, 2H).

4-hydroxy-1-methyl-2-quinolone (147 mg, 839 µmol) and the 4'-(diphenylamino)-[1,1'-biphenyl]-4-benzoyl chloride (323 mg, 841 µmol) were dissolved in dichloromethane (4.20 mL), then N,N-diisopropylethylamine (171 µL, 1.01 mmol) was added, and the resulting mixture was stirred for 4 hours at room temperature. The reaction solution was diluted with dichloromethane (10.0 mL), and then the diluted solution was washed with 1 M hydrochloric acid (20.0 mL), a saturated aqueous solution of sodium hydrogencarbonate (20 mL) and a saturated saline solution (20.0 mL). The resulting organic layer was dried with magnesium sulfate and then concentrated under reduced pressure to give a brown solid. The resulting crude product was dissolved in acetonitrile (4.20 mL), acetone cyanohydrin (84.0 µL, 918 µmol) and triethylamine (130 µL, 933 µmol) were added, and the resulting mixture was stirred for 6 hours at 50°C. Ethyl acetate (10.0 mL) was added to the reaction mixture, and then the mixture was washed with 1 M hydrochloric acid (10.0 mL) and a saturated saline solution (10.0 mL). The resulting organic layer was dried with magnesium sulfate and then concentrated under reduced pressure. The resulting crude product was washed with methanol and then reprecipitated with chloroform/hexane, and the resulting solid was washed with a small amount of acetone to give a yellow solid of 3-[4'-(diphenylamino)-[1,1'-biphenyl]-4-carbonyl]-4-hydroxy-1-methylquinolin-2(1H)-one (actual yield, 104 mg; percent yield, 23%). ¹H-NMR (400 MHz, DMSO-d₆) δ (ppm): 11.89 (s, 1H), 8.09 (dd, J = 8.1, 1.4 Hz, 1H), 7.86 (d, J = 8.5 Hz, 2H), 7.78-7.73 (m, 3H), 7.68 (d, J = 8.5 Hz, 2H), 7.59 (d, J = 8.5 Hz, 1H), 7.38-7.32 (m, 5H), 7.13-7.07 (m, 6H), 7.05 (d, J = 8.5 Hz, 2H), 3.57 (s, 3H).

### (REFERENCE EXAMPLE 45)

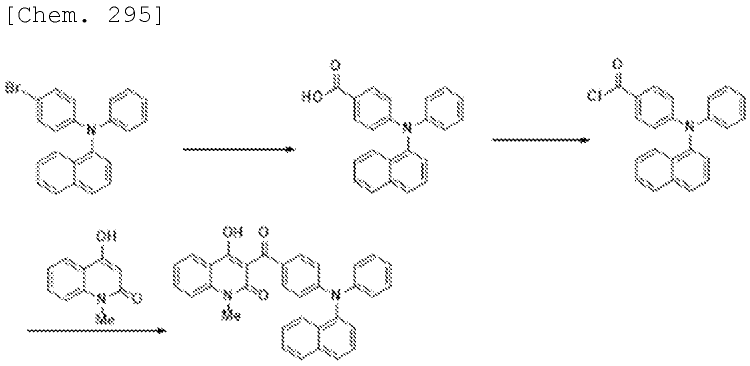

N-(4-bromophenyl)-N-phenyl-1-naphthylamine (2.49 g, 6.66 mmol) was dissolved in tetrahydrofuran (65.0 mL). After cooling to -78°C, a solution of t-butyllithium in pentane (1.62 M, 9.10 mL, 14.7 mmol) was added dropwise, and the resulting mixture was stirred for 30 minutes. Dry ice (3.40 g, 77.3 mmol) was added to the reaction solution, and the resulting mixture was stirred for 5 hours while the temperature was increased to room temperature. The reaction solution was cooled to 0°C, and 1 M hydrochloric acid (20.0 mL). After it was observed that the reaction solution became acidic, ethyl acetate (30.0 mL) and water (60.0 mL) were added, and the aqueous layer was subjected three times to extraction with ethyl acetate. The organic layers were combined, and the combined liquid was dried with sodium sulfate and then concentrated under reduced pressure to give a white solid of 4-[naphthalen-1-yl(phenyl)amino]benzoic acid (actual yield, 2.06 g; percent yield, 910). ¹H-NMR (400 MHz, DMSO-d₆) δ (ppm): 12.43 (s, 1H) 8.04 (d, J = 8.1 Hz, 1H), 7.97 (d, J = 8.1 Hz, 1H), 7.84 (d, J = 8.1 Hz, 1H), 7.74 (d, J = 8.7 Hz, 2H), 7.62 (dd, J = 7.6, 7.6 Hz, 1H), 7.55 (ddd, J = 8.1, 7.0, 1.4 Hz, 1H), 7.51-7.45 (m, 2H), 7.34 (dd, J = 8.7, 7.6 Hz, 2H), 7.20 (dd, J = 8.7, 1.4 Hz, 2H), 7.12 (m, 1H), 6.75 (d, J = 8.7 Hz, 2H).

The 4-[naphthalen-1-yl(phenyl)amino]benzoic acid (2.06 g, 6.06 mmol) was dissolved in dichloromethane (12.0 mL). Oxalyl dichloride (780 µL, 9.09 mmol) and N,N-dimethylformamide (20 µL, 258 µmol) were added at 0°C, and the resulting mixture was stirred for 4 hours while the temperature was increased to room temperature. The reaction mixture was concentrated under reduced pressure to give a green solid of 4-[naphthalen-1-yl(phenyl)amino]benzoyl chloride. This crude product was used in the next reaction without further purification. ¹H-NMR (400 MHz, CDCl₃) δ (ppm): 7.93 (d, J = 8.9 Hz, 1H), 7.89-7.83 (m, 4H), 7.55-7.49 (m, 2H), 7.44 (ddd, J = 8.4, 7.2, 1.3 Hz, 2H), 7.39 (dd, J = 7.3, 1.3 Hz, 1H), 7.36-7.30 (m, 2H), 7.27 (dd, J = 7.3, 1.3 Hz, 1H), 7.16 (m, 1H), 6.79 (d, J = 8.9 Hz, 2H).

4-hydroxy-1-methyl-2-quinolone (1.06 g, 6.06 mmol) and the 4-[naphthalen-1-yl(phenyl)amino]benzoyl chloride (2.17 g, 6.06 mmol) were dissolved in dichloromethane (11.0 mL), then N,N-diisopropylethylamine (1.24 mL, 7.29 mmol) was added, and the resulting mixture was stirred for 3 hours at room temperature. The reaction solution was diluted with dichloromethane (10.0 mL), and then the dilutes solution was washed with 1 M hydrochloric acid (20.0 mL), a saturated aqueous solution of sodium hydrogencarbonate (20 mL) and a saturated saline solution (20.0 mL). The resulting organic layer was dried with magnesium sulfate and then concentrated under reduced pressure to give a brown solid. The resulting crude product was dissolved in acetonitrile (11.0 mL), acetone cyanohydrin (610 µL, 6.67 mmol) and triethylamine (930 µL, 6.67 mol) were added, and then the resulting mixture was stirred for 20 hours at 50°C. Ethyl acetate (10.0 mL) and 1 M hydrochloric acid (20.0 mL) were added to the reaction mixture, and then the precipitated solid was collected by filtration, washed with methanol and then reprecipitated with chloroform/hexane to give a yellow solid of 4-hydroxy-1-methyl-3-{4-[naphthalen-1-yl(phenyl)amino]benzoyl}quinolin-2(1H)-one (actual yield, 1.13 g; percent yield, 38%). ¹H-NMR (400 MHz, DMSO-d₆) δ (ppm): 11.52 (s, 1H), 8.05 (d, J = 8.2 Hz, 1H), 8.03 (dd, J = 8.2, 1.5 Hz, 1H), 7.98 (d, J = 8.2 Hz, 1H), 7.86 (d, J = 8.2 Hz, 1H), 7.70 (ddd, J = 8.6, 7.2, 1.5 Hz, 1H), 7.66-7.59 (m, 3H), 7.59-7.47 (m, 4H), 7.35 (dd, J = 8.6, 7.2 Hz, 2H), 7.30 (ddd, J = 8.2, 7.2, 1.1 Hz, 1H), 7.22 (dd, J = 8.6, 1.1 Hz, 2H), 7.30 (ddd, J = 8.6, 7.2, 1.1 Hz, 1H), 6.73 (d, J = 9.0 Hz, 2H), 3.54 (s, 3H).

### (REFERENCE EXAMPLE 46)

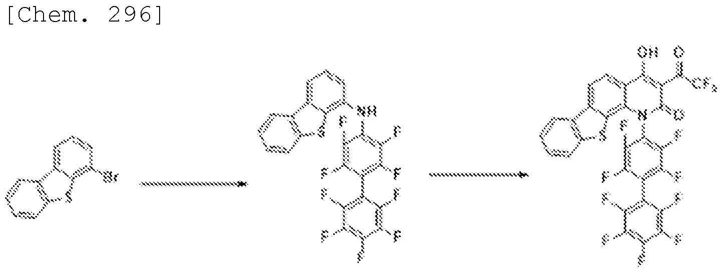

4-aminononafluorobiphenyl (1.82 g, 5.50 mmol), sodium t-butoxide (577 mg, 6.00 mmol), tri-t-butylphosphine (a 1 M solution in hexane) (100 µL, 100 µmol), tris(dibenzylideneacetone)dipalladium(0) (24.0 mg, 26.2 µmol), and 4-bromodibenzothiophene (1.32 g, 5.00 mmol) were suspended in toluene (5.00 mL), and then the resulting suspension was stirred for 24 hours at 100°C. The reaction mixture was diluted with ethyl acetate (20.0 mL) and then filtered through Celite, the residue was washed with ethyl acetate, and then the filtrate was concentrated under reduced pressure. The resulting solid was washed with methanol to give a white solid of N-(perfluoro-[1,1'-biphenyl]-4-yl)dibenzo[b,d]thiophen-4-amine (actual yield, 1.82 g; percent yield, 71%). ¹H-NMR (400 MHz, CDCl₃) δ (ppm): 8.19 (dd, J = 6.0, 3.3 Hz, 1H), 7.99 (brd, J = 7.8 Hz, 1H), 7.91 (dd, J = 6.0, 3.3 Hz, 1H), 7.53-7.49 (m, 2H), 7.47 (dd, J = 7.8, 7.8 Hz, 1H), 7.14 (brd, J = 7.8 Hz, 1H), 5.65 (s, 1H).

The N-(perfluoro-[1,1'-biphenyl]-4-yl)dibenzo[b,d]thiophen-4-amine (191 mg, 372 µmol) and cyanoacetic acid (32.2 mg, 379 µmol) were dissolved in N,N-dimethylformamide (700 µL), trifluoroacetic anhydride (210 µL, 1.50 mmol) was added dropwise, and then the resulting mixture was stirred for 17 hours at room temperature. Water (20 mL) was added to the reaction solution, the precipitated solid was collected by suction filtration, and the resulting solid was recrystallized in chloroform/hexane to give a yellow solid of 4-hydroxy-1-(perfluoro-[1,1'-biphenyl]-4-yl)-3-(2,2,2-trifluoroethan-1-on-1-yl)-[1]benzothieno[3,2-h]quinolin-2(1H)-one (actual yield, 18.7 mg; percent yield, 8%). ¹H-NMR (400 MHz, CDCl₃) δ (ppm): 14.96 (brs, 1H), 8.45 (d, J = 8.6 Hz, 1H), 8.26 (dd, J = 7.1, 2.5 Hz, 1H), 8.18 (d, J = 8.6 Hz, 1H), 7.76 (ddd, J = 6.5, 1.6 Hz, 1H), 7.62-7.54 (m, 2H). ¹⁹F-NMR (376 MHz, CDCl₃) δ (ppm): -74.3 (m, 3F), -136.2 to -135.8 (m, 2F), 136.2 (m, 1F), -141.4 to - 141.6 (m, 2F), -149.0 (m, 1F), -159.5 (ddd, J = 21.3, 21.3, 7.9 Hz, 1F), -160.2 (ddd, J = 21.3, 21.3, 7.9 Hz, 1F).

### (REFERENCE EXAMPLE 47)

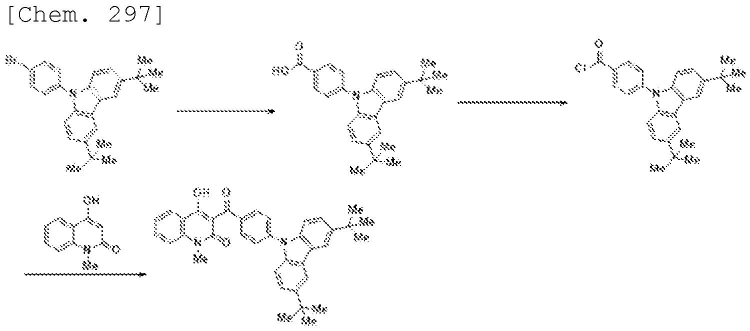

9-(4-bromophenyl)-3,6-di-tert-butyl-9H-carbazole (2.52 g, 5.81 mmol) was dissolved in tetrahydrofuran (60.0 mL). After cooling to -78°C, a solution of t-butyllithium in pentane (1.62 M, 7.90 mL, 12.8 mmol) was added dropwise, and the resulting mixture was stirred for 1 hour. Dry ice (2.87 g, 65.2 mmol) was added to the reaction solution, and the resulting mixture was stirred for 16 hours while the temperature was increased to room temperature. The reaction solution was cooled to 0°C, and 1 M hydrochloric acid (20.0 mL) was added. After it was observed that the reaction solution became acidic, ethyl acetate (30.0 mL) and water (60.0 mL) were added, and the aqueous layer was subjected three times to extraction with ethyl acetate. The organic layers were combined, the combined liquid was dried with sodium sulfate and then concentrated under reduced pressure, and the residue was recrystallized in chloroform/hexane to give a white solid of 4-(3,6-di-tert-butyl-9H-carbazol-9-yl)benzoic acid (actual yield, 1.68 g; percent yield, 85%). ¹H-NMR (400 MHz, CDCl₃) δ (ppm): 8.33 (d, J = 8.6 Hz, 2H), 8.14 (d, J = 1.7 Hz, 2H), 7.72 (d, J = 8.6 Hz, 2H), 7.49 (dd, J = 8.6, 1.7 Hz, 2H), 7.45 (d, J = 8.6 Hz, 2H), 1.47 (s, 18H).

The 4-(3,6-di-tert-butyl-9H-carbazol-9-yl)benzoic acid (1.68 g, 4.94 mmol) was dissolved in dichloromethane (10.0 mL). Oxalyl dichloride (640 µL, 7.46 mmol) and N,N-dimethylformamide (10 µL, 129 µmol) were added at 0°C, and the resulting mixture was stirred for 3 hours while the temperature was increased to room temperature. The reaction mixture was concentrated under reduced pressure to give a green solid of 4-(3,6-di-tert-butyl-9H-carbazol-9-yl)benzoyl chloride. This crude product was used in the next reaction without further purification. ¹H-NMR (400 MHz, CDCl₃) δ (ppm): 8.35 (d, J = 8.8 Hz, 2H), 8.13 (dd, J = 1.7, 0.8 Hz, 2H), 7.78 (d, J = 8.8 Hz, 2H), 7.49-7.47 (m, 4H), 1.47 (s, 18H) .

4-hydroxy-1-methyl-2-quinolone (867 mg, 4.95 mmol) and the 4-(3,6-di-tert-butyl-9H-carbazol-9-yl)benzoyl chloride (2.06 g, 4.97 mmol) were dissolved in dichloromethane (10.0 mL), then N,N-diisopropylethylamine (1.00 mL, 5.88 mmol) was added, and the resulting mixture was stirred for 3 hours at room temperature. The reaction solution was diluted with dichloromethane (10.0 mL), and then the diluted solution was washed with 1 M hydrochloric acid (20.0 mL), a saturated aqueous solution of sodium hydrogencarbonate (20 mL) and a saturated saline solution (20.0 mL). The resulting organic layer was dried with magnesium sulfate and then concentrated under reduced pressure to give a brown solid. The resulting crude product was dissolved in acetonitrile (10.0 mL), acetone cyanohydrin (500 µL, 5.46 mmol) and triethylamine (760 µL, 5.45 mol) were added, and then the resulting mixture was stirred for 21 hours at 50°C. Ethyl acetate (20.0 mL) was added to the reaction mixture, the mixture was washed with 1 M hydrochloric acid (20.0 mL) and a saturated saline solution (20.0 mL), and then the organic layer was dried with magnesium sulfate and concentrated under reduced pressure. The resulting crude product was purified by silica gel column chromatography (eluent: hexane/ethyl acetate) to give a yellow solid of 3-[4-(3,6-di-tert-butyl-9H-carbazol-9-yl)benzoyl]-4-hydroxy-1-methylquinolin-2(1H)-one (actual yield, 872 mg; percent yield, 32%). ¹H-NMR (400 MHz, CDCl₃) δ (ppm): 15.43 (s, 1H), 8.30 (dd, J = 8.0, 1.5 Hz, 1H), 8.33 (d, J = 1.7 Hz, 2H), 7.91 (d, J = 8.6 Hz, 2H), 7.75 (ddd, J = 8.6, 7.6, 1.7 Hz, 1H), 7.66 (d, J = 8.6 Hz, 2H), 7.52-7.45 (m, 4H), 7.37 (d, J = 8.6 Hz, 1H), 7.33 (ddd, J = 8.0, 7.2, 1.0 Hz, 1H), 3.66 (s, 3H), 1.47 (s, 18H).

### (REFERENCE EXAMPLE 48)

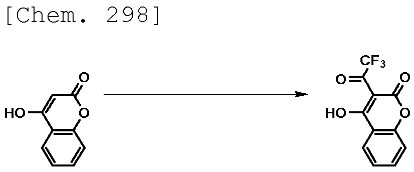

1-(trifluoroacetyl)imidazole (5.00 g, 30.5 mmol) was added to a suspension of 4-hydroxycoumarin (4.05 g, 25.0 mmol) and imidazole (2.04 g, 30.0 mmol) in chloroform (20.0 mL), and the resulting mixture was stirred for 2 hours at room temperature. Water was added to the reaction solution, and separation was performed. The aqueous layer was acidified (pH = 3) with 1 M hydrochloric acid, and the precipitated solid was collected by suction filtration and dried under reduced pressure to give a white solid of 4-hydroxy-3-(2,2,2-trifluoroethan-1-on-1-yl)-2H-chromen-2-one (actual yield, 5.20 g; percent yield, 81%). ¹H-NMR (400 MHz, CDCl₃) δ (ppm): 15.04 (brs, 1H), 8.11 (dd, J = 8.1, 1.6 Hz, 1H), 7.80 (ddd, J = 8.5, 7.3, 1.6 Hz, 1H), 7.41 (ddd, J = 8.1, 7.3, 1.0 Hz, 1H), 7.36 (dd, J = 8.5, 1.0 Hz, 1H). ¹⁹F-NMR (376 MHz, CDCl₃) δ (ppm) : -74.6 (s, 3F).

### (REFERENCE EXAMPLE 49)

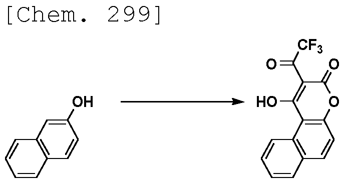

1,3-dimethyl-2-imidazolidinone (20.0 mL) was added to 2-naphthol (2.88 g, 20.0 mmol) and cyanoacetic acid (1.70 g, 20.0 mmol). Trifluoroacetic anhydride (11.3 mL, 80.7 mmol) was added dropwise over 2 hours in a water bath at room temperature, and the resulting mixture was further stirred for 22 hours. The reaction solution was added to water, and the precipitated solid was collected by suction filtration, washed with water, washed with methanol and vacuum-dried to give 1-hydroxy-2-(2,2,2-trifluoroethan-1-on-1-yl)-3H-naphtho[2,1-b]pyran-3-one as a yellow solid (actual yield, 2.72 g; percent yield, 44%). ¹H-NMR (400 MHz, CDCl₃) δ (ppm): 16.50 (brs, 1H), 9.25 (brd, J = 8.8 Hz, 1H), 8.23 (d, J = 8.8 Hz, 1H), 7.95 (brd, J = 8.0 Hz, 1H), 7.80 (ddd, J = 8.8, 7.2, 1.6 Hz, 1H), 7.65 (ddd, J = 8.0, 7.2, 0.9 Hz, 1H), 7.43 (d, J = 8.8 Hz, 1H). ¹⁹F-NMR (376 MHz, CDCl₃) δ (ppm): -74.2 (s, 3F).

### (REFERENCE EXAMPLE 50)

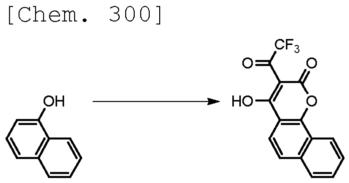

1,3-dimethyl-2-imidazolidinone (20.0 mL) was added to 1-naphthol (2.88 g, 20.0 mmol) and cyanoacetic acid (1.70 g, 20.0 mmol). Trifluoroacetic anhydride (11.3 mL, 80.7 mmol) was added dropwise over 2 hours in a water bath at room temperature, and the resulting mixture was further stirred for 8 hours. The reaction solution was added to water, and the precipitated solid was collected by suction filtration, washed with water, washed with acetonitrile and vacuum-dried to give 4-hydroxy-3-(2,2,2-trifluoroethan-1-on-1-yl)-2H-naphtho[1,2-b]pyran-2-one as a light yellow solid (actual yield, 1.98 g; percent yield, 32%). ¹H-NMR (400 MHz, CDCl₃) δ (ppm): 15.12 (brs, 1H), 8.57 (brd, J = 8.2 Hz, 1H), 7.95 (d, J = 8.8 Hz, 1H), 7.92 (brd, J = 8.2 Hz, 1H), 7.79 (ddd, J = 8.2, 7.0, 1.2 Hz, 1H), 7.75 (d, J = 8.8 Hz, 1H), 7.71 (ddd, J = 8.2, 7.0, 1.2 Hz, 1H). ¹⁹F-NMR (376 MHz, CDCl₃) δ (ppm): -74.7 (s, 3F).

### (REFERENCE EXAMPLE 51)

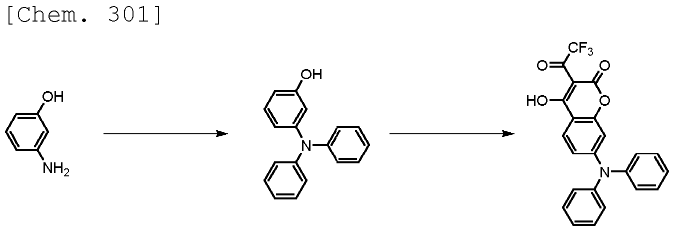

Toluene (60.0 mL) was added to a mixture of 3-aminophenol (2.18 g, 20.0 mmol), tri-t-butylphosphonium tetrafluoroborate (116 mg, 400 µmol), the chloroform adduct of tris(dibenzylideneacetone)dipalladium(0) (104 mg, 100 µmol) and sodium-tert-butoxide (7.69 g, 80.0 mmol), and the temperature was increased to 80°C. Bromobenzene (4.41 mL, 42.1 mmol) was added dropwise, and the resulting mixture was stirred for 4 hours at the same temperature. After natural cooling, undissolved solids were removed from the reaction solution by filtration, the residue was washed with hexane, and the filtrate was concentrated under reduced pressure. The residue was neutralized by adding 1 M hydrochloric acid to it, the aqueous layer was removed, and then the residual viscous substance was washed with water. This viscous substance was vacuum-dried and then allowed to stand overnight, and the resulting solid was suspended in hexane. A small amount of methanol was added, clumps were ground by sonication, and then the solids were collected by suction filtration and vacuum-dried to give a light gray solid of 3-(diphenylamino)phenol (actual yield, 5.21 g; percent yield, quant.). ¹H-NMR (400 MHz, CDCl₃) δ (ppm): 7.28-7.22 (m, 4H), 7.12-7.06 (m, 5H), 7.02 (tt, J = 7.4, 1.1 Hz, 2H), 6.63 (ddd, J = 8.0, 2.2, 0.8 Hz, 1H), 6.52 (dd, J = 2.2, 2.2 Hz, 1H), 6.46 (ddd, J = 8.0, 2.2, 0.8 Hz, 1H), 4.55 (s, 1H).

1,3-dimethyl-2-imidazolidinone (10.0 mL) was added to the 3-(diphenylamino)phenol (2.61 g, 9.99 mmol) and cyanoacetic acid (850 mg, 9.99 mmol). Trifluoroacetic anhydride (5.64 mL, 40.3 mmol) was added dropwise over 2 hours in a water bath at room temperature, and the resulting mixture was further stirred for 8 hours. The reaction solution was added to water, and the precipitated solid was collected by suction filtration, washed with water and dissolved in ethyl acetate. The organic layer was washed with water and then dried with anhydrous magnesium sulfate and concentrated under reduced pressure. The residue was suspended in hexane, a small amount of methanol was added, and undissolved solids were collected by suction filtration, washed with methanol and then with hexane and vacuum-dried to give 7-(diphenylamino)-4-hydroxy-3-(2,2,2-trifluoroethan-1-on-1-yl)-2H-chromen-2-one as a yellow-orange solid (actual yield, 1.56 g; percent yield, 37%). ¹H-NMR (400 MHz, CDCl₃) δ (ppm): 15.05 (brs, 1H), 7.78 (d, J = 9.0 Hz, 1H), 7.44-7.39 (m, 4H), 7.29 (tt, J = 7.5, 1.2 Hz, 2H), 7.24-7.21 (m, 4H), 6.81 (dd, J = 9.0, 2.3 Hz, 1H), 6.60 (d, J = 2.3 Hz, 1H) . ¹⁹F-NMR (376 MHz, CDCl₃) δ (ppm) : -74.3 (s, 3F).

### (REFERENCE EXAMPLE 52)

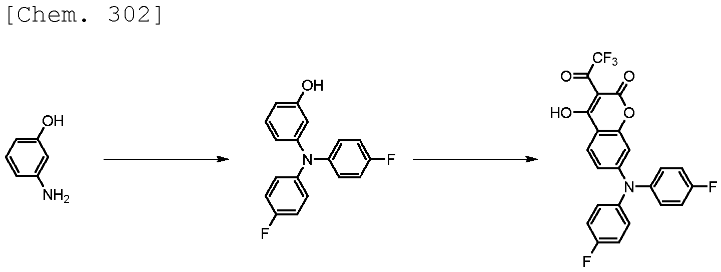

Toluene (60.0 mL) was added to a mixture of 3-aminophenol (4.36 g, 40.0 mmol), tri-t-butylphosphonium tetrafluoroborate (232 mg, 800 µmol), the chloroform adduct of tris(dibenzylideneacetone)dipalladium(0) (208 mg, 201 µmol) and sodium-tert-butoxide (15.4 g, 160 mmol), and the temperature was increased to 80°C. 4-bromofluorobenzene (9.17 mL, 80.1 mmol) was added dropwise, and the resulting mixture was stirred for 4 hours at the same temperature. After natural cooling, undissolved solids were removed from the reaction solution by filtration, the residue was washed with hexane, and the filtrate was concentrated under reduced pressure. The residue was neutralized by adding 1 M hydrochloric acid to it, the aqueous layer was removed, and then the residual viscous substance was washed with water. After transfer of the vessel using ethyl acetate, vacuum drying was performed to give 3-[bis(4-fluorophenyl)amino]phenol as a crude product. This crude product was used in the next reaction directly, without further purification. ¹H-NMR (400 MHz, CDCl₃) δ (ppm): 7.08-7.01 (m, 5H), 6.99-6.92 (m, 4H), 6.52 (m, 1H), 6.44-6.41 (m, 2H), 4.96 (brs, 1H). ¹⁹F-NMR (376 MHz, CDCl₃) δ (ppm): - 119.3 (s, 2F).

1,3-dimethyl-2-imidazolidinone (40.0 mL) was added to the crude product 3-[bis(4-fluorophenyl)amino]phenol and cyanoacetic acid (3.40 g, 40.0 mmol). Trifluoroacetic anhydride (22.6 mL, 161 mmol) was added dropwise over 2 hours in a water bath at room temperature, and the resulting mixture was further stirred for 12 hours. The reaction solution was added to water, and the precipitated solid was collected by suction filtration, washed with methanol and vacuum-dried to give 7-[bis(4-fluorophenyl)amino]-4-hydroxy-3-(2,2,2-trifluoroethan-1-on-1-yl)-2H-chromen-2-one as a yellow-orange solid (actual yield, 7.62 g; total percent yield in the two steps, 41%). ¹H-NMR (400 MHz, CDCl₃) δ (ppm): 15.06 (s, 1H), 7.80 (d, J = 9.1 Hz, 1H), 7.23-7.18 (m, 4H), 7.16-7.10 (m, 4H), 6.73 (dd, J = 9.1, 2.3 Hz, 1H), 6.53 (d, J = 2.3 Hz, 1H). ¹⁹F-NMR (376 MHz, CDCl₃) δ (ppm): -74.4 (s, 3F), -113.6 (s, 2F).

### (REFERENCE EXAMPLE 53)

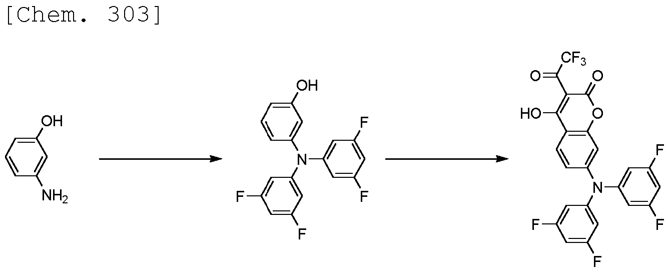

Toluene (60.0 mL) was added to a mixture of 3-aminophenol (2.18 g, 20.0 mmol), tri-t-butylphosphonium tetrafluoroborate (116 mg, 400 µmol), the chloroform adduct of tris(dibenzylideneacetone)dipalladium(0) (104 mg, 100 µmol) and sodium-tert-butoxide (7.69 g, 80.0 mmol), and the temperature was increased to 80°C. 1-bromo-3,5-difluorobenzene (4.81 mL, 37.4 mmol) was added dropwise, and the resulting mixture was stirred for 4 hours. After natural cooling, undissolved solids were removed from the reaction solution by filtration, the residue was washed with hexane, and the filtrate was concentrated under reduced pressure. The residue was neutralized by adding 1 M hydrochloric acid to it, the aqueous layer was removed, and then the residual viscous substance was washed with water. This viscous substance was vacuum-dried and then allowed to stand overnight, and the resulting solid was suspended in hexane. A small amount of methanol was added, clumps were ground by sonication, and then the solids were collected by suction filtration and vacuum-dried to give a light gray solid of 3-[bis(3,5-difluorophenyl)amino]phenol (actual yield, 6.44 g; percent yield, 97%). ¹H-NMR (400 MHz, CDCl₃) δ (ppm): 7.21 (dd, J = 8.1, 8.1 Hz, 1H), 6.69 (ddd, J = 8.2, 2.0, 0.8 Hz, 1H), 6.66 (ddd, J = 8.2, 2.4, 0.8 Hz, 1H), 6.60-6.52 (m, 5H), 6.49 (tt, J = 8.7, 2.3 Hz, 2H), 4.73 (s, 1H) . ¹⁹F-NMR (376 MHz, CDCl₃) δ (ppm): -108.8 (s, 4F) .

1,3-dimethyl-2-imidazolidinone (10.0 mL) was added to the 3-[bis(3,5-difluorophenyl)amino]phenol (3.33 g, 9.99 mmol) and cyanoacetic acid (850 mg, 9.99 mmol). Trifluoroacetic anhydride (5.64 mL, 40.3 mmol) was added dropwise over 2 hours in a water bath at room temperature, and the resulting mixture was further stirred for 8 hours. The reaction solution was added to water, and the precipitated solid was collected by suction filtration, washed with water. The residue was suspended in hexane, a small amount of methanol was added, and undissolved solids were collected by suction filtration, washed with methanol and then with hexane and vacuum-dried to give 7-[bis(3,5-difluorophenyl)amino]-4-hydroxy-3-(2,2,2-trifluoroethan-1-on-1-yl)-2H-chromen-2-one as a yellow solid (actual yield, 1.72 g; percent yield, 35%). ¹H-NMR (400 MHz, CDCl₃) δ (ppm): 15.06 (brs, 1H), 7.93 (d, J = 8.9 Hz, 1H), 6.95 (dd, J = 8.9, 2.3 Hz, 1H), 6.80 (d, J = 2.3 Hz, 1H), 6.76 (tt, J = 8.6, 2.2 Hz, 2H), 6.73-6.67 (m, 4H). ¹⁹F-NMR (376 MHz, CDCl₃) δ (ppm): -74.5 (s, 3F), -106.4 (s, 4F) .

### (REFERENCE EXAMPLE 54)

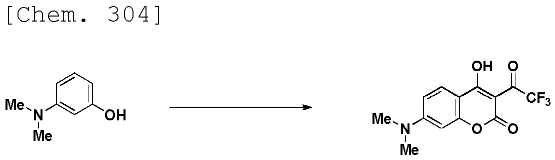

3-(dimethylamino)phenol (2.74 g, 20.0 mmol) and cyanoacetic acid (1.70 g, 20.0 mmol) were dissolved in 1,3-dimethyl-2-imidazolidinone (20.0 mL). After the reaction vessel was cooled in a water bath, trifluoroacetic anhydride (8.40 mL, 60.0 mmol) was added dropwise over 1 hour, and then the resulting mixture was stirred for two days at room temperature. Water (100.0 mL) was added to the reaction solution, the precipitated solid was collected by suction filtration and washed with a 50% aqueous solution of ethanol and diethyl ether and then dissolved in chloroform, and the precipitated solid was removed by filtration. The filtrate was concentrated under reduced pressure, and the residue was reprecipitated with chloroform/hexane to give a brown solid of 7-(dimethylamino)-4-hydroxy-3-(2,2,2-trifluoroethyl-1-on-1-yl)-2H-chromen-2-one (actual yield, 468 mg; percent yield, 7.8%). ¹H-NMR (400 MHz, CDCl₃) δ (ppm): 15.08 (brs, 1H), 7.85 (d, J = 9.2 Hz, 1H), 6.66 (dd, J = 9.2, 2.4 Hz, 1H), 6.38 (d, J = 2.4 Hz, 1H), 3.17 (s, 6H), ¹⁹F-NMR (376 MHz, CDCl₃) δ (ppm) : -74.2 (s, 3F) .

### (REFERENCE EXAMPLE 55)

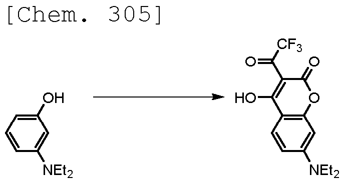

1,3-dimethyl-2-imidazolidinone (20.0 mL) was added to cyanoacetic acid (1.70 g, 20.0 mmol) and 3-(diethylamino)phenol (3.30 g, 20.0 mmol). Trifluoroacetic anhydride (11.3 mL, 80.7 mmol) was added dropwise over 2 hours in a water bath at room temperature, and the resulting mixture was further stirred for 4 hours. The reaction solution was added to water, and the precipitated solid was collected by suction filtration and suspended in a 40% aqueous solution of acetonitrile. Clumps were ground, and the solids were collected by suction filtration. The solids were washed with a 40% aqueous solution of acetonitrile and vacuum-dried to give a yellow-orange solid of 7-(diethylamino)-4-hydroxy-3-(2,2,2-trifluoroethan-1-on-1-yl)-2H-chromen-2-one (actual yield, 1.91 g; percent yield, 29%). ¹H-NMR (400 MHz, CDCl₃) δ (ppm): 15.07 (brs, 1H), 7.82 (d, J = 9.3 Hz, 1H), 6.63 (dd, J = 9.3, 2.2 Hz, 1H), 6.38 (d, J = 2.2 Hz, 1H), 3.48 (q, J = 7.2 Hz, 4H), 1.26 (t, J = 7.2 Hz, 6H) . ¹⁹F-NMR (376 MHz, CDCl₃) δ (ppm) : -74.2 (s, 3F) .

### (REFERENCE EXAMPLE 56)

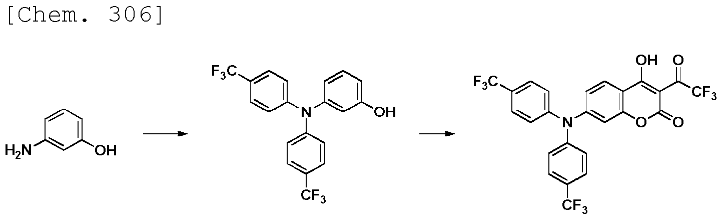

m-aminophenol (109 mg, 999 µmol), sodium-t-butoxide (384 mg, 4.00 mmol), tri-t-butylphosphonium tetrafluoroborate (6.0 mg, 20.7 µmol), tris(dibenzylideneacetone)dipalladium(0) (51.0 mg, 55.7 µmol) and 4-bromobenzotrifluoride (410 µL, 2.97 mmol) were suspended in toluene (3.30 mL), and then the resulting suspension was stirred for 25 minutes at 180°C under microwave irradiation. The reaction mixture was filtered, the residue was washed with hexane, and the filtrate was concentrated under reduced pressure. 2 M hydrochloric acid (10 mL) was added to the resulting crude product, and extraction with chloroform (10 mL × 3) was performed three times. The organic layers were combined, and the combined liquid was dried with magnesium sulfate and then concentrated under reduced pressure. The resulting crude product was purified by silica gel column chromatography (eluent: hexane/ethyl acetate) to give a white solid of 3-{bis[4-(trifluoromethyl)phenyl]amino}phenol (actual yield, 186 mg; percent yield, 47%). ¹H-NMR (400 MHz, CDCl₃) δ (ppm): 7.50 (d, J = 8.5 Hz, 4H), 7.20 (dd, J = 8.0, 8.0 Hz, 1H), 7.15 (d, J = 8.5 Hz, 4H), 6.70 (ddd, J = 8.0, 2.1, 0.9 Hz, 1H), 6.64 (ddd, J = 8.0, 2.5, 0.9 Hz, 1H), 6.59 (dd, J = 2.5, 2.1 Hz, 1H), 4.68 (s, 1H). ¹⁹F-NMR (376 MHz, CDCl₃) δ (ppm) : - 62.0 (s, 6F).

The 3-{bis[4-(trifluoromethyl)phenyl]amino}phenol (641 mg, 1.61 mmol) and cyanoacetic acid (158 mg, 1.86 mmol) were dissolved in N,N-dimethylformamide (3.30 mL). After the reaction vessel was cooled in a water bath, trifluoroacetic anhydride (1.10 mL, 7.86 mmol) was added dropwise over 1 hour, and then the resulting mixture was stirred for 19 hours at room temperature. The reaction solution was put into water, and the precipitated solid was collected by suction filtration and washed with water. The resulting solid was washed with acetonitrile to give an orange solid of 7-{bis[4-(trifluoromethyl)phenyl]amino}-4-hydroxy-3-(2,2,2-trifluoroethyl-1-on-1-yl)-2H-chromen-2-one (actual yield, 227 mg; percent yield, 25%). ¹H-NMR (400 MHz, CDCl₃) δ (ppm): 15.07 (brs, 1H) 7.90 (brd, J = 8.9 Hz, 1H), 7.67 (brd, J = 8.5 Hz, 4H), 7.29 (brd, J = 8.5 Hz, 4H), 6.94 (dd, J = 8.9, 2.3 Hz, 1H), 6.77 (dd, J = 2.3, 2.3 Hz, 1H), ¹⁹F-NMR (376 MHz, CDCl₃) δ (ppm): -62.5 (s, 6F), -74.4 (s, 3F).

### (REFERENCE EXAMPLE 57)

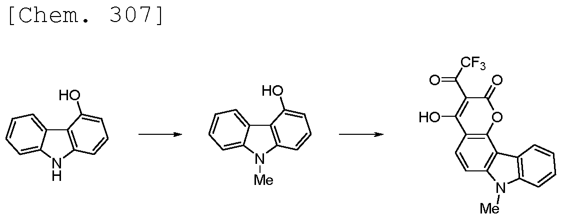

A 55 wt% dispersion of sodium hydride in paraffin liquid (2.74 g, 62.9 mmol) was washed with hexane, and then tetrahydrofuran (41.0 mL) and N,N-dimethylformamide (4.23 mL, 54.6 mmol) were added. After the reaction solution was cooled to 0°C, a suspension of 9H-carbazol-4-ol (5.00 g, 27.3 mmol) in tetrahydrofuran (13.5 mL) was added, and iodomethane (1.87 mL, 30.0 mmol) was added. The resulting mixture was stirred for 26 hours at room temperature, and then water was added at 0°C. After the solution mixture was concentrated so that the tetrahydrofuran would be distilled away, 1 M hydrochloric acid (30.0 mL) and ethyl acetate (150 mL) were added, and separation was performed. The aqueous layer was subjected to extraction with ethyl acetate (50 mL × 2), and the organic layers were combined. The combined liquid was washed with a saturated saline solution (50 mL), dried with sodium sulfate and filtered, and the filtrate was concentrate under reduced pressure. The resulting crude product was purified by silica gel column chromatography (eluent: chloroform/ethyl acetate) to give a solid in light gray of 9-methyl-9H-carbazol-4-ol (actual yield, 2.94 g; percent yield, 55%) . ¹H-NMR (400 MHz, CDCl₃) δ (ppm) : 8.28 (dd, J = 7.8, 1.4 Hz, 1H), 7.46 (ddd, J = 8.2, 7.1, 1.4 Hz, 1H), 7.38 (dd, J = 8.2, 1.1 Hz, 1H), 7.31 (dd, J = 8.2, 7.8 Hz, 1H), 7.25 (ddd, J = 7.8, 7.1, 1.1 Hz, 1H), 7.00 (d, J = 8.2 Hz, 1H), 6.59 (d, J = 7.8 Hz, 1H), 5.28 (s, 1H), 3.84 (s, 3H) .

1,3-dimethyl-2-imidazolidinone (38.0 mL) was added to the 9-methyl-9H-carbazol-4-ol (1.50 g, 7.61 mmol) and cyanoacetic acid (650 mg, 7.64 mmol). In a water bath, trifluoroacetic anhydride (4.26 mL, 30.4 mmol) was added dropwise over 1 hour. After 18 hours of stirring at room temperature, the resulting mixture was poured into cold water (500 mL), and the precipitated solid was filtered and washed with water (100 mL). The solid was dried by heating, and then acetonitrile was added. The resulting mixture was sonicated for 10 minutes and then filtered, and the residue was washed with acetonitrile and dried. Acetonitrile was added to the resulting solid again, the resulting mixture was sonicated for 10 minutes, and then the solid was collected by filtration and dried to give an orange-yellow solid of 4-hydroxy-7-methyl-3-(2,2,2-trifluoroethan-1-on-1-yl)-2H,7H-pyrano[5,6-c]carbazol-2-one (actual yield, 1.34 g; percent yield, 490) . ¹H-NMR (400 MHz, CDCl₃) δ (ppm) : 15.29 (s, 1H), 8.55 (d, J = 7.8 Hz, 1H), 8.10 (d, J = 8.8 Hz, 1H), 7.60 (ddd, J = 7.8, 7.8, 1.1 Hz, 1H), 7.51 (dd, J = 7.8, 1.1 Hz, 1H), 7.45 (ddd, J = 7.8, 7.8, 1.1 Hz, 1H), 7.38 (d, J = 8.8 Hz, 1H), 3.97 (s, 3H). ¹⁹F-NMR (376 MHz, CDCl₃) δ (ppm): -74.4 (s, 3F).

### (REFERENCE EXAMPLE 58)

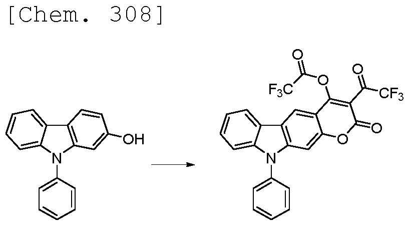

1,3-dimethyl-2-imidazolidinone (54.2 mL) was added to 9-phenyl-9H-carbazol-2-ol (2.81 g, 10.8 mmol) and cyanoacetic acid (927 mg, 10.9 mmol). In a water bath, trifluoroacetic anhydride (6.10 mL, 43.6 mmol) was added dropwise over 1 hour. After 65 hours of stirring at room temperature, the resulting mixture was poured into cold water (500 mL), and the precipitated solid was filtered and washed with water. The solid was dried by heating, and then acetonitrile was added. The resulting mixture was sonicated for 10 minutes, then the solid was collected by filtration, washed with acetonitrile and dried. The resulting solid was washed with diethyl ether. Diethyl ether was added to the resulting solid, the resulting mixture was stirred for 30 minutes, and then the solid was collected by filtration, washed with diethyl ether and dried by heating to give an ocherous solid of 6-phenyl-2-(2,2,2-trifluoroethan-1-on-1-yl)-1-(2,2,2-trifluoroethan-1-on-1-yloxy)-3H,6H-pyrano[6,5-b]carbazol-3-one (actual yield, 1.74 g; percent yield, 38%). ¹H-NMR (400 MHz, Acetone-d₆) δ (ppm): 9.01 (s, 1H), 8.46 (brd, J = 7.7 Hz, 1H), 7.81-7.75 (m, 2H), 7.75-7.69 (m, 2H), 7.66 (brd, J = 7.4, 7.4 Hz, 1H), 7.56 (ddd, J = 8.3, 7.4, 1.2 Hz, 1H), 7.44 (ddd, J = 7.7, 7.4, 0.9 Hz, 1H), 7.40 (brd, J = 8.3 Hz, 1H), 7.17 (s, 1H). ¹⁹F-NMR (376 MHz, Acetone-d₆) δ (ppm) : -74.8 (s, 3F), -76.8 (s, 3F).

### (REFERENCE EXAMPLE 59)

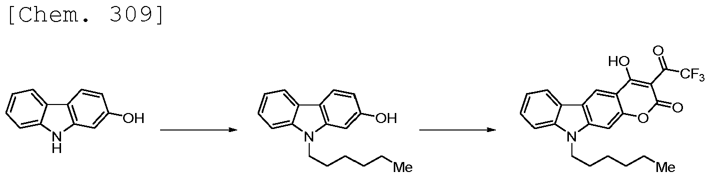

A dispersion of sodium hydride in paraffin liquid (2.75 g, 63.1 mmol) was washed with dehydrated hexane, and then tetrahydrofuran (54.6 mL) and N,N-dimethylformamide (4.23 mL, 54.6 mmol) were added. 9H-carbazol-2-ol (5.00 g, 27.3 mmol) was added at 0°C, and 1-iodohexane (4.42 mL, 30.0 mmol) was added to this. After 23 hours of stirring at room temperature, the addition of 1 M hydrochloric acid (50.0 mL) and ethyl acetate (100 mL) and separation were performed at 0°C. The aqueous layer was subjected to extraction with ethyl acetate (30 mL × 2), the organic layers were combined, and the combined liquid was washed with water (50 mL) and a saturated saline solution (50 mL) and then dried with sodium sulfate and concentrated under reduced pressure. The resulting crude product was recrystallized in ethyl acetate/hexane to give a light brown solid of 9-hexyl-9H-carbazol-2-ol (actual yield, 5.52 g; percent yield, 76%). ¹H-NMR (400 MHz, CDCl₃) δ (ppm): 7.98 (brd, J = 7.7 Hz, 1H), 7.91 (d, J = 8.3 Hz, 1H), 7.38 (ddd, J = 8.3, 6.8, 1.1 Hz, 1H), 7.34 (brd, J = 7.7 Hz, 1H), 7.19 (ddd, J = 7.7, 6.8, 1.4 Hz, 1H), 6.83 (d, J = 2.2 Hz, 1H), 6.72 (dd, J = 8.3, 2.2 Hz, 1H), 4.80 (s, 1H), 4.20 (t, J = 7.3 Hz, 2H), 1.84 (tt, J = 7.3, 7.3 Hz, 2H), 1.44-1.34 (m, 2H), 1.34-1.24 (m, 4H), 0.88 (t, J = 7.0 Hz, 3H).

1,3-dimethyl-2-imidazolidinone (46.8 mL) was added to the 9-hexyl-9H-carbazol-2-ol (2.50 g, 9.36 mmol) and cyanoacetic acid (797 mg, 9.37 mmol). In a water bath, trifluoroacetic anhydride (5.24 mL, 37.4 mmol) was added dropwise over 1 hour. After 43 hours of stirring at room temperature, the resulting mixture was poured into cold water (300 mL), and any residue was washed with water into the cold water. The resulting mixture was stirred for 3 hours, and the supernatant was removed by decantation. Diethyl ether was added to the resulting viscous solid, the resulting mixture was sonicated for 10 minutes, and the solid was collected by filtration, washed with diethyl ether and dried by heating to give an orange-yellow solid of 6-hexyl-1-hydroxy-2-(2,2,2-trifluoroethan-1-on-1-yl)-3H,6H-pyrano[5,6-b]carbazol-3-one (actual yield, 770 mg; percent yield, 19%) . ¹H-NMR (400 MHz, CDCl₃) δ (ppm) : 15.30 (brs, 1H), 8.73 (s, 1H), 8.13 (brd, J = 7.7 Hz, 1H), 7.56 (ddd, J = 8.3, 7.3, 1.2 Hz, 1H), 7.44 (brd, J = 8.3 Hz, 1H), 7.37 (brddd, J = 7.7, 7.3, 0.8 Hz, 1H), 7.17 (s, 1H), 4.28 (t, J = 7.3 Hz, 2H), 1.89 (tt, J = 7.3, 7.3 Hz, 2H), 1.45-1.35 (m, 2H), 1.35-1.24 (m, 4H), 0.87 (t, J = 7.1 Hz, 3H). ¹⁹F-NMR (376 MHz, CDCl₃) δ (ppm) : -74.3 (s, 3F).

### (REFERENCE EXAMPLE 60)

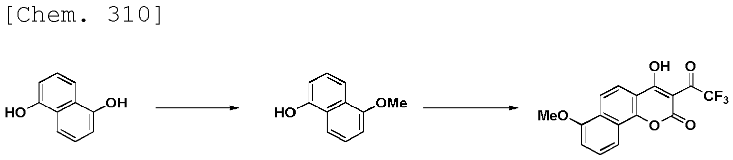

Acetone (20.0 mL) was added to 1,5-dihydroxynaphthalene (3.20 g, 20.0 mmol) and potassium carbonate (1.38 g, 10.0 mmol), and the resulting mixture was stirred for 3 hours at room temperature. The reaction solution was cooled to 0°C, iodomethane (1.24 mL, 19.9 mmol) dissolved in acetone (10.0 mL) was added dropwise, and then the resulting mixture was stirred for 17 hours while the temperature was increased to room temperature. The reaction suspension was filtered, the solid was washed with ethyl acetate, and then the mother liquor was concentrated under reduced pressure. The resulting solid was dissolved in chloroform, and the resulting solution was concentrated under reduced pressure after the removal of undissolved solids by filtration. The resulting solid was dissolved in methanol, undissolved solids were removed, and the mother liquor was concentrated. Then the residue was reprecipitated with chloroform/hexane, and the precipitated solid was removed to give a brown solid of 5-methoxy-1-naphthol (actual yield, 580 mg; percent yield, 33%). ¹H-NMR (400 MHz, CDCl₃) δ (ppm): 7.84 (brd, J = 8.5 Hz, 1H), 7.73 (brd, J = 8.5 Hz, 1H), 7.40 (dd, J = 8.5, 7.5 Hz, 1H), 7.30 (dd, J = 8.5, 7.5 Hz, 1H), 6.86 (brd, J = 7.5 Hz, 1H), 6.85 (brd, J = 7.5 Hz, 1H), 5.13 (s, 1H), 4.00 (s, 3H).

The 5-methoxy-1-naphthol (522 mg, 3.00 mmol) and cyanoacetic acid (255 mg, 3.00 mmol) were dissolved in 1,3-dimethyl-2-imidazolidinone (6.00 mL). After the reaction vessel was cooled in a water bath, trifluoroacetic anhydride (1.68 mL, 12.0 mmol) was added dropwise over 1 hour, and then the resulting mixture was stirred for 40 hours at room temperature. Water (40 mL) was added to the reaction solution, and the precipitated solid was collected by suction filtration and washed with ethanol to give a brown solid of 4-hydroxy-7-methoxy-3-(2,2,2-trifluoroethan-1-on-1-yl)-2H-naphtho[1,2-b]pyran-2-one (actual yield, 251 mg; percent yield, 25%). ¹H-NMR (400 MHz, CDCl₃) δ (ppm): 15.09 (brs, 1H), 8.17 (d, J = 9.0 Hz, 1H), 7.67 (brd, J = 8.1 Hz, 1H), 7.91 (brd, J = 9.0 Hz, 1H), 7.62 (dd, J = 8.1, 8.1, 1H), 7.13 (brd, J = 8.1 Hz, 1H), 4.05 (s, 3H), ¹⁹F-NMR (376 MHz, CDCl₃) δ (ppm): -74.7 (s, 3F) .

### (REFERENCE EXAMPLE 61)

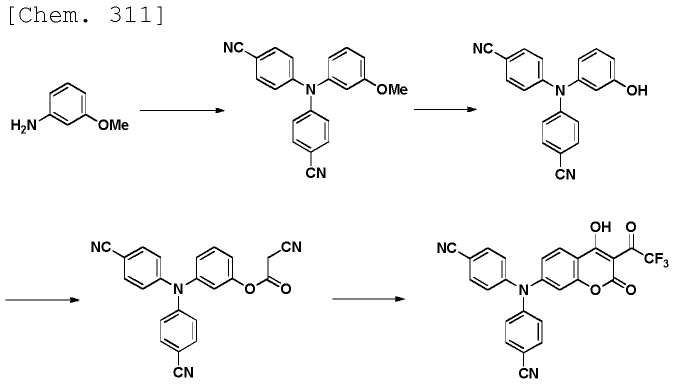

Sodium hydride (1.18 g, 27.0 mmol) was dissolved in 1,3-dimethyl-3,4,5,6-tetrahydro-2(1H)-pyrimidinone (20.0 mL), and 3-methoxyaniline (1.12 mL, 10.0 mmol) and 4-fluorobenzonitrile (3.15 g, 26.0 mmol) were added at 0°C. After 3 hours of stirring at room temperature, the temperature was increased to 60°C, and the mixture was stirred for 4 hours. Water (250 mL) and ethyl acetate (150 mL) were added to the reaction mixture, separation was performed, and then the aqueous layer was subjected twice to extraction with ethyl acetate (50 mL). The organic layers were combined, the combined liquid was washed with a saturated saline solution (50 mL) and then dried with sodium sulfate and concentrated under reduced pressure. Hexane (10 mL), methanol (10 mL) and ethyl acetate (10 mL) were added to the resulting crude product, and the precipitated solid was collected by filtration and dried by heating to give 4,4'-[(3-methoxyphenyl)imino]bisbenzonitrile as a light yellow solid (actual yield, 1.68 g; percent yield, 52%). ¹H-NMR (400 MHz, CDCl₃) δ (ppm): 7.53 (brd, J = 8.9 Hz, 4H), 7.29 (dd, J = 8.1, 8.1 Hz, 1H), 7.11 (brd, J = 8.9 Hz, 4H), 6.80 (dd, J = 8.1, 2.2 Hz, 1H), 6.70 (dd, J = 8.1, 2.2 Hz, 1H), 6.64 (dd, J = 2.2, 2.2 Hz, 1H), 3.77 (s, 3H).

The 4,4'-[(3-methoxyphenyl)imino]bisbenzonitrile (1.60 g, 4.92 mmol) was dissolved in dichloromethane (29.4 mL). At -78°C, boron tribromide (1.0 mol/L, 6.39 mL, 6.39 mmol) was added, and the resulting mixture was stirred for 1 hour. Then another portion of boron tribromide (1.0 mol/L, 6.39 mL, 6.39 mmol) was added, the temperature was increased to room temperature, and then the mixture was stirred for 22 hours. After the reaction mixture was cooled to 0°C, a saturated aqueous solution of sodium hydrogencarbonate (50 mL) and chloroform (150 mL) were added, separation was performed, and the aqueous layer was subjected twice to extraction with chloroform (40 mL). The organic layers were combined, and the combined liquid was washed with water (50 mL) and a saturated saline solution (50 mL) and then dried with sodium sulfate and concentrated under reduced pressure. The resulting crude product was placed on silica gel, washed with chloroform (100 mL) and ethyl acetate (300 mL) and concentrated under reduced pressure, and then the residue was recrystallized in acetone/hexane to give 4,4'-[(3-hydroxyphenyl)imino]bisbenzonitrile as a light yellow solid (actual yield, 1.10 g; percent yield, 720). ¹H-NMR (400 MHz, CDCl₃) δ (ppm): ¹H-NMR (400 MHz, CDCl₃) δ (ppm): 7.53 (brd, J = 8.9 Hz, 4H), 7.24 (dd, J = 8.1 Hz, 1H), 7.12 (brd, J = 8.9 Hz, 4H), 6.72 (dd, J = 8.1, 2.3 Hz, 1H), 6.69 (dd, J = 8.1, 2.3 Hz, 1H), 6.61 (dd, J = 2.3, 2.3 Hz, 1H), 5.0 (brs, 1H) .

The 4,4'-[(3-hydroxyphenyl)imino]bisbenzonitrile (313 mg, 1.01 mmol) and cyanoacetic acid (85.6 mg, 1.01 mmol) were dissolved in acetic anhydride (2.01 mL), and the resulting solution was stirred for 1 hour at 80°C. Water (20 mL) and ethyl acetate (30 mL) were added to the reaction solution, separation was performed, and then extraction with ethyl acetate (10 mL) was performed twice. The organic layers were combined, and the combined liquid was washed with a saturated saline solution (30 mL) and then dried with sodium sulfate and concentrated under reduced pressure. The resulting crude product was purified with a silica gel column chromatograph (eluent: hexane/ethyl acetate) to give a light yellow solid of {3-{bis(4-cyanophenyl)amino}phenyl 2-cyanoacetate (actual yield, 221 mg; percent yield, 58%). ¹H-NMR (400 MHz, CDCl₃) δ (ppm): 7.57 (brd, J = 8.9 Hz, 4H), 7.41 (dd, J = 8.1, 8.1 Hz, 1H), 7.13 (d, J = 8.9 Hz, 4H), 7.06-7.00 (m, 2H), 6.92 (dd, J = 2.2 Hz, 1H), 3.68 (s, 2H).

The {3-{bis(4-cyanophenyl)amino}phenyl 2-cyanoacetate (110 mg, 290 µmol) was dissolved in 1,3-dimethyl-2-imidazolidinone (46.8 mL). In a water bath, trifluoroacetic anhydride (244 µL, 1.74 mmol) was added dropwise over 10 minutes. After 5 hours of stirring at room temperature, the mixture was poured into water (50 mL), and the precipitated solid was collected by filtration and washed with water (20 mL). Diethyl ether (5 mL) was added to the resulting crude product, the resulting mixture was sonicated for 10 minutes, and then the solid was collected by filtration, washed with diethyl ether (10 mL) and dried by heating to give an orange-yellow solid of 7-{bis[4-cyanophenyl]amino}-4-hydroxy-3-(2,2,2-trifluoroethyl-1-on-1-yl)-2H-chromen-2-one (actual yield, 82.1 mg; percent yield, 59%). ¹H-NMR (400 MHz, CDCl₃) δ (ppm): 14.97 (brs, 1H), 7.97 (d, J = 8.8 Hz, 1H), 7.69 (brd, J = 8.8 Hz, 4H), 7.24 (brd, J = 8.8 Hz, 4H), 6.98 (dd, J = 8.8, 2.2 Hz, 1H), 6.85 (d, 2.2 Hz, 1H). ¹⁹F-NMR (376 MHz, CDCl₃) δ (ppm) : -74.5 (s, 3F).

### (REFERENCE EXAMPLE 62)

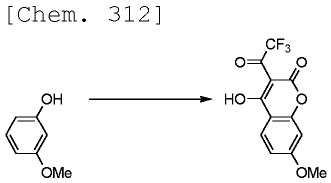

DMF (10 mL) and 3-methoxyphenol (1.08 mL, 10.0 mmol) were added to cyanoacetic acid (850 mg, 9.99 mmol). Trifluoroacetic anhydride (5.64 mL, 40.2 mmol) was added dropwise over 2 hours in a water bath at room temperature, and the resulting mixture was further stirred for 12 hours. The reaction solution was added to water, and the precipitated solid was collected by suction filtration, washed with water, washed with ethanol and vacuum-dried to give 4-hydroxy-7-methoxy-3-(2,2,2-trifluoroethan-1-on-1-yl)-2H-chromen-2-one as a light yellow solid (actual yield, 1.31 g; percent yield, 46%). ¹H-NMR (400 MHz, CDCl₃) δ (ppm): 15.1 (brs, 1H), 7.99 (d, J = 8.9 Hz, 1H), 6.93 (dd, J = 8.9, 2.3 Hz, 1H), 6.77 (d, J = 2.3 Hz, 1H), 3.95 (s, 3H). ¹⁹F-NMR (376 MHz, CDCl₃) δ (ppm): -74.5.

### (REFERENCE EXAMPLE 63)

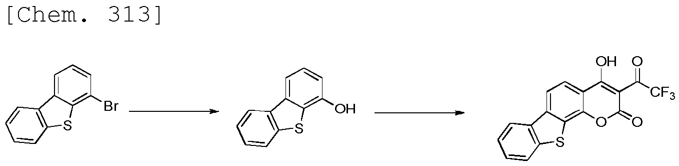

4-bromodibenzothiophene (2.56 g, 9.72 mmol), 2-di-tert-butylphosphino-2',4',6'-triisopropylbiphenyl (333 mg, 784 µmol), tris(dibenzylideneacetone)dipalladium(0) (179 mg, 195 µmol) and potassium hydroxide (1.20 g, 21.4 mmol) were suspended in 1,4-dioxene (22.0 mL) and water (11.0 mL), and the resulting suspension was stirred for 18 hours at 100°C. 1 M hydrochloric acid (22 mL) was added to the reaction mixture, and extraction with chloroform (20 mL) was performed three times. The organic layers were combined, and the combined liquid was dried with magnesium sulfate and then concentrated under reduced pressure. The resulting solid was washed with hexane, methanol was added, undissolved solids were removed by filtration, and the filtrate was concentrated under reduced pressure to give a light yellow individual of 4-hydroxydibenzothiophene (1.44 g, 74%) . ¹H-NMR (400 MHz, CDCl₃) δ (ppm): 8.14 (m, 1H), 7.89 (m, 1H), 7.79 (dd, 7.8, 0.8 Hz, 1H), 7.50-7.44 (m, 2H), 7.35 (dd, 7.8, 7.8 Hz, 1H), 6.89 (dd, 7.8, 0.8 Hz, 1H), 5.25 (brs, 1H).

The 4-hydroxydibenzothiophene (401 mg, 2.00 mmol) and cyanoacetic acid (170 mg, 2.00 mmol) were dissolved in 1,3-dimethyl-2-imidazolidinone (2.00 mL). After the reaction vessel was cooled in a water bath, trifluoroacetic anhydride (1.12 mL, 8.00 mmol) was added dropwise, and the resulting mixture was stirred for 18 hours at room temperature. Water (20 mL) was added to the reaction solution, and the precipitated solid was collected by suction filtration, washed with 30% aqueous ethanol and then washed with a small amount of acetone to give a brown solid of 4-hydroxy-3-(2,2,2-trifluoroethan-1-on-1-yl)-2H-[1]benzothieno[3,2-h]-1-benzopyran-2-one (actual yield, 39.4 mg; percent yield, 5.4 0) . ¹H-NMR (400 MHz, CDCl₃) δ (ppm): 15.23 (brs, 1H), 8.28 (d, J = 7.6 Hz, 1H), 8.13 (brs, 2H), 8.00 (d, J = 7.6 Hz, 1H), 7.65 (ddd, 7.1, 7.1, 1.1 Hz, 1H), 7.59 (ddd, 7.1, 7.1, 1.1 Hz, 1H), ¹⁹F-NMR (376 MHz, CDCl₃) δ (ppm): -74.5 (s) .

### (REFERENCE EXAMPLE 64)

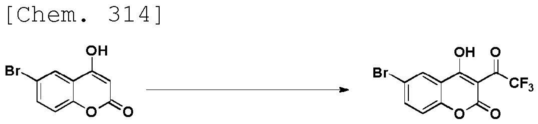

6-bromo-4-hydroxycoumarin (4.65 g, 19.3 mmol) and imidazole (6.57 g, 96.5 mmol) were suspended in chloroform (40.0 mL). After the reaction vessel was cooled in a water bath, trifluoroacetic anhydride (5.60 mL, 40.0 mmol) was added dropwise, and then the resulting mixture was stirred for 4 hours at room temperature. 2 M hydrochloric acid (30 mL) was added to the reaction mixture, and the precipitated solid was collected by suction filtration and washed with water (100 mL) to give a white solid of 6-bromo-4-hydroxy-3-(2,2,2-trifluoroethan-1-on-1-yl)-2H-chromen-2-one (actual yield, 6.17 g; percent yield, 95%). ¹H-NMR (400 MHz, DMSO-d₆) δ (ppm): 7.90 (d, J = 2.5 Hz, 1H), 7.66 (dd, J = 8.6, 2.5 Hz, 1H), 7.15 (d, J = 8.6 Hz, 1H). ¹⁹F-NMR (376 MHz, CDCl₃) δ (ppm): -72.4.

### (REFERENCE EXAMPLE 65)

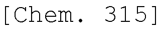

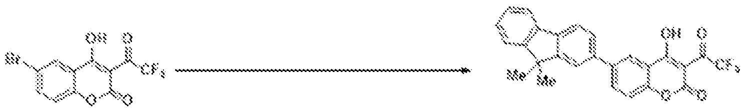

6-bromo-4-hydroxy-3-(2,2,2-trifluoroethan-1-on-1-yl)-2H-chromen-2-one (2.13 g, 6.34 mmol), 9,9-dimethylfluorene-2-boronic acid (2.26 g, 9.51 mmol), tetrakis(triphenylphosphine)palladium(0) (366 mg, 0.317 mmol) and cesium carbonate (3.10 g, 9.51 mmol) were suspended in toluene (30.0 mL), and the resulting suspension was stirred for 24 hours at 100°C. 1 M hydrochloric acid (10 mL) and water (10 mL) were added to the reaction mixture, and extraction with ethyl acetate (20 mL) was performed three times. The organic layers were combined, and the combined liquid was dried with sodium sulfate and then concentrated under reduced pressure. The resulting solid was washed with acetone to give a yellow solid of 6-(9,9-dimethyl-9H-fluoren-2-yl)-4-hydroxy-3-(2,2,2-trifluoroethan-1-on-1-yl)-2H-chromen-2-one (actual yield, 1.19 g; percent yield, 42%). ¹H-NMR (400 MHz, CDCl₃) δ (ppm): 15.15 (brs, 1H), 8.32 (d, 2.3 Hz, 1H), 8.08 (dd, 8.7, 2.3 Hz, 1H), 7.83 (d, 7.8 Hz, 1H), 7.77 (m, 1H), 7.64 (d, 1.6 Hz, 1H), 7.59 (dd, 8.8, 1.6 Hz, 1H), 7.48 (m, 1H), 7.46 (d, 8.7 Hz, 1H), 7.41-7.34 (m, 2H), 1.55 (brs, 6H). ¹⁹F-NMR (376 MHz, CDCl₃) δ (ppm) : -74.5 (brs) .

### (REFERENCE EXAMPLE 66)

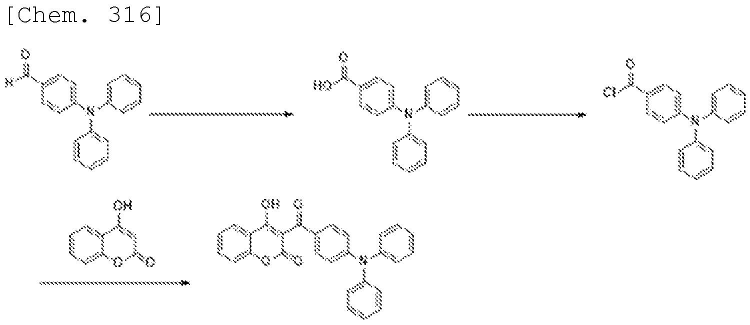

4-(diphenylamino)benzaldehyde (25.0 g, 91.5 mmol) was dissolved in acetone (640 mL) and water (160 mL), and the resulting solution was heated to 60°C. Potassium permanganate (66.5 g, 421 mmol) was added gradually, and the resulting mixture was stirred for 24 hours at 60°C. The reaction mixture was concentrated under reduced pressure, water (500 mL) was added, the resulting mixture was filtered, and 2 M hydrochloric acid (150 mL) was added to the filtrate. The precipitated solid was collected by filtration and dried by heating to give a white solid of 4-(diphenylamino)benzoic acid (actual yield, 26.3 g; percent yield, quant.). ¹H-NMR (400 MHz, CDCl₃) δ (ppm) : 7.90 (d, J = 8.6 Hz, 2H), 7.32 (dd, J = 7.2, 1.1 Hz, 4H), 7.18-7.10 (m, 6H), 6.99 (d, J = 8.6 Hz, 2H).

The 4-(diphenylamino)benzoic acid (2.89 g, 10.0 mmol) was dissolved in dichloromethane (50.0 mL). Oxalyl dichloride (1.29 mL, 15.0 mmol) and N,N-dimethylformamide (10 µL, 130 µmol) were added at 0°C, and the resulting mixture was stirred for 4 hours while the temperature was increased to room temperature. The reaction mixture was concentrated under reduced pressure to give a whitish orange solid of 4-(diphenylamino)benzoyl chloride. This crude product was used continuously in the next reaction without further purification. ¹H-NMR (400 MHz, CDCl₃) δ (ppm): 7.90 (d, J = 9.1 Hz, 2H), 7.36 (dd, J = 7.2, 0.9 Hz, 4H), 7.23-7.16 (m, 6H), 6.93 (d, J = 9.1 Hz, 2H).

4-hydroxycoumarin (1.47 g, 9.07 mmol) and the 4-(diphenylamino)benzoyl chloride (3.07 g, 9.97 mmol) were dissolved in dichloromethane (30.0 mL), then N,N-diisopropylethylamine (1.85 mL, 10.9 mmol) was added, and the resulting mixture was stirred for 16 hours at room temperature. The reaction solution was diluted with dichloromethane (20.0 mL), and then the diluted solution was washed with 1 M hydrochloric acid (40.0 mL), a saturated aqueous solution of sodium hydrogencarbonate (40.0 mL) and a saturated saline solution (40.0 mL). The resulting organic layer was dried with magnesium sulfate and then concentrated under reduced pressure to give a brown solid. The resulting crude product was dissolved in acetonitrile (30.0 mL), acetone cyanohydrin (910 µL, 9.94 mmol) and triethylamine (1.40 mL, 10.0 mmol) were added, and then the resulting mixture was stirred for 19 hours at 50°C. Ethyl acetate (20.0 mL) and 1 M hydrochloric acid (20.0 mL) were added to the reaction mixture, and the precipitated solid was collected by filtration and washed with water and methanol to give a yellow solid of 3-[4-(diphenylamino)benzoyl]-4-hydroxy-2H-chromen-2-one (actual yield, 2.77 g; percent yield, 71%). ¹H-NMR (400 MHz, DMSO-d₆) δ (ppm): 17.01 (s, 1H), 8.09 (dd, J = 8.0, 1.5 Hz, 1H), 7.71-7.64 (m, 3H), 7.37-7.30 (m, 6H), 7.22-7.13 (m, 6H), 6.95 (d, J = 9.0 Hz, 2H) .

### (REFERENCE EXAMPLE 67)

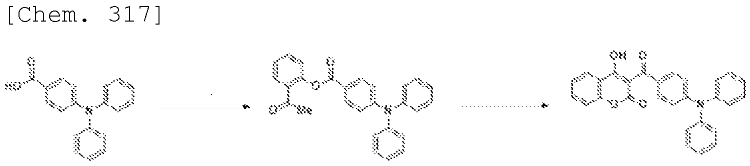

4-(diphenylamino)benzoic acid (1.45 g, 5.00 mmol) and 4-dimethylaminopyridine (675 mg, 5.53 mmol) were dissolved in N,N-diethylformamide (25.0 mL). Then 1-(3-dimethylaminopropyl)-3-ethylcarbodiimide hydrochloride (1.07 g, 5.56 mmol) and 2'-hydroxyacetophenone (1.21 mL, 10.0 mmol) were added, and the resulting mixture was stirred for 17 hours at room temperature. 2 M hydrochloric acid (20.0 mL) and water (70.0 mL) were added to the reaction solution, and the precipitated solid was collected by filtration and washed with water. The resulting solid was recrystallized in hot ethanol to give a yellow solid of 2-acetylphenyl-4-(diphenylamino)benzoyl (actual yield, 1.69 g; percent yield, 78%) . ¹H-NMR (400 MHz, DMSO-d₆) δ (ppm): 17.01 (s, 1H), 8.01 (d, J = 9.0 Hz, 2H), 7.84 (dd, J = 7.5, 1.7 Hz, 1H), 8.56 (ddd, 8.1, 7.5, 1.7 Hz, 1H), 7.37-7.31 (m, 5H), 7.23-7.13 (m, 7H), 7.03 (d, J = 9.0 Hz, 2H).

The 2-acetylphenyl-4-(diphenylamino)benzoyl (1.92 g, 4.71 mmol) was dissolved in N,N-dimethylformamide (10.0 mL), a 55 wt% dispersion of sodium hydride in liquid paraffin (514 mg, 11.8 mmol) was added, and the resulting mixture was stirred for 2 hours at 50°C. Methyl chloroformate (0.400 mL, 5.18 mmol) was added dropwise to the reaction mixture, the resulting mixture was stirred for 5 hours at 50°C and then cooled to 0°C, and 1 M hydrochloric acid (20.0 mL) was added. Water (20.0 mL) and methanol (10.0 mL) were added to the reaction mixture, and the precipitated solid was collected by filtration and washed with acetone to give a yellow solid of 3-[4-(diphenylamino)benzoyl]-4-hydroxy-2H-chromen-2-one (actual yield, 556 mg; percent yield, 27%). ¹H-NMR (400 MHz, DMSO-d₆) δ (ppm): 17.01 (s, 1H), 8.09 (dd, J = 8.0, 1.5 Hz, 1H), 7.71-7.64 (m, 3H), 7.37-7.30 (m, 6H), 7.22-7.13 (m, 6H), 6.95 (d, J = 9.0 Hz, 2H).

### (REFERENCE EXAMPLE 68)

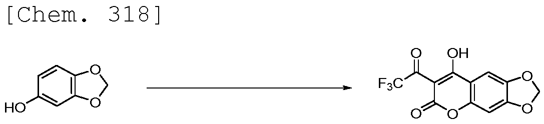

Sesamol (2.12 g, 15.4 mmol) and cyanoacetic acid (1.31 g, 15.4 mmol) were put into 1,3-dimethyl-2-imidazolidinone (29.0 mL). In a water bath, trifluoroacetic anhydride (8.60 mL, 40.0 mmol) was added dropwise over 1 hour, and then the resulting mixture was stirred for 14 hours at room temperature. The reaction solution was poured into water with ice (200 g), and the resulting mixture was stirred for 2 hours at room temperature. The precipitated solid was collected by suction filtration and dried by heating for 3 hours at 60°C under reduced pressure. The resulting solid was washed with acetonitrile and diethyl ether and dried by heating for 30 minutes at 60°C under reduced pressure. The resulting solid was washed with ethanol to give an orange solid of 8-hydroxy-3-(2,2,2-trifluoroethyl-1-on-1-yl)-6H-1,3-dioxolo[4,5-g] [1]benzopyran-6-one (actual yield, 1.51 g; percent yield, 35%) . ¹H-NMR (400 MHz, CDCl₃) δ (ppm) : 15.08 (brs, 1H), 7.35 (s, 1H), 6.79 (s, 1H), 6.17 (s, 2H). ¹⁹F-NMR (376 MHz, CDCl₃) δ (ppm): -74.6 (s).

### (REFERENCE EXAMPLE 69)

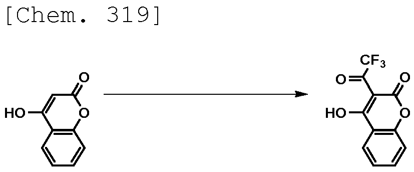

Tetrahydrofuran (530 mL) was added to 4-hydroxycoumarin (57.3 g, 353 mmol) and imidazole (120 g, 1.77 mol), and then a liquid mixture of trifluoroacetic anhydride (99.0 mL, 707 mmol) and tetrahydrofuran (177 mL) was added dropwise over 1.5 hours in an ice bath. Then the resulting mixture was stirred for 4 hours at room temperature, a white solid that formed was isolated by filtration, and the solvents were distilled away under reduced pressure. The residue was stirred with 300 mL of ethyl acetate added to it, and the precipitate that formed was collected by filtration. This was stirred with 534 mL of toluene and 534 mL of 2 M hydrochloric acid added to it, and then the aqueous layer was removed. This was washed twice with 534 mL of water, and then the solvent was distilled away under reduced pressure to give a white solid of 4-hydroxy-3-(2,2,2-trifluoroethan-1-on-1-yl)-2H-chromen-2-one (actual yield, 43.1 g; percent yield, 47%).

### (REFERENCE EXAMPLE 70)

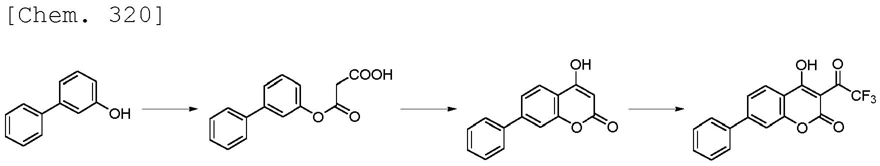

3-phenylphenol (1.92 g, 11.3 mmol) and Meldrum's acid (1.63 g, 11.3 mmol) were mixed together and stirred for 3 hours at 90°C. The reaction mixture was allowed to cool at room temperature and poured into ice (100 g), and water (50 mL) was added. After 30 minutes of stirring at room temperature, the precipitate that formed was collected by filtration and dried at 60°C under reduced pressure to give an orange solid of (3-biphenylyl) propanedioate (actual crude yield, 2.06 g; percent crude yield, 77%). The resulting crude product was used in the next reaction directly, without further purification. ¹H-NMR (400 MHz, CDCl₃) δ (ppm): 7.59-7.55 (m, 2H), 7.52-7.40 (m, 4H), 7.40-7.33 (m, 2H), 7.12 (ddd, J = 7.6, 2.4,1,6 Hz, 1H), 3.73 (s, 2H) .

The (3-biphenylyl) propanedioate (2.05 g, 8.66 mmol) and Eaton's reagent (11 mL) were mixed together and stirred for 5 hours at 70°C. The reaction mixture was allowed to cool at room temperature, ice (20 g) and water (20 mL) were added, and the resulting mixture was stirred for 30 minutes at room temperature. The precipitate that formed was collected by filtration, washed with diethyl ether and then dried at 60°C under reduced pressure to give an orange solid of 7-phenyl-4-hydroxy-2H-chromen-2-one (actual yield, 951 mg; percent yield, 35%). ¹H-NMR (400 MHz, DMSO-d₆) δ (ppm): 12.59 (brs, 1H), 7.93 (d, J = 8.6 Hz, 1H), 7.85-7.81 (m, 2H), 7.74-7.70 (m, 2H), 7.58-7.52 (m, 2H), 7.33 (brt, J = 7.3 Hz, 1H), 5.64 (s, 1H).

Chloroform (20.0 mL) was put into the 7-phenyl-4-hydroxy-2H-chromen-2-one (474 mg, 1.99 mmol) and imidazole (697 mg, 10.2 mmol), and the resulting mixture was cooled to 0°C. Trifluoroacetic anhydride (420 µL, 3.00 mmol) was added dropwise to this, and then the resulting mixture was stirred for 15 hours while the temperature was increased to room temperature. After the reaction mixture was cooled to 0°C, 1 M hydrochloric acid (30 mL) was added, and the precipitated solid was removed by suction filtration. The solid that precipitated in the filtrate was collected by filtration and dried at 60°C under reduced pressure. Chloroform was put into the resulting solid, an undissolved material was removed by filtration, and the filtrate was concentrated under reduced pressure to give a light brown solid of 4-hydroxy-7-phenyl-3-(2,2,2-trifluoroethan-1-on-1-yl)-2H-chromen-2-one (actual yield, 126 mg; percent yield, 19%). ¹H-NMR (400 MHz, CDCl₃) δ (ppm): 15.06 (brs, 1H), 8.15 (d, J = 8.4 Hz, 1H), 7.69-7.65 (m, 2H), 7.64 (dd, J = 8.4, 1.7 Hz, 1H), 7.56 (brd, J = 1.7 Hz, 1H), 7.56-7.46 (m, 3H). ¹⁹F-NMR (376 MHz, CDCl₃) δ (ppm): -74.5.

### (REFERENCE EXAMPLE 71)

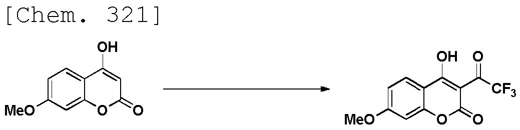

Tetrahydrofuran (20.0 mL) was added to 4-hydroxycoumarin (1.92 g, 10.0 mmol) and imidazole (3.41 g, 50.0 mol), and then trifluoroacetic anhydride (2.80 mL, 20.0 mmol) was added. Then the resulting mixture was stirred for 4 hours at room temperature. After the addition of 2 M hydrochloric acid (20.0 mL), the solution was diluted with ethyl acetate (20.0 mL), and the diluted solution was washed with water and a saturated saline solution. The organic layer was dried with sodium sulfate and then concentrated under reduced pressure, and the residue was washed with ethanol to give 4-hydroxy-7-methoxy-3-(2,2,2-trifluoroethan-1-on-1-yl)-2H-chromen-2-one as a light brown solid (actual yield, 2.21 g; percent yield, 77%). ¹H-NMR (400 MHz, CDCl₃) δ (ppm): 15.1 (brs, 1H), 7.99 (d, J = 8.9 Hz, 1H), 6.93 (dd, J = 8.9, 2.3 Hz, 1H), 6.77 (d, J = 2.3 Hz, 1H), 3.95 (s, 3H). ¹⁹F-NMR (376 MHz, CDCl₃) δ (ppm): -74.5.

### (REFERENCE EXAMPLE 72)

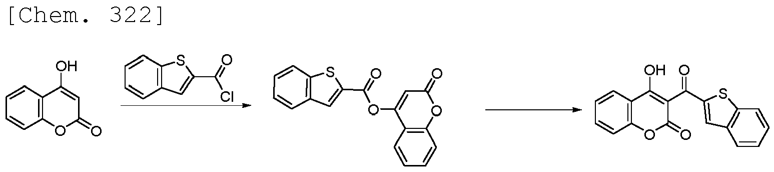

4-hydroxycoumarin (704 mg, 4.34 mmol) was dissolved in dichloromethane (13.0 mL), and then N,N-diisopropylethylamine (750 µL, 4.41 mmol) was added. A solution of 2-benzothiophenecarboxylic acid chloride (866 mg, 4.41 mmol) in dichloromethane (15.0 mL) was added, and the resulting mixture was stirred for 3 hours at room temperature. An additional portion of 4-hydroxycoumarin (318 mg, 1.99 mmol) was added, and the resulting mixture was stirred for 90 minutes at 35°C. The solvent was distilled away under reduced pressure, the resulting crude product was dissolved in acetonitrile (15.0 mL), acetone cyanohydrin (440 µL, 4.82 mmol) and triethylamine (1.80 mL, 13.2 mmol) were added, and then the resulting mixture was stirred for 12 hours at 70°C. Ethyl acetate (100 mL) was added to the reaction mixture, and the resulting mixture was washed three times with 2 M hydrochloric acid (50 mL). The resulting organic layer was concentrated under reduced pressure. The resulting crude product was washed with methanol, then acetone was added, and an undissolved material was removed by filtration. After the solvent was distilled away under reduced pressure, ethyl acetate was added, and an undissolved material was removed by filtration. Reprecipitation with acetone/hexane, furthermore, was performed, and the resulting solid was collected by filtration and dried by heating under reduced pressure to give a yellow solid of 4-hydroxy-3-[benzothiophene-2-carbonyl]-2H-chromen-2-one (actual yield, 93.1 mg; percent yield, 7.2%). ¹H-NMR (400 MHz, CDCl₃) δ (ppm): 16.88 (brs, 1H), 8.68 (s, 1H), 8.13 (dd, J = 8.0, 1.6 Hz, 1H), 7.93 (d, J = 8.0 Hz, 1H), 7.91 (dd, J = 8.0, 0.7 Hz, 1H), 7.74 (ddd, J = 8.7, 7.4, 1.6 Hz, 1H), 7.49 (ddd, J = 8.3, 7.2, 1.2 Hz, 1H), 7.44-7.34 (m, 3H).

### (REFERENCE EXAMPLE 73)

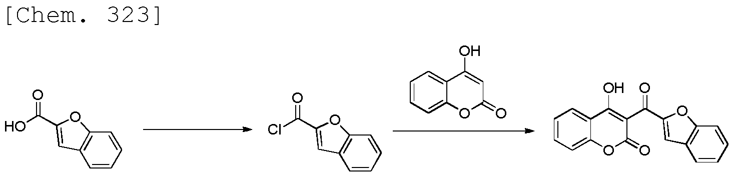

2-benzofurancarboxylic acid (2.05 g, 12.6 mmol) was dissolved in dichloromethane (25.0 mL). Oxalyl dichloride (1.30 mL, 15.1 mmol) and N,N-dimethylformamide (10 µL, 130 µmol) were added at 0°C, and the resulting mixture was stirred for 3 hours while the temperature was increased to room temperature. The reaction mixture was concentrated under reduced pressure to give a white solid of benzofuran-2-carbonyl chloride. This crude product was used in the next reaction without further purification. ¹H-NMR (400 MHz, CDCl₃) δ (ppm): 7.86 (d, J = 0.9 Hz, 1H), 7.77 (ddd, J = 7.9, 0.9, 0.9 Hz, 1H), 7.64-7.54 (m, 2H), 7.38 (ddd, J = 8.0, 6.9, 1.3 Hz, 1H).

4-hydroxycoumarin (2.05 g, 12.6 mmol) and the benzofuran-2-carbonyl chloride (2.28 g, 12.6 mmol) were dissolved in dichloromethane (40.0 mL), then N,N-diisopropylethylamine (2.60 mL, 15.3 mmol) was added, and the resulting mixture was stirred for 16 hours at room temperature. The reaction solution was diluted with dichloromethane (20.0 mL), and then the diluted solution was washed with 1 M hydrochloric acid (40.0 mL), a saturated aqueous solution of sodium hydrogencarbonate (40.0 mL) and a saturated saline solution (40.0 mL). The resulting organic layer was dried with magnesium sulfate and then concentrated under reduced pressure to give a brown solid. The resulting crude product was dissolved in acetonitrile (40.0 mL), acetone cyanohydrin (1.32 mL, 14.4 mmol) and triethylamine (1.95 mL, 14.0 mmol) were added, and then the resulting mixture was stirred for 19 hours at 50°C. 1 M hydrochloric acid (30.0 mL) was added to the reaction mixture, and the precipitated solid was collected by filtration and washed with water and acetonitrile to give a yellow solid of 3-(benzofuran-2-carbonyl)-4-hydroxy-2H-chromen-2-one (actual yield, 2.21 g; percent yield, 57%). ¹H-NMR (400 MHz, CDCl₃) δ (ppm): 17.11 (s, 1H), 8.38 (d, J = 1.0 Hz, 1H), 8.13 (ddd, J = 7.9, 1.6, 0.4 Hz, 1H), 7.79-7.75 (m, 1H), 7.74 (ddd, J = 8.4, 7.3, 1.6 Hz, 1H), 7.63 (dd, J = 8.4, 1.0 Hz, 1H), 7.52 (ddd, J = 8.4, 7.3, 1.3 Hz, 1H), 7.41-7.31 (m, 3H).

### (EXAMPLE 1)

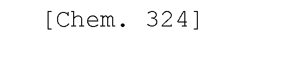

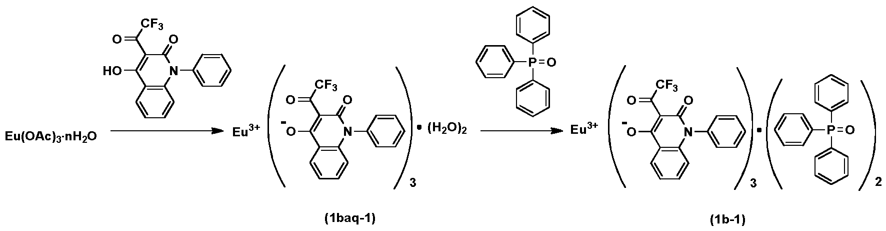

Water (40 mL) and chloroform (40 mL) were added to a mixture of sodium hydrogencarbonate (504 mg, 6.00 mmol) and the 4-hydroxy-1-phenyl-3-(trifluoroacetyl)quinolin-2(1H)-one obtained in Reference Example 1 (2.00 g, 6.00 mmol). Europium acetate n-hydrate (748 mg, 2.00 mmol as a 2.5-hydrate) was added, and the resulting mixture was stirred for 2 hours at room temperature. The reaction mixture was suction-filtered, and the resulting solid was washed with water, giving diaquatris[4-hydroxy-1-phenyl-3-(trifluoroacetyl)quinolin-2(1H)-onato]europium(III) (1baq-1) (2.55 g). This solid (237 mg) was suspended in methanol (4.0 mL), triphenylphosphine oxide (111 mg, 0.40 mmol) was added, and the resulting mixture was stirred for 16 hours at room temperature. The reaction solution was emptied into water, and the precipitated solid was collected by suction filtration and washed with water. This solid was suspended in methylcyclohexane, dehydrated by azeotropic distillation and then allowed to cool, and the solid was collected by suction filtration to give a white solid of tris[4-hydroxy-1-phenyl-3-(2,2,2-trifluoroethan-1-on-1-yl)quinolin-2(1H)-onato]bis(triphenylphosphine oxide)europium(III) (1b-1) (actual yield, 158 mg; percent yield from europium acetate 2.5-hydrate, 50%) . ¹⁹F-NMR (376 MHz, Acetone-d₆) δ (ppm) : - 72.2 (s, 7.1F), -74.3 (s, 1.2F), -74.8 (s, 0.7F). ³¹P-NMR (162 MHz, Acetone-d₆) δ (ppm): -86.6 (brs). ESIMS (m/z), MeCN: 1728.2 [M + Na]⁺, 1373.2 [M - 1-phenyl-3-(trifluoroacetyl)quinolin-2(1H)-onato]⁺.

### (EXAMPLE 2)

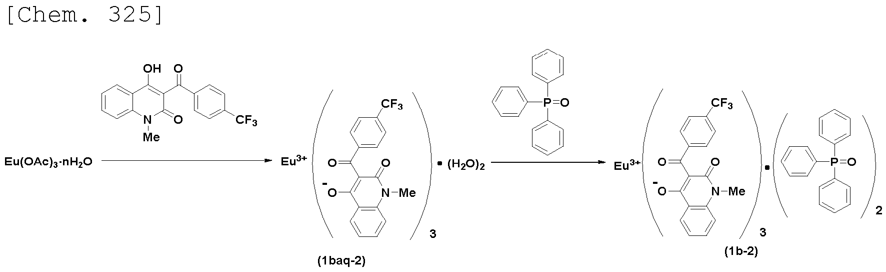

Europium acetate n-hydrate (74.0 mg as a 2.5-hydrate, 200 µmol) and the 4-hydroxy-1-methyl-3-[4-(trifluoromethyl)benzoyl]quinolin-2(1H)-one obtained in Reference Example 2 (206 mg, 600 µmol) were suspended in ethanol (7.5 mL) and water (2.5 mL). Then 1 M sodium hydroxide (600 µL, 600 µmol) was added, and the resulting mixture was stirred for 3 hours at room temperature. The reaction mixture was concentrated under reduced pressure, and the resulting solid was washed with water and then dried by heating to give a yellow solid of diaquatris[4-hydroxy-1-methyl-3-[4-(trifluoromethyl)benzoyl]quinolin-2(1H)-onato]europium(III) (1baq-2) (actual yield, 245 mg; percent yield, quant.). The resulting crude product was used in the next reaction directly, without further purification. ¹⁹F-NMR (376 MHz, CDC1₃) δ (ppm): -62.1. ESIMS (m/z), MeCN: 1214.3 [M - 2H₂O + Na]⁺.

The diaquatris[4-hydroxy-1-methyl-3-[4-(trifluoromethyl)benzoyl]quinolin-2(1H)-onato]europium(III) (245 mg, 200 µmol) and triphenylphosphine oxide (111 mg, 400 µmol) were dissolved in acetone (20 mL), and the resulting solution was stirred for 3 hours at room temperature. The reaction mixture was concentrated under reduced pressure, and the residue was reprecipitated with chloroform/hexane to give a yellow solid of tris[4-hydroxy-1-methyl-3-[4-(trifluoromethyl)benzoyl]quinolin-2(1H)-onato]bis(triphenylphosphine oxide)europium(III) (1b-2) (actual yield, 343 mg; percent yield, 98%). ¹⁹F-NMR (376 MHz, CDCl₃) δ (ppm): -62.5. ³¹P-NMR (162 MHz, CDC1₃) δ (ppm): -76.4 (brs). ESIMS (m/z), MeCN/MeOH: 1768.6 [M + Na]⁺.

### (EXAMPLE 3)

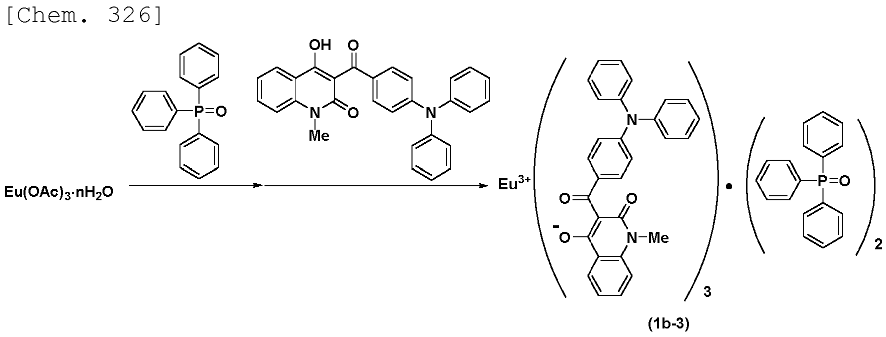

Methanol (20.0 mL) was added to europium acetate n-hydrate (74.8 mg as a 2.5-hydrate, 200 µmol) and triphenylphosphine oxide (111 mg, 400 µmol), and the resulting mixture was stirred for 1 hour at room temperature. The 4-hydroxy-1-methyl-3-[4-(diphenylamino)benzoyl]quinolin-2(1H)-one obtained in Reference Example 3 (268 mg, 600 µmol) was added to the reaction mixture. 14.8 M aqueous ammonia (40.5 µL, 600 µmol) was added dropwise, and the resulting mixture was stirred for three days at room temperature. The reaction solution was concentrated under reduced pressure, then the residue was dissolved by adding chloroform to it, and the resulting solution was concentrated under reduced pressure. The resulting solid was washed with diethyl ether to give a yellow solid of tris[4-hydroxy-1-methyl-3-[4-(diphenylamino)benzoyl]quinolin-2(1H)-onato]bis(triphenylphosphine oxide)europium(III) (1b-3) (actual yield, 149 mg; percent yield, 73%). ³¹P-NMR (162 MHz, CDC1₃) δ (ppm): -76.0 (brs). ESIMS (m/z), MeCN: 1599.0 [M - (4-hydroxy-1-methyl-3-[4-(diphenylamino)benzoyl]quinolin-2(1H)-onato)]⁺.

### (EXAMPLE 4)

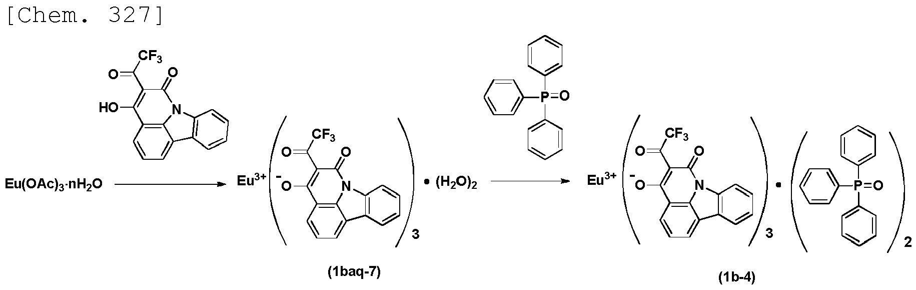

Ethanol (21.4 mL) and water (7.10 mL) were added to europium(III) acetate hydrate (215 mg as a 2.5-hydrate, 573 µmol) and the 4-hydroxy-5-(2,2,2-trifluoroethan-1-on-1-yl)-6H-pyrido[3,2,1-jk]carbazol-6-one obtained in Reference Example 15 (568 mg, 1.72 mmol). A 1 M aqueous solution of sodium hydroxide (1.71 mL, 1.71 mmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give a light orange-yellow solid of diaquatris[4-hydroxy-5-(2,2,2-trifluoroethan-1-on-1-yl)-6H-pyrido[3,2,1-jk]carbazol-6-onato]europium(III) (1baq-7) (actual yield, 648 mg; percent yield, 960). The resulting crude product was used in the next reaction directly, without further purification. ¹⁹F-NMR (376 MHz, Acetone-d₆) δ (ppm): -73.1 (brs, 2.6F), -73.3 (brs, 1.4F), -73.7 (brs, 5.0F). ESIMS (m/z), MeOH: 1165.6 [M - 2H₂O + Na]⁺.

Acetone (8.50 mL) was added to the diaquatris[4-hydroxy-5-(2,2,2-trifluoroethan-1-on-1-yl)-6H-pyrido[3,2,1-jk]carbazol-6-onato]europium(III) (200 mg, 170 µmol) and triphenylphosphine oxide (144 mg, 516 µmol), and the resulting mixture was stirred for 3 hours at room temperature. The reaction mixture was concentrated under reduced pressure, giving a crude product orange-yellow solid. The resulting crude product was dissolved in acetone, and hexane was added to cause a solid in powder form to precipitate. After the system was allowed to stand, the supernatant was removed with a pipette. The same operation was performed once again, and the resulting solid was dried by heating (40°C) to give a light orange-yellow solid of tris[4-hydroxy-5-(2,2,2-trifluoroethan-1-on-1-yl)-6H-pyrido[3,2,1-jk]carbazol-6-onato]bis(triphenylphosphine oxide)europium(III) (1b-4) (actual yield, 251 mg; percent yield, 87%) . ¹⁹F-NMR (376 MHz, Acetone-d₆) δ (ppm) : -68.7 (brs, 0.2F), -70.6 (brs, 0.6F), -70.9 (brs, 2.5F), -71.2 (brs, 0.4F), -71.8 (brs, 1.5F), -72.1 (brs, 1.9F), -72.3 (brs, 0.9F), -73.6 (brs, 0.3F), -74.0 (brs, 0.7F). ³¹P-NMR (162 MHz, Acetone-d₆) δ (ppm): -86.6 (brs). ESIMS (m/z), MeCN: 1719.8 [M + Na]⁺, 1441.8 [M - (triphenylphosphine oxide) + Na]⁺, 1368.8 [M - (4-hydroxy-5-(2,2,2-trifluoroethan-1-on-1-yl)-6H-pyrido[3,2,1-jk]carbazol-6-onato)]⁺.

### (EXAMPLE 5)

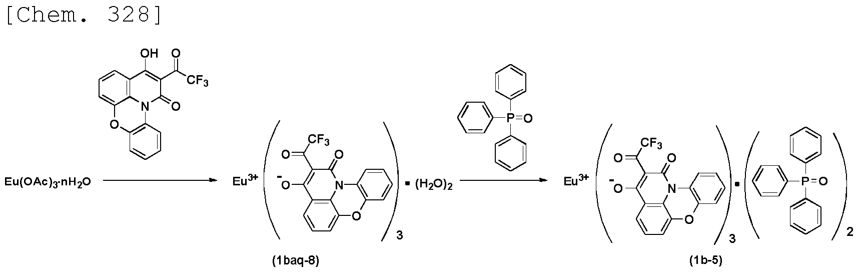

Ethanol (7.5 mL) and water (2.5 mL) were added to europium acetate n-hydrate (74.0 mg as a 2.5-hydrate, 200 µmol) and the 3-hydroxy-2-(2,2,2-trifluoroethan-1-on-1-yl)-1H-pyrido[3,2,1-kl]phenothiazin-1-one obtained in Reference Example 4 (208 mg, 600 µmol). Then 1 M sodium hydroxide (600 µL, 600 µmol) was added, and the resulting mixture was stirred for 3 hours at room temperature. The reaction mixture was concentrated under reduced pressure, and the resulting solid was washed with water and then dried by heating to give a yellow solid of diaquatris[3-hydroxy-2-(2,2,2-trifluoroacetyl)-1H-pyrido[3,2,1-kl]phenoxazin-1-onato]europium(III) (1baq-8) (actual yield, 229 mg; percent yield, 93%). The resulting crude product was used in the next reaction directly, without further purification. ¹⁹F-NMR (376 MHz, Acetone-d₆) δ (ppm): -73.6.

Acetone (20 mL) was added to the diaquatris[3-hydroxy-2-(2,2,2-trifluoroethan-1-on-1-yl)-1H-pyrido[3,2,1-kl]phenoxazin-1-onato]europium(III) (229 mg, 180 µmol) and triphenylphosphine oxide (100 mg, 360 µmol), and the resulting mixture was stirred for 3 hours at room temperature. The reaction mixture was filtered through Celite, the filtrate was concentrated under reduced pressure, and then the residue was reprecipitated with acetone/hexane to give a yellow solid of tris[3-hydroxy-2-(2,2,2-trifluoroethan-1-on-1-yl)-1H-pyrido[3,2,1-kl]phenoxazin-1-onato]bis(triphenylphosphine oxide)europium(III) (1b-5) (actual yield, 131 mg; percent yield, 42%). ¹⁹F-NMR (376 MHz, CDC1₃) δ (ppm): -71.6 (s, 2.0F) -71.7 (s, 6.0F), -73.5 (s, 0.2F), -73.7 (s, 0.8F) ³¹P-NMR (162 MHz, CDC1₃) δ (ppm): -85.8 (brs). ESIMS (m/z), MeCN/MeOH: 1491.5 [M - (triphenylphosphine oxide) + Na]⁺.

### (EXAMPLE 6)

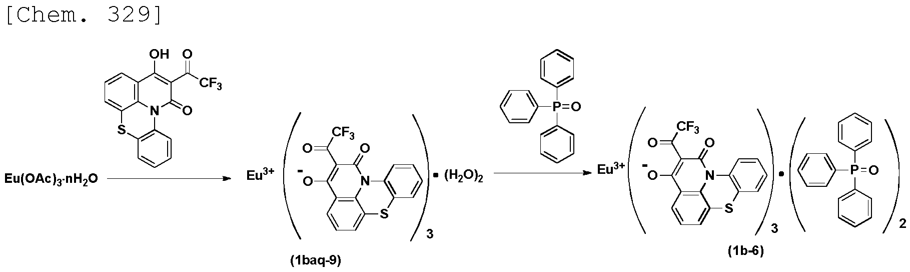

Ethanol (7.5 mL) and water (2.5 mL) were added to europium acetate n-hydrate (74.0 mg as a 2.5-hydrate, 200 µmol) and the 3-hydroxy-2-(2,2,2-trifluoroethan-1-on-1-yl)-1H-pyrido[3,2,1-kl]phenothiazin-1-one obtained in Reference Example 5 (217 mg, 600 µmol). Then 1 M sodium hydroxide (600 µL, 600 µmol) was added, and the resulting mixture was stirred for 3 hours at room temperature. The reaction mixture was concentrated under reduced pressure, and the resulting solid was washed with water and then dried by heating to give a yellow solid of diaquatris[3-hydroxy-2-(2,2,2-trifluoroethan-1-on-1-yl)-1H-pyrido[3,2,1-kl]phenothiazin-1-onato]europium(III) (1baq-9) (actual yield, 216 mg; percent yield, 850). The resulting crude product was used in the next reaction directly, without further purification.

¹⁹F-NMR (376 MHz, Acetone-d₆) δ (ppm): -72.2. ESIMS (m/z), MeCN: 1261.5 [M - 2H₂O + Na]⁺.

The diaquatris[3-hydroxy-2-(2,2,2-trifluoroethan-1-on-1-yl)-1H-pyrido[3,2,1-kl]phenothiazin-1-onato]europium(III) (216 mg, 169 µmol) and triphenylphosphine oxide (94.1 mg, 338 µmol) were dissolved in acetone (20 mL), and the resulting solution was stirred for 3 hours at room temperature. The reaction mixture was filtered through Celite, and the filtrate was concentrated under reduced pressure to give a yellow solid of tris[3-hydroxy-2-(2,2,2-trifluoroacetyl)-1H-pyrido[3,2,1-kl]phenothiazin-1-onato]bis(triphenylphosphine oxide)europium(III) (1b-6) (actual yield, 284 mg; percent yield, 93%). ³¹P-NMR (162 MHz, CDCl₃) δ (ppm) : -85.5 (brs) . ¹⁹F-NMR (376 MHz, CDC1₃) δ (ppm): -71.0 (s, 1.0F), -72.0 (s, 2.2F), -72.2 (s, 4.4F), - 73.4 (s, 0.4F), -73.6 (s, 1.0F). ESIMS (m/z), MeCN/MeOH: 1539.2 [M - (triphenylphosphine oxide) + Na]⁺.

### (EXAMPLE 7)

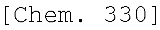

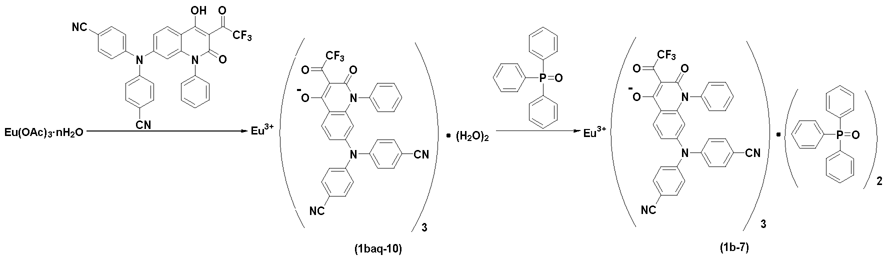

Ethanol (700 µL) and water (230 µL) were added to europium acetate n-hydrate (7.1 mg as a 2.5-hydrate, 19.0 µmol) and the 4,4'-{[4-hydroxy-1-phenyl-3-(2,2,2-trifluoroethan-1-on-1-yl)quinolin-2(1H)-on-7-yl]imino}bisbenzonitrile obtained in Reference Example 6 (31.4 mg, 57.0 µmol). Then 1 M sodium hydroxide (10.0 µL, 10.0 µmol) was added, and the resulting mixture was stirred for 3 hours at room temperature. The reaction mixture was concentrated under reduced pressure, and then the resulting solid was washed with water and dried by heating to give a yellow solid of diaquatris(4,4'-{[4-hydroxy-1-phenyl-3-(2,2,2-trifluoroethan-1-on-1-yl)quinolin-2(1H)-on-7-yl]imino}bisbenzonitril-ato)europium(III) (1baq-10) (actual yield, 24.0 mg; percent yield, 59%). The resulting crude product was used in the next reaction directly, without further purification. ¹⁹F-NMR (376 MHz, CDCl₃) δ (ppm): - 74.0. ESIMS (m/z), MeOH: 1823.3 [M - 2H₂O + Na]⁺.

Acetone (1.3 mL) was added to the diaquatris(4,4'-{[4-hydroxy-1-phenyl-3-(2,2,2-trifluoroethan-1-on-1-yl)quinolin-2(1H)-on-7-yl]imino}bisbenzonitril-ato)europium(III) (24.0 mg, 12.9 µmol) and triphenylphosphine oxide (7.20 mg, 38.5 µmol), and the resulting mixture was stirred for 3 hours at room temperature. The reaction mixture was concentrated under reduced pressure, the residue was reprecipitated with acetone/hexane, and then the resulting solid was washed with diethyl ether to give a yellow solid of tris(4,4'-{[4-hydroxy-1-phenyl-3-(2,2,2-trifluoroethan-1-on-1-yl)quinolin-2 (1H)-on-7-yl]imino}bisbenzonitril-ato)bis(triphenylphosphine oxide)europium(III) (1b-7) (actual yield, 25.0 mg; percent yield, 83%). ¹⁹F-NMR (376 MHz, CDC1₃) δ (ppm): -72.1. ³¹P-NMR (162 MHz, CDCl₃) δ (ppm): -84.5 (brs). ESIMS (m/z), MeOH: 2100.8 [M - (triphenylphosphine oxide) + Na]⁺.

### (EXAMPLE 8)

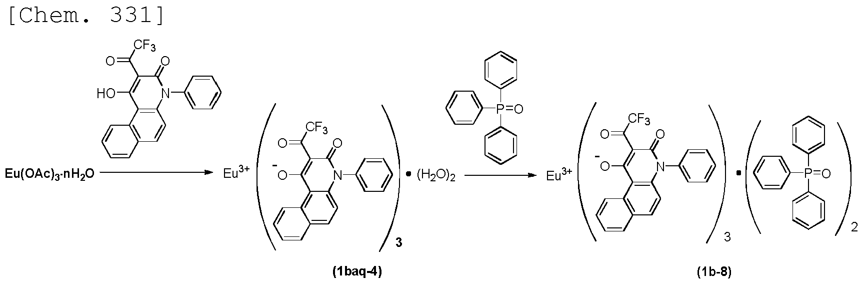

Water (10 mL) and chloroform (10 mL) were added to a mixture of sodium hydrogencarbonate (126 mg, 1.50 mmol) and europium acetate n-hydrate (187 mg, 500 µmol as a 2.5-hydrate). The 1-hydroxy-4-phenyl-2-(2,2,2-trifluoroethan-1-on-1-yl)benzo[f]quinolin-3(4H)-one obtained in Reference Example 7 (575 mg, 1.50 mmol), and the resulting mixture was stirred for 2 hours at room temperature. The reaction mixture was suction-filtered, and the resulting solid was washed with water, giving diaquatris[1-hydroxy-4-phenyl-2-(2,2,2-trifluoroethan-1-on-1-yl)benzo[f]quinolin-1(4H)-onato]europium(III) (1baq-4) (621 mg). This solid (267 mg) was suspended in methanol (4.0 mL), triphenylphosphine oxide (111 mg, 400 µmol) was added, and the resulting mixture was stirred for 20 hours at room temperature. The reaction solution was emptied into water, and the precipitated solid was collected by suction filtration and washed with water. This solid was suspended in methylcyclohexane, dehydrated by azeotropic distillation and then allowed to cool, and the solid was collected by suction filtration to give a yellow solid of tris[4-phenyl-2-(trifluoroacetyl)benzo[f]quinolin-3(4H)-onato]bis(triphenylphosphine oxide)europium(III) (1b-8) (actual yield, 159 mg; percent yield from europium acetate 2.5-hydrate, 40%). ¹⁹F-NMR (376 MHz, Acetone-d₆) δ (ppm): -72.1 (s, 1.4F), -72.4 (s, 4.2F), -72.7 (s, 3.4F). ³¹P-NMR (162 MHz, Acetone-d₆) δ (ppm): -86.4. ESIMS (m/z), MeCN: 1878.7 [M + Na]⁺, 1194.6 [M - (4-phenyl-2-(trifluoroacetyl)benzo[f]quinolin-3(4H)-onato) - (triphenylphosphine oxide)]⁺.

### (EXAMPLE 9)

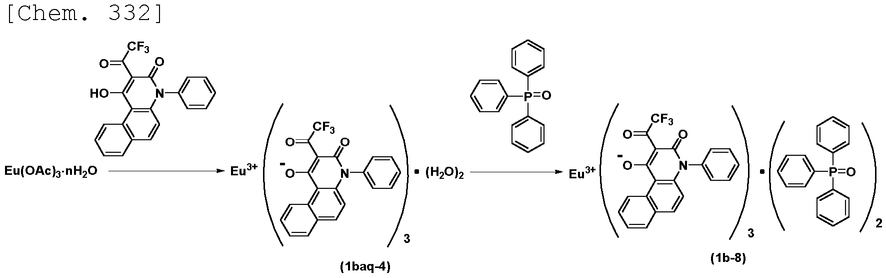

Ethanol (113 mL) and water (37.5 mL) were added to europium(III) acetate hydrate (1.13 g as a 2.5-hydrate, 3.01 mmol) and the 1-hydroxy-4-phenyl-2-(2,2,2-trifluoroethan-1-on-1-yl)benzo[f]quinolin-3(4H)-one obtained in Reference Example 7 (3.45 g, 9.00 mmol). Then a 1 M aqueous solution of sodium hydroxide (9.00 mL, 9.00 mmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give a yellow solid of diaquatris[1-hydroxy-4-phenyl-2-(2,2,2-trifluoroethan-1-on-1-yl)benzo[f]quinolin-1(4H)-onato]europium(III) (1baq-4) (actual yield, 3.72 g; percent yield, 930). The resulting crude product was used in the next reaction directly, without further purification. ¹⁹F-NMR (376 MHz, Acetone-d₆) δ (ppm): -69.6 (brs, 3.4F), -70.0 (brs, 1.5F), -70.2 (brs, 0.4F), -70.4 (brs, 0.5F), -70.9 (brs, 0.5F), -71.1 (brs, 0.3F), -73.0 (brs, 1.0F), -73.9 (brs, 1.0F), -74.1 (brs, 0.4F). ESIMS (m/z), MeOH: 1321.3 [M - 2H₂O + Na]⁺.

Acetone (11.3 mL) was added to the diaquatris[1-hydroxy-4-phenyl-2-(2,2,2-trifluoroethan-1-on-1-yl)benzo[f]quinolin-1(4H)-onato]europium(III) (300 mg, 225 µmol) and triphenylphosphine oxide (129 mg, 463 µmol), and the resulting mixture was stirred for 3 hours at room temperature. The reaction mixture was concentrated under reduced pressure, giving a crude product yellow solid. The resulting crude product was reprecipitated with acetone/hexane to give a light yellow solid of tris[1-hydroxy-4-phenyl-2-(2,2,2-trifluoroethan-1-on-1-yl)benzo[f]quinolin-1(4H)-onato]bis(triphenylphosphine oxide)europium(III) (1b-8) (actual yield, 364 mg; percent yield, 87%) . ¹⁹F-NMR (376 MHz, Acetone-d₆) δ (ppm) : -69.6 (brs, 0.8F), -70.0 (brs, 0.3F), -70.2 (brs, 0.1F), -70.4 (brs, 0.1F), -70.9 (brs, 0.1F), -72.1 (brs, 1.4F), -72.4 (brs, 3.5F), -72.8 (brs, 2.7F). ³¹P-NMR (162 MHz, Acetone-d₆) δ (ppm): -87.4 (brs). ESIMS (m/z), MeCN: 1879.4 [M + Na]⁺, 1600.0 [M - (triphenylphosphine oxide) + Na]⁺, 1472.6 [M - (4-phenyl-2-(2,2,2-trifluoroethan-1-on-1-yl)benzo[f]quinolin-1(4H)-onato)]⁺.

### (EXAMPLE 10)

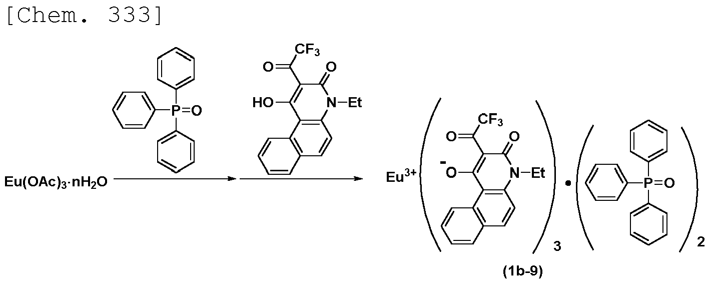

Methanol (4.0 mL) was added to europium acetate n-hydrate (75.0 mg, 200 µmol as a 2.5-hydrate) and triphenylphosphine oxide (111 mg, 40.0 µmol), and the resulting mixture was stirred for 0.5 hours at room temperature. The 4-ethyl-1-hydroxy-2-(2,2,2-trifluoroethan-1-on-1-yl)benzo[f]quinolin-3(4H)-one obtained in Reference Example 19 (201 mg, 600 µmol) was added to the reaction mixture, and the resulting mixture was stirred for 2 hours at room temperature. The reaction solution was emptied into water, and the precipitated solid was collected by suction filtration and then washed with water. This solid was suspended in methylcyclohexane, dehydrated by azeotropic distillation and then allowed to cool, and the solid was collected by suction filtration to give a yellow solid of tris[1-ethyl-3-(2,2,2-trifluoroethan-1-on-1-yl)benzo[f]quinolin-2(1H)-onato]bis(triphenylphosphine oxide)europium(III) (1b-9) (actual yield, 260 mg; percent yield from europium acetate 2.5-hydrate, 76%). ¹⁹F-NMR (376 MHz, Acetone-d₆) δ (ppm): -72.3 (s, 4.1F), -72.6 (s, 2.7F), -72.9 (s, 1.2F), -73.3 (s, 1.0F). ³¹P-NMR (162 MHz, Acetone-d₆) δ (ppm): -86.8 (brs). ESIMS (m/z), MeCN: 1376.6 [M - (1-ethyl-3-(2,2,2-trifluoroethyl-1-on-1-yl)benzo[f]quinolin-2(1H)-onato)]⁺.

### (EXAMPLE 11)

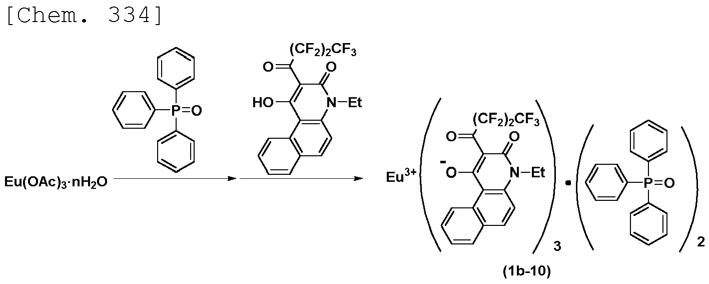

Methylcyclohexane (20.0 mL) was added to europium acetate n-hydrate (75.0 mg, 200 µmol as a 2.5-hydrate) and triphenylphosphine oxide (111 mg, 400 µmol), and the resulting mixture was stirred for 0.5 hours at room temperature. The 4-ethyl-2-(2,2,2,3,3,4,4-heptafluorobutan-1-on-1-yl)-1-hydroxybenzo[f]quinolin-3(4H)-one obtained in Reference Example 20 (261 mg, 600 µmol) to the reaction mixture, and the resulting mixture was stirred for 1 hour with dehydration by azeotropic distillation and with deacetylation. After natural cooling, the precipitated solid was collected by suction filtration. This solid was recrystallized from methylcyclohexane to give a yellow solid of tris[4-ethyl-2-(2,2,2,3,3,4,4-heptafluorobutan-1-on-1-yl)benzo[f]quinolin-3(4H)-onato]bis(triphenylphosphine oxide)europium(III) (1b-10) (actual yield, 95.5 mg; percent yield from europium acetate 2.5-hydrate, 24%). ¹⁹F-NMR (376 MHz, Acetone-d₆) δ (ppm): -80.7 (brs, 9F), -107.8 (brs, 0.7F), -109.1 (brs, 1.5F), -110.8 (brs, 1.4F), -111.9 (brs, 1.9F), -112.7 (brs, 0.5F), -118.6 (brs, 3.8F), -118.9 (brs, 1.7F), -119.6 (brs, 0.5F). ³¹P-NMR (162 MHz, Acetone-d₆) δ (ppm): -93.9 (brs). ESIMS (m/z), MeCN: 2035.3 [M + Na]⁺, 1577.6 [M - (4-ethyl-2-(2,2,2,3,3,4,4-heptafluorobutan-1-on-1-yl)benzo[f]quinolin-3(4H)-onato)]⁺.

### (EXAMPLE 12)

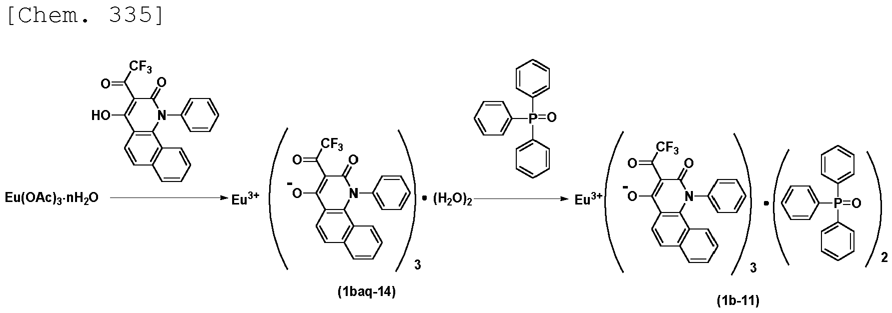

Ethanol (2.48 mL) and water (830 µL) were added to europium(III) acetate hydrate (24.8 mg as a 2.5-hydrate, 66.2 µmol) and the 4-hydroxy-1-phenyl-3-(2,2,2-trifluoroethan-1-on-1-yl)benzo[h]quinolin-2(1H)-one obtained in Reference Example 21 (76.3 mg, 199 µmol). Then a 1 M aqueous solution of sodium hydroxide (199 µL, 199 µmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give a yellow solid of diaquatris[4-hydroxy-1-phenyl-3-(2,2,2-trifluoroethan-1-on-1-yl)benzo[h]quinolin-2(1H)-onato]europium(III) (1baq-14) (actual yield, 73.5 mg; percent yield, 830). The resulting crude product was used in the next reaction directly, without further purification. ¹⁹F-NMR (376 MHz, Acetone-d₆) δ (ppm): -70.8 (brs, 2.7F), -72.1 (brs, 1.1F), -73.4 (brs, 0.4F), -74.1 (brs, 4.0F), -75.2 (brs, 0.8F). ESIMS (m/z), MeOH: 1321.5 [M - 2H₂O + Na]⁺.

Acetone (4.40 mL) was added to the diaquatris[4-hydroxy-1-phenyl-3-(2,2,2-trifluoroethan-1-on-1-yl)benzo[h]quinolin-2(1H)-onato]europium(III) (58.9 mg, 44.1 µmol) and triphenylphosphine oxide (26.9 mg, 96.7 µmol), and the resulting mixture was stirred for 3 hours at room temperature. The reaction mixture was concentrated under reduced pressure, giving a crude product orange-yellow solid. The resulting crude product was reprecipitated with acetone/hexane to give an orange-yellow solid of tris[1-phenyl-3-(2,2,2-trifluoroethan-1-on-1-yl)benzo[h]quinolin-2(1H)-onato]bis(triphenylphosphine oxide)europium(III) (1b-11) (actual yield, 70.9 mg; percent yield, 87%). ¹⁹F-NMR (376 MHz, Acetone-d₆) δ (ppm): -70.7 (brs, 0.7F), -72.0 (brs, 1.4F), -72.1 (brs, 0.5F), -72.2 (brs, 5.6F), -74.1 (brs, 0.2F), -74.3 (brs, 0.6F). ³¹P-NMR (162 MHz, Acetone-d₆) δ (ppm): -84.1 (brs). ESIMS (m/z), MeCN: 1877.2 [M + Na]⁺, 1598.9 [M - (triphenylphosphine oxide) + Ma]⁺, 1743.4 [M - (4-hydroxy-1-phenyl-3-(2,2,2-trifluoroethan-1-on-1-yl)benzo[h]quinolin-2(1H)-onato)]⁺.

### (EXAMPLE 13)

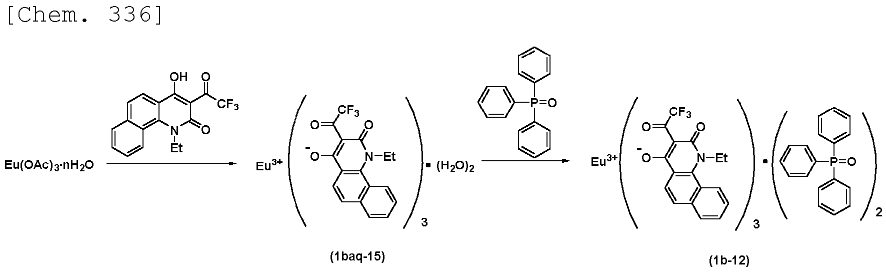

Europium acetate n-hydrate (187 mg as a 2.5-hydrate, 500 µmol) and the 1-ethyl-4-hydroxy-3-(2,2,2-trifluoroethan-1-on-1-yl)benzo[h]quinolin-2(1H)-one obtained in Reference Example 22 (503 mg, 1.50 mmol) were suspended in ethanol (20.0 mL) and water (6.0 mL). Then 1 M sodium hydroxide (1.50 mL, 1.50 mmol) was added, and the resulting mixture was stirred for 3 hours at room temperature. The reaction mixture was concentrated under reduced pressure, and then the resulting solid was washed with water and dried by heating to give a yellow solid of diaquatris[1-ethyl-4-hydroxy-3-(2,2,2-trifluoroethan-1-on-1-yl)benzo[h]quinolin-2(1H)-onato]europium(III) (1baq-15) (actual yield, 460 mg; percent yield, 77%). The resulting crude product was used in the next reaction directly, without further purification. ¹⁹F-NMR (376 MHz, CDC1₃) δ (ppm): -74.0. ESIMS (m/z), MeOH: 1177.9 [M - 2H₂O + Na]⁺.

Acetone (20 mL) was added to the diaquatris[1-ethyl-4-hydroxy-3-(2,2,2-trifluoroethan-1-on-1-yl)benzo[h]quinolin-2(1H)-onato]europium(III) (238 mg, 200 µmol) and triphenylphosphine oxide (111 mg, 400 µmol), and the resulting mixture was stirred for 3 hours at room temperature. The reaction mixture was filtered through Celite, then the filtrate was concentrated under reduced pressure, and the residue was reprecipitated with acetone/hexane to give a yellow solid of tris[1-ethyl-4-hydroxy-3-(2,2,2-trifluoroethan-1-on-1-yl)benzo[h]quinolin-2(1H)-onato]bis(triphenylphosphine oxide)europium(III) (1b-12) (actual yield, 322 mg; percent yield, 94%). ¹⁹F-NMR (376 MHz, CDC1₃) δ (ppm): -71.6 (s, 0.3F), -72.0 (s, 2.3F), -72.5 (s, 4.8F), -74.0 (s, 0.5F), -74.2 (s, 1.1F). ³¹P-NMR (162 MHz, CDCl₃) δ (ppm): -79.7 (brs). ESIMS (m/z), MeOH: 1734.3 [M + Na]⁺.

### (EXAMPLE 14)

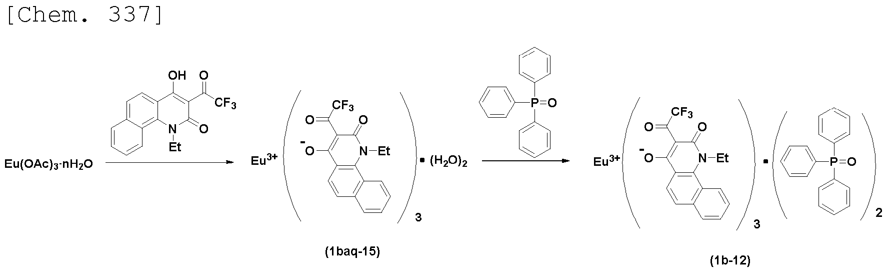

Europium acetate n-hydrate (187 mg as a 2.5-hydrate, 500 µmol) and the 1-ethyl-4-hydroxy-3-(2,2,2-trifluoroethan-1-on-1-yl)benzo[h]quinolin-2(1H)-one obtained in Reference Example 22 (503 mg, 1.50 mmol) were suspended in chloroform (10.0 mL) and water (10.0 mL). Then sodium hydrogencarbonate (126 mg, 1.50 mmol) was added, and the resulting mixture was stirred for 2 hours at room temperature. The reaction mixture was filtered, and the resulting solid was washed with water and then dried by heating to give a yellow solid of diaquatris[1-ethyl-4-hydroxy-3-(2,2,2-trifluoroethan-1-on-1-yl)benzo[h]quinolin-2(1H)-onato]europium(III) (actual yield, 461 mg; percent yield, 770). The resulting crude product was used in the next reaction directly, without further purification. ¹⁹F-NMR (376 MHz, CDC1₃) δ (ppm): -74.0. ESIMS (m/z), MeOH: 1177.9 [M - 2H₂O + Na]⁺.

Acetone (10.0 mL) was added to the diaquatris[1-ethyl-4-hydroxy-3-(2,2,2-trifluoroethan-1-on-1-yl)benzo[h]quinolin-2(1H)-onato]europium(III) (119 mg, 100 µmol) and triphenylphosphine oxide (55.7 mg, 200 µmol), and the resulting mixture was stirred for 3 hours at room temperature. The reaction mixture was filtered through Celite, then the filtrate was concentrated under reduced pressure, and the residue was reprecipitated with acetone/hexane to give a yellow solid of tris[1-ethyl-4-hydroxy-3-(2,2,2-trifluoroethan-1-on-1-yl)benzo[h]quinolin-2(1H)-onato]bis(triphenylphosphine oxide)europium(III) (1b-12) (actual yield, 124 mg; percent yield, 36%). ¹⁹F-NMR (376 MHz, CDC1₃) δ (ppm): -71.6 (s, 0.3F), -72.0 (s, 2.3F), -72.5 (s, 4.8F), -74.0 (s, 0.5F), -74.2 (s, 1.1F). ³¹P-NMR (162 MHz, CDCl₃) δ (ppm): -79.7 (brs). ESIMS (m/z), MeOH: 1734.3 (M + Na) + .

### (EXAMPLE 15)

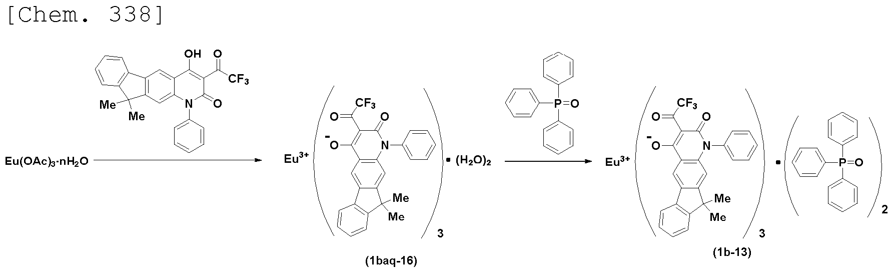

Ethanol (10 mL) and water (3.0 mL) were added to europium acetate n-hydrate (74.0 mg as a 2.5-hydrate, 200 µmol) and the 4-hydroxy-10,10-dimethyl-1-phenyl-3-(2,2,2-trifluoroethyl-1-on-1-yl)-10H-indeno[3,2-g]quinolin-2(1H)-one obtained in Reference Example 23 (267 mg, 600 µmol). Then 1 M sodium hydroxide (600 µL, 600 µmol) was added, and the resulting mixture was stirred for 3 hours at room temperature. The reaction mixture was concentrated under reduced pressure, and the resulting solid was washed with water and then dried by heating to give a yellow solid of diaquatris[4-hydroxy-10,10-dimethyl-1-phenyl-3-(2,2,2-trifluoroethan-1-on-1-yl)-10H-indeno[3,2-g]quinolin-2(1H)-onato]europium(III) (1baq-16) (actual yield, 186 mg; percent yield, 620). The resulting crude product was used in the next reaction directly, without further purification. ¹⁹F-NMR (376 MHz, CDC1₃) δ (ppm): -73.8. ESIMS (m/z), MeOH: 1519.8 [M - 2H₂O + Na]⁺.

Acetone (10.0 mL) was added to the diaquatris[4-hydroxy-10,10-dimethyl-1-phenyl-3-(2,2,2-trifluoroethan-1-on-1-yl)-10H-indeno[3,2-g]quinolin-2(1H)-onato]europium(III) (160 mg, 100 µmol) and triphenylphosphine oxide (58.1 mg, 200 µmol), and the resulting mixture was stirred for 3 hours at room temperature. The reaction mixture was concentrated under reduced pressure, and the residue was reprecipitated with chloroform/hexane to give a yellow solid of tris[4-hydroxy-10,10-dimethyl-1-phenyl-3-(2,2,2-trifluoroethan-1-on-1-yl)-10H-indeno[3,2-g]quinolin-2(1H)-onato]bis(triphenylphosphine oxide)europium(III) (1b-13) (actual yield, 193 mg; percent yield, 90%). ¹⁹F-NMR (376 MHz, CDCl₃) δ (ppm): -71.3 (brs). ³¹P-NMR (162 MHz, CDC1₃) δ (ppm): -85.1 (brs). ESIMS (m/z), MeOH: 1798.5 (M + Na)+.

### (EXAMPLE 16)

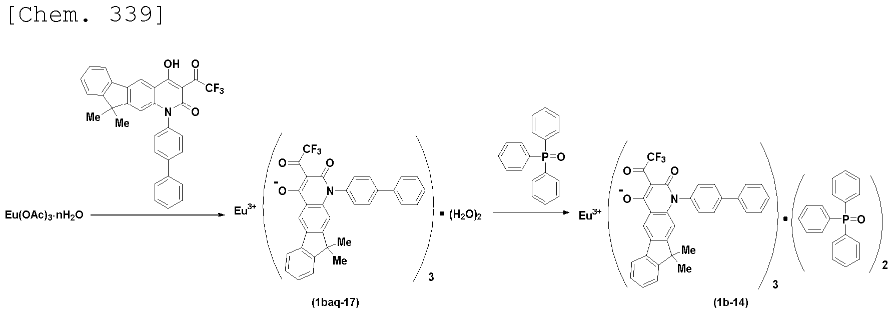

Ethanol (7.5 mL) and water (2.5 mL) were added to europium acetate n-hydrate (74.0 mg as a 2.5-hydrate, 200 µmol) and the 1-(4-biphenylyl)-4-hydroxy-10,10-dimethyl-3-(2,2,2-trifluoroethan-1-on-1-yl)-10H-indeno[3,2-g]quinolin-2(1H)-one obtained in Reference Example 24 (315 mg, 600 µmol). Then 1 M sodium hydroxide (600 µL, 600 µmol) was added, and the resulting mixture was stirred for 3 hours at room temperature. The reaction mixture was allowed to stand for 10 minutes, the precipitated solid was collected by filtration, and then the resulting solid was washed with water and a small amount of ethanol and dried by heating to give a yellow solid of diaquatris[1-(4-biphenylyl)-4-hydroxy-10,10-dimethyl-3-(2,2,2-trifluoroethyl-1-on-1-yl)-10H-indeno[3,2-g]quinolin-2(1H)-onato]europium(III) (1baq-17) (actual yield, 318 mg; percent yield, 90%). The resulting crude product was used in the next reaction directly, without further purification. ¹⁹F-NMR (376 MHz, CDC1₃) δ (ppm): -73.9 (s, 5.OF), -66.5 (s, 4.OF). ESIMS (m/z), MeOH: 1747.3 [M - 2H₂O + Na]⁺.

Acetone (18.0 mL) was added to the diaquatris[1-(4-biphenylyl)-4-hydroxy-10,10-dimethyl-3-(2,2,2-trifluoroethyl-1-on-1-yl)-10H-indeno[3,2-g]quinolin-2(1H)-onato]europium(III) (318 mg, 181 µmol) and triphenylphosphine oxide (101 mg, 362 µmol), and the resulting mixture was stirred for 3 hours at room temperature. The reaction mixture was filtered through Celite, then the filtrate was concentrated under reduced pressure, and the residue was reprecipitated with acetone/hexane to give a yellow solid of tris[1-(4-biphenylyl)-4-hydroxy-10,10-dimethyl-3-(2,2,2-trifluoroethyl-1-on-1-yl)-10H-indeno[3,2-g]quinolin-2(1H)-onato]bis(triphenylphosphine oxide)europium(III) (1b-14) (actual yield, 224 mg; percent yield, 54%). ¹⁹F-NMR (376 MHz, CDCl₃) δ (ppm): -71.3 (s, 6.0F), -71.7 (s, 2.0F), -73.6 (s, 1.0F). ³¹P-NMR (162 MHz, CDC1₃) δ (ppm): -84.0 (brs). ESIMS (m/z), MeOH: 2302.9 [M + Na]⁺.

### (EXAMPLE 17)

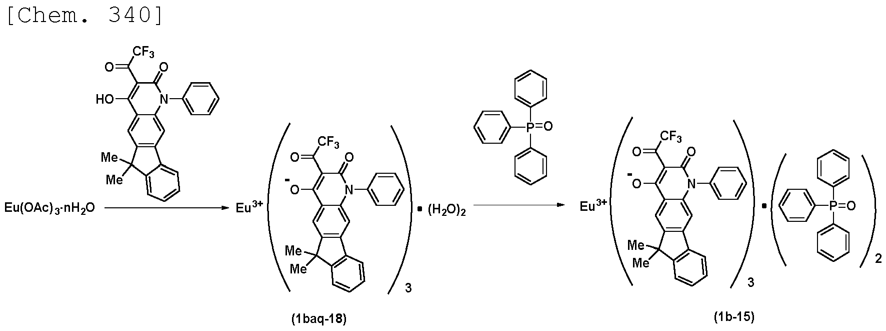

Ethanol (10.5 mL) and water (3.50 mL) were added to europium(III) acetate hydrate (105 mg as a 2.5-hydrate, 281 µmol) and the 1-hydroxy-10,10-dimethyl-4-phenyl-2-(2,2,2-trifluoroethan-1-on-1-yl)-10H-indeno[2,3-g]quinolin-3(4H)-one obtained in Reference Example 11 (382 mg, 849 µmol). Then a 1 M aqueous solution of sodium hydroxide (840 µL, 840 µmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give a light yellow solid of diaquatris[1-hydroxy-10,10-dimethyl-4-phenyl-2-(2,2,2-trifluoroethan-1-on-1-yl)-10H-indeno[2,3-g]quinolin-3(4H)-onato]europium(III) (1baq-18) (actual yield, 386 mg; percent yield, 89%). The resulting crude product was used in the next reaction directly, without further purification. ¹⁹F-NMR (376 MHz, Acetone-d₆) δ (ppm): -69.4 (brs, 4.2F), - 69.7 (brs, 1.8F), -71.2 (brs, 0.4F), -73.0 (brs, 1.4F), - 74.2 (brs, 1.2F). ESIMS (m/z), MeOH: 1519.7 [M - 2H₂O + Na]⁺.

Acetone (9.50 mL) was added to the diaquatris[1-hydroxy-10,10-dimethyl-4-phenyl-2-(2,2,2-trifluoroethan-1-on-1-yl)-10H-indeno[2,3-g]quinolin-3(4H)-onato]europium(III) (145 mg, 94.6 µmol) and triphenylphosphine oxide (53.6 mg, 193 µmol), and the resulting mixture was stirred for 3 hours at room temperature. The reaction mixture was filtered through Celite, and then the filtrate was concentrated under reduce pressure, giving a crude product orange-yellow solid. The resulting crude product was reprecipitated with chloroform/hexane to give a color solid of tris[1-hydroxy-10,10-dimethyl-4-phenyl-2-(2,2,2-trifluoroethan-1-on-1-yl)-10H-indene[2,3-g]quinolin-3 (4H)-onato]bis (triphenylphosphine oxide)europium(III) (1b-15) (actual yield, 102 mg; percent yield, 52%) . ¹⁹F-NMR (376 MHz, CD₃Cl) δ (ppm) : -71.1 (brs, 4.1F), -71.5 (brs, 2.9F), -72.1 (brs, 0.4F), -72.9 (brs, 1.1F), -73.8 (brs, 0.5F). ³¹P-NMR (162 MHz, Acetone-d₆) δ (ppm): -83.7 (brs). ESIMS (m/z), MeCN: 2074.5 [M + Na]⁺, 1797.4 [M - (triphenylphosphine oxide) + Na]⁺, 1605.6 [M - (1-hydroxy-10,10-dimethyl-4-phenyl-2-(2,2,2-trifluoroethan-1-on-1-yl)-10H-indene[2,3-g]quinolin-3(4H)-onato)]⁺.

### (EXAMPLE 18)

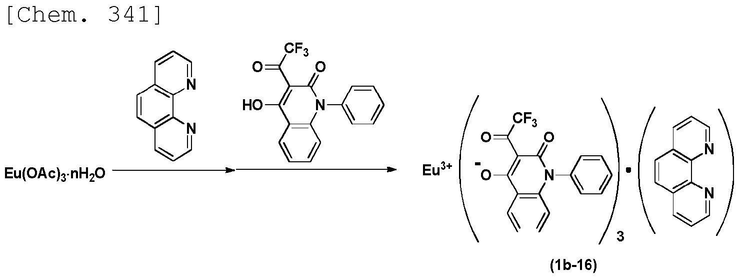

Water (40 mL) and chloroform (40 mL) were added to a mixture of sodium hydrogencarbonate (504 mg, 6.00 mmol) and the 4-hydroxy-1-phenyl-3-(trifluoroacetyl)quinolin-2(1H)-one obtained in Reference Example 1 (2.00 g, 6.00 mmol). Europium acetate n-hydrate (748 mg, 2.00 mmol as a 2.5-hydrate) was added, and the resulting mixture was stirred for 2 hours at room temperature. The reaction mixture was suction-filtered, and the resulting solid was washed with water, giving an intermediate (2.55 g). After methanol (4.0 mL) was added to this solid (237 mg), 1,10-phenanthroline (36.0 mg, 200 µmol) was added, and the resulting mixture was stirred for 16 hours at room temperature. Undissolved solids were collected by suction filtration and washed with methanol. These solids were suspended in methylcyclohexane, dehydrated by azeotropic distillation and then allowed to cool, and the solid was collected by suction filtration to give a slightly yellowish white solid of (1,10-phenanthroline)tris[1-phenyl-3-(trifluoroacetyl)quinolin-2(1H)-on-4-yloxy]europium(III) (1b-16) (actual yield, 177 mg; percent yield from europium acetate 2.5-hydrate, 72%). ESIMS (m/z), MeCN: 1351.5 [M + Na]⁺, 816.9 [M - (4-hydroxy-1-phenyl-3-(trifluoroacetyl)quinolin-2(1H)-onato)]⁺.

### (EXAMPLE 19)

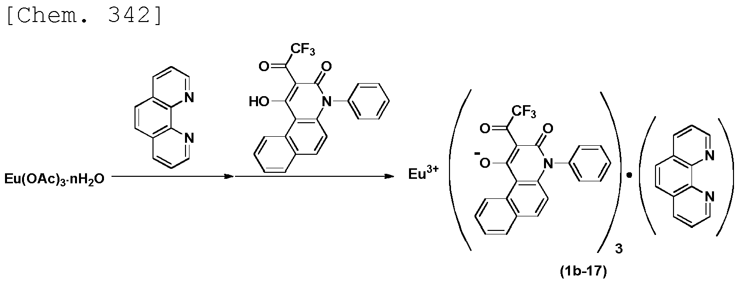

Water (10 mL) and chloroform (10 mL) were added to a mixture of sodium hydrogencarbonate (126 mg, 1.50 mmol) and europium acetate n-hydrate (187 mg, 500 µmol as a 2.5-hydrate). The 1-hydroxy-4-phenyl-2-(trifluoroacetyl)benzo[f]quinolin-3(4H)-one obtained in Reference Example 7 (575 mg, 1.50 mmol) was added, and the resulting mixture was stirred for 2 hours at room temperature. The reaction mixture was suction-filtered, and the resulting solid was washed with water, giving an intermediate (621 mg). This solid (267 mg) was suspended in methanol (4.0 mL), 1,10-phenanthroline (36.0 mg, 200 mol) was added, and the resulting mixture was stirred for 20 hours at room temperature. Undissolved solids were collected by suction filtration and washed with methanol. These solids were suspended in methylcyclohexane, dehydrated by azeotropic distillation and then allowed to cool, and the solid was collected by suction filtration to give a yellow solid of (1,10-phenanthroline)tris[1-hydroxy-4-phenyl-2-(trifluoroacetyl)benzo[f]quinolin-3(4H)-onato]europium(III) (1b-17) (actual yield, 153 mg; percent yield from europium acetate 2.5-hydrate, 48%). ¹⁹F-NMR (376 MHz, Acetone-d₆) δ (ppm): -70.7 (s, 1.38F), -71.1 (s, 1.40F), -71.7 (m, 5.54F), -72.0 (s, 0.68F). ESIMS (m/z), MeCN: 1502.9 [M + Na]⁺, 1097.2 [M - (1-hydroxy-4-phenyl-2-(trifluoroacetyl)benzo[f]quinolin-3(4H)-onato)]⁺, 917.8 [M - (1-hydroxy-4-phenyl-2-(trifluoroacetyl)benzo[f]quinolin-3 (4H)-onato)-1,10-phenanthroline]⁺.

### (EXAMPLE 20)

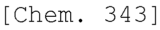

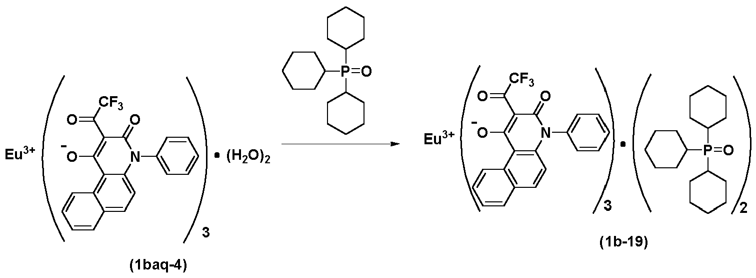

In an argon atmosphere, dehydrated acetone (15.0 mL) was added to the diaquatris[1-hydroxy-4-phenyl-2-(2,2,2-trifluoroethan-1-on-1-yl)benzo[f]quinolin-1(4H)-onato]europium(III) (1baq-4) obtained as an intermediate in Example 9 (203 mg, 152 µmol) and tricyclohexylphosphine oxide (92.2 mg, 311 µmol), and the resulting mixture was stirred for 3 hours at room temperature. The reaction mixture was concentrated under reduced pressure and dried by heating to give an orange-yellow solid of tris[1-hydroxy-4-phenyl-2-(2,2,2-trifluoroethan-1-on-1-yl)benzo[f]quinolin-1(4H)-onato]bis(tricyclohexylphosphine oxide)europium(III) (1b-19) (actual crude yield, 298 mg; percent crude yield, quant.). A measured ESIMS of the resulting solid confirmed the formation of the desired complex. ¹⁹F-NMR (376 MHz, CDCl₃) δ (ppm): -66.8 (brs, 0.6F), -66.9 (brs, 0.8F), -68.5 (brs, 1F), -69.4 (brs, 2.5F), -69.7 (brs, 0.6F), -71.0 (brs, 2.8F), -73.7 (brs, 0.7F). ³¹P-NMR (162 MHz, CDCl₆) δ (ppm): 50.9 (brs), -66.3 (brs). ESIMS (m/z), MeCN: 1913.7 [M + Na]⁺, 1617.4 [M - (tricyclohexylphosphine oxide) + Na]⁺, 1506.6 [M - (1-hydroxy-4-phenyl-2-(2,2,2-trifluoroethan-1-on-1-yl)benzo[f]quinolin-1(4H)-onato)]⁺.

### (EXAMPLE 21)

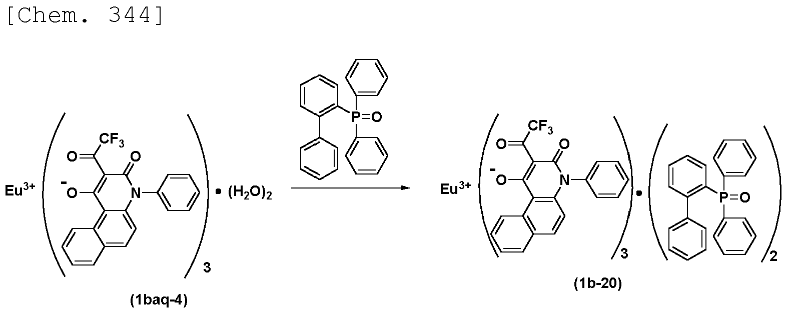

In an argon atmosphere, dehydrated acetone (15.0 mL) was added the diaquatris[1-hydroxy-4-phenyl-2-(2,2,2-trifluoroethan-1-on-1-yl)benzo[f]quinolin-1(4H)-onato]europium(III) (1baq-4) obtained as an intermediate in Example 8 (202 mg, 151 µmol) and (2-biphenylyl)diphenylphosphine oxide (111 mg, 313 µmol), and the resulting mixture was stirred for 3 hours at room temperature. The reaction mixture was concentrated under reduced pressure and dried by heating to give an orange-yellow solid of tris[1-hydroxy-4-phenyl-2-(2,2,2-trifluoroethan-1-on-1-yl)benzo[f]quinolin-1(4H)-onato]bis[(2-biphenylyl)diphenylphosphine oxide]europium(III) (1b-20) (actual crude yield, 320 mg; percent crude yield, quant.). A measured ESIMS of the resulting solid confirmed the formation of the desired complex. ESIMS (m/z), MeCN: 2030.4 [M + Na]⁺, 1676.4 [M - ((2-biphenylyl)diphenylphosphine oxide) + Na]⁺.

### (EXAMPLE 22)

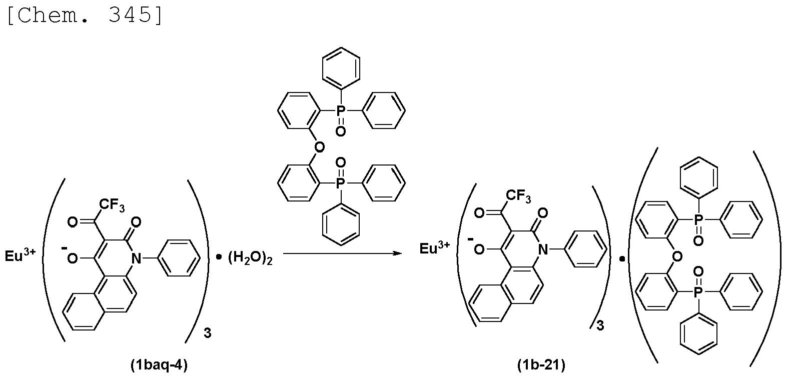

The diaquatris[1-hydroxy-4-phenyl-2-(2,2,2-trifluoroethan-1-on-1-yl)benzo[f]quinolin-1(4H)-onato]europium(III) (1baq-4) obtained as an intermediate in Example 9 (201 mg, 151 µmol) and bis[2-[(oxo)diphenylphosphino]phenyl]ether (87.6 mg, 154 µmol) were dissolved in dehydrated acetone (15.0 mL), and the resulting solution was stirred for 3 hours at room temperature. The reaction mixture was concentrated under reduced pressure and dried by heating. The resulting yellow solid was washed with chloroform (10 mL) and hexane (50 mL) for the removal of slurry. 110 mg of the resulting light yellow solid (222 mg) was washed with chloroform (1 mL) and hexane (7 mL) for further removal of slurry, then washed with hexane (2 mL) and dried by heating to give a light yellow solid of tris[1-hydroxy-4-phenyl-2-(2,2,2-trifluoroethan-1-on-1-yl)benzo[f]quinolin-1(4H)-onato] (bis{2-[(oxo)diphenylphosphino]phenyl}ether)europium(III) (1b-21) (actual yield, 89.9 mg; percent yield, 32%). ¹⁹F-NMR (376 MHz, CDCl₃) δ (ppm): -71.8 (brs, 0.5F), -72.3 (brs, 0.8F), - 72.5 (brs, 2.4F), -72.6 (brs, 1.3F), -73.0 (brs, 2.6F), - 73.5 (brs, 1.1F), -75.2 (brs, 0.3F). ³¹P-NMR (162 MHz, CDCl₆) δ (ppm): -100 (brs). ESIMS (m/z), MeCN: 1891.1 [M + Na]⁺, 1484.6 [M - (1-hydroxy-4-phenyl-2-(2,2,2-trifluoroethan-1-on-1-yl)benzo[f]quinolin-1(4H)-onato)]⁺.

### (EXAMPLE 23)

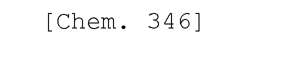

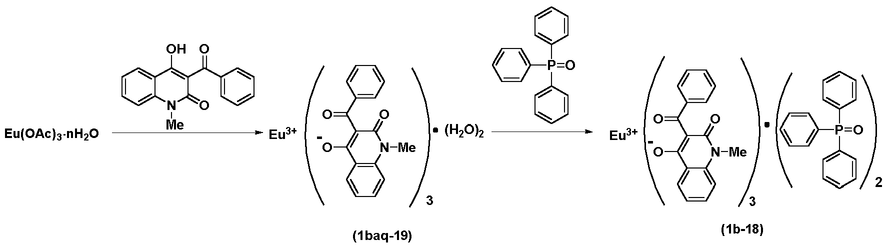

Ethanol (19 mL) and water (6.0 mL) were added to europium acetate n-hydrate (187 mg as a 2.5-hydrate, 500 µmol) and the 3-benzoyl-4-hydroxy-1-methylquinolin-2(1H)-one obtained in Reference Example 9 (419 mg, 1.50 mmol). Then 1 M sodium hydroxide (1.50 mL, 1.50 mmol) was added, and the resulting mixture was stirred for 3 hours at room temperature. The reaction mixture was concentrated under reduced pressure, and the resulting solid was washed with water and then dried by heating to give a yellow solid of diaquatris[3-benzoyl-4-hydroxy-1-methylquinolin-2(1H)-onato]europium(III) (1baq-19) (actual yield, 472 mg; percent yield, 92%). The resulting crude product was used in the next reaction directly, without further purification. ESIMS (m/z), MeOH: 1010.2 [M - 2H₂O + Na]⁺.

Acetone (26 mL) was added to the diaquatris[3-benzoyl-4-hydroxy-1-methylquinolin-2(1H)-onato]europium(III) (262 mg, 260 µmol) and triphenylphosphine oxide (158 mg, 520 µmol), and the resulting mixture was stirred for 3 hours at room temperature. The reaction mixture was concentrated under reduced pressure to give a yellow solid of tris[3-benzoyl-4-hydroxy-1-methylquinolin-2(1H)-onato]bis(triphenylphosphine oxide)europium(III) (1b-18) (actual yield, 420 mg; percent yield, quant.). ³¹P-NMR (162 MHz, CDC1₃) δ (ppm): -75.1 (brs). ESIMS (m/z), MeOH: 1263.5 [M - (3-benzoyl-4-hydroxy-1-methylquinolin-2(1H)-onato)]⁺.

### (EXAMPLE 24)

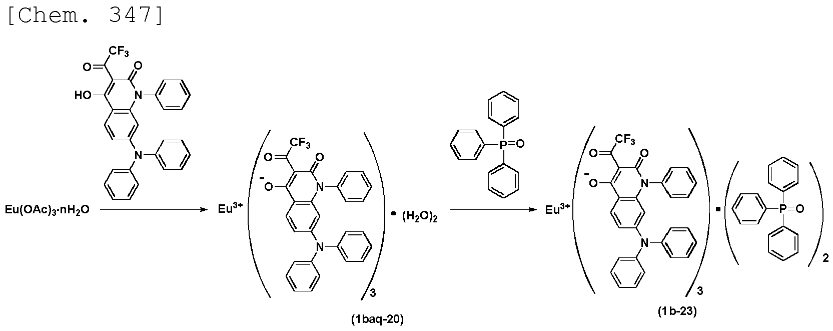

Ethanol (7.50 mL) and water (2.50 mL) were added to europium(III) acetate hydrate (76.8 mg as a 2.5-hydrate, 205 µmol) and the 7-diphenylamino-4-hydroxy-1-phenyl-3-(2,2,2-trifluoroethan-1-on-1-yl)quinolin-2(1H)-one obtained in Reference Example 10 (304 mg, 608 µmol). Then a 1 M aqueous solution of sodium hydroxide (600 µL, 600 µmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give a deep orange powder solid of diaquatris[7-diphenylamino-4-hydroxy-1-phenyl-3-(2,2,2-trifluoroethan-1-on-1-yl)quinolin-2(1H)-onato]europium(III) (1baq-20) (actual yield, 327 mg; percent yield, 94%) . The resulting crude product was used in the next reaction directly, without further purification. ¹⁹F-NMR (376 MHz, Acetone-d₆) δ (ppm): -58.5 (s, 0.4F), -67.7 (s, 0.2F), -70.3 (brs, 3.6F), -70.5 (brs, 2.0F), -71.4 (brs, 0.5F), -72.7 (s, 0.1F), -73.9 (brs, 1.2F), -74.1 (brs, 0.7F), -74.5 (brs, 0.3F). ESIMS (m/z), MeOH: 1673.1 [M - 2H₂O + Na]⁺.

Dehydrated acetone (5.70 mL) was added to the diaquatris[7-diphenylamino-4-hydroxy-1-phenyl-3-(2,2,2-trifluoroethan-1-on-1-yl)quinolin-2(1H)-onato]europium(III) (96.0 mg, 56.9 µmol) and triphenylphosphine oxide (35.2 mg, 126 µmol), and the resulting mixture was stirred for 3 hours at room temperature. The reaction mixture was concentrated under reduced pressure to give an orange-yellow solid of tris[7-diphenylamino-4-hydroxy-1-phenyl-3-(2,2,2-trifluoroethan-1-on-1-yl)quinolin-2(1H)-onato]bis(triphenylphosphine oxide)europium(III) (1b-23) (actual crude yield, 138 mg; percent crude yield, quant.). A measured ESIMS of the resulting solid confirmed the formation of the desired complex. ¹⁹F-NMR (376 MHz, CD₃CN) δ (ppm): -58.6 (s, 0.3F), -67.8 (s, 0.2F), -70.5 (brs, 0.8F), -70.9 (brs, 0.6F), -72.1 (brs, 0.8F), -72.2 (brs, 4.7F), - 72.7 (s, 0.3F), -73.8 (brs, 0.1F), -74.2 (brs, 0.4F), -75.2 (brs, 0.8F). ESIMS (m/z), MeCN: 2228.6 [M + Na]⁺, 1950.8 [M - (triphenylphosphine oxide) + Na]⁺.

### (EXAMPLE 25)

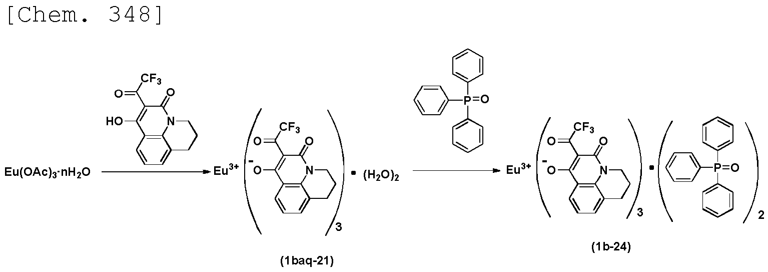

Ethanol (14.0 mL) and water (4.70 mL) were added to europium(III) acetate hydrate (141 mg as a 2.5-hydrate, 376 µmol) and the 6,7-dihydro-1-hydroxy-2-(2,2,2-trifluoroethan-1-on-1-yl)-3H,5H-benzo[ij]quinolizin-3-one obtained in Reference Example 25 (335 mg, 1.13 mmol). Then a 1 M aqueous solution of sodium hydroxide (1.12 mL, 1.12 mmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give a light yellow powder solid of diaquatris[6,7-dihydro-2-(2,2,2-trifluoroethan-1-on-1-yl)-3H,5H-benzo[ij]quinolizin-3-onato]europium(III) (1baq-21) (actual yield, 335 mg; percent yield, 830) . ¹⁹F-NMR (376 MHz, Acetone-d₆) δ (ppm) : -72.9 (brs, 9F). ESIMS (m/z), MeOH: 1063.9 [M - 2H₂O + Na]⁺.

Acetone (9.30 mL) was added to the diaquatris[6,7-dihydro-2-(2,2,2-trifluoroethan-1-on-1-yl)-3H,5H-benzo[ij]quinolizin-3-onato]europium(III) (201 mg, 187 µmol) and triphenylphosphine oxide (105 mg, 379 µmol), and the resulting mixture was stirred for 3 hours at room temperature. The reaction mixture was concentrated under reduced pressure to give an orange-yellow solid of tris[6,7-dihydro-2-(2,2,2-trifluoroethan-1-on-1-yl)-3H,5H-benzo[ij]quinolizin-3-onato]bis(triphenylphosphine oxide)europium(III) (1b-24). (Actual crude yield, 311 mg; percent crude yield, quant.). A measured ESIMS of the resulting solid confirmed the formation of the desired complex. ¹⁹F-NMR (376 MHz, Acetone-d₆) δ (ppm) : -70.2 (brs, 0.7F), 71.7 (brs, 0.5F), -72.0 (s, 2.3F), -72.1 (s, 3.2F), - 73.6 (s, 2.3F) . ³¹P-NMR (162 MHz, Acetone-d₆) δ (ppm) : 28.0 (brs), -82.2 (brs). ESIMS (m/z), MeCN: 1619.8 [M + Na]⁺, 1341.8 [M - (triphenylphosphine oxide) + Na]⁺.

### (EXAMPLE 26)

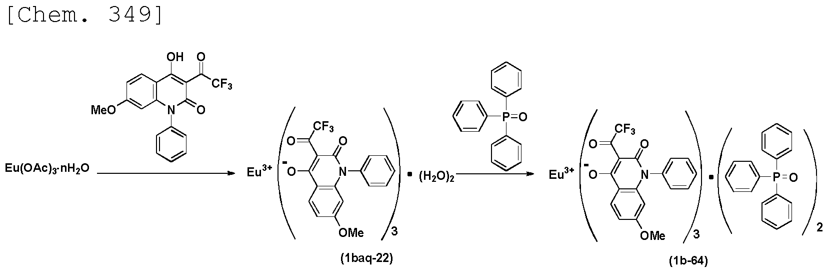

Ethanol (17.2 mL) and water (5.70 mL) were added to europium(III) acetate hydrate (172 mg as a 2.5-hydrate, 459 µmol) and the 4-hydroxy-7-methoxy-1-phenyl-3-(2,2,2-trifluoroethan-1-on-1-yl)quinolin-2(1H)-one obtained in Reference Example 16 (520 mg, 1.38 mmol). Then a 1 M aqueous solution of sodium hydroxide (1.38 mL, 1.38 mmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[4-hydroxy-7-methoxy-1-phenyl-3-(2,2,2-trifluoroethan-1-on-1-yl)quinolin-2(1H)-onato]europium(III) (1baq-22) (actual yield, 459 mg; percent yield, 76%) . The resulting crude product was used in the next reaction directly, without further purification. ¹⁹F-NMR (376 MHz, Acetone-d₆) δ (ppm) : -74.1 (s). ESIMS (m/z), MeOH: 1259.9 [M - 2H₂O + Na]⁺.

Acetone (20 mL) was added to the diaquatris[4-hydroxy-7-methoxy-1-phenyl-3-(2,2,2-trifluoroethan-1-on-1-yl)quinolin-2(1H)-onato]europium(III) (263 mg, 200 µmol) and triphenylphosphine oxide (111 mg, 400 µmol), and the resulting mixture was stirred for 3 hours at room temperature. The reaction mixture was filtered through Celite, then the filtrate was concentrated under reduced pressure, and the residue was reprecipitated with acetone/hexane to give an orange-yellow solid of tris[4-hydroxy-7-methoxy-1-phenyl-3-(2,2,2-trifluoroethan-1-on-1-yl)quinolin-2(1H)-onato]bis(triphenylphosphine oxide)europium(III) (1b-64) (actual yield, 321 mg; percent yield, 87%). ¹⁹F-NMR (376 MHz, CDCl₃) δ (ppm) : -71.8 (s, 0.8F), -72.3 (s, 6.2F), -74.5 (brs, 1.0F). ³¹P-NMR (162 MHz, CDC1₃) δ (ppm) : -78.6 (brs). ESIMS (m/z), MeOH: 1432.2 [M - (4-hydroxy-7-methoxy-1-phenyl-3-(2,2,2-trifluoroethan-1-on-1-yl)quinolin-2(1H)-onato)]⁺.

### (EXAMPLE 27)

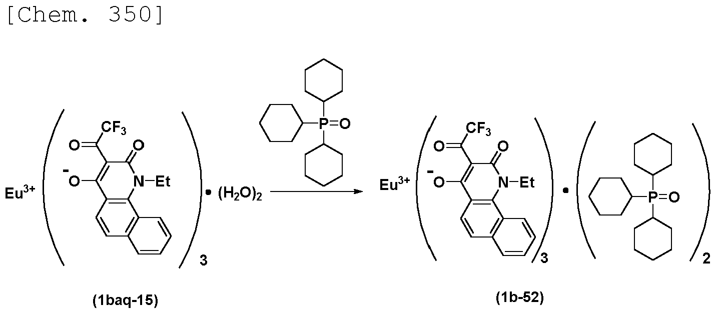

Acetone (20.0 mL) was added to the diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (238 mg, 200 µmol) and tricyclohexylphosphine oxide (120 mg, 400 µmol), and the resulting mixture was stirred for 3 hours at room temperature. The reaction mixture was filtered through Celite, then the filtrate was concentrated under reduced pressure, and the residue was reprecipitated with acetone/hexane to give a yellow solid of tris[1-ethyl-4-hydroxy-3-(2,2,2-trifluoroethan-1-on-1-yl)benzo[h]quinolin-2(1H)-onato]bis(tricyclohexylphosphine oxide)europium(III) (1b-52) (actual yield, 297 mg; percent yield, 85%). ¹⁹F-NMR (376 MHz, CDC1₃) δ (ppm) : -71.2 (s). ³¹P-NMR (162 MHz, CDC1₃) δ (ppm): -66.1 (brs). ESIMS (m/z), MeOH: 1473.9 [M - (tricyclohexylphosphine oxide) + Na]⁺.

### (EXAMPLE 28)

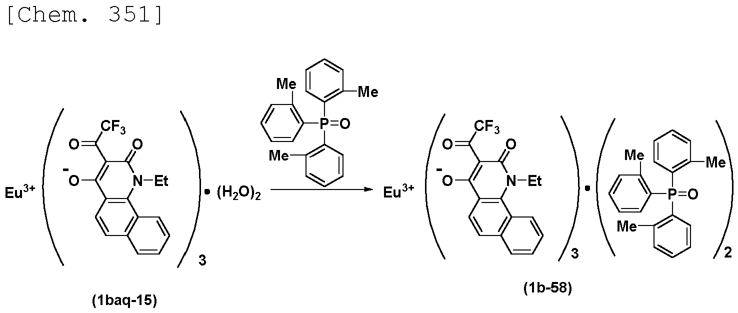

Acetone (20.0 mL) was added to the diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (238 mg, 200 µmol) and tri-o-tolylphosphine oxide (128 mg, 400 µmol), and the resulting mixture was stirred for 3 hours at room temperature. The reaction mixture was filtered through Celite, then the filtrate was concentrated under reduced pressure, and the residue was reprecipitated with acetone/hexane to give a yellow solid of tris[1-ethyl-4-hydroxy-3-(2,2,2-trifluoroethan-1-on-1-yl)benzo[h]quinolin-2(1H)-onato]bis(tri-o-tolylphosphine oxide)europium(III) (1b-58) (actual yield, 237 mg; percent yield, 66%). ¹⁹F-NMR (376 MHz, Acetone-d₆) δ (ppm) : -73.1 (s) . ³¹P-NMR (162 MHz, Acetone-d₆) δ (ppm): -84.3 (brs). ESIMS (m/z), MeOH: 1499.1 [M - (tri-o-tolylphosphine oxide) + Na]⁺.

### (EXAMPLE 29)

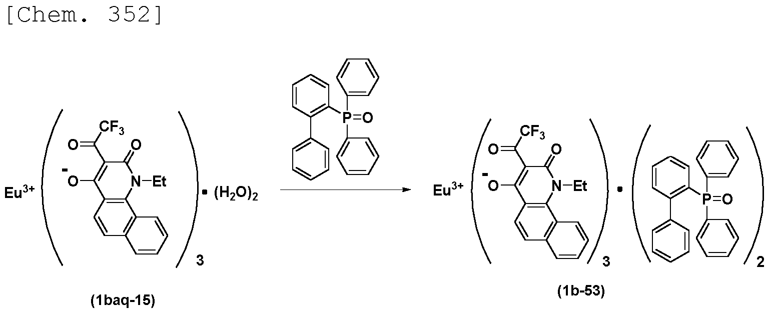

Acetone (20.0 mL) was added to the diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (238 mg, 200 µmol) and 2-biphenylyldiphenylphosphine oxide (142 mg, 400 µmol), and the resulting mixture was stirred for 3 hours at room temperature. The reaction mixture was filtered through Celite, then the filtrate was concentrated under reduced pressure, and the residue was reprecipitated with acetone/hexane to give a yellow solid of tris[1-ethyl-4-hydroxy-3-(2,2,2-trifluoroethan-1-on-1-yl)benzo[h]quinolin-2 (1H)-onato]bis(2-biphenylyldiphenylphosphine oxide)europium(III) (1b-53) (actual yield, 250 mg; percent yield, 67%) . ¹⁹F-NMR (376 MHz, Acetone-d₆) δ (ppm) : -72.0 (s, 2.5F), -72.1 (s, 5.0F), -73.9 (s, 0.5F), -74.1 (s, 1.0F) . ³¹P-NMR (162 MHz, Acetone-d₆) δ (ppm) : -82.6 (brs) . ESIMS (m/z), MeOH: 1884.3 [M + Na]⁺, 1533.9 [M - (2-biphenylyldiphenylphosphine oxide) + Na]⁺.

### (EXAMPLE 30)

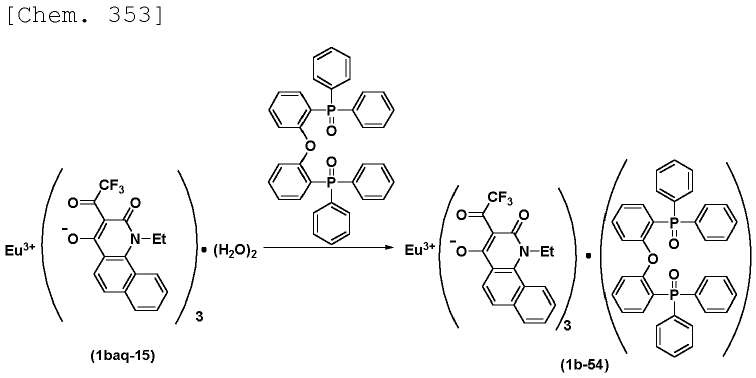

Acetone (20.0 mL) was added to the diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (238 mg, 200 µmol) and bis[2-(diphenylphosphino)phenyl]ether oxide (114 mg, 200 µmol), and the resulting mixture was stirred for 3 hours at room temperature. The reaction mixture was filtered, then the resulting solid was dissolved in chloroform, the resulting solution was filtered through Celite, and then the filtrate was concentrated under reduced pressure to give a yellow solid of [bis[2-(diphenylphosphino)phenyl]ether oxide]tris[1-ethyl-4-hydroxy-3-(2,2,2-trifluoroethan-1-on-1-yl)benzo[h]quinolin-2(1H)-onato]europium(III) (1b-54) (actual yield, 276 mg; percent yield, 80%) . ¹⁹F-NMR (376 MHz, CDCl₃) δ (ppm) : -72.1 (s, 2.5F), -72.6 (s, 5.0F), -74.2 (s, 0.5F), -74.4 (s, 1.0F). ³¹P-NMR (162 MHz, CDCl₃) δ (ppm) : -98.7 (brs, 1.0P), -99.1 (brs, 1.0P). ESIMS (m/z), MeOH: 1749.3 [M + Na]⁺, 1391.0 [M - (1-ethyl-4-hydroxy-3-(2,2,2-trifluoroethan-1-on-1-yl)benzo[h]quinolin-2(1H)-onato)]⁺.

### (EXAMPLE 31)

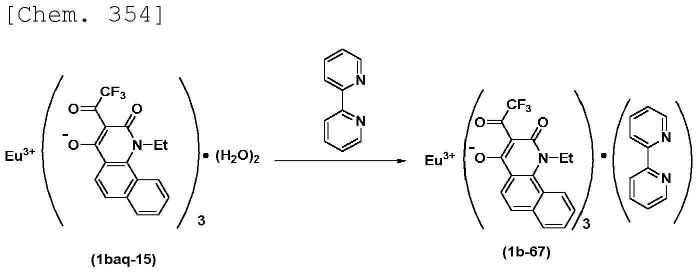

The diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (238 mg, 200 µmol) and 2,2'-bipyridyl (31.2 mg, 200 µmol) were dissolved in acetone (20.0 mL), and the resulting solution was stirred for 3 hours at room temperature. The reaction mixture was filtered through Celite, then the filtrate was concentrated under reduced pressure, and the residue was reprecipitated with acetone/hexane to give a yellow solid of [2,2'-bipyridyl]tris[1-ethyl-4-hydroxy-3-(2,2,2-trifluoroethan-1-on-1-yl)benzo[h]quinolin-2(1H)-onato]europium(III) (1b-67) (actual yield, 96 mg; percent yield, 37%). ¹⁹F-NMR (376 MHz, Acetone-d₆) δ (ppm): -70.1 (s, 2.5F), -70.3 (s, 4.3F), -71.3 (s, 0.9F), -71.6 (s, 1.3F). ESIMS (m/z), MeOH: 1334.9 [M + Na]⁺, 1178.8 [M - (2,2'-bipyridyl) + Na]⁺, 976.7 [M - (1-ethyl-4-hydroxy-3-(2,2,2-trifluoroethan-1-on-1-yl)benzo[h]quinolin-2(1H)-onato)]⁺.

### (EXAMPLE 32)

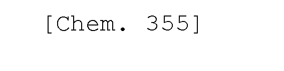

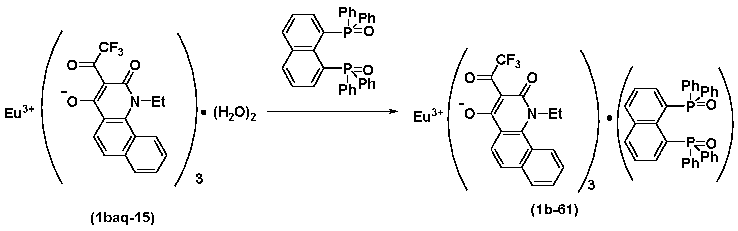

Acetone (20.0 mL) was added to the diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (238 mg, 200 µmol) and naphthalen-1,8-diylbis(diphenylphosphine oxide) (114 mg, 200 µmol), and the resulting mixture was stirred for 3 hours at room temperature. The reaction mixture was filtered through Celite, then the filtrate was concentrated under reduced pressure, and the residue was reprecipitated with acetone/hexane to give a yellow solid of [naphthalen-1,8-diylbis(diphenylphosphine oxide)]tris[1-ethyl-4-hydroxy-3-(2,2,2-trifluoroethan-1-on-1-yl)benzo[h]quinolin-2(1H)-onato]europium(III) (1b-61) (actual yield, 311 mg; percent yield, 92%) . ¹⁹F-NMR (376 MHz, Acetone-d₆) δ (ppm) : -69.9 (s, 2.6F), -70.1 (s, 4.4F), -71.6 (s, 0.7F), -71.7 (s, 1.3F) . ³¹P-NMR (162 MHz, Acetone-d₆) δ (ppm) : -49.5 (brs) . ESIMS (m/z), MeOH: 1707.2 [M + Na]⁺, 1349.0 [M - (1-ethyl-4-hydroxy-3-(2,2,2-trifluoroethan-1-on-1-yl)benzo[h]quinolin-2(1H)-onato)]⁺.

### (EXAMPLE 33)

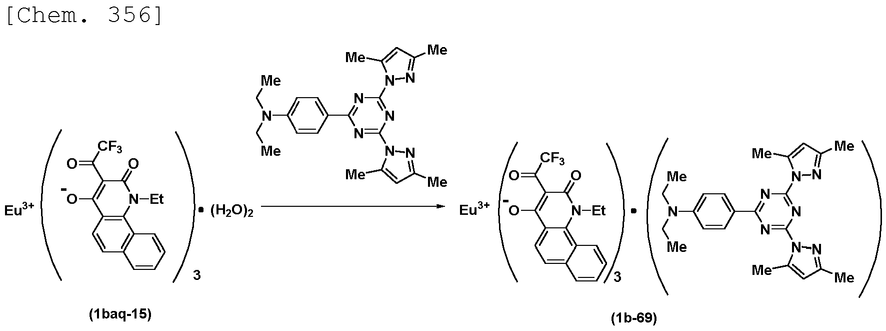

Acetone (20.0 mL) was added to the diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (238 mg, 200 µmol) and the 4-[4,6-bis(3,5-dimethyl-1H-pyrazol-1-yl)-1,3,5-triazin-2-yl]-N,N-diethylaniline obtained in Reference Example 14 (83.3 mg, 200 µmol), and the resulting mixture was stirred for 3 hours at room temperature. The reaction mixture was filtered through Celite, then the filtrate was concentrated under reduced pressure, and the residue was reprecipitated with acetone/hexane to give a yellow solid of {4-[4,6-bis(3,5-dimethyl-1H-pyrazol-1-yl)-1,3,5-triazin-2-yl]-N,N-diethylaniline}tris[1-ethyl-4-hydroxy-3-(2,2,2-trifluoroethan-1-on-1-yl)benzo[h]quinolin-2(1H)-onato]europium(III) (1b-69) (actual yield, 240 mg; percent yield, 76%) . ¹⁹F-NMR (376 MHz, CDCl₃) δ (ppm) : -73.4 (s, 6.2F), -73.8 (s, 1.9F), -74.1 (s, 0.9F). ESIMS (m/z), MeOH: 1178.8 [M - {4-[4,6-bis(3,5-dimethyl-1H-pyrazol-1-yl)-1,3,5-triazin-2-yl]-N,N-diethylaniline} + Na]⁺, 1237.0 [M - (1-ethyl-4-hydroxy-3-(2,2,2-trifluoroethan-1-on-1-yl)benzo[h]quinolin-2(1H)-onato)]⁺.

### (EXAMPLE 34)

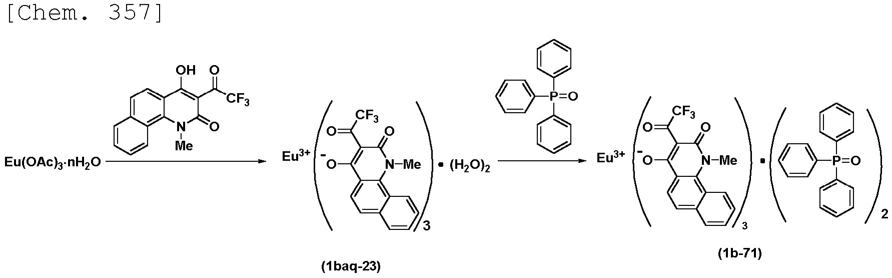

Ethanol (11.3 mL) and water (3.8 mL) were added to europium(III) acetate hydrate (112 mg as a 2.5-hydrate, 300 µmol) and the 4-hydroxy-1-methyl-3-(2,2,2-trifluoroethan-1-on-1-yl)benzo[h]quinolin-2(1H)-one obtained in Reference Example 8 (289 mg, 900 µmol). Then a 1 M aqueous solution of sodium hydroxide (900 µL, 900 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[4-hydroxy-1-methyl-3-(2,2,2-trifluoroethan-1-on-1-yl)benzo[h]quinolin-2(1H)-onato]europium(III) (1baq-23) (actual yield, 294 mg; percent yield, 860). The resulting crude product was used in the next reaction directly, without further purification. ¹⁹F-NMR (376 MHz, Acetone-d₆) δ (ppm) : -73.9 (s) . ESIMS (m/z), MeOH: 1135.9 [M - 2H₂O + Na]⁺.

The diaquatris[4-hydroxy-1-methyl-3-(2,2,2-trifluoroethan-1-on-1-yl)benzo[h]quinolin-2(1H)-onato] europium(III) (230 mg, 200 µmol) and triphenylphosphine oxide (111 mg, 400 µmol) were dissolved in acetone (20 mL), and the resulting solution was stirred for 3 hours at room temperature. The reaction mixture was filtered through Celite, then the filtrate was concentrated under reduced pressure, and the residue was reprecipitated with acetone/hexane to give an orange-yellow solid of tris[4-hydroxy-1-methyl-3-(2,2,2-trifluoroethan-1-on-1-yl)benzo[h]quinolin-2(1H)-onato]bis(triphenylphosphine oxide)europium(III) (1b-71) (actual yield, 310 mg; percent yield, 93%) . ¹⁹F-NMR (376 MHz, Acetone-d₆) δ (ppm) : -72.2 (s, 2.2F), -72.4 (s, 5.6F), -73.5 (brs, 0.4F), -73.7 (brs, 0.8F). ³¹P-NMR (162 MHz, Acetone-d₆) δ (ppm) : -80.4 (brs) . ESIMS (m/z). ESIMS (m/z), MeOH: 1414.8 [M - (triphenylphosphine oxide) + Na]⁺, 1348.6 [M - (4-hydroxy-1-methyl-3-(2,2,2-trifluoroethan-1-on-1-yl)benzo[h]quinolin-2(1H)-onato)]⁺.

### (EXAMPLE 35)

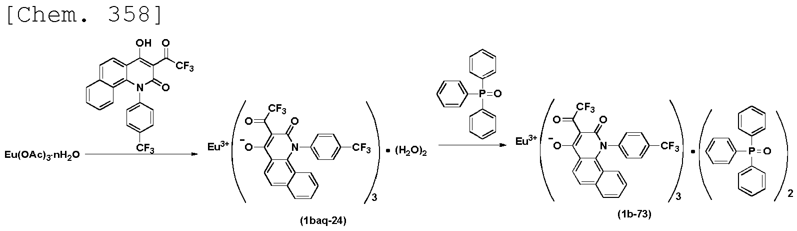

Ethanol (1.6 mL) and water (500 µL) were added to europium(III) acetate hydrate (15.7 mg as a 2.5-hydrate, 41.9 µmol) and the 4-hydroxy-3-(2,2,2-trifluoroethan-1-on-1-yl)-1-[4-(trifluoromethyl)phenyl]benzo[h]quinolin-2(1H)-one obtained in Reference Example 12 (56.8 mg, 126 µmol). Then a 1 M aqueous solution of sodium hydroxide (130 µL, 130 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[4-hydroxy-3-(2,2,2-trifluoroethan-1-on-1-yl)-1-[4-(trifluoromethyl)phenyl]benzo[h]quinolin-2(1H)-onato]europium(III) (1baq-24) (actual yield, 39.1 mg; percent yield, 59%). The resulting crude product was used in the next reaction directly, without further purification. ¹⁹F-NMR (376 MHz, Acetone-d₆) δ (ppm): -63.3 (s, 12.0F), - 74.4 (s, 6.0F). ESIMS (m/z), MeOH: 1525.9 [M - 2H₂O + Na]⁺.

Acetone (2.6 mL) was added to the diaquatris[4-hydroxy-3-(2,2,2-trifluoroethan-1-on-1-yl)-1-[4-(trifluoromethyl)phenyl]benzo[h]quinolin-2(1H)-onato]europium(III) (30.0 mg, 19.5 µmol) and triphenylphosphine oxide (14.5 mg, 52.1 µmol), and the resulting mixture was stirred for 3 hours at room temperature. The reaction mixture was filtered through Celite, then the filtrate was concentrated under reduced pressure, and the residue was reprecipitated with acetone/hexane to give an orange-yellow solid of tris[4-hydroxy-3-(2,2,2-trifluoroethan-1-on-1-yl)-1-[4-(trifluoromethyl)phenyl]benzo[h]quinolin-2(1H)-onato]bis(triphenylphosphine oxide)europium(III) (1b-73) (actual yield, 22 mg; percent yield, 55%). ¹⁹F-NMR (376 MHz, Acetone-d₆) δ (ppm): -63.1 (s, 12.0F), -72.2 (s, 6.0F). ³¹P-NMR (162 MHz, Acetone-d₆) δ (ppm): -86.2 (brs). ESIMS (m/z), MeOH: 2082.2 [M + Na]⁺, 1804.0 [M - (triphenylphosphine oxide) + Na]⁺, 1609.7 [M - {4-hydroxy-3-(2,2,2-trifluoroethan-1-on-1-yl)-1-[4-(trifluoromethyl)phenyl]benzo[h]quinolin-2(1H)-onato}]⁺.

### (EXAMPLE 36)

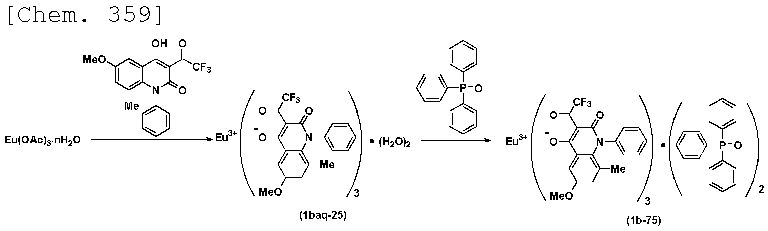

Ethanol (7.5 mL) and water (2.5 mL) were added to europium(III) acetate hydrate (75.0 mg as a 2.5-hydrate, 200 µmol) and the 4-hydroxy-6-methoxy-8-methyl-1-phenyl-3-(2,2,2-trifluoroethan-1-on-1-yl)quinolin-2(1H)-one obtained in Reference Example 17 (181 mg, 600 µmol). Then a 1 M aqueous solution of sodium hydroxide (600 µL, 600 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[4-hydroxy-6-methoxy-8-methyl-1-phenyl-3-(2,2,2-trifluoroethan-1-on-1-yl)quinolin-2(1H)-onato]europium(III) (1baq-25) (actual yield, 144 mg; percent yield, 660). The resulting crude product was used in the next reaction directly, without further purification. ¹⁹F-NMR (376 MHz, Acetone-d₆) δ (ppm): -73.9 (s). ESIMS (m/z), MeOH: 1303.7 [M - 2H₂O + Na]⁺.

Acetone (20 mL) was added to the diaquatris[4-hydroxy-6-methoxy-8-methyl-1-phenyl-3-(2,2,2-trifluoroethan-1-on-1-yl)quinolin-2(1H)-onato]europium(III) (263 mg, 200 µmol) and triphenylphosphine oxide (111 mg, 400 µmol), and the resulting mixture was stirred for 3 hours at room temperature. The reaction mixture was filtered through Celite, then the filtrate was concentrated under reduced pressure, and the residue was reprecipitated with acetone/hexane to give an orange-yellow solid of tris[4-hydroxy-6-methoxy-8-methyl-1-phenyl-3-(2,2,2-trifluoroethan-1-on-1-yl)quinolin-2(1H)-onato]bis(triphenylphosphine oxide)europium(III) (1b-75) (actual yield, 321 mg; percent yield, 87%) . ¹⁹F-NMR (376 MHz, Acetone-d₆) δ (ppm) : -71.5 (s, 0.8F), -71.6 (s, 1.2F), -71.7 (brs, 0.6F), -72.0 (brs, 3.7F), -72.1 (brs, 1.4F), -74.7 (brs, 1.3F). ³¹P-NMR (162 MHz, Acetone-d₆) δ (ppm): -84.0 (brs). ESIMS (m/z), MeOH: 1581.7 [M - (triphenylphosphine oxide) + Na]⁺.

### (EXAMPLE 37)

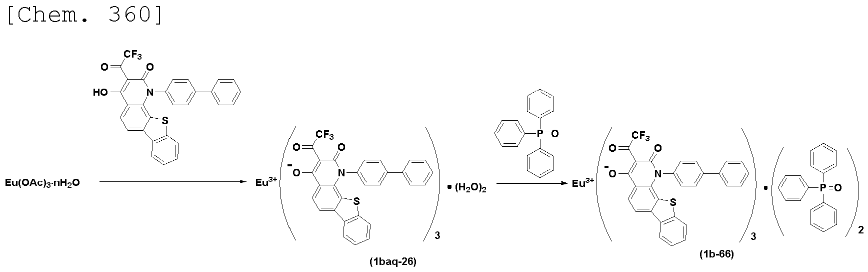

Ethanol (7.5 mL) and water (2.5 mL) were added to europium(III) acetate hydrate (74.0 mg as a 2.5-hydrate, 200 µmol) and the 1-(4-biphenylyl)-4-hydroxy-3-(2,2,2-trifluoroethan-1-on-1-yl)-[1]benzothieno[3,2-h]quinolin-2(1H)-one obtained in Reference Example 18 (305 mg, 600 µmol). Then a 1 M aqueous solution of sodium hydroxide (600 µL, 600 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[1-(4-biphenylyl)-4-hydroxy-3-(2,2,2-trifluoroethan-1-on-1-yl)-[1]benzothieno[3,2-h]quinolin-2(1H)-onato]europium(III) (1baq-26) (actual yield, 320 mg; percent yield, 93%). The resulting crude product was used in the next reaction directly, without further purification. ¹⁹F-NMR (376 MHz, Acetone-d₆) δ (ppm): -74.0 (s). ESIMS (m/z), MeOH: 1719.5 [M - 2H₂O + Na]⁺.

The diaquatris[1-(4-biphenylyl)-4-hydroxy-3-(2,2,2-trifluoroethan-1-on-1-yl)-[1]benzothieno[3,2-h]quinolin-2(1H)-onato]europium(III) (251 mg, 145 µmol) and triphenylphosphine oxide (80.4 mg, 289 µmol) were dissolved in acetone (14 mL), and the resulting solution was stirred for 3 hours at room temperature. The reaction mixture was filtered through Celite, then the filtrate was concentrated under reduced pressure, and the residue was reprecipitated with acetone/hexane to give an orange-yellow solid of tris[1-(4-biphenylyl)-4-hydroxy-3-(2,2,2-trifluoroethan-1-on-1-yl)-[1]benzothieno[3,2-h]quinolin-2(1H)-onato] bis(triphenylphosphine oxide)europium(III) (1b-66) (actual yield, 302 mg; percent yield, 93%). ¹⁹F-NMR (376 MHz, CDCl₃) δ (ppm): -71.4 (s, 2.4F), -71.8 (s, 5.4F), -74.6 (brs, 1.2F). ³¹P-NMR (162 MHz, CDCl₃) δ (ppm): -80.8 (brs). ESIMS (m/z), MeOH: 1996.2 [M - (triphenylphosphine oxide) + Na]⁺, 1736.3 [M - (1-(4-biphenylyl)-4-hydroxy-3-(2,2,2-trifluoroethan-1-on-1-yl)-[1]benzothieno[3,2-h]quinolin-2(1H)-onato)]⁺.

### (EXAMPLE 38)

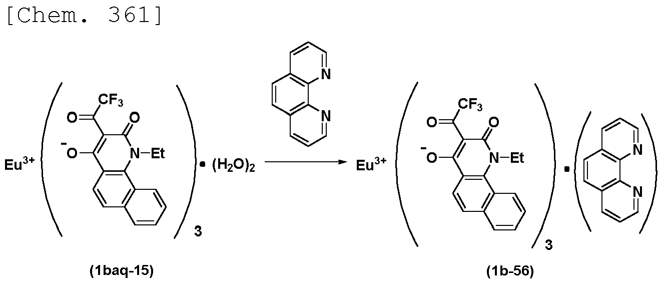

Acetone (10.0 mL) was added to the diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (125 mg, 105 µmol) and 1,10-phenanthroline (18.0 mg, 100 µmol), and the resulting mixture was stirred for 3 hours at 50°C. The reaction mixture was filtered through Celite, then the filtrate was concentrated under reduced pressure, and the residue was reprecipitated with acetone/hexane to give a yellow solid of [1,10-phenanthroline]tris[1-ethyl-4-hydroxy-3-(2,2,2-trifluoroethan-1-on-1-yl)benzo[h]quinolin-2(1H)-onato]europium(III) (1b-56) (actual yield, 85.6 mg; percent yield, 61%) . ¹⁹F-NMR (376 MHz, Acetone-d₆) δ (ppm) : -72.1 (brs). ESIMS (m/z), MeOH: 1356.4 [M+Na]⁺, 1177.4 [M - (1,10-phenanthroline) + Na]⁺, 1000.2 [M - (1-ethyl-4-hydroxy-3-(2,2,2-trifluoroethan-1-on-1-yl)benzo[h]quinolin-2(1H)-onato)]⁺.

### (EXAMPLE 39)

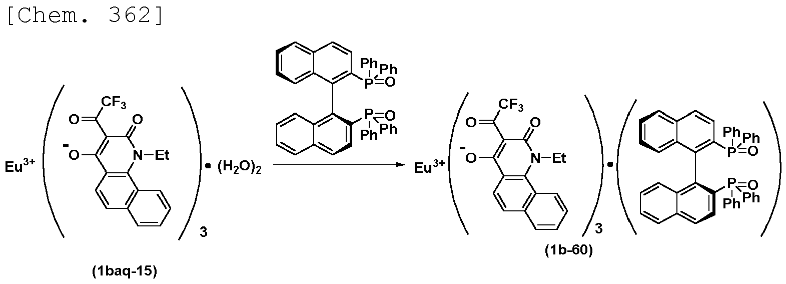

Acetone (20.0 mL) was added to the diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (238 mg, 200 µmol) and (S)-[1,1'-binaphthalen]-2,2'-diylbis(diphenylphosphine oxide) (131 mg, 200 µmol), and the resulting mixture was stirred for 3 hours at 50°C. The reaction mixture was filtered through Celite, then the filtrate was concentrated under reduced pressure, and the residue was reprecipitated with acetone/hexane to give a yellow solid of [(S)-[1,1'-binaphthalene]-2,2'-diylbis(diphenylphosphine oxide)]tris[1-ethyl-4-hydroxy-3-(2,2,2-trifluoroethan-1-on-1-yl)benzo[h]quinolin-2(1H)-onato]europium(III) (1b-60) (actual yield, 160 mg; percent yield, 44%) . ¹⁹F-NMR (376 MHz, Acetone-d₆) δ (ppm) : -70.1 (s, 2.7F), -70.3 (s, 4.4F), -71.3 (brs, 0.7F), -71.6 (brs, 1.2F) . ³¹P-NMR (162 MHz, Acetone-d₆) δ (ppm) : -67.5 (brs, 1P), -68.6 (brs, 1P). ESIMS (m/z), MeOH: 1831.0 [M+Na]⁺, 1474.7 [M - (1-ethyl-4-hydroxy-3-(2,2,2-trifluoroethan-1-on-1-yl)benzo[h]quinolin-2(1H)-onato)]⁺.

### (EXAMPLE 40)

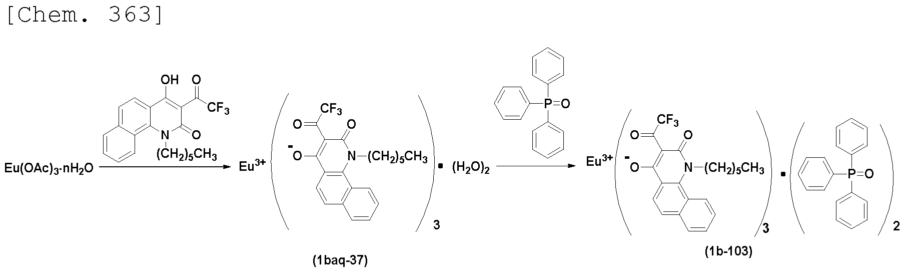

Ethanol (5.19 mL) and water (1.73 mL) were added to europium(III) acetate hydrate (64.8 mg as a 2.5-hydrate, 173 µmol) and the 1-hexyl-4-hydroxy-3-(2,2,2-trifluoroethan-1-on-1-yl)-benzo[h]quinolin-2(1H)-one obtained in Reference Example 26 (203 mg, 519 µmol). Then a 1 M aqueous solution of sodium hydroxide (519 µL, 519 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating under reduced pressure to give a yellow powder solid of diaquatris[1-hexyl-4-hydroxy-3-(2,2,2-trifluoroethan-1-on-1-yl)-benzo[h]quinolin-2(1H)-onato]europium(III) (1baq-37) (actual yield, 182 mg; percent yield, 77%). The resulting crude product was used in the next reaction directly, without further purification. ¹⁹F-NMR (376 MHz, Acetone-d₆) δ (ppm): -70.6 (s, 6.0F), -71.9 (s, 3.0F). ESIMS (m/z), MeOH: 1345.8 [M - 2H₂O + Na]⁺.

Acetone (12.1 mL) was added to the diaquatris[1-hexyl-4-hydroxy-3-(2,2,2-trifluoroethan-1-on-1-yl)-benzo[h]quinolin-2(1H)-onato]europium(III) (164 mg, 121 µmol) and triphenylphosphine oxide (67.3 mg, 242 µmol), and the resulting mixture was stirred for 4 hours at room temperature. The reaction mixture was filtered through Celite, then the filtrate was concentrated under reduced pressure, and the residue was reprecipitated with acetone/hexane to give a yellow solid of tris[1-hexyl-4-hydroxy-3-(2,2,2-trifluoroethan-1-on-1-yl)-benzo[h]quinolin-2(1H)-onato]bis(triphenylphosphine oxide)europium(III) (1b-103) (actual yield, 35 mg; percent yield, 15%). ¹⁹F-NMR (376 MHz, Acetone-d₆) δ (ppm): -70.5 (brs, 1.5F), -71.8 (brs, 0.7F), -72.1 (s, 2.2F), -72.2 (s, 3.2F), -73.7 (s, 0.5F), - 74.1 (s, 0.9) . ³¹P-NMR (162 MHz, Acetone-d₆) δ (ppm) : -86.2 (brs). ESIMS (m/z), MeOH: 1903.7 [M+Na]⁺, 1624.7 [M - (triphenylphosphine oxide) + Na]⁺.

### (EXAMPLE 41)

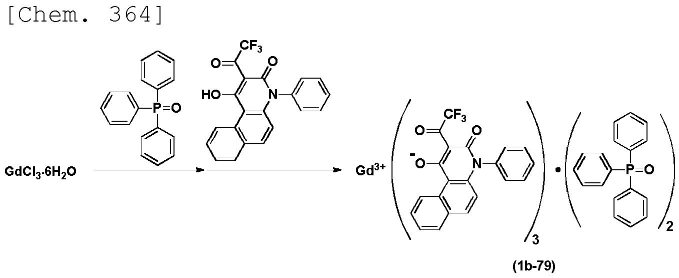

Gadolinium(III) chloride hexahydrate (39.0 mg, 104 µmol) and triphenylphosphine oxide (58.3 mg, 209 µmol) were put into acetone (2 mL), and the resulting mixture was stirred for 1 hour at room temperature. Then the 1-hydroxy-4-phenyl-2-(trifluoroacetyl)benzo[f]quinolin-3(4H)-one obtained in Reference Example 7 (120 mg, 313 µmol) and a 14.8 M aqueous solution of ammonia (21 µL, 313 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[4-phenyl-2-(trifluoroacetyl)benzo[f]quinolin-3(4H)-onato]bis(triphenylphosphine oxide)gadolinium(III) (1b-79). (Actual yield, 83.8 mg; percent yield, 43%). ESIMS (m/z), MeOH: 1604.5 [M - (triphenylphosphine oxide) + Na]⁺, 1478.8 [M - (4-phenyl-2-(trifluoroacetyl)benzo[f]quinolin-3(4H)-onato)]⁺.

### (EXAMPLE 42)

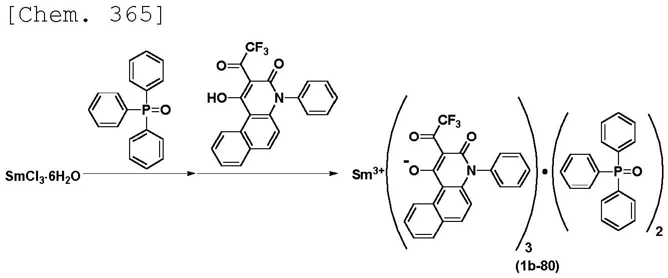

Samarium(III) chloride hexahydrate (75.3 mg, 0.230 mmol) and triphenylphosphine oxide (128 mg, 459 µmol) were put into acetone (4.6 mL), and the resulting mixture was stirred for 1 hour at room temperature. Then the 1-hydroxy-4-phenyl-2-(trifluoroacetyl)benzo[f]quinolin-3(4H)-one obtained in Reference Example 7 (264 mg, 689 µmol) and a 14.8 M aqueous solution of ammonia (50 µL, 740 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[4-phenyl-2-(trifluoroacetyl)benzo[f]quinolin-3(4H)-onato]bis(triphenylphosphine oxide)samarium(III) (1b-80). (Actual yield, 52.6 mg; percent yield, 120). ESIMS (m/z), MeOH: 1599.2 [M - (triphenylphosphine oxide) + Na]⁺.

### (EXAMPLE 43)

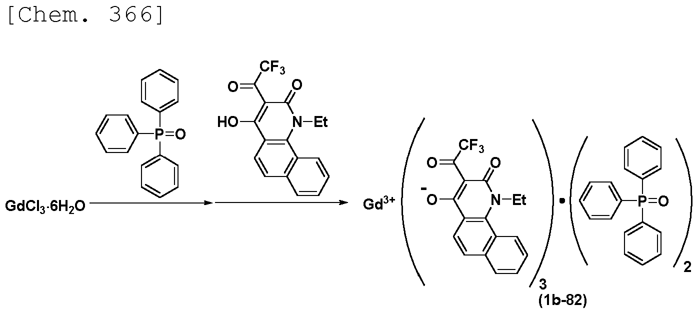

Gadolinium(III) chloride hexahydrate (20.7 mg, 55.7 pmmol) and triphenylphosphine oxide (31.3 mg, 112 µmol) were put into acetone (1.0 mL), and the resulting mixture was stirred for 1 hour at room temperature. Then the 1-ethyl-4-hydroxy-3-(2,2,2-trifluoroethan-1-on-1-yl)benzo[h]quinolin-2(1H)-one obtained in Reference Example 22 (56.3 mg, 168 µmol) and a 14.8 M aqueous solution of ammonia (12 µL, 178 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[1-ethyl-4-hydroxy-3-(2,2,2-trifluoroethan-1-on-1-yl)benzo[h]quinolin-2(1H)-onato]bis(triphenylphosphine oxide)gadolinium(III) (1b-82). (Actual yield, 44.5 mg; percent yield, 47%). ESIMS (m/z), MeOH: 1460.9 [M - (triphenylphosphine oxide) + Na]⁺.

### (EXAMPLE 44)

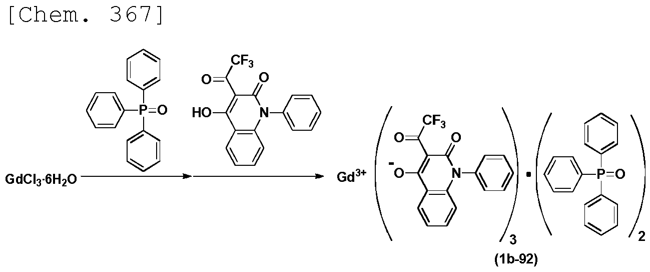

Gadolinium(III) chloride hexahydrate (38.7 mg, 104 µmol) and triphenylphosphine oxide (58.3 mg, 210 µmol) were put into acetone (2.0 mL), and the resulting mixture was stirred for 1 hour at room temperature. Then the 4-hydroxy-1-phenyl-3-(trifluoroacetyl)quinolin-2(1H)-one obtained in Reference Example 1 (105 mg, 315 µmol) and a 14.8 M aqueous solution of ammonia (22 µL, 326 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[1-phenyl-3-(trifluoroacetyl)quinolin-2(1H)-on-4-yloxy]bis(triphenylphosphine oxide)gadolinium(III) (1b-92). (Actual yield, 109 mg; percent yield, 610). ESIMS (m/z), MeOH: 1455.3 [M - (triphenylphosphine oxide) + Na]⁺, 1377.8 [M - (1-phenyl-3-(trifluoroacetyl)quinolin-2(1H)-onato)]⁺.

### (EXAMPLE 45)

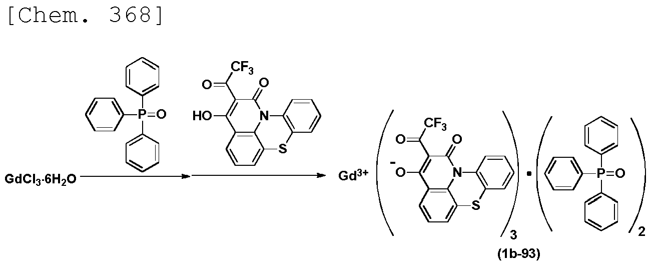

Gadolinium(III) chloride hexahydrate (32.6 mg, 87.7 µmol) and triphenylphosphine oxide (48.8 mg, 175 µmol) were put into acetone (2.0 mL), and the resulting mixture was stirred for 1 hour at room temperature. Then the 3-hydroxy-2-(2,2,2-trifluoroethyl-1-on-1-yl)-1H-pyrido[3,2,1-kl]phenothiazin-1-one obtained in Reference Example 5 (95.6 mg, 263 µmol) and a 14.8 M aqueous solution of ammonia (18 µL, 266 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[3-hydroxy-2-(2,2,2-trifluoroacetyl)-1H-pyrido[3,2,1-kl]phenothiazin-1-onato]bis(triphenylphosphine oxide)gadolinium(III) (1b-93). (Actual yield, 103 mg; percent yield, 65%). ESIMS (m/z), MeOH: 1542.8 [M - (triphenylphosphine oxide) + Na]⁺, 1438.0 [M - (3-hydroxy-2-(2,2,2-trifluoroacetyl)-1H-pyrido[3,2,1-kl]phenothiazin-1-onato)]⁺.

### (EXAMPLE 46)

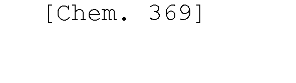

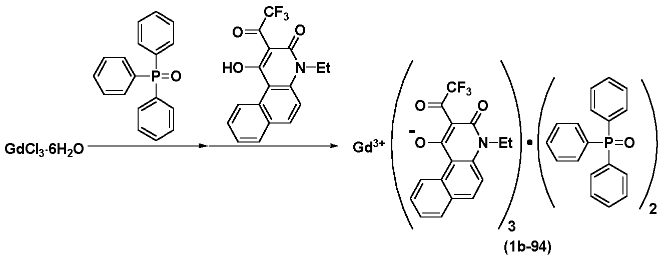

Gadolinium(III) chloride hexahydrate (19.6 mg, 52.7 µmol) and triphenylphosphine oxide (29.7 mg, 107 µmol) were put into acetone (1.0 mL), and the resulting mixture was stirred for 1 hour at room temperature. Then the 4-ethyl-1-hydroxy-2-(2,2,2-trifluoroethan-1-on-1-yl)benzo[f]quinolin-3(4H)-one obtained in Reference Example 19 (53.7 mg, 160 µmol) and a 14.8 M aqueous solution of ammonia (11 µL, 163 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[1-ethyl-3-(2,2,2-trifluoroethan-1-on-1-yl)benzo[f]quinolin-2(1H)-onato]bis(triphenylphosphine oxide)gadolinium(III) (1b-94). (Actual yield, 42.6 mg; percent yield, 47%). ESIMS (m/z), MeOH: 1461.8 [M - (triphenylphosphine oxide) + Na]⁺.

### (EXAMPLE 47)

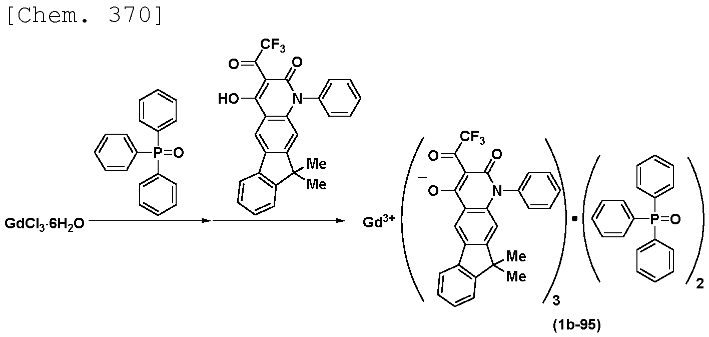

Gadolinium(III) chloride hexahydrate (13.5 mg, 36.3 pmmol) and triphenylphosphine oxide (20.4 mg, 73.3 pmmol) were put into acetone (1.0 mL), and the resulting mixture was stirred for 1 hour at room temperature. Then the 4-hydroxy-10,10-dimethyl-1-phenyl-3-(2,2,2-trifluoroethyl-1-on-1-yl)-10H-indeno[3,2-g]quinolin-2(3H)-one obtained in Reference Example 23 (50.1 mg, 111 µmol) and a 14.8 M aqueous solution of ammonia (7.5 µL, 111 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[4-hydroxy-10,10-dimethyl-1-phenyl-3-(2,2,2-trifluoroethyl-1-on-1-yl)-10H-indeno[3,2-g]quinolin-2(3H)-onato]bis(triphenylphosphine oxide)gadolinium(III) (1b-95). (Actual yield, 60.2 mg; percent yield, 80%). ESIMS (m/z), MeOH: 1803.2 [M - (triphenylphosphine oxide) + Na]⁺, 1610.0 [M - (4-hydroxy-10,10-dimethyl-1-phenyl-3-(2,2,2-trifluoroethyl-1-on-1-yl)-10H-indeno[3,2-g]quinolin-2(3H)-onato)]⁺.

### (EXAMPLE 48)

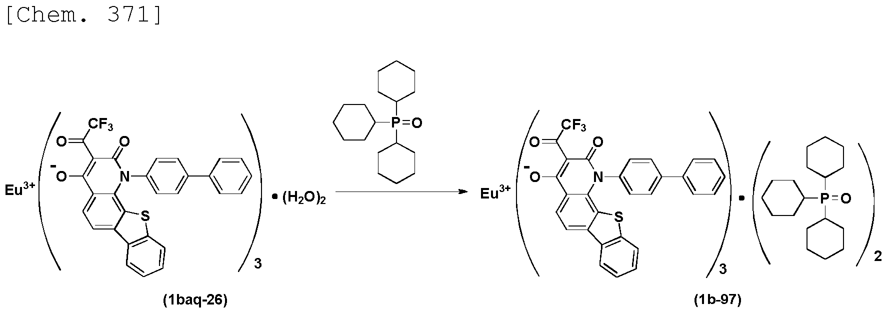

The diaquatris[1-(4-biphenylyl)-4-hydroxy-3-(2,2,2-trifluoroethan-1-on-1-yl)-[1]benzothieno[3,2-h]quinolin-2(1H)-onato]europium(III) (1baq-26) obtained as the intermediate in Example 37 (57.0 mg, 32.9 µmol) and tricyclohexylphosphine oxide (20.0 mg, 67.5 µmol) were dissolved in acetone (6.0 mL), and the resulting solution was stirred for 3 hours at room temperature. The reaction mixture was filtered through Celite, then the filtrate was concentrated under reduced pressure, and the residue was reprecipitated with acetone/hexane to give an orange-yellow solid of tris[1-(4-biphenylyl)-4-hydroxy-3-(2,2,2-trifluoroethan-1-on-1-yl)-[1]benzothieno[3,2-h]quinolin-2(1H)-onato]bis(tricyclohexylphosphine oxide)europium(III) (1b-97) (actual yield, 64.7 mg; percent yield, 86%). ¹⁹F-NMR (376 MHz, Acetone-d₆) δ (ppm): -70.6 (s, 7.1F), -72.6 (s, 1.2F), -75.3 (brs, 0.7F). ³¹P-NMR (162 MHz, Acetone-d₆) δ (ppm): -63.9 (brs). ESIMS (m/z), MeOH: 2306.6 [M+Na]⁺, 2012.6 [M - (tricyclohexylphosphine oxide) + Na]⁺.

### (EXAMPLE 49)

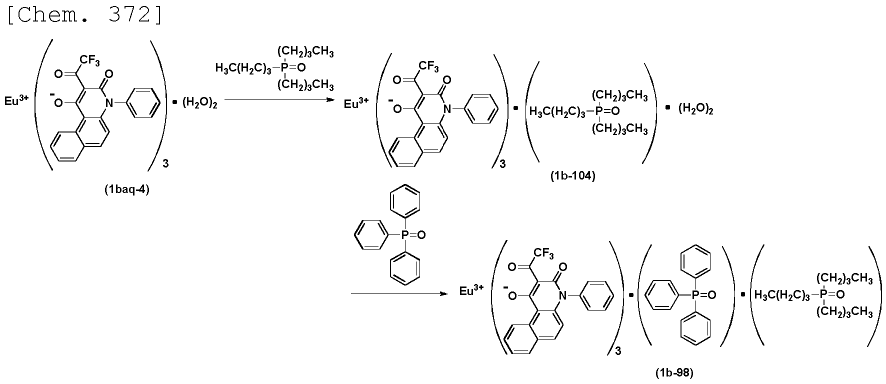

The diaquatris[1-hydroxy-4-phenyl-2-(2,2,2-trifluoroethan-1-on-1-yl)benzo[h]quinolin-1(4H)-onato]europium(III) (1baq-4) obtained as the intermediate in Example 9 (267 mg, 200 µmol) and tributylphosphine oxide (43.7 mg, 200 µmol) were dissolved in acetone (20 mL), and the resulting solution was stirred for 3 hours at room temperature. The reaction mixture was filtered through Celite, and then the filtrate was concentrated under reduced pressure to give a brown solid of aqua(tributylphosphine oxide)tris[1-hydroxy-4-phenyl-2-(2,2,2-trifluoroethan-1-on-1-yl)benzo[h]quinolin-1(4H)-onato]europium(III) (1b-104) (actual yield, 240 mg; percent yield, 780). The resulting crude product was used in the next reaction directly, without further purification. ¹⁹F-NMR (376 MHz, Acetone-d₆) δ (ppm): -70.7 to -71.6 (m, 9F). ³¹P-NMR (162 MHz, Acetone-d₆) δ (ppm): -57.3 (brs). ESIMS (m/z), MeOH: 1539.5 [M - H₂O + Na]⁺, 1134.8 [M - H₂O - (1-hydroxy-4-phenyl-2-(2,2,2-trifluoroethan-1-on-1-yl)benzo[h]quinolin-1(4H)-onato)]⁺.

The aqua(tributylphosphine oxide)tris[1-hydroxy-4-phenyl-2-(2,2,2-trifluoroethan-1-on-1-yl)benzo[h]quinolin-1(4H)-onato]europium(III) (1b-104) (240 mg, 156 µmol) and triphenylphosphine oxide (43.4 mg, 156 µmol) were dissolved in acetone (15 mL), and the resulting solution was stirred for 3 hours at room temperature. The reaction mixture was filtered through Celite, and then the filtrate was concentrated under reduced pressure. The resulting crude product was reprecipitated with diethyl ether/hexane, and then the resulting solid was reprecipitated with acetone/hexane to give a yellow solid of tris[1-hydroxy-4-phenyl-2-(2,2,2-trifluoroethan-1-on-1-yl)benzo[h]quinolin-1(4H)-onato] (triphenylphosphine oxide) (tributylphosphine oxide)europium(III) (1b-98) (actual yield, 98.0 mg; percent yield, 35%) . ¹⁹F-NMR (376 MHz, Acetone-d₆) δ (ppm) : -70.6 to -73.0 (m, 9F) . ³¹P-NMR (162 MHz, Acetone-d₆) δ (ppm) : -58.4 (brs, 1P), -87.7 (brs, 1P). ESIMS (m/z), MeOH: 1818.8 [M + Na]⁺, 1412.7 [M - (1-hydroxy-4-phenyl-2-(2,2,2-trifluoroethan-1-on-1-yl)benzo[h]quinolin-1(4H)-onato)]⁺.

### (EXAMPLE 50)

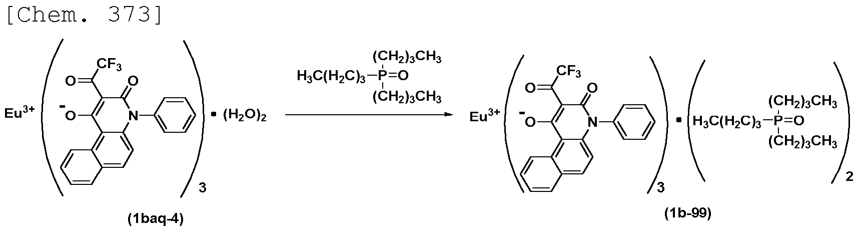

The diaquatris[1-hydroxy-4-phenyl-2-(2,2,2-trifluoroethan-1-on-1-yl)benzo[h]quinolin-1(4H)-onato]europium(III) (1baq-4) obtained as the intermediate in Example 9 (267 mg, 200 µmol) and tributylphosphine oxide (87.3 mg, 400 µmol) were dissolved in acetone (20 mL), and the resulting solution was stirred for 3 hours at room temperature. The reaction mixture was filtered through Celite, then the filtrate was concentrated under reduced pressure, and the resulting crude product was reprecipitated with acetone/hexane to give a yellow solid of tris[1-hydroxy-4-phenyl-2-(2,2,2-trifluoroethan-1-on-1-yl)benzo[h]quinolin-1(4H)-onato]bis(tributylphosphine oxide)europium(III) (1b-99) (actual yield, 156 mg; percent yield, 45%) . ¹⁹F-NMR (376 MHz, Acetone-d₆) δ (ppm) : -70.9 (brs, 3.3F), -71.0 (brs, 1.3F), -71.1 (brs, 2.4F), -71.2 (brs, 2.0F). ³¹P-NMR (162 MHz, Acetone-d₆) δ (ppm) : -57.9 (brs). ESIMS (m/z), MeOH: 1757.6 [M + Na]⁺, 1539.7 [M - (triphenylphosphine oxide) + Ma]⁺, 1352.6 [M - (1-hydroxy-4-phenyl-2-(2,2,2-trifluoroethan-1-on-1-yl)benzo[h]quinolin-1(4H)-onato)]⁺.

### (EXAMPLE 51)

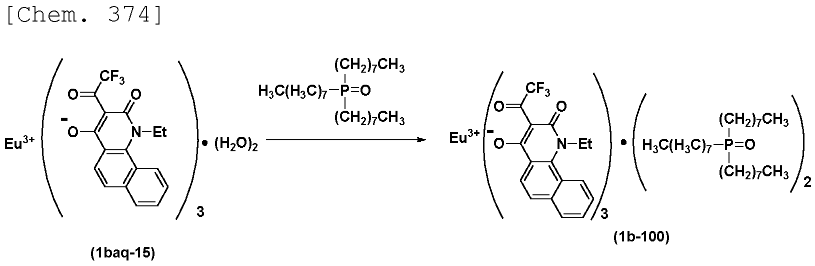

The diaquatris[1-ethyl-4-hydroxy-3-(2,2,2-trifluoroethyl-1-on-1-yl)benzo[h]quinolin-2(1H)-onato]europium(III) (1baq-15) obtained as the intermediate in Example 14 (133 mg, 100 µmol) and trioctylphosphine oxide (77.0 mg, 200 µmol) were dissolved in acetone (10 mL), and the resulting solution was stirred for 3 hours at room temperature. The reaction mixture was filtered through Celite, and then the filtrate was concentrated under reduced pressure. The residue was reprecipitated with acetone/hexane, and then the supernatant was concentrated to give a yellow solid of tris[1-ethyl-4-hydroxy-3-(2,2,2-trifluoroethan-1-on-1-yl)benzo[h]quinolin-2(1H)-onato]bis(trioctylphosphine oxide)europium(III) (1b-100) (actual yield, 155 mg; percent yield, 75%). ¹⁹F-NMR (376 MHz, Acetone-d₆) δ (ppm): -70.9 (s, 4.8F), -71.1 (s, 2.3F), -71.2 (s, 1.2F), -71.3 (s, 0.7F). ³¹P-NMR (162 MHz, Acetone-d₆) δ (ppm): -57.0 (brs). ESIMS (m/z), MeOH: 2095.7 [M + Na]⁺, 1708.1 [M - (trioctylphosphine oxide) + Na]⁺.

### (EXAMPLE 52)

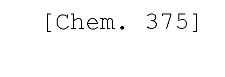

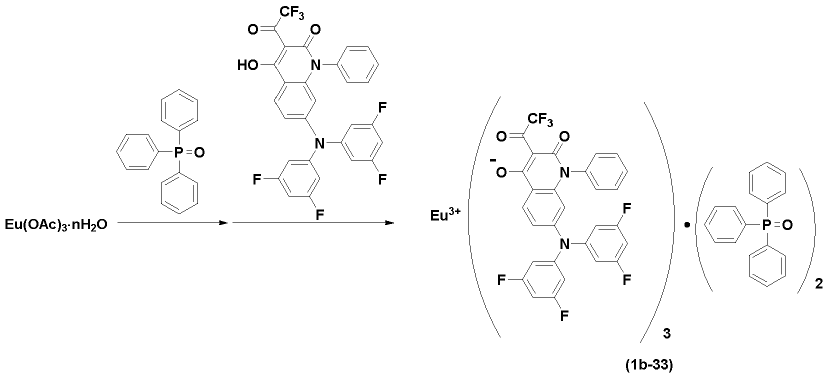

Dichloromethane (2.70 mL) was added to europium acetate n-hydrate (20.1 mg, 53.7 µmol as a 2.5-hydrate) and triphenylphosphine oxide (29.9 mg, 107 µmol), and the resulting mixture was stirred for 1 hour at room temperature. The 7-[bis(3,5-difluorophenyl)amino]-4-hydroxy-1-phenyl-3-(2,2,2-trifluoroethan-1-on-1-yl)quinolin-2(1H)-one obtained in Reference Example 27 (92.1 mg, 160 µmol) was added to the reaction mixture, the resulting mixture was stirred for 1 hour at room temperature and filtered through Celite, and then the filtrate was concentrated under reduced pressure. The resulting crude product was reprecipitated with acetone/hexane to give a yellow solid of tris{7-[bis(3,5-difluorophenyl)amino]-4-hydroxy-1-phenyl-3-(2,2,2-trifluoroethan-1-on-1-yl)quinolin-2(1H)-onato}bis(triphenylphosphine oxide)europium(III) (1b-33) (actual yield, 104 mg; percent yield, 80%). ¹⁹F-NMR (376 MHz, Acetone-d₆) δ (ppm): -71.8 (brs, 9F), -110.0 (brs, 12F) . ³¹P-NMR (162 MHz, Acetone-d₆) δ (ppm) : -87.0 (brs) . ESIMS (m/z), MeOH: 2445.6 [M + Na]⁺, 1849.7 [M - (7-(bis(3,5-difluorophenyl)amino)-4-hydroxy-1-phenyl-3-(2,2,2-trifluoroethan-1-on-1-yl)quinolin-2(1H)-onato)]⁺.

### (EXAMPLE 53)

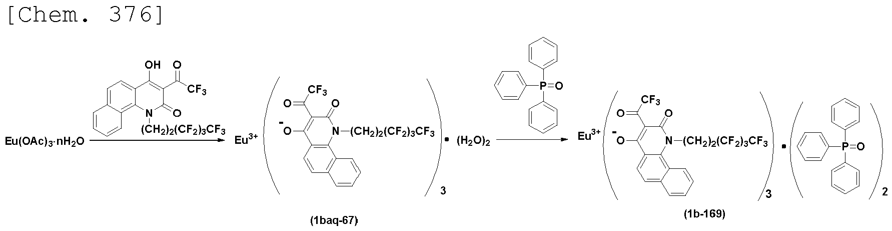

Ethanol (4.88 mL) and water (1.63 mL) were added to europium(III) acetate hydrate (48.7 mg as a 2.5-hydrate, 130 µmol) and the 1-(1H,1H,2H,2H-perfluorohexyl)-4-hydroxy-3-(2,2,2-trifluoroethan-1-on-1-yl)benzo[h]quinolin-2(1H)-one obtained in Reference Example 28 (217 mg, 392 µmol). Then a 1 M aqueous solution of sodium hydroxide (390 µL, 390 µmol) was added, and the resulting mixture was stirred for 3 hours at room temperature. After the ethanol was distilled away, water was put into the reaction mixture, the resulting mixture was filtered, and the collected solid was washed with water. The washed solid was stirred with hexane added to it, then the hexane was distilled away, and the residue was dried by heating under reduced pressure to give a yellow powder solid of diaquatris[1-(1H,1H,2H,2H-perfluorohexyl)-4-hydroxy-3-(2,2,2-trifluoroethan-1-on-1-yl)benzo[h]quinolin-2(1H)-onato]europium(III) (1baq-67) (actual yield, 73.8 mg; percent yield, 31%). The resulting crude product was used in the next reaction directly, without further purification. ¹⁹F-NMR (376 MHz, Acetone-d₆) δ (ppm): -71.2 (s, 3.9F), -74.0 (s, 5.1F), -82.2 (s, 9F), -115.7 (s. 6F), -1250.3 (s, 6F), - 127.1 (s, 6F). ESIMS (m/z), MeOH: 1832.8 [M - 2H₂O + Na]⁺.

Acetone (3.6 mL) was added to the diaquatris[1-(1H,1H,2H,2H-perfluorohexyl)-4-hydroxy-3-(2,2,2-trifluoroethan-1-on-1-yl)benzo[h]quinolin-2(1H)-onato]europium(III) (66 mg, 36 µmol) and triphenylphosphine oxide (19.8 mg, 71 µmol), and the resulting mixture was stirred for 3 hours at room temperature. The reaction mixture was filtered through Celite, and then the filtrate was concentrated under reduced pressure and stirred with hexane added to it. Solids were removed by filtration, the hexane was distilled away, and then the residue was reprecipitated with acetone/water to give a yellow solid of tris[1-(1H,1H,2H,2H-perfluorohexyl)-4-hydroxy-3-(2,2,2-trifluoroethan-1-on-1-yl)benzo[h]quinolin-2(1H)-onato]bis(triphenylphosphine oxide)europium(III) (1b-169) (actual yield, 21 mg; percent yield, 25%). ¹⁹F-NMR (376 MHz, Acetone-d₆) δ (ppm): -72.4 (s, 9F), -82.2 (t, J = 9.6 Hz, 9F), -115.2 to -115.5 (m, 6F), -125.1 to -125.3 (m, 6F), - 126.9 to -127.1 (m, 6F) . ³¹P-NMR (162 MHz, Acetone-d₆) δ (ppm): -86.2 (brs). ESIMS (m/z), MeOH: 2389.0 [M + Na]⁺, 2110.9 [M - (triphenylphosphine oxide) + Na]⁺.

### (EXAMPLE 54)

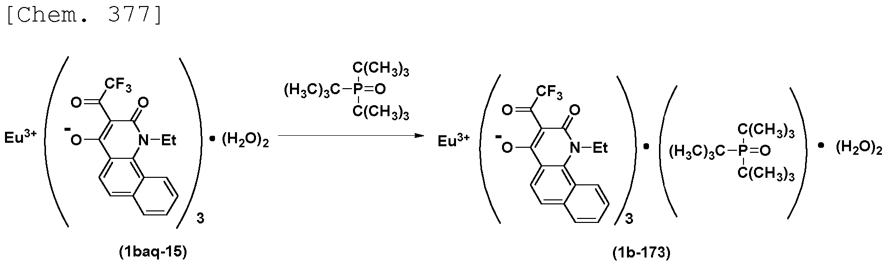

The diaquatris[1-ethyl-4-hydroxy-3-(2,2,2-trifluoroethan-1-on-1-yl)benzo[h]quinolin-2(1H)-onato]europium(III) (1baq-15) obtained as the intermediate in Example 14 (82.0 mg, 68.9 µmol) and tri-t-butylphosphine oxide (15.0 mg, 68.7 µmol) were dissolved in acetone (6.9 mL), and the resulting solution was stirred for 3 hours at room temperature. The reaction mixture was filtered through Celite, then the filtrate was concentrated under reduced pressure, and the resulting solid was washed with hexane to give a yellow solid of aqua(tri-t-butylphosphine oxide)tris[1-ethyl-4-hydroxy-3-(2,2,2-trifluoroethan-1-on-1-yl)benzo[h]quinolin-2(1H)-onato]europium(III) (1b-173) (actual yield, 92.8 mg; percent yield, 970). The resulting crude product was used in the next reaction directly, without further purification. ¹⁹F-NMR (376 MHz, Acetone-d₆) δ (ppm): -67.5 (s, 0.4F), -70.6 (s, 2.3F), -72.0 (s, 5.2F), -73.2 (s, 0.4F), -73.7 (s, 0.7F). ³¹P-NMR (162 MHz, Acetone-d₆) δ (ppm): -54.9 (brs). ESIMS (m/z), MeOH: 1396.3 [M - H₂O + Na]⁺, 1178.1 [M - H₂O - (tri-t-butylphosphine oxide) + Na]⁺.

### (EXAMPLE 55)

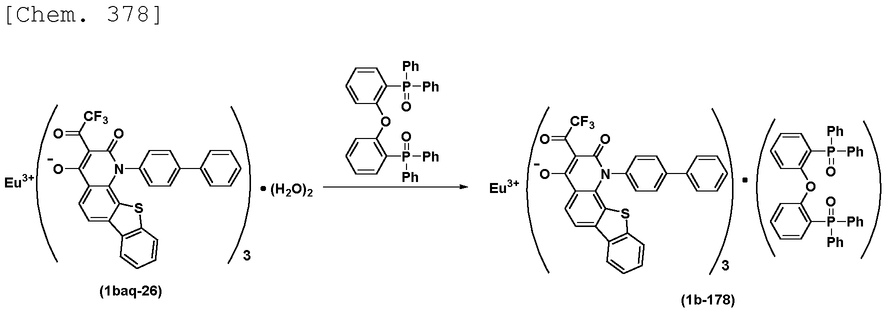

The diaquatris[1-(4-biphenylyl)-4-hydroxy-3-(2,2,2-trifluoroethan-1-on-1-yl)-[1]benzothieno[3,2-h]quinolin-2(1H)-onato]europium(III) (1baq-26) obtained as an intermediate in Example 37 (266 mg, 150 µmol) and bis[2-(diphenylphosphino)phenyl]ether oxide (87.7 mg, 150 µmol) were dissolved in chloroform (15.0 mL), and the resulting solution was stirred for 3 hours at room temperature. The reaction mixture was filtered through Celite, then the filtrate was concentrated under reduced pressure, and the residue was reprecipitated with chloroform/hexane to give a yellow solid of tris[1-(4-biphenylyl)-4-hydroxy-3-(2,2,2-trifluoroethan-1-on-1-yl)-[1]benzothieno[3,2-h]quinolin-2(1H)-onato][bis[2-(diphenylphosphino)phenyl]ether oxide]europium(III) (1b-178) (actual yield, 146 mg; percent yield, 64%) . ¹⁹F-NMR (376 MHz, Acetone-d₆) δ (ppm) : -71.6 (brs) . ³¹P-NMR (162 MHz, Acetone-d₆) δ (ppm) : -105.0 (brs) . ESIMS (m/z), MeOH: 2290.5 [M + Na]⁺, 1753.9 [M - (1-(4-biphenylyl)-4-hydroxy-3-(2,2,2-trifluoroethan-1-on-1-yl)benzo[4,5]thieno[3,2-h]quinolin-2(1H)-onato) + Na]⁺.

### (EXAMPLE 56)

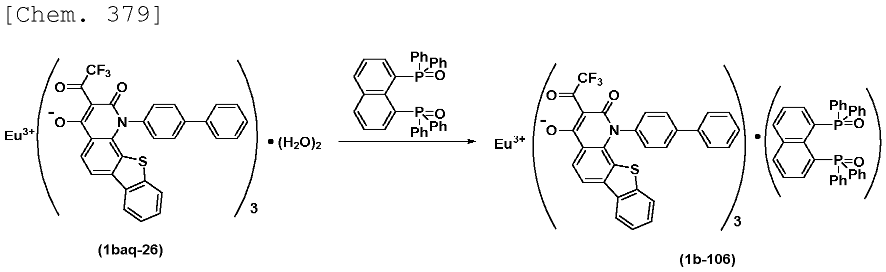

The diaquatris[1-(4-biphenylyl)-4-hydroxy-3-(2,2,2-trifluoroethan-1-on-1-yl)-[1]benzothieno[3,2-h]quinolin-2(1H)-onato]europium(III) (1baq-26) obtained as an intermediate in Example 37 (208 mg, 120 µmol) and naphthalen-1,8-diylbis(diphenylphosphine oxide) (63.5 mg, 120 µmol) were dissolved in acetone (12.0 mL), and the resulting solution was stirred for 3 hours at room temperature. The reaction mixture was filtered through Celite, then the filtrate was concentrated under reduced pressure, and the residue was reprecipitated with chloroform/hexane to give a yellow solid of tris[1-(4-biphenylyl)-4-hydroxy-3-(2,2,2-trifluoroethan-1-on-1-yl)-[1]benzothieno[3,2-h]quinolin-2(1H)-onato][naphthalen-1,8-diylbis(diphenylphosphine oxide)]europium(III) (1b-106) (actual yield, 190 mg; percent yield, 71%). ¹⁹F-NMR (376 MHz, Acetone-d₆) δ (ppm): -68.0 (s, 1.7F), -68.8 (s, 2.4F), -69.2 (s, 4.2F), -72.2 (0.7F). ³¹P-NMR (162 MHz, Acetone-d₆) δ (ppm): -54.9 (brs). ESIMS (m/z), MeOH: 2248.0 [M + Na]⁺, 1709.9 [M - (1-(4-biphenylyl)-4-hydroxy-3-(2,2,2-trifluoroethan-1-on-1-yl)-[1]benzothieno[3,2-h]quinolin-2(1H)-onato) + Na]⁺.

### (EXAMPLE 57)

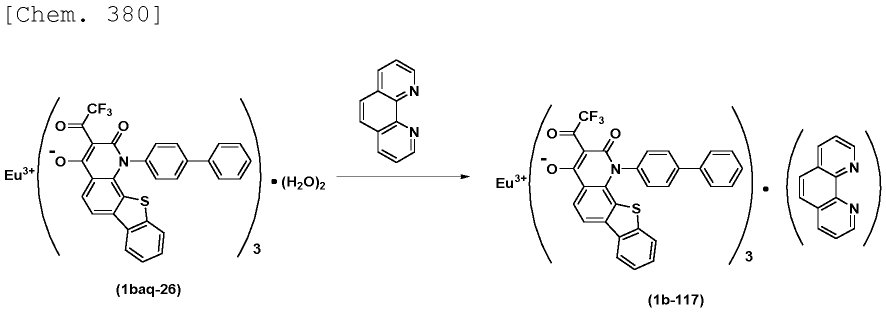

The diaquatris[1-(4-biphenylyl)-4-hydroxy-3-(2,2,2-trifluoroethan-1-on-1-yl)-[1]benzothieno[3,2-h]quinolin-2(1H)-onato]europium(III) (1baq-26) obtained as an intermediate in Example 37 (129 mg, 70 µmol) and 1,10-phenanthroline (13.4 mg, 70 µmol) were dissolved in chloroform (8.0 mL), and the resulting solution was stirred for 3 hours at room temperature. The reaction mixture was filtered through Celite, then the filtrate was concentrated under reduced pressure, and the residue was reprecipitated with chloroform/hexane to give a yellow solid of [1,10-phenanthroline]tris[1-(4-biphenylyl)-4-hydroxy-3-(2,2,2-trifluoroethan-1-on-1-yl)-[1]benzothieno[3,2-h]quinolin-2(1H)-onato]europium(III) (1b-117) (actual yield, 40.4 mg; percent yield, 220) . ¹⁹F-NMR (376 MHz, Acetone-d₆) δ (ppm) : -84.6 (brs). ESIMS (m/z), MeOH: 1899.2 [M + Na]⁺, 1719.8 [M - (1,10-phenanthroline) + Na]⁺.

### (EXAMPLE 58)

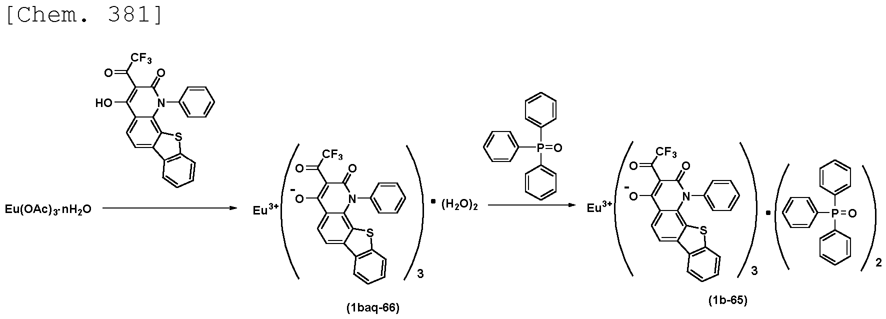

Ethanol (112 mL) and water (37.5 mL) were added to europium(III) acetate hydrate (1.12 g as a 2.5-hydrate, 3.00 mmol) and the 1-phenyl-4-hydroxy-3-(2,2,2-trifluoroethan-1-on-1-yl)-[1]benzothieno[3,2-h]quinolin-2(1H)-one obtained in Reference Example 30 (3.95 g, 9.00 mmol). Then a 1 M aqueous solution of sodium hydroxide (9.00 mL, 9.00 mmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a yellow powder solid of diaquatris[1-phenyl-4-hydroxy-3-(2,2,2-trifluoroethan-1-on-1-yl)-[1]benzothieno[3,2-h]quinolin-2(1H)-onato]europium(III) (1baq-66) (actual yield, 4.28 g; percent yield, 95%). The resulting crude product was used in the next reaction directly, without further purification. ¹⁹F-NMR (376 MHz, Acetone-d₆) δ (ppm): -73.7 (s). ESIMS (m/z), MeOH: 1490.2 [M - 2H₂O+Na]⁺.

The diaquatris[1-phenyl-4-hydroxy-3-(2,2,2-trifluoroethan-1-on-1-yl)-[1]benzothieno[3,2-h]quinolin-2(1H)-onato]europium(III) (1baq-66) (4.27 g, 2.84 mmol) and triphenylphosphine oxide (1.58 g, 5.67 mmol) were dissolved in acetone (284 mL), and the resulting solution was stirred for 3 hours at room temperature. The reaction mixture was filtered, and then the resulting solid was washed with acetone to give a yellow solid of tris[1-phenyl-4-hydroxy-3-(2,2,2-trifluoroethan-1-on-1-yl)-[1]benzothieno[3,2-h]quinolin-2(1H)-onato]bis(triphenylphosphine oxide)europium(III) (1b-65) (actual yield, 3.27 g; percent yield, 57%) . ¹⁹F-NMR (376 MHz, Acetone-d₆) δ (ppm) : -71.5 (s, 2.0F), -71.9 (s, 6.0F), -74.4 (s, 1.0F). ³¹P-NMR (162 MHz, Acetone-d₆) δ (ppm): -81.1 (brs). ESIMS (m/z), MeOH: 2047.1 [M + Na]⁺, 1768.5 [M - (triphenylphosphine oxide) + Na]⁺, 1585.5 [M - (1-phenyl-4-hydroxy-3-(2,2,2-trifluoroethan-1-on-1-yl)-[1]benzothieno[3,2-h]quinolin-2(1H)-onato)]⁺.

### (EXAMPLE 59)

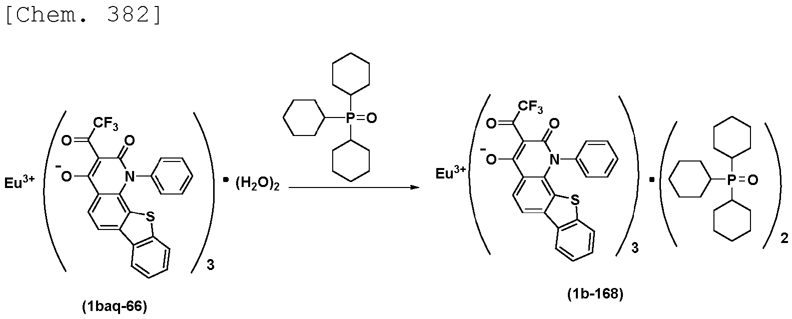

The diaquatris[1-phenyl-4-hydroxy-3-(2,2,2-trifluoroethan-1-on-1-yl)-[1]benzothieno[3,2-h]quinolin-2(1H)-onato]europium(III) (1baq-66) obtained as an intermediate in Example 58 (100 mg, 67 µmol) and tricyclohexylphosphine oxide (39.4 mg, 133 µmol) were dissolved in acetone (6.70 mL), and the resulting solution was stirred for 3 hours at room temperature. The reaction mixture was filtered through Celite, then the filtrate was concentrated under reduced pressure, and the resulting solid was reprecipitated with acetone/hexane to give a yellow solid of tris[1-phenyl-4-hydroxy-3-(2,2,2-trifluoroethan-1-on-1-yl)-[1]benzothieno[3,2-h]quinolin-2(1H)-onato]bis(tricyclohexylphosphine oxide)europium(III) (1b-168) (actual yield, 81.6 mg; percent yield, 60%). ¹⁹F-NMR (376 MHz, Acetone-d₆) δ (ppm): -70.7 (s, 7.4F), -75.4 (s, 1.6F) . ³¹P-NMR (162 MHz, Acetone-d₆) δ (ppm) : -64.2 (brs) . ESIMS (m/z), MeOH: 2084.2 [M + Na]⁺, 1787.0 [M - (tricyclohexylphosphine oxide) + Na]⁺.

### (EXAMPLE 60)

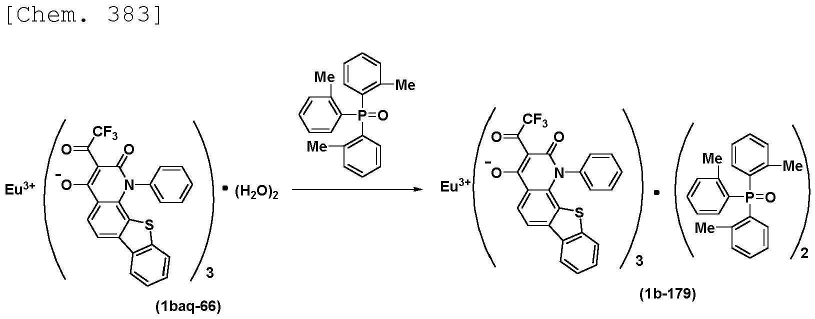

The diaquatris[1-phenyl-4-hydroxy-3-(2,2,2-trifluoroethan-1-on-1-yl)-[1]benzothieno[3,2-h]quinolin-2(1H)-onato]europium(III) (1baq-66) obtained as an intermediate in Example 58 (300 mg, 200 µmol) and tri-o-tolylphosphine oxide (128 mg, 400 µmol) were dissolved in acetone (20.0 mL), and the resulting solution was stirred for 3 hours at room temperature. The reaction mixture was filtered, then the filtrate was concentrated under reduced pressure, and the resulting solid was washed with acetone to give a yellow solid of tris[1-phenyl-4-hydroxy-3-(2,2,2-trifluoroethan-1-on-1-yl)-[1]benzothieno[3,2-h]quinolin-2(1H)-onato]bis(tri-o-tolylphosphine oxide)europium(III) (1b-179) (actual yield, 66.3 mg; percent yield, 16%). ¹⁹F-NMR (376 MHz, CDCl₃) δ (ppm): -71.5 (s, 8.3F), -73.9 (s, 0.7F). ³¹P-NMR (162 MHz, CDCl₃) δ (ppm) : -70.7 (brs) . ESIMS (m/z), MeOH: 1812.5 [M - (tri-o-tolylphosphine oxide) + Na]⁺, 1670.4 [M - (1-phenyl-4-hydroxy-3-(2,2,2-trifluoroethan-1-on-1-yl)-[1]benzothieno[3,2-h]quinolin-2(1H)-onato)]⁺.

### (EXAMPLE 61)

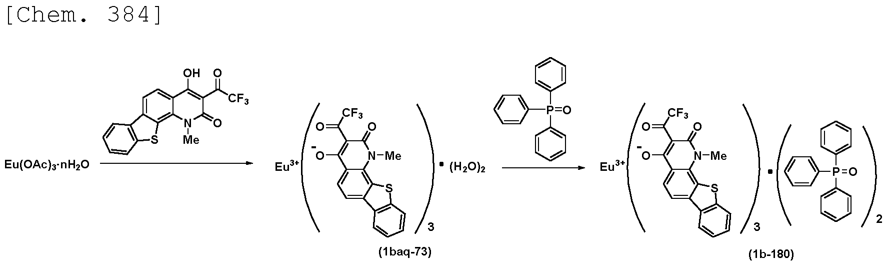

Ethanol (12.0 mL) and water (3.8 mL) were added to europium(III) acetate hydrate (115 mg as a 2.5-hydrate, 307 µmol) and the 1-methyl-4-hydroxy-3-(2,2,2-trifluoroethan-1-on-1-yl)-[1]benzothieno[3,2-h]quinolin-2(1H)-one obtained in Reference Example 32 (347 mg, 921 µmol). Then a 1 M aqueous solution of sodium hydroxide (921 µL, 921 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[1-methyl-4-hydroxy-3-(2,2,2-trifluoroethan-1-on-1-yl)-[1]benzothieno[3,2-h]quinolin-2(1H)-onato]europium(III) (1baq-73) (actual yield, 375 mg; percent yield, 93%). The resulting crude product was used in the next reaction directly, without further purification. ESIMS (m/z), MeOH: 1304.2 [M - 2H₂O + Na]⁺.

The diaquatris[1-methyl-4-hydroxy-3-(2,2,2-trifluoroethan-1-on-1-yl)-[1]benzothieno[3,2-h]quinolin-2(1H)-onato]europium(III) (1baq-73) (132 mg, 100 µmol) and triphenylphosphine oxide (55.8 mg, 200 µmol) were dissolved in acetone (10.0 mL), and the resulting solution was stirred for 3 hours at room temperature. The reaction mixture was filtered through Celite, and then the filtrate was concentrated under reduced pressure. After reprecipitation with acetone/hexane, the precipitated solid was removed by filtration. Hexane was added to the filtrate, and the precipitated solid was collected by filtration to give a yellow solid of tris[1-methyl-4-hydroxy-3-(2,2,2-trifluoroethan-1-on-1-yl)-[1]benzothieno[3,2-h]quinolin-2(1H)-onato]bis(triphenylphosphine oxide)europium(III) (1b-180) (actual yield, 78.2 mg; percent yield, 42%). ¹⁹F-NMR (376 MHz, Acetone-d₆) δ (ppm): -71.9 (brs, 7.5F), -73.3 (brs, 1.6F) . ³¹P-NMR (162 MHz, Acetone-d₆) δ (ppm) : -81.7 (brs). ESIMS (m/z), MeOH: 1860.2 [M + Na]⁺, 1582.1 [M - (triphenylphosphine oxide) + Na]⁺, 1460.2 [M - (1-methyl-4-hydroxy-3-(2,2,2-trifluoroethan-1-on-1-yl)-[1]benzothieno[3,2-h]quinolin-2(1H)-onato)]⁺.

### (EXAMPLE 62)

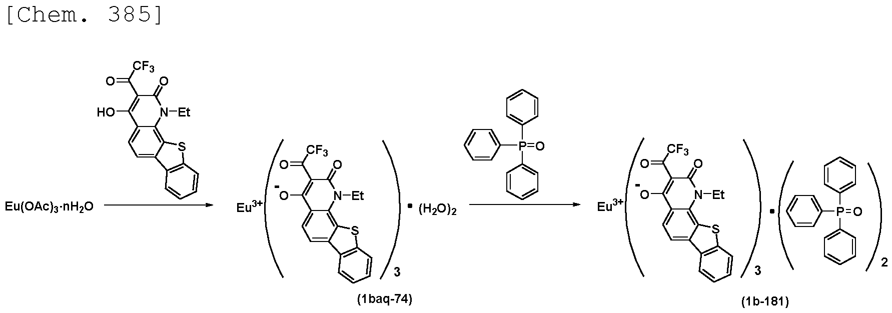

Ethanol (12 mL) and water (3.8 mL) were added to europium(III) acetate hydrate (112 mg as a 2.5-hydrate, 300 µmol) and the 1-ethyl-4-hydroxy-3-(2,2,2-trifluoroethan-1-on-1-yl)-[1]benzothieno[3,2-h]quinolin-2(1H)-one obtained in Reference Example 36 (350.9 mg, 900 µmol). Then a 1 M aqueous solution of sodium hydroxide (900 µL, 900 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a yellow powder solid of diaquatris[1-ethyl-4-hydroxy-3-(2,2,2-trifluoroethan-1-on-1-yl)-[1]benzothieno[3,2-h]quinolin-2(1H)-onato]europium(III) (1baq-74) (actual yield, 385 mg; percent yield, 950). The resulting crude product was used in the next reaction directly, without further purification. ESIMS (m/z), MeOH: 1346.1 [M - 2H₂O + Na]⁺.

The diaquatris[1-ethyl-4-hydroxy-3-(2,2,2-trifluoroethan-1-on-1-yl)-[1]benzothieno[3,2-h]quinolin-2(1H)-onato]europium(III) (1baq-74) (136 mg, 100 µmol) and triphenylphosphine oxide (55.8 mg, 200 µmol) were dissolved in acetone (10.0 mL), and the resulting solution was stirred for 3 hours at room temperature. The reaction mixture was filtered through Celite, then the filtrate was concentrated under reduced pressure, and slurry was washed away by adding hexane (10 mL) and acetone (2 mL). The resulting solid was reprecipitated with acetone/hexane, and then the precipitated solid was removed by filtration. The filtrate was concentrated under reduced pressure, and slurry was washed away with acetone/hexane to give a yellow solid of tris[1-ethyl-4-hydroxy-3-(2,2,2-trifluoroethan-1-on-1-yl)-[1]benzothieno[3,2-h]quinolin-2(1H)-onato] bis(triphenylphosphine oxide)europium(III) (1b-181) (actual yield, 76.0 mg; percent yield, 40%). ¹⁹F-NMR (376 MHz, Acetone-d₆) δ (ppm): -71.7 (brs, 1.6F), -71.9 (brs, 6.0F), -73.8 (brs, 1.4F). ³¹P-NMR (162 MHz, Acetone-d₆) δ (ppm): -86.7 (brs). ESIMS (m/z), MeOH: 1901.4 [M + Na]⁺, 1627.0 [M - (triphenylphosphine oxide) + Na]⁺, 1491.5 [M - (1-ethyl-4-hydroxy-3-(2,2,2-trifluoroethan-1-on-1-yl)-[1]benzothieno[3,2-h]quinolin-2(1H)-onato)]⁺.

### (EXAMPLE 63)

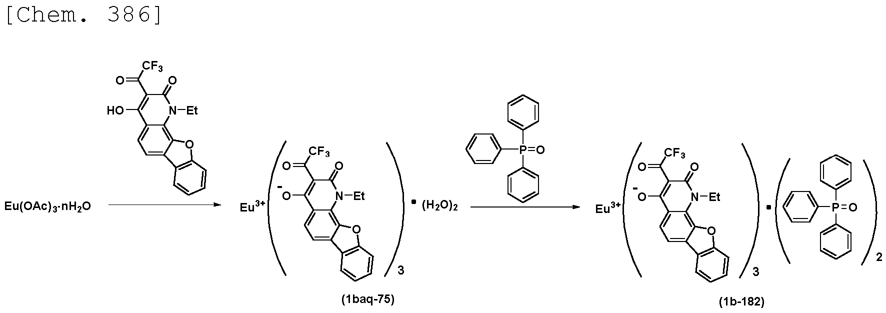

Ethanol (4.2 mL) and water (1.3 mL) were added to europium(III) acetate hydrate (40.0 mg as a 2.5-hydrate, 107 µmol) and the 1-ethyl-4-hydroxy-3-(2,2,2-trifluoroethan-1-on-1-yl)benzofuro[3,2-h]quinolin-2(1H)-one obtained in Reference Example 37 (120 mg, 321 µmol). Then a 1 M aqueous solution of sodium hydroxide (321 µL, 321 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a yellow powder solid of diaquatris[1-ethyl-4-hydroxy-3-(2,2,2-trifluoroethan-1-on-1-yl)benzofuro[3,2-h]quinolin-2(1H)-onato]europium(III) (1baq-75) (actual yield, 91.3 mg; percent yield, 65%). The resulting crude product was used in the next reaction directly, without further purification. ESIMS (m/z), MeOH: 1298.1 [M - 2H₂O + Na]⁺.

The diaquatris[1-ethyl-4-hydroxy-3-(2,2,2-trifluoroethan-1-on-1-yl)benzofuro[3,2-h]quinolin-2(1H)-onato]europium(III) (1baq-75) (91.0 mg, 69.5 µmol) and triphenylphosphine oxide (38.8 mg, 139 µmol) were dissolved in acetone (7.0 mL), and the resulting solution was stirred for 3 hours at room temperature. The reaction mixture was filtered through Celite, then the filtrate was concentrated under reduced pressure, and slurry was washed away with acetone/hexane to give a yellow solid of tris[1-ethyl-4-hydroxy-3-(2,2,2-trifluoroethan-1-on-1-yl)benzofuro[3,2-h]quinolin-2(1H)-onato]bis(triphenylphosphine oxide)europium(III) (1b-182) (actual yield, 96.6 mg; percent yield, 76%) . ¹⁹F-NMR (376 MHz, Acetone-d₆) δ (ppm) : -71.7 (s, 1. 6F) , -71.9 (s, 6.0F), -73.8 (brs, 1.4F). ³¹P-NMR (162 MHz, Acetone-d₆) δ (ppm) : -86.7 (brs). ESIMS (m/z), MeOH: 1854.6 [M + Na]⁺, 1576.5 [M - (triphenylphosphine oxide) + Na]⁺, 1456.4 [M - (1-ethyl-4-hydroxy-3-(2,2,2-trifluoroethan-1-on-1-yl)benzofuro[3,2-h]quinolin-2(1H)-onato)]⁺.

### (EXAMPLE 64)

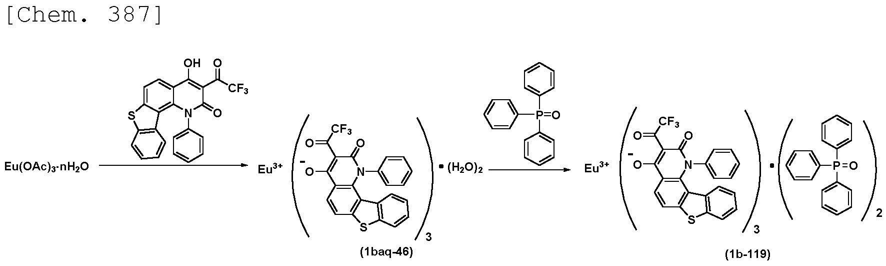

Ethanol (6.3 mL) and water (2.1 mL) were added to europium(III) acetate hydrate (63.0 mg as a 2.5-hydrate, 168 µmol) and the 4-hydroxy-1-phenyl-3-(2,2,2-trifluoroethan-1-on-1-yl)-[1]benzothieno[2,3-h]quinolin-2(1H)-one obtained in Reference Example 33 (222 mg, 505 µmol). Then a 1 M aqueous solution of sodium hydroxide (505 µL, 505 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a yellow powder solid of diaquatris[4-hydroxy-1-phenyl-3-(2,2,2-trifluoroethan-1-on-1-yl)-[1]benzo[4,5]thieno[2,3-h]quinolin-2(1H)-onato]europium(III) (1baq-46) (actual yield, 228 mg; percent yield, 90%). The resulting crude product was used in the next reaction directly, without further purification. ESIMS (m/z), MeOH: 1490.9 [M - 2H₂O + Na]⁺.

The diaquatris[4-hydroxy-1-phenyl-3-(2,2,2-trifluoroethan-1-on-1-yl)benzo[4,5]thieno[2,3-h]quinolin-2(1H)-onato]europium(III) (1baq-46) (228 mg, 152 µmol) and triphenylphosphine oxide (84.6 mg, 304 µmol) were dissolved in acetone (15.2 mL), and the resulting solution was stirred for 3 hours at room temperature. The reaction mixture was filtered through Celite, then the filtrate was concentrated under reduced pressure, and reprecipitation with acetone/hexane was performed twice. The resulting solid acetone was added, an undissolved material was removed, and then the residue was dried by heating to give a yellow solid of tris[4-hydroxy-1-phenyl-3-(2,2,2-trifluoroethan-1-on-1-yl)-[1]benzothieno[2,3-h]quinolin-2(1H)-onato] bis(triphenylphosphine oxide)europium(III) (1b-119) (actual yield, 177 mg; percent yield, 58%). ¹⁹F-NMR (376 MHz, Acetone-d₆) δ (ppm): -71.9 (s, 6.1F), -72.2 (s, 2.3F), -74.1 (s, 0.4F), -74.5 (s, 0.2F). ³¹P-NMR (162 MHz, Acetone-d₆) δ (ppm): -86.4 (brs). ESIMS (m/z), MeOH: 1770.4 [M - (triphenylphosphine oxide) + Na]⁺, 1587.2 [M - (4-hydroxy-1-phenyl-3-(2,2,2-trifluoroethan-1-on-1-yl)-[1]benzothieno[2,3-h]quinolin-2(1H)-onato)]⁺.

### (EXAMPLE 65)

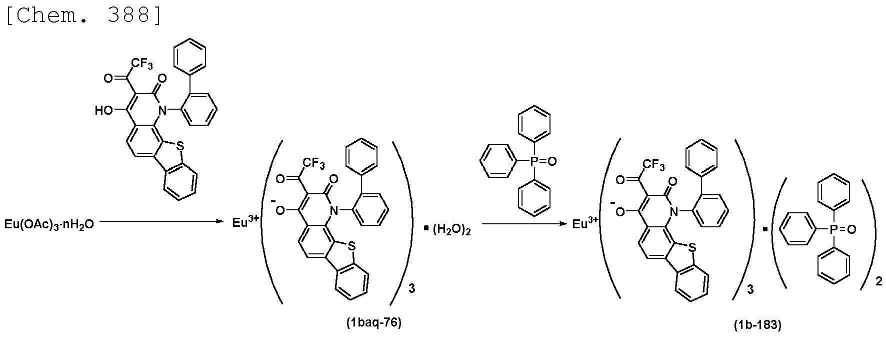

Ethanol (4.60 mL) and water (1.50 mL) were added to europium(III) acetate hydrate (45.9 mg as a 2.5-hydrate, 123 µmol) and the 1-([1,1'-biphenyl]-2-yl)-4-hydroxy-3-(2,2,2-trifluoroethan-1-on-1-yl)-[1]benzothieno[3,2-h]quinolin-2(1H)-one obtained in Reference Example 34 (189 mg, 368 µmol). Then a 1 M aqueous solution of sodium hydroxide (368 µL, 368 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[1-([1,1'-biphenyl]-2-yl)-4-hydroxy-3-(2,2,2-trifluoroethan-1-on-1-yl)-[1]benzothieno[3,2-h]quinolin-2(1H)-onato] europium(III) (1baq-76) (actual yield, 178 mg; percent yield, 850). The resulting crude product was used in the next reaction directly, without further purification. ¹⁹F-NMR (376 MHz, Acetone-d₆) δ (ppm): -74.0 (s). ESIMS (m/z), MeOH: 1717.6 [M - 2H₂O + Na]⁺.

The diaquatris[1-([1,1'-biphenyl]-2-yl)-4-hydroxy-3-(2,2,2-trifluoroethan-1-on-1-yl)-[1]benzothieno[3,2-h]quinolin-2(1H)-onato]europium(III) (1baq-76) (173 mg, 100 µmol) and triphenylphosphine oxide (55.6 mg, 200 µmol) were dissolved in acetone (10.0 mL), and the resulting solution was stirred for 3 hours at room temperature. The reaction mixture was filtered through Celite, then the filtrate was concentrated under reduced pressure, and slurry was washed away with acetone/hexane = 1/2 to give an orange-yellow solid of tris[1-([1,1'-biphenyl]-2-yl)-4-hydroxy-3-(2,2,2-trifluoroethan-1-on-1-yl)-[1]benzothieno[3,2-h]quinolin-2(1H)-onato]bis(triphenylphosphine oxide)europium(III) (1b-183) (actual yield, 174 mg; percent yield, 77%). ¹⁹F-NMR (376 MHz, CDCl₃) δ (ppm): -71.6 (s, 6.8F), -72.6 (s, 2.2F). ³¹P-NMR (162 MHz, CDCl₃) δ (ppm): -78.8 (brs). ESIMS (m/z), MeCN: 2273.4 [M + Na]⁺, 1994.8 [M - (triphenylphosphine oxide) + Na]⁺.

### (EXAMPLE 66)

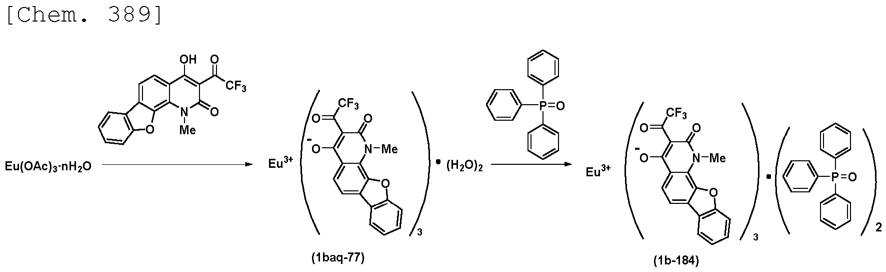

Ethanol (7.5 mL) and water (2.5 mL) were added to europium(III) acetate hydrate (74.8 mg as a 2.5-hydrate, 200 µmol) and the 1-methyl-4-hydroxy-3-(2,2,2-trifluoroethan-1-on-1-yl)benzofuro[3,2-h]quinolin-2(1H)-one obtained in Reference Example 38 (217 mg, 600 µmol). Then a 1 M aqueous solution of sodium hydroxide (600 µL, 600 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a yellow-green powder solid of diaquatris[1-ethyl-4-hydroxy-3-(2,2,2-trifluoroethan-1-on-1-yl)benzofuro[3,2-h]quinolin-2(1H)-onato]europium(III) (1baq-77) (actual yield, 270 mg; percent yield, quant.). The resulting crude product was used in the next reaction directly, without further purification. ESIMS (m/z), MeOH: 1256.2 [M - 2H₂O + Na]⁺.

The diaquatris[1-methyl-4-hydroxy-3-(2,2,2-trifluoroethan-1-on-1-yl)benzofuro[3,2-h]quinolin-2(1H)-onato]europium(III) (1baq-77) (127 mg, 100 µmol) and triphenylphosphine oxide (55.7 mg, 200 µmol) were dissolved in acetone (10.0 mL), and the resulting solution was stirred for 3 hours at room temperature. The reaction mixture was filtered through Celite, then the filtrate was concentrated under reduced pressure, and slurry was washed away with acetone/hexane to give a yellow-green solid of tris[1-methyl-4-hydroxy-3-(2,2,2-trifluoroethan-1-on-1-yl) benzofuro [3, 2-h] quinolin-2 (1H) - onato] bis(triphenylphosphine oxide)europium(III) (1b-184) (actual yield, 146 mg; percent yield, 81%). ¹⁹F-NMR (376 MHz, Acetone-d₆) δ (ppm): -70.2 (brs, 0.8F), -71.6 (brs, 7.2F), -73.1 (brs, 1.0F). ³¹P-NMR (162 MHz, Acetone-d₆) δ (ppm): -87.0 (brs). ESIMS (m/z), MeOH: 1812.5 [M+Na]⁺, 1534.3 [M - (triphenylphosphine oxide) + Na]⁺.

### (EXAMPLE 67)

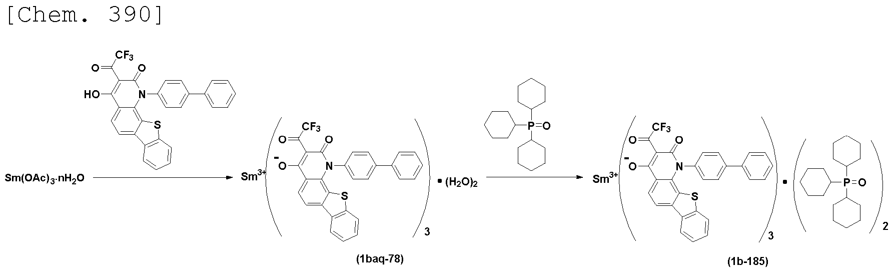

Ethanol (4.00 mL) and water (1.20 mL) were added to samarium(III) acetate (37.3 mg as a 2.5-hydrate, 100 µmol) and the 1-(4-biphenylyl)-4-hydroxy-3-(2,2,2-trifluoroethan-1-on-1-yl)-[1]benzothieno[3,2-h]quinolin-2(1H)-one obtained in Reference Example 18 (156 mg, 303 µmol). A 1 M aqueous solution of sodium hydroxide (300 µL, 300 µmol) was added, and the resulting mixture was stirred for 1 hour at room temperature. After the ethanol was distilled away, water was put into the reaction mixture, the resulting mixture was filtered, and the collected solid was washed with water. The washed solid was dried by heating to give a brown solid of diaquatris[1-(4-biphenylyl)-4-hydroxy-3-(2,2,2-trifluoroethan-1-on-1-yl)-[1]benzothieno[3,2-h]quinolin-2(1H)-onato]samarium(III) (1baq-78) (actual yield, 171 mg; percent yield, 980). The resulting crude product was used in the next reaction directly, without further purification. ESIMS (m/z), MeOH: 1718.4 [M - 2H₂O + Na]⁺.

Acetone (10.0 mL) was added to the diaquatris[1-(4-biphenylyl)-4-hydroxy-3-(2,2,2-trifluoroethan-1-on-1-yl)-[1]benzothieno[3,2-h]quinolin-2(1H)-onato]samarium(III) (1baq-78) (170 mg, 100 µmol) and tricyclohexylphosphine oxide (58.2 mg, 200 µmol), and the resulting mixture was stirred for 3 hours at room temperature. The reaction mixture was filtered through Celite, and then the filtrate was concentrated under reduced pressure. After reprecipitation with chloroform/hexane, the precipitate was removed by filtration, and the filtrate was concentrated under reduced pressure. The resulting solid was washed with chloroform/hexane for the removal of slurry to give a yellow solid of tris[1-(4-biphenylyl)-4-hydroxy-3-(2,2,2-trifluoroethan-1-on-1-yl)-[1]benzothieno[3,2-h]quinolin-2(1H)-onato]bis(tricyclohexylphosphine oxide)samarium(III) (1b-186) (actual yield, 113 mg; percent yield, 50%). ¹⁹F-NMR (376 MHz, CDCl₃) δ (ppm): -71.6 (s). ³¹P-NMR (162 MHz, CDCl₃) δ (ppm): -56.7 (brs). ESIMS (m/z), MeOH: 2309.6 [M + Na]⁺, 2014.8 [M - (tricyclohexylphosphine oxide) + Na]⁺.

### (EXAMPLE 68)

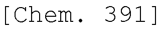

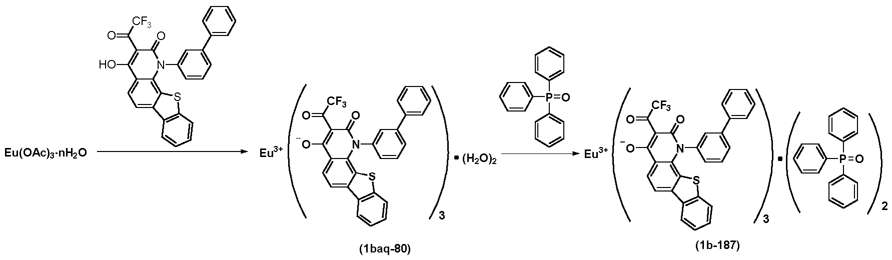

Ethanol (580 µL) and water (190 µL) were added to europium(III) acetate hydrate (5.80 mg as a 2.5-hydrate, 15.5 µmol) and the 1-([1,1'-biphenyl]-3-yl)-4-hydroxy-3-(2,2,2-trifluoroethan-1-on-1-yl)-[1]benzothieno[3,2-h]quinolin-2(1H)-one obtained in Reference Example 35 (24.0 mg, 46.5 µmol). Then a 1 M aqueous solution of sodium hydroxide (46.0 µL, 46.0 µmol) was added, and the resulting mixture was stirred for 1 hour at room temperature. After the ethanol was distilled away, water was put into the reaction mixture, the resulting mixture was filtered, and the collected solid was washed with water. The washed solid was dried by heating to give a deep orange powder solid of diaquatris[1-([1,1'-biphenyl]-3-yl)-4-hydroxy-3-(2,2,2-trifluoroethan-1-on-1-yl)-[1]benzothieno[3,2-h]quinolin-2(1H)-onato]europium(III) (1baq-80) (actual yield, 25.2 mg; percent yield, 94%). The resulting crude product was used in the next reaction directly, without further purification. ESIMS (m/z), MeOH: 1718.5 [M - 2H₂O + Na]⁺.

The diaquatris[1-([1,1'-biphenyl]-3-yl)-4-hydroxy-3-(2,2,2-trifluoroethan-1-on-1-yl)-[1]benzothieno[3,2-h]quinolin-2(1H)-onato]europium(III) (1baq-80) (24.0 mg, 13.9 µmol) and triphenylphosphine oxide (7.70 mg, 27.7 µmol) were dissolved in acetone (1.40 mL), and the resulting solution was stirred for 3 hours at room temperature. The reaction mixture was filtered through Celite, then the filtrate was concentrated under reduced pressure, and slurry was washed away with acetone/hexane to give a yellow solid of tris[1-([1,1'-biphenyl]-3-yl)-4-hydroxy-3-(2,2,2-trifluoroethan-1-on-1-yl)-[1]benzothieno[3,2-h]quinolin-2(1H)-onato]bis(triphenylphosphine oxide)europium(III) (1b-187) (actual yield, 35.6 mg; percent yield, quant.). ¹⁹F-NMR (376 MHz, CDCl₃) δ (ppm): -71.9 (s, 3.0F), -72.0 (s, 3.5F), -72.1 (s, 2.5F). ³¹P-NMR (162 MHz, CDCl₃) δ (ppm): -80.4 (brs). ESIMS (m/z), MeOH: 2275.1 [M + Na]⁺, 1996.8 [M - (triphenylphosphine oxide) + Na]⁺, 1737.0 [M - (1-([1,1'-biphenyl]-3-yl)-4-hydroxy-3-(2,2,2-trifluoroethan-1-on-1-yl)-[1]benzothieno[3,2-h]quinolin-2(1H)-onato)]⁺.

### (EXAMPLE 69)

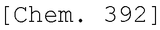

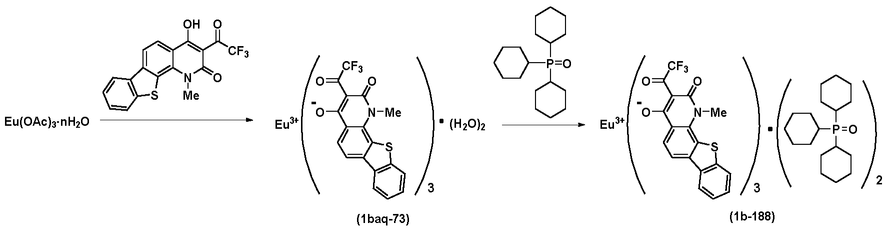

Ethanol (12.0 mL) and water (3.8 mL) were added to europium(III) acetate hydrate (94.6 mg as a 2.5-hydrate, 72.0 µmol) and the 1-methyl-4-hydroxy-3-(2,2,2-trifluoroethan-1-on-1-yl)-[1]benzothieno[3,2-h]quinolin-2(1H)-one obtained in Reference Example 32 (42.6 mg, 144 µmol). Then a 1 M aqueous solution of sodium hydroxide (921 µL, 921 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[1-methyl-4-hydroxy-3-(2,2,2-trifluoroethan-1-on-1-yl)-[1]benzothieno[3,2-h]quinolin-2(1H)-onato]europium(III) (1baq-73) (actual yield, 375 mg; percent yield, 93%). The resulting crude product was used in the next reaction directly, without further purification. ESIMS (m/z), MeOH: 1304.2 [M - 2H₂O + Na]⁺.

The diaquatris[1-methyl-4-hydroxy-3-(2,2,2-trifluoroethan-1-on-1-yl)-[1]benzothieno[3,2-h]quinolin-2(1H)-onato]europium(III) (1baq-73) (94.6 mg, 72.0 µmol) and tricyclohexylphosphine oxide (42.6 mg, 144 µmol) were dissolved in acetone (8.00 mL), and the resulting solution was stirred for 3 hours at room temperature. The reaction mixture was filtered through Celite, and then the filtrate was concentrated under reduced pressure. After reprecipitation with acetone/hexane, the precipitated solid was removed by filtration. The filtrate was concentrated under reduced pressure, and the residue was reprecipitated with acetone/hexane to give a yellow solid of tris[1-methyl-4-hydroxy-3-(2,2,2-trifluoroethan-1-on-1-yl)-[1]benzo[4,5]thieno[3,2-h]quinolin-2(1H)-onato]bis(tricyclohexylphosphine oxide)europium(III) (1b-188) (actual yield, 60.4 mg; percent yield, 45%). ¹⁹F-NMR (376 MHz, CDCl₃) δ (ppm): -70.9 (brs). ³¹P-NMR (162 MHz, CDCl₃) δ (ppm): -65.3 (brs). ESIMS (m/z), MeOH: 1895.7 [M + Na]⁺, 1599.4 [M - (triphenylphosphine oxide) + Na]⁺, 1498.5 [M - (1-methyl-4-hydroxy-3-(2,2,2-trifluoroethan-1-on-1-yl)-[1]benzothieno[3,2-h]quinolin-2(1H)-onato)]⁺.

### (EXAMPLE 70)

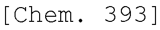

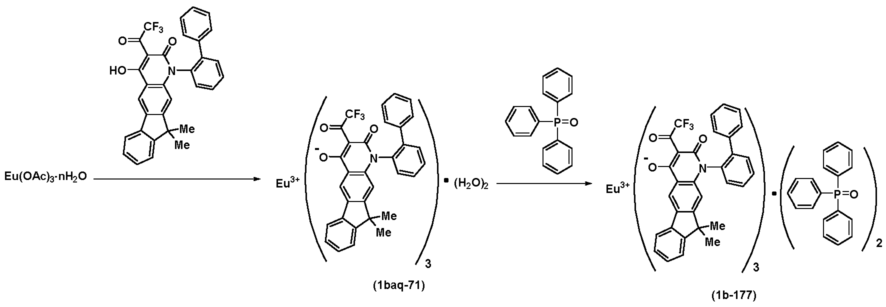

Ethanol (7.36 mL) and water (2.45 mL) were added to europium(III) acetate hydrate (73.5 mg as a 2.5-hydrate, 196 µmol) and the 1-(2-biphenylyl)-4-hydroxy-10,10-dimethyl-3-(2,2,2-trifluoroethyl-1-on-1-yl)-10H-indeno[3,2-g]quinolin-2(1H)-one obtained in Reference Example 39 (310 mg, 590 pmol). Then a 1 M aqueous solution of sodium hydroxide (590 µL, 590 µmol) was added, and the resulting mixture was stirred for 4 hours at room temperature. After concentration under reduced pressure, water was added to the reaction mixture, the resulting mixture was filtered, and the collected solid was washed with water. The washed solid was dried by heating under reduced pressure to give a yellow powder solid of diaquatris[1-(2-biphenylyl)-4-hydroxy-10,10-dimethyl-3-(2,2,2-trifluoroethyl-1-on-1-yl)-10H-indeno[3,2-g]quinolin-2(1H)-onato]europium(III) (1baq-71) (actual yield, 246 mg; percent yield, 710). The resulting crude product was used in the next reaction directly, without further purification. ¹⁹F-NMR (376 MHz, Acetone-d₆) δ (ppm): -68.8 (s, 0.6F), -72.4 (s, 3.6F), -73.0 (s, 0.6F), -73.5 (s. 1.8F), -74.0 (s, 1.8F), -74.3 (s, 0.6F). ESIMS (m/z), MeOH: 1747.5 [M - 2H₂O + Na]⁺.

Acetone (12.7 mL) was added to the diaquatris[1-(2-biphenylyl)-4-hydroxy-10,10-dimethyl-3-(2,2,2-trifluoroethyl-1-on-1-yl)-10H-indeno[3,2-g]quinolin-2(1H)-onato]europium(III) (224 mg, 127 µmol) and triphenylphosphine oxide (70.8 mg, 256 µmol), and the resulting mixture was stirred for 3 hours at room temperature. The reaction mixture was filtered through Celite, and then the solvent was distilled away under reduced pressure. The residue was reprecipitated with chloroform/hexane, and the solid that formed was collected by filtration and then dried by heating under reduced pressure to give a yellow solid of tris[1-(2-biphenylyl)-4-hydroxy-10,10-dimethyl-3-(2,2,2-trifluoroethyl-1-on-1-yl)-10H-indeno[3,2-g]quinolin-2(1H)-onato]bis(triphenylphosphine oxide)europium(III) (1b-177) (actual yield, 220 mg; percent yield, 76%) . ¹⁹F-NMR (376 MHz, Acetone-d₆) δ (ppm) : -70.7 (s, 5.1F), -71.3 (s, 2.4F), -71.8 (s, 0.6F), -72.8 (brs, 0.9F) . ³¹P-NMR (162 MHz, Acetone-d₆) δ (ppm) : -84.8 (brs) . ESIMS (m/z), MeOH: 2305.5 [M + Na]⁺, 2026.1 [M - (triphenylphosphine oxide) + Na]⁺.

### (EXAMPLE 71)

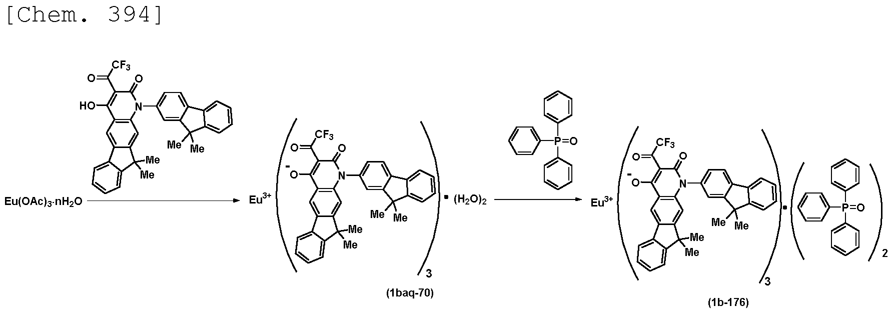

Ethanol (7.87 mL) and water (2.62 mL) were added to europium(III) acetate hydrate (78.6 mg as a 2.5-hydrate, 210 µmol) and the 1-(2-9,9-dimethyl-9H-fluorene)-4-hydroxy-10,10-dimethyl-3-(2,2,2-trifluoroethyl-1-on-1-yl)-10H-indeno[3,2-g]quinolin-2(1H)-one obtained in Reference Example 40 (357 mg, 630 µmol). Then a 1 M aqueous solution of sodium hydroxide (630 µL, 630 µmol) was added, and the resulting mixture weas stirred for 17 hours at room temperature. After concentration under reduced pressure, water was added to the reaction mixture, the resulting mixture was filtered, and the collected solid was washed with water. The washed solid was dried by heating under reduced pressure to give a yellow powder solid of diaquatris[1-(2-9,9-dimethyl-9H-fluorene)-4-hydroxy-10,10-dimethyl-3-(2,2,2-trifluoroethyl-1-on-1-yl)-10H-indeno[3,2-g]quinolin-2(1H)-onato]europium(III) (1baq-70) (actual yield, 238 mg; percent yield, 60%). The resulting crude product was used in the next reaction directly, without further purification. 19F-NMR (376 MHz, Acetone-d6) δ (ppm): -69.4 (s, 2.1F), -69.7 (brd, 0.6F), -73.2 (s, 3.9F), -73.9 (brs, 1.5F), -74.7 (brs, 0.9F). ESIMS (m/z), MeOH: 1869.3 [M - 2H2O + Na]+.

Acetone (12 mL) was added to the diaquatris[1-(2-9,9-dimethyl-9H-fluorene)-4-hydroxy-10,10-dimethyl-3-(2,2,2-trifluoroethyl-1-on-1-yl)-10H-indeno[3,2-g]quinolin-2(1H)-onato]europium(III) (225 mg, 120 µmol) and triphenylphosphine oxide (66.6 mg, 240 µmol), and the resulting mixture was stirred for 3 hours at room temperature. The reaction mixture was filtered through Celite, then the solvent was distilled away under reduced pressure. Reprecipitation with chloroform/hexane was performed, and a yellow solid that formed was collected by filtration. The resulting solid was dried by heating under reduced pressure to give a yellow solid of tris[1-(1-biphenylyl)-4-hydroxy-10,10-dimethyl-3-(2,2,2-trifluoroethyl-1-on-1-yl)-10H-indeno[3,2-g]quinolin-2(1H)-onato]bis(triphenylphosphine oxide)europium(III) (1b-176) (actual yield, 57.8 mg; percent yield, 20%). 19F-NMR (376 MHz, Acetone-d6) δ (ppm): -71.3 (s, 9F). 31P-NMR (162 MHz, Acetone-d6) δ (ppm): -86.8 (brs). ESIMS (m/z), MeOH: 2424.6 [M + Na]⁺, 2146.7 [M - (triphenylphosphine oxide) + Na]⁺.

### (EXAMPLE 72)

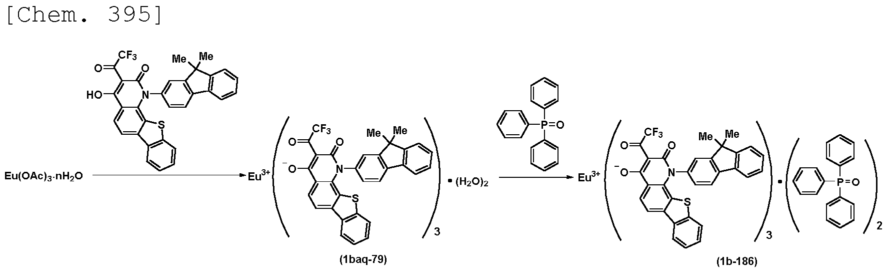

Ethanol (3.70 mL) and water (1.20 mL) were added to europium(III) acetate hydrate (37.4 mg as a 2.5-hydrate, 100 µmol) and the 1-(9,9-dimethyl-9H-fluoren-2-yl)-4-hydroxy-3-(2,2,2-trifluoroethan-1-on-1-yl)-[1]benzothieno[3,2-h]quinolin-2(1H)-one obtained in Reference Example 41 (167 mg, 300 µmol). Then a 1 M aqueous solution of sodium hydroxide (300 µL, 300 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a deep orange powder solid of diaquatris[1-(9,9-dimethyl-9H-fluoren-2-yl)-4-hydroxy-3-(2,2,2-trifluoroethan-1-on-1-yl)-[1]benzothieno[3,2-h]quinolin-2(1H)-onato]europium(III) (1baq-79) (actual yield, 174 mg; percent yield, 950). The resulting crude product was used in the next reaction directly, without further purification. ESIMS (m/z), MeOH: 1837.4 [M - 2H₂O + Na]⁺.

The diaquatris[1-(9,9-dimethyl-9H-fluoren-2-yl)-4-hydroxy-3-(2,2,2-trifluoroethan-1-on-1-yl)-[1]benzothieno[3,2-h]quinolin-2(1H)-onato]europium(III) (1baq-79) (171 mg, 92.4 µmol) and triphenylphosphine oxide (52.0 mg, 187 µmol) were dissolved in acetone (10.0 mL), and the resulting solution was stirred for 3 hours at room temperature. The reaction mixture was filtered through Celite, then the filtrate was concentrated under reduced pressure, and the residue was reprecipitated with acetone/hexane. The precipitated solid was removed by filtration, the filtrate was concentrated under reduced pressure, and then the residue was reprecipitated with acetone/hexane to give a yellow solid of tris[1-(9,9-dimethyl-9H-fluoren-2-yl)-4-hydroxy-3-(2,2,2-trifluoroethan-1-on-1-yl)-[1]benzothieno[3,2-h]quinolin-2(1H)-onato]bis(triphenylphosphine oxide)europium(III) (1b-188) (actual yield, 63.5 mg; percent yield, 29%). ¹⁹F-NMR (376 MHz, CDCl₃) δ (ppm): -71.8 (s, 2.1F), -71.9 (s, 2.3F), -72.0 (s, 4.6F). ³¹P-NMR (162 MHz, CDCl₃) δ (ppm): -81.1 (brs). ESIMS (m/z), MeOH: 2394.7 [M + Na]⁺, 2115.5 [M - (triphenylphosphine oxide) + Na]⁺.

### (EXAMPLE 73)

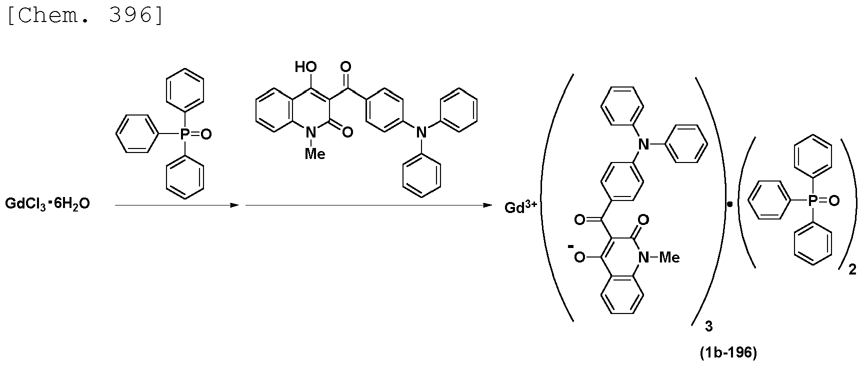

Gadolinium(III) chloride hexahydrate (15.3 mg, 41.7 pmmol) and triphenylphosphine oxide (23.1 mg, 83.0 µmol) were put into acetone (1.0 mL), and the resulting mixture was stirred for 1 hour at room temperature. Then the 4-hydroxy-1-methyl-3-[4-(diphenylamino)benzoyl]quinolin-2(1H)-one (55.4 mg, 124 µmol) obtained in Reference Example 3 and a 14.8 M aqueous solution of ammonia (8.4 µL, 124 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[4-hydroxy-1-methyl-3-[4-(diphenylamino)benzoyl]quinolin-2(1H)-onato]bis(triphenylphosphine oxide)gadolinium(III) (1b-196). (Actual yield, 27.3 mg; percent yield, 32%). ESIMS (m/z), MeOH: 1794.2 [M - (triphenylphosphine oxide) + Na]⁺.

### (EXAMPLE 74)

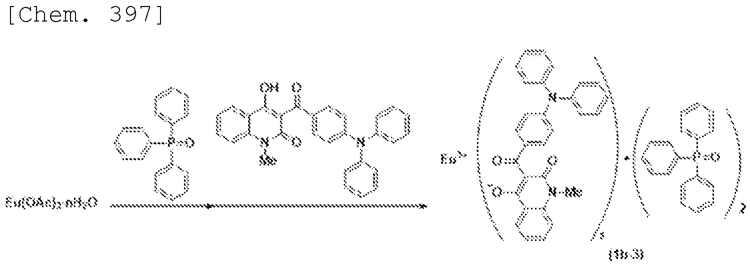

Europium acetate n-hydrate (1.81 g, 4.84 mmol) was dissolved in methanol (140 mL), triphenylphosphine oxide (2.69 g, 9.68 mmol) dissolved in methanol (100 mL) was added, and the resulting mixture was stirred for 1 hour at room temperature. The 4-hydroxy-1-methyl-3-[4-(diphenylamino)benzoyl]quinolin-2(1H)-one obtained in Reference Example 3 (6.48 g, 14.5 mmol) was added to the reaction mixture, then 14.8 M aqueous ammonia (981 µL, 14.5 mmol) was added dropwise, and the resulting mixture was stirred for 3 hours at room temperature. The reaction solution was concentrated under reduced pressure, and then the residue was dried for 20 hours at 60°C under reduced pressure. The resulting solid was washed with water and then dissolved by adding chloroform to it, and the resulting solution was concentrated under reduced pressure. The resulting solid was washed with diethyl ether to give a yellow solid of tris[4-hydroxy-1-methyl-3-[4-(diphenylamino)benzoyl]quinolin-2(1H)-onato]bis(triphenylphosphine oxide)europium(III) (1b-3) (actual yield, 9.68 g; percent yield, 98%). ³¹P-NMR (162 MHz, Acetone-d₆) δ (ppm): -76.0 (brs). ESIMS (m/z), MeOH: 2068.6 [M + Na]⁺, 1599.0 [M - (4-hydroxy-1-methyl-3-[4-(diphenylamino)benzoyl]quinolin-2(1H)-onato)]⁺.

### (EXAMPLE 75)

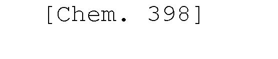

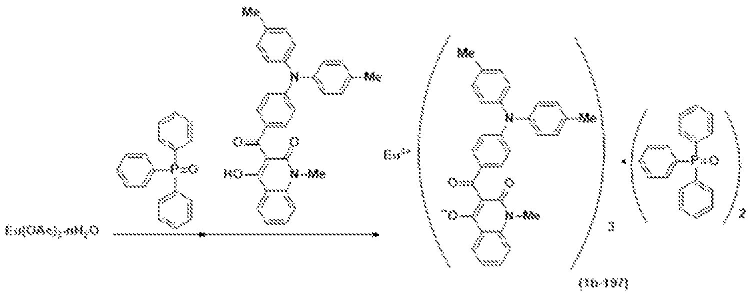

Methanol (20.0 mL) was added to europium acetate n-hydrate (74.8 mg as a 2.5-hydrate, 200 µmol) and triphenylphosphine oxide (111 mg, 400 µmol), and the resulting mixture was stirred for 1 hour at room temperature. The 3-[4-(di-p-tolylamino)benzoyl]-4-hydroxy-1-methylquinolin-2(1H)-one obtained in Reference Example 42 (281 mg, 600 µmol) was added to the reaction mixture. 14.8 M aqueous ammonia (40.0 µL, 600 µmol) was added dropwise, and the resulting mixture was stirred for 3 hours at room temperature. The reaction solution was concentrated under reduced pressure, then the residue was dissolved by adding chloroform to it, and the resulting solution was concentrated under reduced pressure. The resulting solid was washed with diethyl ether to give a yellow solid of tris{3-[4-(di-p-tolylamino)benzoyl]-4-hydroxy-1-methylquinolin-2(1H)-onato}bis(triphenylphosphine oxide)europium(III) (1b-197) (actual yield, 349 mg; percent yield, 82%). ³¹P-NMR (162 MHz, Acetone-d₆) δ (ppm): -76.8 (brs) . ESIMS (m/z), MeOH: 2152.0 [M + Na]⁺, [M - (3-[4-(dip-tolylamino)benzoyl]-4-hydroxy-1-methylquinolin-2(1H)-onato)]⁺.

### (EXAMPLE 76)

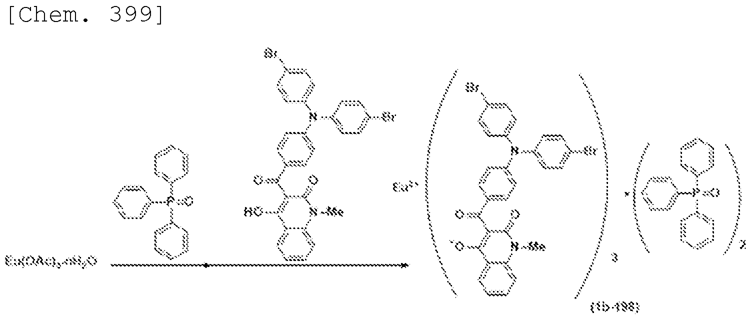

Methanol (7.50 mL) was added to europium acetate n-hydrate (56.2 mg as a 2.5-hydrate, 150 µmol) and triphenylphosphine oxide (84.2 mg, 303 µmol), and the resulting mixture was stirred for 1 hour at room temperature. The 3-{4-[bis(4-bromophenyl)amino]benzoyl}-4-hydroxy-1-methylquinolin-2(1H)-one obtained in Reference Example 43 (272 mg, 450 µmol) was added to the reaction mixture. 14.8 M aqueous ammonia (30.0 µL, 444 µmol) was added dropwise, and the resulting mixture was stirred for 3 hours at room temperature. The reaction solution was concentrated under reduced pressure, then the residue was dissolved by adding chloroform to it, and the resulting solution was concentrated under reduced pressure. The resulting solid was washed with diethyl ether to give a yellow solid of tris(3-{4-[bis(4-bromophenyl)amino]benzoyl}-4-hydroxy-1-methylquinolin-2(1H)-onato)bis(triphenylphosphine oxide)europium(III) (1b-198) (actual yield, 336 mg; percent yield, 89%). ³¹P-NMR (162 MHz, Acetone-d₆) δ (ppm): -75.3 (brs). ESIMS (m/z), MeOH: 2541.1 [M + Na]⁺, 1916.1 [M - (3-{4-[bis(4-bromophenyl)amino]benzoyl}-4-hydroxy-1-methylquinolin-2(1H)-onato)]⁺.

### (EXAMPLE 77)

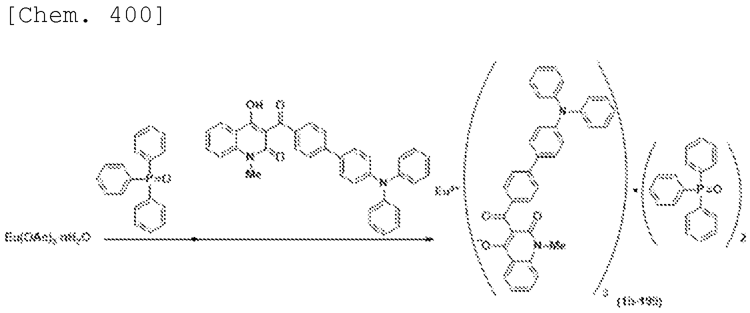

Methanol (3.00 mL) was added to europium acetate n-hydrate (24.1 mg as a 2.5-hydrate, 64.4 µmol) and triphenylphosphine oxide (34.2 mg, 123 µmol), and the resulting mixture was stirred for 1 hour at room temperature. The 3-[4'-(diphenylamino)-[1,1'-biphenyl]-4-carbonyl]-4-hydroxy-1-methylquinolin-2(1H)-one obtained in Reference Example 44 (101 mg, 193 µmol) was added to the reaction mixture. 14.8 M aqueous ammonia (10.0 µL, 148 µmol) was added dropwise, and the resulting mixture was stirred for 3 hours at room temperature. The reaction solution was concentrated under reduced pressure, then the residue was dissolved by adding chloroform to it, and the resulting solution was concentrated under reduced pressure. The resulting solid was washed with diethyl ether to give a yellow solid of tris{3-[4'-(diphenylamino)-[1,1'-biphenyl]-4-carbonyl]-4-hydroxy-1-methylquinolin-2(1H)-onato}bis(triphenylphosphine oxide)europium(III) (1b-199) (actual yield, 142 mg; percent yield, 97%). ³¹P-NMR (162 MHz, toluene-d₈) δ (ppm): -70.0 (brs). ESIMS (m/z), MeOH: 1752.4 [M - (3-[4'-(diphenylamino)-[1,1'-biphenyl]-4-carbonyl]-4-hydroxy-1-methylquinolin-2(1H)-onato)]⁺, 1740.4 [M - (triphenylphosphine oxide) + Na]⁺.

### (EXAMPLE 78)

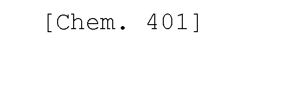

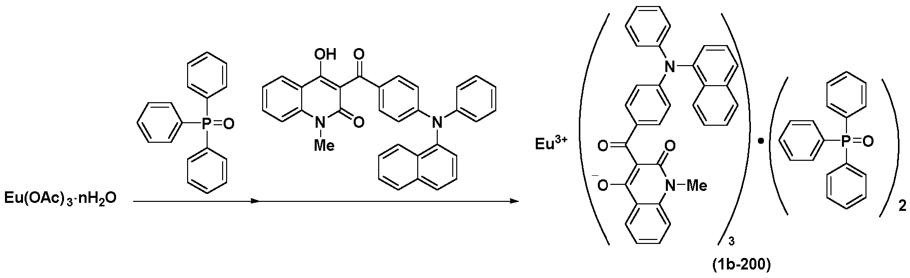

Methanol (20.0 mL) was added to europium acetate n-hydrate (154 mg as a 2.5-hydrate, 411 µmol) and triphenylphosphine oxide (228 mg, 820 µmol), and the resulting mixture was stirred for 1 hour at room temperature. The 4-hydroxy-1-methyl-3-{4-[naphthalen-1-yl(phenyl)amino]benzoyl}quinolin-2(1H)-one obtained in Reference Example 45 (612 mg, 1.23 mmol) was added to the reaction mixture. 14.8 M aqueous ammonia (85.0 µL, 1.26 mmol) was added dropwise, and the resulting mixture was stirred for 3 hours at room temperature. The reaction solution was concentrated under reduced pressure, then the residue was dissolved by adding chloroform to it, and the resulting solution was concentrated under reduced pressure. The resulting solid was washed with diethyl ether to give a yellow solid of tris(4-hydroxy-1-methyl-3-{4-[naphthalen-1-yl(phenyl)amino]benzoyl}quinolin-2(1H)-onato)bis(triphenylphosphine oxide)europium(III) (1b-200) (actual yield, 800 mg; percent yield, 89%). ³¹P-NMR (162 MHz, toluene-d₈) δ (ppm): -65.6 (brs). ESIMS (m/z), MeOH: 2217.9 [M + Na]⁺, 1701.5 [M - (4-hydroxy-1-methyl-3-[4-(naphthalen-1-yl(phenyl)amino)benzoyl]quinolin-2(1H)-onato)]⁺, 1740.4 [M - (triphenylphosphine oxide) + Na]⁺.

### (EXAMPLE 79)

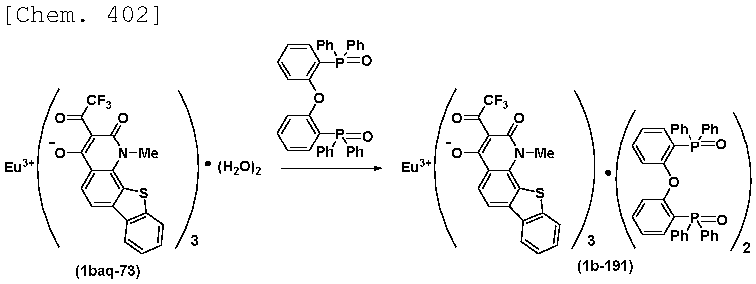

The diaquatris[1-methyl-4-hydroxy-3-(2,2,2-trifluoroethan-1-on-1-yl)-[1]benzothieno[3,2-h]quinolin-2(1H)-onato]europium(III) (1baq-73) obtained as an intermediate in Example 69 (300 mg, 230 µmol) and bis[2-(diphenylphosphino)phenyl]ether oxide (130 mg, 230 µmol) were dissolved in acetone (22.8 mL), and the resulting solution was stirred for 3 hours at room temperature. The reaction mixture was filtered, and then the filtrate was concentrated under reduced pressure. After reprecipitation with acetone/hexane, the precipitated solid was removed by filtration. Hexane (5.00 mL) was added to the filtrate, and the precipitated solid was collected by filtration to give a yellow solid of tris[1-methyl-4-hydroxy-3-(2,2,2-trifluoroethan-1-on-1-yl)-[1]benzothieno[3,2-h]quinolin-2(1H)-onato]bis[2-(diphenylphosphino)phenyl]ether oxide europium(III) (1b-191) (actual yield, 106 mg; percent yield, 25%). ¹⁹F-NMR (376 MHz, CDCl₃) δ (ppm): -71.6 (s, 0.3F), - 71.7 (s, 2.2F), -71.8 (s, 5.2F), -72.9 (s, 0.3F), -73.0 (s, 1.0F). ³¹P-NMR (162 MHz, CDCl₃) δ (ppm): -103.7 (brs). ESIMS (m/z), MeOH: 1874.8 [M + Na]⁺, 1474.5 [M - (1-methyl-4-hydroxy-3-(2,2,2-trifluoroethan-1-on-1-yl)-[1]benzothieno[3,2-h]quinolin-2(1H)-onato)]⁺.

### (EXAMPLE 80)

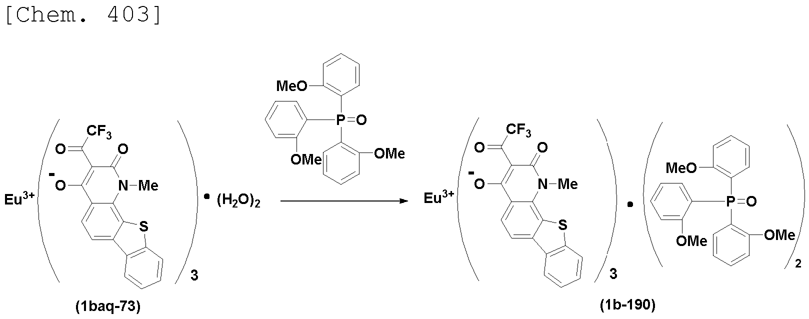

The diaquatris[1-methyl-4-hydroxy-3-(2,2,2-trifluoroethan-1-on-1-yl)-[1]benzothieno[3,2-h]quinolin-2(1H)-onato]europium(III) (1baq-73) obtained as an intermediate in Example 69 (300 mg, 230 µmol) and tris(2-methoxyphenyl)phosphine oxide (168 mg, 460 pmol) were dissolved in acetone (23.0 mL), and the resulting solution was stirred for 3 hours at room temperature. The reaction mixture was filtered through Celite, and then the filtrate was concentrated under reduced pressure. After slurry was washed away with acetone/hexane, the residue was reprecipitated with acetone/hexane to give a yellow solid of tris[1-methyl-4-hydroxy-3-(2,2,2-trifluoroethan-1-on-1-yl)benzo[4,5]thieno[3,2-h]quinolin-2(1H)-onato]bis[tris(2-methoxyphenyl)phosphine oxide]europium(III) (1b-190) (actual yield, 346 mg; percent yield, 75%). ¹⁹F-NMR (376 MHz, CDCl₃) δ (ppm): -71.3 (s, 2.4F), -71.4 (s, 5.3F), -72.7 (s, 1.3F). ³¹P-NMR (162 MHz, CDCl₃) δ (ppm): -84.0 (brs). ESIMS (m/z), MeOH: 2042.0 [M + Na]⁺, 1671.8 [M - (triphenylphosphine oxide) + Na]⁺, 1640.5 [M - (1-methyl-4-hydroxy-3-(2,2,2-trifluoroethan-1-on-1-yl)-[1]benzothieno[3,2-h]quinolin-2(1H)-onato)]⁺.

### (EXAMPLE 81)

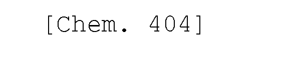

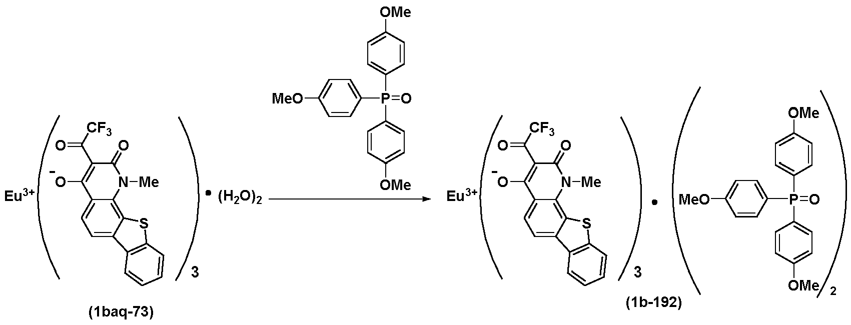

The diaquatris[1-methyl-4-hydroxy-3-(2,2,2-trifluoroethan-1-on-1-yl)-[1]benzothieno[3,2-h]quinolin-2(1H)-onato]europium(III) (1baq-73) obtained as an intermediate in Example 69 (264 mg, 201 µmol) and tris(4-methoxyphenyl)phosphine oxide (148 mg, 400 µmol) were dissolved in acetone (20.0 mL), and the resulting solution was stirred for 3 hours at room temperature. The reaction mixture was filtered through Celite, then the filtrate was concentrated under reduced pressure, and the residue was reprecipitated with acetone/hexane to give a yellow solid of tris[1-methyl-4-hydroxy-3-(2,2,2-trifluoroethan-1-on-1-yl)benzo[4,5]thieno[3,2-h]quinolin-2(1H)-onato]bis[tris(4-methoxyphenyl)phosphine oxide]europium(III) (1b-192) (actual yield, 309 mg; percent yield, 76%). ¹⁹F-NMR (376 MHz, Acetone-d₆) δ (ppm): -71.3 (s, 7.0F), -72.5 (s, 2.0F). ³¹P-NMR (162 MHz, Acetone-d₆) δ (ppm): -88.6 (brs). ESIMS (m/z), MeOH: 2039.8 [M + Na]⁺, 1671.8 [M - (triphenylphosphine oxide) + Na]⁺, 1641.2 [M - (1-methyl-4-hydroxy-3-(2,2,2-trifluoroethan-1-on-1-yl)-[1]benzothieno[3,2-h]quinolin-2(1H)-onato)]⁺.

### (EXAMPLE 82)

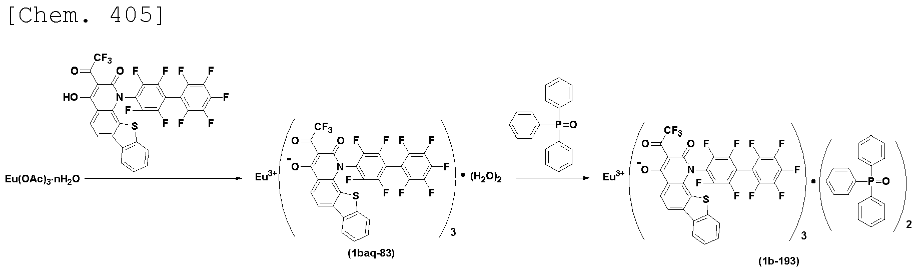

Ethanol (1.00 mL) and water (300 µL) were added to europium(III) acetate hydrate (10.2 mg as a 2.5-hydrate, 27.5 µmol) and the 4-hydroxy-1-(perfluoro-[1,1'-biphenyl]-4-yl)-3-(2,2,2-trifluoroethan-1-on-1-yl)-[1]benzothieno[3,2-h]quinolin-2(1H)-one obtained in Reference Example 46 (55.4 mg, 81.8 pmol). Then a 1 M aqueous solution of sodium hydroxide (820 µL, 820 µmol) was added, and the resulting mixture was stirred for 1 hour at room temperature. After the ethanol was distilled away, water was put into the reaction mixture, the resulting mixture was filtered, and the collected solid was washed with water. The washed solid was dried by heating to give a deep orange powder solid of diaquatris[4-hydroxy-1-(perfluoro-[1,1'-biphenyl]-4-yl)-3-(2,2,2-trifluoroethan-1-on-1-yl)-[1]benzothieno[3,2-h]quinolin-2(1H)-onato]europium(III) (1baq-83) (actual yield, 38.4 mg; percent yield, 630). The resulting crude product was used in the next reaction directly, without further purification.

The diaquatris[4-hydroxy-1-(perfluoro-[1,1'-biphenyl]-4-yl)-3-(2,2,2-trifluoroethan-1-on-1-yl)-[1]benzothieno[3,2-h]quinolin-2(1H)-onato]europium(III) (1baq-83) (38.4 mg, 17.3 µmol) and triphenylphosphine oxide (9.60 mg, 34.5 µmol) were dissolved in acetone (1.80 mL), and the resulting solution was stirred for 3 hours at room temperature. The reaction mixture was filtered, then the filtrate was concentrated under reduced pressure, and the residue was washed with diethyl ether to give a yellow solid of tris[4-hydroxy-1-(perfluoro-[1,1'-biphenyl]-4-yl)-3-(2,2,2-trifluoroethan-1-on-1-yl)-[1]benzothieno[3,2-h]quinolin-2(1H)-onato]bis(triphenylphosphine oxide)europium(III) (1b-193) (actual yield, 27.0 mg; percent yield, 57%). ¹⁹F-NMR (376 MHz, CDCl₃) δ (ppm): -72.3 (s, 9F), -136.2 (dd, J = 14.7, 7.7 Hz, 3F), -137.3 (dd, J = 14.7, 7.7 Hz, 3F), -138.2 (dd, J = 20.3, 7.7 Hz, 6F), -142.5 (dd, J = 20.3, 7.7 Hz, 6F), -149.8 (t, J = 20.3 Hz, 3F), -160.0 (ddd, J = 20.3, 20.3, 7.7 Hz, 3F), -160.6 (ddd, J = 20.3, 20.3, 7.7 Hz, 3F). ³¹P-NMR (162 MHz, CDCl₃) δ (ppm): -80.7 (brs). ESIMS (m/z), MeOH: 2760.1 [M + Na]⁺, 2480.3 [M - (triphenylphosphine oxide) + Na]⁺, 2059.6 [M - (4-hydroxy-1-(perfluoro-[1,1'-biphenyl]-4-yl)-3-(2,2,2-trifluoroethan-1-on-1-yl)-[1]benzothieno[3,2-h]quinolin-2(1H)-onato)]⁺.

### (EXAMPLE 83)

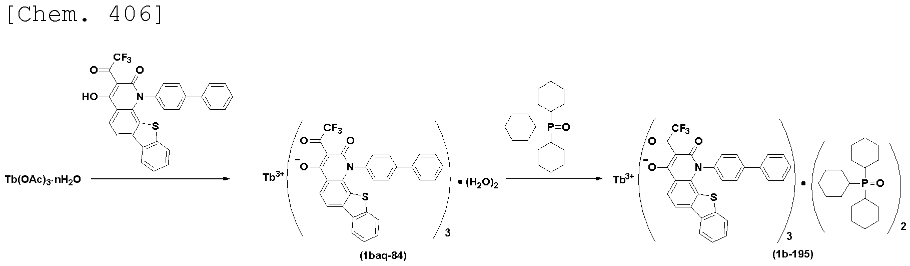

Ethanol (3.70 mL) and water (1.20 mL) were added to terbium(III) acetate (40.8 mg as a 2.5-hydrate, 100 µmol) and the 1-(4-biphenylyl)-4-hydroxy-3-(2,2,2-trifluoroethan-1-on-1-yl)-[1]benzothieno[3,2-h]quinolin-2(1H)-one obtained in Reference Example 18 (155 mg, 300 pmol). A 1 M aqueous solution of sodium hydroxide (300 µL, 300 µmol) was added, and the resulting mixture was stirred for 1 hour at room temperature. After the ethanol was distilled away, water was put into the reaction mixture, the resulting mixture was filtered, and the collected solid was washed with water. The washed solid was dried by heating to give a brown solid of diaquatris[1-(4-biphenylyl)-4-hydroxy-3-(2,2,2-trifluoroethan-1-on-1-yl)-[1]benzothieno[3,2-h]quinolin-2(1H)-onato]terbium(III) (1baq-84) (actual yield, 120 mg; percent yield, 69%). The resulting crude product was used in the next reaction directly, without further purification. ESIMS (m/z), MeOH: 1723.7 [M - 2H₂O + Na]⁺.

Acetone (6.90 mL) was added to the diaquatris[1-(4-biphenylyl)-4-hydroxy-3-(2,2,2-trifluoroethan-1-on-1-yl)-[1]benzothieno[3,2-h]quinolin-2(1H)-onato]terbium(III) (1baq-84) (120 mg, 69.0 µmol) and tricyclohexylphosphine oxide (41.2 mg, 139 pmol), and the resulting mixture was stirred for 3 hours at room temperature. The reaction mixture was filtered, then the filtrate was concentrated under reduced pressure, and the residue was reprecipitated with acetone/hexane to give a yellow solid of tris[1-(4-biphenylyl)-4-hydroxy-3-(2,2,2-trifluoroethan-1-on-1-yl)-[1]benzothieno[3,2-h]quinolin-2(1H)-onato]bis(tricyclohexylphosphine oxide)terbium(III) (1b-195) (actual yield, 75.9 mg; percent yield, 48%). ESIMS (m/z), MeOH: 2318.3 [M + Na]⁺, 2020.1 [M - (tricyclohexylphosphine oxide) + Na]⁺, 1779.2 [M - (1-(4-biphenylyl)-4-hydroxy-3-(2,2,2-trifluoroethan-1-on-1-yl)-[1]benzothieno[3,2-h]quinolin-2(1H)-onato)]⁺.

### (EXAMPLE 84)

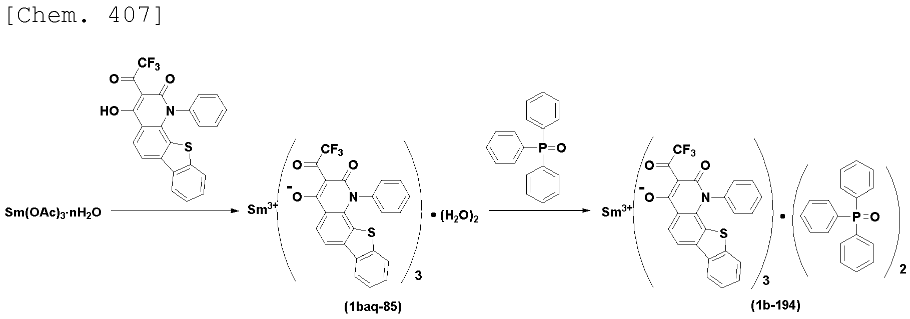

Ethanol (11.2 mL) and water (3.74 mL) were added to samarium(III) acetate (98.0 mg as a 2.5-hydrate, 299 µmol) and the 1-phenyl-4-hydroxy-3-(2,2,2-trifluoroethan-1-on-1-yl)-[1]benzothieno[3,2-h]quinolin-2(1H)-one obtained in Reference Example 30 (395 mg, 899 µmol). A 1 M aqueous solution of sodium hydroxide (890 µL, 890 µmol) was added, and the resulting mixture was stirred for 1 hour at room temperature. After the ethanol was distilled away, water was put into the reaction mixture, the resulting mixture was filtered, and the collected solid was washed with water. The washed solid was dried by heating to give a brown solid of diaquatris[1-(4-biphenylyl)-4-hydroxy-3-(2,2,2-trifluoroethan-1-on-1-yl)-[1]benzothieno[3,2-h]quinolin-2(1H)-onato]samarium(III) (1baq-85) (actual yield, 364 mg; percent yield, 81%). The resulting crude product was used in the next reaction directly, without further purification.

Acetone (24.0 mL) was added to the diaquatris[1-phenyl-4-hydroxy-3-(2,2,2-trifluoroethan-1-on-1-yl)-[1]benzothieno[3,2-h]quinolin-2(1H)-onato]samarium(III) (1baq-85) (364 mg, 242 µmol) and triphenylphosphine oxide (135 mg, 485 µmol), and the resulting mixture was stirred for 3 hours at room temperature. The reaction mixture was filtered, then the filtrate was concentrated under reduced pressure, and the residue was reprecipitated with acetone/hexane to give a yellow solid of tris[1-phenyl-4-hydroxy-3-(2,2,2-trifluoroethan-1-on-1-yl)-[1]benzothieno[3,2-h]quinolin-2(1H)-onato]bis(triphenylphosphine oxide)samarium(III) (1b-194) (actual yield, 362 mg; percent yield, 74%). ¹⁹F-NMR (376 MHz, CDCl₃) δ (ppm): -71.7 (s). ³¹P-NMR (162 MHz, CDCl₃) δ (ppm): -31.3 (brs). ESIMS (m/z), MeOH: 1584.1 [M - (1-phenyl-4-hydroxy-3-(2,2,2-trifluoroethan-1-on-1-yl)-[1]benzothieno[3,2-h]quinolin-2(1H)-onato)]⁺.

### (EXAMPLE 85)

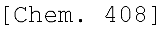

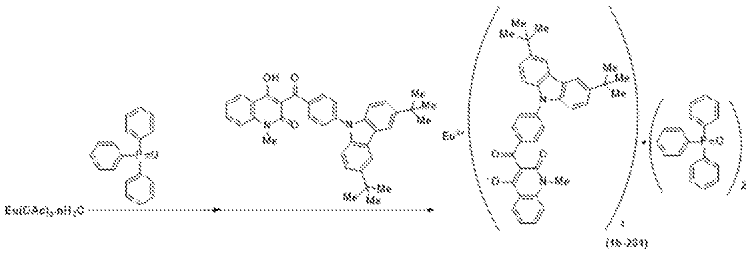

Methanol (20.0 mL) was added to europium acetate n-hydrate (147 mg as a 2.5-hydrate, 392 µmol) and triphenylphosphine oxide (219 mg, 787 µmol), and the resulting mixture was stirred for 1 hour at room temperature. The 3-[4-(3,6-di-tert-butyl-9H-carbazol-9-yl)benzoyl]-4-hydroxy-1-methylquinolin-2(1H)-one obtained in Reference Example 47 (654 mg, 1.17 mmol) was added to the reaction mixture. 14.8 M aqueous ammonia (80.0 µL, 1.18 mmol) was added dropwise, and the resulting mixture was stirred for 3 hours at room temperature. The reaction solution was concentrated under reduced pressure, then the residue was dissolved by adding chloroform to it, and the resulting solution was concentrated under reduced pressure. The resulting solid was washed with a small amount of diethyl ether to give a yellow solid of tris{3-[4-(3,6-di-tert-butyl-9H-carbazol-9-yl)benzoyl]-4-hydroxy-1-methylquinolin-2(1H)-onato}bis(triphenylphosphine oxide)europium(III) (1b-201) (actual yield, 405 mg; percent yield, 44%) . ³¹P-NMR (162 MHz, CDCl₃) δ (ppm) : -76.4 (brs) . ESIMS (m/z), MeOH: 2399.5 [M + Na]⁺, 1841.1 [M - (triphenylphosphine oxide) + Na]⁺, 1818.0 [M - (3-[4-(3,6-di-tert-butyl-9H-carbazol-9-yl)benzoyl]-4-hydroxy-1-methylquinolin-2(1H)-onato)]⁺.

### (EXAMPLE 86)

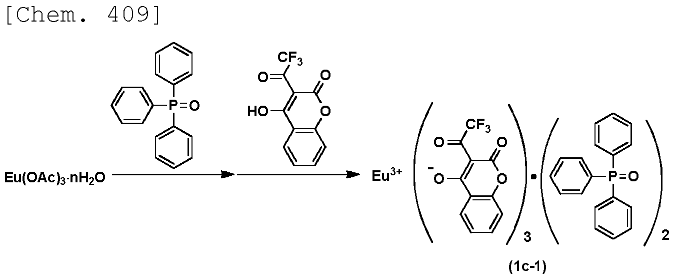

Methanol (15.0 mL) was added to europium acetate n-hydrate (1.12 g, 2.99 mmol as a 2.5-hydrate) and triphenylphosphine oxide (1.67 g, 6.00 mmol), and the resulting mixture was stirred for 1 hour at room temperature. The 4-hydroxy-3-(2,2,2-trifluoroethan-1-on-1-yl)-2H-chromen-2-one obtained in Reference Example 48 (2.32 g, 8.99 mmol) was added to the reaction mixture, and the resulting mixture was stirred for 3 hours at room temperature. The reaction solution was emptied into water, and the precipitated solid was collected by suction filtration and then washed with water. This solid was suspended in methylcyclohexane, dehydrated by azeotropic distillation and then allowed to cool, and the solid was collected by suction filtration to give a white solid of tris[4-hydroxy-3-(2,2,2-trifluoroethan-1-on-1-yl)-2H-chromen-2-onato]bis(triphenylphosphine oxide)europium(III) (1c-1) (4.08 g, 92%). ¹⁹F-NMR (376 MHz, CDCl₃) δ (ppm): - 73.1 (brs) . ³¹P-NMR (162 MHz, CDCl₃) δ (ppm) : -85.1 (brs) . ESIMS (m/z), MeOH: 1223.3 [M - (4-hydroxy-3-(2,2,2-trifluoroethan-1-on-1-yl)-2H-chromen-2-onato)]⁺.

### (EXAMPLE 87)

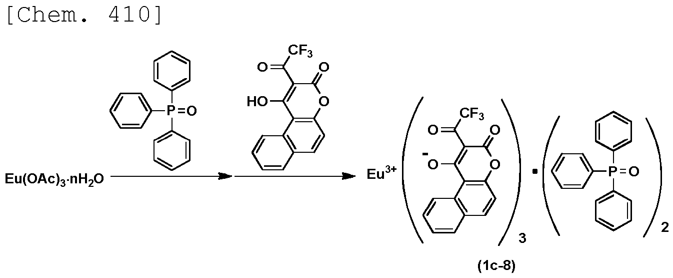

Methanol (4.00 mL) was added to europium acetate n-hydrate (75.0 mg, 204 µmol as a 2.5-hydrate) and triphenylphosphine oxide (111 mg, 399 µmol), and the resulting mixture was stirred for 1.5 hours at room temperature. The 1-hydroxy-2-(2,2,2-trifluoroethan-1-on-1-yl)-3H-naphtho[2,1-b]pyran-3-one obtained in Reference Example 49 (185 mg, 600 µmol) was added to the reaction mixture, and the resulting mixture was stirred for 4 hours at room temperature. The reaction solution was emptied into water, and the precipitated solid was collected by suction filtration and then washed with water. This solid was suspended in methylcyclohexane, dehydrated by azeotropic distillation and then allowed to cool, and the solid was collected by suction filtration to give an ocherous solid of tris[1-hydroxy-2-(2,2,2-trifluoroethan-1-on-1-yl)-3H-naphtho[2,1-b]pyran-3-onato]bis(triphenylphosphine oxide)europium(III) (1c-8) (actual yield, 183 mg; percent yield from europium acetate 2.5-hydrate, 56%). ¹⁹F-NMR (376 MHz, CDCl₃) δ (ppm): -71.4 (s, 0.5F), -73.4 (s, 7.3F), -74.0 (s, 1.2F). ³¹P-NMR (162 MHz, CDCl₃) δ (ppm): -80.7 (brs, 0.4P), -81.8 (brs, 1.6P). ESIMS (m/z), MeOH: 1323.1 [M - (1-hydroxy-2-(2,2,2-trifluoroethan-1-on-1-yl)-3H-naphtho[2,1-b]pyran-3-onato)]⁺.

### (EXAMPLE 88)

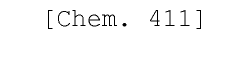

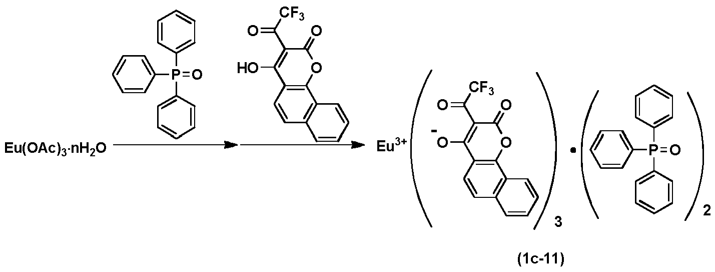

Acetone (4.00 mL) was added to europium acetate n-hydrate (75.0 mg, 200 µmol as a 2.5-hydrate) and triphenylphosphine oxide (111 mg, 399 µmol), and the resulting mixture was stirred for 0.5 hours at room temperature. The 4-hydroxy-3-(2,2,2-trifluoroethan-1-on-1-yl)-2H-naphtho[1,2-b]pyran-2-one obtained in Reference Example 50 (185 mg, 600 µmol) was added to the reaction mixture, and the resulting mixture was stirred for 2 hours at room temperature. Undissolved solids were removed by filtration, the filtrate was emptied into water, and the precipitated solid was collected by suction filtration and then washed with water. This solid was suspended in methylcyclohexane, dehydrated by azeotropic distillation and then allowed to cool, and the solid was collected by suction filtration to give a light yellow solid of tris[4-hydroxy-3-(2,2,2-trifluoroethan-1-on-1-yl)-2H-naphtho[1,2-b]pyran-2-onato]bis(triphenylphosphine oxide)europium(III) (1c-11) (actual yield, 123 mg; percent yield from europium acetate 2.5-hydrate, 38%) . ¹⁹F-NMR (376 MHz, CDCl₃) δ (ppm): -73.3 (s, 9F). ³¹P-NMR (162 MHz, CDCl₃) δ (ppm): -83.8. ESIMS (m/z), MeOH: 1323.1 [M - (4-hydroxy-3-(2,2,2-trifluoroethan-1-on-1-yl)-2H-naphtho[1,2-b]pyran-2-onato)]⁺.

### (EXAMPLE 89)

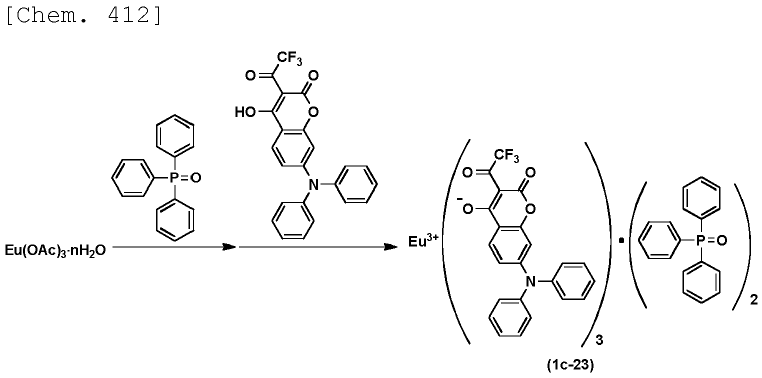

Methylcyclohexane (10.0 mL) was added to europium acetate n-hydrate (75.0 mg, 200 µmol as a 2.5-hydrate) and triphenylphosphine oxide (111 mg, 399 µmol), and the resulting mixture was stirred for 0.5 hours at room temperature. The 7-(diphenylamino)-4-hydroxy-3-(2,2,2-trifluoroethan-1-on-1-yl)-2H-chromen-2-one obtained in Reference Example 51 (255 mg, 599 µmol) was added to the reaction mixture. A Dean-Stark apparatus was attached, and reflux was performed for 5 hours. After natural cooling, undissolved solids were collected by suction filtration and then washed with methylcyclohexane to give a yellow solid of tris[7-(diphenylamino)-4-hydroxy-3-(2,2,2-trifluoroethan-1-on-1-yl)-2H-chromen-2-onato]bis(triphenylphosphine oxide)europium(III) (1c-23) (actual yield, 197 mg; percent yield from europium acetate 2.5-hydrate, 50%). ¹⁹F-NMR (376 MHz, CDCl₃) δ (ppm) : -73.3. ³¹P-NMR (162 MHz, CDCl₃) δ (ppm) : -81.3 (brs). ESIMS (m/z), MeOH: 1557.5 [M - (7-(diphenylamino)-4-hydroxy-3-(2,2,2-trifluoroethan-1-on-1-yl)-2H-chromen-2-onato)]⁺.

### (EXAMPLE 90)

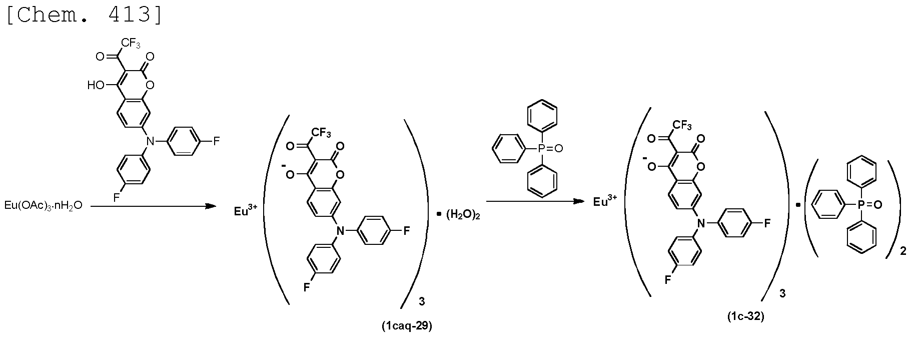

Methanol (20.0 mL) and water (10.0 mL) were added to sodium hydrogencarbonate (126 mg, 1.50 mmol) and europium acetate n-hydrate (187 mg, 500 µmol as a 2.5-hydrate). Subsequently, the 7-[bis(4-fluorophenyl)amino]-4-hydroxy-3-(2,2,2-trifluoroethan-1-on-1-yl)-2H-chromen-2-one obtained in Reference Example 52 (692 mg, 1.50 mmol) was added to the reaction mixture, and the resulting mixture was stirred for 2 hours at room temperature. The reaction mixture was suction-filtered, and the resulting solid was washed with water to give diaquatris[7-[bis(4-fluorophenyl)amino]-3-(2,2,2-trifluoroethan-1-on-1-yl)-2H-chromen-2-onato]europium(III) (1caq-29) (actual yield, 943 mg; percent yield, quant.). The resulting crude product was used in the next reaction directly, without further purification.

Then the diaquatris[7-[bis(4-fluorophenyl)amino]-3-(2,2,2-trifluoroethan-1-on-1-yl)-2H-chromen-2-onato]europium(III) (157 mg, 100 µmol) was suspended in methylcyclohexane (20 mL), and triphenylphosphine oxide (55.7 mg, 200 µmol) was added. A Dean-Stark apparatus was attached, and reflux was performed for 1 hour. After natural cooling, undissolved solids were collected by suction filtration and washed with methylcyclohexane to give a yellow solid of tris{7-[bis(4-fluorophenyl)amino]-3-(2,2,2-trifluoroethan-1-on-1-yl-2H-chromen-2-onato}bis(triphenylphosphine oxide)europium(III) (1c-32) (actual yield, 35.3 mg; percent yield from europium acetate 2.5-hydrate, 20%) . ¹⁹F-NMR (376 MHz, CDCl₃) δ (ppm) : -73.3 (s, 9F), -115.9 (s, 6F). ³¹P-NMR (162 MHz, CDCl₃) δ (ppm): - 82.3 (brs). ESIMS (m/z), MeOH: 1628.3 [M - (7-[bis(4-fluorophenyl)amino]-3-(2,2,2-trifluoroethan-1-on-1-yl)-2H-chromen-2-onato)]⁺.

### (EXAMPLE 91)

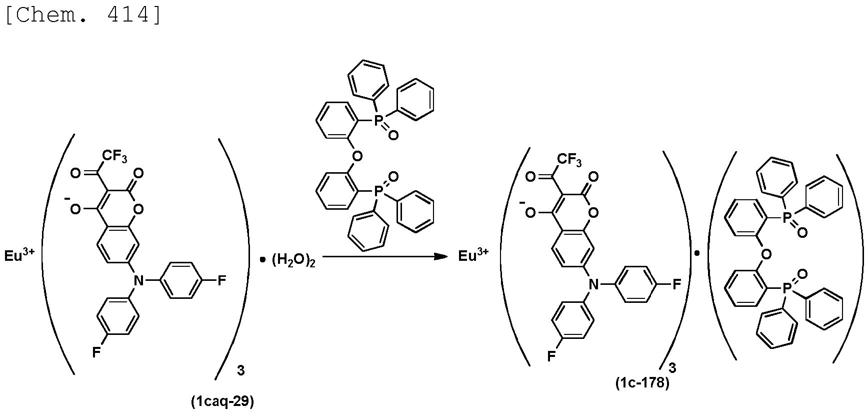

The diaquatris[7-[bis(4-fluorophenyl)amino]-3-(2,2,2-trifluoroethan-1-on-1-yl)-2H-chromen-2-onatoJeuropium(III) (1caq-29) obtained as an intermediate in Example 90 (157 mg, 100 µmol) was suspended in methylcyclohexane (20.0 mL), and bis[2-[(oxo)diphenylphosphino]phenyl]ether (57.1 mg, 100 µmol) was added. A Dean-Stark apparatus was attached, and reflux was performed for 1 hour. After natural cooling, undissolved solids were collected by suction filtration, refluxed in methylcyclohexane again and allowed to cool. The solid was collected by suction filtration to give a yellow solid of {bis[2-(diphenylphosphino)phenyl]ether oxide}tris[7-[bis(4-fluorophenyl)amino]-3-(2,2,2-trifluoroethan-1-on-1-yl)-2H-chromen-2-onatoJeuropium(III) (1c-178) (actual yield, 139 mg; percent yield from europium acetate 2.5-hydrate, 790) . ¹⁹F-NMR (376 MHz, CDCl₃) δ (ppm) : -73.4 (s, 9F), -116.2 (s, 6F). ³¹P-NMR (162 MHz, CDCl₃) δ (ppm): -101.6. ESIMS (m/z), MeOH: 1643.2 [M - (7-[bis(4-fluorophenyl)amino]-3-(2,2,2-trifluoroethan-1-on-1-yl)-2H-chromen-2-onato)]⁺.

### (EXAMPLE 92)

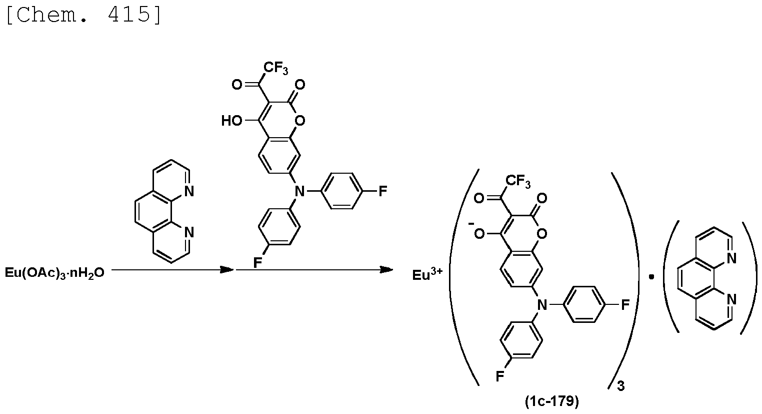

Methanol (4.00 mL) was added to europium acetate n-hydrate (75.0 mg, 200 µmol as a 2.5-hydrate) and 1,10-phenanthroline (36.0 mg, 200 mmol), and the resulting mixture was stirred for 0.5 hours at room temperature. The 7-[bis(4-fluorophenyl)amino]-4-hydroxy-3-(2,2,2-trifluoroethan-1-on-1-yl)-2H-chromen-2-one obtained in Reference Example 52 (277 mg, 600 µmol) was added to the reaction mixture, and the resulting mixture was stirred for 3 hours at room temperature. The reaction solution was emptied into water, and the precipitated solid was collected by suction filtration, dehydrated by azeotropic distillation in methylcyclohexane and then collected by suction filtration again to give an orange solid of tris[7-[bis(4-fluorophenyl)amino]-3-(2,2,2-trifluoroethan-1-on-1-yl)-2H-chromen-2-onato](1,10-phenanthroline)europium(III) (1c-179) (actual yield, 294 mg; percent yield from europium acetate 2.5-hydrate, 860) . ¹⁹F-NMR (376 MHz, CDCl₃) δ (ppm) : -72.6 (s, 9F), -115.3 (s, 6F). ESIMS (m/z), MeCN: 1253.1 [M - (7-[bis(4-fluorophenyl)amino]-3-(2,2,2-trifluoroethan-1-on-1-yl)-2H-chromen-2-onato)]⁺.

### (EXAMPLE 93)

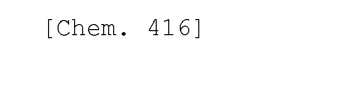

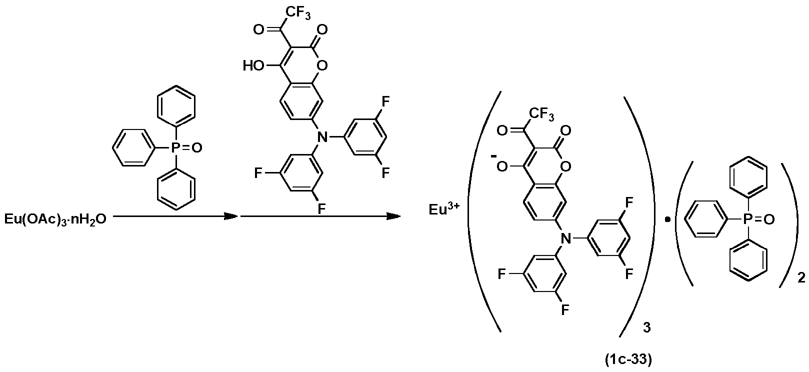

Methylcyclohexane (10.0 mL) was added to europium acetate n-hydrate (75.0 mg, 200 µmol as a 2.5-hydrate) and triphenylphosphine oxide (111 mg, 399 µmol), and the resulting mixture was stirred for 0.5 hours at room temperature. The 7-[bis(3,5-difluorophenyl)amino]-4-hydroxy-3-(2,2,2-trifluoroethan-1-on-1-yl)-2H-chromen-2-one obtained in Reference Example 53 (299 mg, 601 µmol) was added to the reaction mixture. A Dean-Stark apparatus was attached, and reflux was performed for 1 hour. After natural cooling, the precipitated solid was collected by suction filtration and then washed with methylcyclohexane to give a yellow solid of tris{7-[bis(3,5-difluorophenyl)amino]-3-(2,2,2-trifluoroethan-1-on-1-yl)-2H-chromen-2-onato}bis(triphenylphosphine oxide)europium(III) (1c-33) (actual yield, 492 mg; percent yield from europium acetate 2.5-hydrate, quant.). ¹⁹F-NMR (376 MHz, CDCl₃) δ (ppm): -73.0 (s, 9F), -107.8 (s, 12F). ³¹P-NMR (162 MHz, CDCl₃) δ (ppm): -86.3. ESIMS (m/z), MeOH: 1701.0 [M - (7-[bis(3,5-difluorophenyl)amino]-3-(2,2,2-trifluoroethan-1-on-1-yl)-2H-chromen-2-onato)]⁺.

### (EXAMPLE 94)

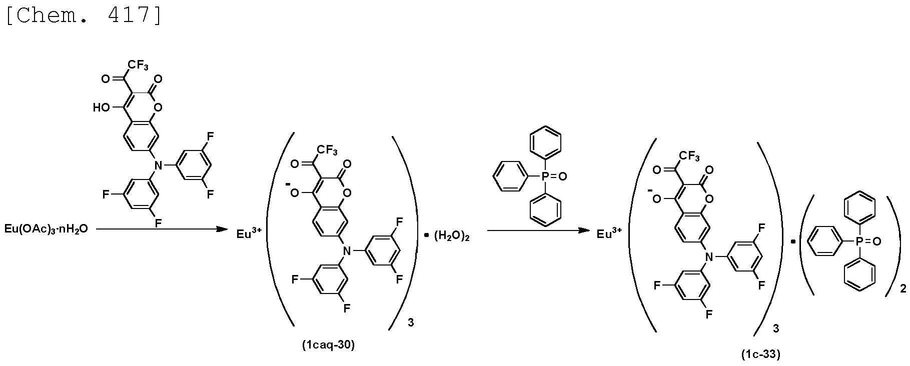

Water (10.0 mL) and chloroform (10 mL) were added to sodium hydrogencarbonate (126 mg, 1.50 mmol) and europium acetate n-hydrate (187 mg, 500 µmol as a 2.5-hydrate). The 7-[bis(3,5-difluorophenyl)amino]-4-hydroxy-3-(2,2,2-trifluoroethan-1-on-1-yl)-2H-chromen-2-one obtained in Reference Example 53 (746 mg, 1.50 mmol) was added, and the resulting mixture was stirred for 2 hours at room temperature. The reaction mixture was suction-filtered, and the resulting solid was washed with water, giving diaquatris{7-[bis(3,5-difluorophenyl)amino]-3-(2,2,2-trifluoroethan-1-on-1-yl)-2H-chromen-2-onato}europium(III) (1caq-30) (actual yield, 1.22 g; percent yield, quant.). The resulting crude product was used in the next reaction directly, without further purification.

The diaquatris{7-[bis(3,5-difluorophenyl)amino]-3-(2,2,2-trifluoroethan-1-on-1-yl)-2H-chromen-2-onato}europium(III) (168 mg, 100 µmol) was suspended in methylcyclohexane (20.0 mL), and triphenylphosphine oxide (55.7 mg, 200 mmol) was added. A Dean-Stark apparatus was attached, and reflux was performed for 1 hour. After natural cooling, the precipitated solid was collected by suction filtration and recrystallized from methylcyclohexane to give a yellow solid of tris{7-[bis(3,5-difluorophenyl)amino]-3-(2,2,2-trifluoroethan-1-on-1-yl)-2H-chromen-2-onato}bis(triphenylphosphine oxide)europium(III) (1c-33) (actual yield, 103 mg; percent yield from europium acetate 2.5-hydrate, 68%). ¹⁹F-NMR (376 MHz, CDCl₃) δ (ppm): -73.0 (s, 9F), -107.8 (s, 12F). ³¹P-NMR (162 MHz, CDCl₃) δ (ppm): -85.4. ESIMS (m/z), MeOH: 1701.5 [M - (7-[bis(3,5-difluorophenyl)amino]-3-(2,2,2-trifluoroethan-1-on-1-yl)-2H-chromen-2-onato)]⁺, 1423.3 [M - (7-[bis(3,5-difluorophenyl)amino]-3-(2,2,2-trifluoroethan-1-on-1-yl)-2H-chromen-2-onato) - (triphenylphosphine oxide)]⁺.

### (EXAMPLE 95)

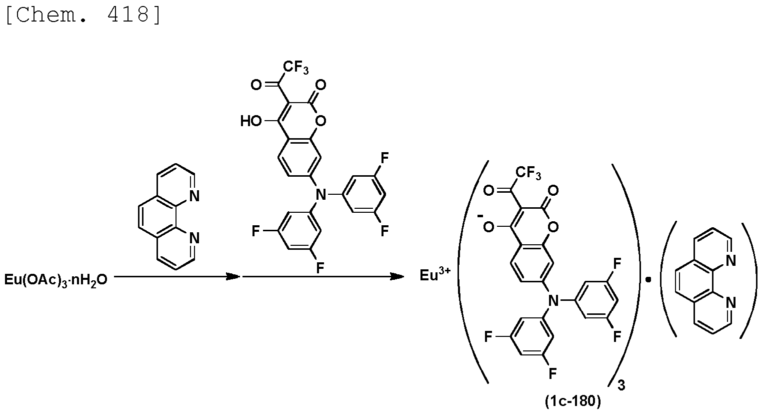

Methanol (4.00 mL) was added to europium acetate n-hydrate (75.0 mg, 200 µmol as a 2.5-hydrate) and 1,10-phenanthroline (36.0 mg, 200 µmol), and the resulting mixture was stirred for 0.5 hours at room temperature. The 7-[bis(3,5-difluorophenyl)amino]-4-hydroxy-3-(2,2,2-trifluoroethan-1-on-1-yl)-2H-chromen-2-one obtained in Reference Example 53 (298 mg, 600 µmol) was added to the reaction mixture, and the resulting mixture was stirred for 3 hours at room temperature. Undissolved solids were removed, and the filtrate was emptied into water. The precipitated solid was collected by suction filtration, dehydrated by azeotropic distillation in methylcyclohexane and collected by suction filtration again to give a yellow solid of tris{7-[bis(3,5-difluorophenyl)amino]-3-(2,2,2-trifluoroethan-1-on-1-yl)-2H-chromen-2-onato} (1,10-phenanthroline)europium(III) (1c-180) (actual yield, 267 mg; percent yield from europium acetate 2.5-hydrate, 73%). ¹⁹F-NMR (376 MHz, CDCl₃) δ (ppm): -72.7 (s, 9F), -107.6 (s, 12F). ESIMS (m/z), MeCN: 1843.9 [M + Na]⁺.

### (EXAMPLE 96)

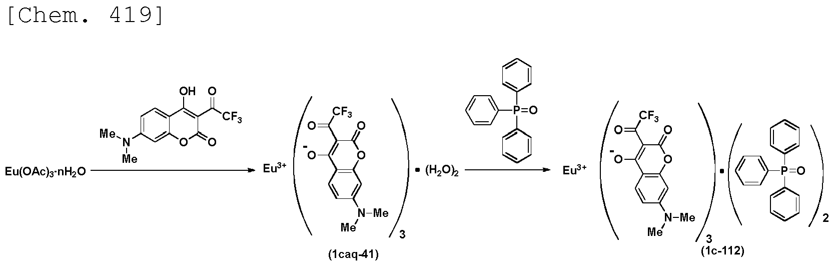

Ethanol (7.50 mL) and water (2.50 mL) were added to europium(III) acetate hydrate (75.0 mg as a 2.5-hydrate, 200 pmol) and the 7-(dimethylamino)-4-hydroxy-3-(2,2,2-trifluoroethyl-1-on-1-yl)-2H-chromen-2-one obtained in Reference Example 54 (181 mg, 600 pmol). A 1 M aqueous solution of sodium hydroxide (600 µL, 600 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a brown solid of diaquatris[7-(dimethylamino)-4-hydroxy-3-(2,2,2-trifluoroethyl-1-on-1-yl)-2H-chromen-2-onato]europium(III) (1caq-41) (actual yield, 144 mg; percent yield, 66%). The resulting crude product was used in the next reaction directly, without further purification.

Acetone (12.0 mL) was added to the diaquatris[7-(dimethylamino)-4-hydroxy-3-(2,2,2-trifluoroethyl-1-on-1-yl)-2H-chromen-2-onato]europium(III) (130 mg, 120 µmol) and triphenylphosphine oxide (66.5 mg, 240 µmol), and the resulting mixture was stirred for 3 hours at room temperature. The precipitated solid was collected by filtration and washed with acetone to give a yellow solid of tris[7-(dimethylamino)-4-hydroxy-3-(2,2,2-trifluoroethyl-1-on-1-yl)-2H-chromen-2-onato]bis(triphenylphosphine oxide)europium(III) (1c-112) (actual yield, 101 mg; percent yield, 52%) . ¹⁹F-NMR (376 MHz, CDCl₃) δ (ppm) : -73.2 (s) . ³¹P-NMR (162 MHz, CDCl₃) δ (ppm): -57.8 (brs). ESIMS (m/z), MeOH: 1354.2 [M - (triphenylphosphine oxide) + Na]⁺, 1308.2 [M - (7-(dimethylamino)-4-hydroxy-3-(2,2,2-trifluoroethyl-1-on-1-yl)-2H-chromen-2-onato)]⁺.

### (EXAMPLE 97)

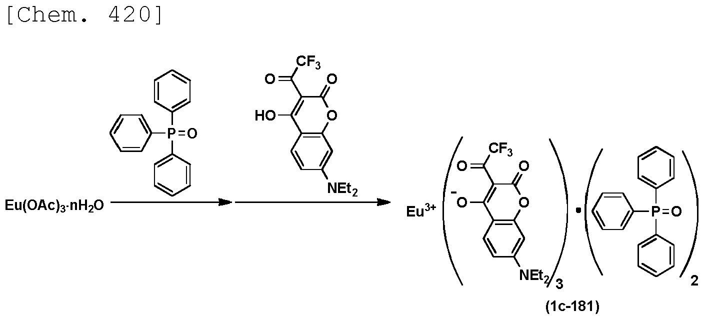

Methanol (4.00 mL) was added to europium acetate n-hydrate (75.0 mg, 200 µmol as a 2.5-hydrate) and triphenylphosphine oxide (111 mg, 399 µmol), and the resulting mixture was stirred for 1.5 hours at room temperature. The 7-(diethylamino)-4-hydroxy-3-(2,2,2-trifluoroethan-1-on-1-yl)-2H-chromen-2-one obtained in Reference Example 55 (198 mg, 600 µmol) was added to the reaction mixture, and the resulting mixture was stirred for 4 hours at room temperature. The reaction solution was poured into water, and the precipitated solid was collected by suction filtration and then washed with water. This solid was suspended in methylcyclohexane, dehydrated by azeotropic distillation and then allowed to cool, and the solid was collected by suction filtration to give an orange-yellow solid of tris[7-(diethylamino)-3-(2,2,2-trifluoroethan-1-on-1-yl)-2H-chromen-2-onato]bis(triphenylphosphine oxide)europium(III) (1c-181) (actual yield, 202 mg; percent yield from europium acetate 2.5-hydrate, 60%) . ¹⁹F-NMR (376 MHz, CDCl₃) δ (ppm) : -73.3. ³¹P-NMR (162 MHz, CDCl₃) δ (ppm): -75.7. ESIMS (m/z), MeOH: 1365.4 [M - (7-(diethylamino)-3-(2,2,2-trifluoroethan-1-on-1-yl)-2H-chromen-2-onato)]⁺.

### (EXAMPLE 98)

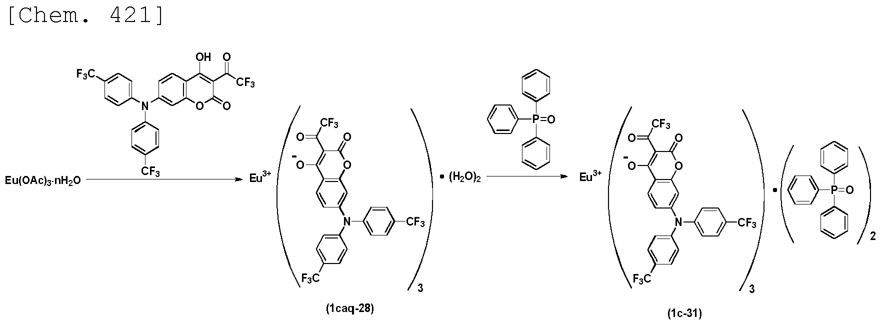

Europium acetate n-hydrate (37.0 mg as a 2.5-hydrate, 100 µmol) and the 7-{bis[4-(trifluoromethyl)phenyl]amino}-4-hydroxy-3-(2,2,2-trifluoroethyl-1-on-1-yl)-2H-chromen-2-one obtained in Reference Example 56 (168 mg, 300 mmol) were suspended in ethanol (4.90 mL) and water (1.6 mL). Then a 1 M aqueous solution of sodium hydroxide (300 µL) was added, and the resulting mixture was stirred for 3 hours at room temperature. The reaction mixture was concentrated under reduced pressure, and then the resulting solid was washed with water and dried by heating to give a yellow solid of diaquatris(7-{bis[4-(trifluoromethyl)phenyl]amino}-4-hydroxy-3-(2,2,2-trifluoroethyl-1-on-1-yl)-2H-chromen-2-onato)europium(III) (1caq-28) (actual yield, 156 mg; percent yield, 780). The resulting crude product was used in the next reaction directly, without further purification. ESIMS (m/z), MeOH: 1892.5 [M + Na]⁺, 1855.3 [M - 2H₂O + Na]⁺.

The diaquatris(7-{bis[4-(trifluoromethyl)phenyl]amino}-4-hydroxy-3-(2,2,2-trifluoroethyl-1-on-1-yl)-2H-chromen-2-onato)europium(III) (143 mg, 76.5 µmol) and triphenylphosphine oxide (45.1 mg, 162 µmol) were dissolved in acetone (7.50 mL), and the resulting solution was stirred for 3 hours at room temperature. The reaction mixture was filtered through Celite, then the filtrate was concentrated under reduced pressure, and the residue was reprecipitated with acetone/hexane to give a yellow solid of tris(7-{bis[4-(trifluoromethyl)phenyl]amino}-4-hydroxy-3-(2,2,2-trifluoroethyl-1-on-1-yl)-2H-chromen-2-onato)bis(triphenylphosphine oxide)europium(III) (1c-31) (actual yield, 176 mg; percent yield, 96%). ¹⁹F-NMR (162 MHz, CDCl₃) δ (ppm): -62.3 (s, 18F), -73.1 (s, 9F). ³¹P-NMR (162 MHz, CDCl₃) δ (ppm): -85.2 (brs). ESIMS (m/z), MeOH: 2410.6 [M + Na]⁺.

### (EXAMPLE 99)

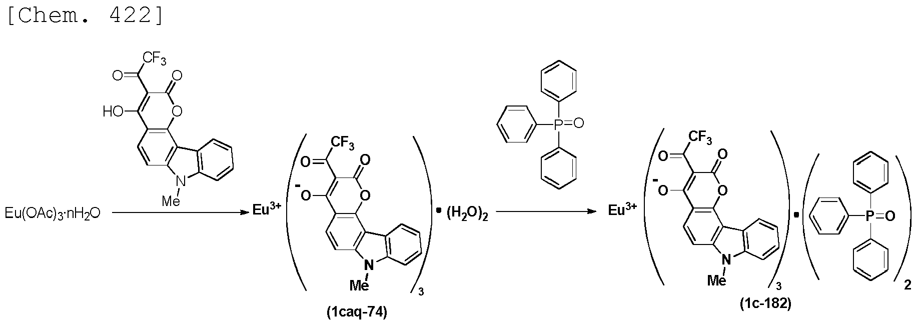

Europium(III) acetate n-hydrate (116 mg as a 2.5-hydrate, 309 µmol) and the 4-hydroxy-7-methyl-3-(2,2,2-trifluoroethan-1-on-1-yl)-2H,7H-pyrano[5,6-c]carbazol-2-one obtained in Reference Example 57 (332 mg, 919 µmol) were suspended in ethanol (11.4 mL) and water (3.80 mL). Then a 1 M aqueous solution of sodium hydroxide (910 µL, 910 µmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give an orange-yellow solid of diaquatris[4-hydroxy-7-methyl-3-(2,2,2-trifluoroethan-1-on-1-yl)-2H,7H-pyrano[5,6-c]carbazol-2-onato]europium(III) (1caq-74) (actual yield, 363 mg; percent yield, 93%). The resulting crude product was used in the next reaction directly, without further purification. ¹⁹F-NMR (376 MHz, Acetone-d₆) δ (ppm) : -71.9 (brs, 9F) . ESIMS (m/z), MeOH: 1255.0 [M - 2H₂O + Na]⁺.

The diaquatris[4-hydroxy-7-methyl-3-(2,2,2-trifluoroethan-1-on-1-yl)-2H,7H-pyrano[5,6-c]carbazol-2-onato]europium(III) (202 mg, 159 µmol) and triphenylphosphine oxide (90.3 mg, 0.324 mmol) were dissolved in acetone (15.8 mL), and the resulting solution was stirred for 3 hours at room temperature. The reaction mixture was filtered through Celite, and then the filtrate was concentrated under reduced pressure, giving a crude product orange-yellow solid (287 mg). 137 mg of the resulting crude product was reprecipitated with acetone/hexane to give a yellow solid of tris[4-hydroxy-7-methyl-3-(2,2,2-trifluoroethan-1-on-1-yl)-2H,7H-pyrano[5,6-c]carbazol-2-onato]bis(triphenylphosphine oxide)europium(III) (1c-182) (actual yield, 114 mg; percent yield, 40%) . ¹⁹F-NMR (376 MHz, Acetone-d₆) δ (ppm) : 72.0 (brs, 2.2F), -73.0 (brs, 6.8F). ³¹P-NMR (162 MHz, Acetone-d₆) δ (ppm): -86.7 (brs, 9F). ESIMS (m/z), MeCN: 1810.6 [M + Na]⁺, 1533.0 [M - (triphenylphosphine oxide) + Na]⁺, 1428.0 [M - (4-hydroxy-7-methyl-3-(2,2,2-trifluoroethan-1-on-1-yl)-2H,7H-pyrano[5,6-c]carbazol-2-onato)]⁺.

### (EXAMPLE 100)

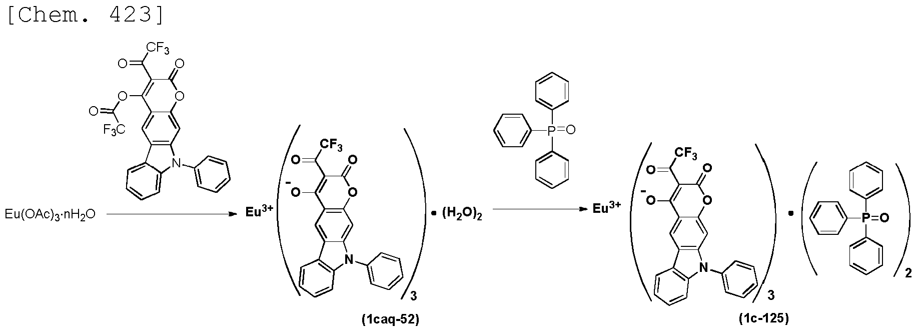

Europium(III) acetate hydrate (75.2 mg as a 2.5-hydrate, 201 µmol) and the 6-phenyl-2-(2,2,2-trifluoroethan-1-on-1-yl)-1-(2,2,2-trifluoroethan-1-on-1-yloxy)-3H,6H-pyrano[6,5-b]carbazol-3-one obtained in Reference Example 58 (313 mg, 603 µmol) were suspended in ethanol (7.50 mL) and water (2.50 mL). Then a 1 M aqueous solution of sodium hydroxide (600 µL, 600 µmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give a solid in yellow of diaquatris[1-hydroxy-6-phenyl-2-(2,2,2-trifluoroethan-1-on-1-yl)-3H,6H-pyrano[6,5-b]carbazol-3-onato]europium(III) (1caq-52) (actual yield, 296 mg; percent yield, quant.). The resulting crude product was used in the next reaction directly, without further purification. ¹⁹F-NMR (376 MHz, Acetone-d₆) δ (ppm): -72.1 (brs, 2.8F), -74.7 (brs, 0.1F), -74.7 (s, 0.1F), -75.0 (s, 0.2F), -75.3 (brs, 5.8F). ESIMS (m/z), MeOH: 1440.5 [M - 2H₂O + Na]⁺.

The diaquatris[1-hydroxy-6-phenyl-2-(2,2,2-trifluoroethan-1-on-1-yl)-3H,6H-pyrano[6,5-b]carbazol-3-onato]europium(III) (201 mg, 138 µmol) and triphenylphosphine oxide (79.2 mg, 285 µmol) were dissolved in acetone (13.8 mL), and the resulting solution was stirred for 3 hours at room temperature. The reaction mixture was concentrated under reduced pressure, giving a crude product dark green solid. The resulting crude product was reprecipitated with acetone/hexane to give an ocherous solid of tris[1-hydroxy-6-phenyl-2-(2,2,2-trifluoroethan-1-on-1-yl)-3H,6H-pyrano[6,5-b]carbazol-3-onato]bis(triphenylphosphine oxide)europium(III) (1c-125) (actual yield, 256 mg; percent yield, 94%). ¹⁹F-NMR (376 MHz, Acetone-d₆) δ (ppm): -71.4 (brs, 1.6F), -71.9 (brs, 1.4F), -72.5 (brs, 6.0F). ³¹P-NMR (162 MHz, Acetone-d₆) δ (ppm): -91.3 (brs). ESIMS (m/z), MeCN: 1997.6 [M + Na]⁺, 1717.7 [M - (triphenylphosphine oxide) + Na]⁺.

### (EXAMPLE 101)

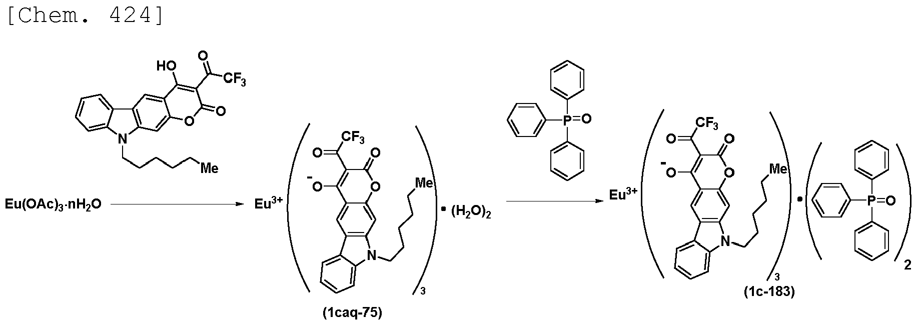

Europium(III) acetate hydrate (124 mg as a 2.5-hydrate, 331 µmol) and the 6-hexyl-1-hydroxy-2-(2,2,2-trifluoroethan-1-on-1-yl)-3H,6H-pyrano[5,6-b]carbazol-3-one obtained in Reference Example 59 (432 mg, 1.00 mmol) were suspended in ethanol (12.4 mL) and water (4.10 mL). Then a 1 M aqueous solution of sodium hydroxide (994 µL, 994 µmol) was added, and the resulting mixture was stirred for 3 hours at room temperature. After the ethanol was distilled away, water was put into the reaction mixture, the resulting mixture was filtered, and the collected solid was washed with water and then with a very small amount of ethanol. The washed solid was dried by heating to give a yellow solid of diaquatris[6-hexyl-1-hydroxy-2-(2,2,2-trifluoroethan-1-on-1-yl)-3H,6H-pyrano[5,6-b]carbazol-3-onato]europium(III) (11caq-75) (actual yield, 469 mg; percent yield, 96). The resulting crude product was used in the next reaction directly, without further purification. ¹⁹F-NMR (376 MHz, Acetone-d₆) δ (ppm): -70.5 (s, 0.1F), -70.7 (s, 0.1F), -71.9 (brs, 7.1F), -73.0 (brs, 0.4F), -74.3 (brs, 1.3F). ESIMS (m/z), MeOH: 1465.8 [M - 2H₂O + Na]⁺.

The diaquatris[6-hexyl-1-hydroxy-2-(2,2,2-trifluoroethan-1-on-1-yl)-3H,6H-pyrano[5,6-b]carbazol-3-onato]europium(III) (256 mg, 173 µmol) and triphenylphosphine oxide (99.7 mg, 358 µmol) were dissolved in acetone (17.3 mL), and the resulting solution was stirred for 3 hours at room temperature. The reaction mixture was concentrated under reduced pressure, giving a crude product orange-yellow solid. Acetone/hexane was added to the resulting crude product, the supernatant was removed, and the residue was dried by heating to give an orange-yellow amorphous mass of tris[6-hexyl-1-hydroxy-2-(2,2,2-trifluoroethan-1-on-1-yl)-3H,6H-pyrano[5,6-b]carbazol-3-onato]bis(triphenylphosphine oxide)europium(III) (1c-183) (actual yield, 337 mg; percent yield, 97%). ¹⁹F-NMR (376 MHz, Acetone-d₆) δ (ppm): -71.2 (brs, 0.7F), -71.8 (brs, 0.9F), -72.5 (brs, 7.0F), -73.0 (brs, 0.2F), -73.3 (brs, 0.2F). ³¹P-NMR (162 MHz, Acetone-d₆) δ (ppm) : -89.1 (brs) . ESIMS (m/z), MeCN: 2022.5 [M + Na]⁺, 1742.0 [M - (triphenylphosphine oxide) + Na]⁺.

### (EXAMPLE 102)

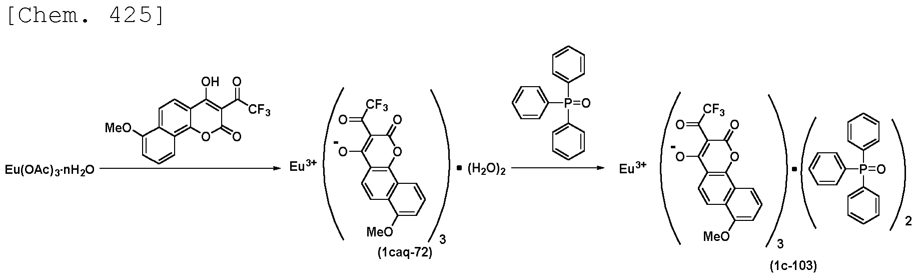

Ethanol (7.50 mL) and water (2.50 mL) were added to europium(III) acetate hydrate (74.8 mg as a 2.5-hydrate, 200 µmol) and the 4-hydroxy-7-methoxy-3-(2,2,2-trifluoroethan-1-on-1-yl)-2H-naphtho[1,2-b]pyran-2-one obtained in Reference Example 60 (203 mg, 600 µmol). A 1 M aqueous solution of sodium hydroxide (600 µL, 600 µmol) was added, and the resulting mixture was stirred for 3 hours at room temperature. After the ethanol was distilled away, water was put into the reaction mixture, the resulting mixture was filtered, and the collected solid was washed with water. The washed solid was dried by heating to give a brown solid of diaquatris[4-hydroxy-7-methoxy-3-(2,2,2-trifluoroethan-1-on-1-yl)-2H-naphtho[1,2-b]pyran-2-onato]europium(III) (1caq-72) (actual yield, 218 mg; percent yield, 91%) . The resulting crude product was used in the next reaction directly, without further purification.

Acetone (15.0 mL) was added to the diaquatris[4-hydroxy-7-methoxy-3-(2,2,2-trifluoroethan-1-on-1-yl)-2H-naphtho[1,2-b]pyran-2-onato]europium(III) (180 mg, 150 µmol) and triphenylphosphine oxide (83.5 mg, 300 µmol), and the resulting mixture was stirred for 3 hours at room temperature. The reaction mixture was filtered through Celite, and then the filtrate was concentrated under reduced pressure. Diethyl ether was added, and the precipitated brown solid was removed by filtration. The filtrate was concentrated under reduced pressure, and then the residue was reprecipitated with chloroform/hexane to give a yellow solid of tris[4-hydroxy-7-methoxy-3-(2,2,2-trifluoroethan-1-on-1-yl)-2H-naphtho[1,2-b]pyran-2-onato]bis(triphenylphosphine oxide)europium(III) (1c-103) (actual yield, 181 mg; percent yield, 70%). ¹⁹F-NMR (376 MHz, CDCl₃) δ (ppm) : -73.3. ³¹P-NMR (162 MHz, CDCl₃) δ (ppm) : -83.7 (brs). ESIMS (m/z), MeOH: 1464.6 [M - (triphenylphosphine oxide) + Na]⁺, 1382.6 [M - (4-hydroxy-7-methoxy-3-(2,2,2-trifluoroethan-1-on-1-yl)-2H-naphtho[1,2-b]pyran-2-onato)]⁺.

### (EXAMPLE 103)

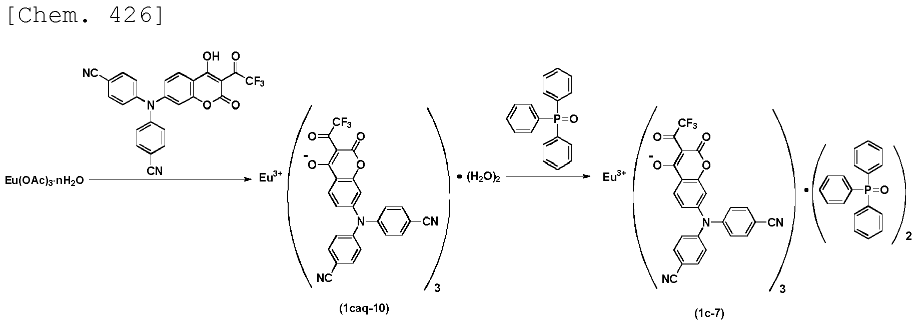

Europium acetate n-hydrate (18.9 mg as a 2.5-hydrate, 50.4 µmol) and the 7-{bis[4-cyanophenyl]amino}-4-hydroxy-3-(2,2,2-trifluoroethyl-1-on-1-yl)-2H-chromen-2-one obtained in Reference Example 61 (168 mg, 300 mmol) were suspended in ethanol (1.90 mL) and water (631 µL). Then a 1 M aqueous solution of sodium hydroxide (151 µL, 151 µmol) was added, and the resulting mixture was stirred for 3 hours at room temperature. The reaction mixture was concentrated under reduced pressure, and then the resulting solid was washed with water and dried by heating to give a yellow solid of diaquatris{7-[bis(4-cyanophenyl)amino]-4-hydroxy-3-(2,2,2-trifluoroethyl-1-on-1-yl)-2H-chromen-2-onato}europium(III) (1caq-10) (actual yield, 76.3 mg; percent yield, 940). The resulting crude product was used in the next reaction directly, without further purification. ESIMS (m/z), MeOH: 1597.8 [M - 2H₂O + Na]⁺.

The diaquatris{7-[bis(4-cyanophenyl)amino]-4-hydroxy-3-(2,2,2-trifluoroethyl-1-on-1-yl)-2H-chromen-2-onato}europium(III) (58.7 mg, 36.4 µmol) and triphenylphosphine oxide (20.3 mg, 72.8 µmol) were dissolved in acetone (3.60 mL), and the resulting solution was stirred for 3 hours at room temperature. Acetone (1 mL) and hexane (10 mL) were added to the reaction mixture, the resulting mixture was allowed to stand, and then the supernatant was removed. Acetone (0.50 mL) and hexane (7.0 mL) were added to the resulting solid, and the resulting mixture was allowed to stand. The supernatant was removed, and the residue was dried by heating to give a yellow solid of tris{7-[bis(4-cyanophenyl)amino]-4-hydroxy-3-(2,2,2-trifluoroethyl-1-on-1-yl)-2H-chromen-2-onato}bis(triphenylphosphine oxide)europium(III) (1c-7) (actual yield, 65.1 mg; percent yield, 84%). ¹⁹F-NMR (162 MHz, CDCl₃) δ (ppm): -71.6 (s, 1.8F), -72.1 (s, 1.3F), -3.0 (s, 5.9F). ³¹P-NMR (162 MHz, CDCl₃) δ (ppm): -85.2 (brs). ESIMS (m/z), MeCN: 2153.4 [M + Na]⁺.

### (EXAMPLE 104)

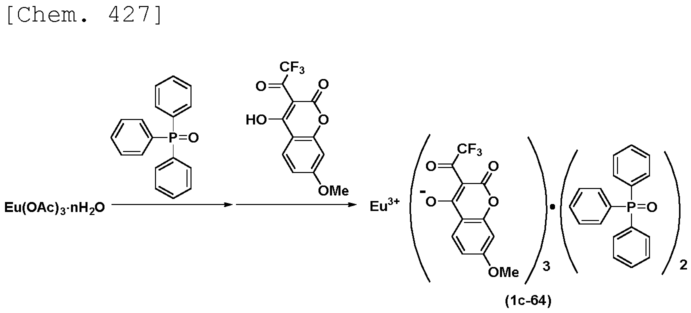

Methanol (7.5 mL) was added to europium acetate n-hydrate (560 mg, 1.50 mmol as a 2.5-hydrate) and triphenylphosphine oxide (835 mg, 3.00 mmol), and the resulting mixture was stirred for 1 hour at room temperature. The 4-hydroxy-7-methoxy-3-(2,2,2-trifluoroethan-1-on-1-yl)-2H-chromen-2-one obtained in Reference Example 62 (1.30 g, 4.50 mmol) was added to the reaction mixture, and the resulting mixture was stirred for 3 hours at room temperature. The reaction solution was emptied into water, and the precipitated solid was collected by suction filtration and then washed with water. This solid was suspended in methylcyclohexane, dehydrated by azeotropic distillation and then allowed to cool, and the solid was collected by suction filtration to give a light yellow solid of tris[4-hydroxy-7-methoxy-3-(2,2,2-trifluoroethan-1-on-1-yl)-2H-chromen-2-onato]bis(triphenylphosphine oxide)europium(III) (1c-64) (actual yield, 1.95 g; percent yield, 83%). ¹⁹F-NMR (376 MHz, CDCl₃) δ (ppm): -73.3. ³¹P-NMR (162 MHz, CDCl₃) δ (ppm): -82.5. ESIMS (m/Z), MeOH: 1593.1 [M + Na]⁺.

### (EXAMPLE 105)

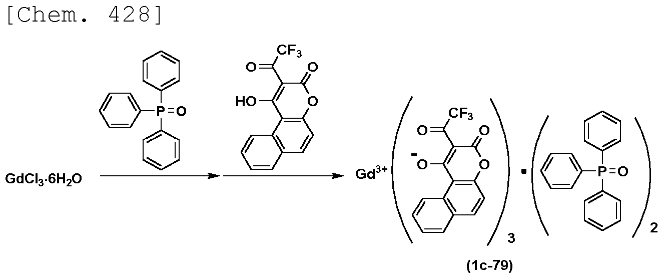

Gadolinium(III) chloride hexahydrate (40.5 mg, 109 µmol) and triphenylphosphine oxide (60.8 mg, 218 µmol) were added to acetone (2.20 mL), and the resulting mixture was stirred for 1 hour at room temperature. Then the 1-hydroxy-2-(2,2,2-trifluoroethan-1-on-1-yl)-3H-naphtho[2,1-b]pyran-3-one obtained in Reference Example 49 (101 mg, 327 µmol) and a 14.8 M aqueous solution of ammonia (22.2 µL, 329 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[1-hydroxy-2-(2,2,2-trifluoroethan-1-on-1-yl)-3H-naphtho[2,1-b]pyran-3-onato]bis(triphenylphosphine oxide)gadolinium(III) (1c-79). (Actual yield, 105 mg; percent yield, 59%). ESIMS (m/z), MeOH: 1379.9 [M - (triphenylphosphine oxide) + Na]⁺, 1327.9 [M - (1-hydroxy-2-(2,2,2-trifluoroethan-1-on-1-yl)-3H-naphtho[2,1-b]pyran-3-onato)]⁺.

### (EXAMPLE 106)

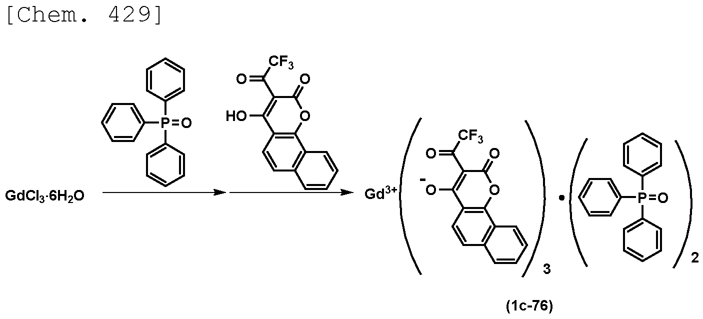

Gadolinium(III) chloride hexahydrate (18.1 mg, 48.7 µmol) and triphenylphosphine oxide (27.7 mg, 99.5 µmol) were added to acetone (1.00 mL), and the resulting mixture was stirred for 1 hour at room temperature. Then the 4-hydroxy-3-(2,2,2-trifluoroethan-1-on-1-yl)-2H-naphtho[1,2-b]pyran-2-one obtained in Reference Example 50 (45.4 mg, 147 µmol) and a 14.8 M aqueous solution of ammonia (10 µL, 148 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[4-hydroxy-3-(2,2,2-trifluoroethan-1-on-1-yl)-2H-naphtho[1,2-b]pyran-2-onato]bis(triphenylphosphine oxide)gadolinium(III) (1c-76) (actual yield, 21 mg; percent yield, 25%). ESIMS (m/z), MeOH: 1380.0 [M - (triphenylphosphine oxide) + Na]⁺, 1327.7 [M - (4-hydroxy-3-(2,2,2-trifluoroethan-1-on-1-yl)-2H-naphtho[1,2-b]pyran-2-onato)]⁺.

### (EXAMPLE 107)

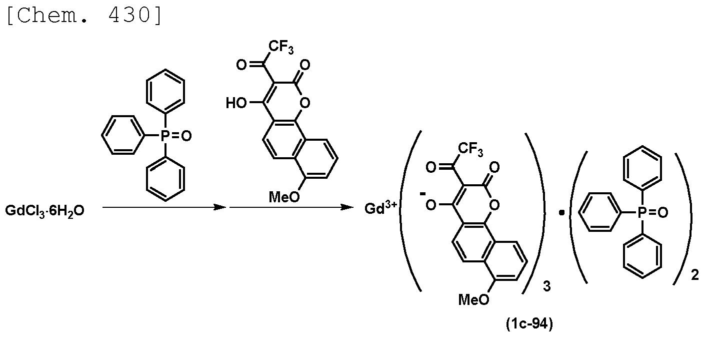

Gadolinium(III) chloride hexahydrate (20.0 mg, 59.2 µmol) and triphenylphosphine oxide (33.2 mg, 119 µmol) were added to acetone (1.00 mL), and the resulting mixture was stirred for 1 hour at room temperature. Then the 4-hydroxy-7-methoxy-3-(2,2,2-trifluoroethan-1-on-1-yl)-2H-naphtho[1,2-b]pyran-2-one obtained in Reference Example 60 (61.1 mg, 181 µmol) and a 14.8 M aqueous solution of ammonia (12 µL, 179 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a light yellow solid of tris[4-hydroxy-7-methoxy-3-(2,2,2-trifluoroethan-1-on-1-yl)-2H-naphtho[1,2-b]pyran-2-onato]bis(triphenylphosphine oxide)gadolinium(III) (1c-94). (Actual yield, 42.8 mg; percent yield, 42%). ESIMS (m/z), MeOH: 1470.3 [M - (triphenylphosphine oxide) + Na]⁺, 1388.2 [M - (4-hydroxy-7-methoxy-3-(2,2,2-trifluoroethan-1-on-1-yl)-2H-naphtho[1,2-b]pyran-2-onato)]⁺.

### (EXAMPLE 108)

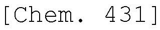

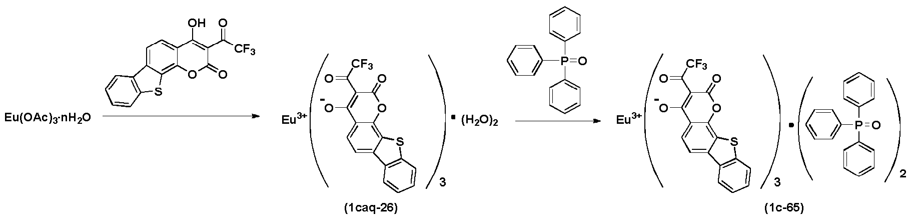

Ethanol (2.00 mL) and water (700 µL) were added to europium(III) acetate hydrate (19.9 mg as a 2.5-hydrate, 53.3 µmol) and the 4-hydroxy-3-(2,2,2-trifluoroethan-1-on-1-yl)-2H-[1]benzothieno[3,2-h]-1-benzopyran-2-one obtained in Reference Example 63 (58.2 mg, 160 µmol). A 1 M aqueous solution of sodium hydroxide (160 µL, 160 µmol) was added, and the resulting mixture was stirred for 1 hour at room temperature. After the ethanol was distilled away, water was put into the reaction mixture, the resulting mixture was filtered, and the collected solid was washed with water. The washed solid was dried by heating to give a brown solid of diaquatris[4-hydroxy-3-(2,2,2-trifluoroethan-1-on-1-yl)-2H-[1]benzothieno[3,2-h]-1-benzopyran-2-onato]europium(III) (1caq-26) (actual yield, 42.1 mg; percent yield, 62%). The resulting crude product was used in the next reaction directly, without further purification. ESIMS (m/z), MeOH: 1265.1 [M - 2H₂O + Na]⁺.

Acetone (3.3 mL) was added to the diaquatris[4-hydroxy-3-(2,2,2-trifluoroethan-1-on-1-yl)-2H-[1]benzothieno[3,2-h]-1-benzopyran-2-onatoJeuropium(III) (130 mg, 120 µmol) and triphenylphosphine oxide (42.1 mg, 32.9 µmol), and the resulting mixture was stirred for 3 hours at room temperature. The precipitated solid was collected by filtration and washed with acetone to give a yellow solid of tris[4-hydroxy-3-(2,2,2-trifluoroethan-1-on-1-yl)-2H-[1]benzothieno[3,2-h]-1-benzopyran-2-onato]bis(triphenylphosphine oxide)europium(III) (1c-65) (actual yield, 20.1 mg; percent yield, 34%). ¹⁹F-NMR (376 MHz, CDCl₃) δ (ppm): -72.9 (s). ³¹P-NMR (162 MHz, CDCl₃) δ (ppm): -86.3 (brs). ESIMS (m/z), MeCN: 1543.6 [M - (triphenylphosphine oxide) + Na]⁺.

### (EXAMPLE 109)

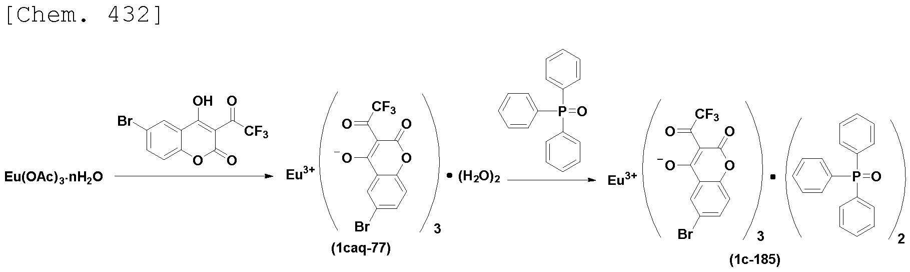

Ethanol (12.0 mL) and water (4.00 mL) were added to europium(III) acetate hydrate (112 mg as a 2.5-hydrate, 300 µmol) and the 6-bromo-4-hydroxy-3-(2,2,2-trifluoroethan-1-on-1-yl)-2H-chromen-2-one obtained in Reference Example 64 (303 mg, 900 µmol). A 1 M aqueous solution of sodium hydroxide (900 µL, 900 µmol) was added, and the resulting mixture was stirred for 1 hour at room temperature. After the ethanol was distilled away, water was put into the reaction mixture, the resulting mixture was filtered, and the collected solid was washed with water. The washed solid was dried by heating to give a yellow solid of diaquatris[6-bromo-4-hydroxy-3-(2,2,2-trifluoroethan-1-on-1-yl)-2H-chromen-2-onato]europium(III) (1caq-77) (actual yield, 266 mg; percent yield, 74%). The resulting crude product was used in the next reaction directly, without further purification. ESIMS (m/z), MeOH: 1184.3 [M - 2H₂O + Na]⁺.

Acetone (20.0 mL) was added to the diaquatris[6-bromo-4-hydroxy-3-(2,2,2-trifluoroethan-1-on-1-yl)-2H-chromen-2-onato]europium(III) (239 mg, 200 µmol) and triphenylphosphine oxide (111 mg, 400 µmol), and the resulting mixture was stirred for 3 hours at room temperature. The reaction mixture was filtered through Celite, then the filtrate was concentrated under reduced pressure, and the residue was reprecipitated with chloroform/hexane to give a yellow solid of tris[6-bromo-4-hydroxy-3-(2,2,2-trifluoroethan-1-on-1-yl)-2H-chromen-2-onato]bis(triphenylphosphine oxide)europium(III) (1c-185) (actual yield, 312 mg; percent yield, 91%). ¹⁹F-NMR (376 MHz, CDCl₃) δ (ppm): -72.7 (brs). ³¹P-NMR (162 MHz, CDCl₃) δ (ppm): -89.7 (brs). ESIMS (m/z), MeOH: 1739.0 [M + Na]⁺, 1460.9 [M - (triphenylphosphine oxide) + Na]⁺, 1381.0 [M - (6-bromo-4-hydroxy-3-(2,2,2-trifluoroethan-1-on-1-yl)-2H-chromen-2-onato)]⁺.

### (EXAMPLE 110)

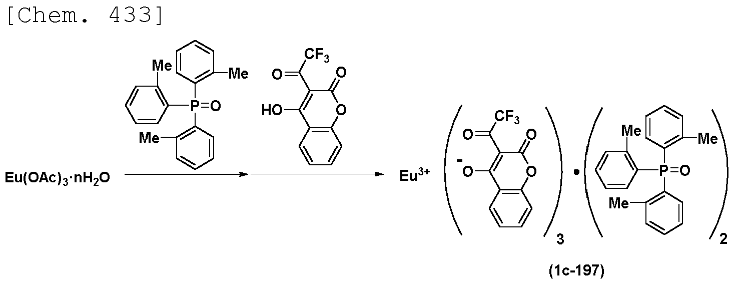

Europium(III) acetate n-hydrate (90.0 mg, 273 pmmol as a 2.5-hydrate) and tri-o-tolylphosphine oxide (175.4 mg, 548 µmol) were put into methanol (1.3 mL), and the resulting mixture was stirred for 1 hour at room temperature. Then the 4-hydroxy-3-(2,2,2-trifluoroethan-1-on-1-yl)-2H-chromen-2-one obtained in Reference Example 48 (212.0 mg, 821.2 µmol) and a 14.8 M aqueous solution of ammonia (12 µL, 178 µmol) were added, and the resulting mixture was stirred for 3 hours at room temperature. After the reaction, water was put into the reaction mixture to cause a precipitate to form, and the solid was collected by suction filtration and washed with water and then with a very small amount of ethanol. The washed solid was dried by heating to give a white solid of tris[4-hydroxy-3-(2,2,2-trifluoroethan-1-on-1-yl)-2H-chromen-2-onato]bis(tri-o-tolylphosphine oxide)europium(III) (1c-197). (Actual yield, 44.5 mg; percent yield, 47%). ESIMS (m/z), MeOH: 1267.1 [M - (tri-o-tolylphosphine oxide) + Na]⁺.

### (EXAMPLE 111)

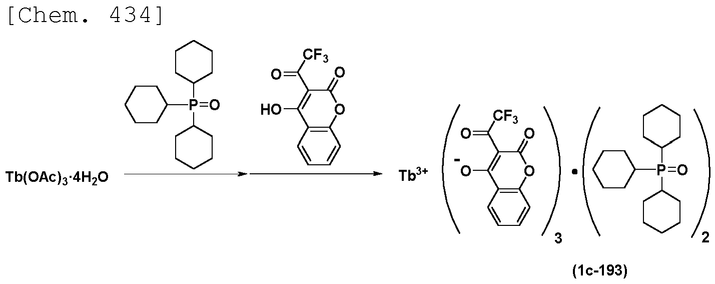

Terbium(III) acetate tetrahydrate (150.9 mg, 370 pmmol) and tricyclohexylphosphine oxide (220.1 mg, 743 µmol) were put into methanol (2.0 mL), and the resulting mixture was stirred for 2 hours at room temperature. Then the 4-hydroxy-3-(2,2,2-trifluoroethan-1-on-1-yl)-2H-chromen-2-one obtained in Reference Example 48 (286.3 mg, 1.11 mmol) was added, and the resulting mixture was stirred for 3 hours at room temperature. After the reaction, the reaction solution was concentrated under reduced pressure, and water was added to cause a precipitate to form. By suction filtration, the solid was washed with water and then with a very small amount of ethanol and collected. The washed solid was dried by heating to give a white solid of tris[4-hydroxy-3-(2,2,2-trifluoroethan-1-on-1-yl)-2H-chromen-2-onato]bis(tricyclohexylphosphine oxide)terbium(III) (1c-193). (Actual yield, 446.9 mg; percent yield, 79%). ESIMS (m/z), MeOH: 1249.3 [M - (tricyclohexylphosphine oxide) + Na]⁺.

### (EXAMPLE 112)

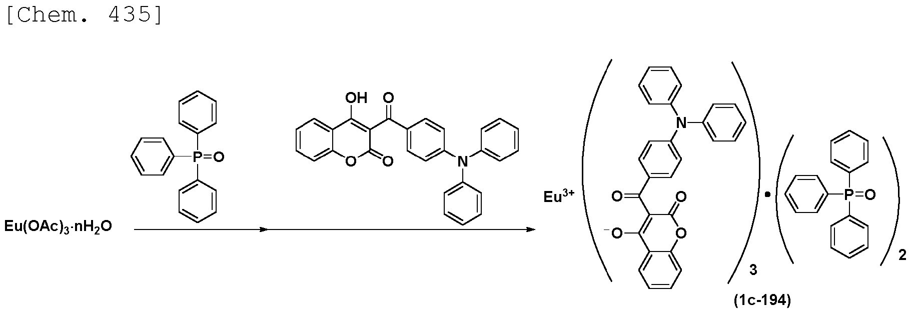

Methanol (10.0 mL) was added to europium acetate n-hydrate (74.8 mg as a 2.5-hydrate, 200 µmol) and triphenylphosphine oxide (111 mg, 592 µmol), and the resulting mixture was stirred for 1 hour at room temperature. The 3-[4-(diphenylamino)benzoyl]-4-hydroxy-2H-chromen-2-one obtained in Reference Example 66 (260 mg, 601 µmol) was added to the reaction mixture. 14.8 M aqueous ammonia (40.0 µL, 592 µmol) was added dropwise, and the resulting mixture was stirred for 3 hours at room temperature. The reaction solution was concentrated under reduced pressure, then the residue was dissolved by adding chloroform to it, and the resulting solution was concentrated under reduced pressure. The resulting solid was washed with diethyl ether to give a yellow solid of tris[3-[4-(diphenylamino)benzoyl]-4-hydroxy-2H-chromen-2-onato]bis(triphenylphosphine oxide)europium(III) (1c-194) (actual yield, 363 mg; percent yield, 91%). ³¹P-NMR (162 MHz, CDCl₃) δ (ppm): -78.5 (brs). ESIMS (m/z), MeOH: 2029.82 [M + Na]⁺, 1573.4 [M - (3-[4-(diphenylamino)benzoyl]-4-hydroxy-2H-chromen-2-onato)]⁺.

### (EXAMPLE 113)

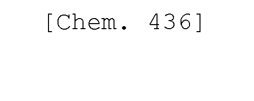

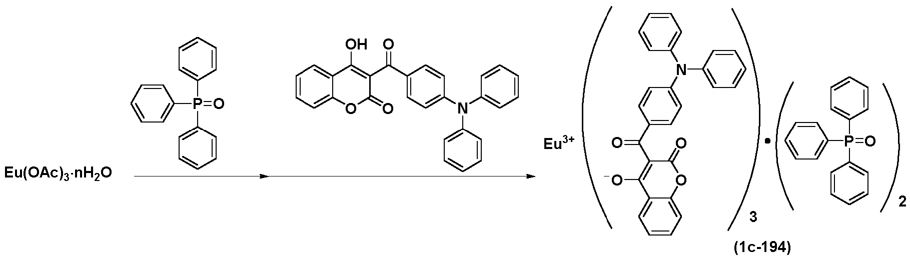

Europium acetate n-hydrate (187 mg as a 2.5-hydrate, 500 µmol) was dissolved in methanol (4.00 mL), triphenylphosphine oxide (278 mg, 1.00 mol) dissolved in toluene (10.0 mL) was added, and the resulting mixture was stirred for 1 hour at room temperature. The 3-[4-(diphenylamino)benzoyl]-4-hydroxy-2H-chromen-2-one obtained in Reference Example 66 (260 mg, 601 µmol) and 14.8 M aqueous ammonia (40.0 µL, 592 µmol) were suspended in toluene (10.0 mL), the resulting suspension was added dropwise to the reaction mixture, and the resulting mixture was stirred for 3 hours at room temperature. The reaction solution was concentrated under reduced pressure, and then the resulting solid was washed with water and diethyl ether to give a yellow solid of tris[3-[4-(diphenylamino)benzoyl]-4-hydroxy-2H-chromen-2-onato]bis(triphenylphosphine oxide)europium(III) (1c-194) (actual yield, 950 mg; percent yield, 93%) . ³¹P-NMR (162 MHz, CDCl₃) δ (ppm): -78.5 (brs). ESIMS (m/z), MeOH: 2029.82 [M + Na]⁺, 1573.4 [M - (3-[4-(diphenylamino)benzoyl]-4-hydroxy-2H-chromen-2-onato)]⁺.

### (EXAMPLE 114)

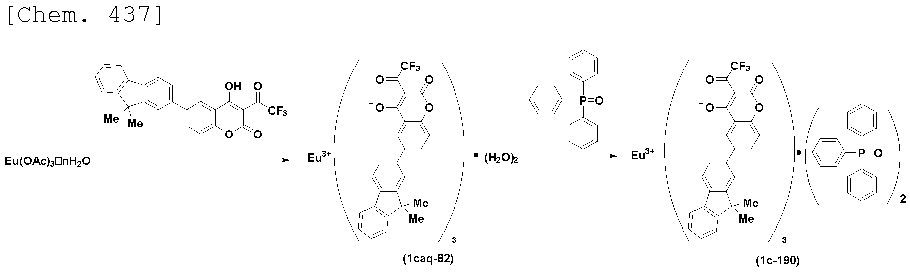

Ethanol (7.50 mL) and water (2.50 mL) were added to europium(III) acetate hydrate (74.8 mg as a 2.5-hydrate, 200 µmol) and the 6-(9,9-dimethyl-9H-fluoren-2-yl)-4-hydroxy-3-(2,2,2-trifluoroethan-1-on-1-yl)-2H-chromen-2-one obtained in Reference Example 65 (271 mg, 602 µmol). Then a 1 M aqueous solution of sodium hydroxide (600 µL, 600 µmol) was added, and the resulting mixture was stirred for 1 hour at room temperature. After the ethanol was distilled away, water was put into the reaction mixture, the resulting mixture was filtered, and the collected solid was washed with water. The washed solid was dried by heating to give a deep orange powder solid of diaquatris[6-(9,9-dimethyl-9H-fluoren-2-yl)-4-hydroxy-3-(2,2,2-trifluoroethan-1-on-1-yl)-2H-chromen-2-onato]europium(III) (1caq-82) (actual yield, 292 mg; percent yield, 95%). The resulting crude product was used in the next reaction directly, without further purification.

The diaquatris[6-(9,9-dimethyl-9H-fluoren-2-yl)-4-hydroxy-3-(2,2,2-trifluoroethan-1-on-1-yl)-2H-chromen-2-onato]europium(III) (1caq-82) (280 mg, 182 µmol) and triphenylphosphine oxide (102 mg, 367 µmol) were dissolved in acetone (20.0 mL), and the resulting solution was stirred for 3 hours at room temperature. The reaction mixture was filtered through Celite, then the filtrate was concentrated under reduced pressure, and the residue was reprecipitated with acetone/hexane to give a yellow solid of tris[6-(9,9-dimethyl-9H-fluoren-2-yl)-4-hydroxy-3-(2,2,2-trifluoroethan-1-on-1-yl)-2H-chromen-2-onato]bis(triphenylphosphine oxide)europium(III) (1c-190) (actual yield, 168 mg; percent yield, 45%). ¹⁹F-NMR (376 MHz, CDCl₃) δ (ppm): -72.2 (s, 0.8F), -72.4 (s, 7.2F), -73.0 (s, 1F). ³¹P-NMR (162 MHz, CDCl₃) δ (ppm): -87.7 (brs, 0.3P), -90.1 (brs, 1.7P). ESIMS (m/z), MeOH: 2078.8 [M + Na]⁺, 1799.9 [M - (triphenylphosphine oxide) + Na]⁺, 1607.0 [M - (6-(9,9-dimethyl-9H-fluoren-2-yl)-4-hydroxy-3-(2,2,2-trifluoroethan-1-on-1-yl)-2H-chromen-2-onato)]⁺.

### (EXAMPLE 115)

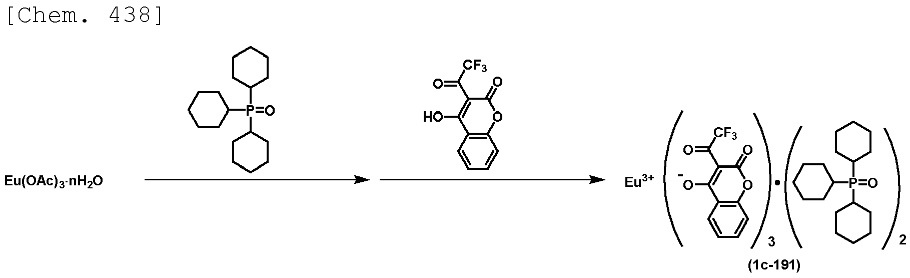

Ethanol (6.8 mL) was added to europium(III) acetate hydrate (506 mg as a 2.5-hydrate, 1.35 mmol) and tricyclohexylphosphine oxide (912 mg, 3.08 mmol), and the resulting mixture was stirred for 1 hour at room temperature. The 4-hydroxy-3-(2,2,2-trifluoroethan-1-on-1-yl)-2H-chromen-2-one obtained in Reference Example 69 (1.19 g, 4.62 mmol) was added to the reaction mixture, and the resulting mixture was stirred for 4 hours at room temperature. Ethanol (38.0 mL) was added to the reaction solution, then water (22.5 mL) was added, and the precipitate that formed was collected by filtration to give a white solid of tris[4-hydroxy-3-(2,2,2-trifluoroethan-1-on-1-yl)-2H-chromen-2-onato]bis(tricyclohexylphosphine oxide)europium(III) (1c-191) (actual yield, 1.34 g; percent yield, 66%) . ¹⁹F-NMR (376 MHz, CDCl₃) δ (ppm) : -70.9 (brs, 1.0F), -71.3 (brs, 1.7F), -71.9 (brs, 6.3F). ³¹P-NMR (162 MHz, CDCl₃) δ (ppm): -69.7 (brs, 1.2P), -71.6 (brs, 0.8P).

### (EXAMPLE 116)

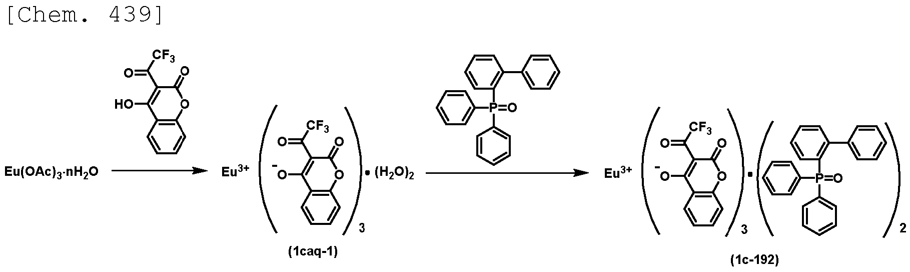

Ethanol (14.9 mL) and water (4.95 mL) were added to europium(III) acetate hydrate (148 mg as a 2.5-hydrate, 396 µmol) and the 4-hydroxy-3-(2,2,2-trifluoroethan-1-on-1-yl)-2H-chromen-2-one obtained in Reference Example 48 (308 mg, 1.19 mmol). Then a 1 M aqueous solution of sodium hydroxide (1.19 mL, 1.19 mmol) was added, and the resulting mixture was stirred for 4 hours at room temperature. The reaction mixture was concentrated under reduced pressure, then water was added, the resulting mixture was filtered, and the collected solid was washed with water. The washed solid was dried by heating under reduced pressure to give a white solid of diaquatris[4-hydroxy-3-(2,2,2-trifluoroethan-1-on-1-yl)-2H-chromen-2-onato]europium(III) (1caq-1) (actual yield, 262 mg; percent yield, 690). The resulting crude product was used in the next reaction directly, without further purification. ¹⁹F-NMR (376 MHz, CD₃OD) δ (ppm): - 74.7 (brs). ESIMS (m/z), MeOH: 946.9 [M - 2H₂O + Na]⁺.

Acetone (25.8 mL) was added to the diaquatris[4-hydroxy-3-(2,2,2-trifluoroethan-1-on-1-yl)-2H-chromen-2-onato]europium(III) (1caq-1) (248 mg, 258 µmol) and (2-biphenyl)diphenylphosphine oxide (183 mg, 517 µmol), and the resulting mixture was stirred for 25 hours at room temperature. The reaction mixture was filtered through Celite, and then the solvent was distilled away under reduced pressure. Chloroform was added to the resulting solid, the resulting mixture was filtered through Celite, and then the solvent was distilled away under reduced pressure. The resulting solid was reprecipitated with acetone/hexane to give a white solid of tris[4-hydroxy-3-(2,2,2-trifluoroethan-1-on-1-yl)-2H-chromen-2-onato]bis[(2-biphenyl)diphenylphosphine oxide]europium(III) (1c-192) (actual yield, 264 mg; percent yield, 63%). ¹⁹F-NMR (376 MHz, CDCl₃) δ (ppm): -72.8 (s). ³¹P-NMR (162 MHz, CDCl₃) δ (ppm): -83.0 (brs). ESIMS (m/z), MeOH: 1655.2 [M + Na]⁺, 1375.2 [M - (4-hydroxy-3-(2,2,2-trifluoroethan-1-on-1-yl)-2H-chromen-2-onato)]⁺, 1301.1 [M - ((2-biphenyl)diphenylphosphine oxide) + Na]⁺, 1021.1 [M - (4-hydroxy-3-(2,2,2-trifluoroethan-1-on-1-yl)-2H-chromen-2-onato-(2-biphenyl)diphenylphosphine oxide)]⁺.

### (EXAMPLE 117)

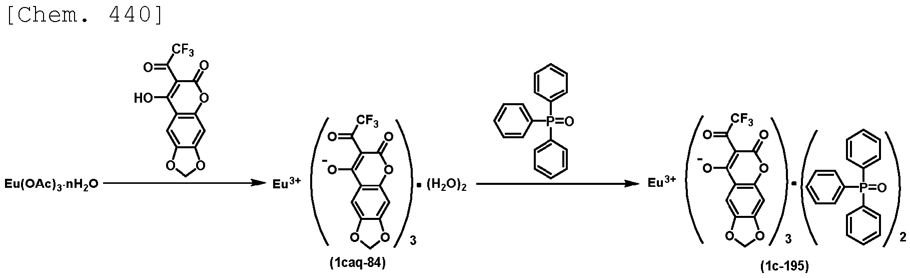

Ethanol (30.0 mL) and water (10.0 mL) were added to europium(III) acetate hydrate (299 mg as a 2.5-hydrate, 799 µmol) and the 8-hydroxy-3-(2,2,2-trifluoroethyl-1-on-1-yl)-6H-1,3-dioxolo[4,5-g][1]benzopyran-6-one obtained in Reference Example 68 (724 mg, 2.40 mmol). Then a 1 M aqueous solution of sodium hydroxide (2.40 mL, 2.40 mmol) was added, and the resulting mixture was stirred for 1 hour at room temperature. After concentration under reduced pressure, water was added to the reaction mixture, the resulting mixture was filtered, and the collected solid was washed with water. The washed solid was dried by heating under reduced pressure to give a light yellow solid of diaquatris[8-hydroxy-3-(2,2,2-trifluoroethyl-1-on-1-yl)-6H-1,3-dioxolo[4,5-g][1]benzopyran-6-onato]europium(III) (1caq-84) (actual yield, 702 mg; percent yield, 810). The resulting crude product was used in the next reaction directly, without further purification. 19F-NMR (376 MHz, CD₃OD) δ (ppm): -74.6 (brs). ESIMS (m/z), MeOH: 1079.4 [M - 2H₂O + Na]⁺.

Acetone (27.5 mL) was added to the diaquatris[8-hydroxy-3-(2,2,2-trifluoroethyl-1-on-1-yl)-6H-1,3-dioxolo[4,5-g][1]benzopyran-6-onato]europium(III) (1caq-84) (301 mg, 276 µmol) and triphenylphosphine oxide (155 mg, 555 µmol), and the resulting mixture was stirred for 3 hours at room temperature. The reaction mixture was filtered through Celite, and then the solvent was distilled away under reduced pressure. Chloroform was added to the resulting solid, the resulting mixture was filtered through Celite, and then the solvent was distilled away under reduced pressure. The resulting solid was reprecipitated with acetone/hexane to give a white solid of tris[8-hydroxy-3-(2,2,2-trifluoroethyl-1-on-1-yl)-6H-1,3-dioxolo[4,5-g] [1]benzopyran-6-onato]bis[bis(triphenylphosphine oxide)europium(III) (1c-195) (actual yield, 356 mg; percent yield, 80%). 19F-NMR (376 MHz, CDCl₃) δ (ppm): -73.3 (s). 31P-NMR (162 MHz, CDCl₃) δ (ppm): -84.5 (brs). ESIMS (m/z), MeOH: 1310.1 [M - (8-hydroxy-3-(2,2,2-trifluoroethyl-1-on-1-yl)-6H-1,3-dioxolo[4,5-g][1]benzopyran-6-onato)]⁺, 1357.0 [M - triphenylphosphine oxide + Na]⁺, 1635.1 [M + Na]⁺.

### (EXAMPLE 118)

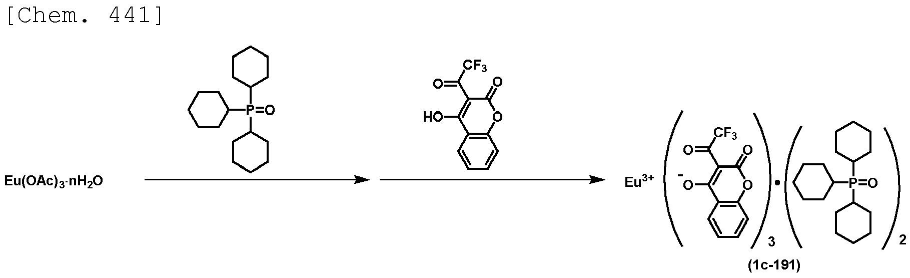

Ethanol (245 mL) was added to europium(III) acetate hydrate (18.3 g as a 2.5-hydrate, 48.9 mmol) and tricyclohexylphosphine oxide (33.0 g, 111 mmol), and the resulting mixture was stirred for 1 hour at room temperature. The 4-hydroxy-3-(2,2,2-trifluoroethan-1-on-1-yl)-2H-chromen-2-one obtained in Reference Example 69 (43.1 g, 167 mmol) was added to the reaction mixture, and the resulting mixture was stirred for 3 hours at room temperature. Ethanol (1.39 L) was added to the reaction solution, then water (815 mL) was added, and the precipitate that formed was collected by filtration and washed with a 60% aqueous solution of ethanol to give a white solid of tris[4-hydroxy-3-(2,2,2-trifluoroethan-1-on-1-yl)-2H-chromen-2-onato]bis(tricyclohexylphosphine oxide)europium(III) (1c-191) (actual yield, 57.3 g; percent yield, 77%).

### (EXAMPLE 119)

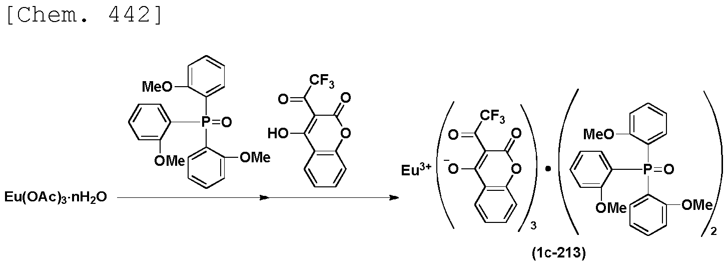

Ethanol (7.00 mL) was added to europium(III) acetate hydrate (467 mg as a 2.5-hydrate, 1.25 mmol) and tris(2-methoxyphenyl)phosphine oxide (922 mg, 2.50 mmol), and the resulting mixture was stirred for 1 hour at room temperature. The 4-hydroxy-3-(2,2,2-trifluoroethan-1-on-1-yl)-2H-chromen-2-one obtained in Reference Example 69 (966 mg, 3.74 mmol) was added to the reaction mixture, and the resulting mixture was stirred for 3 hours at room temperature. Ethanol (20.0 mL) was added to the reaction solution, then water (10.0 mL) was added, and the precipitate that formed was collected by filtration and washed with ethanol to give a white solid of tris[4-hydroxy-3-(2,2,2-trifluoroethan-1-on-1-yl)-2H-chromen-2-onato]bis[tris(2-methoxyphenyl)phosphine oxide]europium(III) (1c-213) (actual yield, 1.17 g; percent yield, 56%). ¹⁹F-NMR (376 MHz, CDCl₃) δ (ppm): -71.3 (s). ³¹P-NMR (162 MHz, CDCl₃) δ (ppm): -124 (brs). ESIMS (m/z), MeOH: 1685.0 [M + Na]⁺, 1403.0 [M - (4-hydroxy-3-(2,2,2-trifluoroethan-1-on-1-yl)-2H-chromen-2-onato)]⁺, 1314.9 [M - (tris(2-methoxyphenyl)phosphine oxide) + Na]⁺.

### (EXAMPLE 120)

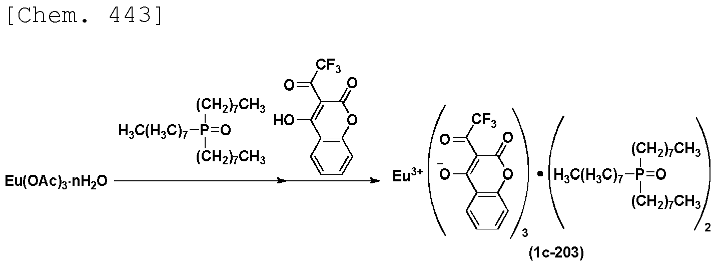

Ethanol (10.0 mL) was added to europium(III) acetate hydrate (749 mg as a 2.5-hydrate, 2.00 mmol) and tri-n-octylphosphine oxide (1.55 g, 4.00 mmol), and the resulting mixture was stirred for 1 hour at room temperature. The 4-hydroxy-3-(2,2,2-trifluoroethan-1-on-1-yl)-2H-chromen-2-one obtained in Reference Example 69 (1.58 g, 6.12 mmol) was added to the reaction mixture, and the resulting mixture was stirred for 3 hours at room temperature. The reaction solution was concentrated under reduced pressure to give a colorless oily substance, tris[4-hydroxy-3-(2,2,2-trifluoroethan-1-on-1-yl)-2H-chromen-2-onato]bis(tri-n-octylphosphine oxide)europium(III) (1c-203) (actual yield, 3.39 g; percent yield, quant.). ¹⁹F-NMR (376 MHz, CDCl₃) δ (ppm): -72.4 (s). ³¹P-NMR (162 MHz, CDCl₃) δ (ppm): -64.0 (brs, 1.6P), -66.5 (brs, 0.4P). ESIMS (m/z), MeOH: 1720.6 [M + Na]⁺, 1437.6 [M - (4-hydroxy-3-(2,2,2-trifluoroethan-1-on-1-yl)-2H-chromen-2-onato)]⁺, 1333.4 [M - (tri-n-octylphosphine oxide) + Na]⁺.

### (EXAMPLE 121)

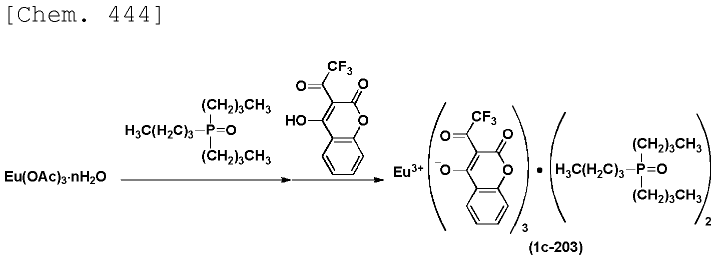

Ethanol (10.0 mL) was added to europium(III) acetate hydrate (749 mg as a 2.5-hydrate, 2.00 mmol) and tributylphosphine oxide (873 mg, 4.00 mmol), and the resulting mixture was stirred for 1 hour at room temperature. The 4-hydroxy-3-(2,2,2-trifluoroethan-1-on-1-yl)-2H-chromen-2-one obtained in Reference Example 69 (1.58 g, 6.12 mmol) was added to the reaction mixture, and the resulting mixture was stirred for 3 hours at room temperature. The reaction solution was concentrated under reduced pressure, and then a 10% aqueous solution of ethanol was added. The resulting mixture was allowed to stand for 1 hour, and the precipitated solid was collected by filtration to give a white solid of tris[4-hydroxy-3-(2,2,2-trifluoroethan-1-on-1-yl)-2H-chromen-2-onato]bis(tributylphosphine oxide)europium(III) (1c-204) (actual yield, 2.28 g; percent yield, 84%). ¹⁹F-NMR (376 MHz, CDCl₃) δ (ppm): -72.3 (s). ³¹P-NMR (162 MHz, CDCl₃) δ (ppm): -62.8 (brs, 1.6P), -65.2 (brs, 0.4P). ESIMS (m/z), MeOH: 1384.2 [M + Na]⁺, 1165.1 [M - (tributylphosphine oxide) + Na]⁺, 1103.3 [M - (4-hydroxy-3-(2,2,2-trifluoroethan-1-on-1-yl)-2H-chromen-2-onato)]⁺.

### (EXAMPLE 122)

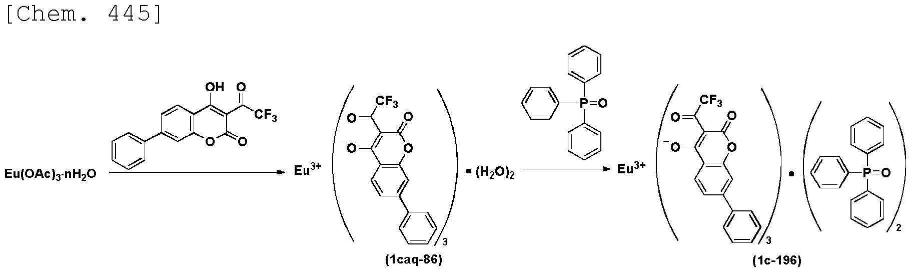

Ethanol (4.50 mL) and water (1.50 mL) were added to europium(III) acetate hydrate (44.5 mg as a 2.5-hydrate, 119 µmol) and the 4-hydroxy-7-phenyl-3-(2,2,2-trifluoroethan-1-on-1-yl)-2H-chromen-2-one obtained in Reference Example 70 (120 mg, 359 µmol), and the resulting mixture was stirred for 1 hour at room temperature. After the ethanol was distilled away, water was put into the reaction mixture, the resulting mixture was filtered, and the collected solid was washed with water. The washed solid was dried by heating to give a light brown powder solid of diaquatris[4-hydroxy-7-phenyl-3-(2,2,2-trifluoroethan-1-on-1-yl)-2H-chromen-2-onato]europium(III) (1caq-86) (actual yield, 150 mg; percent yield, quant.). The resulting crude product was used in the next reaction directly, without further purification.

The diaquatris[4-hydroxy-7-phenyl-3-(2,2,2-trifluoroethan-1-on-1-yl)-2H-chromen-2-onatoJeuropium(III) (150 mg, 126 µmol) and triphenylphosphine oxide (70.3 mg, 253 µmol) were dissolved in acetone (13.0 mL), and the resulting solution was stirred for 3 hours at room temperature. The reaction mixture was filtered, then the filtrate was concentrated under reduced pressure, and the residue was reprecipitated with acetone/hexane to give a yellow solid of tris[4-hydroxy-7-phenyl-3-(2,2,2-trifluoroethan-1-on-1-yl)-2H-chromen-2-onato]bis(triphenylphosphine oxide)europium(III) (1c-196) (actual yield, 134 mg; percent yield, 62%). ¹⁹F-NMR (376 MHz, CDCl₃) δ (ppm): -73.0 (s). ³¹P-NMR (162 MHz, CDCl₃) δ (ppm): -85.1 (brs). ESIMS (m/z), MeOH: 1730.8 [M + Na]⁺, 1451.9 [M - (triphenylphosphine oxide) + Na]⁺, 1375.1 [M - (4-hydroxy-7-phenyl-3-(2,2,2-trifluoroethan-1-on-1-yl)-2H-chromen-2-onato)]⁺.

### (EXAMPLE 123)

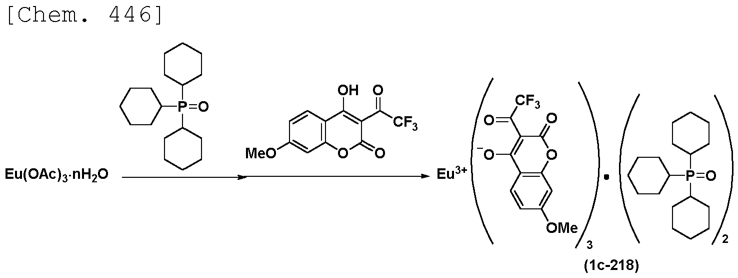

Ethanol (15.0 mL) was added to europium(III) acetate hydrate (112 mg as a 2.5-hydrate, 300 µmol) and tricyclohexylphosphine oxide (178 mg, 600 µmol), and the resulting mixture was stirred for 1 hour at room temperature. The 4-hydroxy-7-methoxy-3-(2,2,2-trifluoroethan-1-on-1-yl)-2H-chromen-2-one obtained in Reference Example 71 (261 mg, 906 µmol) was added to the reaction mixture. 14.8 M aqueous ammonia (60.0 µL, 888 µmol) was added dropwise, and the resulting mixture was stirred for 3 hours at room temperature. The precipitated solid was collected by filtration and washed with water and ethanol to give a white solid of tris[4-hydroxy-7-methoxy-3-(2,2,2-trifluoroethan-1-on-1-yl)-2H-chromen-2-onato]bis(tricyclohexylphosphine oxide)europium(III) (1c-218) (actual yield, 263 mg; percent yield, 55%). ¹⁹F-NMR (376 MHz, CDCl₃) δ (ppm): -72.0 (s, 7.3F), -72.2 (s, 1.7F). ³¹P-NMR (162 MHz, CDCl₃) δ (ppm): -62.6 (brs). ESIMS (m/z), MeOH: 1630.6 [M + Na]⁺, 1333.2 [M - (tricyclohexylphosphine oxide) + Na]⁺.

### (EXAMPLE 124)

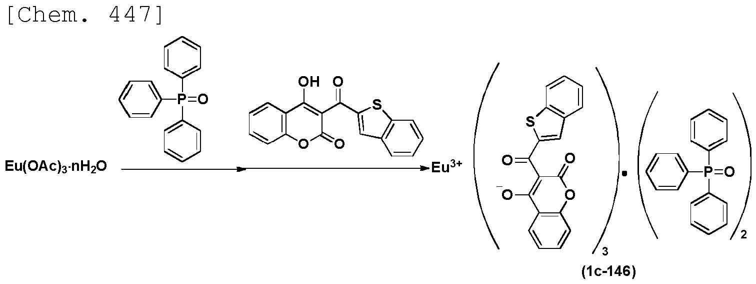

Ethanol (5.00 mL) was added to europium(III) acetate hydrate (35.0 mg as a 2.5-hydrate, 93.5 µmol) and triphenylphosphine oxide (52.0 mg, 187 µmol), and the resulting mixture was stirred for 1 hour at room temperature. The 4-hydroxy-3-(benzothiophene-2-carbonyl)-2H-chromen-2-one obtained in Reference Example 72 (90.4 mg, 280 µmol) was added to the reaction mixture. 14.8 M aqueous ammonia (80.0 µL, 1.18 mmol) was added dropwise, and the resulting mixture was stirred for 3 hours at room temperature. The reaction solution was concentrated under reduced pressure, the residue was washed with water and ethanol and then dissolved in chloroform, and an undissolved portion was removed by filtration through Celite to give a light brown solid of tris[3-(benzo[b]thiophene-2-carbonyl)-4-hydroxy-2H-chromen-2-onato]bis(triphenylphosphine oxide)europium(III) (lc-146) (actual yield, 136 mg; percent yield, 870) . ³¹P-NMR (162 MHz, CDCl₃) δ (ppm): -78.9 (brs). ESIMS (m/z), MeOH: 1648.0 [M + Na]⁺, 1368.9 [M - (triphenylphosphine oxide) + Na]⁺.

### (EXAMPLE 125)

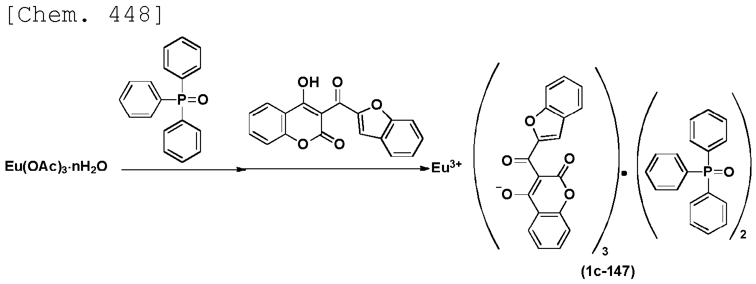

Ethanol (15.0 mL) was added to europium(III) acetate hydrate (112 mg as a 2.5-hydrate, 300 µmol) and triphenylphosphine oxide (167 mg, 600 µmol), and the resulting mixture was stirred for 1 hour at room temperature. The 3-(benzofuran-2-carbonyl)-4-hydroxy-2H-chromen-2-one obtained in Reference Example 73 (276 mg, 901 µmol) was added to the reaction mixture. 14.8 M aqueous ammonia (60.0 µL, 888 µmol) was added dropwise, and the resulting mixture was stirred for 3 hours at room temperature. The reaction solution was concentrated under reduced pressure, and the residue was washed with a 60% aqueous solution of ethanol to give a yellow solid of tris[3-(benzofuran-2-carbonyl)-4-hydroxy-2H-chromen-2-onato]bis(triphenylphosphine oxide)europium(III) (lc-147) (actual yield, 449 mg; percent yield, 92%). ³¹P-NMR (162 MHz, CDCl₃) δ (ppm): -77.5 (brs). ESIMS (m/z), MeOH: 1697.4 [M + Na]⁺, 1351.2 [M - (3-(benzofuran-2-carbonyl)-4-hydroxy-2H-chromen-2-onato)]⁺.

### (COMPARATIVE EXAMPLE 1)

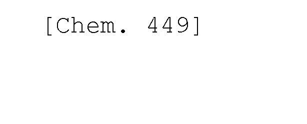

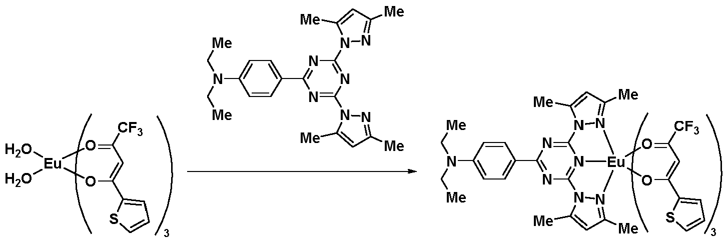

Synthesis was performed according to NPL 3. Tetrahydrofuran (160 mL) was added to the diaquatris[4,4,4-trifluoro-1-(2-thienyl)-1,3-butanedionato]europium(III) obtained in Reference Example 13 (667 mg, 800 µmol) and the 4-[4,6-bis(3,5-dimethyl-1H-pyrazol-1-yl)-1,3,5-triazin-2-yl]-N,N-diethylaniline obtained in Reference Example 14 (334 mg, 800 µmol), and the resulting mixture was stirred for 2 hours at room temperature. The reaction mixture was filtered, and the residue was washed with tetrahydrofuran and dried at room temperature under reduced pressure. Recrystallization from the resulting crude product was performed twice (hexane/ethyl acetate and hexane/chloroform) to give a yellow powder of bis{4-[4,6-bis(3,5-dimethyl-1H-pyrazol-1-yl)-1,3,5-triazin-2-yl]-N,N-diethylaniline}tris[4,4,4-trifluoro-1-(2-thienyl)-1,3-butanedionato]europium(III) (actual yield, 808 mg; percent yield, 82%) . ¹⁹F-NMR (376 MHz, CDCl₃) δ (ppm) : -81.1 (brs). ESIMS (m/z): 1008.9 [M - (4,4,4-trifluoro-1-phenyl-1,3-butanedionato)]⁺.

### EVALUATION EXAMPLE

In the measurement of the emission spectrum of the rare earth complexes according to the present invention obtained in Examples 1 to 125 and the rare earth complex obtained in Comparative Example 1, and as samples for the evaluation of light resistance, thin films of PMMA (polymethyl methacrylate resin) containing 1.0 wt% of the rare earth complexes obtained in Examples 1 to 125 and the rare earth complex obtained in Comparative Example 1 were used. The 1.0 wt% rare earth complex-containing thin PMMA films were prepared by dissolving 4.5 mg of the rare earth complex and 445 mg of PMMA pellets (G-1000, Kuraray Co., Ltd.'s product) in 2 mL of chloroform, bar-coating the resulting PMMA solution on water-white glass and air-drying the coating.

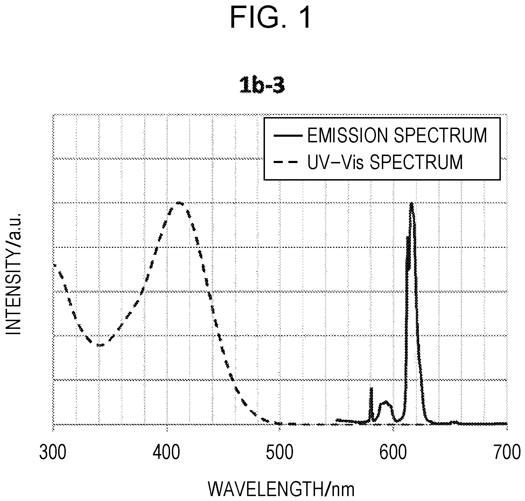

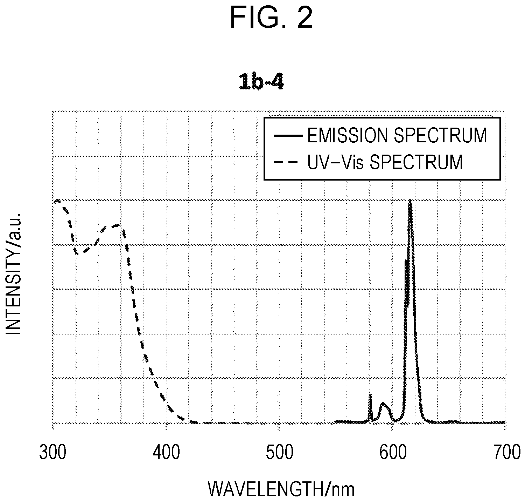

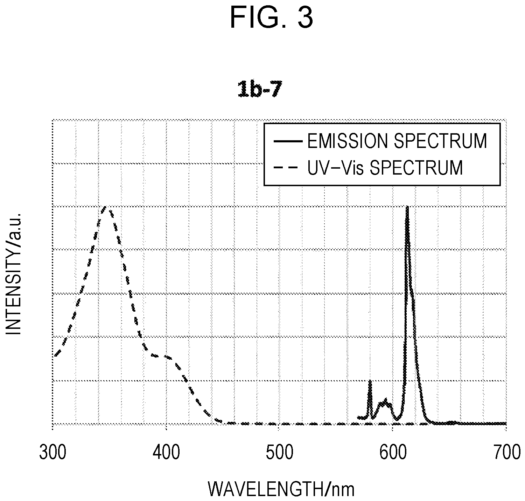

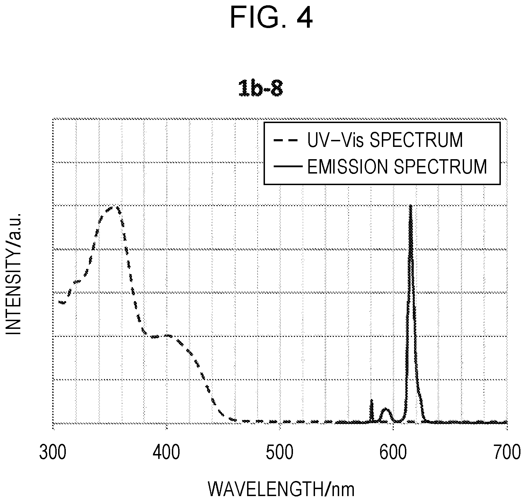

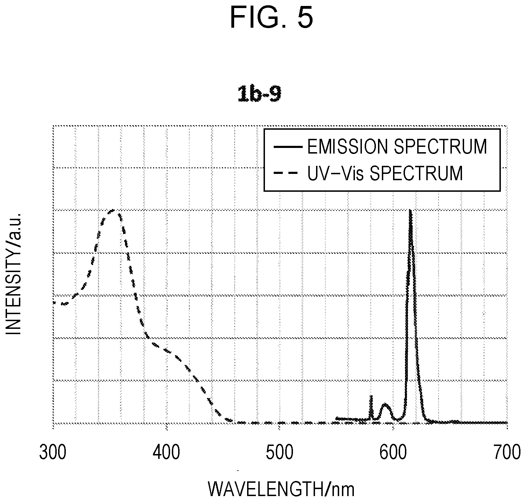

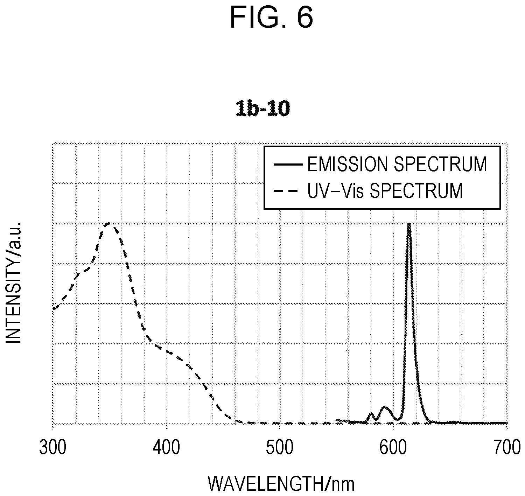

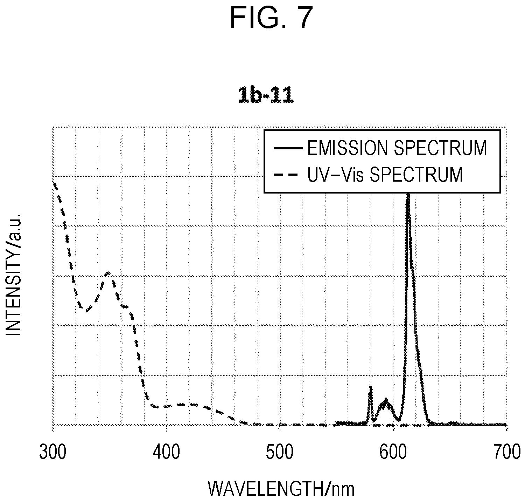

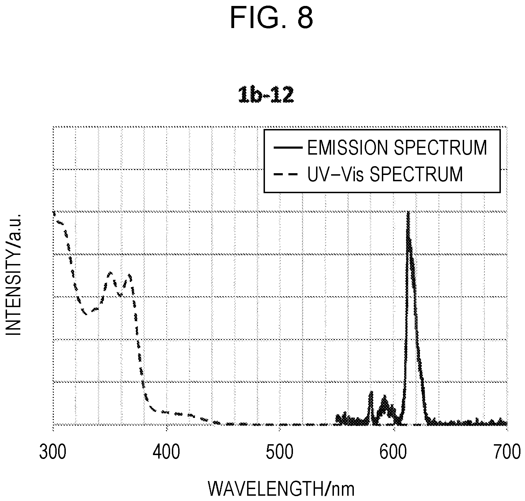

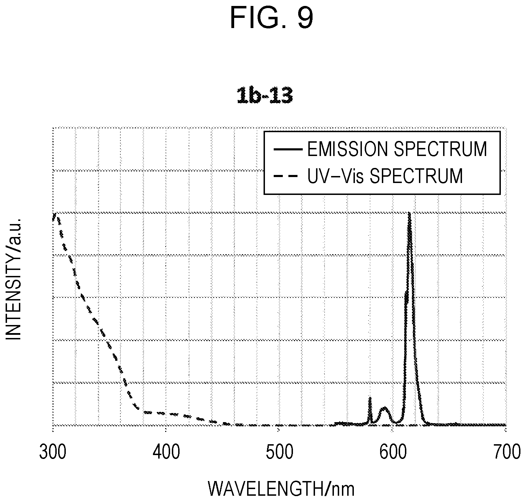

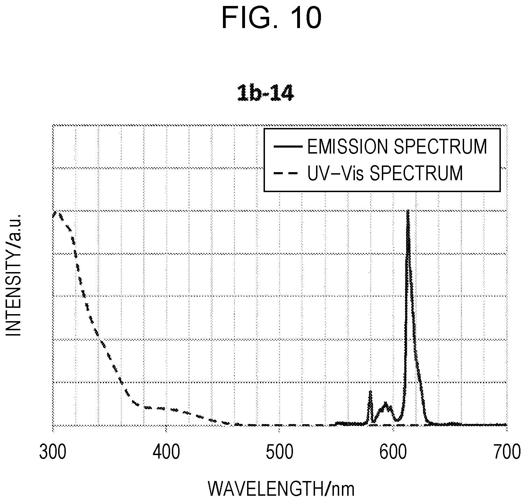

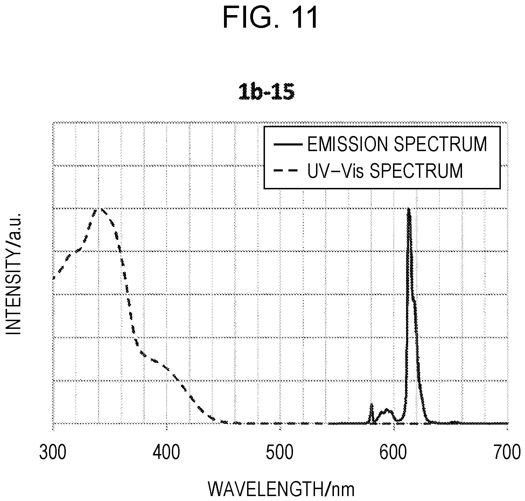

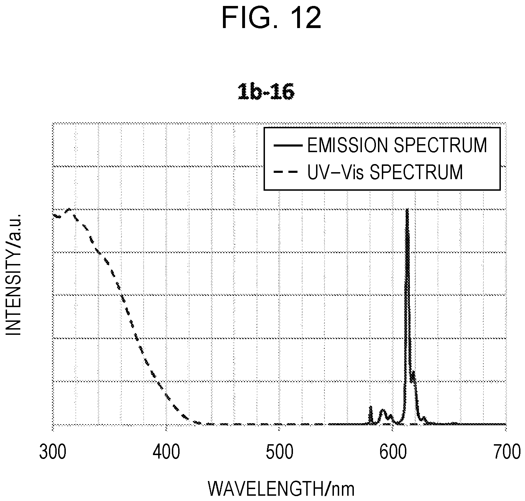

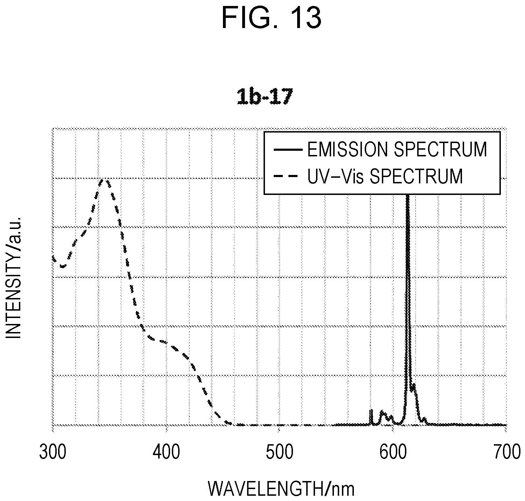

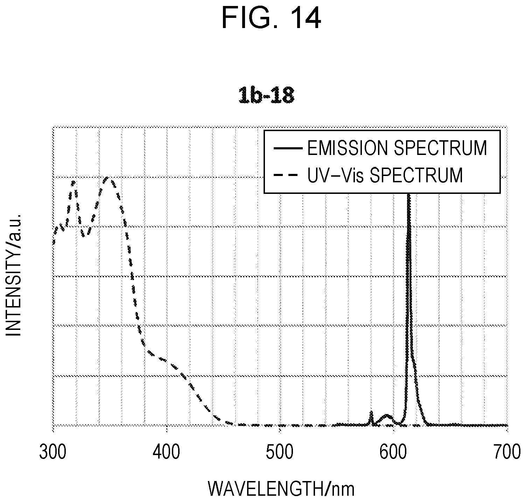

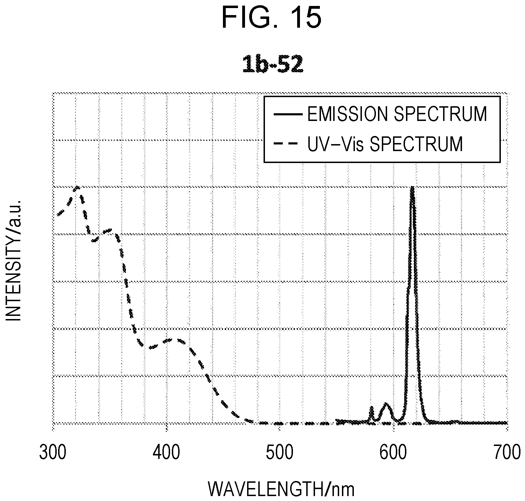

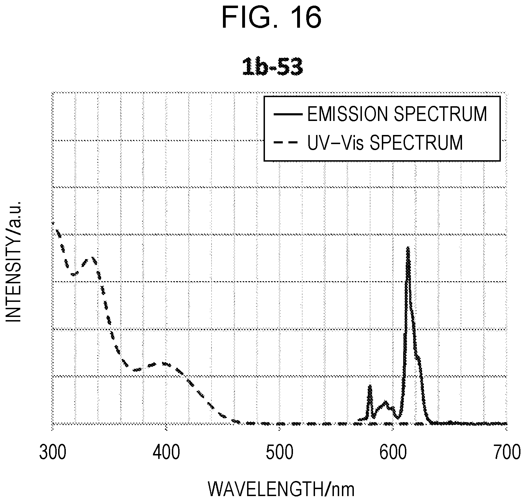

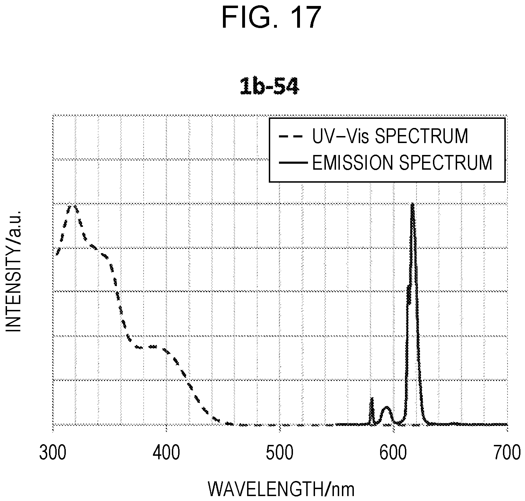

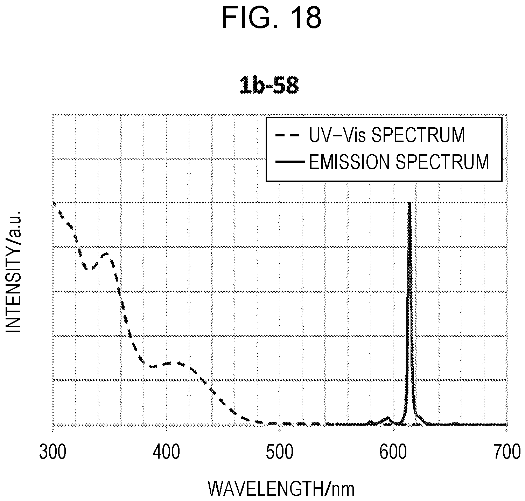

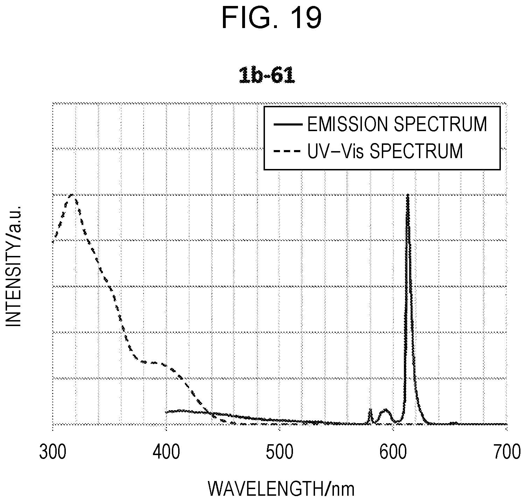

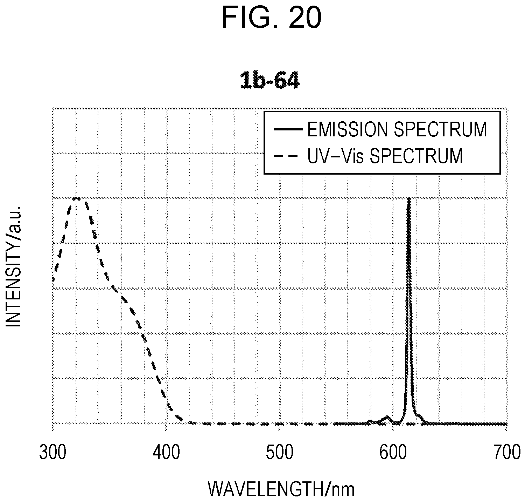

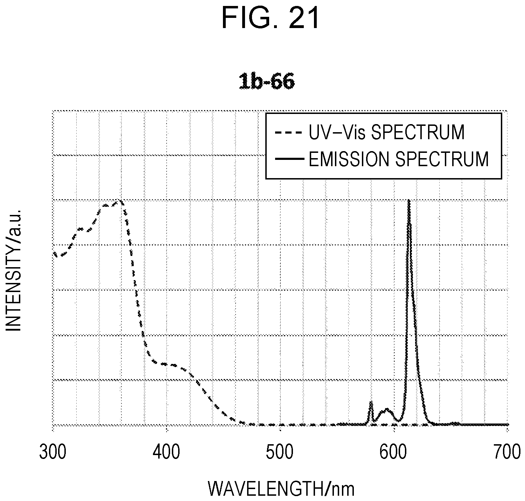

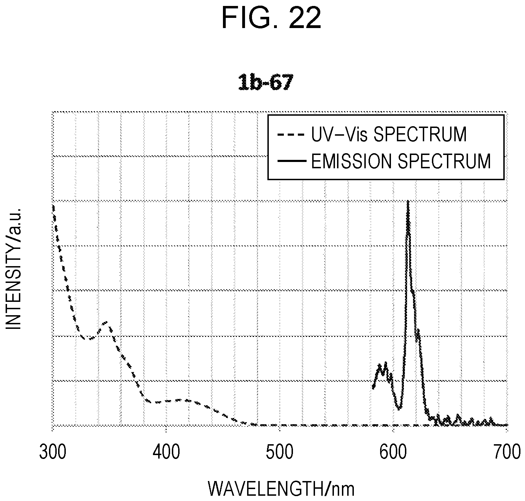

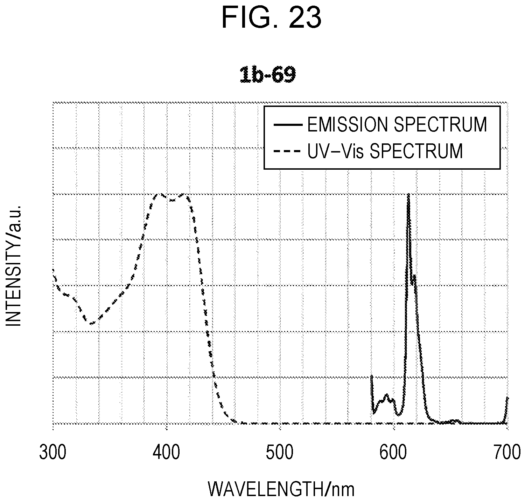

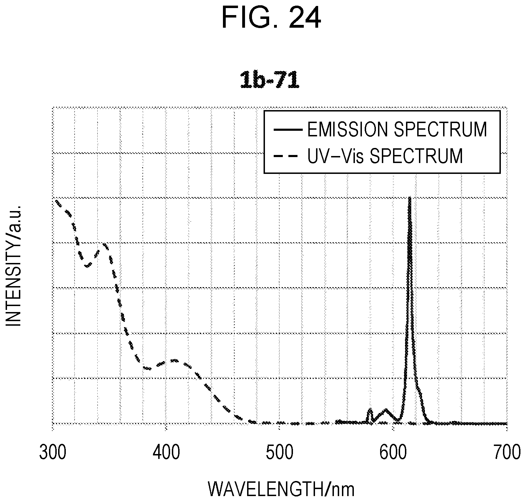

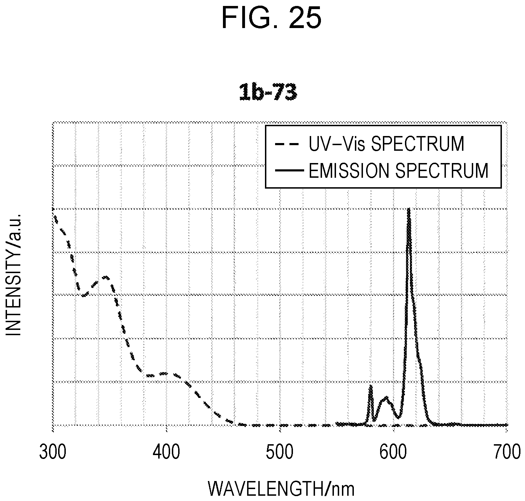

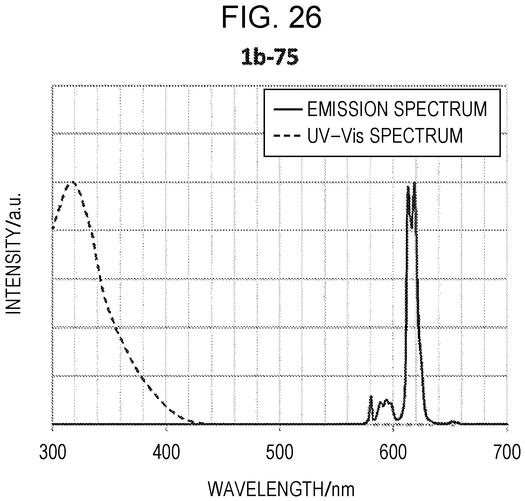

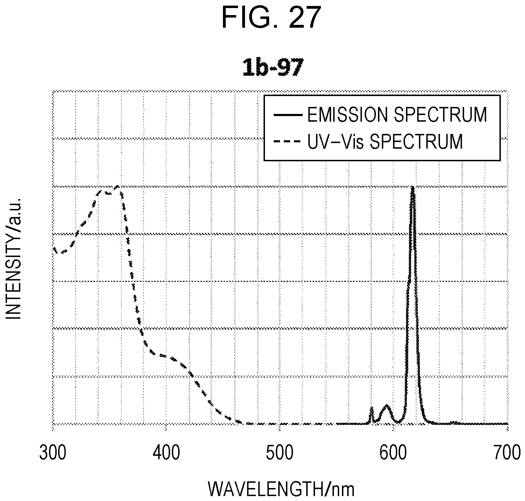

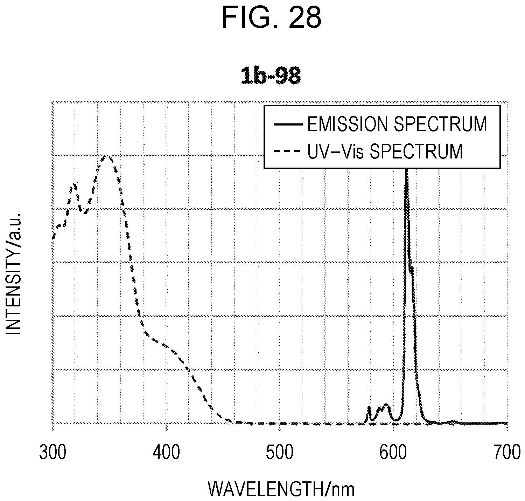

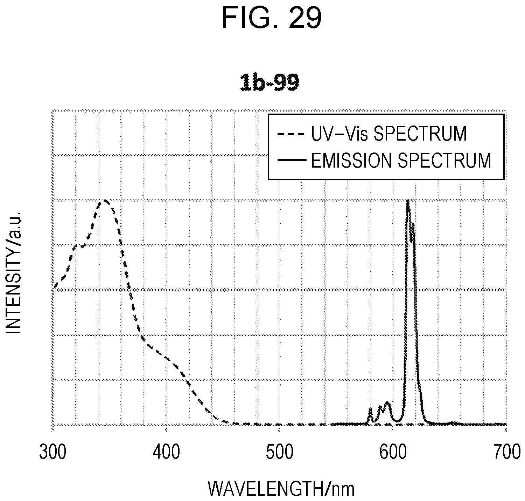

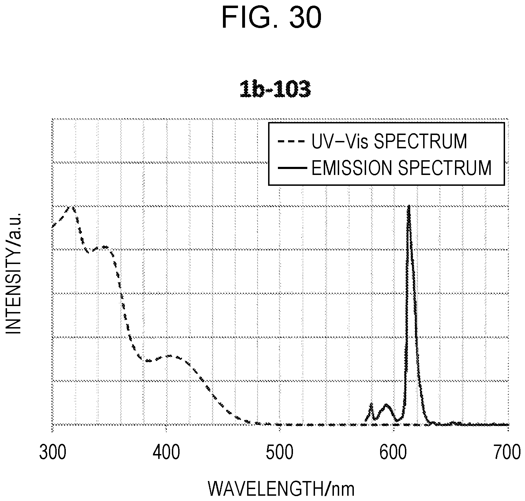

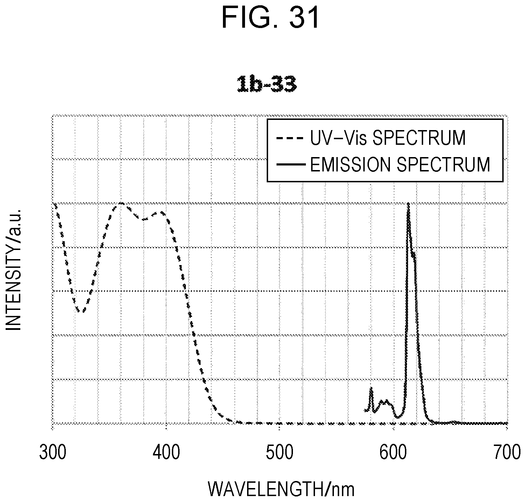

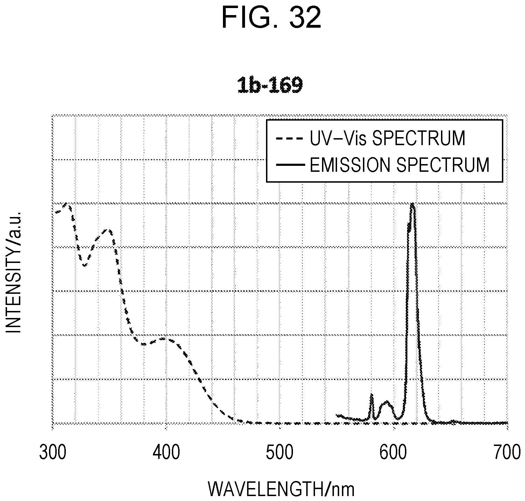

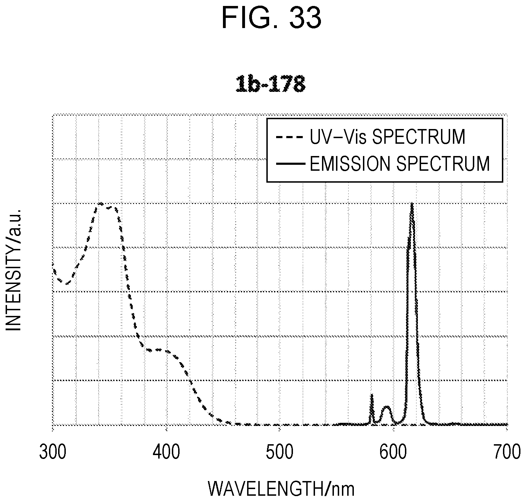

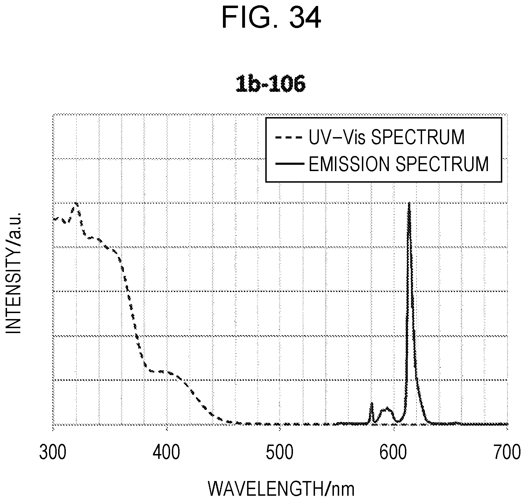

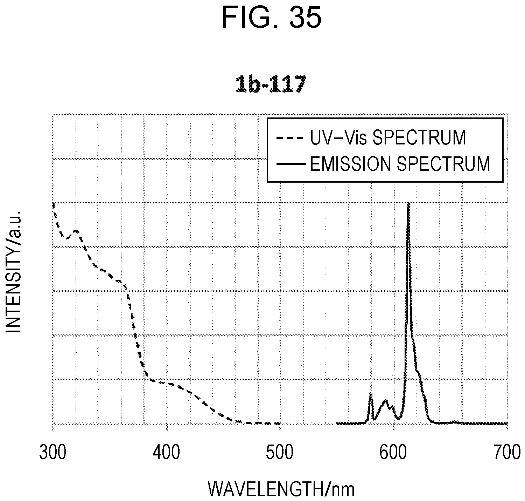

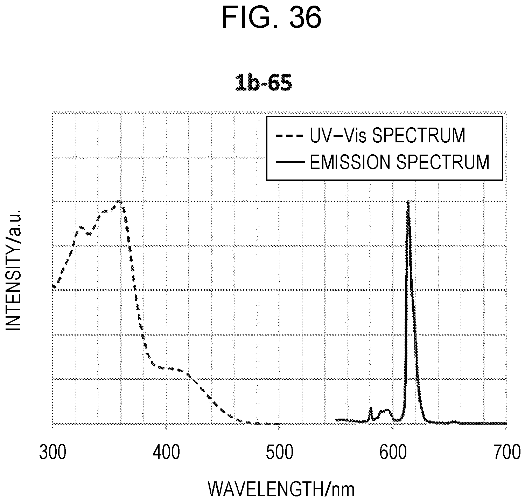

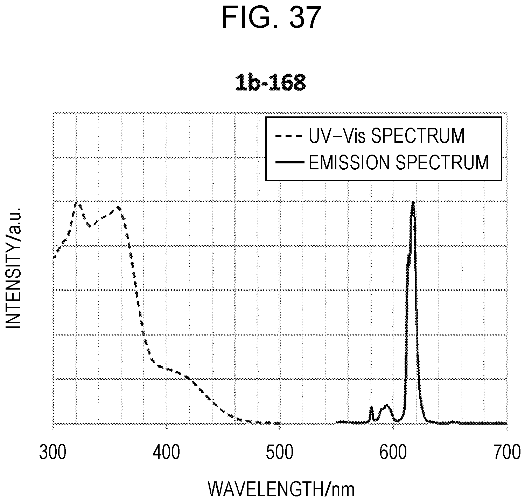

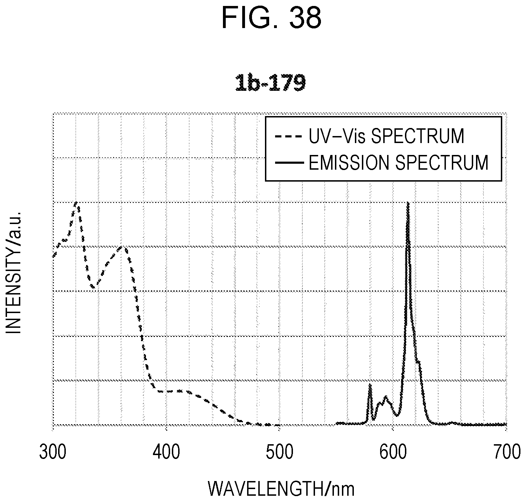

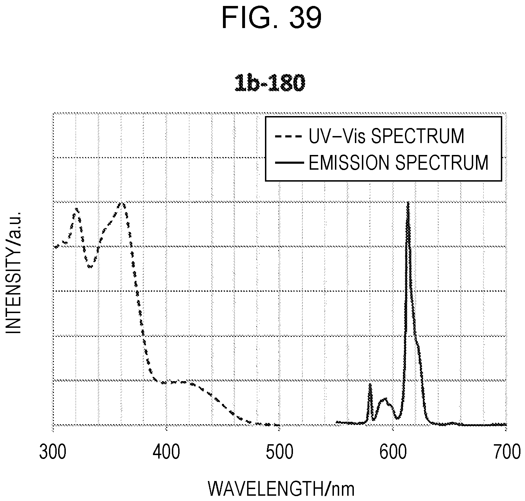

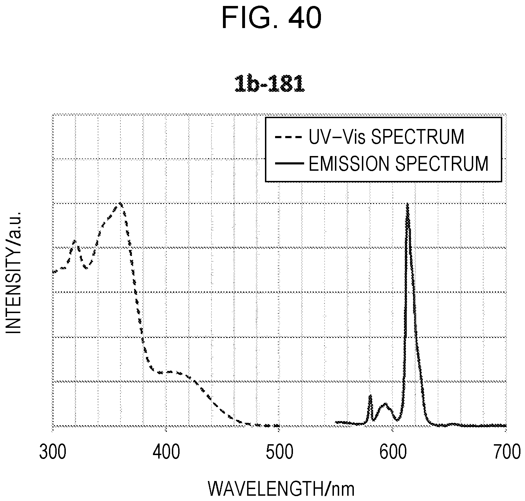

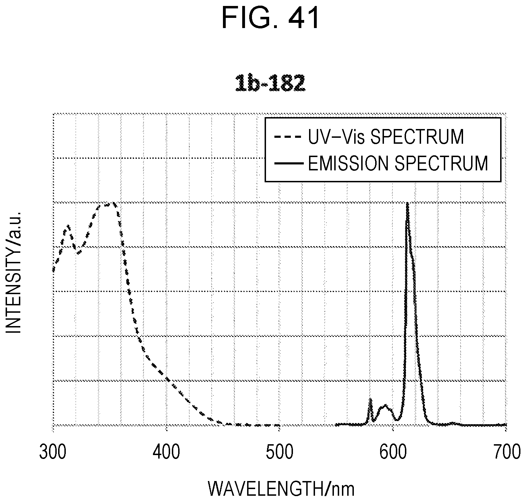

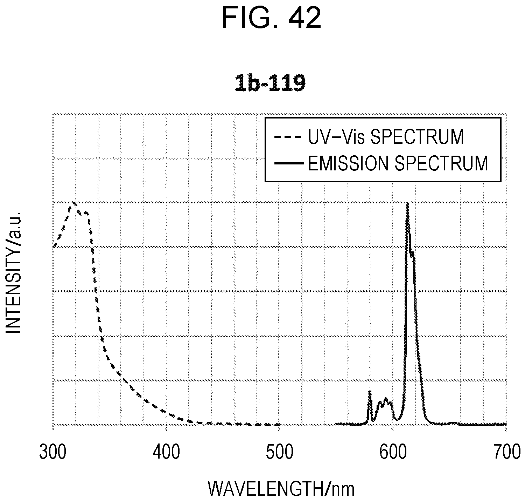

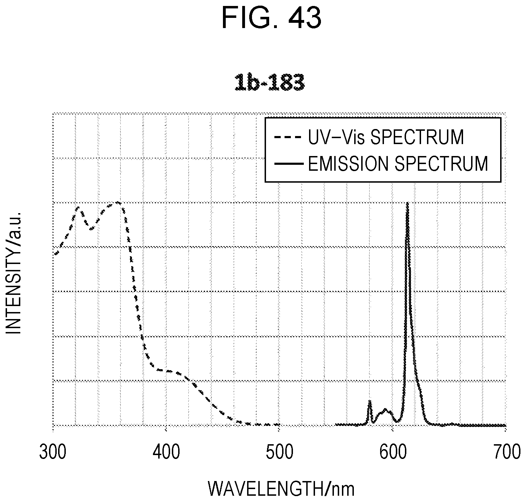

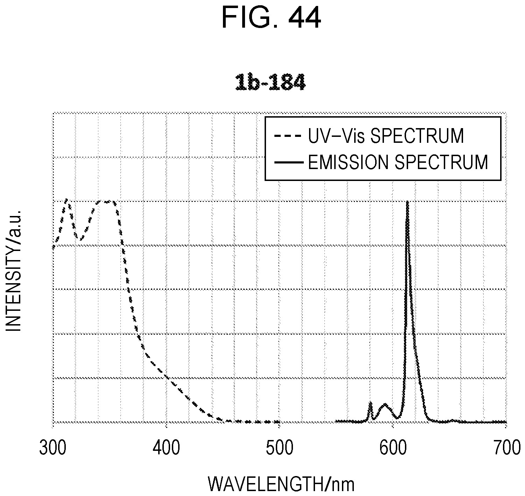

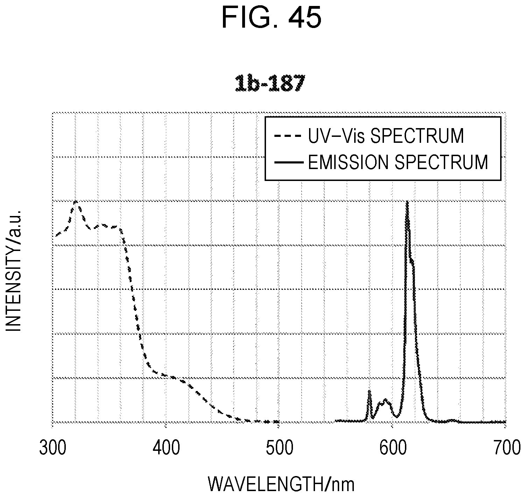

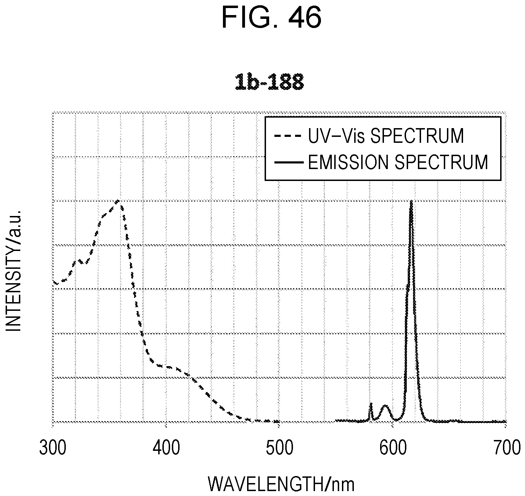

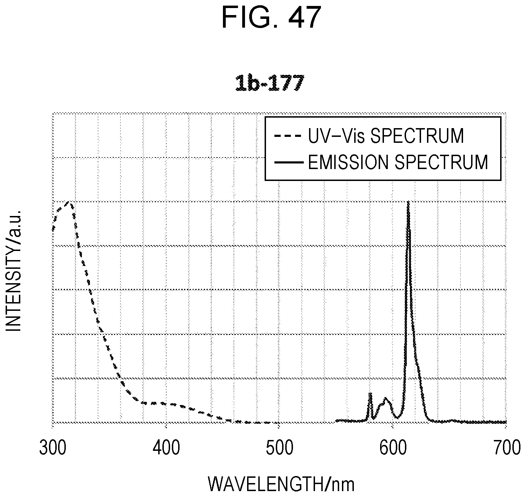

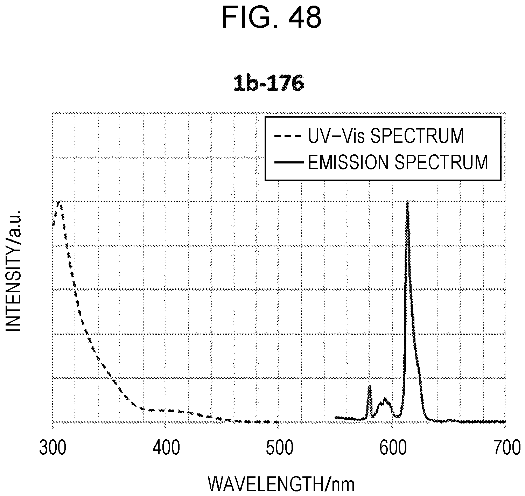

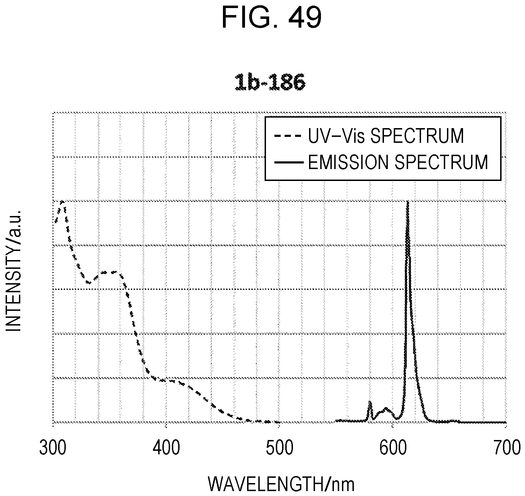

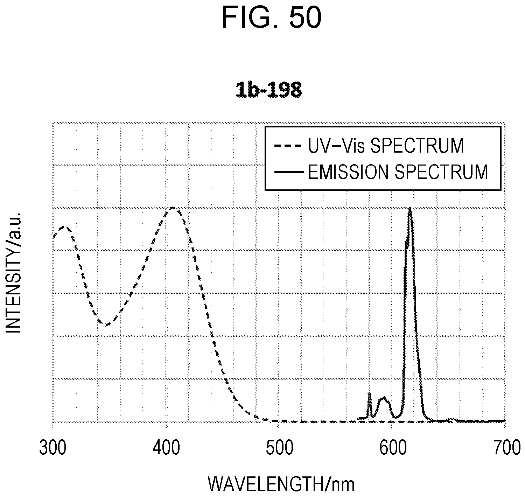

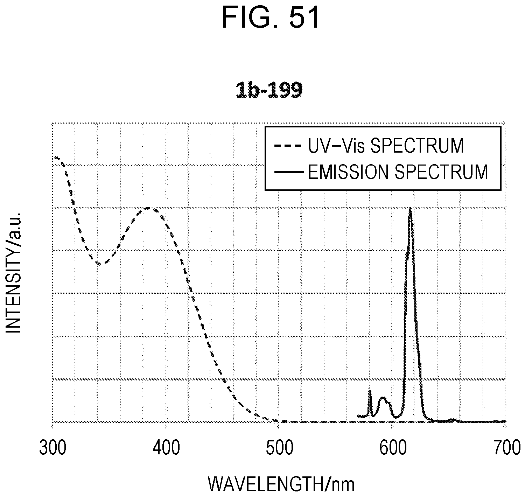

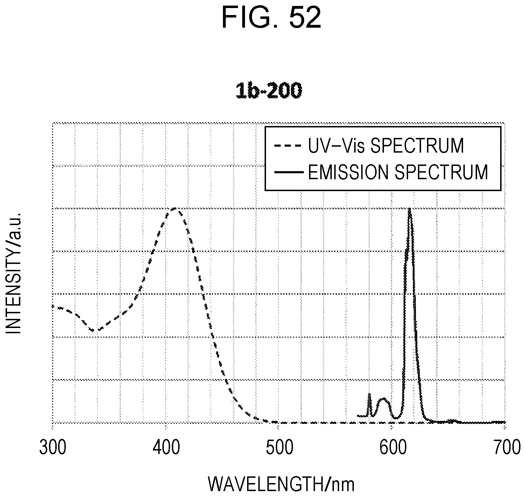

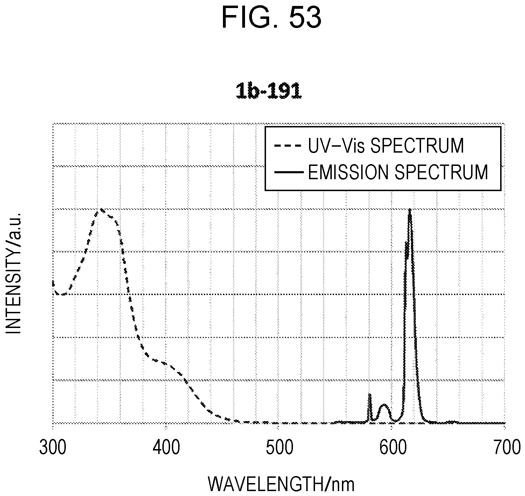

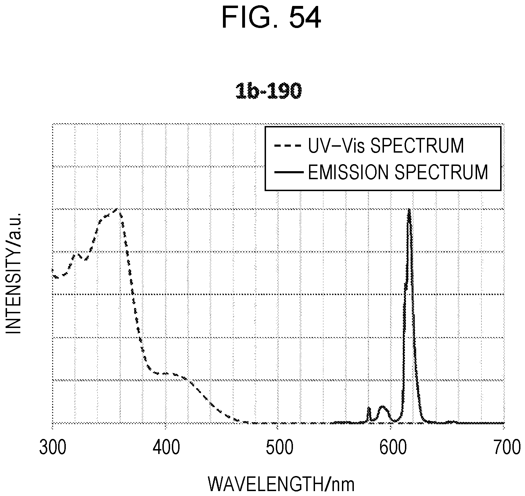

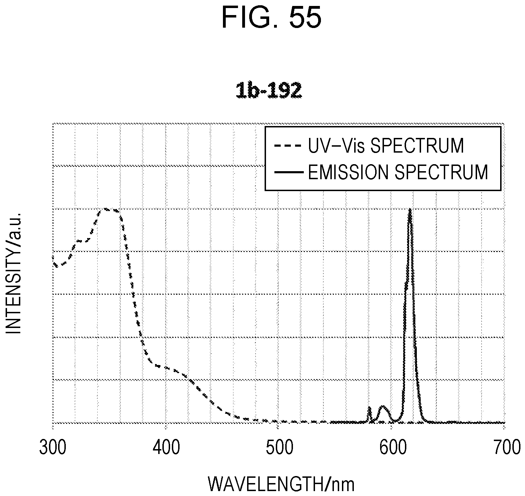

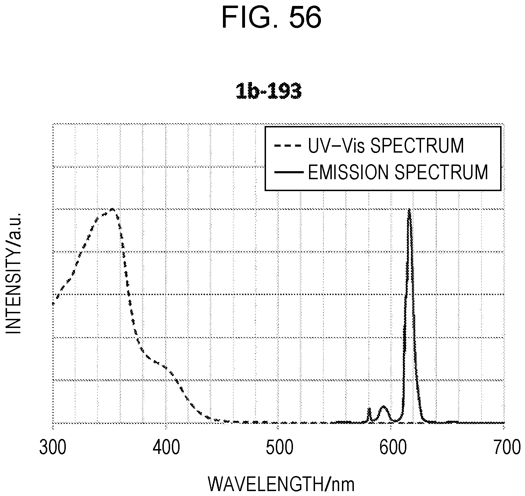

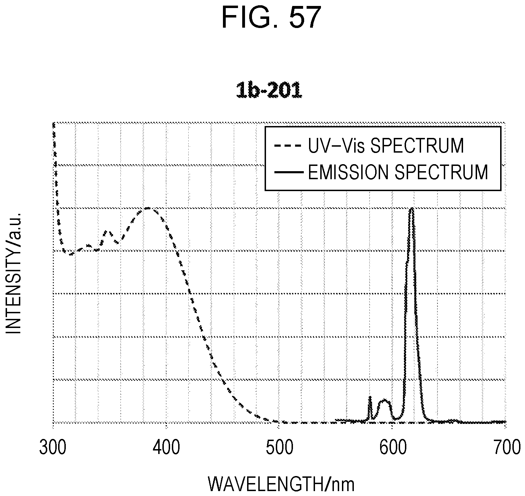

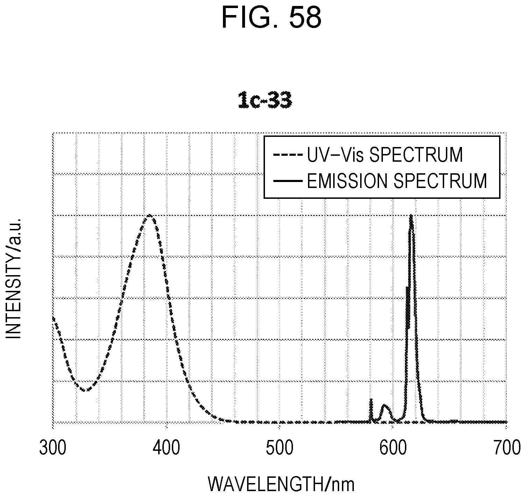

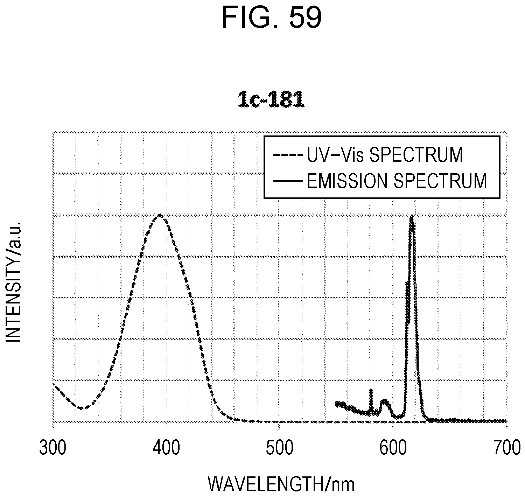

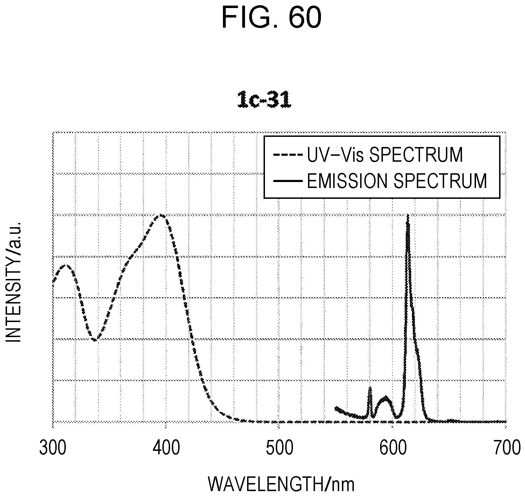

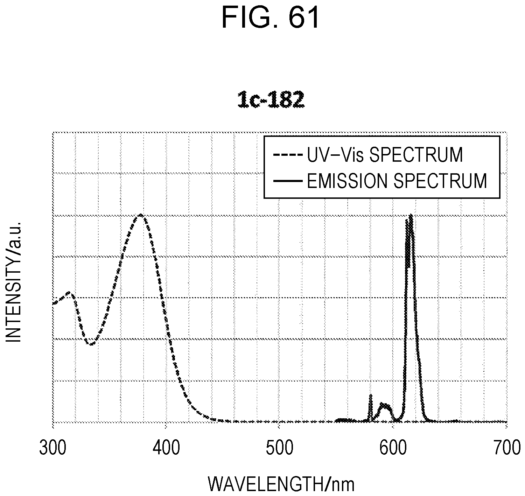

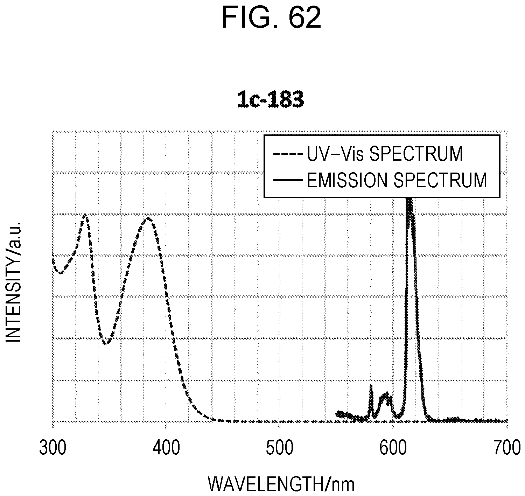

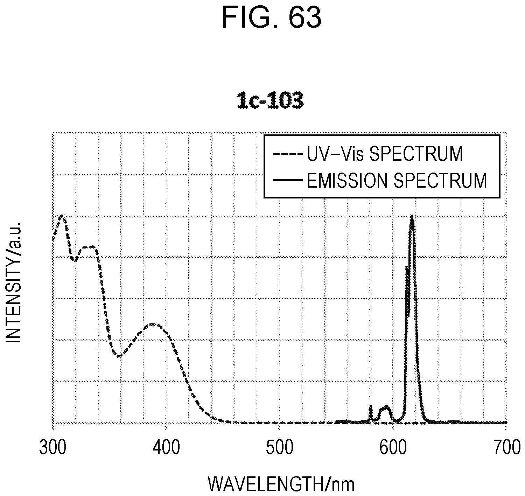

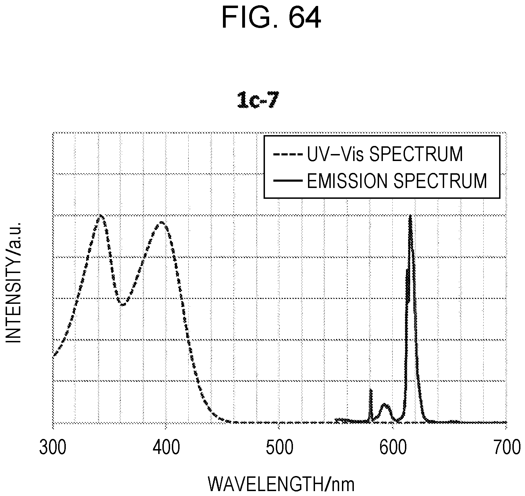

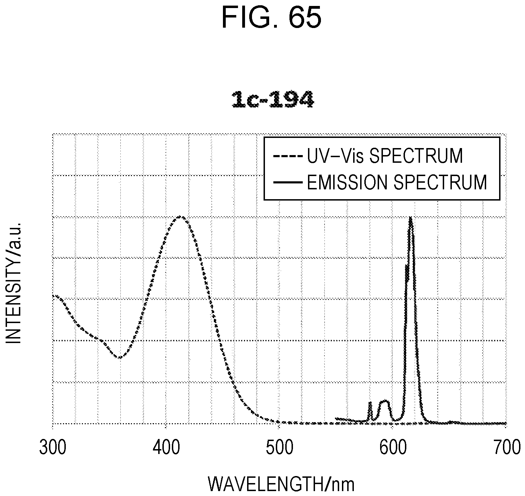

Measured emission spectra (excitation light, 350 nm) of the rare earth complexes obtained in Examples 1 to 125 are presented in Figs. 1 to 65.

Light resistance was evaluated by the method described below. In the evaluation of the light resistance of Examples 1 to 125 and Comparative Example 1, the emission intensity at a maximum emission wavelength near 615 nm was measured using a spectrophotometer (FP-6500, manufactured by JASCO Corporation). The wavelength of the excitation light was set to 350 nm. Then, at room temperature, the sample was irradiated with blue light having a luminance of 1000 Cd/m² for a predetermined length of time (6 hours) using a 450-nm LED light source (CL-1503 and CL-H1-450-9-1, manufactured by Asahi Spectra Co., Ltd.). After the irradiation with blue light, the emission intensity of the sample was measured using the spectrophotometer again, and light resistance was evaluated by calculating the percentage preservation of emission intensity compared with the initial state according to the following equation.

Percentage preservation (%) of emission intensity (I/I0) = (Emission intensity at the maximum emission wavelength after the irradiation with blue light)/(Emission intensity at the maximum emission wavelength before the irradiation with blue light) × 100

For the rare earth complexes according to the present invention obtained in Examples 1 to 125, the results for the percentage preservation of emission intensity at 6 hours after irradiation with UV light are presented in Tables 1 and 2.

**[Table 1]**

| Sample | Percentage preservation of emission intensity | Sample | Percentage preservation of emission intensity | Sample | Percentage preservation of emission intensity |
|---|---|---|---|---|---|
| 1b-1 | 99% | 1b-61 | 97% | 1b-178 | >99% |
| 1b-2 | 97% | 1b-64 | >99% | 1b-179 | 94% |
| 1b-3 | 88% | 1b-65 | 99% | 1b-1 80 | 94% |
| 1b-5 | 93% | 1b-66 | >99% | 1b-181 | 97% |
| 1b-6 | 90% | 1b-67 | 97% | 1b-182 | 99% |
| 1b-8 | 99% | 1b-69 | 89% | 1b-183 | >99% |
| 1b-9 | 97% | 1b-71 | 91% | 1b-184 | 99% |
| 1b-10 | 97% | 1b-73 | 99% | 1b-186 | 98% |
| 1b-11 | 98% | 1b-75 | >99% | 1b-187 | >99% |
| 1b-12 | 92% | 1b-97 | 99% | 1b-188 | >99% |
| 1b-13 | >99% | 1b-98 | 95% | 1b-1 90 | >99% |
| 1b-14 | 98% | 1b-99 | >99% | 1b-191 | >99% |
| 1b-15 | 99% | 1b-103 | 94% | 1b-192 | >99% |
| 1b-16 | 96% | 1b-106 | >99% | 1b-193 | >99% |
| 1b-17 | 97% | 1b-117 | 99% | 1 b-197 | 82% |
| 1b-33 | 99% | 1b-119 | 98% | 1b-198 | >99% |
| 1b-52 | 94% | 1b-168 | >99% | 1b-199 | 85% |
| 1b-53 | 93% | 1b-169 | >99% | 1b-200 | 83% |
| 1b-54 | 92% | 1b-176 | 99% | 1b-201 | 96% |
| 1b-58 | 88% | 1b-177 | 97% | Comparative Example 1 | 73% |

**[Table 2]**

| Sample | Percentage preservation of emission intensity | Sample | Percentage preservation of emission intensity |
|---|---|---|---|
| 1c-1 | 97% | 1c-179 | >99% |
| 1c-7 | 97% | 1c-180 | >99% |
| 1c-8 | 99% | 1c-181 | 95% |
| 1c-11 | 99% | 1c-182 | 97% |
| 1c-23 | >99% | 1c-183 | 98% |
| 1c-31 | 99% | 1c-185 | >99% |
| 1c-32 | 89% | 1c-191 | >99% |
| 1c-33 | 96% | 1c-192 | >99% |
| 1c-64 | 98% | 1c-193 | >99% |
| 1c-65 | >99% | 1c-194 | 91% |
| 1c-103 | >99% | 1c-195 | 89% |
| 1c-112 | >99% | 1c-197 | 98% |
| 1c-178 | 98% | Comparative Example 1 | 73% |

As shown in Tables 1 and 2, it was found that rare earth complexes (1) according to the present invention have high light resistance by virtue of the introduction of specific ligands.

### Industrial Applicability

The rare earth complex (1) according to the present invention is excitable with blue light and superior in light resistance. The rare earth complex (1), therefore, is useful as a film for solar cells, an agricultural film, an LED phosphor and a light-emitting material, for example for security inks, and a wavelength conversion material used in them.

## Claims

1. A rare earth complex represented by general formula (1) .
[Chem. 1]
**M³⁺ • (HC)ₙ • (L¹)ₘ₁ • (L²)ₘ₂ • (X^{L})₃₋ₙ** **(1)**
{HC is a quinolinonato ligand represented by general formula (lqu) or coumarinato ligand represented by general formula (lcu). 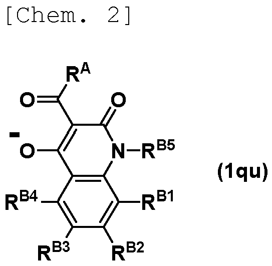
[In the formula, R^{A} represents a C1 to C6 haloalkyl group, C6 to C22 aryl group or C3 to C20 heteroaryl group. The aryl group and the heteroaryl group are optionally substituted with one or more substituents selected from the group consisting of C1 to C6 fluoroalkyl groups; C1 to C6 alkyloxy groups; C1 to C6 fluoroalkyloxy groups; C5 to C14 aryloxy groups; C1 to C6 monoalkylamino groups; C6 to C12 monoarylamino groups; C4 to C12 monoheteroarylamino groups; C2 to C12 dialkylamino groups; C10 to C24 diarylamino groups optionally substituted with one or more cyano groups, halogen atoms or C1 to C6 fluoroalkyl groups; C8 to C24 diheteroarylamino groups optionally substituted with one or more fluorine atoms or C1 to C6 fluoroalkyl groups; C3 to C20 heteroaryl groups; C2 to C13 acyl groups; methylenedioxy groups; ethylenedioxy groups; halogen atoms; hydroxyl groups; nitro groups and cyano groups.
R^{B1}, R^{B2}, R^{B3}, R^{B4} and R^{B5} each independently represent a hydrogen atom, halogen atom, C1 to C10 alkyl group, C2 to C4 alkenyl group, C1 to C6 haloalkyl group, C6 to C22 aryl group, C3 to C20 heteroaryl group, cyano group, nitro group, C7 to C14 aralkyl group optionally substituted with one or more halogen atoms, substituted or unsubstituted amino group indicated by general formula (BA1) below, substituted or unsubstituted oxy group indicated by general formula (BOl) below or substituted or unsubstituted thio group indicated by general formula (BS1) below, with the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, cyano groups and nitro groups. 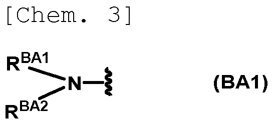
(In the formula, R^{BA1} and R^{BA2} each independently represent a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups. R^{BA1} and R^{BA2} optionally form a five-membered, six-membered or seven-membered ring including the nitrogen atom to which the R^{BA1} and R^{BA2} are bound.) 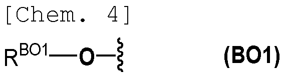
(In the formula, R^{BO1} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.) 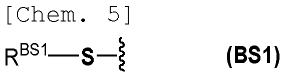
(In the formula, R^{BS1} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)
Of R^{B1}, R^{B2}, R^{B3} and R^{B4}, two adjacent substituents optionally form a five-membered, six-membered or seven-membered ring together with the benzene ring to which the substituents are bound.
R^{B5} and R^{B1} optionally form a five-membered, six-membered or seven-membered ring together with the quinolinone ring to which the R^{B5} and R^{B1} are bound.] 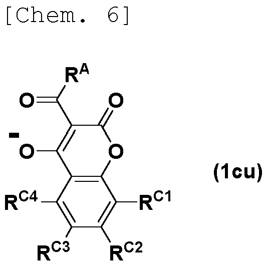
[In the formula, R^{A} is synonymous with R^{A} in general formula (lqu).
R^{C1}, R^{C2}, R^{C3} and R^{C4} each independently represent a hydrogen atom, halogen atom, C1 to C10 alkyl group, C2 to C4 alkenyl group optionally substituted with one or more C1 to C6 alkyloxy groups, C1 to C6 haloalkyl group, C6 to C22 aryl group, C3 to C20 heteroaryl group, cyano group, nitro group, C7 to C14 aralkyl group optionally substituted with one or more halogen atoms, substituted or unsubstituted amino group indicated by general formula (BA1) below, substituted or unsubstituted oxy group indicated by general formula (BOl) below or substituted or unsubstituted thio group indicated by general formula (BS1) below, with the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, cyano groups and nitro groups. 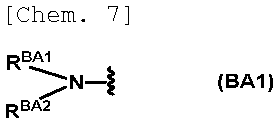
(In the formula, R^{BA1} and R^{BA2} are synonymous with the meanings described above.) 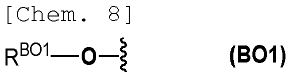
(In the formula, R^{BO1} is synonymous with the meaning described above.) 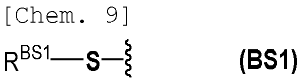
(In the formula, R^{BS1} is synonymous with the meaning described above.)
Of R^{C1}, R^{C2}, R^{C3} and R^{C4}, two adjacent substituents optionally form a five-membered, six-membered or seven-membered ring together with the benzene ring to which the substituents are bound.]
n represents 1, 2 or 3. When n is 2 or 3, the plurality of quinolinonato ligands or coumarinato ligands may be the same or different from each other.
m¹ represents 0, 1 or 2, m² represents 0 to 3, and 0 ≤ m¹ + m² ≤ 3.
L¹ represents a nitrogen-containing ligand having two or more nitrogen atoms possessing a lone pair or phosphorus ligand represented by general formula (3a) below or general formula (3b) below. When m¹ is 2, the L¹s may be the same or different from each other. 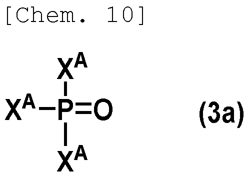 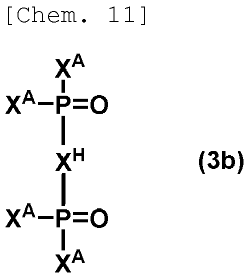
[In the formulae, the X^{A}s each independently represent a C1 to C10 alkyl group, aryl group represented by general formula (3c) below, substituted or unsubstituted amino group indicated by general formula (BA2) below or substituted or unsubstituted oxy group indicated by general formula (BO2) below. 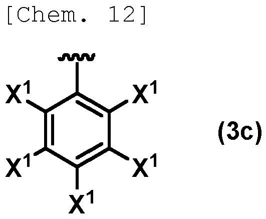
(In the formula, the X¹s each independently represent a hydrogen atom, halogen atom, C1 to C6 alkyl group, C1 to C6 alkyloxy group, C6 to C22 aryl group, C3 to C20 heteroaryl group, C5 to C14 aryloxy group, C1 to C6 haloalkyl group or C1 to C6 haloalkyloxy group, with the aryl group optionally being substituted with one or more substituents selected from the group consisting of halogen atoms, C1 to C6 alkyloxy groups, C1 to C6 haloalkyl groups, C1 to C6 haloalkyloxy groups, hydroxyl groups, cyano groups and nitro groups.) 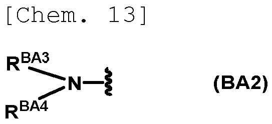
(In the formula, R^{BA3} and R^{BA4} each independently represent a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups. R^{BA3} and R^{BA4} optionally form a five-membered, six-membered or seven-membered ring including the nitrogen atom to which the R^{BA3} and R^{BA4} are bound.) 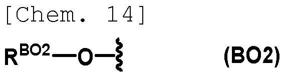
(In the formula, R^{BO2} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)
X^{H} represents a C1 to C10 alkylene group, C2 to C10 alkenylene group, C2 to C10 alkynylene group, C5 to C24 arylene group or C4 to C23 heteroarylene group.]
L² represents a neutral ligand selected from the group consisting of water, deuterium oxide, a sulfoxide compound, sulfone compound, amide compound, nitrile compound, ester compound, carbonyl compound, ether compound and alcohol.
When m² is 2 or 3, the L²s may be the same or different from each other.
X^{L} represents a halide ion, nitrate ion, carboxylate ion, sulfonate ion or C5 to C12 β-diketonato ion.
M³⁺ represents a trivalent rare earth metal ion.}

2. The rare earth complex according to Claim 1, wherein HC is a quinolinonato ligand represented by general formula (1qu), and n is 3.

3. The rare earth complex according to Claim 1, wherein HC is a quinolinonato ligand represented by general formula (1qu), and M³⁺ is a europium(III) ion, terbium(III) ion, samarium(III) ion or gadolinium(III) ion.

4. The rare earth complex according to Claim 1, wherein HC is a quinolinonato ligand represented by general formula (1qu), and R^{A} is a phenyl group optionally substituted with one or more substituents selected from the group consisting of fluorine atoms, diphenylamino groups or C1 to C6 fluoroalkyl groups, with the diphenylamino group optionally being substituted with one or more fluorine atoms, C1 to C6 fluoroalkyl groups or cyano groups.

5. The rare earth complex according to Claim 1, wherein the general formula (lqu) is a quinolinonato ligand indicated by any of general formulae (lqu-1) to (lqu-8) below. 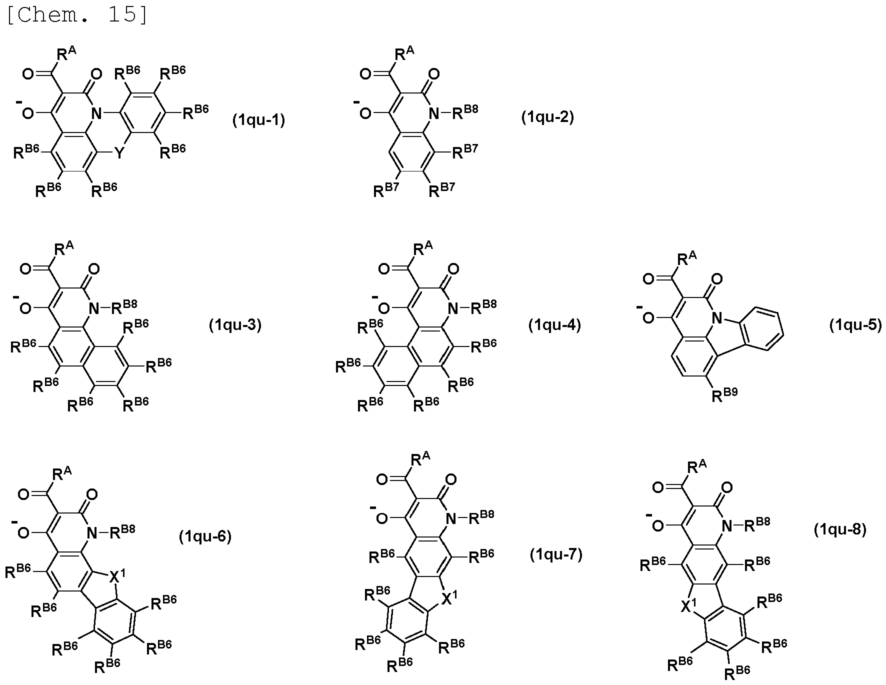 [In the formulae, R^{A} is synonymous with R^{A} in general formula (lqu).
R^{B6}s each independently represent a hydrogen atom, halogen atom or C1 to C4 fluoroalkyl group.
R^{B7}s each independently represent a hydrogen atom, halogen atom, cyano group, C1 to C8 alkyl group, C1 to C4 fluoroalkyl group, C6 to C22 aryl group, substituted or unsubstituted amino group indicated by general formula (BA3) below or substituted or unsubstituted oxy group indicated by general formula (BO3) below. 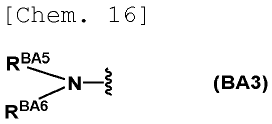
(In the formula, R^{BA5} and R^{BA6} each independently represent a C1 to C4 alkyl group or C6 to C12 aryl group, with the alkyl group and the aryl group optionally being substituted with one or more substituents selected from the group consisting of halogen atoms, C1 to C4 alkyloxy groups, C1 to C4 fluoroalkyl groups, C2 to C13 acyl groups, cyano groups and nitro groups. R^{BA5} and R^{BA6} optionally form a five-membered, six-membered or seven-membered ring including the nitrogen atom to which the R^{BA5} and R^{BA6} are bound.) 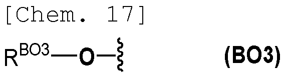
(In the formula, R^{BO3} represents a C1 to C4 alkyl group or C6 to C12 aryl group, with the alkyl group and the aryl group optionally being substituted with one or more substituents selected from the group consisting of halogen atoms, C1 to C4 alkyloxy groups, C1 to C4 fluoroalkyl groups, C2 to C13 acyl groups, cyano groups and nitro groups.)
Adjacent R^{B7}s optionally form a five-membered, six-membered or seven-membered ring together with the benzene ring to which the R^{B7}s are bound.
R^{B8} represents a hydrogen atom, C1 to C10 alkyl group or C6 to C22 aryl group optionally substituted with one or more fluorine atoms or C1 to C4 fluoroalkyl groups.
R^{B9} represents a hydrogen atom or diphenylamino group optionally substituted with one or more fluorine atoms or C1 to C4 fluoroalkyl groups.
Y represents a single bond, ethylene group, vinylene group optionally substituted with one or more methoxy groups or chlorine atoms, oxygen atom, sulfur atom, nitrogen atom optionally substituted with a C1 to C12 hydrocarbon group or methylene group optionally substituted with one or more C1 to C12 hydrocarbon groups.
W¹ represents an oxygen atom, sulfur atom, nitrogen atom optionally substituted with a C1 to C12 hydrocarbon group or methylene group optionally substituted with one or more C1 to C12 hydrocarbon groups.]

6. The rare earth complex according to Claim 5, wherein R^{B6} is a hydrogen atom.

7. The rare earth complex according to Claim 5, wherein R^{B7} is a hydrogen atom, C1 to C4 alkyl group, C1 to C4 alkyloxy group, C2 to C8 dialkylamino group or diphenylamino group optionally substituted with one or more fluorine atoms; C1 to C4 fluoroalkyl groups; or; cyano groups.

8. The rare earth complex according to Claim 5, wherein W¹ is an oxygen atom, sulfur atom, methylene group optionally substituted with one or more C1 to C4 alkyl groups or nitrogen atom optionally substituted with a C1 to C4 alkyl group or C6 to C12 aryl group.

9. The rare earth complex according to Claim 1, wherein HC is a quinolinonato ligand represented by general formula (1qu), m¹ is 1 or 2, and L¹ is phenanthroline optionally substituted with one or more methyl groups or phenyl groups, bipyridine optionally substituted with one or more methyl groups or phenyl groups, N,N-diethyl-4-{[4,6-bis(3,5-dimethyl-1H-pyrazol-1-yl)-1,3,5-triazin-2-yl]}aniline or dipyrido[3,2-a:2',3'-c]phenazine.

10. The rare earth complex according to Claim 1, wherein HC is a quinolinonato ligand represented by general formula (1qu), m² is 1, 2 or 3, and L² is water.

11. The rare earth complex according to Claim 1, wherein HC is a quinolinonato ligand represented by general formula (1qu), and, in general formulae (3a) and (3b), X^{A} is a C4 to C8 alkyl group or aryl group represented by general formula (3d) below, and X^{H} is a C1 to C4 alkylene group; diphenylether-2,2'-diyl group; naphthalen-1,8-diyl group; biphenyl-2,2'-diyl group; binaphthyl-2,2'-diyl group; bipyridin-2,2'-diyl group; or xanthendiyl group optionally substituted with one or more methyl groups. 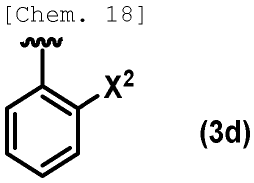 (In the formula, X² represents a hydrogen atom; fluorine atom; C1 to C4 alkyl group; C1 to C4 alkyloxy group; naphthyl group; pyridyl group; C1 to C4 fluoroalkyl group; or phenyl group optionally substituted with one or more fluorine atoms, C1 to C4 alkyl groups, C1 to C4 alkyloxy groups or C1 to C4 fluoroalkyl groups.)

12. The rare earth complex according to Claim 11, wherein X² in the general formula (3d) is a hydrogen atom, C1 to C4 alkyl group, C1 to C4 alkyloxy group or phenyl group optionally substituted with one or more C1 to C4 alkyl groups or C1 to C4 alkyloxy groups.

13. The rare earth complex according to Claim 1, wherein HC is a quinolinonato ligand represented by general formula (1qu), m¹ is 1 or 2, and m² is 0.

14. The rare earth complex according to Claim 1, wherein HC is a coumarinato ligand represented by general formula (lcu), and n is 3.

15. The rare earth complex according to Claim 1, wherein HC is a coumarinato ligand represented by general formula (lcu), and M³⁺ is a europium(III) ion, terbium(III) ion, samarium(III) ion or gadolinium(III) ion.

16. The rare earth complex according to Claim 1, wherein HC is a coumarinato ligand represented by general formula (lcu), and R^{A} is a phenyl group optionally substituted with one or more substituents selected from the group consisting of fluorine atoms, diphenylamino groups or C1 to C6 fluoroalkyl groups, with the diphenylamino group optionally being substituted with one or more fluorine atoms, C1 to C6 fluoroalkyl groups or cyano groups.

17. The rare earth complex according to Claim 1, wherein the general formula (lcu) is a coumarinato ligand indicated by any of general formulae (lcu-1) to (lcu-6) below. 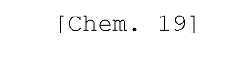 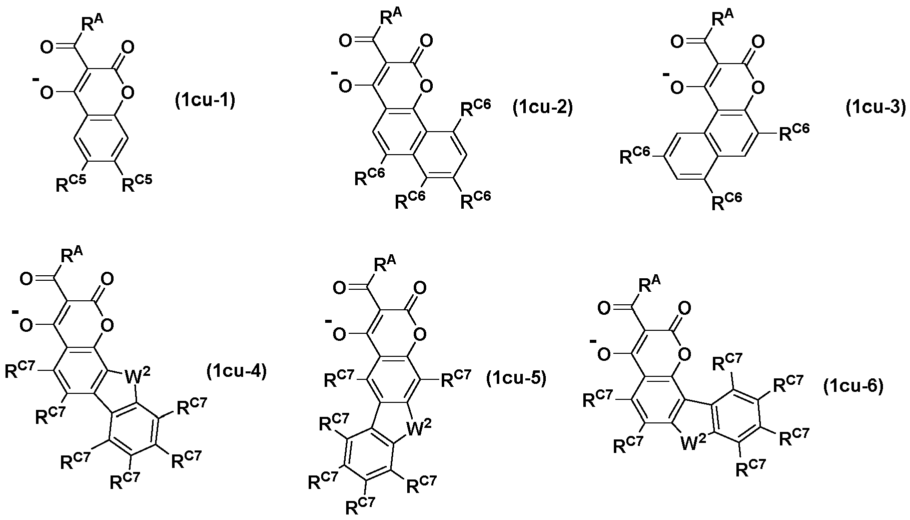 [In the formulae, R^{A} is synonymous with R^{A} in general formula (lcu).
R^{C5}s each independently represent a hydrogen atom, halogen atom, cyano group, C1 to C8 alkyl group, C1 to C4 fluoroalkyl group, C6 to C22 aryl group, substituted or unsubstituted amino group indicated by general formula (BA3) below or substituted or unsubstituted oxy group indicated by general formula (BO3) below. 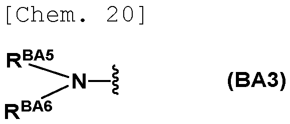
(In the formula, R^{BA5} and R^{BA6} each independently represent a C1 to C4 alkyl group or C6 to C12 aryl group, with the alkyl group and the aryl group optionally being substituted with one or more substituents selected from the group consisting of halogen atoms, C1 to C4 alkyloxy groups, C1 to C4 fluoroalkyl groups, C2 to C13 acyl groups, cyano groups and nitro groups. R^{BA5} and R^{BA6} optionally form a five-membered, six-membered or seven-membered ring including the nitrogen atom to which the R^{BA5} and R^{BA6} are bound.) 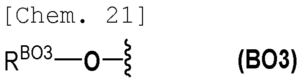
(In the formula, R^{BO3} represents a C1 to C4 alkyl group or C6 to C12 aryl group, with the alkyl group and the aryl group optionally being substituted with one or more substituents selected from the group consisting of halogen atoms, C1 to C4 alkyloxy groups, C1 to C4 fluoroalkyl groups, C2 to C13 acyl groups, cyano groups and nitro groups.)
The adjacent R^{C5}s optionally form a five-membered, six-membered or seven-membered ring together with the benzene ring to which the R^{C5}s are bound.
R^{C6}s each independently represent a hydrogen atom or C1 to C4 alkyloxy group.
R^{C7}s each independently represent a hydrogen atom, halogen atom or C1 to C4 fluoroalkyl group.
W² represents an oxygen atom, sulfur atom, nitrogen atom optionally substituted with a C1 to C12 hydrocarbon group or methylene group optionally substituted with one or more C1 to C12 hydrocarbon groups.]

18. The rare earth complex according to Claim 17, wherein R^{C7} is a hydrogen atom.

19. The rare earth complex according to Claim 17, wherein R^{C5} is a hydrogen atom, C1 to C4 alkyl group, C1 to C4 alkyloxy group, C2 to C8 dialkylamino group or diphenylamino group optionally substituted with one or more fluorine atoms; C1 to C4 fluoroalkyl groups; or; cyano groups.

20. The rare earth complex according to Claim 17, wherein W² is an oxygen atom, sulfur atom, methylene group optionally substituted with one or more C1 to C4 alkyl groups or nitrogen atom optionally substituted with a C1 to C10 alkyl group or C6 to C12 aryl group.

21. The rare earth complex according to Claim 1, wherein HC is a coumarinato ligand represented by general formula (lcu), m¹ is 1 or 2, and L¹ is phenanthroline optionally substituted with one or more methyl groups or phenyl groups, bipyridine optionally substituted with one or more methyl groups or phenyl groups, N,N-diethyl-4-{[4,6-bis(3,5-dimethyl-1H-pyrazol-1-yl)-1,3,5-triazin-2-yl]}aniline or dipyrido[3,2-a:2',3'-c]phenazine.

22. The rare earth complex according to Claim 1, wherein HC is a coumarinato ligand represented by general formula (lcu), m² is 1, 2 or 3, and L² is water.

23. The rare earth complex according to Claim 1, wherein HC is a coumarinato ligand represented by general formula (lcu), and, in general formulae (3a) and (3b), X^{A} is a C4 to C8 alkyl group or aryl group represented by general formula (3d) below, and X^{H} is a C1 to C4 alkylene group; diphenylether-2,2'-diyl group; naphthalen-1,8-diyl group; biphenyl-2,2'-diyl group; binaphthyl-2,2'-diyl group; bipyridin-2,2'-diyl group; or xanthendiyl group optionally substituted with one or more methyl groups. 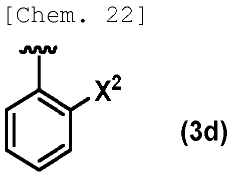 (In the formula, X² represents a hydrogen atom; fluorine atom; C1 to C4 alkyl group; C1 to C4 alkyloxy group; naphthyl group; pyridyl group; C1 to C4 fluoroalkyl group; or phenyl group optionally substituted with one or more fluorine atoms, C1 to C4 alkyl groups, C1 to C4 alkyloxy groups or C1 to C4 fluoroalkyl groups.)

24. The rare earth complex according to Claim 23, wherein X² in the general formula (3d) is a hydrogen atom, C1 to C4 alkyl group, C1 to C4 alkyloxy group or phenyl group optionally substituted with one or more C1 to C4 alkyl groups or C1 to C4 alkyloxy groups.

25. The rare earth complex according to Claim 1, wherein HC is a coumarinato ligand represented by general formula (1cu), m¹ is 1 or 2, and m² is 0.

26. A method for manufacturing a rare earth complex (1), the method comprising allowing an enol represented by general formula (4b) or general formula (4c) below, a phosphorus ligand indicated by general formula (3a) below; a phosphorus ligand indicated by general formula (3b) below; or a nitrogen-containing compound having two or more nitrogen atoms possessing a lone pair, which is represented by L¹, or/and a neutral ligand, which is represented by L², selected from the group consisting of water; deuterium oxide; a sulfoxide compound; sulfone compound; amide compound; nitrile compound; ester compound; carbonyl compound; ether compound and alcohol and a rare earth compound to react together. 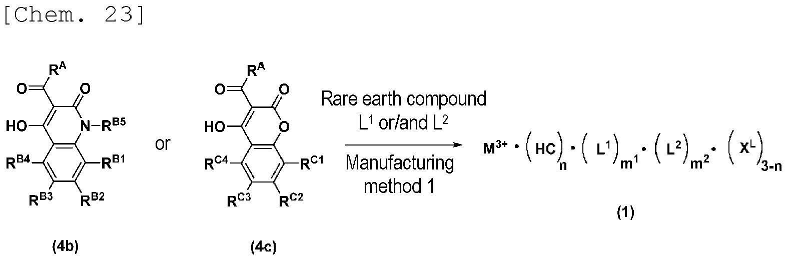 {HC is a quinolinonato ligand represented by general formula (lqu) or coumarinato ligand represented by general formula (lcu). 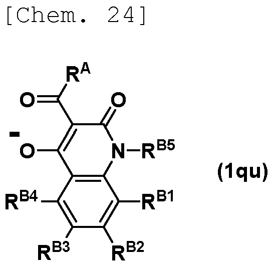 [In the formula, R^{A} represents a C1 to C6 haloalkyl group, C6 to C22 aryl group or C3 to C20 heteroaryl group. The aryl group and the heteroaryl group are optionally substituted with one or more substituents selected from the group consisting of C1 to C6 fluoroalkyl groups; C1 to C6 alkyloxy groups; C1 to C6 fluoroalkyloxy groups; C5 to C14 aryloxy groups; C1 to C6 monoalkylamino groups; C6 to C12 monoarylamino groups; C4 to C12 monoheteroarylamino groups; C2 to C12 dialkylamino groups; C10 to C24 diarylamino groups optionally substituted with one or more cyano groups, halogen atoms or C1 to C6 fluoroalkyl groups; C8 to C24 diheteroarylamino groups optionally substituted with one or more fluorine atoms or C1 to C6 fluoroalkyl groups; C3 to C20 heteroaryl groups; C2 to C13 acyl groups; methylenedioxy groups; ethylenedioxy groups; halogen atoms; hydroxyl groups; nitro groups and cyano groups.
R^{B1}, R^{B2}, R^{B3}, R^{B4} and R^{B5} each independently represent a hydrogen atom, halogen atom, C1 to C10 alkyl group, C2 to C4 alkenyl group, C1 to C6 haloalkyl group, C6 to C22 aryl group, C3 to C20 heteroaryl group, cyano group, nitro group, C7 to C14 aralkyl group optionally substituted with one or more halogen atoms, substituted or unsubstituted amino group indicated by general formula (BA1) below, substituted or unsubstituted oxy group indicated by general formula (BO1) below or substituted or unsubstituted thio group indicated by general formula (BS1) below, with the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, cyano groups and nitro groups. 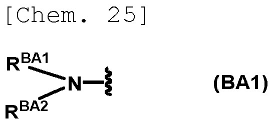
(In the formula, R^{BA1} and R^{BA2} each independently represent a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups. R^{BA1} and R^{BA2} optionally form a five-membered, six-membered or seven-membered ring including the nitrogen atom to which the R^{BA1} and R^{BA2} are bound.) 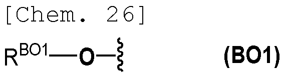
(In the formula, R^{BO1} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.) 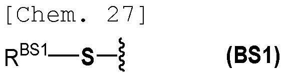
(In the formula, R^{BS1} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)
Of R^{B1}, R^{B2}, R^{B3} and R^{B4}, two adjacent substituents optionally form a five-membered, six-membered or seven-membered ring together with the benzene ring to which the substituents are bound.
R^{B5} and R^{B1} optionally form a five-membered, six-membered or seven-membered ring together with the quinolinone ring to which the R^{B5} and R^{B1} are bound.] 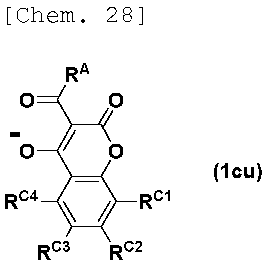
[In the formula, R^{A} is synonymous with R^{A} in general formula (lqu).
R^{C1}, R^{C2}, R^{C3} and R^{C4} each independently represent a hydrogen atom, halogen atom, C1 to C10 alkyl group, C2 to C4 alkenyl group optionally substituted with one or more C1 to C6 alkyloxy groups, C1 to C6 haloalkyl group, C6 to C22 aryl group, C3 to C20 heteroaryl group, cyano group, nitro group, C7 to C14 aralkyl group optionally substituted with one or more halogen atoms, substituted or unsubstituted amino group indicated by general formula (BA1) below, substituted or unsubstituted oxy group indicated by general formula (BO1) below or substituted or unsubstituted thio group indicated by general formula (BS1) below, with the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, cyano groups and nitro groups. 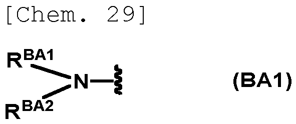
(In the formula, R^{BA1} and R^{BA2} are synonymous with the meanings described above.) 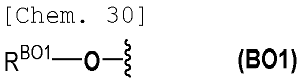
(In the formula, R^{BO1} is synonymous with the meaning described above.) 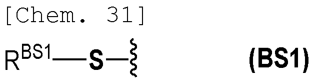
(In the formula, R^{BS1} is synonymous with the meaning described above.)
Of R^{C1}, R^{C2}, R^{C3} and R^{C4}, two adjacent substituents optionally form a five-membered, six-membered or seven-membered ring together with the benzene ring to which the substituents are bound.]
n represents 1, 2 or 3. When n is 2 or 3, the plurality of quinolinonato ligands or coumarinato ligands may be the same or different from each other.
m¹ represents 0, 1 or 2, m² represents 0, 1, 2 or 3, and 0 ≤ m¹ + m² ≤ 3.
L¹ represents a nitrogen-containing ligand having two or more nitrogen atoms possessing a lone pair or phosphorus ligand represented by general formula (3a) below or general formula (3b) below. When m¹ is 2, the L¹s may be the same or different from each other. 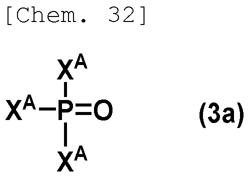 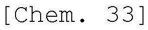 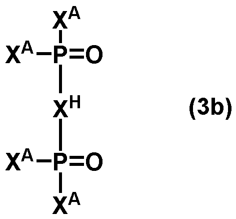
[In the formulae, the X^{A}s each independently represent a C1 to C10 alkyl group, aryl group represented by general formula (3c) below, substituted or unsubstituted amino group indicated by general formula (BA2) below or substituted or unsubstituted oxy group indicated by general formula (BO2) below. 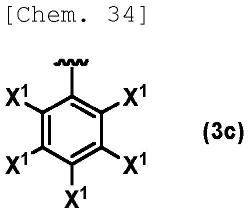
(In the formula, the X¹s each independently represent a hydrogen atom, halogen atom, C1 to C6 alkyl group, C1 to C6 alkyloxy group, C6 to C22 aryl group, C3 to C20 heteroaryl group, C5 to C14 aryloxy group, C1 to C6 haloalkyl group or C1 to C6 haloalkyloxy group, with the aryl group optionally being substituted with one or more substituents selected from the group consisting of halogen atoms, C1 to C6 alkyloxy groups, C1 to C6 haloalkyl groups, C1 to C6 haloalkyloxy groups, hydroxyl groups, cyano groups and nitro groups.) 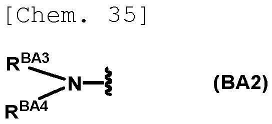
(In the formula, R^{BA3} and R^{BA4} each independently represent a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups. R^{BA3} and R^{BA4} optionally form a five-membered, six-membered or seven-membered ring including the nitrogen atom to which the R^{BA3} and R^{BA4} are bound.) 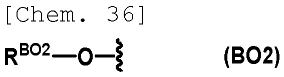
(In the formula, R^{BO2} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)
X^{H} represents a C1 to C10 alkylene group, C2 to C10 alkenylene group, C2 to C10 alkynylene group, C5 to C24 arylene group or C4 to C23 heteroarylene group.]
L² represents a neutral ligand selected from the group consisting of water, deuterium oxide, a sulfoxide compound, sulfone compound, amide compound, nitrile compound, ester compound, carbonyl compound, ether compound and alcohol.
When m² is 2 or 3, the L²s may be the same or different from each other.
X^{L} represents a halide ion, nitrate ion, carboxylate ion, sulfonate ion or C5 to C12 β-diketonato ion.
M³⁺ represents a trivalent rare earth metal ion.}

27. A method for manufacturing a rare earth complex (1), the method comprising allowing a diketonato complex represented by general formula (lag) below and a phosphorus ligand represented by general formula (3a) below; a phosphorus ligand represented by general formula (3b) below; or a nitrogen-containing ligand having two or more nitrogen atoms possessing a lone pair; which is represented by L¹, or/and a neutral ligand, which is represented by L², selected from the group consisting of water; deuterium oxide; a sulfoxide compound; sulfone compound; amide compound; nitrile compound; ester compound; carbonyl compound; ether compound and alcohol to react together. 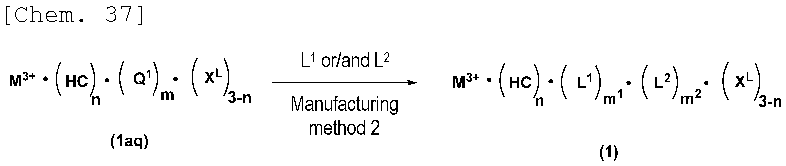 {HC is a quinolinonato ligand represented by general formula (lqu) or coumarinato ligand represented by general formula (lcu). 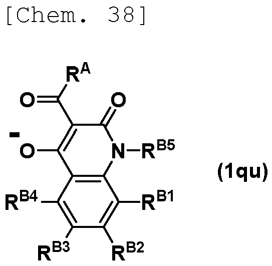 [In the formula, R^{A} represents a C1 to C6 haloalkyl group, C6 to C22 aryl group or C3 to C20 heteroaryl group. The aryl group and the heteroaryl group are optionally substituted with one or more substituents selected from the group consisting of C1 to C6 fluoroalkyl groups; C1 to C6 alkyloxy groups; C1 to C6 fluoroalkyloxy group; C5 to C14 aryloxy groups; C1 to C6 monoalkylamino groups; C6 to C12 monoarylamino groups; C4 to C12 monoheteroarylamino groups; C2 to C12 dialkylamino groups; C10 to C24 diarylamino groups optionally substituted with one or more cyano groups, halogen atoms or C1 to C6 fluoroalkyl groups; C8 to C24 diheteroarylamino groups optionally substituted with one or more fluorine atoms or C1 to C6 fluoroalkyl groups; C3 to C20 heteroaryl groups; C2 to C13 acyl groups; methylenedioxy groups; ethylenedioxy groups; halogen atoms; hydroxyl groups; nitro groups and cyano groups.
R^{B1}, R^{B2}, R^{B3}, R^{B4} and R^{B5} each independently represent a hydrogen atom, halogen atom, C1 to C10 alkyl group, C2 to C4 alkenyl group, C1 to C6 haloalkyl group, C6 to C22 aryl group, C3 to C20 heteroaryl group, cyano group, nitro group, C7 to C14 aralkyl group optionally substituted with one or more halogen atoms, substituted or unsubstituted amino group indicated by general formula (BA1) below, substituted or unsubstituted oxy group indicated by general formula (BO1) below or substituted or unsubstituted thio group indicated by general formula (BS1) below, with the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, cyano groups and nitro groups. 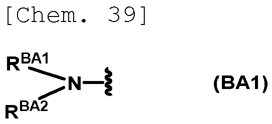
(In the formula, R^{BA1} and R^{BA2} each independently represent a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups. R^{BA1} and R^{BA2} optionally form a five-membered, six-membered or seven-membered ring including the nitrogen atom to which the R^{BA1} and R^{BA2} are bound.) 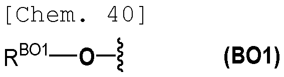
(In the formula, R^{BO1} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.) 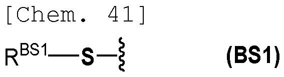
(In the formula, R^{BS1} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)
Of R^{B1}, R^{B2}, R^{B3} and R^{B4}, two adjacent substituents optionally form a five-membered, six-membered or seven-membered ring together with the benzene ring to which the substituents are bound.
R^{B5} and R^{B1} optionally form a five-membered, six-membered or seven-membered ring together with the quinolinone ring to which the R^{B5} and R^{B1} are bound.] 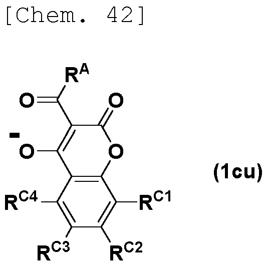
[In the formula, R^{A} is synonymous with R^{A} in general formula (lqu).
R^{C1}, R^{C2}, R^{C3} and R^{C4} each independently represent a hydrogen atom, halogen atom, C1 to C10 alkyl group, C2 to C4 alkenyl group optionally substituted with one or more C1 to C6 alkyloxy groups, C1 to C6 haloalkyl group, C6 to C22 aryl group, C3 to C20 heteroaryl group, cyano group, nitro group, C7 to C14 aralkyl group optionally substituted with one or more halogen atoms, substituted or unsubstituted amino group indicated by general formula (BA1) below, substituted or unsubstituted oxy group indicated by general formula (BO1) below or substituted or unsubstituted thio group indicated by general formula (BS1) below, with the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, cyano groups and nitro groups. 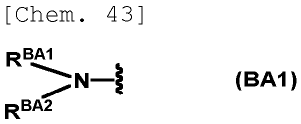
(In the formula, R^{BA1} and R^{BA2} are synonymous with the meanings described above.) 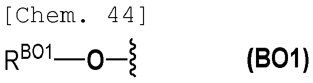
(In the formula, R^{BO1} is synonymous with the meaning described above.) 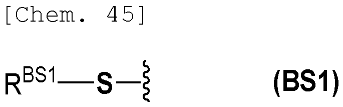
(In the formula, R^{BS1} is synonymous with the meaning described above.)
Of R^{C1}, R^{C2}, R^{C3} and R^{C4}, two adjacent substituents optionally form a five-membered, six-membered or seven-membered ring together with the benzene ring to which the substituents are bound.]
n represents 1, 2 or 3. When n is 2 or 3, the plurality of quinolinonato ligands or coumarinato ligands may be the same or different from each other.
m¹ represents 0, 1 or 2, m² represents 0 to 3, and 0 ≤ m¹ + m² ≤ 3.
L¹ represents a nitrogen-containing ligand having two or more nitrogen atoms possessing a lone pair or phosphorus ligand represented by general formula (3a) below or general formula (3b) below. When m¹ is 2, the L²s may be the same or different from each other. 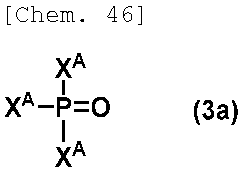 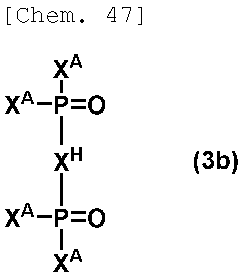
[In the formulae, the X^{A}s each independently represent a C1 to C10 alkyl group, aryl group represented by general formula (3c) below, substituted or unsubstituted amino group indicated by general formula (BA2) below or substituted or unsubstituted oxy group indicated by general formula (BO2) below. 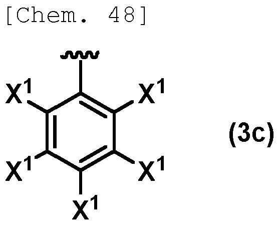
(In the formula, the X¹s each independently represent a hydrogen atom, halogen atom, C1 to C6 alkyl group, C1 to C6 alkyloxy group, C6 to C22 aryl group, C3 to C20 heteroaryl group, C5 to C14 aryloxy group, C1 to C6 haloalkyl group or C1 to C6 haloalkyloxy group, with the aryl group optionally being substituted with one or more substituents selected from the group consisting of halogen atoms, C1 to C6 alkyloxy groups, C1 to C6 haloalkyl groups, C1 to C6 haloalkyloxy groups, hydroxyl groups, cyano groups and nitro groups.) 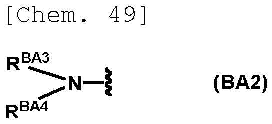
(In the formula, R^{BA3} and R^{BA4} each independently represent a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups. R^{BA3} and R^{BA4} optionally form a five-membered, six-membered or seven-membered ring including the nitrogen atom to which the R^{BA3} and R^{BA4} are bound.) 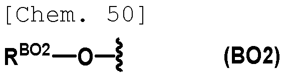
(In the formula, R^{BO2} represents a hydrogen atom, C1 to C6 alkyl group, C6 to C22 aryl group or C3 to C20 heteroaryl group, with the alkyl group, the aryl group and the heteroaryl group optionally being substituted with one or more substituents selected from the group consisting of halogen atoms, C1 to C6 alkyloxy groups, C1 to C6 fluoroalkyl groups, C2 to C13 acyl groups, hydroxyl groups, cyano groups and nitro groups.)
X^{H} represents a C1 to C10 alkylene group, C2 to C10 alkenylene group, C2 to C10 alkynylene group, C5 to C24 arylene group or C4 to C23 heteroarylene group.]
L² represents a neutral ligand selected from the group consisting of water, deuterium oxide, a sulfoxide compound, sulfone compound, amide compound, nitrile compound, ester compound, carbonyl compound, ether compound and alcohol.
When m² is 2 or 3, the L²s may be the same or different from each other.
X^{L} represents a halide ion, nitrate ion, carboxylate ion, sulfonate ion or C5 to C12 β-diketonato ion.
M³⁺ represents a trivalent rare earth metal ion.
In the formulae, m represents 0, 1, 2 or 3.
In the formulae, Q¹ represents a neutral molecule. When m is not 0 and when m¹ is 0, it is impossible that Q¹ and L² are the same, and m and m² are equal at the same time.

28. An optical material comprising the rare earth complex according to Claim 1.

29. An optical material comprising a resin material, inorganic glass, organic low-molecular-weight material or solvent and the rare earth complex according to Claim 1.

30. The optical material according to Claim 29, wherein the resin material is polymethyl methacrylate, polyethyl methacrylate, polypropyl methacrylate, polybutyl methacrylate, polymethyl acrylate, polyethyl acrylate, polypropyl acrylate, polybutyl acrylate, polyethylene, polystyrene, polyvinyl acetate and a copolymer thereof; an epoxy resin; a polyimide resin; or a silicone resin.

31. The optical material according to Claim 29, wherein the solvent is halogenated hydrocarbons, alcohols, esters, glycol ethers, ethers, ketones or hydrocarbons.

32. The optical material according to any one of Claims 28 to 31, wherein the optical material is a film for a solar cell, agricultural film, LED phosphor, light-emitting material, fluorescent material or wavelength conversion material.
